(19) Europäisches Patentamt
European Patent Office
Office européen des brevets

(11) EP 4 722 207 A1

(12) EUROPEAN PATENT APPLICATION
published in accordance with Art. 153(4) EPC

(43) Date of publication:
**08.04.2026 Bulletin 2026/15**

(21) Application number: **24814638.3**

(22) Date of filing: **03.06.2024**

(51) International Patent Classification (IPC):
***C07D 403/04*** (2006.01) ***C07D 401/04*** (2006.01)
***C07D 401/14*** (2006.01) ***C07D 487/00*** (2006.01)
***A61K 31/505*** (2006.01) ***A61K 31/454*** (2006.01)
***A61K 31/517*** (2006.01) ***A61P 37/02*** (2006.01)
***A61P 35/00*** (2006.01)

(52) Cooperative Patent Classification (CPC):
**A61K 31/454; A61K 31/505; A61K 31/517; A61P 35/00; A61P 37/02; C07D 401/04; C07D 401/14; C07D 403/04; C07D 487/00**

(86) International application number:
**PCT/CN2024/097059**

(87) International publication number:
**WO 2024/245444 (05.12.2024 Gazette 2024/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.06.2023 CN 202310646305**

(71) Applicant: **Gluetacs Therapeutics (Shanghai) Co., Ltd.**
**Shanghai 201306 (CN)**

(72) Inventors:
- **YANG, Xiaobao**
**Shanghai 201306 (CN)**
- **LI, Yan**
**Shanghai 201306 (CN)**
- **ZHOU, Yuedong**
**Shanghai 201306 (CN)**
- **SUN, Renhong**
**Shanghai 201306 (CN)**
- **ZHAO, Baoyin**
**Shanghai 201306 (CN)**

(74) Representative: **Dragsted Partners A/S**
**Rådhuspladsen 16**
**1550 Copenhagen V (DK)**

# (54) OXOISOINDOLINYL-SUBSTITUTED TETRAHYDROPYRIMIDINEDIONE DERIVATIVE AND USE THEREOF

(57) The present disclosure provides a compound of Formula (I) or salts, enantiomers, stereoisomers, solvates, or polymorphs thereof and uses thereof. The present disclosure also provides pharmaceutical compositions comprising, as an active ingredient, the compound of Formula (I) or salts, enantiomers, stereoisomers, solvates, or polymorphs thereof and uses thereof. The series of compounds designed and synthesized in the present disclosure can effectively prevent and/or treat diseases or disorders associated with the cereblon protein.

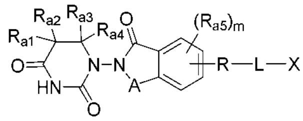

Formula (I)

EP 4 722 207 A1

## Description

### Technical Field

The present disclosure relates to a compound of Formula (I) or salts, enantiomers, stereoisomers, solvates, or polymorphs thereof and uses thereof, especially their use in the prevention or treatment of diseases or disorders associated with cereblon protein (CRBN).

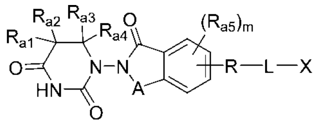

Formula (I)

### Background

Immunomodulatory drugs (IMiDs) of the phthalimide class, such as thalidomide and lenalidomide, have shown remarkable efficacy in treating multiple myeloma and autoimmune diseases. However, it was not until 2010 that their direct binding target was identified as the E3 ubiquitin ligase cereblon (CRBN). Subsequent studies confirmed that upon binding to CRBN, these drugs act as molecular glues, recruiting substrate proteins (e.g., the transcription factors IKZF1/3) and inducing protein-protein interactions between CRBN and the substrates. This leads to the ubiquitination of the substrate proteins, which are then recognized and degraded by the proteasome, thereby eliciting pharmacological effects such as antitumor and immunomodulatory activities. Molecular glue degraders directly target and degrade specific proteins, offering potential advantages such as targeting "undruggable" proteins. Moreover, molecular glues typically exhibit low molecular weight and favorable drug-like properties, making their development highly challenging. Based on the CRBN E3 ubiquitin ligase and the molecular glue degradation mechanism, a series of compounds have been developed, including the approved pomalidomide and several candidates currently in clinical trials, such as Bristol Myers Squibb's CC-122, CC-220, CC-90009, CC-99282, and CC-92480; Novartis's DKY709; and C4 Therapeutics's CFT7455. The range of substrates degraded by these molecular glues has expanded from the initially identified transcription factors IKZF1/3 to include casein kinase 1α (CK1α), zinc finger protein 91 (ZFP91), and the translation factor GSPT1. Degradation of these protein substrates enables molecular glues to exert immunomodulatory, anti-inflammatory, and antitumor activities. Currently, the structural novelty of designed molecular glue candidates remains quite limited, making the diversification of molecular scaffolds and the development of novel molecular glues a key research focus in the field.

Many existing IMiD-based compounds often contain a piperidine-2,6-dione-substituted isoindolinone structure (i.e.,

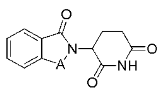

, where A represents CO or $CH_2$), which includes a chiral center existing in R and S configurations. Obtaining a single chiral compound typically requires costly methods such as asymmetric synthesis, enzymatic catalysis, or chiral resolution. The isoindolinyl-substituted tetrahydropyrimidinedione scaffold designed in the present invention addresses the chirality issue associated with the existing piperidine-2,6-dione-substituted isoindolinone structures. Moreover, it exhibits CRBN binding affinity, favorable pharmacokinetic properties, or antitumor activity.

### Summary of Invention

In view of the foregoing, the objectvies of the present disclosure are to provide novel molecular glue protein degraders based on an oxoisoindolinyl-substituted tetrahydropyrimidinedione scaffold, as well as their preparation methods, uses, and usage methods.

To achieve the above objectives and other related goals, in one aspect, the present disclosure provides a compound of Formula (I):

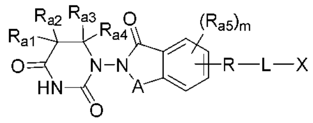

Formula (I)

or salts, stereoisomers (including enantiomers and diastereoisomer), solvates, or polymorphs thereof,

wherein A represents CO, $CH_2$, or $CD_2$;
$R_{a1}$, $R_{a2}$, $R_{a3}$, and $R_{a4}$ each independently represent hydrogen, deuterium, halogen, $C_{1-6}$ alkyl, halogenated $C_{1-6}$ alkyl, deuterated $C_{1-6}$ alkyl, $C_{1-6}$ alkoxy, deuterated $C_{1-6}$ alkoxy, or halogenated $C_{1-6}$ alkoxy;
$(R_{a5})_m$ indicates that the isoindoline ring to which it is attached is optionally substituted with m $R_{a5}$, with each $R_{a5}$ being the same or different and each independently representing deuterium, halogen, hydroxy, mercapto, nitro, amino, cyano, $C_{1-6}$ alkyl, halogenated $C_{1-6}$ alkyl, deuterated $C_{1-6}$ alkyl, $C_{1-6}$ alkoxy, deuterated $C_{1-6}$ alkoxy, halogenated $C_{1-6}$ alkoxy, $C_{2-6}$ alkenyl or $C_{2-6}$ alkynyl;
m represents an integer of 0, 1, 2, or 3;
R represents O, S, S(O), $S(O)_2$, alkenylene, alkynylene, or $N(R_{a6})$, wherein $R_{a6}$ represents H or $C_{1-3}$ alkyl, or R represents a bond; or
R represents:

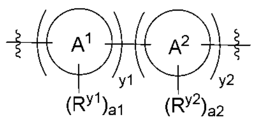

wherein each ring $A^1$ is the same or different and independently represents nitrogen-containing heterocyclylene, arylene, or heteroarylene, y1 represents an integer of 1 or 2, and $(R^{y1})_{a1}$ indicates that each ring $A^1$ is independently optionally substituted with a1 $R^{y1}$ substituents, wherein each $R^{y1}$ independently represents deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, $C_{2-6}$ alkynyl or $C_{2-6}$ alkenyl, and a1 represents an integer of 0-20;
each ring $A^2$ is the same or different and independently represents heterocyclylene, cycloalkylene, arylene, or heteroarylene, y2 represents an integer of 0 or 1, and $(R^{y2})_{a2}$ indicates that each ring $A^2$ is independently optionally substituted with a2 $R^{y2}$ substituents, wherein each $R^{y2}$ independently represents deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, $C_{2-6}$ alkynyl or $C_{2-6}$ alkenyl, and a2 represents an integer of 0-20;

L represents:

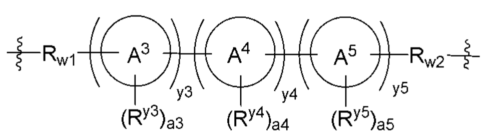

wherein $R_{w1}$ is linked to R, and $R_{w2}$ is linked to X;
$R_{w2}$ represents a bond, C(O), C(O)NH, NHC(O), or $N(R_{a6})$, wherein $R_{a6}$ represents H or $C_{1-3}$ alkyl;
each ring $A^3$ is the same or different and independently represents heterocyclylene, cycloalkylene, arylene, or heteroarylene, y3 represents an integer of 0, 1, 2, or 3, and $(R^{y3})_{a3}$ indicates that each ring $A^3$ is independently optionally substituted with a3 $R^{y3}$ substituents, wherein each $R^{y3}$ independently represents deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, $C_{2-6}$ alkynyl or $C_{2-6}$ alkenyl, and a3 represents an integer of 0-20;
each ring $A^4$ is the same or different and independently represents heterocyclylene, cycloalkylene, arylene, or heteroarylene, y4 represents an integer of 0, 1, 2, or 3, and $(R^{y4})_{a4}$ indicates that each ring $A^4$ is independently optionally substituted with a4 $R^{y4}$ substituents, wherein each $R^{y4}$ independently represents deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, $C_{2-6}$ alkynyl or $C_{2-6}$ alkenyl, and a4 represents an integer of 0-20;
each ring $A^5$ is the same or different and independently represents heterocyclylene, cycloalkylene, arylene, or heteroarylene, y5 represents an integer of 0, 1, 2, or 3, and $(R^{y5})_{a5}$ indicates that each ring $A^5$ is independently

optionally substituted with a5 $R^{y5}$ substituents, wherein each $R^{y5}$ independently represents deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, $C_{2-6}$ alkynyl or $C_{2-6}$ alkenyl, and a5 represents an integer of 0-20;

$R_{w1}$ represents a bond or optionally substituted linear or branched $C_{1-20}$ alkylene, wherein any one or more methylene groups in the main carbon chain skeleton of the linear or branched $C_{1-20}$ alkylene are optionally replaced by one or more groups selected from $R_a$, $R_b$, and any combination thereof; wherein each $R_a$ is independently selected from the group consisting of: O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, where each $R_{a7}$ independently represents H or $C_{1-6}$ alkyl, and when any two or more methylene in the main carbon chain skeleton of the linear or branched $C_{1-20}$ alkylene are replaced by two or more $R_a$ groups, the $R_a$ groups are not directly connected to each other; and wherein each $R_b$ is independently selected from the group consisting of: optionally substituted cycloalkylene, optionally substituted arylene, optionally substituted heterocyclylene, optionally substituted heteroarylene, alkynylene, alkenylene, and any combination thereof;

X represents:

$NR_1R_2$, $C(O)NR_3R_4$, $NHC(O)R_5$, $NHC(O)NR_6R_7$, $OR_8$, $SR_9$, $S(O)_2NR_{10}R_{11}$, or $N(R_{12})S(O)_2R_{13}$, wherein $R_1$, $R_3$, $R_4$, $R_5$, $R_6$, $R_7$, $R_9$, $R_{10}$, $R_{11}$, and $R_{12}$ each independently represent H, optionally substituted linear or branched alkyl, optionally substituted cycloalkyl, optionally substituted aryl, optionally substituted heterocyclyl, or optionally substituted heteroaryl, and wherein $R_2$, $R_8$, and $R_{13}$ each independently represent optionally substituted linear or branched alkyl, optionally substituted cycloalkyl, optionally substituted aryl, optionally substituted heterocyclyl, or optionally substituted heteroaryl, or wherein $R_3$ and $R_4$, together with the nitrogen atom to which they are attached, form an optionally substituted 4- to 30-membered nitrogen-containing heterocyclyl; or

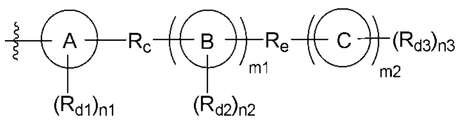

wherein ring A represents heterocyclylene, cycloalkylene, arylene, or heteroarylene, and $(R_{d1})_{n1}$ indicates that ring A is optionally substituted with n1 $R_{d1}$ substituents, wherein each $R_{d1}$ independently represents deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, oxo, $C_{2-6}$ alkynyl or $C_{2-6}$ alkenyl, and n1 represents an integer of 0-20;

$R_c$ represents $NR_{c1}$, C(O), $CR_{c2}R_{c3}$, or a bond, wherein $R_{c1}$ represents H, optionally substituted linear or branched alkyl, optionally substituted cycloalkyl, optionally substituted aryl, optionally substituted heterocyclyl, or optionally substituted heteroaryl, wherein $R_{c2}$ and $R_{c3}$ each independently represent H, halogen, optionally substituted linear or branched alkyl, optionally substituted cycloalkyl, optionally substituted aryl, optionally substituted heterocyclyl, or optionally substituted heteroaryl;

each ring B is the same or different and independently represents heterocyclylene, cycloalkylene, arylene, or heteroarylene, m1 represents an integer of 0, 1, 2, or 3, and $(R_{d2})_{n2}$ indicates that each ring B is independently optionally substituted with n2 $R_{d2}$ substituents, wherein each $R_{d2}$ independently represents deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, oxo, $C_{2-6}$ alkynyl or $C_{2-6}$ alkenyl, and n2 represents an integer of 0-20;

$R_c$ represents S, O, $CR_{c1}R_{c2}$, or a bond, wherein $R_{c1}$ and $R_{c2}$ each independently represent H, halogen, optionally substituted linear or branched alkyl, optionally substituted cycloalkyl, optionally substituted aryl, optionally substituted heterocyclyl, or optionally substituted heteroaryl; and

each ring C is the same or different and independently represents heterocyclyl, cycloalkyl, aryl, or heteroaryl, m2 represents an integer of 0, 1, 2, or 3, and $(R_{d3})_{n3}$ indicates that each ring C is independently optionally substituted with n3 $R_{d3}$ substituents, wherein each $R_{d3}$ independently represents deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, oxo, $C_{2-6}$ alkynyl or $C_{2-6}$ alkenyl, and n3 represents an integer of 0-20; or

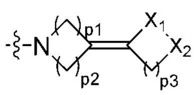

wherein $-X_1-X_2-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;

p1, p2, and p3 each independently represent an integer of 1, 2, 3, or 4; and

the ring containing $-X_1-X_2-$ is optionally substituted with one or more substituents selected from the group consisting of deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, oxo, $C_{2-6}$ alkynyl, $C_{2-6}$ alkenyl, or any combination thereof; or

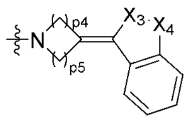

wherein $-X_3-X_4-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;

p4 and p5 each independently represent an integer of 1, 2, 3, or 4; and

the benzo-fused ring containing-$X_3$-$X_4$- is optionally substituted with one or more substituents selected from the group consisting of deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, oxo, $C_{2-6}$ alkynyl, $C_{2-6}$ alkenyl, or any combination thereof.

In a further aspect, the present disclosure provides a pharmaceutical composition comprising the compound of Formula (I) or pharmaceutically acceptable salts, stereoisomers (including enantiomers and diastereomers), solvates, isotopically enriched analogs, prodrugs, or polymorphs thereof, and at least one pharmaceutically acceptable carrier.

In a further aspect, the present disclosure further provides a medicine kit or reagent kit comprising: the compound of Formula (I) or a pharmaceutically acceptable salt, or the pharmaceutical composition comprising the same.

In a further aspect, the present disclosure provides the compound of Formula (I) or pharmaceutically acceptable salts, enantiomers, diastereomers, solvates, prodrugs, or polymorphs thereof, for use as a medicament.

In a further aspect, the present disclosure provides the compound of Formula (I) or pharmaceutically acceptable salts, stereoisomers (including enantiomers and diastereomers), solvates, isotopically enriched analogs, prodrugs, or polymorphs thereof, or the pharmaceutical composition of the present disclosure, for use in the treatment or prevention of tumors or cancers.

In a further aspect, the present disclosure provides the use of the compound of Formula (I) or pharmaceutically acceptable salts, stereoisomers (including enantiomers and diastereomers), solvates, isotopically enriched analogs, prodrugs, or polymorphs thereof, or the pharmaceutical composition of the present disclosure, for the manufacture of a medicament for the prevention or treatment of tumors or cancers.

In a further aspect, the present disclosure provides a method for treating or preventing tumors or cancers in a subject, comprising administering to the subject a therapeutically effective amount of the compound of Formula (I) or pharmaceutically acceptable salts, stereoisomers (including enantiomers and diastereomers), solvates, isotopically enriched analogs, prodrugs, or polymorphs thereof, or the pharmaceutical composition of the present disclosure.

In a further aspect, the present disclosure provides a method for preparing a compound of Formula (II), comprising:

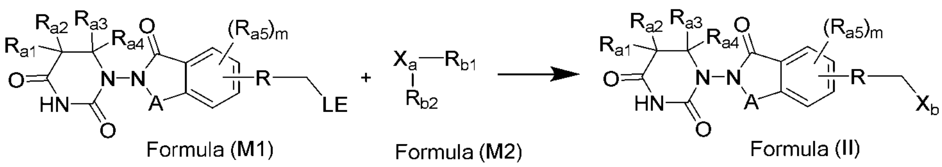

Formula (M1) Formula (M2) Formula (II)

wherein $R_{a1}$, $R_{a2}$, $R_{a3}$, $R_{a4}$, $(R_{a5})_m$, A, and R are as defined in the compound of Formula (I) and its various sub-embodiments in the present disclosure; and

wherein

(i) group LE represents Cl, Br, I, OMs, OTs, or ONs; $X_a$ represents NH; $R_{b1}$ represents $R_1$ which is as defined in the compound of Formula (I) and its various sub-embodiments in the present disclosure; $R_{b2}$ represents $R_2$ which is as defined in the compound of Formula (I) and its various sub-embodiments in the present disclosure; and $X_b$ accordingly represents $NR_1R_2$, wherein $R_1$ and $R_2$ are as defined in the compound of Formula (I) and its various sub-embodiments in the present disclosure; or

(ii) group LE represents Cl, Br, I, OMs, OTs, or ONs; groups $R_{b1}$ and $R_{b2}$, together with the nitrogen atom to which they are attached, form an optionally substituted nitrogen-containing heterocycle $A_a$ represented by the following general formula:

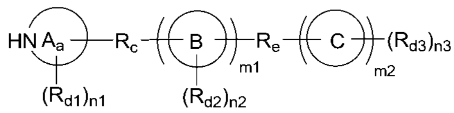

and $X_b$ accordingly represents a structure represented by the following formula:

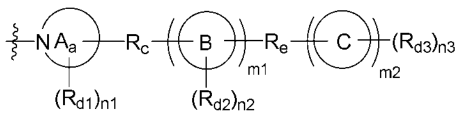

wherein nitrogen-containing heterocycle $A_a$ represents 4- to 30-membered nitrogen-containing heterocyclyl (preferably, 4- to 20-membered nitrogen-containing heterocyclyl; more preferably, 4- to 15-membered nitrogen-containing heterocyclyl); and $(R_{d1})_{n1}$, $R_c$, ring B, m1, $(R_{d2})_{n2}$, $R_c$, ring C, m2, and $(R_{d3})_{n3}$ are as defined in the compound of Formula (I) and its various sub-embodiments in the present disclosure; or

(iii) the group LE represents C(O)Cl, COOH, or $S(O)_2Cl$; the compound of formula (M2), $X_aR_{b1}R_{b2}$, is $NHR_3R_4$ or $NHR_{10}R_{11}$; and $X_b$ accordingly represents $C(O)NR_3R_4$ or $S(O)_2NR_{10}R_{11}$, wherein $R_3$, $R_4$, $R_{10}$, and $R_{11}$ are as defined in the compound of Formula (I) and its various sub-embodiments in the present disclosure; or

(iv) the group LE represents $NH_2$; group $X_a$ represents C(O) or $S(O)_2$; group $R_{b1}$ represents OH or Cl; $R_{b2}$ is group $R_5$ or $R_{13}$ which are as defined in the compound of Formula (I) and its various sub-embodiments in the present disclosure; and $X_b$ accordingly represents $NHC(O)R_5$ or $NHS(O)_2R_{13}$, wherein $R_5$ and $R_{13}$ are as defined in the compound of Formula (I) and its various sub-embodiments in the present disclosure; or

(v) the group LE represents Cl, Br, or I; group $X_a$ represents O or S; group $R_{b1}$ represents H; $R_{b2}$ is group $R_8$ or $R_9$ which are as defined in the compound of Formula (I) and its various sub-embodiments in the present disclosure; and $X_b$ accordingly represents $OR_8$ or $SR_9$, wherein $R_8$ and $R_9$ are as defined in the compound of Formula (I) and its various sub-embodiments in the present disclosure.

In a further aspect, the present disclosure provides a method for preparing a compound of Formula (M1), comprising preparing the compound of Formula (M1) by a halogenation reaction of a compound of Formula (M3) with a hydrohalic acid:

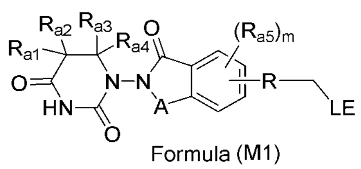

Formula (M1)

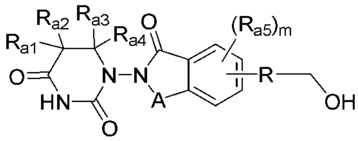

Formula (M3)

wherein group LE represents Cl, Br, or I; $R_{a1}$, $R_{a2}$, $R_{a3}$, $R_{a4}$, $(R_{a5})_m$, and A are as defined in the compound of Formula (I) and its various sub-embodiments in the present disclosure; and R represents a bond.

In a further aspect, the present disclosure provides a method for preparing a compound of Formula (M4), comprising preparing the compound of Formula (M4) by an aminolysis reaction of an ester with an amine, wherein the ester is a compound of Formula (M6) and the amine is a compound of Formula (M5):

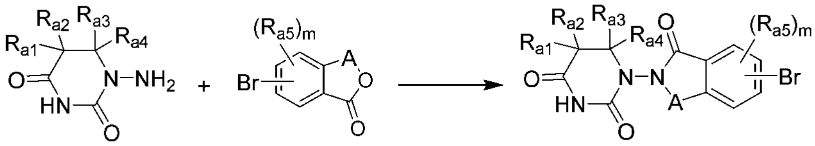

Formula (M5) Formula (M6) Formula (M4)

wherein $R_{a1}$, $R_{a2}$, $R_{a3}$, $R_{a4}$, $(R_{a5})_m$, and A are as defined in the compound of Formula (I) and its various sub-embodiments in the present disclosure.

## Description of Drawings

Figure 1 shows the Western blot experimental results of the degradation of the target substrate protein by the compounds of the present disclosure in cells.

## Detailed Description of the Invention

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

## I. Compounds

### Compounds of Formula (I)

The present disclosure provides a compound of Formula (I) or salts (including pharmaceutically acceptable salts), stereoisomers (including enantiomers and diastereomers), solvates, isotopically enriched analogs, prodrugs, or polymorphs thereof:

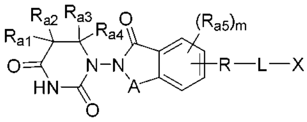

Formula (I)

wherein A, $R_{a1}$, $R_{a2}$, $R_{a3}$, $R_{a4}$, $(R_{a5})_m$, R, L, and X are as defined in the compound of Formula (I) and its various sub-embodiments in the present disclosure.

The compound of Formula (I) of the present disclosure, or salts (including pharmaceutically acceptable salts), stereoisomers (including enantiomers), solvates, isotopically enriched analogs, prodrugs, or polymorphs thereof, can act as molecular glues binding to the CRBN E3 ubiquitin ligase. The compound of Formula (I) of the present disclosure contains a dihydropyrimidine-2,4-dione-substituted isoindolinone backbone structure (i.e.,

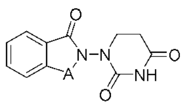

, wherein A represents CO or $CH_2$). The design of this backbone structure skillfully avoids the generation of chirality, which can address the chirality issues associated with existing piperidine-2,6-dione-substituted isoindolinone structures (i.e.,

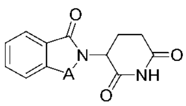

, wherein A represents CO or $CH_2$). It also exhibits CRBN binding affinity, favorable pharmacokinetic properties, or antitumor activity.

In some embodiments of the present disclosure, $R_{a1}$, $R_{a2}$, $R_{a3}$, and $R_{a4}$ are the same or different and each independently represent H, deuterium (i.e., D), $C_{1-6}$ alkyl (e.g., $C_{1-3}$ alkyl, such as methyl, ethyl, or propyl), halogenated $C_{1-6}$ alkyl (e.g., halogenated $C_{1-4}$ alkyl, such as trifluoromethyl), deuterated $C_{1-6}$ alkyl, $C_{1-6}$ alkoxy (e.g., $C_{1-4}$ alkoxy, such as methoxy), deuterated $C_{1-6}$ alkoxy, or halogenated $C_{1-6}$ alkoxy (e.g., halogenated $C_{1-4}$ alkoxy, such as trifluoromethoxy). In some sub-embodiments of the present disclosure, $R_{a1}$, $R_{a2}$, $R_{a3}$, and $R_{a4}$ each independently represent H.

In some embodiments of the present disclosure, A represents C(O).

In some embodiments of the present disclosure, A represents $CH_2$.

In some embodiments of the present disclosure, A represents $CD_2$.

In some embodiments of the present disclosure, $(R_{a5})_m$ indicates that the isoindoline ring in Formula (I) to which it is attached is optionally substituted with m $R_{a5}$, wherein each $R_{a5}$ is the same or different and each independently represents deuterium, halogen (e.g., fluorine, chlorine, bromine, or iodine), hydroxy, mercapto, nitro, amino, cyano, $C_{1-6}$ alkyl (e.g., $C_{1-5}$ alkyl, $C_{1-4}$ alkyl, or $C_{1-3}$ alkyl, such as methyl, ethyl, propyl, isopropyl, butyl, sec-butyl, tert-butyl, pentyl, or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), deuterated $C_{1-6}$ alkyl (e.g., perdeuterated $C_{1-6}$ alkyl,

perdeuterated $C_{1-5}$ alkyl, or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, n-butoxy, sec-butoxy, or tert-butoxy), deuterated $C_{1-6}$ alkoxy, halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-), $C_{2-6}$ alkenyl (e.g., vinyl), or $C_{2-6}$ alkynyl (e.g., ethynyl), and m represents an integer of 0, 1, 2, or 3. Alternatively, in some sub-embodiments of the present disclosure, m represents an integer of 0, 1, or 2. Alternatively, in some sub-embodiments of the present disclosure, each $R_{a5}$ is the same or different and each independently represents deuterium, halogen (e.g., fluorine, chlorine, bromine, or iodine), hydroxy, mercapto, nitro, amino, cyano, $C_{1-4}$ alkyl (e.g., $C_{1-3}$ alkyl, such as methyl, ethyl, propyl, isopropyl, butyl, sec-butyl, or tert-butyl,), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), $C_{1-4}$ alkoxy (e.g., $C_{1-3}$ alkoxy, such as methoxy, ethoxy, propoxy, isopropoxy, n-butoxy, sec-butoxy, or tert-butoxy), or halogenated $C_{1-4}$ alkoxy (e.g., halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-).

In embodiments of the present disclosure, the number of substituents is not theoretically limited in any way, or is automatically limited by the size of the building units.

In some embodiments of the present disclosure, R represents O, S, S(O), $S(O)_2$, alkenylene, alkynylene, or $N(R_{a6})$, wherein $R_{a6}$ represents H or $C_{1-3}$ alkyl. Alternatively, in some sub-embodiments of the present disclosure, R represents O, S, S(O), $S(O)_2$, $C_{2-6}$ alkenylene (e.g., vinylene), $C_{2-6}$ alkynylene (e.g., ethynylene), NH, or $N(CH_3)$.

In some embodiments of the present disclosure, R represents a bond.

In some embodiments of the present disclosure, R represents:

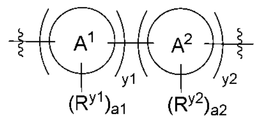

wherein each ring $A^1$ is the same or different and independently represents nitrogen-containing heterocyclylene, arylene, or heteroarylene, y1 represents an integer of 1 or 2, and $(R^{y1})_{a1}$ indicates that each ring $A^1$ is independently optionally substituted with a1 $R^{y1}$ substituents, wherein each $R^{y1}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a1 represents an integer of 0-20; and/or

each ring $A^2$ is the same or different and independently represents heterocyclylene, cycloalkylene, arylene, or heteroarylene, y2 represents an integer of 0 or 1, and $(R^{y2})_{a2}$ indicates that each ring $A^2$ is independently optionally substituted with a2 $R^{y2}$ substituents, wherein each $R^{y2}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a2 represents an integer of 0-20.

In some embodiments of the present disclosure, y1 represents an integer of 1 or 2, and each ring $A^1$ is the same or different and independently represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene) or 5- to 30-membered heteroarylene (e.g., 5- to 20-membered heteroarylene, or 5- to 15-membered heteroarylene).

In some embodiments of the present disclosure, y1 represents an integer of 1 or 2, and each ring $A^1$ independently represents:

piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo [2.2.2]octanylene, 3-azaspiro[5.5]undecanylene, 8-azaspiro[4.5]decanylene, 2-oxa-8-azaspiro[4.5]decanylene, 3-methyl-2-oxa-8-azaspiro[4.5]decenylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene.

In some embodiments of the present disclosure, y1 represents an integer of 1 or 2, and each ring $A^1$

independently represents:

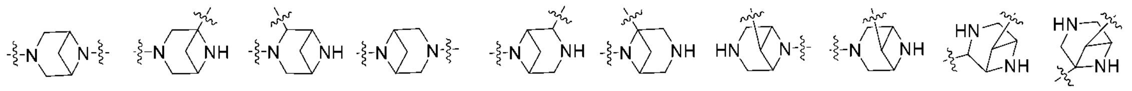

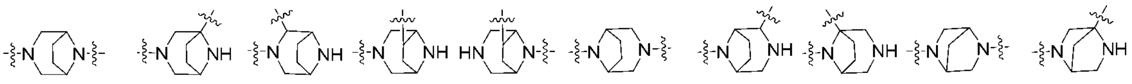

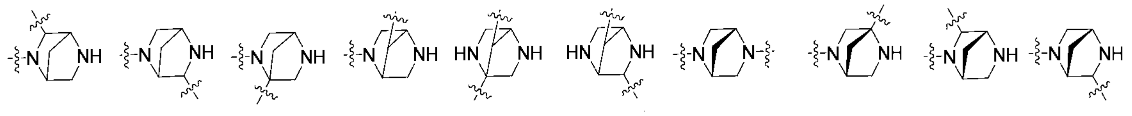

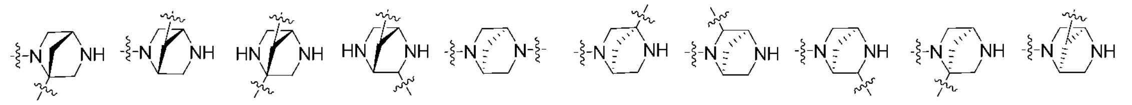

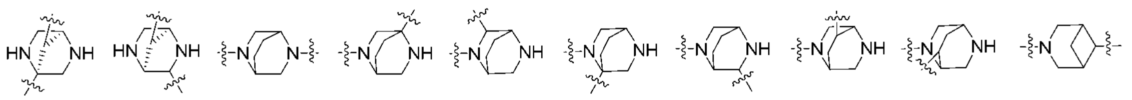

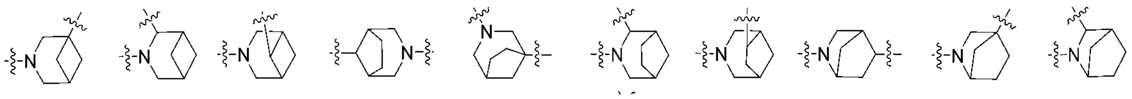

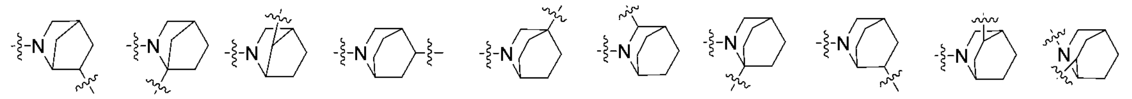

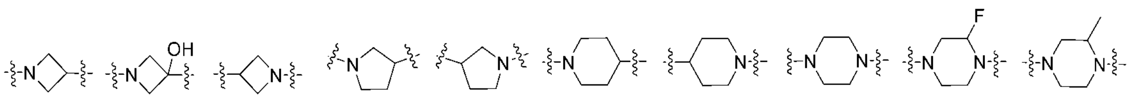

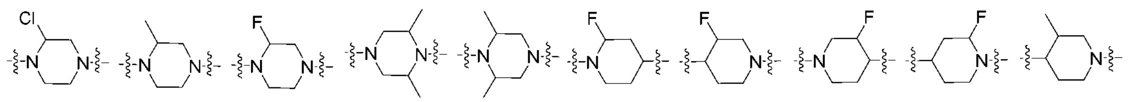

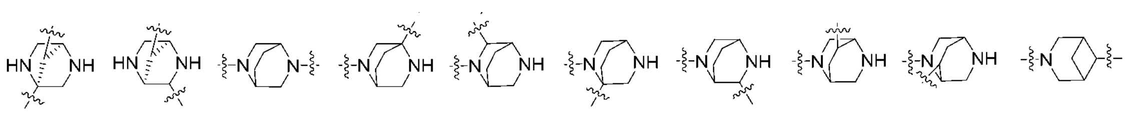

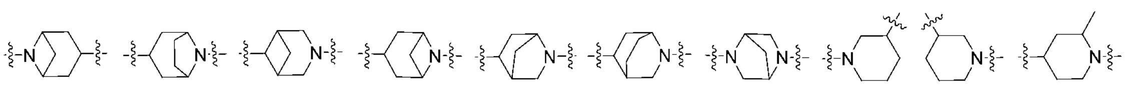

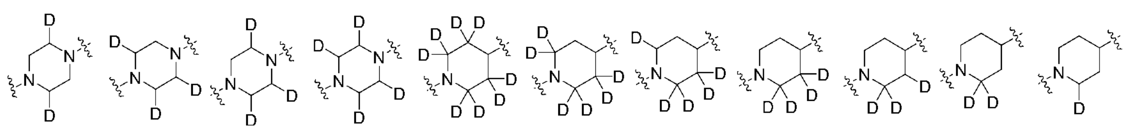

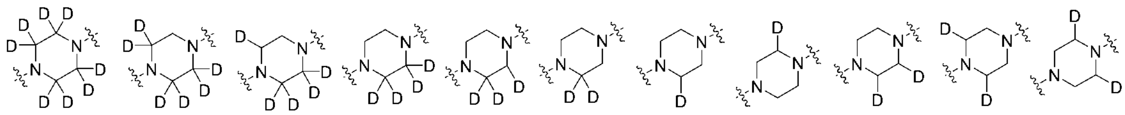

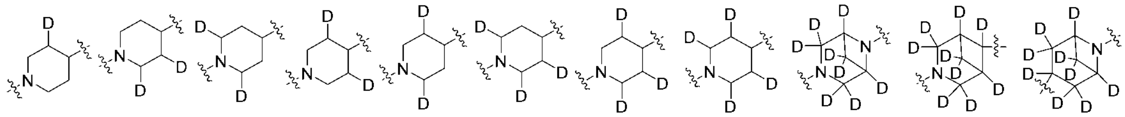

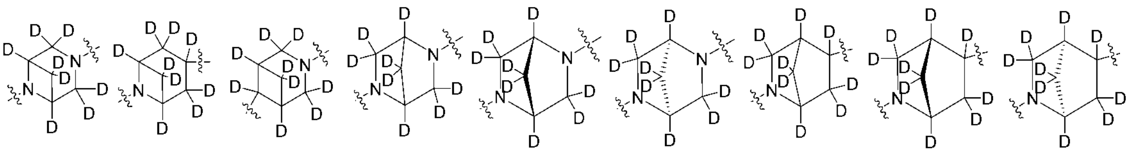

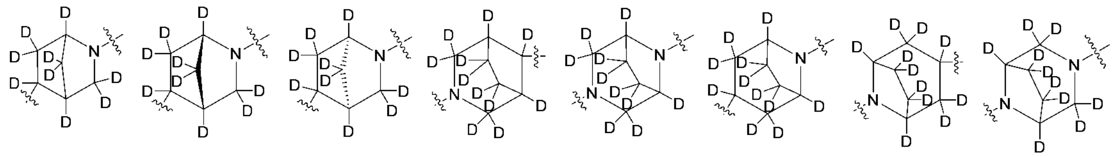

, or

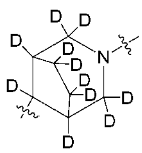

.

In some embodiments of the present disclosure, y1 represents an integer of 1 or 2, and each ring $A^1$ is independently optionally substituted with a1 $R^{y1}$ substituents, wherein each $R^{y1}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a1 represents an integer of 0-20. Alternatively, in some sub-embodiments of the present disclosure, each $R^{y1}$ independently represents deuterium, halogen (e.g., fluorine, chlorine, bromine, or iodine), hydroxy, mercapto, amino, cyano, $C_{1-4}$ alkyl (e.g., $C_{1-3}$ alkyl, such as methyl, ethyl, propyl, isopropyl, butyl, sec-butyl, or tert-butyl,), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), $C_{1-4}$ alkoxy (e.g., $C_{1-3}$ alkoxy, such as methoxy, ethoxy, propoxy, isopropoxy, n-butoxy, sec-butoxy, or tert-butoxy), or halogenated $C_{1-4}$ alkoxy (e.g., halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-). Alternatively, in some sub-embodiments of the present disclosure, a1 represents an integer of 0, 1, 2, 3, 4, 5, 6, 7, 8, 9, or 10.

In some embodiments of the present disclosure, y2 represents an integer of 0 or 1.

In some embodiments of the present disclosure, ring $A^2$ represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene) or 5- to 30-membered heteroarylene (e.g., 5- to 20-membered heteroarylene, or 5- to 15-membered heteroarylene).

In some embodiments of the present disclosure, ring $A^2$ represents:

azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, $C_{5-15}$ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene.

In some embodiments of the present disclosure, y2 represents an integer of 0 or 1, and ring A$^2$ represents:

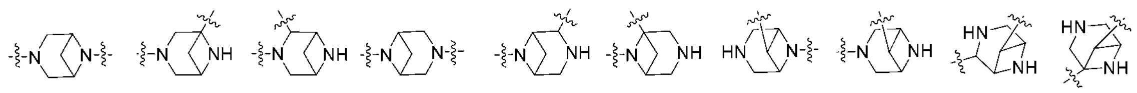
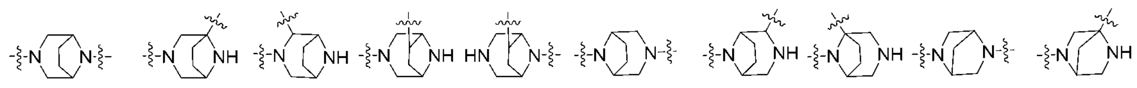
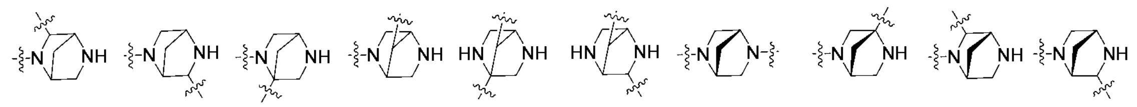
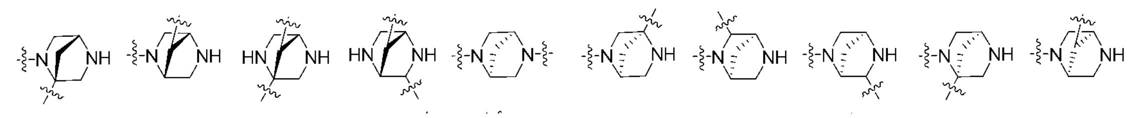
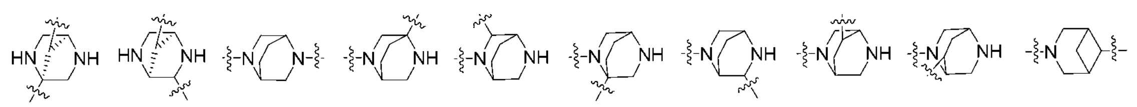
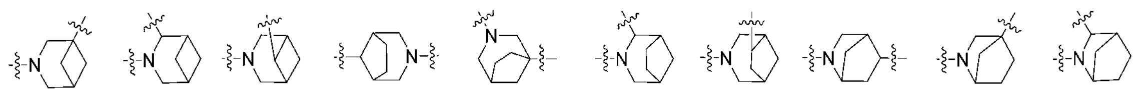
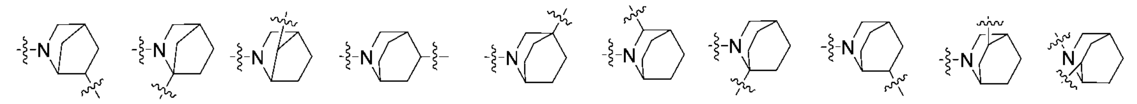
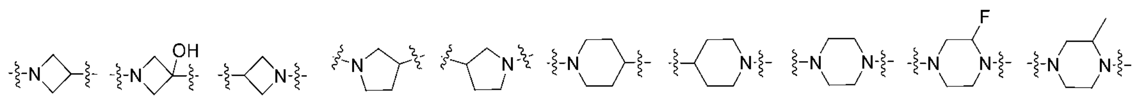
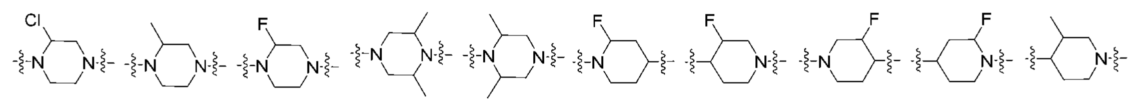
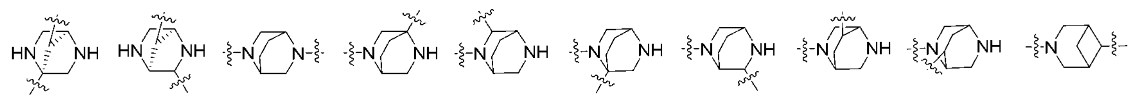
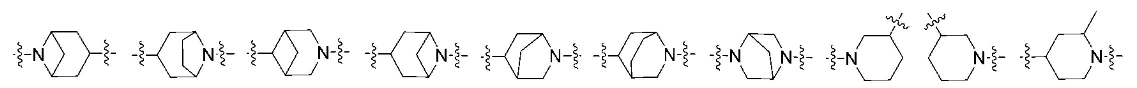

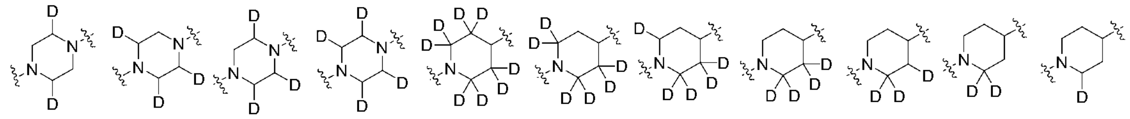

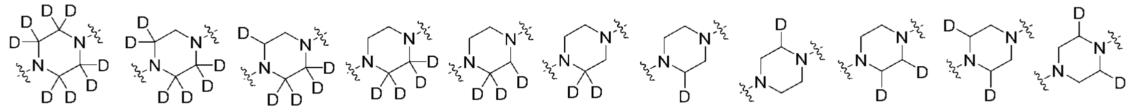

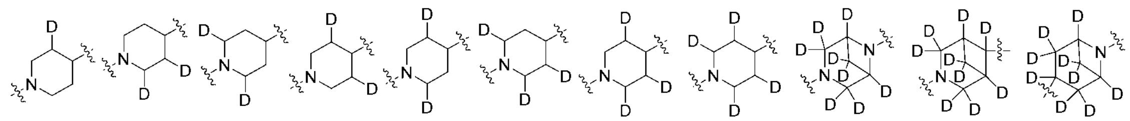

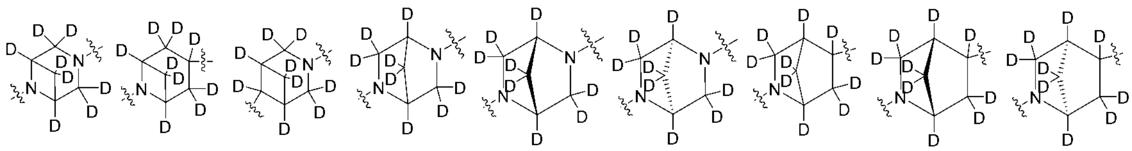

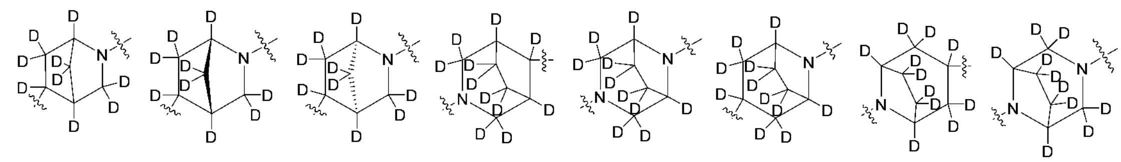

, or

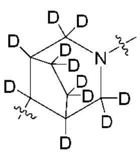

.

In some embodiments of the present disclosure, y2 represents an integer of 0 or 1, and ring $A^2$ is optionally substituted with a2 $R^{y2}$ substituents, wherein each $R^{y2}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a2 represents an integer of 0-20. Alternatively, in some sub-embodiments of the present disclosure, each $R^{y2}$ independently represents deuterium, halogen (e.g., fluorine, chlorine, bromine, or iodine), hydroxy, mercapto, amino, cyano, $C_{1-4}$ alkyl (e.g., $C_{1-3}$ alkyl, such as methyl, ethyl, propyl, isopropyl, butyl, sec-butyl, or tert-butyl,), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), $C_{1-4}$ alkoxy (e.g., $C_{1-3}$ alkoxy, such as methoxy, ethoxy, propoxy, isopropoxy, n-butoxy, sec-butoxy, or tert-butoxy), or halogenated $C_{1-4}$ alkoxy (e.g., halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-). Alternatively, in some sub-embodiments of the present disclosure, a2 represents an integer of 0, 1, 2, 3, 4, 5, 6, 7, 8, 9, or 10.

In some embodiments of the present disclosure, R represents:

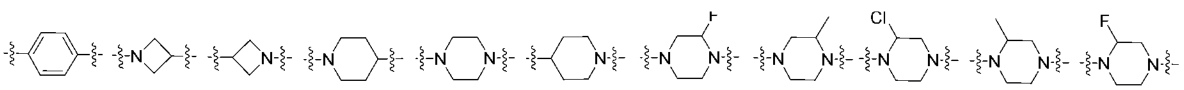

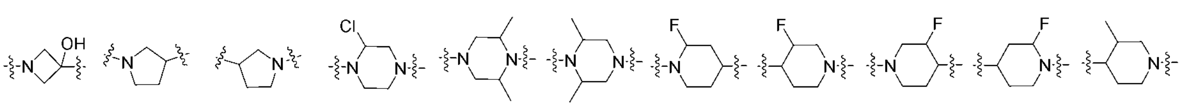

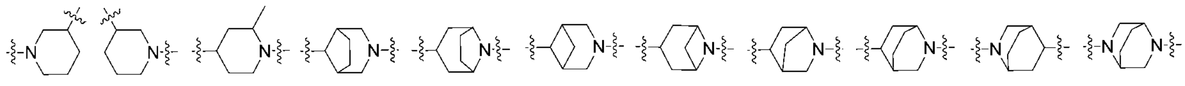
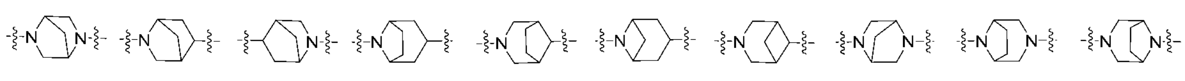
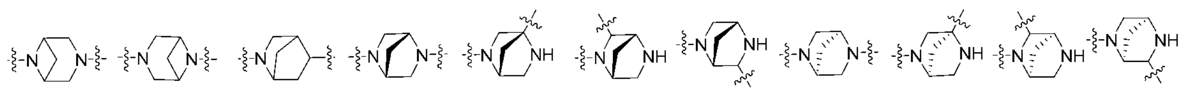
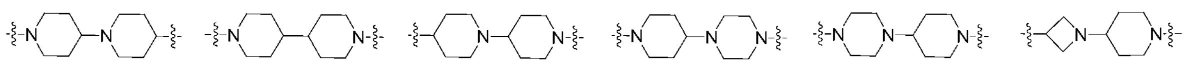
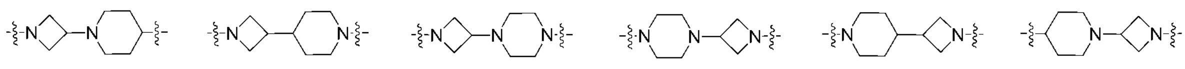
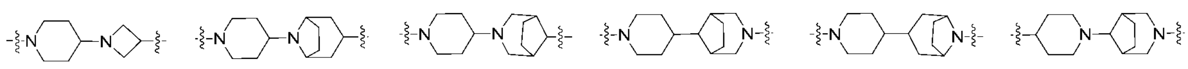
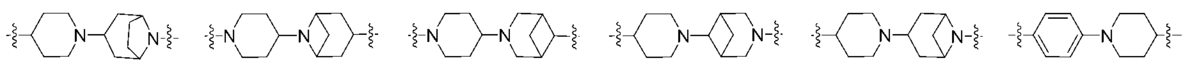
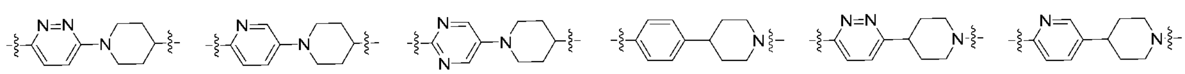
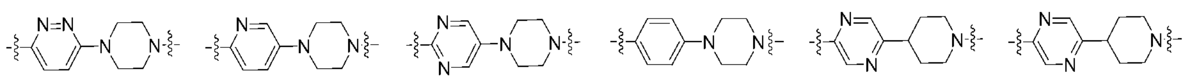
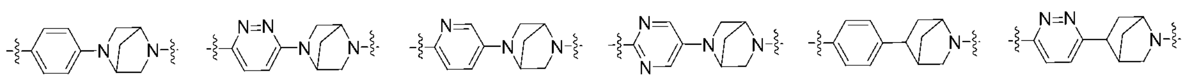
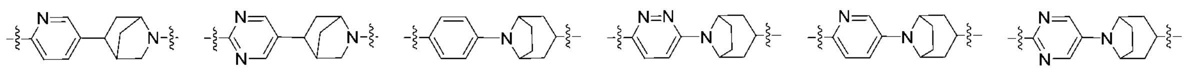
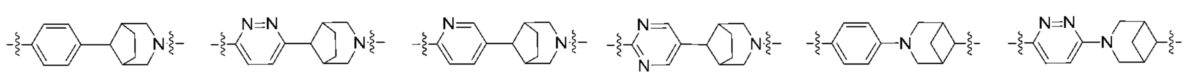
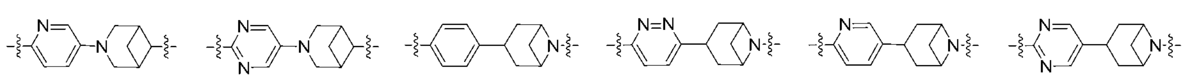
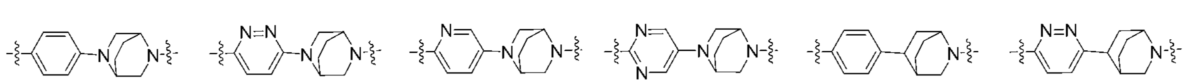
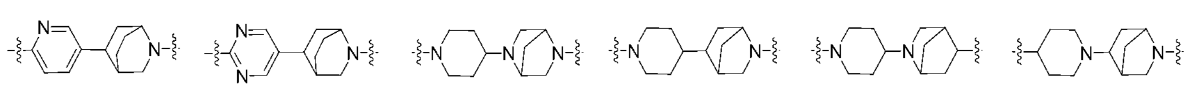
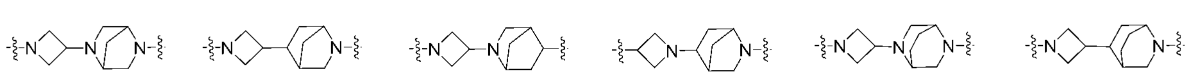
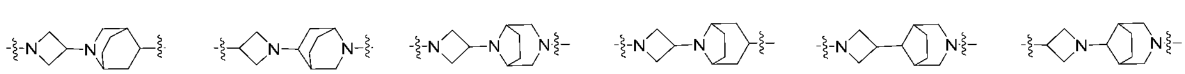

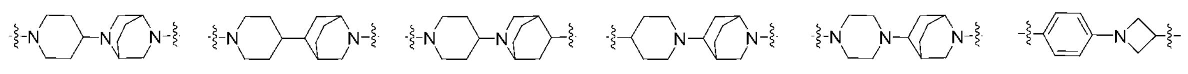

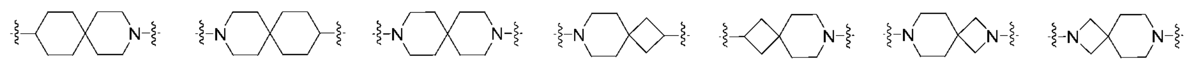

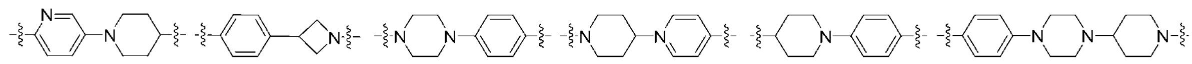

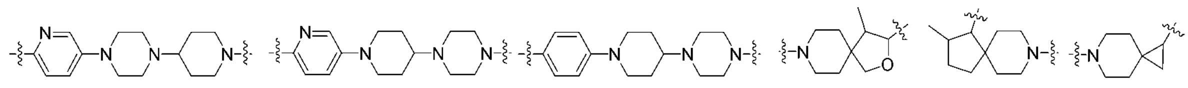

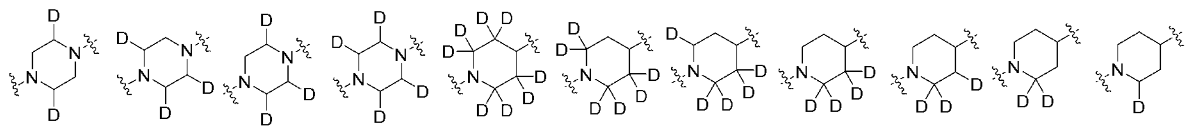

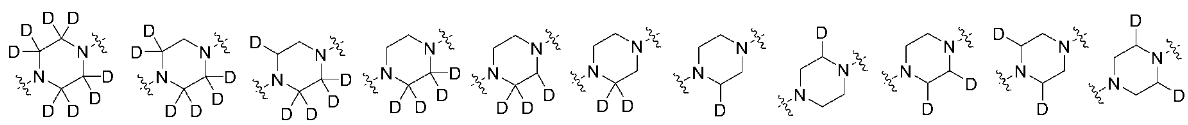

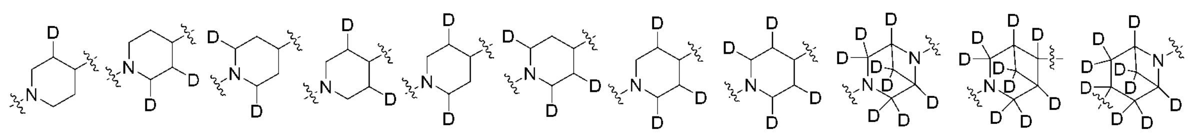

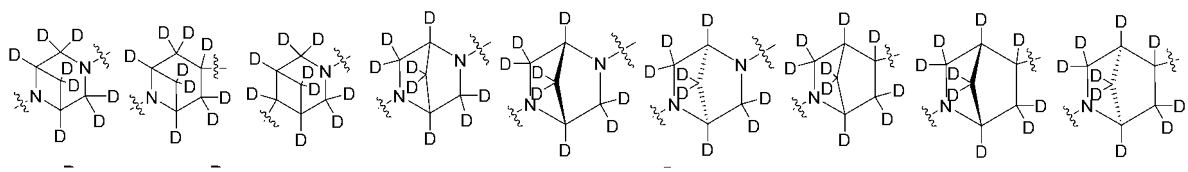

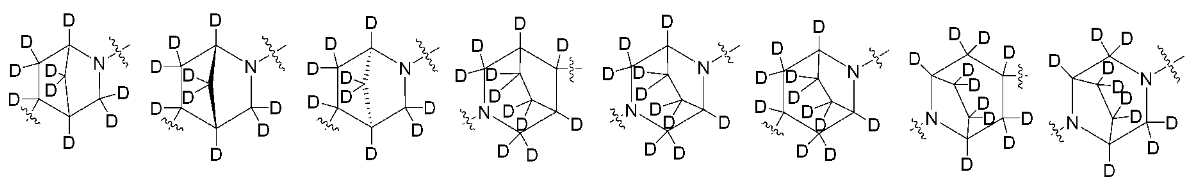

, or

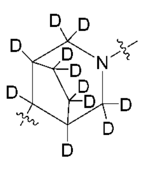

;

wherein the groups are optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl.

In some embodiments of the present disclosure, L represents:

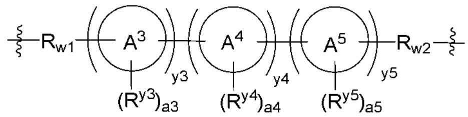

wherein $R_{w1}$ is linked to R, and $R_{w2}$ is linked to X;

$R_{w2}$ represents a bond, C(O), C(O)NH, NHC(O), or N($R_{a6}$), wherein $R_{a6}$ represents H or $C_{1-3}$ alkyl;

each ring $A^3$ is the same or different and independently represents heterocyclylene, cycloalkylene, arylene, or heteroarylene, y3 represents an integer of 0, 1, 2, or 3, and $(R^{y3})_{a3}$ indicates that each ring $A^3$ is independently optionally substituted with a3 $R^{y3}$ substituents, wherein each $R^{y3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a3 represents an integer of 0-20;

each ring $A^4$ is the same or different and independently represents heterocyclylene, cycloalkylene, arylene, or heteroarylene, y4 represents an integer of 0, 1, 2, or 3, and $(R^{y4})_{a4}$ indicates that each ring $A^4$ is independently optionally substituted with a4 $R^{y4}$ substituents, wherein each $R^{y4}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a4 represents an integer of 0-20;

each ring $A^5$ is the same or different and independently represents heterocyclylene, cycloalkylene, arylene, or heteroarylene, y5 represents an integer of 0, 1, 2, or 3, and $(R^{y5})_{a5}$ indicates that each ring $A^5$ is independently optionally substituted with a5 $R^{y5}$ substituents, wherein each $R^{y5}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a5 represents an integer of 0-20; and/or

$R_{w1}$ represents a bond or optionally substituted linear or branched $C_{1-20}$ alkylene, wherein any one or more (e.g., 1-15, 1-10, 1-6, 1-4, 2-6, or 1) methylene groups in the main carbon chain skeleton of the linear or branched $C_{1-20}$ alkylene are optionally replaced by one or more groups selected from: $R_a$, $R_b$, and any combination thereof; wherein each $R_a$ is independently selected from the group consisting of: O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, where each $R_{a7}$ independently represents H or $C_{1-6}$ alkyl, and when any two or more methylene in the main carbon chain skeleton of the linear or branched $C_{1-20}$ alkylene are replaced by two or more $R_a$ groups, the $R_a$ groups are not directly connected to each other; and wherein each $R_b$ is independently selected from the group consisting of: optionally substituted cycloalkylene, optionally substituted arylene, optionally substituted heterocyclylene, optionally substituted heteroarylene, alkynylene, alkenylene, and any combination thereof.

In some embodiments of the present disclosure, $R_{w2}$ represents a bond, C(O), C(O)NH, NHC(O), or N($R_{a6}$), wherein $R_{a6}$ represents H or $C_{1-3}$ alkyl.

In some embodiments of the present disclosure, $R_{w2}$ represents a bond.

In some embodiments of the present disclosure, y3 represents an integer of 0, 1, 2, or 3, and each ring $A^3$ independently represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene) or 5- to 30-membered heteroarylene (e.g., 5- to 20-membered heteroarylene, or 5- to 15-membered heteroarylene).

In some embodiments of the present disclosure, y3 represents an integer of 0, 1, 2, or 3, and each ring $A^3$ independently represents:

azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, $C_{5-15}$ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, phenylene, naphthylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene.

In some embodiments of the present disclosure, y3 represents an integer of 0, 1, 2, or 3, and each ring $A^3$ independently represents:

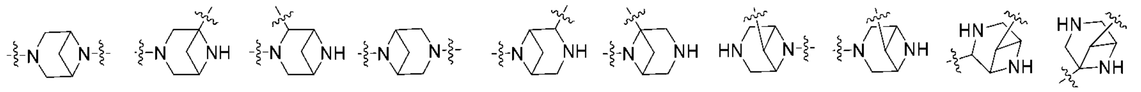
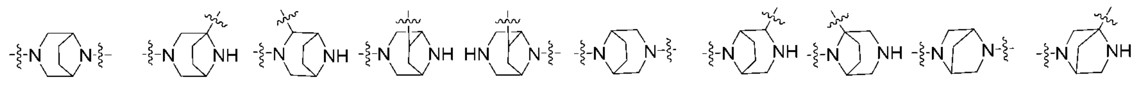
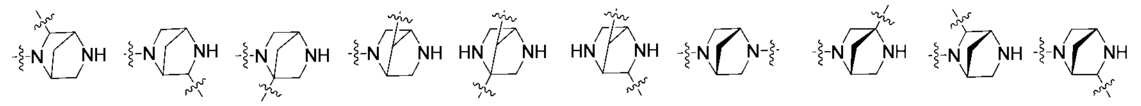
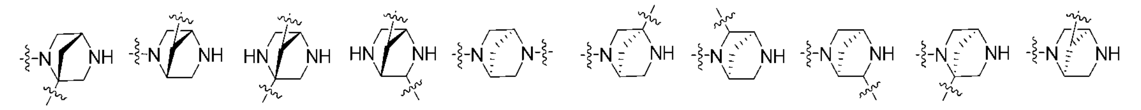
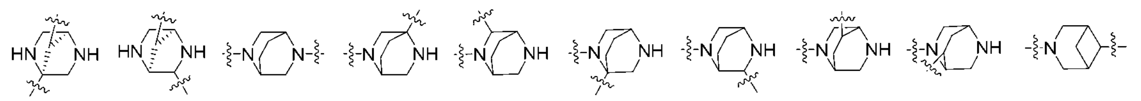
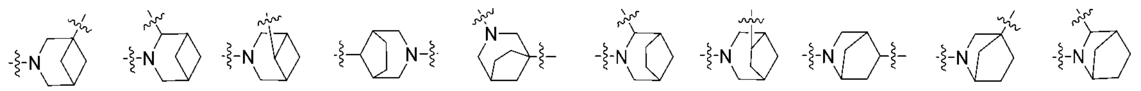
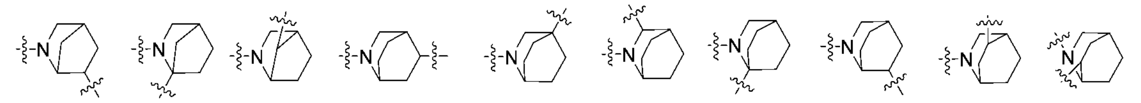
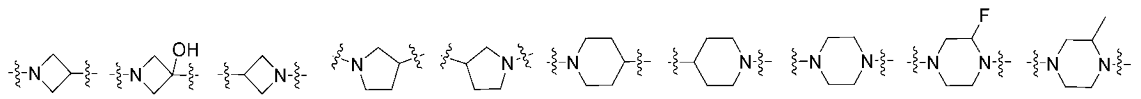
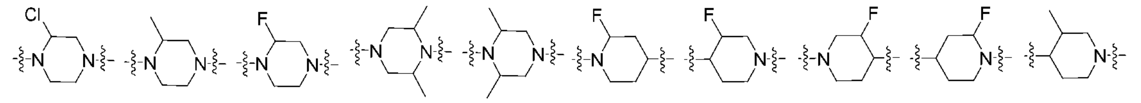
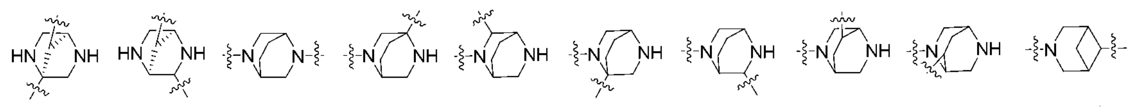
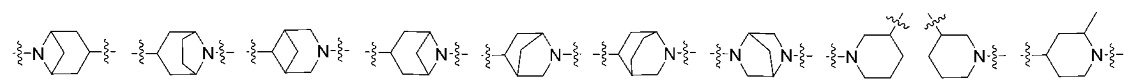
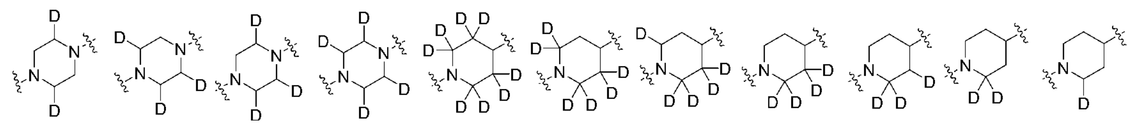

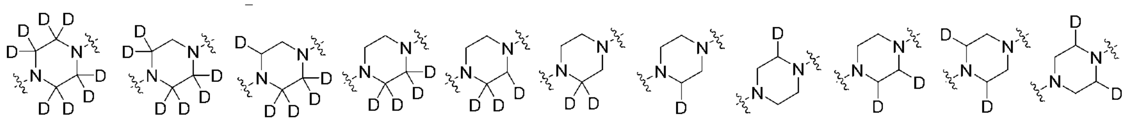

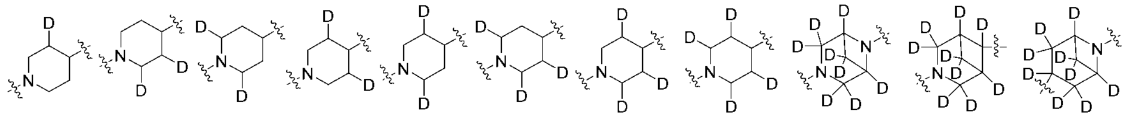

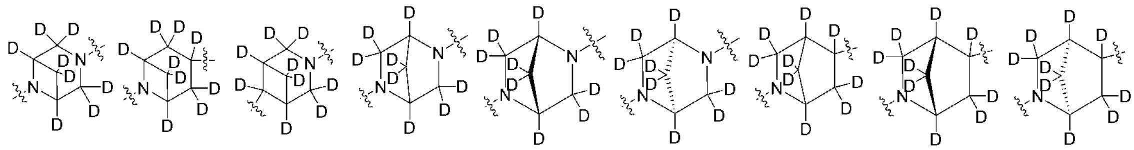

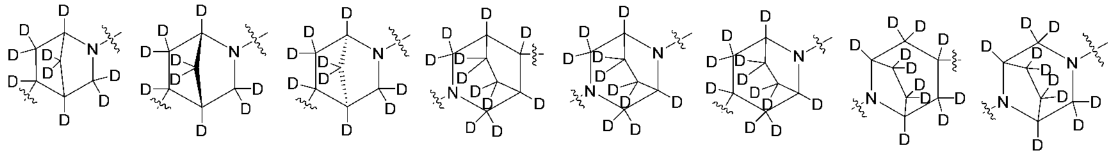

, or

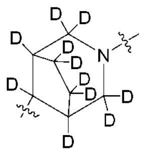

.

In some embodiments of the present disclosure, y3 represents an integer of 0, 1, 2, or 3, and each ring $A^3$ is independently optionally substituted with a3 $R^{y3}$ substituents, wherein each $R^{y3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a3 represents an integer of 0-20. Alternatively, in some sub-embodiments of the present disclosure, each $R^{y3}$ independently represents deuterium, halogen (e.g., fluorine, chlorine, bromine, or iodine), hydroxy, mercapto, amino, cyano, $C_{1-4}$ alkyl (e.g., $C_{1-3}$ alkyl, such as methyl, ethyl, propyl, isopropyl, butyl, sec-butyl, or tert-butyl,), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), $C_{1-4}$ alkoxy (e.g., $C_{1-3}$ alkoxy, such as methoxy, ethoxy, propoxy, isopropoxy, n-butoxy, sec-butoxy, or tert-butoxy), or halogenated $C_{1-4}$ alkoxy (e.g., halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-). Alternatively, in some sub-embodiments of the present disclosure, a3 represents an integer of 0, 1, 2, 3, 4, 5, 6, 7, 8, 9, 10, 11, 12, 13, 14, or 15.

In some embodiments of the present disclosure, y4 represents an integer of 0, 1, 2, or 3, and each ring $A^4$ independently represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene) or 5- to 30-membered heteroarylene (e.g., 5- to 20-membered heteroarylene, or 5- to 15-membered heteroarylene).

In some embodiments of the present disclosure, y4 represents an integer of 0, 1, 2, or 3, and each ring $A^4$ independently represents:

azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, $C_{5-15}$

spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, phenylene, naphthylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene.

In some embodiments of the present disclosure, y4 represents an integer of 0, 1, 2, or 3, and each ring $A^4$ independently represents:

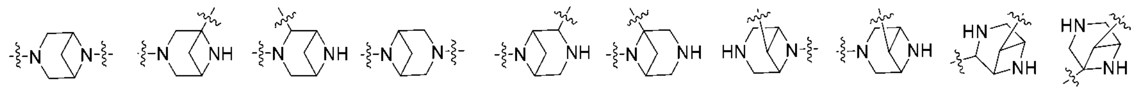

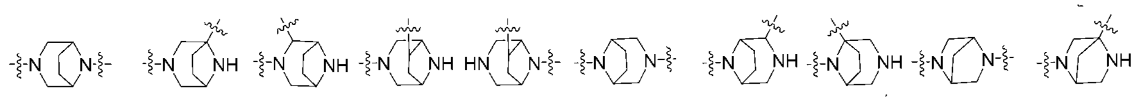

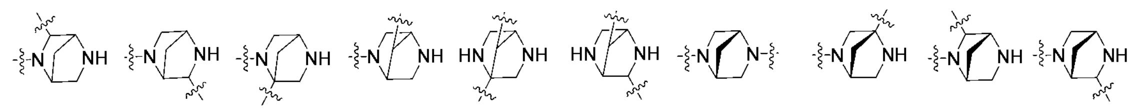

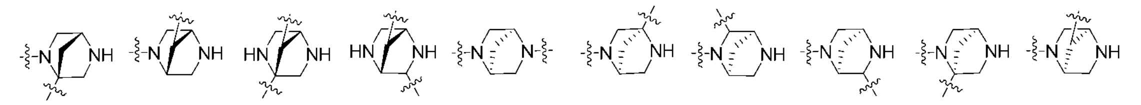

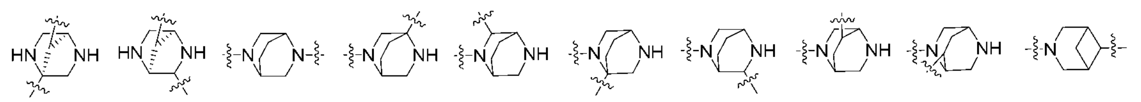

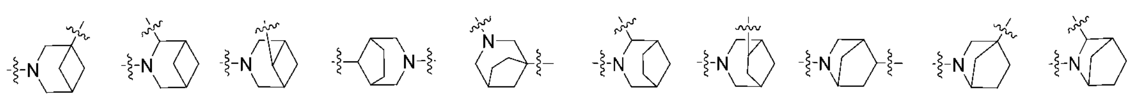

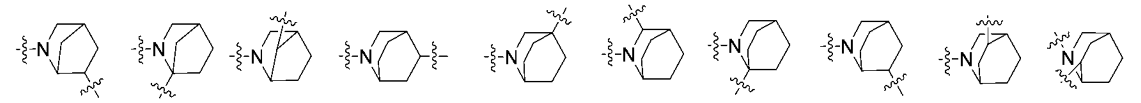

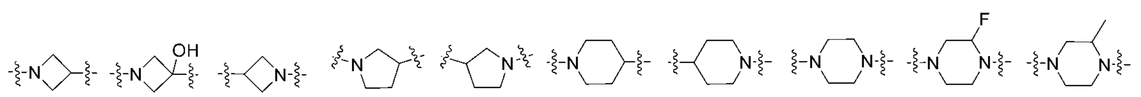

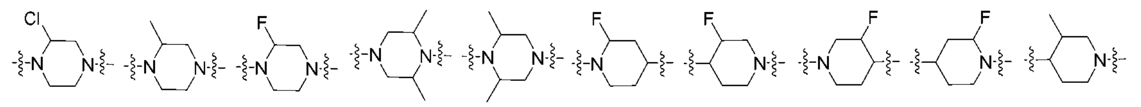

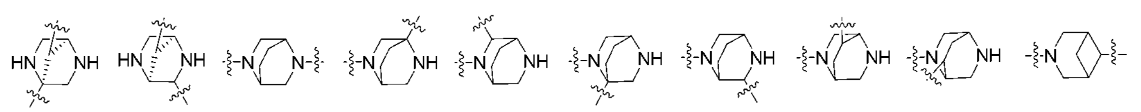

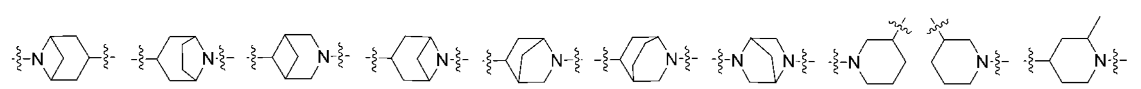

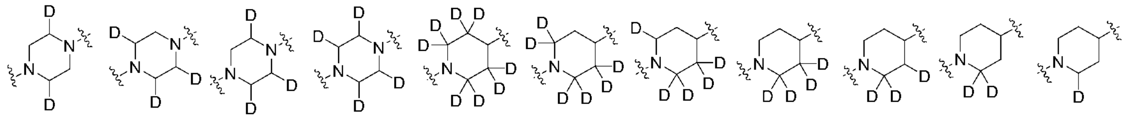

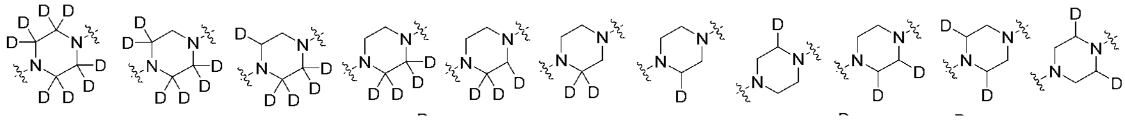

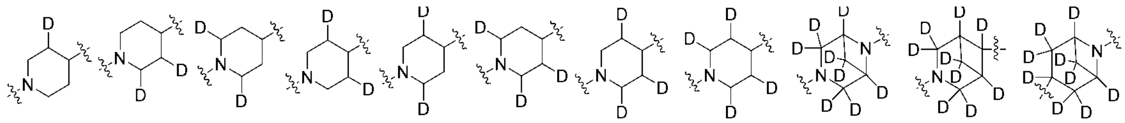

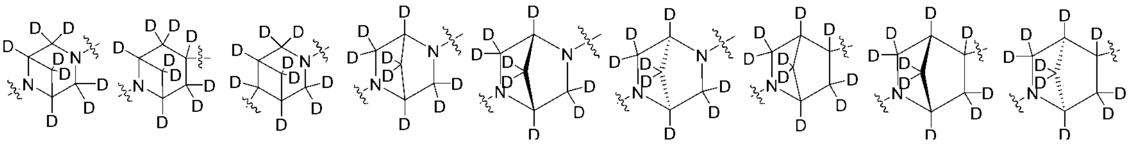

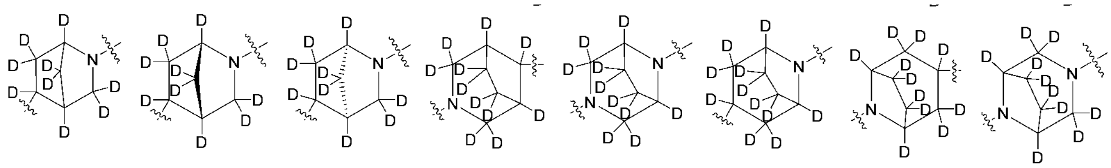

, or

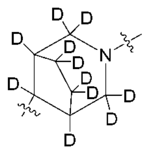

.

In some embodiments of the present disclosure, y4 represents an integer of 0, 1, 2, or 3, and each ring $A^4$ is independently optionally substituted with a4 $R^{y4}$ substituents, wherein each $R^{y4}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a4 represents an integer of 0-20. Alternatively, in some sub-embodiments of the present disclosure, each $R^{y4}$ independently represents deuterium, halogen (e.g., fluorine, chlorine, bromine, or iodine), hydroxy, mercapto, amino, cyano, $C_{1-4}$ alkyl (e.g., $C_{1-3}$ alkyl, such as methyl, ethyl, propyl, isopropyl, butyl, sec-butyl, or tert-butyl,), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), $C_{1-4}$ alkoxy (e.g., $C_{1-3}$ alkoxy, such as methoxy, ethoxy, propoxy, isopropoxy, n-butoxy, sec-butoxy, or tert-butoxy), or halogenated $C_{1-4}$ alkoxy (e.g., halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-). Alternatively, in some sub-embodiments of the present disclosure, a4 represents an integer of 0, 1, 2, 3, 4, 5, 6, 7, 8, 9, 10, 11, 12, 13, 14, or 15.

In some embodiments of the present disclosure, y5 represents an integer of 0, 1, 2, or 3, and each ring $A^5$ independently represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene) or 5- to 30-membered heteroarylene (e.g., 5- to 20-membered heteroarylene, or 5- to 15-membered heteroarylene).

In some embodiments of the present disclosure, y5 represents an integer of 0, 1, 2, or 3, and each ring $A^5$ independently represents:

azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, $C_{5-15}$ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene.

In some embodiments of the present disclosure, y5 represents an integer of 0, 1, 2, or 3, and each ring $A^5$ independently represents:

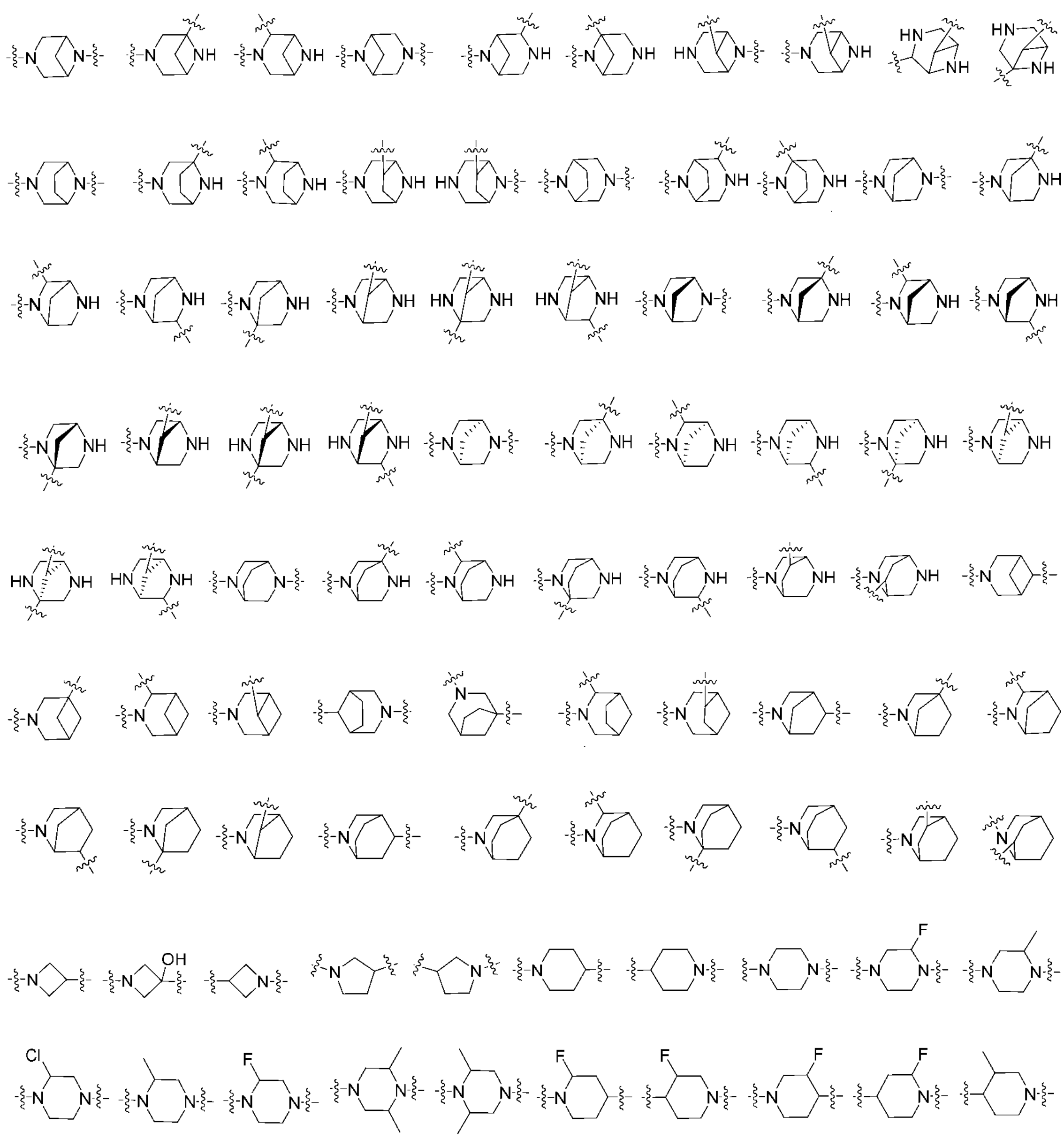

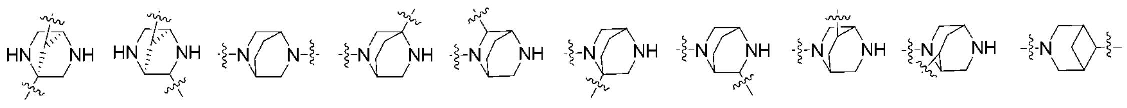

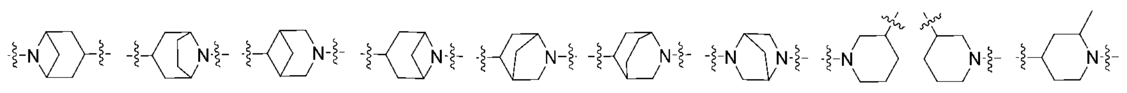

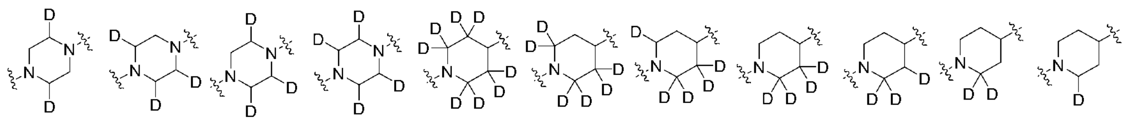

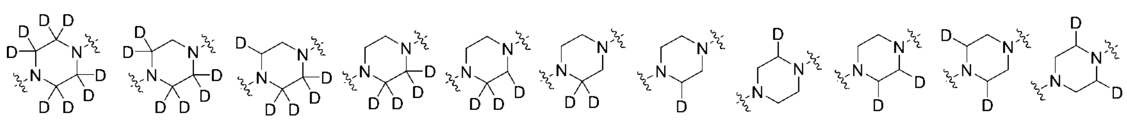

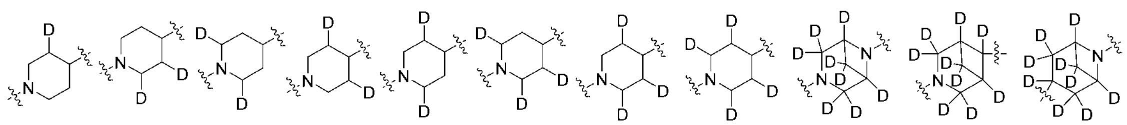

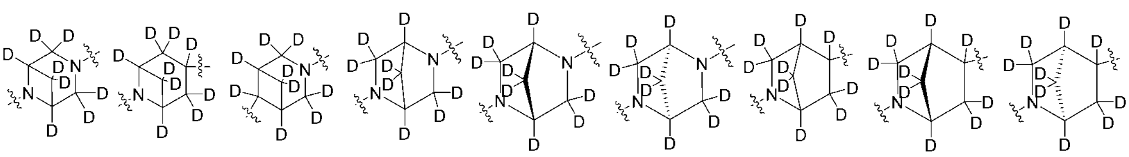

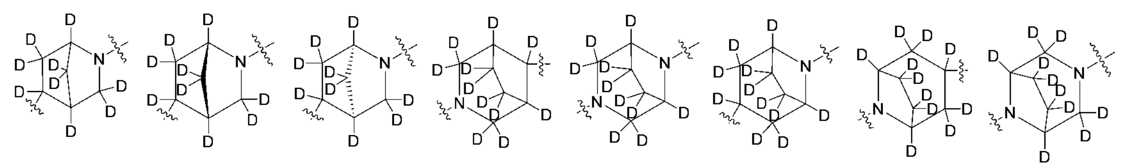

, or

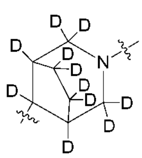

.

In some embodiments of the present disclosure, y5 represents an integer of 0, 1, 2, or 3, and each ring $A^5$ is independently optionally substituted with a5 $R^{y5}$ substituents, wherein each $R^{y5}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a5 represents an integer of 0-20. Alternatively, in some sub-embodiments of the present disclosure, each $R^{y5}$ independently represents deuterium, halogen (e.g., fluorine, chlorine, bromine, or iodine), hydroxy, mercapto, amino, cyano, $C_{1-4}$ alkyl (e.g., $C_{1-3}$ alkyl, such as methyl, ethyl, propyl, isopropyl, butyl, sec-butyl, or tert-butyl,), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), $C_{1-4}$ alkoxy (e.g., $C_{1-3}$ alkoxy, such as methoxy, ethoxy, propoxy, isopropoxy, n-butoxy, sec-butoxy, or tert-butoxy), or halogenated $C_{1-4}$ alkoxy (e.g., halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-). Alternatively, in some sub-embodiments of the present disclosure, a5 represents an integer of 0, 1, 2, 3, 4, 5, 6, 7, 8, 9, 10, 11, 12, 13, 14, or 15.

In some embodiments of the present disclosure, the moiety

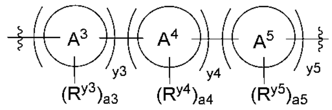

in each of the general formulae of L represents the following groups:

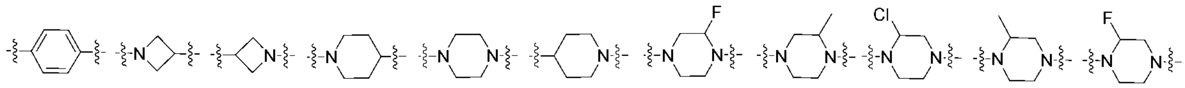

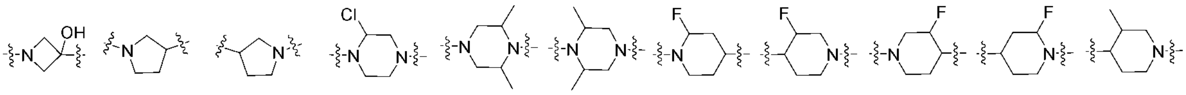

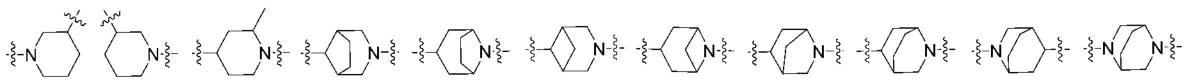

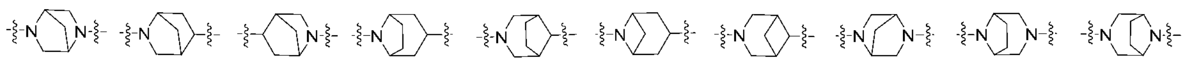

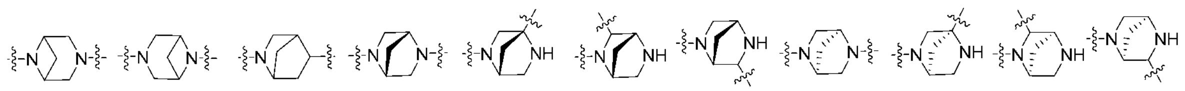

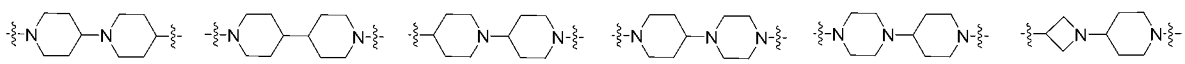

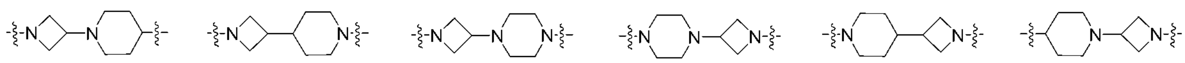

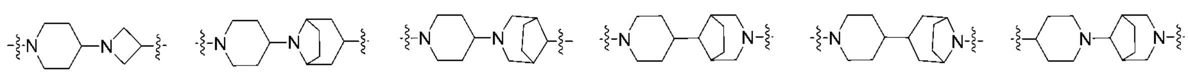

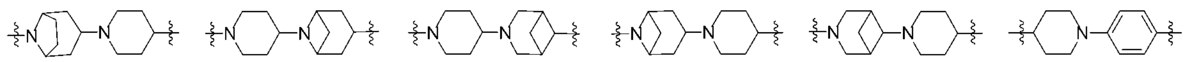

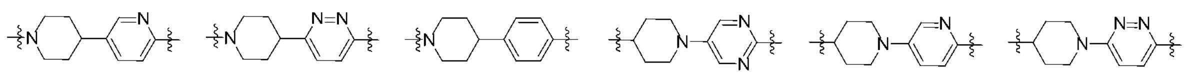

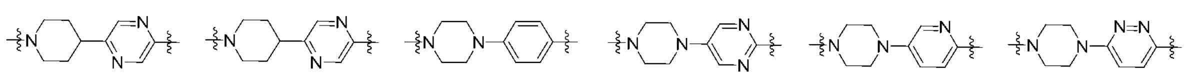

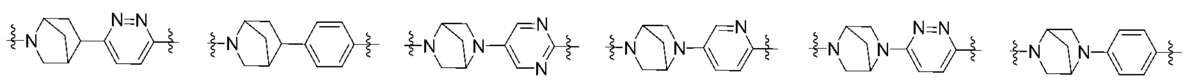

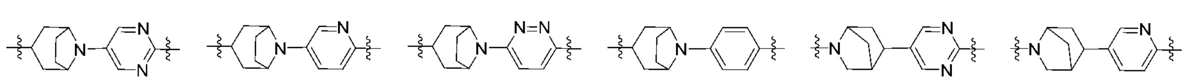

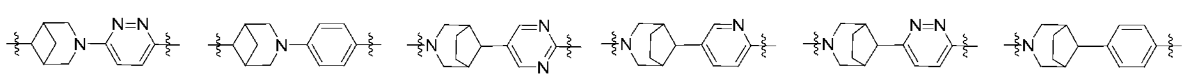

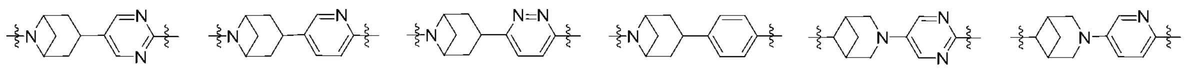

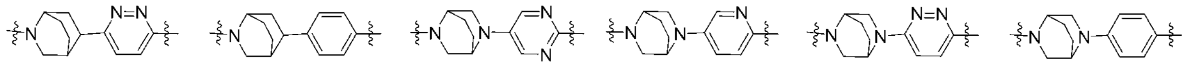

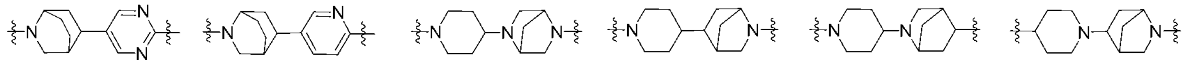

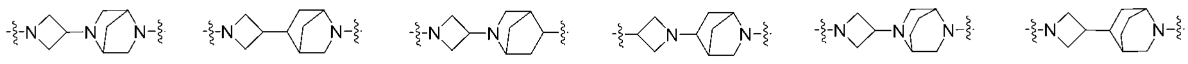

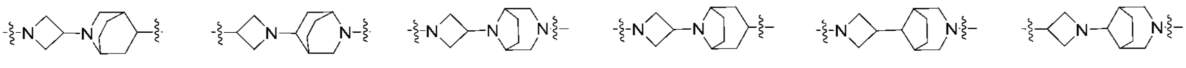

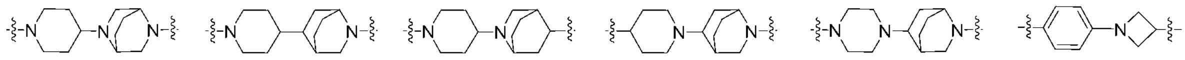

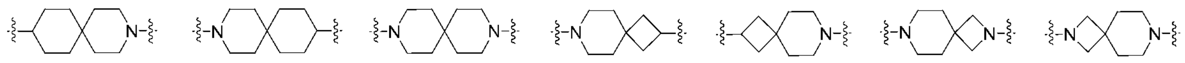

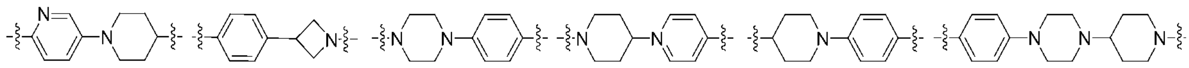

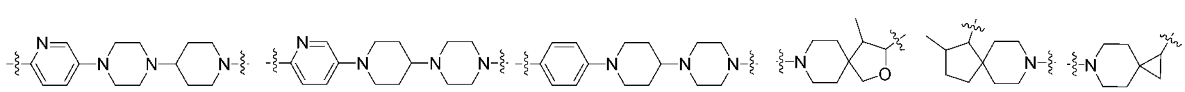

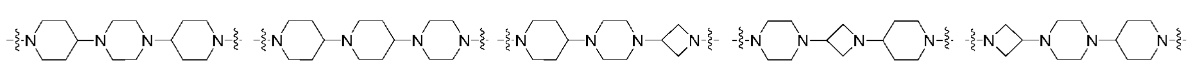

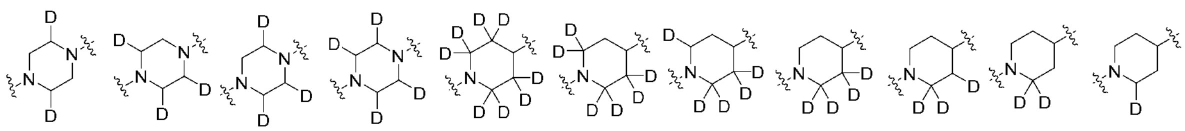

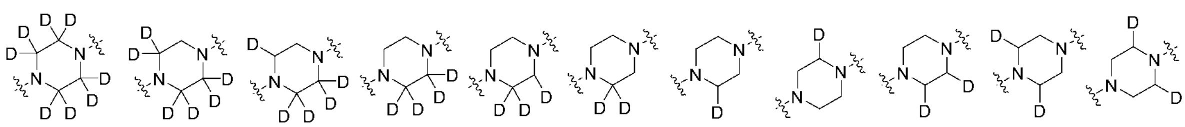

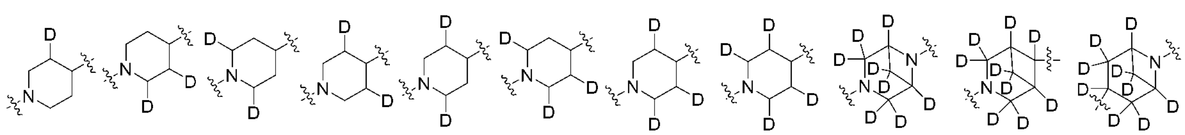

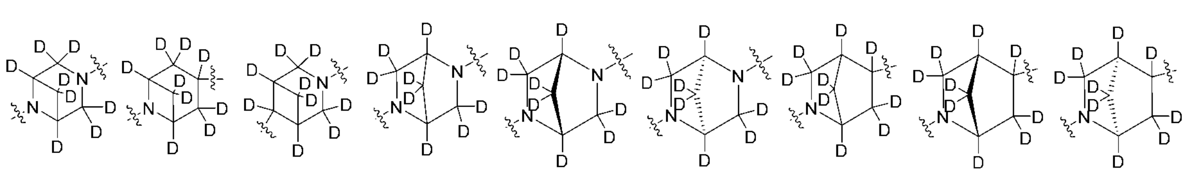

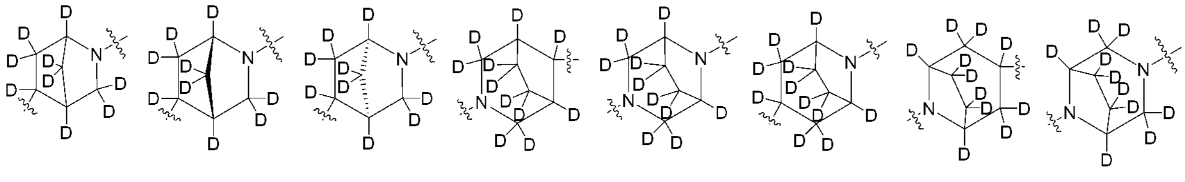

, or

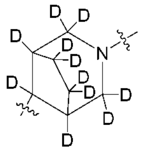

;

wherein the groups are optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl.

In some embodiments of the present disclosure, $R_{w1}$ represents a bond.

In some embodiments of the present disclosure, $R_{w1}$ represents a bond or optionally substituted linear or branched $C_{1-20}$ alkylene, wherein any one or more (e.g., 1-15, 1-10, 1-9, 1-8, 1-7, 1-6, 1-5, 1-4, 1-3, or 1-2, or 1) methylene groups in the main carbon chain skeleton of the linear or branched C1-20 alkylene are optionally replaced by one or more (e.g., 1-15, 1-10, 1-9, 1-8, 1-7, 1-6, 1-5, 1-4, 1-3, or 1-2, or 1) groups selected from: $R_a$, $R_b$, and any combination thereof; wherein each $R_a$ is independently selected from the group consisting of: O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, where each $R_{a7}$ independently represents H or $C_{1-6}$ alkyl (e.g., $C_{1-4}$ alkyl, or $C_{1-3}$ alkyl), and when any two or more methylene in the main carbon chain skeleton of the linear or branched $C_{1-20}$ alkylene are replaced by two or more $R_a$ groups, the $R_a$ groups are not directly connected to each other; and wherein each $R_b$ is independently selected from the group consisting of: optionally substituted cycloalkylene, optionally substituted arylene, optionally substituted heterocyclylene, optionally substituted heteroarylene, alkenylene, alkynylene, and any combination thereof. In some sub-embodiments, each $R_b$ is independently selected from the group consisting of: optionally substituted $C_{3-30}$ cycloalkylene, optionally substituted $C_{5-30}$ arylene, optionally substituted 4- to 30-membered heterocyclylene, optionally substituted 5- to 30-membered heteroarylene, $C_{2-6}$ alkenylene, or $C_{2-6}$ alkynylene (preferably, optionally substituted $C_{3-20}$ cycloalkylene, optionally substituted $C_{5-20}$ arylene, optionally substituted 4- to 20-membered heterocyclylene, optionally substituted 5- to 20-membered heteroarylene, $C_{2-4}$ alkenylene, or $C_{2-4}$ alkynylene; or more preferably, optionally substituted $C_{3-15}$ cycloalkylene, optionally substituted $C_{5-15}$ arylene, optionally substituted 4- to 15-membered heterocyclylene, optionally substituted 5- to 15-membered heteroarylene, $C_{2-4}$ alkenylene, or $C_{2-4}$ alkynylene) or any combination thereof. In some sub-embodiments, the cycloalkylene, arylene, heterocyclylene, and heteroarylene are each independently optionally substituted with a substituent selected from the group consisting of deuterium, hydroxy, amino, mercapto, nitro, halogen, cyano, oxo, $C_{1-6}$ alkyl (e.g., $C_{1-5}$ alkyl, $C_{1-4}$ alkyl, or $C_{1-3}$ alkyl, such as methyl, ethyl, propyl, isopropyl, butyl, sec-butyl, tert-butyl, pentyl, or hexyl), deuterated $C_{1-6}$ alkyl (e.g., perdeuterated $C_{1-6}$ alkyl, perdeuterated $C_{1-5}$ alkyl, or perdeuterated $C_{1-4}$ alkyl, such as $CD_3$, $CD_3CD_2$-, $CD_3CD_2CD_2$- etc.), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), $C_{1-6}$ alkoxy (e.g., $C_{1-4}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-), $C_{3-6}$ cycloalkyl, halogenated $C_{3-6}$ cycloalkyl, deuterated $C_{3-6}$ cycloalkyl, 4- to 10-membered heterocyclyl, and any combination thereof. In some sub-embodiments, one or more (e.g., 1-40, 1-35, 1-30, 1-25, 1-20, 1-15, 1-10, 1-9, 1-8, 1-7, 1-6, 1-5, 1-4, 1-3, or 1-2, or 1) hydrogens of said linear or branched $C_{1-20}$ alkylene are optionally replaced by a substituent selected from: deuterium, hydroxy, amino, mercapto, nitro, halogen, cyano, deuterated $C_{1-6}$ alkyl (e.g., perdeuterated $C_{1-6}$ alkyl, perdeuterated $C_{1-5}$ alkyl, or perdeuterated $C_{1-4}$ alkyl, such as $CD_3$, $CD_3CD_2$-, $CD_3CD_2CD_2$- etc.), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), $C_{1-6}$ alkoxy (e.g., $C_{1-4}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-), $C_{3-6}$ cycloalkyl, halogenated $C_{3-6}$ cycloalkyl, deuterated $C_{3-6}$ cycloalkyl, 4- to 10-membered heterocyclyl, and any combination thereof.

In some embodiments of the present disclosure, $R_{w1}$ represents:

$-C_{1-15}$ alkylene-;

$-(C(R_{a8})(R_{a9}))_{r1}-(R_a(C(R_{a10})(R_{a11}))_{r2})_{s1}-*$;

$-(C(R_{a8})(R_{a9}))_{r1}-(R_a(C(R_{a10})(R_{a11}))_{r2})_{s1}-(R_a(C(R_{a12})(R_{a13}))_{r3})_{s2}-*$;

$-(C(R_{a8})(R_{a9}))_{r1}-(R_a(C(R_{a10})(R_{a11}))_{r2})_{s1}-(R_a(C(R_{a12})(R_{a13}))_{r3})_{s2}-(R_a(C(R_{a14})(R_{a15}))_{r4})_{s3}-*$;

$-(C(R_{a8})(R_{a9}))_{r1}-((C(R_{a10})(R_{a11}))_{r2}R_a)_{s1}-*$;

$-(C(R_{a8})(R_{a9}))_{r1}-((C(R_{a10})(R_{a11}))_{r2}R_a)_{s1}-((C(R_{a12})(R_{a13}))_{r3}R_a)_{s2}-*$;

$-(C(R_{a8})(R_{a9}))_{r1}-((C(R_{a10})(R_{a11}))_{r2}R_a)_{s1}-((C(R_{a12})(R_{a13}))_{r3}R_a)_{s2}-((C(R_{a14})(R_{a15}))_{r4}R_a)_{s3}-*$;

$-(C(R_{a8})(R_{a9}))_{r1}-(R_b(C(R_{a10})(R_{a11}))_{r2})_{s1}-*$;

$-(CR_{a8})(R_{a9}))_{r1}-(R_b(C(R_{a10})(R_{a11}))_{r2})_{s1}-(R_b(C(R_{a12})(R_{a13}))_{r3})_{s2}-*$;

$-(C(R_{a8})(R_{a9}))_{r1}-(R_b(C(R_{a10})(R_{a11}))_{r2})_{s1}-(R_b(C(R_{a12})(R_{a13}))_{r3})_{s2}-(R_b(C(R_{a14})(R_{a15}))_{r4})_{s3}-*$;

$-(C(R_{a8})(R_{a9}))_{r1}-((C(R_{a10})(R_{a11}))_{r2}R_b)_{s1}-*$;

$-(C(R_{a8})(R_{a9}))_{r1}-((C(R_{a10})(R_{a11}))_{r2}R_b)_{s1}-((C(R_{a12})(R_{a13}))_{r3}R_b)_{s2}-*$;

$-(C(R_{a8})(R_{a9}))_{r1}-((C(R_{a10})(R_{a11}))_{r2}R_b)_{s1}-((C(R_{a12})(R_{a13}))_{r3}R_b)_{s2}-((C(R_{a14})(R_{a15}))_{r4}R_b)_{s3}-*$;

$-(C(R_{a8})(R_{a9}))_{r1}-(R_a-R_b-(C(R_{a10})(R_{a11}))_{r2})_{s1}-*$;

$-(C(R_{a8})(R_{a9}))_{r1}-(R_a(C(R_{a10})(R_{a11}))_{r2})_{s1}-(R_b(C(R_{a12})(R_{a13}))_{r3})_{s2}-*$;

$-(C(R_{a8})(R_{a9}))_{r1}-(R_b-R_a-(C(R_{a10})(R_{a11}))_{r2})_{s1}-*$;

$-(C(R_{a8})(R_{a9}))_{r1}-(R_b(C(R_{a10})(R_{a11}))_{r2})_{s1}-(R_a(C(R_{a12})(R_{a13}))_{r3})_{s2}-*$;

$-(C(R_{a8})(R_{a9}))_{r1}-((C(R_{a10})(R_{a11}))_{r2}-R_a-R_b)_{s1}-*$;

$-(C(R_{a8})(R_{a9}))_{r1}-((C(R_{a10}))(R_{a11}))_{r2}R_a)_{s1}-((C(R_{a12})(R_{a13}))_{r3}R_b)_{s2}-*$;

$-(C(R_{a8})(R_{a9}))_{r1}-((C(R_{a10})(R_{a11}))_{r2}-R_b-R_a)_{s1}-*$;

or

$-(C(R_{a8})(R_{a9}))_{r1}-((C(R_{a10})(R_{a11}))_{r2}R_b)_{s1}-((C(R_{a12})(R_{a13}))_{r3}R_a)_{s2}-*$;

wherein each $R_a$ is independently selected from the group consisting of O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, where each $R_{a7}$ independently represents H or $C_{1-6}$ alkyl; and wherein each $R_b$ is independently selected from the group consisting of: optionally substituted cycloalkylene (e.g., optionally substituted $C_{3-20}$ cycloalkylene), optionally substituted arylene (e.g., optionally substituted $C_{5-20}$ arylene), optionally substituted heterocyclylene (e.g., optionally substituted 4- to 20-membered heterocyclylene), optionally substituted heteroarylene (e.g., 5- to 20-membered heteroarylene), alkynylene (e.g., $C_{2-6}$ alkynylene), alkenylene (e.g., $C_{2-6}$ alkenylene), and any combination thereof, wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof;

$R_{a8}$, $R_{a9}$, $R_{a10}$, $R_{a11}$, $R_{a12}$, $R_{a13}$, $R_{a14}$, and $R_{a15}$ each independently represent H, deuterium, hydroxy, amino, mercapto, nitro, halogen, cyano, oxo, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and

any combination thereof; and
r1, r2, r3, r4, s1, s2, and s3 each independently represent an integer of 1, 2, 3, 4, 5, 6, 7, 8, 9, or 10;
symbol * indicates the point of attachment to R; and
any one or more (e.g., 1-30, 1-25, 1-20, 1-15, 1-10, 1-9, 1-8, 1-7, 1-6, 1-5, 1-4, 1-3, or 1-2, or 1) hydrogens in the main carbon chain skeleton of said linear or branched $C_{1-15}$ alkylene are optionally replaced by a substituent selected from: deuterium, hydroxy, amino, mercapto, nitro, halogen, cyano, oxo, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof. In the present disclosure, unless otherwise expressly specified, the total number of carbon atoms in each general formula of $R_{w1}$ (excluding the carbon atoms of $R_a$ and $R_b$ groups), namely the sum of r1+ r2*s1, the sum of r1+ r2*s1+ r3*s2, the sum of r1+ r2*s1+ r3*s2+ r4*s5, is in each case less than or equal to 20.

**In** some embodiments of the present disclosure, $R_{w1}$ represents $-C_{1-15}$ alkylene-, wherein any one or more (e.g., 1-30, 1-25, 1-20, 1-15, 1-10, 1-9, 1-8, 1-7, 1-6, 1-5, 1-4, 1-3, or 1-2, or 1) hydrogens in the main carbon chain skeleton of said linear or branched $C_{1-15}$ alkylene are optionally replaced by a substituent selected from: deuterium, hydroxy, amino, mercapto, nitro, halogen, cyano, oxo, halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), deuterated $C_{1-6}$ alkyl (e.g., perdeuterated $C_{1-6}$ alkyl, perdeuterated $C_{1-5}$ alkyl, or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-), $C_{3-6}$ cycloalkyl, halogenated $C_{3-6}$ cycloalkyl, deuterated $C_{3-6}$ cycloalkyl, 4- to 10-membered heterocyclyl, and any combination thereof.

In some embodiments of the present disclosure, $R_{w1}$ represents the following groups: $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, or $-(CH_2)_{15}-$; wherein any one or more hydrogens of the groups are optionally replaced by a substituent selected from: deuterium, hydroxy, amino, mercapto, nitro, halogen, cyano, oxo, halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), deuterated $C_{1-6}$ alkyl (e.g., perdeuterated $C_{1-6}$ alkyl, perdeuterated $C_{1-5}$ alkyl, or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-), $C_{3-6}$ cycloalkyl, halogenated $C_{3-6}$ cycloalkyl, deuterated $C_{3-6}$ cycloalkyl, 4- to 10-membered heterocyclyl, and any combination thereof.

In some embodiments of the present disclosure, $R_{w1}$ represents:

$-(C(R_{a8})(R_{a9}))_{r1}-(O(C(R_{a10})(R_{a11}))_{r2})_{s1}-*$;

$-(C(R_{a8})(R_{a9}))_{r1}-(O(C(R_{a10})(R_{a11}))_{r2})_{s1}-(O(C(R_{a12})(R_{a13}))_{r3})_{s2}-*$;

$-(C(R_{a8})(R_{a9}))_{r1}-(O(C(R_{a10})(R_{a11}))_{r2})_{s1}-(O(C(R_{a12})(R_{a13}))_{r3})_{s2}-(O(C(R_{a14})(R_{a15}))_{r4})_{s3}-*$;

$-(C(R_{a8})(R_{a9}))_{r1}-((C(R_{a10})(R_{a11}))_{r2}O)_{s1}-*$;

$-(C(R_{a8})(R_{a9}))_{r1}-((C(R_{a10})(R_{a11}))_{r2}O)_{s1}-((C(R_{a12})(R_{a13}))_{r3}O)_{s2}-*$;

$-(C(R_{a8})(R_{a9}))_{r1}-((C(R_{a10})(R_{a11}))_{r2}O)_{s1}-((C(R_{a12})(R_{a13}))_{r3}O)_{s2}-((C(R_{a14})(R_{a15}))_{r4}O)_{s3}-*$;

$-(C(R_{a8})(R_{a9}))_{r1}-(N(R_{a7})(C(R_{a10})(R_{a11}))_{r2})_{s1}-*$;

$-(C(R_{a8})(R_{a9}))_{r1}-(N(R_{a7})(C(R_{a10})(R_{a11}))_{r2})_{s1}-(N(R_{a7})(C(R_{a12})(R_{a13}))_{r3})_{s2}-*$;

$-(C(R_{a8})(R_{a9}))_{r1}-(N(R_{a7})(C(R_{a10})(R_{a11}))_{r2})_{s1}-(N(R_{a7})(C(R_{a12})(R_{a13}))_{r3})_{s2}-(N(R_{a7})(C(R_{a14})(R_{a15}))_{r4})_{s3}-*$;

$-(C(R_{a8})(R_{a9}))_{r1}-((C(R_{a10})(R_{a11}))_{r2}N(R_{a7}))_{s1}-*$;

$-(C(R_{a8})(R_{a9}))_{r1}-((C(R_{a10})(R_{a11}))_{r2}N(R_{a7}))_{s1}-((C(R_{a12})(R_{a13}))_{r3}N(R_{a7}))_{s2}-*$;

$-(C(R_{a8})(R_{a9}))_{r1}-((C(R_{a10})(R_{a11}))_{r2}N(R_{a7}))_{s1}-((C(R_{a12})(R_{a13}))_{r3}N(R_{a7}))_{s2}-((C(R_{a14})(R_{a15}))_{r4}N(R_{a7}))_{s3}-*$;

$-(C(R_{a8})(R_{a9}))_{r1}-(C(O)N(R_{a7})-(C(R_{a10})(R_{a11}))_{r2})_{s1}-*$;

$-(C(R_{a8})(R_{a9}))_{r1}-(C(O)N(R_{a7})-(C(R_{a10})(R_{a11}))_{r2})_{s1}-(C(O)N(R_{a7})-(C(R_{a12})(R_{a13}))_{r3})_{s2}-*$;

$-(C(R_{a8})(R_{a9}))_{r1}-(C(O)N(R_{a7})-(C(R_{a10})(R_{a11}))_{r2})_{s1}-(C(O)N(R_{a7})-(C(R_{a12})(R_{a13}))_{r3})_{s2}-(C(O)N(R_{a7})-(C(R_{a14})(R_{a15}))_{r4})_{s3}-*$;

$-(C(R_{a8})(R_{a9}))_{r1}-((C(R_{a10})(R_{a11}))_{r2}-C(O)N(R_{a7}))_{s1}-*$;

$-(C(R_{a8})(R_{a9}))_{r1}-((C(R_{a10})(R_{a11}))_{r2}-C(O)N(R_{a7}))_{s1}-((C(R_{a12})(R_{a13}))_{r3}-C(O)N(R_{a7}))_{s2}-*$;

$-(C(R_{a8})(R_{a9}))_{r1}-((C(R_{a10})(R_{a11}))_{r2}-C(O)N(R_{a7}))_{s1}-((C(R_{a12})(R_{a13}))_{r3}-C(O)N(R_{a7}))_{s2}-((C(R_{a14})(R_{a15}))_{r4}-C(O)N(R_{a7}))_{s3}-*$;

$-(C(R_{a8})(R_{a9}))_{r1}-(C(O)N(R_{a7})-(C(R_{a10})(R_{a11}))_{r2})_{s1}-(O-(C(R_{a12})(R_{a13}))_{r3})_{s2}-*$;

$-(C(R_{a8})(R_{a9}))_{r1}-C(O)N(R_{a7})-(C(R_{a10})(R_{a11}))_{r2}-(O(C(R_{a12})(R_{a13}))_{r3})_{s1}-*$;

$-(C(R_{a8})(R_{a9}))_{r1}-((C(R_{a10})(R_{a11}))_{r2}-C(O)N(R_{a7}))_{s1}-( (C(R_{a12})(R_{a13}))_{r3}O)_{s2}-*$;

$-(C(R_{a8})(R_{a9}))_{r1}-(C(R_{a10})(R_{a11}))_{r2}-C(O)N(R_{a7})-((C(R_{a12})(R_{a13}))_{r3}O)_{s1}-*$;

$-(CR_{a8})(R_{a9})_{r1}-(N(R_{a7})C(O)-(C(R_{a10})(R_{a11}))_{r2})_{s1}-*$;

$-(C(R_{a8})(R_{a9}))_{r1}-(N(R_{a7})C(O)-(C(R_{a10})(R_{a11}))_{r2})_{s1}-(O-(C(R_{a12})(R_{a13})))_{r3})_{s2}-*$;

$-(C(R_{a8})(R_{a9}))_{r1}-(N(R_{a7})C(O)-(C(R_{a10})(R_{a11}))_{r2})_{s1}-(O-(C(R_{a12})(R_{a13}))_{r3})_{s2}-(O-(C(R_{a14})(R_{a15}))r_4)s_3-*$;

$-(C(R_{a8})(R_{a9}))_{r1}-N(R_{a7})C(O)-(C(R_{a10})(R_{a11}))_{r2}-(O(C(R_{a12})(R_{a13}))_{r3})_{s1}-*$;

$-(C(R_{a8})(R_{a9}))_{r1}-N(R_{a7})C(O)N(R_{a7})-(C(R_{a10})(R_{a11}))_{r2}-*$;

$-(C(R_{a8})(R_{a9}))_{r1}-((C(R_{a10})(R_{a11}))_{r2}-N(R_{a7})C(O))_{s1}-*$;

$-(C(R_{a8})(R_{a9}))_{r1}-((C(R_{a10})(R_{a11}))_{r2}-N(R_{a7})C(O))_{s1}-((C(R_{a12})(R_{a13})))_{r3}-O)_{s2}-*$;

$-(C(R_{a8})(R_{a9}))_{r1}-((C(R_{a10})(R_{a11}))_{r2}-N(R_{a7})C(O))_{s1}-((C(R_{a12})(R_{a13}))_{r3}O)_{s2}-((C(R_{a14})(R_{a15}))_{r4}O)_{s3}-*$;

$-(C(R_{a8})(R_{a9}))_{r1}-(C(R_{a10})(R_{a11}))_{r2}-N(R_{a7})C(O)-((C(R_{a12})(R_{a13}))_{r3}O)_{s1}-*$;

$-(C(R_{a8})(R_{a9}))_{r1}-(\text{arylene}-(C(R_{a10})(R_{a11}))_{r2})_{s1}-*$;

$-(C(R_{a8})(R_{a9}))_{r1}-(\text{arylene}-(C(R_{a10})(R_{a11}))_{r2})_{s1}-\text{arylene}-(C(R_{a12})(R_{a13}))_{r3}-*$;

$-(C(R_{a8})(R_{a9}))_{r1}-((C(R_{a10})(R_{a11}))_{r2}-\text{arylene})_{s1}-*$;

$-(C(R_{a8})(R_{a9}))_{r1}-((C(R_{a10})(R_{a11}))_{r2}-\text{arylene})_{s1}-(C(R_{a12})(R_{a13}))_{r3}-\text{arylene}-*$;

$-(C(R_{a8})(R_{a9}))_{r1}-(\text{heterocyclylene}-(C(R_{a10})(R_{a11}))_{r2})_{s1}-*$;

$-(C(R_{a8})(R_{a9}))_{r1}-(\text{heterocyclylene}-(C(R_{a10})(R_{a11}))_{r2})_{s1}-(\text{heterocyclylene}-(C(R_{a12})(R_{a13}))_{r3})_{s2}-*$;

$-(C(R_{a8})(R_{a9}))_{r1}-((C(R_{a10})(R_{a11}))_{r2}-\text{heterocyclylene})_{s1}-*$;

$-(C(R_{a8})(R_{a9}))_{r1}-((C(R_{a10})(R_{a11}))_{r2}-\text{heterocyclylene})_{s1}-((C(R_{a12})(R_{a13}))_{r3}-\text{heterocyclylene})_{s2}-*$;

$-(C(R_{a8})(R_{a9}))_{r1}$-(heteroarylene-$(C(R_{a10})(R_{a11}))_{r2})_{s1}$-*;

$-(C(R_{a8})(R_{a9}))_{r1}$-(heteroarylene-$(C(R_{a10})(R_{a11}))_{r2})_{s1}$-(heteroarylene-$(C(R_{a12})(R_{a13}))_{r3})_{s2}$-*;

$-(C(R_{a8})(R_{a9}))_{r1}$-$((C(R_{a10})(R_{a11}))_{r2}$-heteroarylene$)_{s1}$-*;

$-(C(R_{a8})(R_{a9}))_{r1}$-$((C(R_{a10})(R_{a11}))_{r2}$-heteroarylene$)_{s1}$-$((C(R_{a12})(R_{a13}))_{r3}$-heteroarylene$)_{s2}$-*;

$-(C(R_{a8})(R_{a9}))_{r1}$-(cycloalkylene-$(C(R_{a10})(R_{a11}))_{r2})_{s1}$-*;

$-(CR_{a8})(R_{a9}))_{r1}$-(cycloalkylene-$(C(R_{a10})(R_{a11}))_{r2})_{s1}$-(cycloalkylene-$(C(R_{a12})(R_{a13}))_{r3})_{s2}$-*;

$-(C(R_{a8})(R_{a9}))_{r1}$-$((C(R_{a10})(R_{a11}))_{r2}$- cycloalkylene$)_{s1}$-*; or

$-(C(R_{a8})(R_{a9}))_{r1}$-$((C(R_{a10})(R_{a11}))_{r2}$- cycloalkylene$)_{s1}$-$((C(R_{a12})(R_{a13}))_{r3}$- cycloalkylene$)_{s2}$-*;

wherein each $R_{a7}$ independently represents H or $C_{1-6}$ alkyl;
the cycloalkylene (e.g., $C_{3-20}$ cycloalkylene), arylene (e.g., $C_{5-20}$ arylene), heterocyclylene (e.g., 4- to 20-membered heterocyclylene), and heteroarylene (e.g., 5- to 20-membered heteroarylene) are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof;
$R_{a8}$, $R_{a9}$, $R_{a10}$, $R_{a11}$, $R_{a12}$, $R_{a13}$, $R_{a14}$, and $R_{a15}$ each independently represent H, deuterium, hydroxy, amino, mercapto, nitro, halogen, cyano, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; and
r1, r2, r3, r4, s1, s2, and s3 each independently represent an integer of 1, 2, 3, 4, 5, 6, 7, 8, 9, or 10; and
symbol * indicates the point of attachment to R.

In some embodiments, examples of cycloalkylene in the aforementioned general structures represented by $R_{w1}$ include, but are not limited to $C_{3-20}$ cycloalkylene, $C_{3-15}$ cycloalkylene, and $C_{3-11}$ cycloalkylene, such as cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., $C_{5-15}$ spiro-cycloalkylene), adamantanylene, noradamantanylene, and norbornylene. The cycloalkylene is optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.

In some embodiments, examples of arylene in the aforementioned general structures represented by $R_{w1}$ include, but are not limited to $C_{5-20}$ arylene, $C_{6-20}$ arylene, $C_{5-15}$ arylene, and $C_{6-15}$ arylene, such as phenylene or naphthylene. The arylene is optionally substituted with a substituent selected from the group consisting of deuterium, hydroxy, amino, mercapto, nitro, halogen, cyano, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.

In some embodiments, examples of heterocyclylene in the aforementioned general structures represented by $R_{w1}$ include, but are not limited to 4- to 20-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, and 5- to 9-membered heterocyclylene, such as azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and 5- to 20-membered azaspirocycloalkylene. The heterocyclylene is optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.

In some embodiments, examples of heteroarylene in the aforementioned general structures represented by $R_{w1}$ include, but are not limited to 5- to 20-membered heteroarylene, 5- to 15-membered, 5-to 10-membered, 5- to 9-membered, 5- to 8-membered, 5- to 7-membered, and 5- to 6-membered heteroarylene, such as oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. The heteroarylene is optionally substituted with a substituent selected from the group consisting of deuterium, hydroxy, amino, mercapto, nitro, halogen, cyano, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.

In some embodiments, examples of $R_{w1}$ include, but are not limited to, the following groups: $-O-CH_2-$, $-O-(CH_2)_2-$, $-O-(CH_2)_3-$, $-O-(CH_2)_4-$, $-O-(CH_2)_5-$, $-O-(CH_2)_6-$, $-O-(CH_2)_7-$, $-O-(CH_2)_8-$, $-O-(CH_2)_9-$, $-O-(CH_2)_{10}-$, $-(CH_2)_1-O-$, $-(CH_2)_2-O-$, $-(CH_2)_3-O-$, $-(CH_2)_4-O-$, $-(CH_2)_5-O-$, $-(CH_2)_6-O-$, $-(CH_2)_7-O-$, $-(CH_2)_8-O-$, $-(CH_2)_9-O-$, $-(CH_2)_{10}-O-$, $-CH_2-O-CH_2-$, $-CH_2-O-(CH_2)_2-$, $-(CH_2)_1-O-(CH_2)_3-$, $-(CH_2)_1-O-(CH_2)_4-$, - $(CH_2)_1-O-(CH_2)_5-$, $-(CH_2)_1-O-(CH_2)_6-$, $-(CH_2)_1-O-(CH_2)_7-$, $-(CH_2)_1-O-(CH_2)_8-$, $-(CH_2)_1-O-(CH_2)_9-$, $-(CH_2)_1-O-(CH_2)_{10}-$, $-(CH_2)_2-O-(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, $-(CH_2)_2-O-(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$, $-(CH_2)_3-O-(CH_2)_1-$, $-(CH_2)_3-O-(CH_2)_2-$, $-(CH_2)_3-O-(CH_2)_3-$, $-(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_1-$, $-(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, $-(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, $-(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, $-(CH_2)_7-O-(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$, $-CH(CH_3)-O-(CH_2)_2-$, $-CH(CH_3)-O-(CH_2)_3-$, $-CH(CH_3)-O-(CH_2)_4-$, - $CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-CH(CH_3)-O-(CH_2)_9-$, $-CH(CH_3)-O-(CH_2)_{10}-$, $-(O(CH_2)_2)_2-$, $-(O(CH_2)_2)_3-$, $-(O(CH_2)_2)_4-$, $-(O(CH_2)_2)_5-$, $-(O(CH_2)_2)_6-$, - $(O(CH_2)_2)_7-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_7-$, $-CH_2-(O(CH_2)_2)_1-OCH_2-$, $-CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, $-(CH_2)_2-(O(CH_2)_2)_5-$, $-(CH_2)_2-(O(CH_2)_2)_6-$, $-(CH_2)_3-(O(CH_2)_2)_2-$, $-(CH_2)_3-(O(CH_2)_2)_3-$, $-(CH_2)_3-(O(CH_2)_2)_4-$, $-(CH_2)_3-(O(CH_2)_2)_5-$, $-(CH_2)_4-(O(CH_2)_2)_2-$, $-(CH_2)_4-(O(CH_2)_2)_3-$, $-(CH_2)_4-(O(CH_2)_2)_4-$, $-(CH_2)_4-(O(CH_2)_2)_5-$, $-CH_2-(O(CH_2)_3)_2-$, $-CH_2-(O(CH_2)_3)_3-$, $-CH_2-(O(CH_2)_3)_4-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_3-(O(CH_2)_3)_2-$, $-(CH_2)_3-(O(CH_2)_3)_3-$, $-(CH_2)_3-(O(CH_2)_3)_4-$, $-CH_2-O-(CH_2)_2-O-(CH_2)_3-$, $-CH_2-(O(CH_2)_2)_2-(O(CH_2)_3)_2-$, $(CH_2)_2-O-(CH_2)_2-O-(CH_2)_3-$, $-(CH_2)_2-(O(CH_2)_2)_2-(O(CH_2)_3)_2-$, $-(CH_2)_3-O-(CH_2)_2-O-(CH_2)_3-$, $-(CH_2)_3-(O(CH_2)_2)_2-(O(CH_2)_3)_2-$, $-CH_2-O-(CH_2)_3-O-(CH_2)_2-$, - $CH_2-(O(CH_2)_3)_2-(O(CH_2)_2)_2-$, $-(CH_2)_2-O-(CH_2)_3-O-(CH_2)_2-$, $-(CH_2)_2-(O(CH_2)_3)_2-(O(CH_2)_2)_2-$, $-(CH_2)_3-O-(CH_2)_3-O-(CH_2)_2-$, $-(CH_2)_3-(O(CH_2)_3)_2-(O(CH_2)_2)_2-$, $-CH_2-O-(CH_2)_2-O-CH_2-$, $-(CH_2)_2-O-(CH_2)_2-O-CH_2-$, - $(CH_2)_2-(O(CH_2)_2)_2-O-(CH_2)_3-$, $-(CH_2)_2-(O(CH_2)_2)_3-O-(CH_2)_3-$, $-(CH_2)_2-(O(CH_2)_2)_4-O-(CH_2)_3-$, $-(CH_2)_5-(O(CH_2)_2)_2-O-(CH_2)_5-$, $-(CH_2)_5-(O(CH_2)_2)_2-O-(CH_2)_6-$, $-(CH_2)_1-N(R_{a7})-(CH_2)_1-$, $-(CH_2)_1-N(R_{a7})-(CH_2)_2-$, - $(CH_2)_1-N(R_{a7})-(CH_2)_3-$, $-(CH_2)_1-N(R_{a7})-(CH_2)_4-$, $-(CH_2)_1-N(R_{a7})-(CH_2)_5-$, $-(CH_2)_1-N(R_{a7})-(CH_2)_6-$, $-(CH_2)_1-N(R_{a7})-(CH_2)_7-$, $-(CH_2)_1-N(R_{a7})-(CH_2)_8-$, $-(CH_2)_1-N(R_{a7})-(CH_2)_9-$, $-(CH_2)_1-N(R_{a7})-(CH_2)_{10}-$, $-N(R_{a7})-(CH_2)_1-$, $-N(R_{a7})-(CH_2)_2-$, $-N(R_{a7})-(CH_2)_3-$, $-N(R_{a7})-(CH_2)_4-$, $-N(R_{a7})-(CH_2)_5-$, $-N(R_{a7})-(CH_2)_6-$, $-N(R_{a7})-(CH_2)_7-$, - $N(R_{a7})-(CH_2)_8-$, $-N(R_{a7})-(CH_2)_9-$, $-N(R_{a7})-(CH_2)_{10}-$, $-(CH_2)_2-N(R_{a7})-(CH_2)_1-$, $-(CH_2)_2-N(R_{a7})-(CH_2)_2-$, - $(CH_2)_2-N(Ra_7)-(CH_2)_3-$, $-(CH_2)_2-N(R_{47})-(CH_2)_4-$, $-(CH_2)_2-N(Ra_7)-(CH_2)_5-$, $-(CH_2)_2-N(R_{a7})-(CH_2)_6-$, $-(CH_2)_2-N(R_{a7})-(CH_2)_7-$, $-(CH_2)_2-N(R_{a7})-(CH_2)_8-$, $-(CH_2)_2-N(R_{a7})-(CH_2)_9-$, $-(CH_2)_2-N(R_{a7})-(CH_2)_{10}-$, $-(CH_2)_3-N(R_{a7})-(CH_2)_1-$, $-(CH_2)_3-N(R_{a7})-(CH_2)_2-$, $-(CH_2)_3-N(R_{a7})-(CH_2)_3-$, $-(CH_2)_4-N(R_{a7})-(CH_2)_1-$, $-(CH_2)_4-N(R_{a7})-(CH_2)_2-$, $-(CH_2)_4-N(R_{a7})-(CH_2)_3-$, $-(CH_2)_4-N(R_{a7})-(CH_2)_4-$, $-(CH_2)_5-N(R_{a7})-(CH_2)_1-$, $-(CH_2)_5-N(R_{a7})-(CH_2)_2-$, $-(CH_2)_5-N(R_{a7})-(CH_2)_3-$, $-(CH_2)_5-N(R_{a7})-(CH_2)_4-$, $-(CH_2)_5-N(R_{a7})-(CH_2)_5-$, $-(CH_2)_6-N(R_{a7})-(CH_2)_1-$, $-(CH_2)_6-N(R_{a7})-(CH_2)_2-$, $-(CH_2)_6-N(R_{a7})-(CH_2)_3-$, $-(CH_2)_7-N(R_{a7})-(CH_2)_1-$, $-(CH_2)_7-N(R_{a7})-(CH_2)_2-$, $-(CH_2)_7-N(R_{a7})-(CH_2)_3-$, $-(CH_2)_8-N(R_{a7})-(CH_2)_1-$, $-(CH_2)_8-N(R_{a7})-(CH_2)_2-$, $-(CH_2)_8-N(R_{a7})-(CH_2)_3-$, $-CH(CH_3)-N(R_{a7})-(CH_2)_1-$, - $CH(CH_3)-N(R_{a7})-(CH_2)_2-$, $-CH(CH_3)-N(R_{a7})-(CH_2)_3-$, $-CH(CH_3)-N(R_{a7})-(CH_2)_4-$, $-CH(CH_3)-N(R_{a7})-(CH_2)_5-$, $-CH(CH_3)-N(R_{a7})-(CH_2)_6-$, $-CH(CH_3)-N(R_{a7})-(CH_2)_7-$, $-CH(CH_3)-N(R_{a7})-(CH_2)_8-$, $-CH(CH_3)-N(R_{a7})-(CH_2)_9-$, $-CH(CH_3)-N(R_{a7})-(CH_2)_{10}-$, $-(CH_2)_1-N(R_{a7})-$, $-(CH_2)_2-N(R_{a7})-$, $-(CH_2)_3-N(R_{a7})-$, $-(CH_2)_4-N(R_{a7})-$, - $(CH_2)_5-N(R_{a7})-$, $-(CH_2)_6-N(R_{a7})-$, $-(CH_2)_7-N(R_{a7})-$, $-(CH_2)_8-N(R_{a7})-$, $-(CH_2)_9-N(R_{a7})-$, $-(CH_2)_{10}-N(R_{a7})-$, - $C(O)NHCH_2-$, $-C(O)NH(CH_2)_2-$, $-C(O)NH(CH_2)_3-$, $-C(O)NH(CH_2)_4-$, $-C(O)NH(CH_2)_5-$, $-CH_2C(O)NHCH_2-$, $-(CH_2)_2C(O)NH(CH_2)_2-$, $-(CH_2)_2C(O)NH(CH_2)_3-$, $-(CH_2)_2C(O)NH(CH_2)_4-$, $-(CH_2)_2C(O)NH(CH_2)_5-$, - $(CH_2)_3C(O)NH(CH_2)_3-$, $-(CH_2)_3C(O)NH(CH_2)_4-$, $-(CH_2)_4C(O)NH(CH_2)_4-$,

-(CH$_2$)$_5$C(O)NH(CH$_2$)$_5$-, - (CH$_2$)$_6$C(O)NH(CH$_2$)$_7$-, -(CH$_2$)$_6$C(O)NH(CH$_2$)$_6$-, -(CH$_2$)$_7$C(O)NH(CH$_2$)$_7$-, -(CH$_2$)$_2$C(O)NH(CH$_2$)$_2$-O-(CH$_2$)$_2$-, -NHC(O)CH$_2$-, -NHC(O)(CH$_2$)$_2$-, -NHC(O)(CH$_2$)$_3$-, -NHC(O)(CH$_2$)$_4$-, -NHC(O)(CH$_2$)$_5$-, -CH$_2$NHC(O)CH$_2$-, -(CH$_2$)$_2$NHC(O)(CH$_2$)$_2$-, -(CH$_2$)$_2$NHC(O)(CH$_2$)$_3$-, -(CH$_2$)$_2$NHC(O)(CH$_2$)$_4$-, - (CH$_2$)$_2$NHC(O)(CH$_2$)$_5$-, -(CH$_2$)$_3$NHC(O)(CH$_2$)$_3$-, -(CH$_2$)$_3$NHC(O)(CH$_2$)$_4$-, -(CH$_2$)$_4$NHC(O)(CH$_2$)$_4$-, - (CH$_2$)$_5$NHC(O)(CH$_2$)$_5$-, -(CH$_2$)$_6$NHC(O)(CH$_2$)$_7$-, -(CH$_2$)$_6$NHC(O)(CH$_2$)$_6$-, -(CH$_2$)$_7$NHC(O)(CH$_2$)$_7$-, - (CH$_2$)$_4$NHC(O)(CH$_2$)$_8$-, -(CH$_2$)$_2$NHC(O)(CH$_2$)$_2$-O-(CH$_2$)$_2$-, -(CH$_2$)$_4$NHC(O)CH$_2$-, -phenylene-CH$_2$-, - phenylene-(CH$_2$)$_2$-, -phenylene-(CH$_2$)$_3$-, -phenylene-(CH$_2$)$_4$-, -phenylene-(CH$_2$)$_5$-, -phenylene-(CH$_2$)$_6$-, - phenylene-(CH$_2$)$_7$-, -phenylene-(CH$_2$)$_8$-, -CH$_2$-phenylene-CH$_2$-, -CH$_2$-phenylene-(CH$_2$)$_2$-, -CH$_2$-phenylene-(CH$_2$)$_3$-, -CH$_2$-phenylene-(CH$_2$)$_4$-, -CH$_2$-phenylene-(CH$_2$)$_5$-, -CH$_2$-phenylene-(CH$_2$)$_6$-, -CH$_2$-phenylene-(CH$_2$)$_7$-, -CH$_2$-phenylene-(CH$_2$)$_8$-, -(CH$_2$)$_2$-phenylene-(CH$_2$)$_1$-, -(CH$_2$)$_2$-phenylene-(CH$_2$)$_2$-, -(CH$_2$)$_2$-phenylene-(CH$_2$)$_3$-, -(CH$_2$)$_2$-phenylene-(CH$_2$)$_4$-, -(CH$_2$)$_2$-phenylene-(CH$_2$)$_5$-, -(CH$_2$)$_2$-phenylene-(CH$_2$)$_6$-, - (CH$_2$)$_2$-phenylene-(CH$_2$)$_7$-, -(CH$_2$)$_2$-phenylene-(CH$_2$)$_8$-, -(CH$_2$)$_3$-phenylene-CH$_2$-, -(CH$_2$)$_3$-phenylene-(CH$_2$)$_2$-, -(CH$_2$)$_3$-phenylene-(CH$_2$)$_3$-, -(CH$_2$)$_3$-phenylene-(CH$_2$)$_4$-, -(CH$_2$)$_3$-phenylene-(CH$_2$)$_5$-, -(CH$_2$)$_3$-phenylene-(CH$_2$)$_6$-, -(CH$_2$)$_3$-phenylene-(CH$_2$)$_7$-, -(CH$_2$)$_3$-phenylene-(CH$_2$)$_8$-, -(CH$_2$)$_4$-phenylene-CH$_2$-, - (CH$_2$)$_4$-phenylene-(CH$_2$)$_2$-, -(CH$_2$)$_4$-phenylene-(CH$_2$)$_3$-, -(CH$_2$)$_4$-phenylene-(CH$_2$)$_4$-, -(CH$_2$)$_4$-phenylene-(CH$_2$)$_5$-, -(CH$_2$)$_4$-phenylene-(CH$_2$)$_6$-, -(CH$_2$)$_4$-phenylene-(CH$_2$)$_7$-, -(CH$_2$)$_4$-phenylene-(CH$_2$)$_8$-, -(CH$_2$)$_5$-phenylene-(CH$_2$)$_1$-, -(CH$_2$)$_5$-phenylene-(CH$_2$)$_2$-, -(CH$_2$)$_5$-phenylene-(CH$_2$)$_3$-, -(CH$_2$)$_5$-phenylene-(CH$_2$)$_4$-, - (CH$_2$)$_5$-phenylene-(CH$_2$)$_5$-, -(CH$_2$)$_5$-phenylene-(CH$_2$)$_6$-, -(CH$_2$)$_5$-phenylene-(CH$_2$)$_7$-, -(CH$_2$)$_5$-phenylene-(CH$_2$)$_8$-, -(CH$_2$)$_6$-phenylene-(CH$_2$)$_1$-, -(CH$_2$)$_6$-phenylene-(CH$_2$)$_2$-, -(CH$_2$)$_6$-phenylene-(CH$_2$)$_3$-, -(CH$_2$)$_6$-phenylene-(CH$_2$)$_4$-, -(CH$_2$)$_6$-phenylene-(CH$_2$)$_5$-, -(CH$_2$)$_6$-phenylene-(CH$_2$)$_6$-, -(CH$_2$)$_6$-phenylene-(CH$_2$)$_7$-, - (CH$_2$)$_6$-phenylene-(CH$_2$)$_8$-, -(CH$_2$)$_7$-phenylene-(CH$_2$)$_1$-, -(CH$_2$)$_7$-phenylene-(CH$_2$)$_2$-, -(CH$_2$)$_7$-phenylene-(CH$_2$)$_3$-, -(CH$_2$)$_7$-phenylene-(CH$_2$)$_4$-, -(CH$_2$)$_7$-phenylene-(CH$_2$)$_8$-, -(CH$_2$)$_8$-phenylene-CH$_2$-, -(CH$_2$)$_8$-phenylene-(CH$_2$)$_2$-, -(CH$_2$)$_8$-phenylene-(CH$_2$)$_3$-, -(CH$_2$)$_8$-phenylene-(CH$_2$)$_4$-, -(CH$_2$)$_8$-phenylene-(CH$_2$)$_5$-, - (CH$_2$)$_8$-phenylene-(CH$_2$)$_6$-, -(CH$_2$)$_8$-phenylene-(CH$_2$)$_7$-, -N(R$_{a7}$)-CH$_2$-phenylene-CH$_2$-, -N(R$_{a7}$)-CH$_2$-phenylene-(CH$_2$)$_2$-, -N(R$_{a7}$)-CH$_2$-phenylene-(CH$_2$)$_3$-, -N(R$_{a7}$)-CH$_2$-phenylene-(CH$_2$)$_4$-, -N(R$_{a7}$)-CH$_2$-phenylene-(CH$_2$)$_5$-, -N(R$_{a7}$)-CH$_2$-phenylene-(CH$_2$)$_6$-, -N(R$_{a7}$)-CH$_2$-phenylene-(CH$_2$)$_7$-, -N(R$_{a7}$)-CH$_2$-phenylene-(CH$_2$)$_8$-, -CH$_2$-N(R$_{a7}$)-CH$_2$-phenylene-CH$_2$-, -CH$_2$-N(R$_{a7}$)-CH$_2$-phenylene-(CH$_2$)$_2$-, -CH$_2$-N(R$_{a7}$)-CH$_2$-phenylene-(CH$_2$)$_3$-, -CH$_2$-N(R$_{a7}$)-CH$_2$-phenylene-(CH$_2$)$_4$-, -CH$_2$-N(R$_{a7}$)-CH$_2$-phenylene-(CH$_2$)$_5$-, -CH$_2$-N(R$_{a7}$)-CH$_2$-phenylene-(CH$_2$)$_6$-, -CH$_2$-N(R$_{a7}$)-CH$_2$-phenylene-(CH$_2$)$_7$-, -CH$_2$-N(R$_{a7}$)-CH$_2$-phenylene-(CH$_2$)$_8$-, -CH$_2$-N(R$_{a7}$)-(CH$_2$)$_2$-phenylene-CH$_2$-, -CH$_2$-N(R$_{a7}$)-(CH$_2$)$_2$-phenylene-(CH$_2$)$_2$-, -CH$_2$-N(R$_{a7}$)-(CH$_2$)$_2$-phenylene-(CH$_2$)$_3$-, -CH$_2$-N(R$_{a7}$)-(CH$_2$)$_2$-phenylene-(CH$_2$)$_4$-, -CH$_2$-N(R$_{a7}$)-(CH$_2$)$_2$-phenylene-(CH$_2$)$_5$-, -CH$_2$-N(R$_{a7}$)-(CH$_2$)$_2$-phenylene-(CH$_2$)$_6$-, -CH$_2$-N(R$_{a7}$)-(CH$_2$)$_2$-phenylene-(CH$_2$)$_7$-, - CH$_2$-N(R$_{a7}$)-(CH$_2$)$_2$-phenylene-(CH$_2$)$_8$-, -(CH$_2$)$_2$-N(R$_{a7}$)-CH$_2$-phenylene-CH$_2$-, -(CH$_2$)$_2$-N(R$_{a7}$)-CH$_2$-phenylene-(CH$_2$)$_2$-, -(CH$_2$)$_2$-N(R$_{a7}$)-CH$_2$-phenylene-(CH$_2$)$_3$-, -(CH$_2$)$_2$-N(R$_{a7}$)-CH$_2$-phenylene-(CH$_2$)$_4$-, - (CH$_2$)$_2$-N(R$_{a7}$)-CH$_2$-phenylene-(CH$_2$)$_5$-, -(CH$_2$)$_2$-N(R$_{a7}$)-CH$_2$-phenylene-(CH$_2$)$_6$-, -(CH$_2$)$_2$-N(R$_{a7}$)-CH$_2$-phenylene-(CH$_2$)$_7$-, -(CH$_2$)$_2$-N(R$_{a7}$)-CH$_2$-phenylene-(CH$_2$)$_8$-, -(CH$_2$)$_3$-N(R$_{a7}$)-CH$_2$-phenylene-CH$_2$-, - (CH$_2$)$_3$-N(R$_{a7}$)-CH$_2$-phenylene-(CH$_2$)$_2$-, -(CH$_2$)$_3$-N(R$_{a7}$)-CH$_2$-phenylene-(CH$_2$)$_3$-, -(CH$_2$)$_3$-N(R$_{a7}$)-CH$_2$-phenylene-(CH$_2$)$_8$-, -(CH$_2$)$_4$-N(R$_{a7}$)-CH$_2$-phenylene-CH$_2$-, -(CH$_2$)$_4$-N(R$_{a7}$)-CH$_2$-phenylene-(CH$_2$)$_2$-, - (CH$_2$)$_4$-N(R$_{a7}$)-CH$_2$-phenylene-(CH$_2$)$_3$-, -(CH$_2$)$_4$-N(R$_{a7}$)-CH$_2$-phenylene-(CH$_2$)$_8$-, -(CH$_2$)$_5$-N(R$_{a7}$)-CH$_2$-phenylene-(CH$_2$)$_3$-, -(CH$_2$)$_5$-N(R$_{a7}$)-CH$_2$-phenylene-(CH$_2$)$_8$-, -(CH$_2$)$_6$-N(R$_{a7}$)-CH$_2$-phenylene-(CH$_2$)$_3$-, - (CH$_2$)$_6$-N(R$_{a7}$)-CH$_2$-phenylene-(CH$_2$)$_8$-, -(CH$_2$)$_7$-N(R$_{a7}$)-CH$_2$-phenylene-(CH$_2$)$_3$-, -(CH$_2$)$_8$-N(R$_{a7}$)-CH$_2$-phenylene-CH$_2$-, -(CH$_2$)$_8$-N(R$_{a7}$)-CH$_2$-phenylene-(CH$_2$)$_2$-, -(CH$_2$)$_8$-N(R$_{a7}$)-CH$_2$-phenylene-(CH$_2$)$_3$- - (CH$_2$)$_8$-N(R$_{a7}$)-CH$_2$-phenylene-(CH$_2$)$_4$-, -(CH$_2$)$_8$-N(R$_{a7}$)-CH$_2$-phenylene-(CH$_2$)$_5$-, -(CH$_2$)$_8$-N(R$_{a7}$)-CH$_2$-phenylene-(CH$_2$)$_6$-, -piperidinylene-CH$_2$-, -piperidinylene-(CH$_2$)$_2$-, -piperidinylene-(CH$_2$)$_3$-, - piperidinylene-(CH$_2$)$_4$-, -piperidinylene-(CH$_2$)$_5$-, -piperidinylene-(CH$_2$)$_6$-, -piperidinylene-(CH$_2$)$_7$-, - piperidinylene-(CH$_2$)$_8$-, -CH$_2$-piperidinylene-CH$_2$-, -CH$_2$-piperidinylene-(CH$_2$)$_2$-, -CH$_2$-piperidinylene-(CH$_2$)$_3$-, -CH$_2$-piperidinylene-(CH$_2$)$_4$-, -CH$_2$-piperidinylene-(CH$_2$)$_5$-, -CH$_2$-piperidinylene-(CH$_2$)$_6$-, -CH$_2$-piperidinylene-(CH$_2$)$_7$-, -CH$_2$-piperidinylene-(CH$_2$)$_8$-, -(CH$_2$)$_2$-piperidinylene-(CH$_2$)$_1$-, -(CH$_2$)$_2$-piperidinylene-(CH$_2$)$_2$-, -(CH$_2$)$_2$-piperidinylene-(CH$_2$)$_3$-, -(CH$_2$)$_2$-piperidinylene-(CH$_2$)$_4$-, -(CH$_2$)$_2$-piperidinylene-(CH$_2$)$_5$-, -(CH$_2$)$_2$-piperidinylene-(CH$_2$)$_6$-, -(CH$_2$)$_2$-piperidinylene-(CH$_2$)$_7$-, -(CH$_2$)$_2$-piperidinylene-(CH$_2$)$_8$-, -(CH$_2$)$_3$-piperidinylene-CH$_2$-, -(CH$_2$)$_3$-piperidinylene-(CH$_2$)$_2$-, -(CH$_2$)$_3$-piperidinylene-(CH$_2$)$_3$-, -(CH$_2$)$_3$-piperidinylene-(CH$_2$)$_4$-, -(CH$_2$)$_3$-piperidinylene-(CH$_2$)$_5$-, -(CH$_2$)$_3$-piperidinylene-(CH$_2$)$_6$-, -(CH$_2$)$_3$-piperidinylene-(CH$_2$)$_7$-, -(CH$_2$)$_3$-piperidinylene-(CH$_2$)$_8$-, -(CH$_2$)$_4$-piperidinylene-CH$_2$-, -(CH$_2$)$_4$-piperidinylene-(CH$_2$)$_2$-, -(CH$_2$)$_4$-piperidinylene-(CH$_2$)$_3$-, -(CH$_2$)$_4$-piperidinylene-(CH$_2$)$_4$-, -(CH$_2$)$_4$-piperidinylene-(CH$_2$)$_5$-, -(CH$_2$)$_4$-piperidinylene-(CH$_2$)$_6$-, -(CH$_2$)$_4$-piperidinylene-(CH$_2$)$_7$-, -(CH$_2$)$_4$-piperidinylene-(CH$_2$)$_8$-, -(CH$_2$)$_5$-piperidinylene-(CH$_2$)$_1$-, -(CH$_2$)$_5$-piperidinylene-(CH$_2$)$_2$-, -(CH$_2$)$_5$-piperidinylene-(CH$_2$)$_3$-, -(CH$_2$)$_5$-piperidinylene-(CH$_2$)$_4$-, -(CH$_2$)$_5$-piperidinylene-(CH$_2$)$_5$-, -(CH$_2$)$_5$-piperidinylene-(CH$_2$)$_6$-, -(CH$_2$)$_5$-piperidinylene-(CH$_2$)$_7$-, -(CH$_2$)$_5$-piperidinylene-(CH$_2$)$_8$-, -(CH$_2$)$_6$-piperidinylene-(CH$_2$)$_1$-, -(CH$_2$)$_6$-piperidinylene-(CH$_2$)$_2$-, -(CH$_2$)$_6$-piperidinylene-(CH$_2$)$_3$-, -(CH$_2$)$_6$-piperidinylene-(CH$_2$)$_4$-, -(CH$_2$)$_6$-piperidinylene-(CH$_2$)$_5$-, -(CH$_2$)$_6$-piperidinylene-(CH$_2$)$_6$-, -(CH$_2$)$_6$-piperidinylene-(CH$_2$)$_7$-, -(CH$_2$)$_6$-piperidinylene-(CH$_2$)$_8$-, -(CH$_2$)$_7$-piperidinylene-(CH$_2$)$_1$-, -(CH$_2$)$_7$-piperidinylene-(CH$_2$)$_2$-, -(CH$_2$)$_7$-piperidinylene-(CH$_2$)$_3$-, -(CH$_2$)$_7$-piperidinylene-(CH$_2$)$_4$-, -(CH$_2$)$_7$-piperidinylene-(CH$_2$)$_8$-, -(CH$_2$)$_8$-piperidinylene-CH$_2$-, -(CH$_2$)$_8$-piperidinylene-(CH$_2$)$_2$-, -(CH$_2$)$_8$-piperidinylene-(CH$_2$)$_3$-, -(CH$_2$)$_8$-piperidinylene-(CH$_2$)$_4$-, -(CH$_2$)$_8$-piperidinylene-(CH$_2$)$_5$-, -(CH$_2$)$_8$-piperidinylene-(CH$_2$)$_6$-, -(CH$_2$)$_8$-piperidinylene-(CH$_2$)$_7$-, -N(R$_{a7}$)-CH$_2$-piperidinylene-CH$_2$-, -N(R$_{a7}$)-CH$_2$-piperidinylene-(CH$_2$)$_2$-, -N(R$_{a7}$)-CH$_2$-piperidinyle-

ne-$(CH_2)_3$-, -N($R_{a7}$)-$CH_2$-piperidinylene-$(CH_2)_4$-, -N($R_{a7}$)-$CH_2$-piperidinylene-$(CH_2)_5$-, -N($R_{a7}$)-$CH_2$-piperidinylene-$(CH_2)_6$-, - N($R_{a7}$ )-$CH_2$-piperidinylene-$(CH_2)_7$-, -N($R_{a7}$)-$CH_2$-piperidinylene-$(CH_2)_8$-, -$CH_2$-N($R_{a7}$)-$CH_2$-piperidinylene-$CH_2$-, -$CH_2$-N($R_{a7}$)-$CH_2$-piperidinylene-$(CH_2)_2$-, -$CH_2$-N($R_{a7}$)-$CH_2$-piperidinylene-$(CH_2)_3$-, -$CH_2$-N($R_{a7}$)-$CH_2$-piperidinylene-$(CH_2)_4$- -$CH_2$-N($R_{a7}$)-$CH_2$-piperidinylene-$(CH_2)_5$-, -$CH_2$-N($R_{a7}$ )-$CH_2$-piperidinylene-$(CH_2)_6$-, -$CH_2$-N($R_{a7}$ )-$CH_2$-piperidinylene-($CH_{2h}$-, -$CH_2$-N($R_{a7}$)-$CH_2$-piperidinylene-$(CH_2)_8$-, -$CH_2$-N($R_{a7}$)-$(CH_2)_2$-piperidinylene-$CH_2$-, -$CH_2$-N($R_{a7}$)-$(CH_2)_2$-piperidinylene-$(CH_2)_2$-, -$CH_2$-N($R_{a7}$)-$(CH_2)_2$-piperidinylene-$(CH_2)_3$-, -$CH_2$-N($R_{a7}$)-$(CH_2)_2$-piperidinylene-$(CH_2)_4$-, -$CH_2$-N($R_{a7}$)-$(CH_2)_2$-piperidinylene-$(CH_2)_5$-, -$CH_2$-N($R_{a7}$)-$(CH_2)_2$-piperidinylene-$(CH_2)_6$-, -$CH_2$-N($R_{a7}$)-$(CH_2)_2$-piperidinylene-$(CH_2)_7$-, -$CH_2$-N($R_{a7}$)-$(CH_2)_2$-piperidinylene-$(CH_2)_8$-, -$(CH_2)_2$-N($R_{a7}$)-$CH_2$-piperidinylene-$CH_2$-, -$(CH_2)_2$-N($R_{a7}$)-$CH_2$-piperidinylene-$(CH_2)_2$-, -$(CH_2)_2$-N($R_{a7}$)-$CH_2$-piperidinylene-$(CH_2)_3$-, -$(CH_2)_2$-N($R_{a7}$)-$CH_2$-piperidinylene-$(CH_2)_4$-, -$(CH_2)_2$-N($R_{a7}$ )-$CH_2$-piperidinylene-$(CH_2)_5$-, -$(CH_2)_2$-N($R_{a7}$)-$CH_2$-piperidinylene-$(CH_2)_6$-, -$(CH_2)_2$-N($R_{a7}$)-$CH_2$-piperidinylene-$(CH_2)_7$-, -$(CH_2)_2$-N($R_{a7}$)-$CH_2$-piperidinylene-$(CH_2)_8$-, -$(CH_2)_3$-N($R_{a7}$)-$CH_2$-piperidinylene-$CH_2$-, -$(CH_2)_3$-N($R_{a7}$)-$CH_2$-piperidinylene-$(CH_2)_2$-, -$(CH_2)_3$-N($R_{a7}$)-$CH_2$-piperidinylene-$(CH_2)_3$-, -$(CH_2)_3$-N($R_{a7}$)-$CH_2$-piperidinylene-$(CH_2)_8$-, -$(CH_2)_4$-N($R_{a7}$)-$CH_2$-piperidinylene-$CH_2$-, -$(CH_2)_4$-N($R_{a7}$)-$CH_2$-piperidinylene-$(CH_2)_2$-, -$(CH_2)_4$-N($R_{a7}$)-$CH_2$-piperidinylene-$(CH_2)_3$-, -$(CH_2)_4$-N($R_{a7}$)-$CH_2$-Piperidinylene-$(CH_2)_8$-, -$(CH_2)_5$-N($R_{a7}$)-$CH_2$-piperidinylene-$(CH_2)_3$-, -$(CH_2)_5$-N($R_{a7}$)-$CH_2$-piperidinylene-$(CH_2)_8$-, -$(CH_2)_6$-N($R_{a7}$)-$CH_2$-piperidinylene-$(CH_2)_3$-, -$(CH_2)_6$-N($R_{a7}$)-$CH_2$-piperidinylene-$(CH_2)_8$-, -($CH_{2h}$-N($R_{a7}$)-$CH_2$-piperidinylene-$(CH_2)_3$-, -$(CH_2)_8$-N($R_{a7}$)-$CH_2$-piperidinylene-$CH_2$-, -$(CH_2)_8$-N($R_{a7}$)-$CH_2$-piperidinylene-$(CH_2)_2$-, -$(CH_2)_8$-N($R_{a7}$)-$CH_2$-piperidinylene-$(CH_2)_3$-, -$(CH_2)_8$-N($R_{a7}$)-$CH_2$-piperidinylene-$(CH_2)_4$-, -$(CH_2)_8$-N($R_{a7}$)-$CH_2$-piperidinylene-$(CH_2)_5$-, -$(CH_2)_8$-N($R_{a7}$)-$CH_2$-piperidinylene-$(CH_2)_6$-, -piperazinylene-$CH_2$-, -piperazinylene-$(CH_2)_2$-, -piperazinylene-$(CH_2)_3$-, -piperazinylene-$(CH_2)_4$-, -piperazinylene-$(CH_2)_5$-, -piperazinylene-$(CH_2)_6$-, -piperazinylene-$(CH_2)_7$-, -piperazinylene-$(CH_2)_8$-, -$CH_2$-piperazinylene-$CH_2$-, -$CH_2$-piperazinylene-$(CH_2)_2$-, -$CH_2$-piperazinylene-$(CH_2)_3$-, -$CH_2$-piperazinylene-$(CH_2)_4$-, -$CH_2$-piperazinylene-$(CH_2)_5$-, -$CH_2$-piperazinylene-$(CH_2)_6$-, -$CH_2$-piperazinylene-$(CH_2)_7$-, -$CH_2$-piperazinylene-$(CH_2)_8$- -$(CH_2)_2$-piperazinylene-$(CH_2)_1$-, -$(CH_2)_2$-piperazinylene-$(CH_2)_2$-, -(CH2)2-piperazinylene-$(CH_2)_3$-, -$(CH_2)_2$-piperazinylene-$(CH_2)_4$-, -$(CH_2)_2$-piperazinylene-$(CH_2)_5$-, -$(CH_2)_2$-piperazinylene-$(CH_2)_6$-, -$(CH_2)_2$-piperazinylene-$(CH_2)_7$-, -$(CH_2)_2$-piperazinylene-$(CH_2)_8$-, -$(CH_2)_3$-piperazinylene-$CH_2$-, -$(CH_2)_3$-piperazinylene-$(CH_2)_2$-, -$(CH_2)_3$-piperazinylene-$(CH_2)_3$-, -$(CH_2)_3$-piperazinylene-$(CH_2)_4$-, -$(CH_2)_3$-piperazinylene-$(CH_2)_5$-, -$(CH_2)_3$-piperazinylene-$(CH_2)_6$-, -$(CH_2)_3$-piperazinylene-$(CH_2)_7$-, -$(CH_2)_3$-piperazinylene-$(CH_2)_8$-, -$(CH_2)_4$-piperazinylene-$CH_2$-, -$(CH_2)_4$-piperazinylene-$(CH_2)_2$-, -$(CH_2)_4$-piperazinylene-$(CH_2)_3$-, -$(CH_2)_4$-piperazinylene-$(CH_2)_4$-, -$(CH_2)_4$-piperazinylene-$(CH_2)_5$-, -$(CH_2)_4$-piperazinylene-$(CH_2)_6$-, -$(CH_2)_4$-piperazinylene-$(CH_2)_7$-, -$(CH_2)_4$-piperazinylene-$(CH_2)_8$-, -$(CH_2)_5$-piperazinylene-$(CH_2)_1$-, -$(CH_2)_5$-piperazinylene-$(CH_2)_2$-, -$(CH_2)_5$-piperazinylene-$(CH_2)_3$-, -$(CH_2)_5$-piperazinylene-$(CH_2)_4$-, -$(CH_2)_5$-piperazinylene-$(CH_2)_5$-, -$(CH_2)_5$-piperazinylene-$(CH_2)_6$-, -$(CH_2)_5$-piperazinylene-$(CH_2)_7$-, -$(CH_2)_5$-piperazinylene-$(CH_2)_8$-, -$(CH_2)_6$-piperazinylene-$(CH_2)_1$-, -$(CH_2)_6$-piperazinylene-$(CH_2)_2$-, -$(CH_2)_6$-piperazinylene-$(CH_2)_3$-, -$(CH_2)_6$-piperazinylene-$(CH_2)_4$-, -$(CH_2)_6$-piperazinylene-$(CH_2)_5$-, -$(CH_2)_6$-piperazinylene-$(CH_2)_6$-, -$(CH_2)_6$-piperazinylene-$(CH_2)_7$-, -$(CH_2)_6$-piperazinylene-$(CH_2)_8$-, -$(CH_2)_7$-piperazinylene-$(CH_2)_1$-, -$(CH_2)_7$-piperazinylene-$(CH_2)_2$-, -$(CH_2)_7$-piperazinylene-$(CH_2)_3$-, -$(CH_2)_7$-piperazinylene-$(CH_2)_4$-, -$(CH_2)_8$-piperazinylene-$CH_2$-, -$(CH_2)_8$-piperazinylene-$(CH_2)_2$-, -$(CH_2)_8$-piperazinylene-$(CH_2)_3$-, -$(CH_2)_8$-piperazinylene-$(CH_2)_4$-, -$(CH_2)_8$-piperazinylene-$(CH_2)_5$-, or -$(CH_2)_8$-piperazinylene-$(CH_2)_6$-;

wherein each $R_{a7}$ independently represents H or $C_1$-$_6$ alkyl (e.g., methyl, ethyl, propyl, or isopropyl);
each phenylene is independently optionally substituted with a substituent selected from the group consisting of deuterium, hydroxy, amino, mercapto, nitro, halogen, cyano, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; and
the piperazinylene and piperidinylene are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof. In the present disclosure, unless otherwise specified, any one end of the general formulas provided above may be connected to R of the compound of Formula (I), while the other end is connected to ring $A^3$. For example, in the group -O-$CH_2$-, the O can be connected to R of the compound of Formula (I), and the $CH_2$ can be connected to ring $A^3$ of the compound of Formula (I), and vice versa.

In some embodiments, L represents a bond.

In some embodiments, examples of L include, but are not limited to, the following groups:

C(O), C(O)NH, NHC(O), -$CH_2$-, -$(CH_2)_2$-, -$(CH_2)_3$-, -$(CH_2)_4$-, -$(CH_2)_5$-, -$(CH_2)_6$-, -$(CH_2)_7$-, -$(CH_2)_8$-, - $(CH_2)_9$-, -$(CH_2)_{10}$-, -$(CH_2)_{11}$-, -$(CH_2)_{12}$-, -$(CH_2)_{13}$-, -$(CH_2)_{14}$-, -$(CH_2)_{15}$-; -O-$CH_2$-, -O-$(CH_2)_2$-, -O-$(CH_2)_3$-, - O-$(CH_2)_4$-, -O-$(CH_2)_5$-, -O-$(CH_2)_6$-, -O-$(CH_2)_7$-, -O-$(CH_2)_8$-, -O-$(CH_2)_9$-, -O-$(CH_2)_{10}$-, -$(CH_2)_1$-O-, -$(CH_2)_2$-O-, -$(CH_2)_3$-O-,

$-(CH_2)_4-O-$, $-(CH_2)_5-O-$, $-(CH_2)_6-O-$, $-(CH_2)_7-O-$, $-(CH_2)_8-O-$, $-(CH_2)_9-O-$, $-(CH_2)_{10}-O-$, $- CH_2-O-CH_2-$, $-CH_2-O-(CH_2)_2-$, $-(CH_2)_1-O-(CH_2)_3-$, $-(CH_2)_1-O-(CH_2)_4-$, $-(CH_2)_1-O-(CH_2)_5-$, $-(CH_2)_1-O-(CH_2)_6-$, $-(CH_2)_1-O-(CH_2)_7-$, $-(CH_2)_1-O-(CH_2)_8-$, $-(CH_2)_2-O-(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, $-(CH_2)_2-O-(CH_2)_6-$, $-(CH_2)_2-O-(CH_2)_7-$, $-(CH_2)_2-O-(CH_2)_8-$, $-(CH_2)_3-O-(CH_2)_1-$, $-(CH_2)_3-O-(CH_2)_2-$, $-(CH_2)_3-O-(CH_2)_3-$, $-(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_1-$, $-(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, $-(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, $-(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, $-(CH_2)_7-O-(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$, $-CH(CH_3)-O-(CH_2)_2-$, $-CH(CH_3)-O-(CH_2)_3-$, $-CH(CH_3)-O-(CH_2)_4-$, $-CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-(O(CH_2)_2)_2-$, $-(O(CH_2)_2)_3-$, $- (O(CH_2)_2)_4-$, $-(O(CH_2)_2)_5-$, $-(O(CH_2)_2)_6-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_1-OCH_2-$, $-CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-CH_2-(O(CH_2)_2)_6-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, $-(CH_2)_2-(O(CH_2)_2)_5-$, $-(CH_2)_2-(O(CH_2)_2)_6-$, $-(CH_2)_3-(O(CH_2)_2)_2-$, $-(CH_2)_3-(O(CH_2)_2)_3-$, $-(CH_2)_3-(O(CH_2)_2)_4-$, $-(CH_2)_3-(O(CH_2)_2)_5-$, $-(CH_2)_4-(O(CH_2)_2)_2-$, $-(CH_2)_4-(O(CH_2)_2)_3-$, $-(CH_2)_4-(O(CH_2)_2)_4-$, $-(CH_2)_4-(O(CH_2)_2)_5-$, $-CH_2-(O(CH_2)_3)_2-$, $-CH_2-(O(CH_2)_3)_3-$, $-CH_2-(O(CH_2)_3)_4-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_3-(O(CH_2)_3)_2-$, $-(CH_2)_3-(O(CH_2)_3)_3-$, $-CH_2-O-(CH_2)_2-O-(CH_2)_3-$, $-CH_2-(O(CH_2)_2)_2-(O(CH_2)_3)_2-$, $(CH_2)_2-O-(CH_2)_2-O-(CH_2)_3-$, $-(CH_2)_2-(O(CH_2)_2)_2-(O(CH_2)_3)_2-$, $-(CH_2)_3-O-(CH_2)_2-O-(CH_2)_3-$, $-(CH_2)_3-(O(CH_2)_2)_2-(O(CH_2)_3)_2-$, $-CH_2-O-(CH_2)_3-O-(CH_2)_2-$,$-CH_2-(O(CH_2)_3)_2-(O(CH_2)_2)_2-$, $(CH_2)_1-N(R_{a7})-(CH_2)_1-$, $-(CH_2)_1-N(R_{a7})-(CH_2)_2-$, $-(CH_2)_1-N(R_{a7})-(CH_2)_3-$,$-(CH_2)_1-N(R_{a7})-(CH_2)_4-$, $-(CH_2)_1-N(R_{a7})-(CH_2)_5-$, $-(CH_2)_1-N(R_{a7})-(CH_2)_6-$, $-N(R_{a7})-(CH_2)_1-$, $-N(R_{a7})-(CH_2)_2-$, $-N(R_{a7})-(CH_2)_3-$, $-N(R_{a7})-(CH_2)_4-$, $-N(R_{a7})-(CH_2)_5-$, $-N(R_{a7})-(CH_2)_6-$, $-(CH_2)_2-N(R_{a7})-(CH_2)_1-$, $-(CH_2)_2-N(R_{a7})-(CH_2)_2-$, $-(CH_2)_2-N(R_{a7})-(CH_2)_3-$, $-(CH_2)_2-N(R_{a7})-(CH_2)_4-$, $-(CH_2)_2-N(R_{a7})-(CH_2)_5-$, $-(CH_2)_2-N(R_{a7})-(CH_2)_6-$, $-(CH_2)_3-N(Ra_7)-(CH_2)_1-$, $-(CH_2)_3-N(R_{a7})-(CH_2)_2-$, $-(CH_2)_3-N(R_{a7})-(CH_2)_3-$, $-(CH_2)_4-N(R_{a7})-(CH_2)_1-$, $-(CH_2)_4-N(R_{a7})-(CH_2)_2-$, $-(CH_2)_4-N(R_{a7})-(CH_2)_3-$, $-(CH_2)_4-N(R_{a7})-(CH_2)_4-$, $-(CH_2)_5-N(R_{a7})-(CH_2)_1-$, $-(CH_2)_5-N(R_{a7})-(CH_2)_2-$, $-(CH_2)_5-N(R_{a7})-(CH_2)_3-$, $-(CH_2)_5-N(R_{a7})-(CH_2)_4-$, $-(CH_2)_5-N(R_{a7})-(CH_2)_5-$, $-(CH_2)_6-N(R_{a7})-(CH_2)_1-$, $-(CH_2)_6-N(R_{a7})-(CH_2)_2-$, $-(CH_2)_6-N(R_{a7})-(CH_2)_3-$, $-(CH_2)_7-N(R_{a7})-(CH_2)_1-$, $-(CH_2)_7-N(R_{a7})-(CH_2)_2-$, $-(CH_2)_7-N(R_{a7})-(CH_2)_3-$, $-(CH_2)_8-N(R_{a7})-(CH_2)_1-$, $-(CH_2)_8-N(R_{a7})-(CH_2)_2-$, $-(CH_2)_8-N(R_{a7})-(CH_2)_3-$,$-CH(CH_3)-N(Ra_7)-(CH_2)_1-$, $-CH(CH_3)-N(R_{a7})-(CH_2)_2-$, $-CH(CH_3)-N(R_{a7})-(CH_2)_3-$, $-CH(CH_3)-N(R_{a7})-(CH_2)_4-$, $-CH(CH_3)-N(R_{a7})-(CH_2)_5-$, $-CH(CH_3)-N(R_{a7})-(CH_2)_6-$, $-(CH_2)_1-N(R_{a7})-$, $-(CH_2)_2-N(R_{a7})-$, $-(CH_2)_3-N(R_{a7})-$,$-(CH_2)_4-N(R_{a7})-$, $-(CH_2)_5-N(R_{a7})-$, $-(CH_2)_6-N(R_{a7})-$, $-(CH_2)_7-N(R_{a7})-$, $-(CH_2)_8-N(R_{a7})-$, $-(CH_2)_9-N(R_{a7})-$,$-(CH_2)_{10}-N(R_{a7})-$, $-C(O)NHCH_2-$, $-C(O)NH(CH_2)_2-$, $-C(O)NH(CH_2)_3-$, $-C(O)NH(CH_2)_4-$, $-C(O)NH(CH_2)_5-$,$-CH_2C(O)NHCH_2-$, $-(CH_2)_2C(O)NH(CH_2)_2-$,$-(CH_2)_2C(O)NH(CH_2)_3-$, $-(CH_2)_2C(O)NH(CH_2)_4-$,$-(CH_2)_2C(O)NH(CH_2)_5-$, $-(CH_2)_3C(O)NH(CH_2)_3-$, $-(CH_2)_3C(O)NH(CH_2)_4-$, $-(CH_2)_4C(O)NH(CH_2)_4-$,$-(CH_2)_5C(O)NH(CH_2)_5-$, $-(CH_2)_2C(O)NH(CH_2)_2-O-(CH_2)_2-$, $-NHC(O)CH_2-$, $-NHC(O)(CH_2)_2-$,$-NHC(O)(CH_2)_3-$, $-NHC(O)(CH_2)_4-$, $-NHC(O)(CH_2)_5-$, $-CH_2NHC(O)CH_2-$, $-(CH_2)_2NHC(O)(CH_2)_2-$,$-(CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$,$-(CH_2)_3NHC(O)(CH_2)_4-$, $-(CH_2)_4NHC(O)(CH_2)_4-$, $-(CH_2)_5NHC(O)(CH_2)_5-$, $-(CH_2)_2NHC(O)(CH_2)_2-O-(CH_2)_2-$, $-(CH_2)_4NHC(O)CH_2-$, $-CH_2$-phenylene-$CH_2-$, $-CH_2$-phenylene-$(CH_2)_2-$, $-CH_2$-phenylene-$(CH_2)_3-$,$-CH_2$-phenylene-$(CH_2)_4-$, $-CH_2$-phenylene-$(CH_2)_5-$, $-CH_2$-phenylene-$(CH_2)_6-$, $-(CH_2)_2$-phenylene-$(CH_2)_1-$,$-(CH_2)_2$-phenylene-$(CH_2)_2-$, $-(CH_2)_2$-phenylene-$(CH_2)_3-$, $-(CH_2)_2$-phenylene-$(CH_2)_4-$, $-(CH_2)_2$-phenylene-$(CH_2)_5-$, $-(CH_2)_3$-phenylene-$CH_2-$, $-(CH_2)_3$-phenylene-$(CH_2)_2-$, $-(CH_2)_3$-phenylene-$(CH_2)_3-$, $-(CH_2)_3$-phenylene-$(CH_2)_4-$, $-(CH_2)_3$-phenylene-$(CH_2)_5-$, $-(CH_2)_4$-phenylene-$CH_2-$, $-(CH_2)_4$-phenylene-$(CH_2)_2-$,$-(CH_2)_4$-phenylene-$(CH_2)_3-$, $-(CH_2)_4$-phenylene-$(CH_2)_4-$, $-(CH_2)_4$-phenylene-$(CH_2)_5-$, $-CH_2$-piperidinylene-$CH_2-$, $-CH_2$-piperidinylene-$(CH_2)_2-$, $-CH_2$-piperidinylene-$(CH_2)_3-$, $-CH_2$-piperidinylene-$(CH_2)_4-$, $-CH_2$-piperidinylene-$(CH_2)_5-$, $-CH_2$-piperidinylene-$(CH_2)_6-$, $-(CH_2)_2$-piperidinylene-$(CH_2)_1-$, $-(CH_2)_2$-piperidinylene-$(CH_2)_2-$, $-(CH_2)_2$-piperidinylene-$(CH_2)_3-$, $-(CH_2)_2$-piperidinylene-$(CH_2)_4-$, $-(CH_2)_2$-piperidinylene-$(CH_2)_5-$, $-(CH_2)_3$-piperidinylene-$CH_2-$, $-(CH_2)_3$-piperidinylene-$(CH_2)_2-$, $-(CH_2)_3$-piperidinylene-$(CH_2)_3-$, $-(CH_2)_3$-piperidinylene-$(CH_2)_4-$, $-(CH_2)_3$-piperidinylene-$(CH_2)_5-$, $-CH_2$-piperazinylene-$CH_2-$, $-CH_2$-piperazinylene-$(CH_2)_2-$, $-CH_2$-piperazinylene-$(CH_2)_3-$, $-CH_2$-piperazinylene-$(CH_2)_4-$, $-CH_2$-piperazinylene-$(CH_2)_5-$, $-(CH_2)_2$-piperazinylene-$(CH_2)_1-$, $-(CH_2)_2$-piperazinylene-$(CH_2)_2-$,$-(CH_2)_2$-piperazinylene-$(CH_2)_3-$, $-(CH_2)_2$-piperazinylene-$(CH_2)_4-$, $-(CH_2)_2$-piperazinylene-$(CH_2)_5-$, $-(CH_2)_3$-piperazinylene-$CH_2-$, $-(CH_2)_3$-piperazinylene-$(CH_2)_2-$, $-(CH_2)_3$-piperazinylene-$(CH_2)_3-$, $-(CH_2)_3$-piperazinylene-$(CH_2)_4-$, $-(CH_2)_3$-piperazinylene-$(CH_2)_5-$, $-(CH_2)_4$-piperazinylene-$CH_2-$, $-(CH_2)_4$-piperazinylene-$(CH_2)_2-$, $-(CH_2)_4$-piperazinylene-$(CH_2)_3-$, $-(CH_2)_4$-piperazinylene-$(CH_2)_4-$, $-(CH_2)_4$-piperazinylene-$(CH_2)_5-$, $-(CH_2)_8$-piperazinylene-$CH_2-$, $-(CH_2)_8$-piperazinylene-$(CH_2)_2-$, $-(CH_2)_8$-piperazinylene-$(CH_2)_3-$, $-(CH_2)_8$-piperazinylene-$(CH_2)_4-$, or $-(CH_2)_8$-piperazinylene-$(CH_2)_5-$;

wherein each $R_{a7}$ independently represents H, or $C_{1-6}$ alkyl (e.g., methyl, ethyl, propyl, or isopropyl);

each phenylene is independently optionally substituted with a substituent selected from the group consisting of

deuterium, hydroxy, amino, mercapto, nitro, halogen, cyano, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof;

the piperazinylene and piperidinylene are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.

In some embodiments, examples of L include, but are not limited to, the following groups:

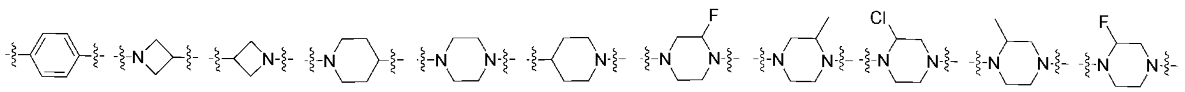

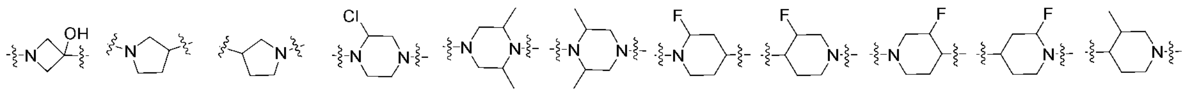

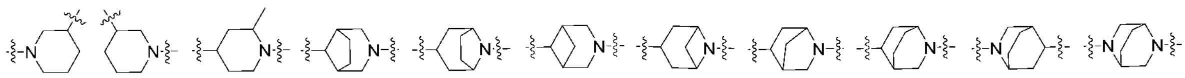

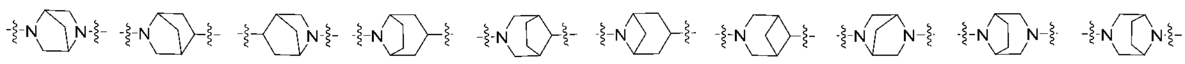

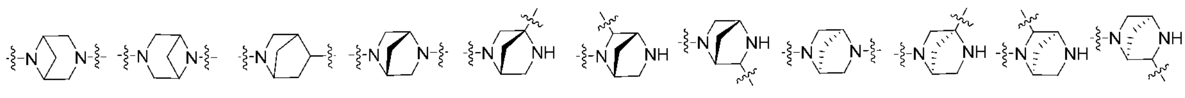

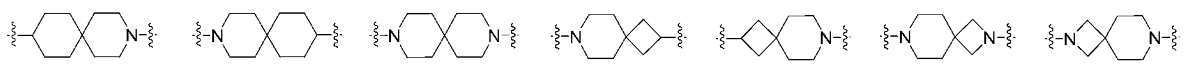

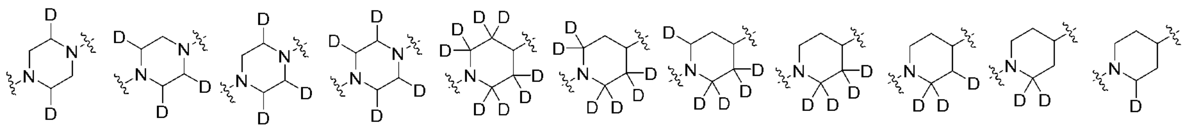

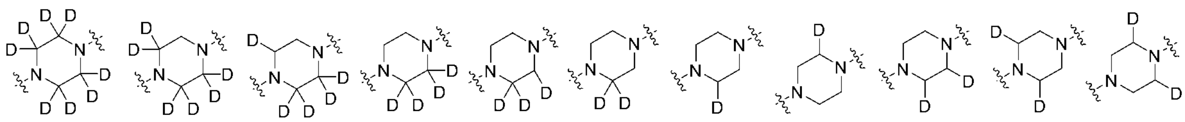

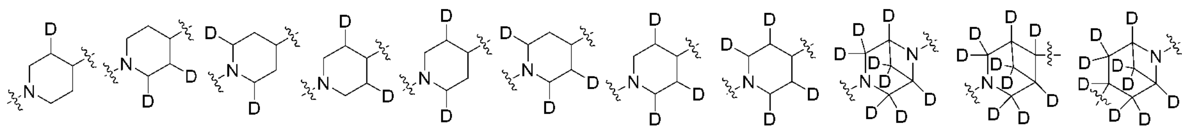

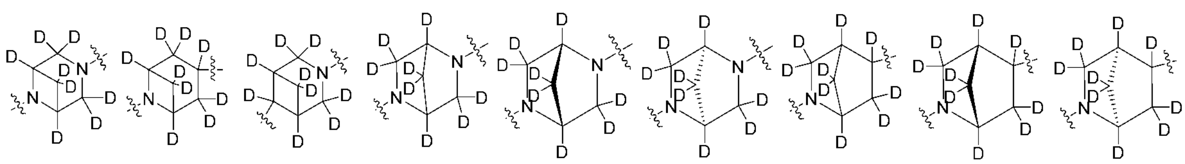

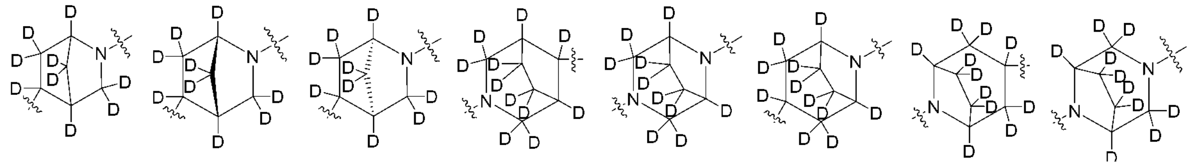

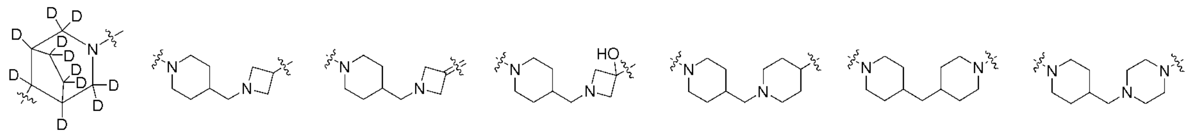

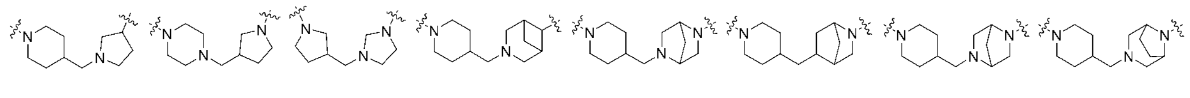

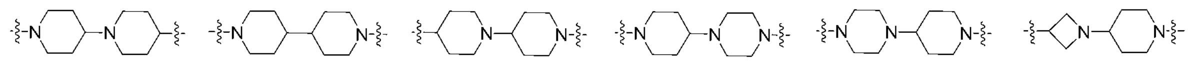

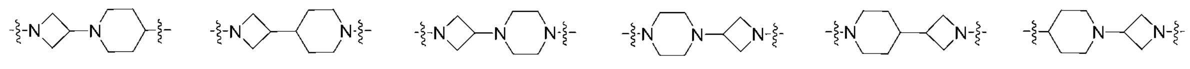

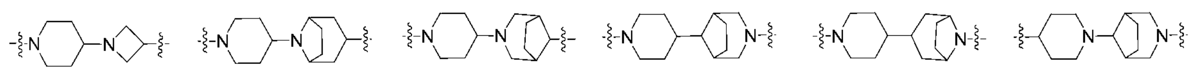

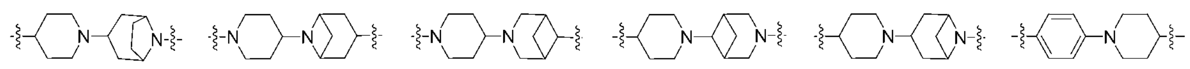

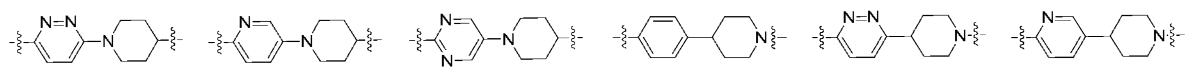

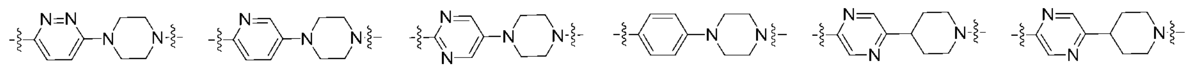

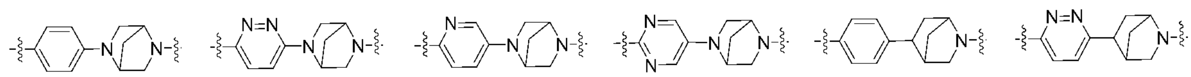

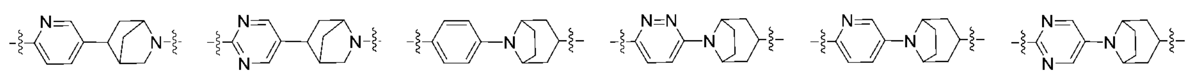

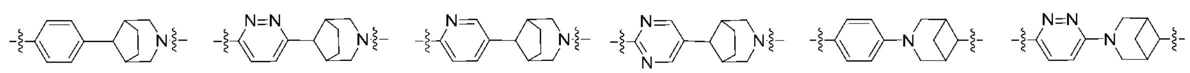

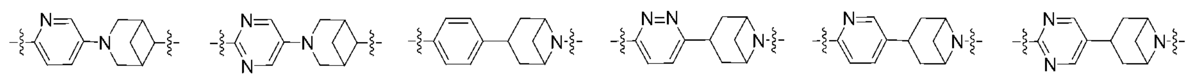

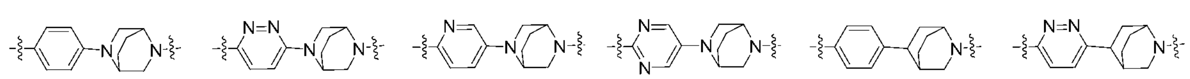

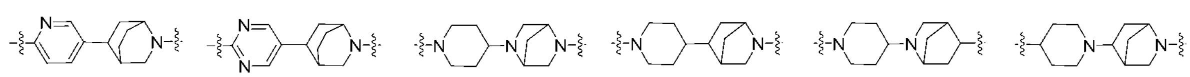

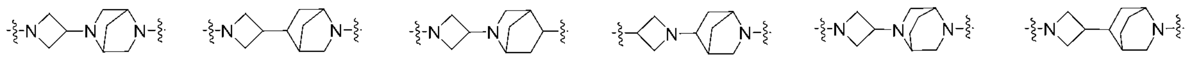

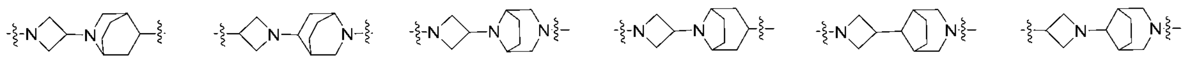

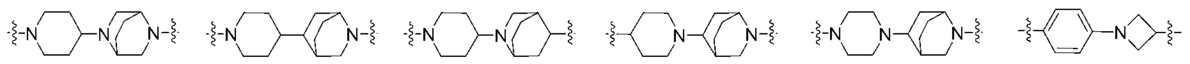

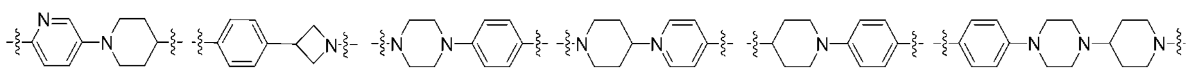

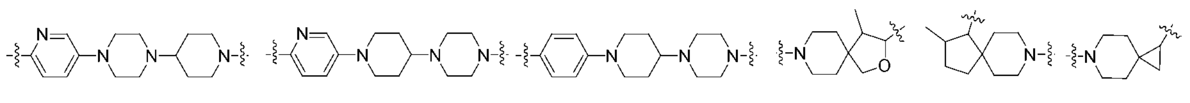

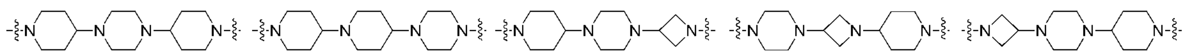

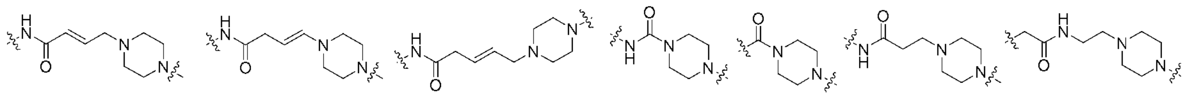

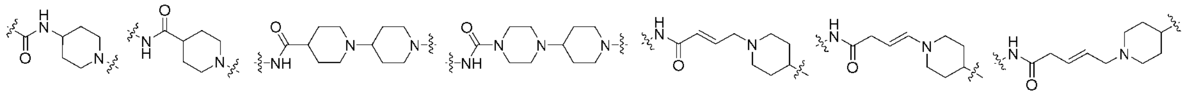

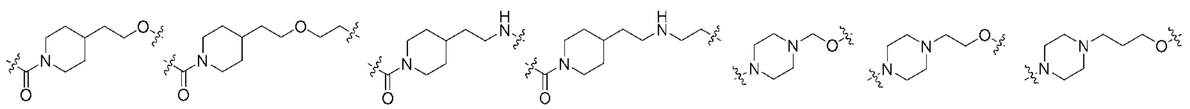

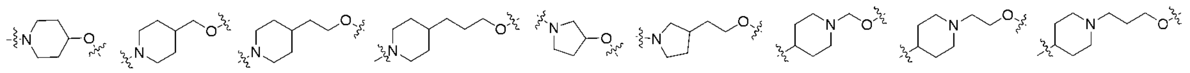

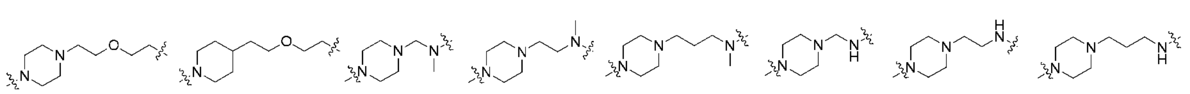

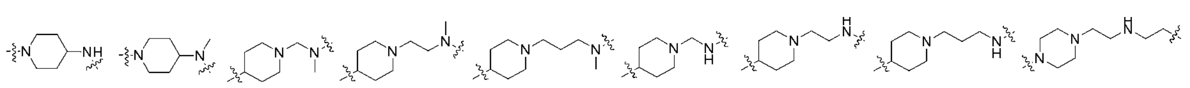

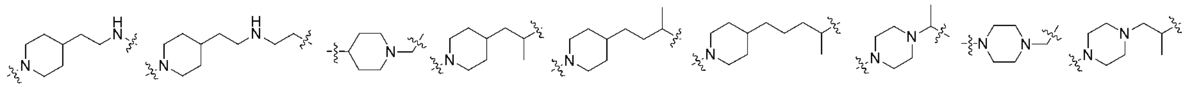

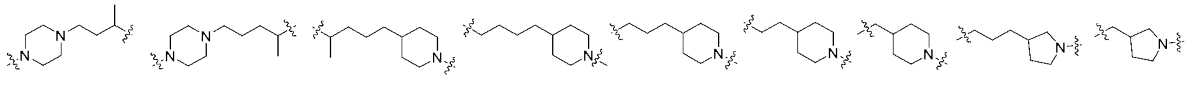

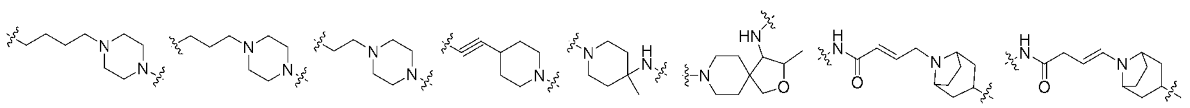

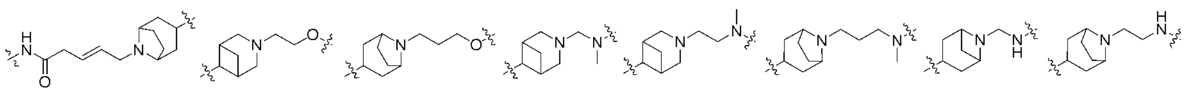

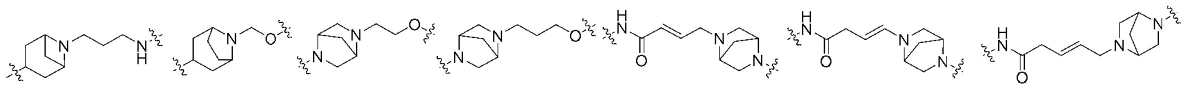

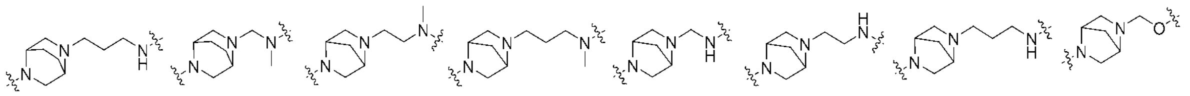

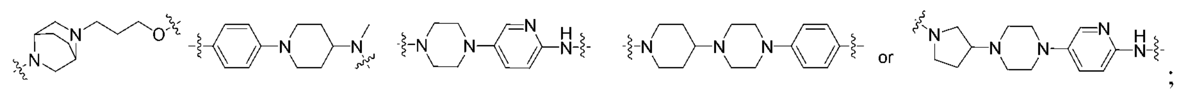

or ;

wherein the groups are optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl.

In some embodiments, X represents:

$NR_1R_2$, $C(O)NR_3R_4$, $NHC(O)R_5$, $NHC(O)NR_6R_7$, $OR_8$, $SR_9$, $S(O)_2NR_{10}R_{11}$, or $N(R_{12})S(O)_2R_{13}$, wherein $R_1$, $R_3$, $R_4$, $R_5$, $R_6$, $R_7$, $R_9$, $R_{10}$, $R_{11}$, and $R_{12}$ each independently represent H, optionally substituted linear or branched alkyl (e.g., optionally substituted linear or branched $C_{1-10}$ alkyl, or optionally substituted linear or branched $C_{1-6}$ alkyl), optionally substituted cycloalkyl (e.g., optionally substituted $C_{3-30}$ cycloalkyl, optionally substituted $C_{3-20}$ cycloalkyl, or optionally substituted $C_{3-15}$ cycloalkyl), optionally substituted aryl (e.g., optionally substituted $C_{5-30}$ aryl, optionally substituted $C_{5-20}$ aryl, or optionally substituted $C_{5-15}$ aryl), optionally substituted heterocyclyl (e.g., optionally substituted 4- to 30-membered heterocyclyl, optionally substituted 4- to 20-membered heterocyclyl, or optionally substituted 4- to 15-membered heterocyclyl) or optionally substituted heteroaryl (e.g., optionally substituted 5- to 30-membered heteroaryl, optionally substituted 5- to 20-membered heteroaryl, or optionally substituted 5- to 15-membered heteroaryl); and $R_2$, $R_8$, and $R_{13}$ each independently represent optionally substituted linear or branched alkyl (e.g., optionally substituted linear or branched $C_{1-10}$ alkyl, or optionally substituted linear or branched $C_{1-6}$ alkyl), optionally substituted cycloalkyl (e.g., optionally substituted $C_{3-30}$ cycloalkyl, optionally substituted $C_{3-20}$ cycloalkyl, or optionally substituted $C_{3-15}$ cycloalkyl), optionally substituted aryl (e.g., optionally substituted $C_{5-30}$ aryl, optionally substituted $C_{5-20}$ aryl, or optionally substituted $C_{5-15}$ aryl), optionally substituted heterocyclyl (e.g., optionally substituted 4- to 30-membered heterocyclyl, optionally substituted 4- to 20-membered heterocyclyl, or optionally substituted 4- to 15-membered heterocyclyl) or optionally substituted heteroaryl (e.g., optionally substituted 5- to 30-membered heteroaryl, optionally substituted 5- to 20-membered heteroaryl, or optionally substituted 5- to 15-membered heteroaryl); or wherein $R_3$ and $R_4$, together with the nitrogen atom to which they are attached, form an optionally substituted nitrogen-containing heterocyclyl (e.g., optionally substituted 4- to 30-membered nitrogen-containing heterocyclyl, optionally substituted 4-to 20-membered nitrogen-containing heterocyclyl, or optionally substituted 4- to 15-membered nitrogen-containing heterocyclyl). In some embodiments, each linear or branched alkyl is independently optionally substituted with a substituent selected from the group consisting of deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl or $C_{2-6}$ alkenyl. In some embodiments, the cycloalkylene, arylene, heterocyclylene, heteroarylene, cycloalkyl, aryl, heterocyclyl, heteroaryl, and nitrogen-containing heterocyclyl are each independently optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl.

In some embodiments, $R_1$, $R_3$, $R_4$, $R_5$, $R_6$, $R_7$, $R_9$, $R_{10}$, $R_{11}$, and $R_{12}$ each independently represent: H;

linear or branched chain $C_{1-10}$ alkyl (e.g., linear or branched $C_{1-6}$ alkyl, such as methyl, ethyl, propyl, isopropyl, butyl, isobutyl, sec-butyl, tert-butyl, pentyl, isopentyl, neopentyl, tert-pentyl, and hexyl), optionally substituted with one or more (e.g., 1-20, 1-15, 1-10, 1-6, 1-4, 2-6, or 1) substituents selected from the group consisting of: deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl or $C_{2-6}$ alkenyl;

cyclopropyl, cyclobutyl, cyclopentyl, cyclopentenyl, cyclohexyl, cyclohexenyl, cycloheptyl, cyclooctyl, decalinyl,

octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornyl, bicyclo[2.2.1]heptanyl, 2-oxobicyclo[2.2.1]heptanyl, or bicyclo[2.2.1]heptenyl), each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl or $C_{2-6}$ alkynyl, and any combination thereof;

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydro-pyridinyl, dihydroxy-piperidinyl, difluoro-piperidinyl, morpholinyl, thiomorpholinyl,azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

phenyl or naphthyl, each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_2$, $R_8$, and $R_{13}$ each independently represent:

linear or branched $C_{1-10}$ alkyl, optionally substituted with one or more (e.g., 1-20, 1-15, 1-10, 1-6, 1-4, 2-6, or 1) substituents selected from the group consisting of: deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl or $C_{2-6}$ alkenyl;

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornyl, bicyclo[2.2.1]heptanyl, 2-oxobicyclo[2.2.1]heptanyl, or bicyclo[2.2.1]heptenyl), each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

azetidinyl, oxetanyl, pyrrolidinyl, imidazolidinyl, pyrazolidyl, tetrahydrofuranyl, tetrahydropyranyl, tetrahydrothienyl,

tetrahydrothiopyranyl, oxazolidinyl, thiazolidinyl, piperidinyl, piperazinyl, tetrahydro-pyridinyl, dihydroxy-piperidinyl, difluoro-piperidinyl, morpholinyl, thiomorpholinyl,azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

phenyl or naphthyl, each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, X represents $C(O)NR_3R_4$, wherein $R_3$ and $R_4$, together with the nitrogen atom to which they are attached, form an optionally substituted 4- to 30-membered nitrogen-containing heterocyclyl (e.g., 4- to 20-membered nitrogen-containing heterocyclyl, or 4- to 15-membered nitrogen-containing heterocyclyl). In some embodiments, examples of the 4- to 30-membered nitrogen-containing heterocyclyl include, but are not limited to, azetidinyl, pyrrolidinyl, imidazolidinyl, pyrazolidyl, oxazolidinyl, thiazolidinyl, piperidinyl, piperazinyl, tetrahydro-pyridinyl, dihydroxy-piperidinyl, difluoro-piperidinyl, morpholinyl, thiomorpholinyl,azacycloheptyl, azacyclooctyl, diazacycloheptanyl, diazacyclooctyl, nitrogen-containing bridged heterocyclyl (e.g., 6- to 20-membered nitrogen-containing bridged heterocyclyl, such as 3-azabicyclo[3.1.0]hexanyl, 3-azabicyclo[3.1.1]heptanyl, 2-azabicyclo[2.2.1]heptanyl, 6-azabicyclo[3.1.1]heptanyl, 2-azabicyclo[2.2.2]octanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 2,5-diazabicyclo[2.2.2]octanyl, and quinuclidinyl), or azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl. In some embodiments, the 4- to 30-membered nitrogen-containing heterocyclyl is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, X represents:

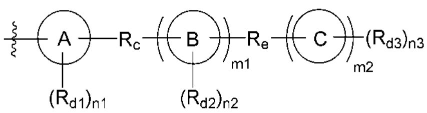

wherein ring A represents heterocyclylene, cycloalkylene, arylene, or heteroarylene, and $(R_{d1})_{n1}$ indicates that ring A

is optionally substituted with n1 $R_{d1}$ substituents, wherein each $R_{d1}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n1 represents an integer of 0-20;

$R_c$ represents $NR_{c1}$, C(O), $CR_{c2}R_{c3}$, or a bond, wherein $R_{c1}$ represents H, optionally substituted linear or branched alkyl, optionally substituted cycloalkyl, optionally substituted aryl, optionally substituted heterocyclyl, or optionally substituted heteroaryl, wherein $R_{c2}$ and $R_{c3}$ each independently represent H, halogen, optionally substituted linear or branched alkyl, optionally substituted cycloalkyl, optionally substituted aryl, optionally substituted heterocyclyl, or optionally substituted heteroaryl;

each ring B is the same or different and independently represents heterocyclylene, cycloalkylene, arylene, or heteroarylene, m1 represents an integer of 0, 1, 2, or 3, and $(R_{d2})_{n2}$ indicates that each ring B is independently optionally substituted with n2 $R_{d2}$ substituents, wherein each $R_{d2}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n2 represents an integer of 0-20;

$R_c$ represents S, O, $CR_{c1}R_{c2}$, or a bond, wherein $R_{c1}$ and $R_{c2}$ each independently represent H, halogen, optionally substituted linear or branched alkyl, optionally substituted cycloalkyl, optionally substituted aryl, optionally substituted heterocyclyl, or optionally substituted heteroaryl; and

each ring C is the same or different and independently represents heterocyclyl, cycloalkyl, aryl, or heteroaryl, m2 represents an integer of 0, 1, 2, or 3, and $(R_{d3})_{n3}$ indicates that each ring C is independently optionally substituted with n3 $R_{d3}$ substituents, wherein each $R_{d3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n3 represents an integer of 0-20.

**In** some embodiments, ring A represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene) or 5- to 30-membered heteroarylene (e.g., 5- to 20-membered heteroarylene, or 5- to 15-membered heteroarylene). In some embodiments, ring A is optionally substituted with n1 $R_{d1}$ substituents, wherein each $R_{d1}$ independently represents deuterium, $C_{1-6}$ alkyl (e.g., $C_{1-4}$ alkyl, or $C_{1-3}$ alkyl, such as methyl, ethyl, propyl, isopropyl, butyl, sec-butyl, or tert-butyl,), deuterated $C_{1-6}$ alkyl, halogenated $C_{1-6}$ alkyl (e.g., halogenated $C_{1-4}$ alkyl, or halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), $C_{1-6}$ alkoxy (e.g., $C_{1-4}$ alkoxy, or $C_{1-3}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy, or halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-), halogen (e.g., fluorine, chlorine, bromine, or iodine), amino, hydroxy, mercapto, cyano, oxo, $C_{2-6}$ alkynyl (e.g., ethynyl) or $C_{2-6}$ alkenyl (e.g., vinyl), and n1 represents an integer of 0, 1, 2, 3, 4, 5, 6, 7, 8, 9, 10, 11, 12, 13, 14, 15, 16, 17, 18, 19, or 20.

In some embodiments, each ring B is the same or different and independently represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene) or 5- to 30-membered heteroarylene (e.g., 5- to 20-membered heteroarylene, or 5- to 15-membered heteroarylene). In some embodiments, m1 represents an integer of 0, 1, 2, or 3. In some embodiments, each ring B is optionally substituted with n2 $R_{d2}$ substituents, wherein each $R_{d2}$ independently represents deuterium, $C_{1-6}$ alkyl (e.g., $C_{1-4}$ alkyl, or $C_{1-3}$ alkyl, such as methyl, ethyl, propyl, isopropyl, butyl, sec-butyl, or tert-butyl,), deuterated $C_{1-6}$ alkyl, halogenated $C_{1-6}$ alkyl (e.g., halogenated $C_{1-4}$ alkyl, or halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), $C_{1-6}$ alkoxy (e.g., $C_{1-4}$ alkoxy, or $C_{1-3}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy, or halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-), halogen (e.g., fluorine, chlorine, bromine, or iodine), amino, hydroxy, mercapto, cyano, oxo, $C_{2-6}$ alkynyl (e.g., ethynyl) or $C_{2-6}$ alkenyl (e.g., vinyl), and n2 represents an integer of 0, 1, 2, 3, 4, 5, 6, 7, 8, 9, 10, 11, 12, 13, 14, 15, 16, 17, 18, 19, or 20.

In some embodiments, ring A and ring B each independently represent $C_{3-30}$ cycloalkylene (e.g., $C_{3-20}$ cycloalkylene, or $C_{3-15}$ cycloalkylene). In some embodiments, examples of the $C_{3-30}$ cycloalkylene include, but are not limited to, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_5$-$C_{20}$spiro-cycloalkylene, such as spiro[3.3]heptylene, spiro[2.5]octylene, spiro[3.5]nonylene, spiro[4.4]nonylene, spiro[4.5]decylene, or spiro[5.5]undecylene), p-menthanylene, m-menthanylene, or bridged cycloalkylene (e.g., $C_6$-$C_{20}$ bridged cycloalkylene, such as adamantanylene, noradamantanylene, bornylene, norbornylene, bicyclo[2.2.1]heptanylene, 2-oxobicyclo[2.2.1]heptanylene, or bicyclo[2.2.1]heptenylene). In some embodiments, the $C_{3-30}$ cycloalkylene is optionally substituted with one or more (e.g., 1, 2, 3, 4, 5, 6, 7, 8, 9, 10, 11, 12, 13, 14, 15, 16, 17, 18, 19, or 20)

substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, ring A and ring B each independently represent 4- to 30-membered heterocyclylene (e.g., 4- to 20-membered heterocyclylene, or 4- to 15-membered heterocyclylene). In some embodiments, examples of the 4- to 30-membered heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, tetrahydro-pyridinylene, dihydroxy-piperidinylene, difluoro-piperidinylene, morpholinylene, thiomorpholinylene, azacycloheptanylene, azacyclooctylene, dioxacyclohexylene, azacycloheptanylene, azacyclooctylene, diazacycloheptanylene (e.g., 1,4-diazacycloheptanylene, 4,5-diazacycloheptanylene, 1,3-diazacycloheptanylene), diazacyclooctylene, bridged heterocyclylene (e.g., 6-to 20-membered bridged heterocyclylene, such as 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and quinuclidinylene), azaspirocycloalkylene (e.g., 5- to 20-membered azaspirocycloalkylene, such as 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene, and 7-azaspiro[3.5]nonanylene), or octahydropyrrolo[3,4-c]pyrrolylene. In some embodiments, the 4- to 30-membered heterocyclylene is optionally substituted with one or more (e.g., 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, ring A and ring B each independently represent $C_{5-30}$ arylene (e.g., $C_{5-20}$ arylene, or $C_{5-15}$ arylene). In some embodiments, examples of the $C_{5-30}$ arylene include, but are not limited to, phenylene or naphthylene. In some embodiments, the $C_{5-30}$ arylene is optionally substituted with one or more (e.g., 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, ring A and ring B each independently represent 5- to 30-membered heteroarylene (e.g., 5- to 20-membered heteroarylene, or 5- to 15-membered heteroarylene). In some embodiments, examples of the 5- to 30-membered heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, triazinylene, indolylene, isoindolylene, isoindolinylene, benzofuranylene, chromanylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, 2-oxo-2,3-dihydro-1H-benzo[d]imidazolylene, benzo[b][1,4]oxazinylene, 3,4-dihydro-2H-benzo[b][1,4]oxazinylene, quinolinylene, isoquinolinylene, 1,2,3,4-tetrahydroquinolinylene, naphthyridinylene, cinnolinylene, quinazolinylene, quinoxalinylene, 1,2,3,4-tetrahydroquinoxalinylene, phthalazinylene, 5,6,7,8-tetrahydro-[1,2,4]triazolo[4,3-a]pyrazinylene, 4,5,6,7-tetrahydrothieno[3,2-c]pyridinylene, 5-oxo-6,7-dihydrothieno[3,2-d]pyrimidinylene, thieno[2,3-d]pyrimidinylene, thieno[3,2-d]pyrimidinylene, isoxazolo[4,5-c]pyridinylene, isoxazolo[4,5-c]pyrimidinylene, isoxazolo[4,5-d]pyrimidinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, 5,6,7,8-tetrahydrobenzo[4,5]thieno[2,3-d]pyrimidinylene, or 6,7-dihydro-5H-cyclopenta[4,5]thieno[2,3-d]pyrimidinylene. In some embodiments, 5- to 30-membered heteroarylene is optionally substituted with one or more (e.g., 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, each ring C is the same or different and independently represents 4- to 30-membered heterocyclyl (e.g., 4- to 20-membered heterocyclyl, or 4- to 15-membered heterocyclyl), $C_{3-30}$ cycloalkyl (e.g., $C_{3-20}$ cycloalkyl, or $C_{3-15}$ cycloalkyl), $C_{5-30}$ aryl (e.g., $C_{5-20}$ aryl, or $C_{5-15}$ aryl) or 5- to 30-membered heteroaryl (e.g., 5- to 20-membered heteroaryl, or 5- to 15-membered heteroaryl). In some embodiments, m2 represents an integer of 0, 1, 2, or 3. In some embodiments, $(R_{d3})_{n3}$ indicates that each ring C is independently optionally substituted with n3 $R_{d3}$ substituents, wherein each $R_{d3}$ independently represents deuterium, $C_{1-6}$ alkyl (e.g., $C_{1-4}$ alkyl, or $C_{1-3}$ alkyl, such as methyl, ethyl, propyl, isopropyl, butyl, sec-butyl, or tert-butyl,), deuterated $C_{1-6}$ alkyl, halogenated $C_{1-6}$ alkyl (e.g., halogenated $C_{1-4}$ alkyl, or halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, or $CH_2ClCH_2$-), $C_{1-6}$ alkoxy (e.g., $C_{1-4}$ alkoxy, or $C_{1-3}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy, or halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-), halogen (e.g., fluorine, chlorine, bromine, or iodine), amino, hydroxy, mercapto, cyano,

oxo, $C_{2-6}$ alkynyl (e.g., ethynyl) or $C_{2-6}$ alkenyl (e.g., vinyl), and n3 represents an integer of 0, 1, 2, 3, 4, 5, 6, 7, 8, 9, 10, 11, 12, 13, 14, 15, 16, 17, 18, 19, or 20.

In some embodiments, each ring C is the same or different and independently represents $C_{3-30}$ cycloalkyl (e.g., $C_{3-20}$ cycloalkyl, or $C_{3-15}$ cycloalkyl). In some embodiments, examples of the $C_{3-30}$ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornyl, bicyclo[2.2.1]heptanyl, 2-oxobicyclo[2.2.1]heptanyl, or bicyclo[2.2.1]heptenyl). In some embodiments, the $C_{3-30}$ cycloalkyl is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, each ring C is the same or different and independently represents 4- to 30-membered heterocyclyl (e.g., 4- to 20-membered heterocyclyl, or 4- to 15-membered heterocyclyl). In some embodiments, examples of the 4- to 30-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydro-pyridinyl, dihydroxy-piperidinyl, difluoro-piperidinyl, morpholinyl, thiomorpholinyl,azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl. In some embodiments, 4- to 30-membered heterocyclyl is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, each ring C is the same or different and independently represents $C_{5-30}$ aryl (e.g., $C_{5-20}$ aryl, or $C_{5-15}$ aryl). In some embodiments, examples of the $C_{5-30}$ aryl include, but are not limited to, phenyl or naphthyl. In some embodiments, the $C_{5-30}$ aryl is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, each ring C is the same or different and independently represents 5- to 30-membered heteroaryl (e.g., 5- to 20-membered heteroaryl, or 5- to 15-membered heteroaryl). In some embodiments, examples of the 5- to 30-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl. In some embodiments, 5- to 30-membered heteroaryl is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_c$ represents C(O) or a bond.

In some embodiments, $R_c$ represents $NR_{c1}$, wherein $R_{c1}$ represents H, optionally substituted linear or branched $C_{1-10}$ alkyl (e.g., optionally substituted linear or branched $C_{1-6}$ alkyl), optionally substituted $C_{3-30}$ cycloalkyl (e.g., optionally substituted $C_{3-20}$ cycloalkyl, or optionally substituted $C_{3-15}$ cycloalkyl), optionally substituted $C_{5-30}$ aryl (e.g., optionally substituted $C_{5-20}$ aryl, or optionally substituted $C_{5-15}$ aryl), optionally substituted 4- to 30-membered heterocyclyl (e.g., optionally substituted 4- to 20-membered heterocyclyl, or optionally substituted 4- to 15-membered heterocyclyl) or

optionally substituted 5- to 30-membered heteroaryl (e.g., optionally substituted 5- to 20-membered heteroaryl, or optionally substituted 5- to 15-membered heteroaryl).

In some embodiments, $R_c$ represents $NR_{c1}$, wherein $R_{c1}$ represents H, or linear or branched $C_{1-10}$ alkyl (e.g., linear or branched $C_{1-6}$ alkyl). In some embodiments, examples of the linear or branched $C_{1-10}$ alkyl include, but are not limited to, methyl, ethyl, propyl, isopropyl, butyl, isobutyl, sec-butyl, tert-butyl, pentyl, isopentyl, neopentyl, tert-pentyl, and hexyl. In some embodiments, the linear or branched $C_{1-10}$ alkyl is optionally substituted with one or more (e.g., 1-20, 1-15, 1-10, 1-6, 1-4, 2-6, or 1) substituents selected from the group consisting of: deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl or $C_{2-6}$ alkenyl.

In some embodiments, $R_c$ represents $NR_{c1}$, wherein $R_{c1}$ represents optionally substituted $C_{3-30}$ cycloalkyl (e.g., optionally substituted $C_{3-20}$ cycloalkyl, or optionally substituted $C_{3-15}$ cycloalkyl). In some embodiments, examples of the $C_{3-30}$ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spirocycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornyl, bicyclo[2.2.1]heptanyl, 2-oxobicyclo[2.2.1]heptanyl, or bicyclo[2.2.1]heptenyl). In some embodiments, the $C_{3-30}$ cycloalkyl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_c$ represents $NR_{c1}$, wherein $R_{c1}$ represents optionally substituted 4- to 30-membered heterocyclyl (e.g., optionally substituted 4- to 20-membered heterocyclyl, or optionally substituted 4- to 15-membered heterocyclyl). In some embodiments, examples of the 4- to 30-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydro-pyridinyl, dihydroxy-piperidinyl, difluoropiperidinyl, morpholinyl, thiomorpholinyl,azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl. In some embodiments, the 4- to 30-membered heterocyclyl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_c$ represents $NR_{c1}$, wherein $R_{c1}$ represents optionally substituted $C_{5-30}$ aryl (e.g., optionally substituted $C_{5-20}$ aryl, or optionally substituted $C_{5-15}$ aryl). In some embodiments, examples of the $C_{5-30}$ aryl include, but are not limited to, phenyl or naphthyl. In some embodiments, the $C_{5-30}$ aryl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_c$ represents $NR_{c1}$, wherein $R_{c1}$ represents optionally substituted 5- to 30-membered heteroaryl (e.g., optionally substituted 5- to 20-membered heteroaryl, or optionally substituted 5- to 15-membered heteroaryl). In some embodiments, examples of the 5- to 30-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl. In some embodiments, the 5- to 30-membered

heteroaryl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_c$ represents $CR_{c2}R_{c3}$, wherein $R_{c2}$ and $R_{c3}$ each independently represent H, halogen, optionally substituted linear or branched $C_{1-10}$ alkyl (e.g., optionally substituted linear or branched $C_{1-6}$ alkyl), optionally substituted $C_{3-30}$ cycloalkyl (e.g., optionally substituted $C_{3-20}$ cycloalkyl, or optionally substituted $C_{3-15}$ cycloalkyl), optionally substituted $C_{5-30}$ aryl (e.g., optionally substituted $C_{5-20}$ aryl, or optionally substituted $C_{5-15}$ aryl), optionally substituted 4- to 30-membered heterocyclyl (e.g., optionally substituted 4- to 20-membered heterocyclyl, or optionally substituted 4- to 15-membered heterocyclyl) or optionally substituted 5- to 30-membered heteroaryl (e.g., optionally substituted 5- to 20-membered heteroaryl, or optionally substituted 5- to 15-membered heteroaryl). In some embodiments, examples of the linear or branched $C_{1-10}$ alkyl include, but are not limited to, methyl, ethyl, propyl, isopropyl, butyl, isobutyl, sec-butyl, tert-butyl, pentyl, isopentyl, neopentyl, tert-pentyl, and hexyl. In some embodiments, the linear or branched $C_{1-10}$ alkyl is optionally substituted with one or more (e.g., 1-20, 1-15, 1-10, 1-6, 1-4, 2-6, or 1) substituents selected from the group consisting of: deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl or $C_{2-6}$ alkenyl. In some embodiments, examples of the $C_{3-30}$ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornyl, bicyclo[2.2.1]heptanyl, 2-oxobicyclo[2.2.1]heptanyl, or bicyclo[2.2.1]heptenyl). In some embodiments, the $C_{3-30}$ cycloalkyl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof. In some embodiments, examples of the 4- to 30-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydro-pyridinyl, dihydroxy-piperidinyl, difluoro-piperidinyl, morpholinyl, thiomorpholinyl,azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl. In some embodiments, the 4- to 30-membered heterocyclyl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof. In some embodiments, examples of the $C_{5-30}$ aryl include, but are not limited to, phenyl or naphthyl. In some embodiments, the $C_{5-30}$ aryl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof. In some embodiments, examples of the 5- to 30-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl. In some embodiments, the 5- to 30-membered heteroaryl is optionally substituted with one or more (e.g., 1-20, 1-15, 1-10, 1-6, 1-4, 2-6, or 1) substituents selected from the group consisting of:

deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_c$ represents S, O, or a bond.

In some embodiments, $R_c$ represents $CR_{c1}R_{c2}$, wherein $R_{c1}$ and $R_{c2}$ each independently represent H, halogen, optionally substituted linear or branched $C_{1-10}$ alkyl (e.g., optionally substituted linear or branched $C_{1-6}$ alkyl), optionally substituted $C_{3-30}$ cycloalkyl (e.g., optionally substituted $C_{3-20}$ cycloalkyl, or optionally substituted $C_{3-15}$ cycloalkyl), optionally substituted $C_{5-30}$ aryl (e.g., optionally substituted $C_{5-20}$ aryl, or optionally substituted $C_{5-15}$ aryl), optionally substituted 4- to 30-membered heterocyclyl (e.g., optionally substituted 4- to 20-membered heterocyclyl, or optionally substituted 4- to 15-membered heterocyclyl) or optionally substituted 5- to 30-membered heteroaryl (e.g., optionally substituted 5- to 20-membered heteroaryl, or optionally substituted 5- to 15-membered heteroaryl). In some embodiments, examples of the linear or branched $C_{1-10}$ alkyl include, but are not limited to, methyl, ethyl, propyl, isopropyl, butyl, isobutyl, sec-butyl, tert-butyl, pentyl, isopentyl, neopentyl, tert-pentyl, and hexyl. In some embodiments, the linear or branched $C_{1-10}$ alkyl is optionally substituted with one or more (e.g., 1-20, 1-15, 1-10, 1-6, 1-4, 2-6, or 1) substituents selected from the group consisting of: deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl or $C_{2-6}$ alkenyl. In some embodiments, examples of the $C_{3-30}$ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornyl, bicyclo[2.2.1]heptanyl, 2-oxobicyclo[2.2.1]heptanyl, or bicyclo[2.2.1]heptenyl). In some embodiments, the $C_{3-30}$ cycloalkyl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof. In some embodiments, examples of the 4- to 30-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydro-pyridinyl, dihydroxy-piperidinyl, difluoro-piperidinyl, morpholinyl, thiomorpholinyl,azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl. In some embodiments, the 4- to 30-membered heterocyclyl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof. In some embodiments, examples of the $C_{5-30}$ aryl include, but are not limited to, phenyl or naphthyl. In some embodiments, the $C_{5-30}$ aryl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof. In some embodiments, examples of the5- to 30-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl. In some embodiments, 5- to 30-membered heteroaryl is optionally substituted with one or more (e.g., 1-20, 1-15, 1-10, 1-6, 1-4, 2-6, or 1) substituents selected from the group consisting

of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, X represents:

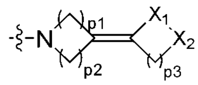

wherein $-X_1-X_2-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$,$-CH_2NH(CH_2)_{1-4}-$, or $-(CH_2)_{1-4}NHCH_2-$;

p1, p2, and p3 each independently represent an integer of 1, 2, 3, or 4; and

the ring containing $-X_1-X_2-$ is optionally substituted with one or more (e.g., 1-20, 1-15, 1-10, 1-6, 1-4, 2-6, or 1) substituents selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, $C_{2-6}$ alkenyl, or any combination thereof.

In some embodiments, p1, p2, and p3 each independently represent an integer of 1, 2, or 3.

In some embodiments, X represents:

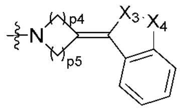

wherein -X3-X4- represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;

p4 and p5 each independently represent an integer of 1, 2, 3, or 4; and

the benzo-fused ring containing$-X_3-X_4-$ is optionally substituted with one or more (e.g., 1-20, 1-15, 1-10, 1-6, 1-4, 2-6, or 1) substituents selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, $C_{2-6}$ alkenyl, or, or any combination thereof.

In some embodiments, p4 and p5 each independently represent an integer of 1, 2, or 3.

In some embodiments, X represents:

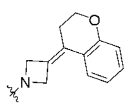 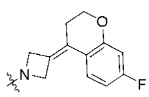 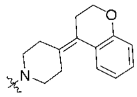 或 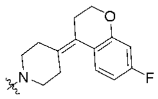 .

In some embodiments, examples of the X include, but are not limited to

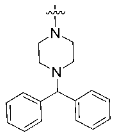 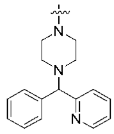 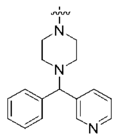 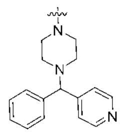 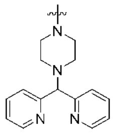 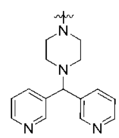 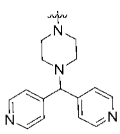 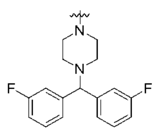 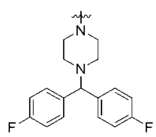

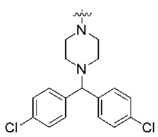 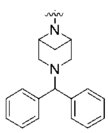 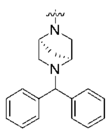 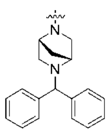 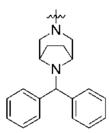 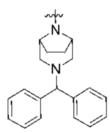 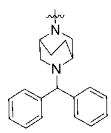 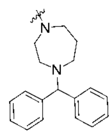 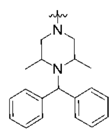 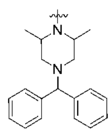

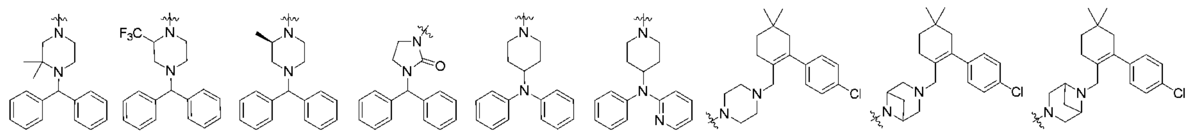

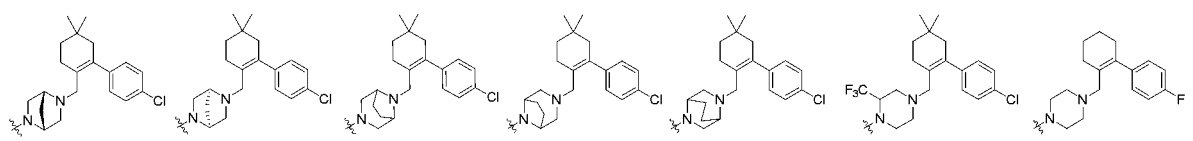

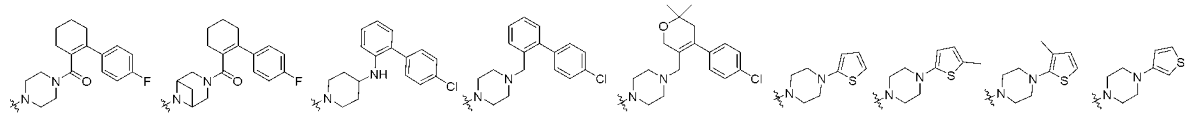

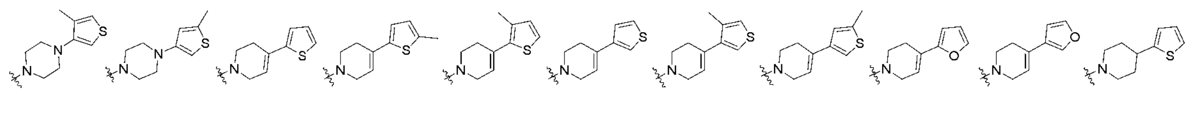

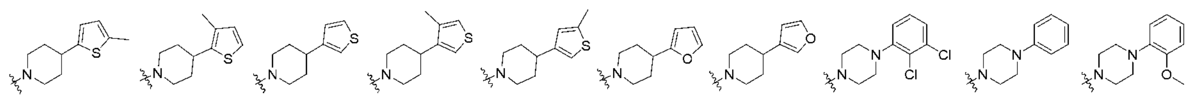

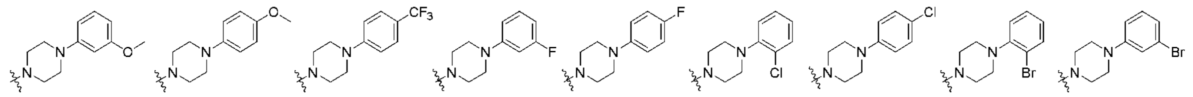

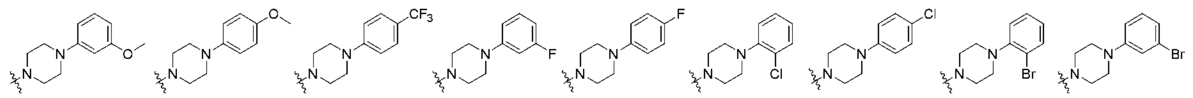

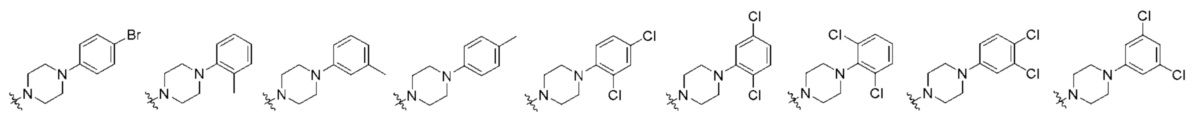

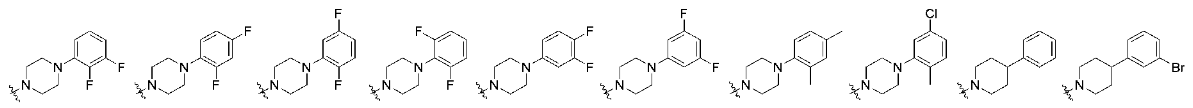

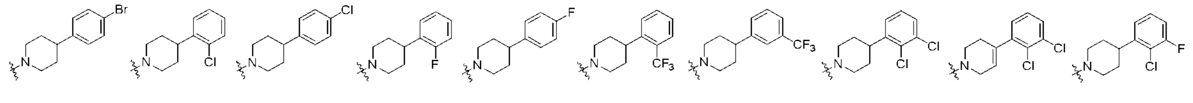

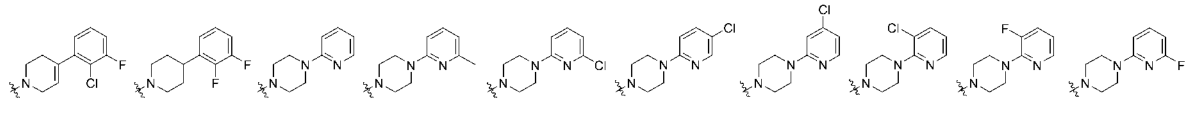

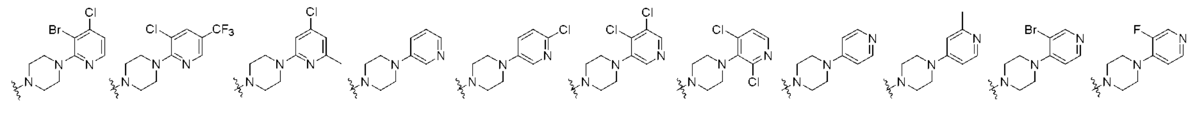

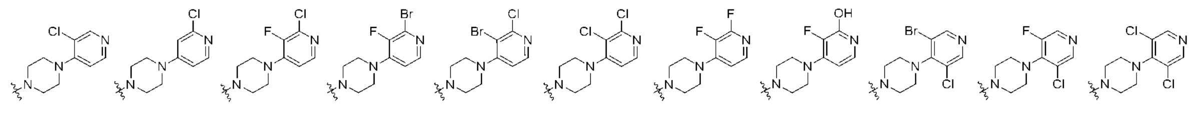

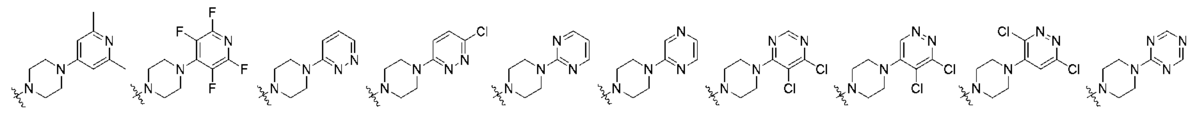

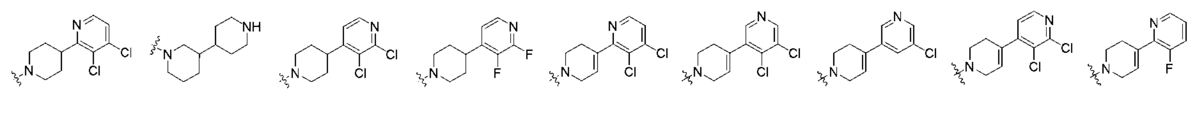

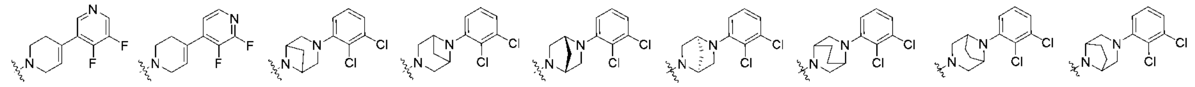

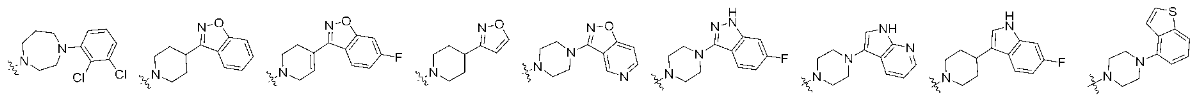

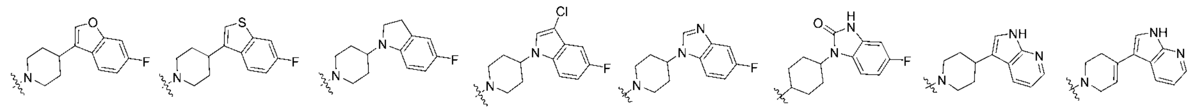

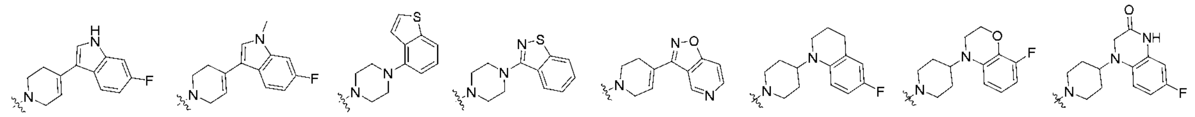

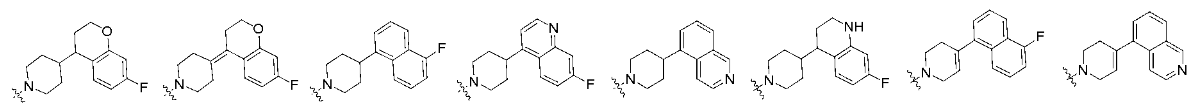

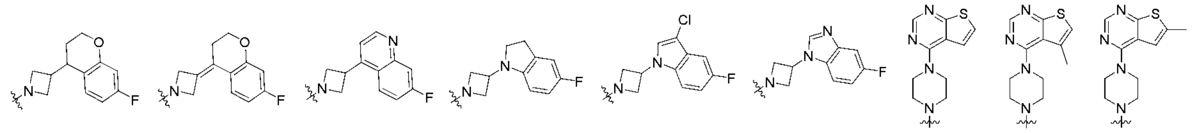

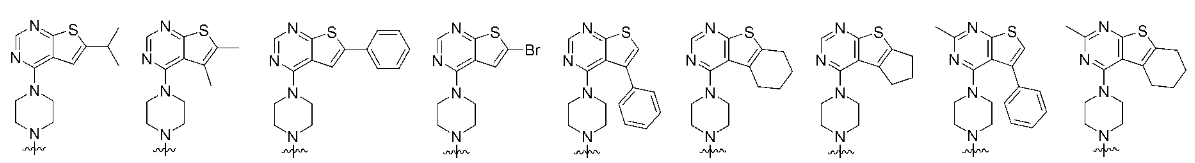

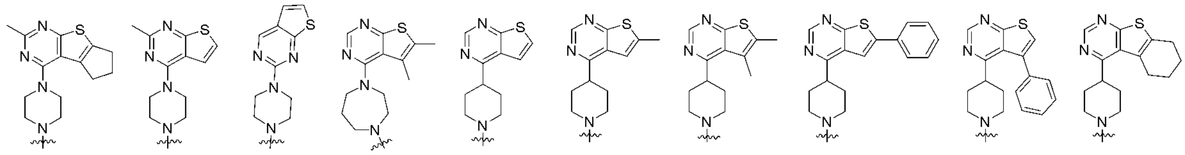

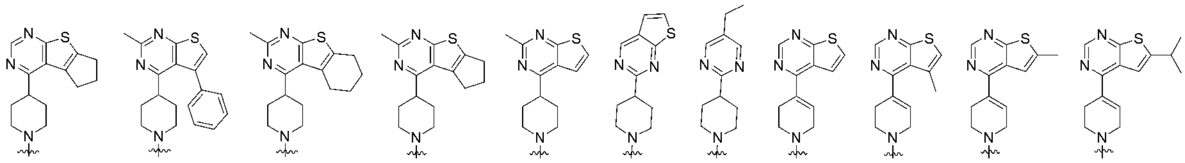

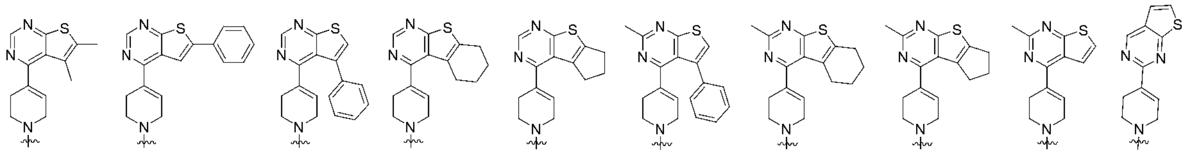

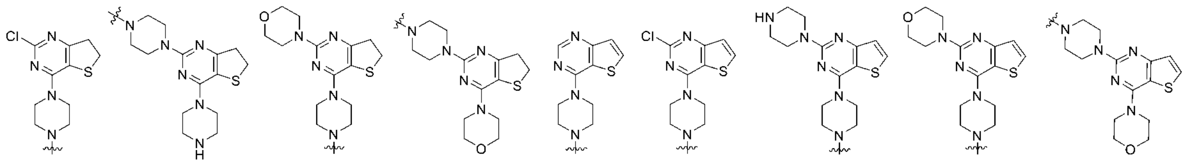

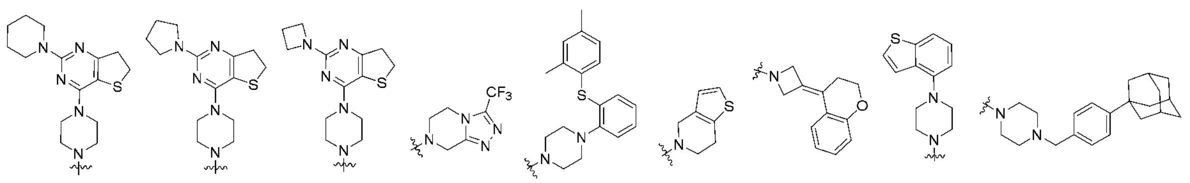

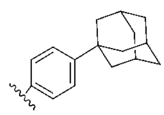 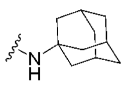 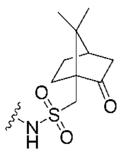 or 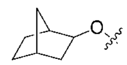.

In some embodiments of the present disclosure, the compound of Formula (I) is also represented by Formula (I-1), Formula (I-2), Formula (I-3), or Formula (I-4):

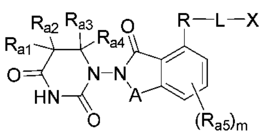
Formula (I-1)

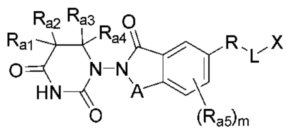
Formula (I-2)

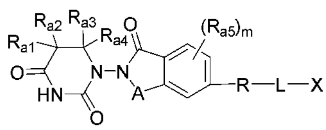
Formula (I-3)

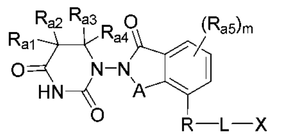
Formula (I-4)

wherein $R_{a1}$, $R_{a2}$, $R_{a3}$, $R_{a4}$, A, $(R_{a5})_m$, R, L, and X are as defined in the compound of Formula (I) and its various embodiments in the present disclosure.

Preferably the compounds of the present invention and their salts (especially pharmaceutically acceptable salts, such as hydrochloride, etc.), enantiomers, diastereomers, solvates, or polymorphs thereof in Table 1 below are provided:

Table 1. The compounds of the present invention

| Compound No. | Structure of the compounds | The compounds' name | Chinese translation of the compounds' name |
|---|---|---|---|
| GT-04889 | 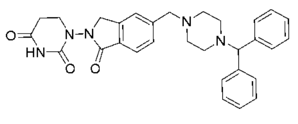 | 1-(5-((4-benzhydrylpipera-zin-1-yl)methyl)-1-oxoisoindo-lin-2-yl)dihydropyrimidi-ne-2,4(1H,3H)-dione | 1-(5-((4-benzhydrylpiperazin-1-yl)methyl)-1-oxoisoindo-lin-2-yl)dihydropyrimidi-ne-2,4(1H,3H)-dione |
| GT-0489 0 | 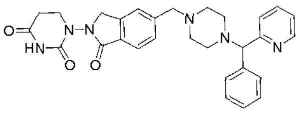 | 1-(1-oxo-5-((4-(phenyl(pyridin-2-yl)methyl)piperazin-1-yl) methyl)isoindolin-2-yl)dihy-dropyrimidi-ne-2,4(1H,3H)-dione | 1-(1-oxo-5-((4-(phenyl(pyridin-2-yl)methyl)piperazin-1-yl) methyl)isoindolin-2-yl)dihydropyrimidine-2,4(1H,3H)-dione |
| GT-0637 6 | 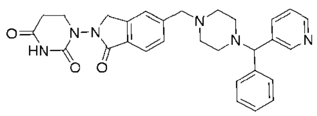 | 1-(1-oxo-5-((4-(phenyl(pyridin-3-yl)methyl)piperazin-1-yl) methyl)isoindolin-2-yl)dihy-dropyrimidi-ne-2,4(1H,3H)-dione | 1-(1-oxo-5-((4-(phenyl(pyridin-3-yl)methyl)piperazin-1-yl) methyl)isoindolin-2-yl)dihydropyrimidine-2,4(1H,3H)-dione |
| GT-0489 1 | 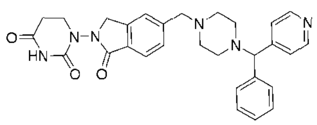 | 1-(1-oxo-5-((4-(phenyl(pyridin-4-yl)methyl)piperazin-1-yl) methyl)isoindolin-2-yl)dihy-dropyrimidi-ne-2,4(1H,3H)-dione | 1-(1-oxo-5-((4-(phenyl(pyridin-4-yl)methyl)piperazin-1-yl) methyl)isoindolin-2-yl)dihydropyrimidine-2,4(1H,3H)-dione |
| GT-0489 2 | 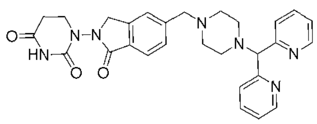 | 1-(5-((4-(di(pyridin-2-yl) methyl)piperazin-1-yl) methyl)-1-oxoisoindolin-2-yl) dihydropyrimidine-2,4(1H,3H)-dione | 1-(5-((4-(di(pyridin-2-yl) methyl)piperazin-1-yl) methyl)-1-oxoisoindolin-2-yl) dihydropyrimidine-2,4(1H,3H)-dione |
| GT-0489 3 | 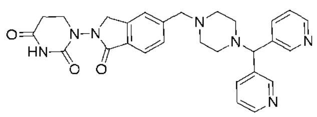 | 1-(5-((4-(di(pyridin-3-yl) methyl)piperazin-1-yl) methyl)-1-oxoisoindolin-2-yl) dihydropyrimidine-2,4(1H,3H)-dione | 1-(5-((4-(di(pyridin-3-yl) methyl)piperazin-1-yl) methyl)-1-oxoisoindolin-2-yl) dihydropyrimidine-2,4(1H,3H)-dione |
| | 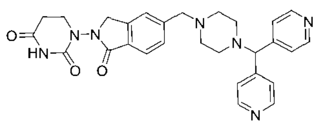 | 1-(5-((4-(di(pyridin-4-yl) methyl)piperazin-1-yl) methyl)-1-oxoisoindolin-2-yl) dihydropyrimidine-2,4(1H,3H)-dione | 1-(5-((4-(di(pyridin-4-yl) methyl)piperazin-1-yl) methyl)-1-oxoisoindolin-2-yl) dihydropyrimidine-2,4(1H,3H)-dione |
| GT-0729 3 | 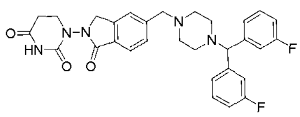 | 1-(5-((4-(bis(3-fluorophenyl) methyl)piperazi n-1-yl) methyl)-1-oxoisoindolin-2-yl) dihydropyrimidine-2,4(1H,3H)-dione | 1-(5-((4-(bis(3-fluorophenyl) methyl)piperazi n-1-yl) methyl)-1-oxoisoindolin-2-yl) dihydropyrimidine-2,4(1H,3H)-dione |
| GT-0489 4 | 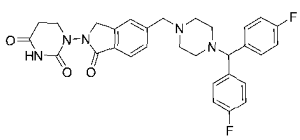 | 1-(5-((4-(bis(4-fluorophenyl) methyl)piperazi n-1-yl) methyl)-1-oxoisoindolin-2-yl) dihydropyrimidine-2,4(1H,3H)-dione | 1-(5-((4-(bis(4-fluorophenyl) methyl)piperazi n-1-yl) methyl)-1-oxoisoindolin-2-yl) dihydropyrimidine-2,4(1H,3H)-dione |
| GT-0489 5 | 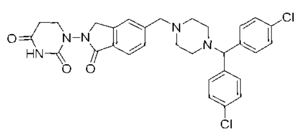 | 1-(5-((4-(bis(4-chlorophenyl) methyl)piperaz in-1-yl) methyl)-1-oxoisoindolin-2-yl) dihydropyrimidine-2,4(1H,3H)-dione | 1-(5-((4-(bis(4-chlorophenyl) methyl)piperaz in-1-yl) methyl)-1-oxoisoindolin-2-yl) dihydropyrimidine-2,4(1H,3H)-dione |

(continued)

| Compound No. | Structure of the compounds | The compounds' name | Chinese translation of the compounds' name |
|---|---|---|---|
| GT-0638 0 | 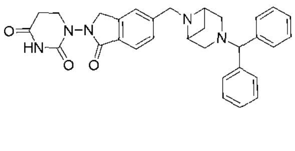 | 1-(5-((3-benzhydryl-3,6-diazabicyclo[3.1.1]heptan-6-yl) methyl)-1-oxoisoindolin-2-yl) dihydropyrimidine-2,4(1H,3H)-dione | 1-(5-((3-benzhydryl-3,6-diazabicyclo[3.1.1]heptan-6-yl) methyl)-1-oxoisoindolin-2-yl) dihydropyrimidine-2,4(1H,3H)-dione |
| GT-0493 8 | 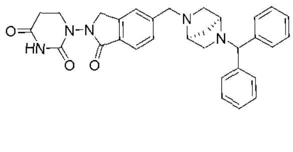 | 1-(5-(((1S,4S)-5-benzhydryl-2,5-diazabicyclo[2.2.1] heptan-2-yl)methyl)-1-oxoisoindolin-2-yl)dihydropyrimidine-2,4(1H,3H)-dione | 1-(5-(((1S,4S)-5-benzhydryl-2,5-diazabicyclo[2.2.1] heptan-2-yl)methyl)-1-oxoisoindolin-2-yl)dihydropyrimidine-2,4(1H,3H)-dione |
| GT-0638 2 | 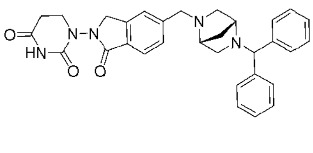 | 1-(5-(((1R,4R)-5-benzhydryl-2,5-diazabicyclo[2.2.1] heptan-2-yl)methyl)-1-oxoisoindolin-2-yl)dihydropyrimidine-2,4(1H,3H)-dione | 1-(5-(((1R,4R)-5-benzhydryl-2,5-diazabicyclo[2.2.1] heptan-2-yl)methyl)-1-oxoisoindolin-2-yl)dihydropyrimidine-2,4(1H,3H)-dione |
| GT-0645 2 | 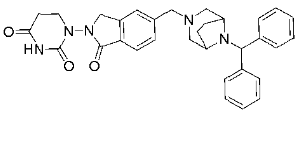 | 1-(5-((8-benzhydryl-3,8-diazabicyclo[3.2.1]octan-3-yl) methyl)-1-oxoisoindolin-2-yl) dihydropyrimidine-2,4(1H,3H)-dione | 1-(5-((8-benzhydryl-3,8-diazabicyclo[3.2.1]octan-3-yl) methyl)-1-oxoisoindolin-2-yl) dihydropyrimidine-2,4(1H,3H)-dione |
| GT-0645 4 | 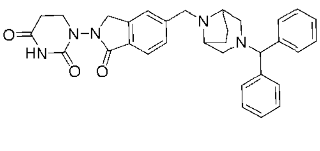 | 1-(5-((3-benzhydryl-3,8-diazabicyclo[3.2.1]octan-8-yl) methyl)-1-oxoisoindolin-2-yl) dihydropyrimidine-2,4(1H,3H)-dione | 1-(5-((3-benzhydryl-3,8-diazabicyclo[3.2.1]octan-8-yl) methyl)-1-oxoisoindolin-2-yl) dihydropyrimidine-2,4(1H,3H)-dione |
| GT-0645 0 | 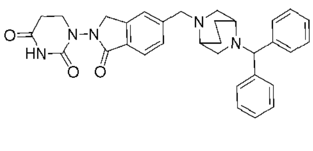 | 1-(5-((5-benzhydryl-2,5-diazabicyclo[2.2.2]octan-2-yl) methyl)-1-oxoisoindolin-2-yl) dihydropyrimidine-2,4(1H,3H)-dione | 1-(5-((5-benzhydryl-2,5-diazabicyclo[2.2.2]octan-2-yl) methyl)-1-oxoisoindolin-2-yl) dihydropyrimidine-2,4(1H,3H)-dione |
| GT-0729 4 | 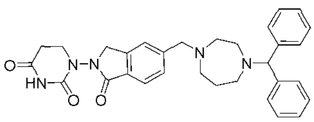 | 1-(5-((4-benzhydryl-1,4-diazepan-1-yl)methyl)-1-oxoisoindolin-2-yl)dihydropyrimidine-2,4(1H,3H)-dione | 1-(5-((4-benzhydryl-1,4-diazepan-1-yl)methyl)-1-oxoisoindolin-2-yl)dihydropyrimidine-2,4(1H,3H)-dione |
| GT-0643 1 | 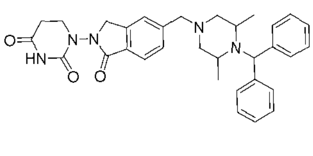 | 1-(5-((4-benzhydryl-3,5-dimethylpiperazin-1-yl) methyl)-1-oxoisoindolin-2-yl) dihydropyrimidine-2,4(1H,3H)-dione | 1-(5-((4-benzhydryl-3,5-dimethylpiperazin-1-yl) methyl)-1-oxoisoindolin-2-yl) dihydropyrimidine-2,4(1H,3H)-dione |
| GT-0743 3 | 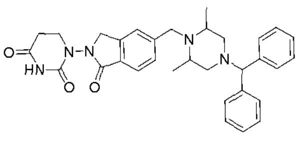 | 1-(5-((4-benzhydryl-2,6-dimethylpiperazin-1-yl) methyl)-1-oxoisoindolin-2-yl) dihydropyrimidine-2,4(1H,3H)-dione | 1-(5-((4-benzhydryl-2,6-dimethylpiperazin-1-yl) methyl)-1-oxoisoindolin-2-yl) dihydropyrimidine-2,4(1H,3H)-dione |
| GT-0638 4 | 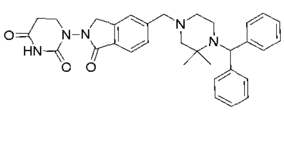 | 1-(5-((4-benzhydryl-3,3-dimethylpiperazin-1-yl) methyl)-1-oxoisoindolin-2-yl) dihydropyrimidine-2,4(1H,3H)-dione | 1-(5-((4-benzhydryl-3,3-dimethylpiperazin-1-yl) methyl)-1-oxoisoindolin-2-yl) dihydropyrimidine-2,4(1H,3H)-dione |

(continued)

| Compound No. | Structure of the compounds | The compounds' name | Chinese translation of the compounds' name |
|---|---|---|---|
| | 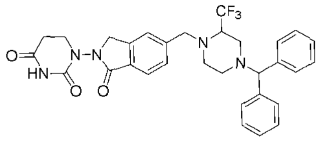 | 1-(5-((4-benzhydryl-2-(trifluoromethyl)piperazin-1-yl)methyl)-1-oxoisoindolin-2-yl)dihydropyrimidine-2,4(1H,3H)-dione | 1-(5-((4-benzhydryl-2-(trifluoromethyl)piperazin-1-yl)methyl)-1-oxoisoindolin-2-yl)dihydropyrimidine-2,4(1H,3H)-dione |
| GT-0644 8 | 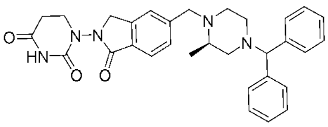 | (R)-1-(5-((4-benzhydryl-2-methylpiperazin-1-yl)methyl)-1-oxoisoindolin-2-yl)dihydropyrimidine-2,4(1H,3H)-dione | (R)-1-(5-((4-benzhydryl-2-methylpiperazin-1-yl)methyl)-1-oxoisoindolin-2-yl)dihydropyrimidine-2,4(1H,3H)-dione |
| | 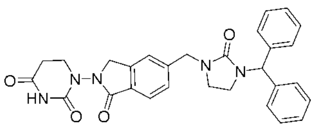 | 1-(5-((3-benzhydryl-2-oxoimidazolidin-1-yl)methyl)-1-oxoisoindolin-2-yl)dihydropyrimidine-2,4(1H,3H)-dione | 1-(5-((3-benzhydryl-2-oxoimidazolidin-1-yl)methyl)-1-oxoisoindolin-2-yl)dihydropyrimidine-2,4(1H,3H)-dione |
| GT-0493 9 | 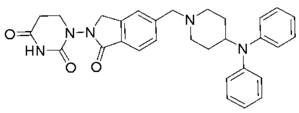 | 1-(5-((4-(diphenylamino)piperidin-1-yl)methyl)-1-oxoisoindolin-2-yl)dihydropyrimidine-2,4(1H,3H)-dione | 1-(5-((4-(diphenylamino)piperidin-1-yl)methyl)-1-oxoisoindolin-2-yl)dihydropyrimidine-2,4(1H,3H)-dione |
| GT-0494 0 | 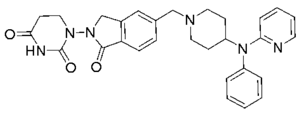 | 1-(1-oxo-5-((4-(phenyl(pyridin-2-yl)amino)piperidin-1-yl)methyl)isoindolin-2-yl)dihydropyrimidine-2,4(1H,3H)-dione | 1-(1-oxo-5-((4-(phenyl(pyridin-2-yl)amino)piperidin-1-yl)methyl)isoindolin-2-yl)dihydropyrimidine-2,4(1H,3H)-dione |
| GT-0494 4 | 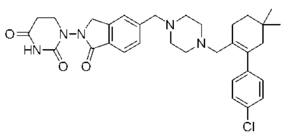 | 1-(5-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)dihydropyrimidine-2,4(1H,3H)-dione | 1-(5-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)dihydropyrimidine-2,4(1H,3H)-dione |
| GT-0645 6 | 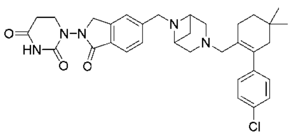 | 1-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-1-oxoisoindolin-2-yl)dihydropyrimidine-2,4(1H,3H)-dione | 1-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-1-oxoisoindolin-2-yl)dihydropyrimidine-2,4(1H,3H)-dione |
| GT-0645 8 | 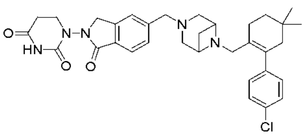 | 1-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)dihydropyrimidine-2,4(1H,3H)-dione | 1-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| | 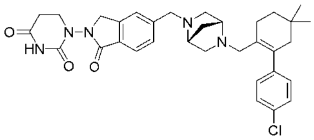 | 1-(5-(((1R,4R)-5-((4'-chloro-5,5-dimethyl-3,4,5,6-tetrahydro-[1,1'-biphenyl]-2-yl)methyl)-2,5-diazabicyclo [2.2.1]heptan-2-yl)methyl)-1-oxoisoindolin-2-yl)dihydropyrimidine-2,4(1H,3H)-dione | 1-(5-(((1R,4R)-5-((4'-chloro-5,5-dimethyl-3,4,5,6-tetrahydro-[1,1'-biphenyl]-2-yl) methyl)-2,5-diazabicyclo [2.2.1]heptan-2-yl)methyl)-1-oxoisoindolin-2-yl)dihydropyrimidine-2,4(1H,3H)-dione |
| | 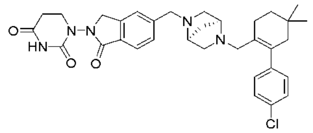 | 1-(5-(((1S,4S)-5-((4'-chloro-5,5-dimethyl-3,4,5,6-tetrahydro-[1,1'-biphenyl]-2-yl)methyl)-2,5-diazabicyclo [2.2.1]heptan-2-yl)methyl)-1-oxoisoindolin-2-yl)dihydropyrimidine-2,4(1H,3H)-dione | 1-(5-(((1S,4S)-5-((4'-chloro-5,5-dimethyl-3,4,5,6-tetrahydro-[1,1'-biphenyl]-2-yl) methyl)-2,5-diazabicyclo [2.2.1]heptan-2-yl)methyl)-1-oxoisoindolin-2-yl)dihydropyrimidine-2,4(1H,3H)-dione |
| GT-0732 9 | 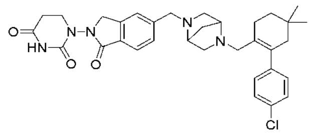 | 1-(5-((5-((4'-chloro-5,5-dimethyl-3,4,5,6-tetrahydro-[1,1'-biphenyl]-2-yl) methyl)-2,5-diazabicyclo [2.2.1]heptan-2-yl)methyl)-1-oxoisoindolin-2-yl)dihydropyrimidine-2,4(1H,3H)-dione | 1-(5-((5-((4'-chloro-5,5-dimethyl-3,4,5,6-tetrahydro-[1,1'-biphenyl]-2-yl) methyl)-2,5-diazabicyclo [2.2.1]heptan-2-yl)methyl)-1-oxoisoindolin-2-yl)dihydropyrimidine-2,4(1H,3H)-dione |
| GT-0788 6 | 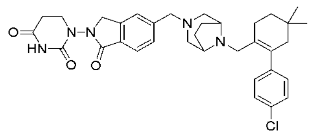 | 1-(5-((8-((4'-chloro-5,5-dimethyl-3,4,5,6-tetrahydro-[1,1'-biphenyl]-2-yl) methyl)-3,8-diazabicyclo [3.2.1]octan-3-yl)methyl)-1-oxoisoindolin-2-yl)dihydropyrimidine-2,4(1H,3H)-dione | 1-(5-((8-((4'-chloro-5,5-dimethyl-3,4,5,6-tetrahydro-[1,1'-biphenyl]-2-yl) methyl)-3,8-diazabicyclo [3.2.1]octan-3-yl)methyl)-1-oxoisoindolin-2-yl)dihydropyrimidine-2,4(1H,3H)-dione |
| GT-0733 0 | 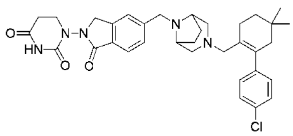 | 1-(5-((3-((4'-chloro-5,5-dimethyl-3,4,5,6-tetrahydro-[1,1'-biphenyl]-2-yl) methyl)-3,8-diazabicyclo [3.2.1]octan-8-yl)methyl)-1-oxoisoindolin-2-yl)dihydropyrimidine-2,4(1H,3H)-dione | 1-(5-((3-((4'-chloro-5,5-dimethyl-3,4,5,6-tetrahydro-[1,1'-biphenyl]-2-yl) methyl)-3,8-diazabicyclo [3.2.1]octan-8-yl)methyl)-1-oxoisoindolin-2-yl)dihydropyrimidine-2,4(1H,3H)-dione |
| GT-0733 1 | 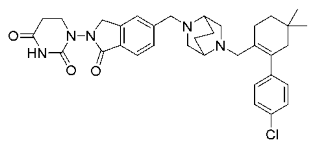 | 1-(5-((5-((4'-chloro-5,5-dimethyl-3,4,5,6-tetrahydro-[1,1'-biphenyl]-2-yl) methyl)-2,5-diazabicyclo [2.2.2]octan-2-yl)methyl)-1-oxoisoindolin-2-yl)dihydropyrimidine-2,4(1H,3H)-dione | 1-(5-((5-((4'-chloro-5,5-dimethyl-3,4,5,6-tetrahydro-[1,1'-biphenyl]-2-yl) methyl)-2,5-diazabicyclo [2.2.2] octan-2-yl)methyl)-1-oxoisoindolin-2-yl)dihydropyrimidine-2,4(1H,3H)-dione |
| GT-0729 6 | 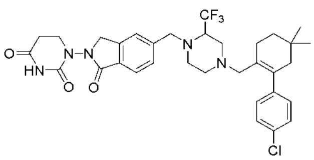 | 1-(5-((4-((4'-chloro-5,5-dimethyl-3,4,5,6-tetrahydro-[1,1'-biphenyl]-2-yl) methyl)-2-(trifluoromethyl)piperazin-1-yl)methyl)-1-oxoisoindolin-2-yl)dihydropyrimidine-2,4(1H,3H)-dione | 1-(5-((4-((4'-chloro-5,5-dimethyl-3,4,5,6-tetrahydro-[1,1'-biphenyl]-2-yl) methyl)-2-(trifluoromethyl)piperazin-1-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0729 7 | 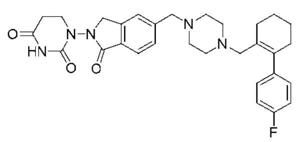 | 1-(5-((4-((4'-fluoro-3,4,5,6-tetrahydro-[1,1'-biphenyl]-2-yl)methyl)piperazin-1-yl)methyl)-1-oxoisoindolin-2-yl)dihydropyrimidine-2,4(1H,3H)-dione | 1-(5-((4-((4'-fluoro-3,4,5,6-tetrahydro-[1,1'-biphenyl]-2-yl)methyl)piperazin-1-yl)methyl)-1-oxoisoindolin-2-yl)dihydropyrimidine-2,4(1H,3H)-dione |
| GT-0729 9 | 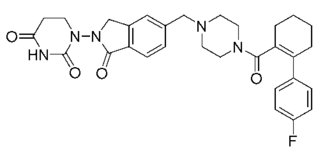 | 1-(5-((4-(4'-fluoro-3,4,5,6-tetrahydro-[1,1'-biphenyl]-2-carbonyl)piperazin-1-yl)methyl)-1-oxoisoindolin-2-yl)dihydropyrimidine-2,4(1H,3H)-dione | 1-(5-((4-(4'-fluoro-3,4,5,6-tetrahydro-[1,1'-biphenyl]-2-carbonyl)piperazin-1-yl)methyl)-1-oxoisoindolin-2-yl)dihydropyrimidine-2,4(1H,3H)-dione |
| GT-0737 6 | 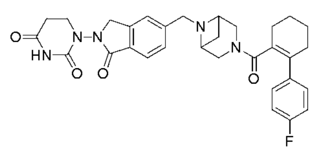 | 1-(5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)dihydropyrimidine-2,4(1H,3H)-dione | 1-(5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)dihydropyrimidine-2,4(1H,3H)-dione |
| GT-0494 3 | 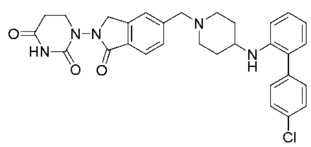 | 1-(5-((4-((4'-chloro-[1,1'-biphenyl]-2-yl)amino)piperidin-1-yl)methyl)-1-oxoisoindolin-2-yl)dihydropyrimidine-2,4(1H,3H)-dione | 1-(5-((4-((4'-chloro-[1,1'-biphenyl]-2-yl)amino)piperidin-1-yl)methyl)-1-oxoisoindolin-2-yl)dihydropyrimidine-2,4(1H,3H)-dione |
| GT-0643 3 | 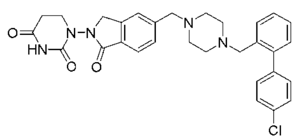 | 1-(5-((4-((4'-chloro-[1,1'-biphenyl]-2-yl)methyl)piperazin-1-yl)methyl)-1-oxoisoindolin-2-yl)dihydropyrimidine-2,4(1H,3H)-dione | 1-(5-((4-((4'-chloro-[1,1'-biphenyl]-2-yl)methyl)piperazin-1-yl)methyl)-1-oxoisoindolin-2-yl)dihydropyrimidine-2,4(1H,3H)-dione |
| GT-0733 2 | 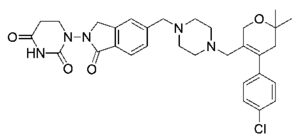 | 1-(5-((4-((4-(4-chlorophenyl)-6,6-dimethyl-5,6-dihydro-2H-pyran-3-yl)methyl)piperazin-1-yl)methyl)-1-oxoisoindolin-2-yl)dihydropyrimidine-2,4(1H,3H)-dione | 1-(5-((4-((4-(4-chlorophenyl)-6,6-dimethyl-5,6-dihydro-2H-pyran-3-yl)methyl)piperazin-1-yl)methyl)-1-oxoisoindolin-2-yl)dihydropyrimidine-2,4(1H,3H)-dione |
| GT-0507 3 | 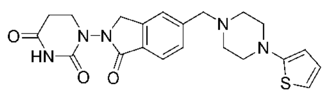 | 1-(1-oxo-5-((4-(thiophen-2-yl)piperazin-1-yl)methyl)isoindolin-2-yl)dihydropyrimidine-2,4(1H,3H)-dione | 1-(1-oxo-5-((4-(thiophen-2-yl)piperazin-1-yl)methyl)isoindolin-2-yl)dihydropyrimidine-2,4(1H,3H)-dione |
| GT-0508 3 | 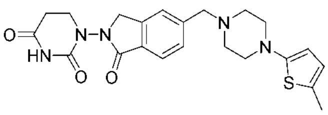 | 1-(5-((4-(5-methylthiophen-2-yl)piperazin-1-yl)methyl)-1-oxoisoindolin-2-yl)dihydropyrimidine-2,4(1H,3H)-dione | 1-(5-((4-(5-methylthiophen-2-yl)piperazin-1-yl)methyl)-1-oxoisoindolin-2-yl)dihydropyrimidine-2,4(1H,3H)-dione |
| GT-0508 4 | 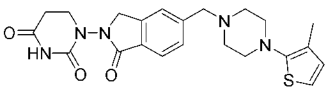 | 1-(5-((4-(3-methylthiophen-2-yl)piperazin-1-yl)methyl)-1-oxoisoindolin-2-yl)dihydropyrimidine-2,4(1H,3H)-dione | 1-(5-((4-(3-methylthiophen-2-yl)piperazin-1-yl)methyl)-1-oxoisoindolin-2-yl)dihydropyrimidine-2,4(1H,3H)-dione |
| GT-0508 5 | 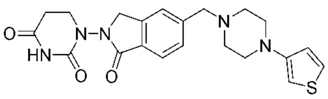 | 1-(1-oxo-5-((4-(thiophen-3-yl)piperazin-1-yl)methyl)isoindolin-2-yl)dihydropyrimidine-2,4(1H,3H)-dione | 1-(1-oxo-5-((4-(thiophen-3-yl)piperazin-1-yl)methyl)isoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0508 6 | 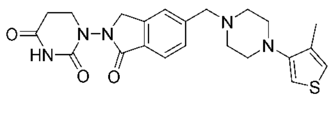 | 1-(5-((4-(4-methylthiophen-3-yl)piperazin-1-yl)methyl)-1-oxoisoindolin-2-yl)dihydropyrimidine-2,4(1H,3H)-dione | 1-(5-((4-(4-methylthiophen-3-yl)piperazin-1-yl)methyl)-1-oxoisoindolin-2-yl)dihydropyrimidine-2,4(1H,3H)-dione |
| GT-0508 7 | 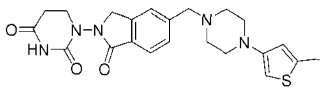 | 1-(5-((4-(5-methylthiophen-3-yl)piperazin-1-yl)methyl)-1-oxoisoindolin-2-yl)dihydropyrimidine-2,4(1H,3H)-dione | 1-(5-((4-(5-methylthiophen-3-yl)piperazin-1-yl)methyl)-1-oxoisoindolin-2-yl)dihydropyrimidine-2,4(1H,3H)-dione |
| GT-0486 8 | 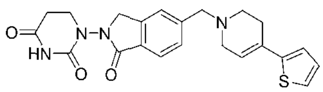 | 1-(1-oxo-5-((4-(thiophen-2-yl)-3,6-dihydropyridin-1(2H)-yl)methyl)isoindolin-2-yl)dihydropyrimidine-2,4(1H,3H)-dione | 1-(1-oxo-5-((4-(thiophen-2-yl)-3,6-dihydropyridin-1(2H)-yl)methyl)isoindolin-2-yl)dihydropyrimidine-2,4(1H,3H)-dione |
| GT-0486 9 | 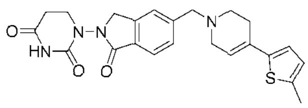 | 1-(5-((4-(5-methylthiophen-2-yl)-3,6-dihydropyridin-1(2H)-yl)methyl)-1-oxoisoindolin-2-yl)dihydropyrimidine-2,4(1H,3H)-dione | 1-(5-((4-(5-methylthiophen-2-yl)-3,6-dihydropyridin-1(2H)-yl)methyl)-1-oxoisoindolin-2-yl)dihydropyrimidine-2,4(1H,3H)-dione |
| GT-0487 0 | 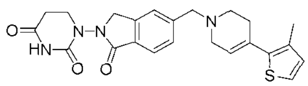 | 1-(5-((4-(3-methylthiophen-2-yl)-3,6-dihydropyridin-1(2H)-yl)methyl)-1-oxoisoindolin-2-yl)dihydropyrimidine-2,4(1H,3H)-dione | 1-(5-((4-(3-methylthiophen-2-yl)-3,6-dihydropyridin-1(2H)-yl)methyl)-1-oxoisoindolin-2-yl)dihydropyrimidine-2,4(1H,3H)-dione |
| GT-0487 1 | 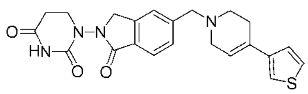 | 1-(1-oxo-5-((4-(thiophen-3-yl)-3,6-dihydropyridin-1(2H)-yl)methyl)isoindolin-2-yl)dihydropyrimidine-2,4(1H,3H)-dione | 1-(1-oxo-5-((4-(thiophen-3-yl)-3,6-dihydropyridin-1(2H)-yl)methyl)isoindolin-2-yl)dihydropyrimidine-2,4(1H,3H)-dione |
| GT-0487 2 | 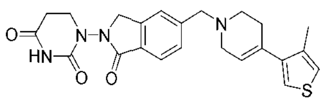 | 1-(5-((4-(4-methylthiophen-3-yl)-3,6-dihydropyridin-1(2H)-yl)methyl)-1-oxoisoindolin-2-yl)dihydropyrimidine-2,4(1H,3H)-dione | 1-(5-((4-(4-methylthiophen-3-yl)-3,6-dihydropyridin-1(2H)-yl)methyl)-1-oxoisoindolin-2-yl)dihydropyrimidine-2,4(1H,3H)-dione |
| GT-0487 3 | 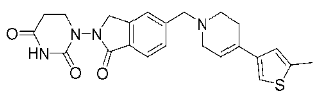 | 1-(5-((4-(5-methylthiophen-3-yl)-3,6-dihydropyridin-1(2H)-yl)methyl)-1-oxoisoindolin-2-yl)dihydropyrimidine-2,4(1H,3H)-dione | 1-(5-((4-(5-methylthiophen-3-yl)-3,6-dihydropyridin-1(2H)-yl)methyl)-1-oxoisoindolin-2-yl)dihydropyrimidine-2,4(1H,3H)-dione |
| GT-0487 4 | 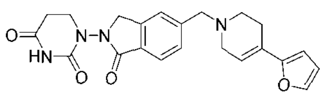 | 1-(5-((4-(furan-2-yl)-3,6-dihydropyridin-1(2H)-yl)methyl)-1-oxoisoindolin-2-yl)dihydropyrimidine-2,4(1H,3H)-dione | 1-(5-((4-(furan-2-yl)-3,6-dihydropyridin-1(2H)-yl)methyl)-1-oxoisoindolin-2-yl)dihydropyrimidine-2,4(1H,3H)-dione |
| GT-0487 5 | 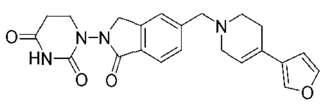 | 1-(5-((4-(furan-3-yl)-3,6-dihydropyridin-1(2H)-yl)methyl)-1-oxoisoindolin-2-yl)dihydropyrimidine-2,4(1H,3H)-dione | 1-(5-((4-(furan-3-yl)-3,6-dihydropyridin-1(2H)-yl)methyl)-1-oxoisoindolin-2-yl)dihydropyrimidine-2,4(1H,3H)-dione |
| GT-0751 1 | 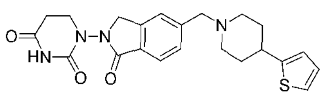 | 1-(1-oxo-5-((4-(thiophen-2-yl)piperidin-1-yl)methyl)isoindolin-2-yl)dihydropyrimidine-2,4(1H,3H)-dione | 1-(1-oxo-5-((4-(thiophen-2-yl)piperidin-1-yl)methyl)isoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0742 5 | 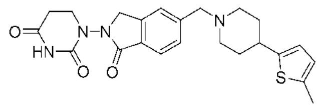 | 1-(5-((4-(5-methylthiophen-2-yl)piperidin-1-yl)methyl)-1-oxoisoindolin-2-yl)dihydropyrimidine-2,4(1H,3H)-dione | 1-(5-((4-(5-methylthiophen-2-yl)piperidin-1-yl)methyl)-1-oxoisoindolin-2-yl)dihydropyrimidine-2,4(1H,3H)-dione |
| GT-0730 5 | 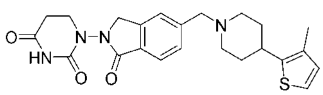 | 1-(5-((4-(3-methylthiophen-2-yl)piperidin-1-yl)methyl)-1-oxoisoindolin-2-yl)dihydropyrimidine-2,4(1H,3H)-dione | 1-(5-((4-(3-methylthiophen-2-yl)piperidin-1-yl)methyl)-1-oxoisoindolin-2-yl)dihydropyrimidine-2,4(1H,3H)-dione |
| GT-0737 1 | 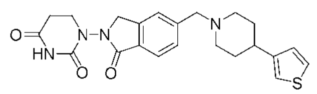 | 1-(1-oxo-5-((4-(thiophen-3-yl) piperidin-1-yl)methyl)isoindolin-2-yl)dihydropyrimidine-2,4(1H,3H)-dione | 1-(1-oxo-5-((4-(thiophen-3-yl) piperidin-1-yl)methyl)isoindolin-2-yl)dihydropyrimidine-2,4(1H,3H)-dione |
| GT-0737 3 | 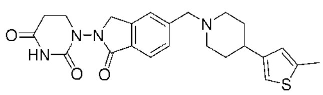 | 1-(5-((4-(5-methylthiophen-3-yl)piperidin-1-yl)methyl)-1-oxoisoindolin-2-yl)dihydropyrimidine-2,4(1H,3H)-dione | 1-(5-((4-(5-methylthiophen-3-yl)piperidin-1-yl)methyl)-1-oxoisoindolin-2-yl)dihydropyrimidine-2,4(1H,3H)-dione |
| GT-0737 2 | 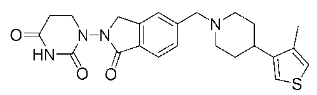 | 1-(5-((4-(4-methylthiophen-3-yl)piperidin-1-yl)methyl)-1-oxoisoindolin-2-yl)dihydropyrimidine-2,4(1H,3H)-dione | 1-(5-((4-(4-methylthiophen-3-yl)piperidin-1-yl)methyl)-1-oxoisoindolin-2-yl)dihydropyrimidine-2,4(1H,3H)-dione |
| GT-0737 4 | 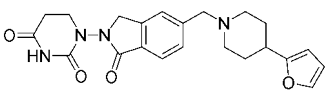 | 1-(5-((4-(furan-2-yl)piperidin-1-yl)methyl)-1-oxoisoindolin-2-yl)dihydropyrimidine-2,4(1H,3H)-dione | 1-(5-((4-(furan-2-yl)piperidin-1-yl)methyl)-1-oxoisoindolin-2-yl)dihydropyrimidine-2,4(1H,3H)-dione |
| GT-0737 5 | 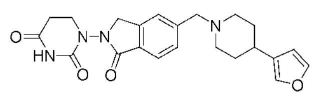 | 1-(5-((4-(furan-3-yl)piperidin-1-yl)methyl)-1-oxoisoindolin-2-yl)dihydropyrimidine-2,4(1H,3H)-dione | 1-(5-((4-(furan-3-yl)piperidin-1-yl)methyl)-1-oxoisoindolin-2-yl)dihydropyrimidine-2,4(1H,3H)-dione |
| GT-0485 3 | 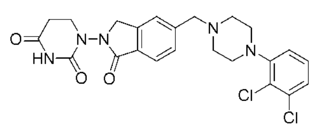 | 1-(5-((4-(2,3-dichlorophenyl) piperazin-1-yl)methyl)-1-oxoisoindolin-2-yl)dihydropyrimidine-2,4(1H,3H)-dione | 1-(5-((4-(2,3-dichlorophenyl) piperazin-1-yl)methyl)-1-oxoisoindolin-2-yl)dihydropyrimidine-2,4(1H,3H)-dione |
| GT-0669 6 | 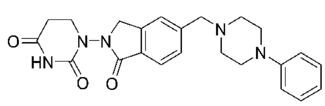 | 1-(1-oxo-5-((4-phenylpiperazin-1-yl)methyl)isoindolin-2-yl) dihydropyrimidine-2,4(1H,3H)-dione | 1-(1-oxo-5-((4-phenylpiperazin-1-yl)methyl)isoindolin-2-yl) dihydropyrimidine-2,4(1H,3H)-dione |
| GT-0669 7 | 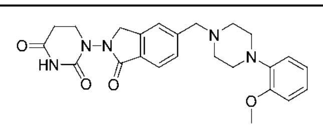 | 1-(5-((4-(2-methoxyphenyl)piperazin-1-yl)methyl)-1-oxoisoindolin-2-yl)dihydropyrimidine-2,4(1H,3H)-dione | 1-(5-((4-(2-methoxyphenyl)piperazin-1-yl)methyl)-1-oxoisoindolin-2-yl)dihydropyrimidine-2,4(1H,3H)-dione |
| GT-0669 9 | 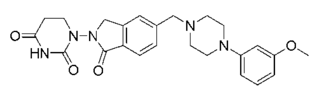 | 1-(5-((4-(3-methoxyphenyl)piperazin-1-yl)methyl)-1-oxoisoindolin-2-yl)dihydropyrimidine-2,4(1H,3H)-dione | 1-(5-((4-(3-methoxyphenyl)piperazin-1-yl)methyl)-1-oxoisoindolin-2-yl)dihydropyrimidine-2,4(1H,3H)-dione |
| GT-0676 7 | 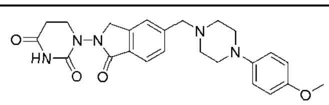 | 1-(5-((4-(4-methoxyphenyl)piperazin-1-yl)methyl)-1-oxoisoindolin-2-yl)dihydropyrimidine-2,4(1H,3H)-dione | 1-(5-((4-(4-methoxyphenyl)piperazin-1-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0670 0 | 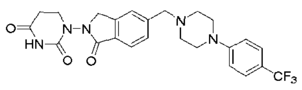 | 1-(1-oxo-5-((4-(4-(trifluoromethyl)phenyl)piper azin-1-yl) methyl)isoindolin-2-yl)dihydropyrimidine-2,4(1H,3H)-dione | 1-(1-oxo-5-((4-(4-(trifluoromethyl)phenyl)piper azin-1-yl) methyl)isoindolin-2-yl)dihydropyrimidine-2,4(1H,3H)-dione |
| GT-0670 1 | 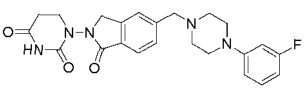 | 1-(5-((4-(3-fluorophenyl)piperazin-1-yl)methyl)-1-oxoisoindolin-2-yl)dihydropyrimidine-2,4(1H,3H)-dione | 1-(5-((4-(3-fluorophenyl)piperazin-1-yl)methyl)-1-oxoisoindolin-2-yl)dihydropyrimidine-2,4(1H,3H)-dione |
| GT-0670 2 | 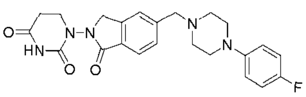 | 1-(5-((4-(4-fluorophenyl)piperazin-1-yl)methyl)-1-oxoisoindolin-2-yl)dihydropyrimidine-2,4(1H,3H)-dione | 1-(5-((4-(4-fluorophenyl)piperazin-1-yl)methyl)-1-oxoisoindolin-2-yl)dihydropyrimidine-2,4(1H,3H)-dione |
| GT-0670 3 | 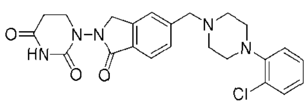 | 1-(5-((4-(2-chlorophenyl)piperazin-1-yl)methyl)-1-oxoisoindolin-2-yl)dihydropyrimidine-2,4(1H,3H)-dione | 1-(5-((4-(2-chlorophenyl)piperazin-1-yl)methyl)-1-oxoisoindolin-2-yl)dihydropyrimidine-2,4(1H,3H)-dione |
| GT-0676 5 | 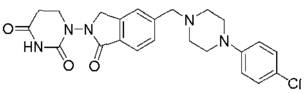 | 1-(5-((4-(4-chlorophenyl)piperazin-1-yl)methyl)-1-oxoisoindolin-2-yl)dihydropyrimidine-2,4(1H,3H)-dione | 1-(5-((4-(4-chlorophenyl)piperazin-1-yl)methyl)-1-oxoisoindolin-2-yl)dihydropyrimidine-2,4(1H,3H)-dione |
| GT-0676 6 | 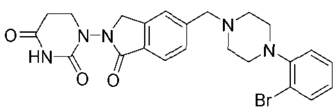 | 1-(5-((4-(2-bromophenyl)piperazin-1-yl)methyl)-1-oxoisoindolin-2-yl)dihydropyrimidine-2,4(1H,3H)-dione | 1-(5-((4-(2-bromophenyl)piperazin-1-yl)methyl)-1-oxoisoindolin-2-yl)dihydropyrimidine-2,4(1H,3H)-dione |
| GT-0669 8 | 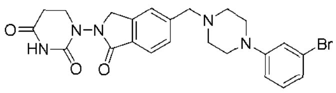 | 1-(5-((4-(3-bromophenyl)piperazin-1-yl)methyl)-1-oxoisoindolin-2-yl)dihydropyrimidine-2,4(1H,3H)-dione | 1-(5-((4-(3-bromophenyl)piperazin-1-yl)methyl)-1-oxoisoindolin-2-yl)dihydropyrimidine-2,4(1H,3H)-dione |
| GT-0676 8 | 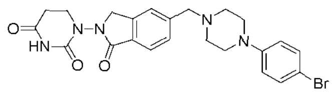 | 1-(5-((4-(4-bromophenyl)piperazin-1-yl)methyl)-1-oxoisoindolin-2-yl)dihydropyrimidine-2,4(1H,3H)-dione | 1-(5-((4-(4-bromophenyl)piperazin-1-yl)methyl)-1-oxoisoindolin-2-yl)dihydropyrimidine-2,4(1H,3H)-dione |
| GT-0676 9 | 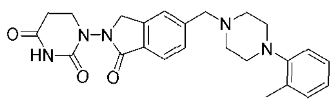 | 1-(1-oxo-5-((4-(o-tolyl)piperazin-1-yl)methyl)isoindolin-2-yl) dihydropyrimidine-2,4(1H,3H)-dione | 1-(1-oxo-5-((4-(o-tolyl)piperazin-1-yl)methyl)isoindolin-2-yl) dihydropyrimidine-2,4(1H,3H)-dione |
| GT-0677 0 | 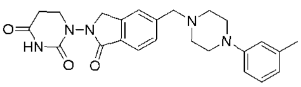 | 1-(1-oxo-5-((4-(m-tolyl)piperazin-1-yl)methyl)isoindolin-2-yl) dihydropyrimidine-2,4(1H,3H)-dione | 1-(1-oxo-5-((4-(m-tolyl)piperazin-1-yl)methyl)isoindolin-2-yl) dihydropyrimidine-2,4(1H,3H)-dione |
| GT-0677 1 | 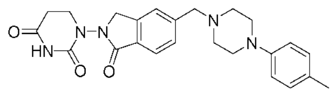 | 1-(1-oxo-5-((4-(p-tolyl)piperazin-1-yl)methyl)isoindolin-2-yl) dihydropyrimidine-2,4(1H,3H)-dione | 1-(1-oxo-5-((4-(p-tolyl)piperazin-1-yl)methyl)isoindolin-2-yl) dihydropyrimidine-2,4(1H,3H)-dione |
| GT-0677 2 | 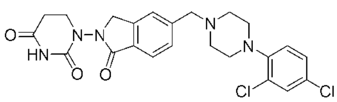 | 1-(5-((4-(2,4-dichlorophenyl) piperazin-1-yl)methyl)-1-oxoisoindolin-2-yl)dihydropyrimidine-2,4(1H,3H)-dione | 1-(5-((4-(2,4-dichlorophenyl) piperazin-1-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0677 3 | 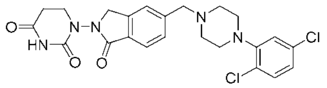 | 1-(5-((4-(2,5-dichlorophenyl) piperazin-1-yl)methyl)-1-oxoisoindolin-2-yl)dihydropyrimidine-2,4(1H,3H)-dione | 1-(5-((4-(2,5-dichlorophenyl) piperazin-1-yl)methyl)-1-oxoisoindolin-2-yl)dihydropyrimidine-2,4(1H,3H)-dione |
| GT-0677 4 | 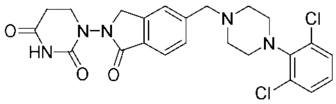 | 1-(5-((4-(2,6-dichlorophenyl) piperazin-1-yl)methyl)-1-oxoisoindolin-2-yl)dihydropyrimidine-2,4(1H,3H)-dione | 1-(5-((4-(2,6-dichlorophenyl) piperazin-1-yl)methyl)-1-oxoisoindolin-2-yl)dihydropyrimidine-2,4(1H,3H)-dione |
| GT-0677 5 | 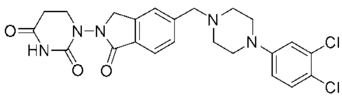 | 1-(5-((4-(3,4-dichlorophenyl) piperazin-1-yl)methyl)-1-oxoisoindolin-2-yl)dihydropyrimidine-2,4(1H,3H)-dione | 1-(5-((4-(3,4-dichlorophenyl) piperazin-1-yl)methyl)-1-oxoisoindolin-2-yl)dihydropyrimidine-2,4(1H,3H)-dione |
| GT-0677 6 | 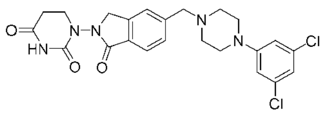 | 1-(5-((4-(3,5-dichlorophenyl) piperazin-1-yl)methyl)-1-oxoisoindolin-2-yl)dihydropyrimidine-2,4(1H,3H)-dione | 1-(5-((4-(3,5-dichlorophenyl) piperazin-1-yl)methyl)-1-oxoisoindolin-2-yl)dihydropyrimidine-2,4(1H,3H)-dione |
| GT-0677 7 | 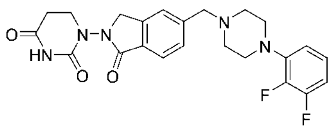 | 1-(5-((4-(2,3-difluorophenyl) piperazin-1-yl)methyl)-1-oxoisoindolin-2-yl)dihydropyrimidine-2,4(1H,3H)-dione | 1-(5-((4-(2,3-difluorophenyl) piperazin-1-yl)methyl)-1-oxoisoindolin-2-yl)dihydropyrimidine-2,4(1H,3H)-dione |
| GT-0677 8 | 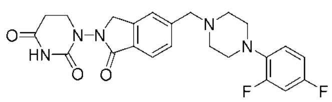 | 1-(5-((4-(2,4-difluorophenyl) piperazin-1-yl)methyl)-1-oxoisoindolin-2-yl)dihydropyrimidine-2,4(1H,3H)-dione | 1-(5-((4-(2,4-difluorophenyl) piperazin-1-yl)methyl)-1-oxoisoindolin-2-yl)dihydropyrimidine-2,4(1H,3H)-dione |
| GT-0677 9 | 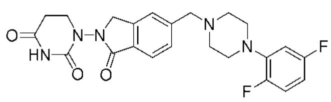 | 1-(5-((4-(2,5-difluorophenyl) piperazin-1-yl)methyl)-1-oxoisoindolin-2-yl)dihydropyrimidine-2,4(1H,3H)-dione | 1-(5-((4-(2,5-difluorophenyl) piperazin-1-yl)methyl)-1-oxoisoindolin-2-yl)dihydropyrimidine-2,4(1H,3H)-dione |
| GT-0678 0 | 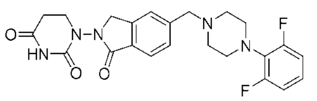 | 1-(5-((4-(2,6-difluorophenyl) piperazin-1-yl)methyl)-1-oxoisoindolin-2-yl)dihydropyrimidine-2,4(1H,3H)-dione | 1-(5-((4-(2,6-difluorophenyl) piperazin-1-yl)methyl)-1-oxoisoindolin-2-yl)dihydropyrimidine-2,4(1H,3H)-dione |
| GT-0678 1 | 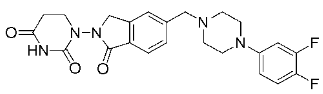 | 1-(5-((4-(3,4-difluorophenyl) piperazin-1-yl)methyl)-1-oxoisoindolin-2-yl)dihydropyrimidine-2,4(1H,3H)-dione | 1-(5-((4-(3,4-difluorophenyl) piperazin-1-yl)methyl)-1-oxoisoindolin-2-yl)dihydropyrimidine-2,4(1H,3H)-dione |
| GT-0678 2 | 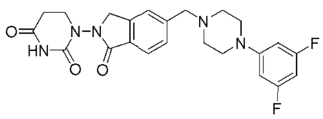 | 1-(5-((4-(3,5-difluorophenyl) piperazin-1-yl)methyl)-1-oxoisoindolin-2-yl)dihydropyrimidine-2,4(1H,3H)-dione | 1-(5-((4-(3,5-difluorophenyl) piperazin-1-yl)methyl)-1-oxoisoindolin-2-yl)dihydropyrimidine-2,4(1H,3H)-dione |
| GT-0688 2 | 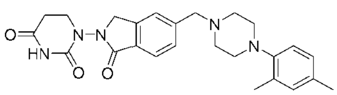 | 1-(5-((4-(2,4-dimethylphenyl) piperazin-1-yl)methyl)-1-oxoisoindolin-2-yl)dihydropyrimidine-2,4(1H,3H)-dione | 1-(5-((4-(2,4-dimethylphenyl) piperazin-1-yl)methyl)-1-oxoisoindolin-2-yl)dihydropyrimidine-2,4(1H,3H)-dione |
| GT-0688 3 | 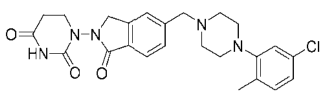 | 1-(5-((4-(5-chloro-2-methylphenyl)piperazin-1-yl) methyl)-1-oxoisoindolin-2-yl) dihydropyrimidine-2,4(1H,3H)-dione | 1-(5-((4-(5-chloro-2-methylphenyl)piperazin-1-yl) methyl)-1-oxoisoindolin-2-yl) dihydropyrimidine-2,4(1H,3H)-dione |

(continued)

| Compound No. | Structure of the compounds | The compounds' name | Chinese translation of the compounds' name |
|---|---|---|---|
| GT-0688 4 | 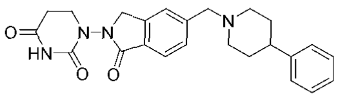 | 1-(1-oxo-5-((4-phenylpiperidin-1-yl)methyl)isoindolin-2-yl)dihydropyrimidine-2,4(1H,3H)-dione | 1-(1-oxo-5-((4-phenylpiperidin-1-yl)methyl)isoindolin-2-yl) dihydropyrimidine-2,4(1H,3H)-dione |
| GT-0688 5 | 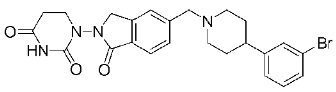 | 1-(5-((4-(3-bromophenyl)piperidin-1-yl)methyl)-1-oxoisoindolin-2-yl)dihydropyrimidine-2,4(1H,3H)-dione | 1-(5-((4-(3-bromophenyl)piperidin-1-yl)methyl)-1-oxoisoindolin-2-yl)dihydropyrimidine-2,4(1H,3H)-dione |
| GT-0688 6 | 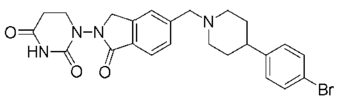 | 1-(5-((4-(4-bromophenyl)piperidin-1-yl)methyl)-1-oxoisoindolin-2-yl)dihydropyrimidine-2,4(1H,3H)-dione | 1-(5-((4-(4-bromophenyl)piperidin-1-yl)methyl)-1-oxoisoindolin-2-yl)dihydropyrimidine-2,4(1H,3H)-dione |
| GT-0688 7 | 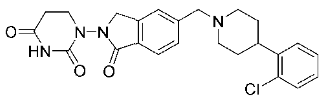 | 1-(5-((4-(2-chlorophenyl)piperidin-1-yl)methyl)-1-oxoisoindolin-2-yl)dihydropyrimidine-2,4(1H,3H)-dione | 1-(5-((4-(2-chlorophenyl)piperidin-1-yl)methyl)-1-oxoisoindolin-2-yl)dihydropyrimidine-2,4(1H,3H)-dione |
| GT-0688 8 | 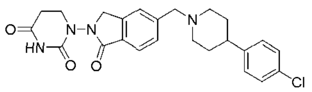 | 1-(5-((4-(4-chlorophenyl)piperidin-1-yl)methyl)-1-oxoisoindolin-2-yl)dihydropyrimidine-2,4(1H,3H)-dione | 1-(5-((4-(4-chlorophenyl)piperidin-1-yl)methyl)-1-oxoisoindolin-2-yl)dihydropyrimidine-2,4(1H,3H)-dione |
| GT-0688 9 | 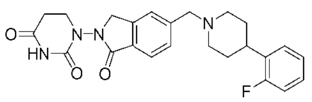 | 1-(5-((4-(2-fluorophenyl)piperidin-1-yl)methyl)-1-oxoisoindolin-2-yl)dihydropyrimidine-2,4(1H,3H)-dione | 1-(5-((4-(2-fluorophenyl)piperidin-1-yl)methyl)-1-oxoisoindolin-2-yl)dihydropyrimidine-2,4(1H,3H)-dione |
| GT-0689 0 | 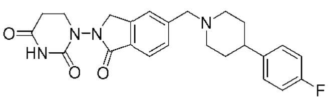 | 1-(5-((4-(4-fluorophenyl)piperidin-1-yl)methyl)-1-oxoisoindolin-2-yl)dihydropyrimidine-2,4(1H,3H)-dione | 1-(5-((4-(4-fluorophenyl)piperidin-1-yl)methyl)-1-oxoisoindolin-2-yl)dihydropyrimidine-2,4(1H,3H)-dione |
| GT-0689 1 | 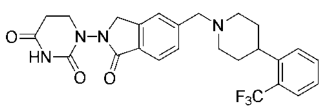 | 1-(1-oxo-5-((4-(2-(trifluoromethyl)phenyl)piper idin-1-yl) methyl)isoindolin-2-yl)dihydropyrimidine-2,4(1H,3H)-dione | 1-(1-oxo-5-((4-(2-(trifluoromethyl)phenyl)piper idin-1-yl) methyl)isoindolin-2-yl)dihydropyrimidine-2,4(1H,3H)-dione |
| GT-0689 2 | 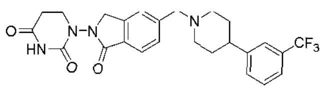 | 1-(1-oxo-5-((4-(3-(trifluoromethyl)phenyl)piper idin-1-yl) methyl)isoindolin-2-yl)dihydropyrimidine-2,4(1H,3H)-dione | 1-(1-oxo-5-((4-(3-(trifluoromethyl)phenyl)piper idin-1-yl) methyl)isoindolin-2-yl)dihydropyrimidine-2,4(1H,3H)-dione |
| GT-0689 3 | 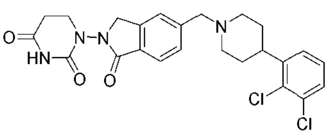 | 1-(5-((4-(2,3-dichlorophenyl) piperidin-1-yl)methyl)-1-oxoisoindolin-2-yl)dihydropyrimidine-2,4(1H,3H)-dione | 1-(5-((4-(2,3-dichlorophenyl) piperidin-1-yl)methyl)-1-oxoisoindolin-2-yl)dihydropyrimidine-2,4(1H,3H)-dione |
| GT-0689 4 | 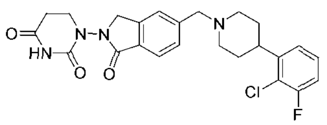 | 1-(5-((4-(2-chloro-3-fluorophenyl)piperidin-1-yl) methyl)-1-oxoisoindolin-2-yl) dihydropyrimidine-2,4(1H,3H)-dione | 1-(5-((4-(2-chloro-3-fluorophenyl)piperidin-1-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0819 4 | 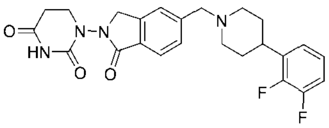 | 1-(5-((4-(2,3-difluorophenyl) piperidin-1-yl)methyl)-1-oxoisoindolin-2-yl)dihydropyrimidine-2,4(1H,3H)-dione | 1-(5-((4-(2,3-difluorophenyl) piperidin-1-yl)methyl)-1-oxoisoindolin-2-yl)dihydropyrimidine-2,4(1H,3H)-dione |
| GT-0805 4 | 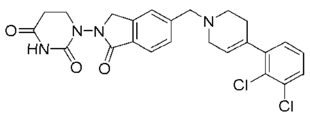 | 1-(5-((4-(2,3-dichlorophenyl)-3,6-dihydropyridin-1(2H)-yl)methyl)-1-oxoisoindolin-2-yl)dihydropyrimidine-2,4(1H,3H)-dione | 1-(5-((4-(2,3-dichlorophenyl)-3,6-dihydropyridin-1(2H)-yl)methyl)-1-oxoisoindolin-2-yl)dihydropyrimidine-2,4(1H,3H)-dione |
| GT-0805 5 | 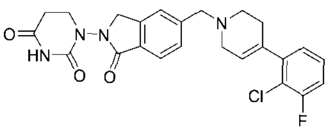 | 1-(5-((4-(2-chloro-3-fluorophenyl)-3,6-dihydropyrdin-1(2H)-yl)methyl)-1-oxoisoindolin-2-yl)dihydropyrimidine-2,4(1H,3H)-dione | 1-(5-((4-(2-chloro-3-fluorophenyl)-3,6-dihydropyridin-1(2H)-yl)methyl)-1-oxoisoindolin-2-yl)dihydropyrimidine-2,4(1H,3H)-dione |
| GT-0512 4 | 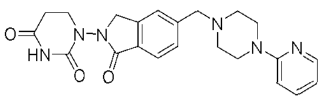 | 1-(1-oxo-5-((4-(pyridin-2-yl)piperazin-1-yl)methyl)isoindolin-2-yl)dihydropyrimidine-2,4(1H,3H)-dione | 1-(1-oxo-5-((4-(pyridin-2-yl)piperazin-1-yl)methyl)isoindolin-2-yl)dihydropyrimidine-2,4(1H,3H)-dione |
| GT-0512 5 | 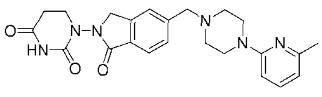 | 1-(5-((4-(6-methylpyridin-2-yl) piperazin-1-yl)methyl)-1-oxoisoindolin-2-yl)dihydropyrimidine-2,4(1H,3H)-dione | 1-(5-((4-(6-methylpyridin-2-yl) piperazin-1-yl)methyl)-1-oxoisoindolin-2-yl)dihydropyrimidine-2,4(1H,3H)-dione |
| GT-0512 6 | 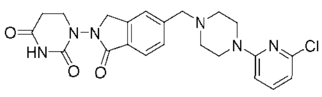 | 1-(5-((4-(6-chloropyridin-2-yl) piperazin-1-yl)methyl)-1-oxoisoindolin-2-yl)dihydropyrimidine-2,4(1H,3H)-dione | 1-(5-((4-(6-chloropyridin-2-yl) piperazin-1-yl)methyl)-1-oxoisoindolin-2-yl)dihydropyrimidine-2,4(1H,3H)-dione |
| GT-0512 7 | 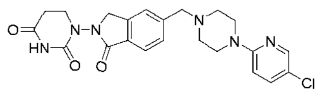 | 1-(5-((4-(5-chloropyridin-2-yl) piperazin-1-yl)methyl)-1-oxoisoindolin-2-yl)dihydropyrimidine-2,4(1H,3H)-dione | 1-(5-((4-(5-chloropyridin-2-yl) piperazin-1-yl)methyl)-1-oxoisoindolin-2-yl)dihydropyrimidine-2,4(1H,3H)-dione |
| GT-0512 8 | 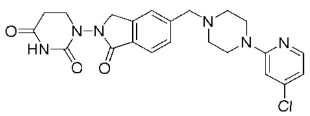 | 1-(5-((4-(4-chloropyridin-2-yl) piperazin-1-yl)methyl)-1-oxoisoindolin-2-yl)dihydropyrimidine-2,4(1H,3H)-dione | 1-(5-((4-(4-chloropyridin-2-yl) piperazin-1-yl)methyl)-1-oxoisoindolin-2-yl)dihydropyrimidine-2,4(1H,3H)-dione |
| | 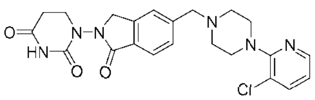 | 1-(5-((4-(3-chloropyridin-2-yl) piperazin-1-yl)methyl)-1-oxoisoindolin-2-yl)dihydropyrimidine-2,4(1H,3H)-dione | 1-(5-((4-(3-chloropyridin-2-yl) piperazin-1-yl)methyl)-1-oxoisoindolin-2-yl)dihydropyrimidine-2,4(1H,3H)-dione |
| GT-0512 9 | 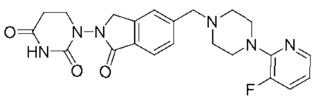 | 1-(5-((4-(3-fluoropyridin-2-yl) piperazin-1-yl)methyl)-1-oxoisoindolin-2-yl)dihydropyrimidine-2,4(1H,3H)-dione | 1-(5-((4-(3-fluoropyridin-2-yl) piperazin-1-yl)methyl)-1-oxoisoindolin-2-yl)dihydropyrimidine-2,4(1H,3H)-dione |
| GT-0513 0 | 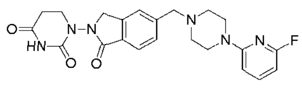 | 1-(5-((4-(6-fluoropyridin-2-yl) piperazin-1-yl)methyl)-1-oxoisoindolin-2-yl)dihydropyrimidine-2,4(1H,3H)-dione | 1-(5-((4-(6-fluoropyridin-2-yl) piperazin-1-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0513 1 | 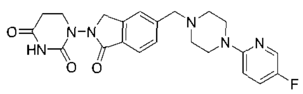 | 1-(5-((4-(5-fluoropyridin-2-yl)piperazin-1-yl)methyl)-1-oxoisoindolin-2-yl)dihydropyrimidine-2,4(1H,3H)-dione | 1-(5-((4-(5-fluoropyridin-2-yl)piperazin-1-yl)methyl)-1-oxoisoindolin-2-yl)dihydropyrimidine-2,4(1H,3H)-dione |
| GT-0517 4 | 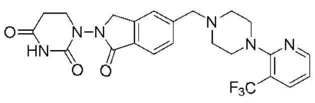 | 1-(1-oxo-5-((4-(3-(trifluoromethyl)pyridin-2-yl)piperazin-1-yl)methyl)isoindolin-2-yl)dihydropyrimidine-2,4(1H,3H)-dione | 1-(1-oxo-5-((4-(3-(trifluoromethyl)pyridin-2-yl)piperazin-1-yl)methyl)isoindolin-2-yl)dihydropyrimidine-2,4(1H,3H)-dione |
| GT-0517 5 | 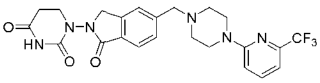 | 1-(1-oxo-5-((4-(6-(trifluoromethyl)pyridin-2-yl)piperazin-1-yl)methyl)isoindolin-2-yl)dihydropyrimidine-2,4(1H,3H)-dione | 1-(1-oxo-5-((4-(6-(trifluoromethyl)pyridin-2-yl)piperazin-1-yl)methyl)isoindolin-2-yl)dihydropyrimidine-2,4(1H,3H)-dione |
| GT-0517 6 | 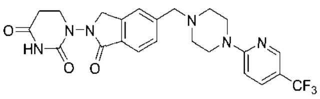 | 1-(1-oxo-5-((4-(5-(trifluoromethyl)pyridin-2-yl)piperazin-1-yl)methyl)isoindolin-2-yl)dihydropyrimidine-2,4(1H,3H)-dione | 1-(1-oxo-5-((4-(5-(trifluoromethyl)pyridin-2-yl)piperazin-1-yl)methyl)isoindolin-2-yl)dihydropyrimidine-2,4(1H,3H)-dione |
| | 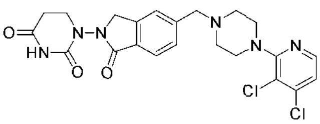 | 1-(5-((4-(3,4-dichloropyridin-2-yl)piperazin-1-yl)methyl)-1-oxoisoindolin-2-yl)dihydropyrimidine-2,4(1H,3H)-dione | 1-(5-((4-(3,4-dichloropyridin-2-yl)piperazin-1-yl)methyl)-1-oxoisoindolin-2-yl)dihydropyrimidine-2,4(1H,3H)-dione |
| GT-0535 6 | 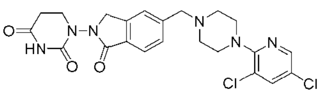 | 1-(5-((4-(3,5-dichloropyridin-2-yl)piperazin-1-yl)methyl)-1-oxoisoindolin-2-yl)dihydropyrimidine-2,4(1H,3H)-dione | 1-(5-((4-(3,5-dichloropyridin-2-yl)piperazin-1-yl)methyl)-1-oxoisoindolin-2-yl)dihydropyrimidine-2,4(1H,3H)-dione |
| | 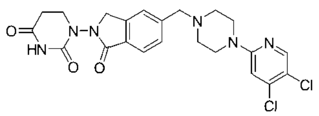 | 1-(5-((4-(4,5-dichloropyridin-2-yl)piperazin-1-yl)methyl)-1-oxoisoindolin-2-yl)dihydropyrimidine-2,4(1H,3H)-dione | 1-(5-((4-(4,5-dichloropyridin-2-yl)piperazin-1-yl)methyl)-1-oxoisoindolin-2-yl)dihydropyrimidine-2,4(1H,3H)-dione |
| GT-0535 7 | 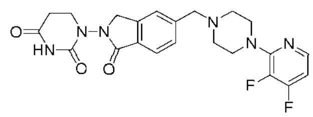 | 1-(5-((4-(3,4-difluoropyridin-2-yl)piperazin-1-yl)methyl)-1-oxoisoindolin-2-yl)dihydropyrimidine-2,4(1H,3H)-dione | 1-(5-((4-(3,4-difluoropyridin-2-yl)piperazin-1-yl)methyl)-1-oxoisoindolin-2-yl)dihydropyrimidine-2,4(1H,3H)-dione |
| GT-0535 8 | 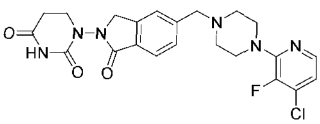 | 1-(5-((4-(4-chloro-3-fluoropyridin-2-yl)piperazin-1-yl)methyl)-1-oxoisoindolin-2-yl)dihydropyrimidine-2,4(1H,3H)-dione | 1-(5-((4-(4-chloro-3-fluoropyridin-2-yl)piperazin-1-yl)methyl)-1-oxoisoindolin-2-yl)dihydropyrimidine-2,4(1H,3H)-dione |
| GT-0540 6 | 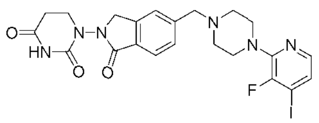 | 1-(5-((4-(3-fluoro-4-iodopyridin-2-yl)piperazin-1-yl)methyl)-1-oxoisoindolin-2-yl)dihydropyrimidine-2,4(1H,3H)-dione | 1-(5-((4-(3-fluoro-4-iodopyridin-2-yl)piperazin-1-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0543 2 | 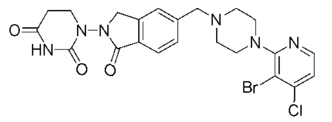 | 1-(5-((4-(3-bromo-4-chloropyridin-2-yl)piperazin-1-yl) methyl)-1-oxoisoindolin-2-yl) dihydropyrimidine-2,4(1H,3H)-dione | 1-(5-((4-(3-bromo-4-chloropyridin-2-yl)piperazin-1-yl) methyl)-1-oxoisoindolin-2-yl) dihydropyrimidine-2,4(1H,3H)-dione |
| GT-0543 3 | 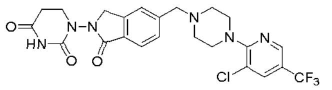 | 1-(5-((4-(3-chloro-5-(trifluoro-methyl)pyridin-2-yl)pipera-zin-1-yl)methyl)-1-oxoisoindo-lin-2-yl)dihydropyrimidi-ne-2,4(1H,3H)-dione | 1-(5-((4-(3-chloro-5-(trifluoro-methyl)pyridin-2-yl)pipera-zin-1-yl)methyl)-1-oxoisoindo-lin-2-yl)dihydropyrimidi-ne-2,4(1H,3H)-dione |
| | 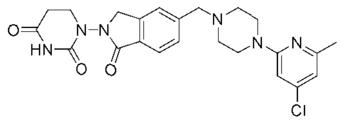 | 1-(5-((4-(4-chloro-6-methylpyridin-2-yl)piperazin-1-yl) methyl)-1-oxoisoindolin-2-yl) dihydropyrimidine-2,4(1H,3H)-dione | 1-(5-((4-(4-chloro-6-methylpyridin-2-yl)piperazin-1-yl) methyl)-1-oxoisoindolin-2-yl) dihydropyrimidine-2,4(1H,3H)-dione |
| GT-0543 4 | 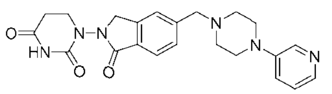 | 1-(1-oxo-5-((4-(pyridin-3-yl)pi-perazin-1-yl)methyl)isoindo-lin-2-yl)dihydropyrimidi-ne-2,4(1H,3H)-dione | 1-(1-oxo-5-((4-(pyridin-3-yl)pi-perazin-1-yl)methyl)isoindo-lin-2-yl)dihydropyrimidi-ne-2,4(1H,3H)-dione |
| GT-0543 5 | 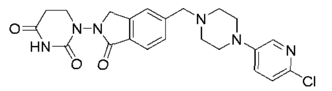 | 1-(5-((4-(6-chloropyridin-3-yl) piperazin-1-yl)methyl)-1-oxoi-soindolin-2-yl)dihydropyrimidi-ne-2,4(1H,3H)-dione | 1-(5-((4-(6-chloropyridin-3-yl) piperazin-1-yl)methyl)-1-oxoi-soindolin-2-yl)dihydropyrimidi-ne-2,4(1H,3H)-dione |
| GT-0543 6 | 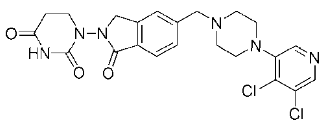 | 1-(5-((4-(4,5-dichloropyridin-3-yl)piperazin-1-yl) methyl)-1-oxoisoindolin-2-yl) dihydropyrimidine-2,4(1H,3H)-dione | 1-(5-((4-(4,5-dichloropyridin-3-yl)piperazin-1-yl) methyl)-1-oxoisoindolin-2-yl) dihydropyrimidine-2,4(1H,3H)-dione |
| GT-0697 4 | 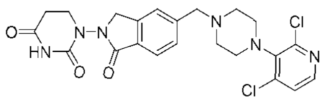 | 1-(5-((4-(2,4-dichloropyridin-3-yl)piperazin-1-yl) methyl)-1-oxoisoindolin-2-yl) dihydropyrimidine-2,4(1H,3H)-dione | 1-(5-((4-(2,4-dichloropyridin-3-yl)piperazin-1-yl) methyl)-1-oxoisoindolin-2-yl) dihydropyrimidine-2,4(1H,3H)-dione |
| GT-0543 7 | 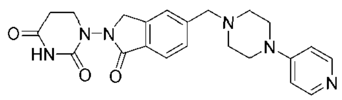 | 1-(1-oxo-5-((4-(pyridin-4-yl)pi-perazin-1-yl)methyl)isoindo-lin-2-yl)dihydropyrimidi-ne-2,4(1H,3H)-dione | 1-(1-oxo-5-((4-(pyridin-4-yl)pi-perazin-1-yl)methyl)isoindo-lin-2-yl)dihydropyrimidi-ne-2,4(1H,3H)-dione |
| GT-0543 8 | 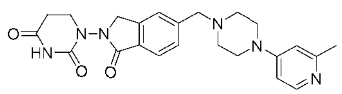 | 1-(5-((4-(2-methylpyridin-4-yl) piperazin-1-yl)methyl)-1-oxoi-soindolin-2-yl)dihydropyrimidi-ne-2,4(1H,3H)-dione | 1-(5-((4-(2-methylpyridin-4-yl) piperazin-1-yl)methyl)-1-oxoi-soindolin-2-yl)dihydropyrimidi-ne-2,4(1H,3H)-dione |
| GT-0543 9 | 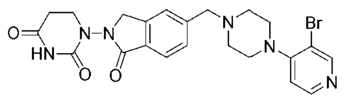 | 1-(5-((4-(3-bromopyridin-4-yl) piperazin-1-yl)methyl)-1-oxoi-soindolin-2-yl)dihydropyrimidi-ne-2,4(1H,3H)-dione | 1-(5-((4-(3-bromopyridin-4-yl) piperazin-1-yl)methyl)-1-oxoi-soindolin-2-yl)dihydropyrimidi-ne-2,4(1H,3H)-dione |
| GT-0569 7 | 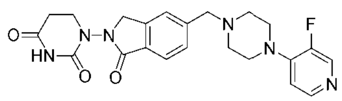 | 1-(5-((4-(3-fluoropyridin-4-yl) piperazin-1-yl)methyl)-1-oxoi-soindolin-2-yl)dihydropyrimidi-ne-2,4(1H,3H)-dione | 1-(5-((4-(3-fluoropyridin-4-yl) piperazin-1-yl)methyl)-1-oxoi-soindolin-2-yl)dihydropyrimidi-ne-2,4(1H,3H)-dione |

(continued)

| Compound No. | Structure of the compounds | The compounds' name | Chinese translation of the compounds' name |
|---|---|---|---|
| GT-0569 8 | 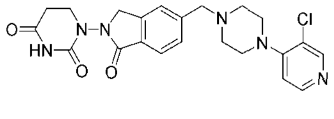 | 1-(5-((4-(3-chloropyridin-4-yl) piperazin-1-yl)methyl)-1-oxoisoindolin-2-yl)dihydropyrimidine-2,4(1H,3H)-dione | 1-(5-((4-(3-chloropyridin-4-yl) piperazin-1-yl)methyl)-1-oxoisoindolin-2-yl)dihydropyrimidine-2,4(1H,3H)-dione |
| GT-0569 9 | 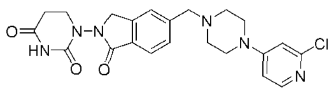 | 1-(5-((4-(2-chloropyridin-4-yl) piperazin-1-yl)methyl)-1-oxoisoindolin-2-yl)dihydropyrimidine-2,4(1H,3H)-dione | 1-(5-((4-(2-chloropyridin-4-yl) piperazin-1-yl)methyl)-1-oxoisoindolin-2-yl)dihydropyrimidine-2,4(1H,3H)-dione |
| GT-0706 9 | 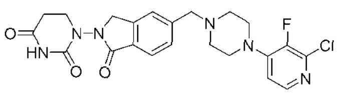 | 1-(5-((4-(2-chloro-3-fluoropyridin-4-yl)piperazin-1-yl) methyl)-1-oxoisoindolin-2-yl) dihydropyrimidine-2,4(1H,3H)-dione | 1-(5-((4-(2-chloro-3-fluoropyridin-4-yl)piperazin-1-yl) methyl)-1-oxoisoindolin-2-yl) dihydropyrimidine-2,4(1H,3H)-dione |
| GT-0707 1 | 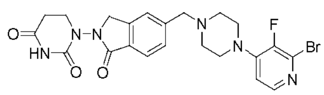 | 1-(5-((4-(2-bromo-3-fluoropyridin-4-yl)piperazin-1-yl) methyl)-1-oxoisoindolin-2-yl) dihydropyrimidine-2,4(1H,3H)-dione | 1-(5-((4-(2-bromo-3-fluoropyridin-4-yl)piperazin-1-yl) methyl)-1-oxoisoindolin-2-yl) dihydropyrimidine-2,4(1H,3H)-dione |
| GT-0707 2 | 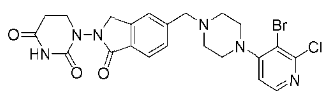 | 1-(5-((4-(3-bromo-2-chloropyridin-4-yl)piperazin-1-yl) methyl)-1-oxoisoindolin-2-yl) dihydropyrimidine-2,4(1H,3H)-dione | 1-(5-((4-(3-bromo-2-chloropyridin-4-yl)piperazin-1-yl) methyl)-1-oxoisoindolin-2-yl) dihydropyrimidine-2,4(1H,3H)-dione |
| GT-0716 9 | 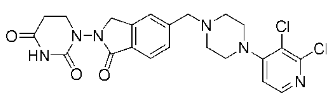 | 1-(5-((4-(2,3-dichloropyridin-4-yl)piperazin-1-yl) methyl)-1-oxoisoindolin-2-yl) dihydropyrimidine-2,4(1H,3H)-dione | 1-(5-((4-(2,3-dichloropyridin-4-yl)piperazin-1-yl) methyl)-1-oxoisoindolin-2-yl) dihydropyrimidine-2,4(1H,3H)-dione |
| | 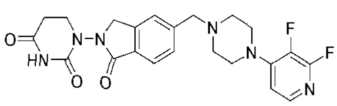 | 1-(5-((4-(2,3-difluoropyridin-4-yl)piperazin-1-yl)methyl)-1-oxoisoindolin-2-yl)dihydropyrimidine-2,4(1H,3H)-dione | 1-(5-((4-(2,3-difluoropyridin-4-yl)piperazin-1-yl)methyl)-1-oxoisoindolin-2-yl)dihydropyrimidine-2,4(1H,3H)-dione |
| | 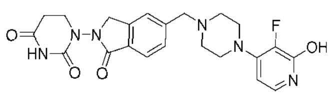 | 1-(5-((4-(3-fluoro-2-hydroxypyridin-4-yl)piperazin-1-yl) methyl)-1-oxoisoindolin-2-yl) dihydropyrimidine-2,4(1H,3H)-dione | 1-(5-((4-(3-fluoro-2-hydroxypyridin-4-yl)piperazin-1-yl) methyl)-1-oxoisoindolin-2-yl) dihydropyrimidine-2,4(1H,3H)-dione |
| GT-0717 0 | 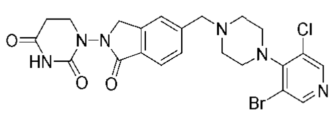 | 1-(5-((4-(3-bromo-5-chloropyridin-4-yl)piperazin-1-yl) methyl)-1-oxoisoindolin-2-yl) dihydropyrimidine-2,4(1H,3H)-dione | 1-(5-((4-(3-bromo-5-chloropyridin-4-yl)piperazin-1-yl) methyl)-1-oxoisoindolin-2-yl) dihydropyrimidine-2,4(1H,3H)-dione |
| GT-0717 1 | 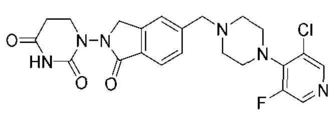 | 1-(5-((4-(3-chloro-5-fluoropyridin-4-yl)piperazin-1-yl) methyl)-1-oxoisoindolin-2-yl) dihydropyrimidine-2,4(1H,3H)-dione | 1-(5-((4-(3-chloro-5-fluoropyridin-4-yl)piperazin-1-yl) methyl)-1-oxoisoindolin-2-yl) dihydropyrimidine-2,4(1H,3H)-dione |

(continued)

| Compound No. | Structure of the compounds | The compounds' name | Chinese translation of the compounds' name |
|---|---|---|---|
| GT-0703 2 | 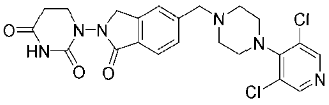 | 1-(5-((4-(3,5-dichloropyridin-4-yl)piperazin-1-yl) methyl)-1-oxoisoindolin-2-yl) dihydropyrimidine-2,4(1H,3H)-dione | 1-(5-((4-(3,5-dichloropyridin-4-yl)piperazin-1-yl) methyl)-1-oxoisoindolin-2-yl) dihydropyrimidine-2,4(1H,3H)-dione |
| GT-0703 3 | 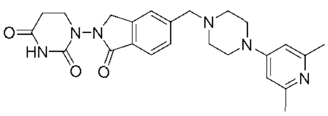 | 1-(5-((4-(2,6-dimethylpyridin-4-yl)piperazin-1-yl) methyl)-1-oxoisoindolin-2-yl) dihydropyrimidine-2,4(1H,3H)-dione | 1-(5-((4-(2,6-dimethylpyridin-4-yl)piperazin-1-yl) methyl)-1-oxoisoindolin-2-yl) dihydropyrimidine-2,4(1H,3H)-dione |
| GT-0704 0 | 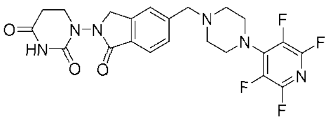 | 1-(1-oxo-5-((4-(perfluoropyridin-4-yl)piperazin-1-yl)methyl) isoindolin-2-yl)dihydropyrimidine-2,4(1H,3H)-dione | 1-(1-oxo-5-((4-(perfluoropyridin-4-yl)piperazin-1-yl)methyl) isoindolin-2-yl)dihydropyrimidine-2,4(1H,3H)-dione |
| | 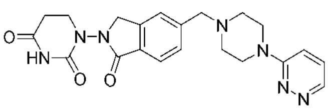 | 1-(1-oxo-5-((4-(pyridazin-3-yl) piperazin-1-yl)methyl)isoindolin-2-yl)dihydropyrimidine-2,4(1H,3H)-dione | 1-(1-oxo-5-((4-(pyridazin-3-yl) piperazin-1-yl)methyl)isoindolin-2-yl)dihydropyrimidine-2,4(1H,3H)-dione |
| GT-0704 2 | 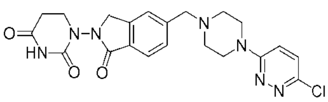 | 1-(5-((4-(6-chloropyridazin-3-yl)piperazin-1-yl)methyl)-1-oxoisoindolin-2-yl)dihydropyrimidine-2,4(1H,3H)-dione | 1-(5-((4-(6-chloropyridazin-3-yl)piperazin-1-yl)methyl)-1-oxoisoindolin-2-yl)dihydropyrimidine-2,4(1H,3H)-dione |
| GT-0715 9 | 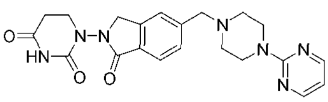 | 1-(1-oxo-5-((4-(pyrimidin-2-yl) piperazin-1-yl)methyl)isoindolin-2-yl)dihydropyrimidine-2,4(1H,3H)-dione | 1-(1-oxo-5-((4-(pyrimidin-2-yl) piperazin-1-yl)methyl)isoindolin-2-yl)dihydropyrimidine-2,4(1H,3H)-dione |
| GT-0716 0 | 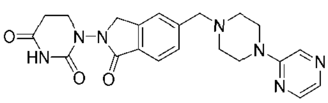 | 1-(1-oxo-5-((4-(pyrazin-2-yl) piperazin-1-yl)methyl)isoindolin-2-yl)dihydropyrimidine-2,4(1H,3H)-dione | 1-(1-oxo-5-((4-(pyrazin-2-yl) piperazin-1-yl)methyl)isoindolin-2-yl)dihydropyrimidine-2,4(1H,3H)-dione |
| GT-0702 8 | 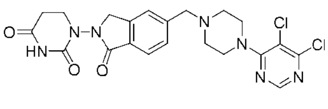 | 1-(5-((4-(5,6-dichloropyrimidin-4-yl)piperazin-1-yl) methyl)-1-oxoisoindolin-2-yl) dihydropyrimidine-2,4(1H,3H)-dione | 1-(5-((4-(5,6-dichloropyrimidin-4-yl)piperazin-1-yl) methyl)-1-oxoisoindolin-2-yl) dihydropyrimidine-2,4(1H,3H)-dione |
| GT-0702 9 | 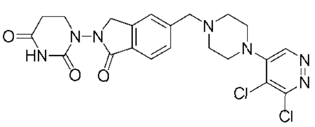 | 1-(5-((4-(5,6-dichloropyridazin-4-yl)piperazin-1-yl) methyl)-1-oxoisoindolin-2-yl) dihydropyrimidine-2,4(1H,3H)-dione | 1-(5-((4-(5,6-dichloropyridazin-4-yl)piperazin-1-yl) methyl)-1-oxoisoindolin-2-yl) dihydropyrimidine-2,4(1H,3H)-dione |
| GT-0703 0 | 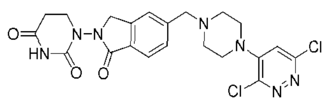 | 1-(5-((4-(3,6-dichloropyridazin-4-yl)piperazin-1-yl) methyl)-1-oxoisoindolin-2-yl) dihydropyrimidine-2,4(1H,3H)-dione | 1-(5-((4-(3,6-dichloropyridazin-4-yl)piperazin-1-yl) methyl)-1-oxoisoindolin-2-yl) dihydropyrimidine-2,4(1H,3H)-dione |
| GT-0703 1 | 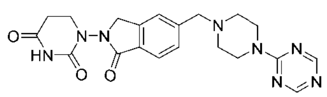 | 1-(5-((4-(1,3,5-triazin-2-yl)piperazin-1-yl)methyl)-1-oxoisoindolin-2-yl)dihydropyrimidine-2,4(1H,3H)-dione | 1-(5-((4-(1,3,5-triazin-2-yl)piperazin-1-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0638 8 | 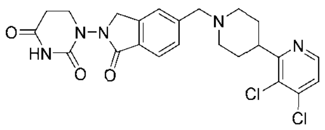 | 1-(5-((4-(3,4-dichloropyridin-2-yl)piperidin-1-yl)methyl)-1-oxoisoindolin-2-yl)dihydropyrimidine-2,4(1H,3H)-dione | 1-(5-((4-(3,4-dichloropyridin-2-yl)piperidin-1-yl)methyl)-1-oxoisoindolin-2-yl)dihydropyrimidine-2,4(1H,3H)-dione |
| GT-0639 0 | 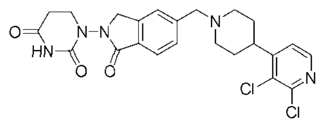 | 1-(5-((4-(2,3-dichloropyridin-4-yl)piperidin-1-yl)methyl)-1-oxoisoindolin-2-yl)dihydropyrimidine-2,4(1H,3H)-dione | 1-(5-((4-(2,3-dichloropyridin-4-yl)piperidin-1-yl)methyl)-1-oxoisoindolin-2-yl)dihydropyrimidine-2,4(1H,3H)-dione |
| GT-0639 6 | 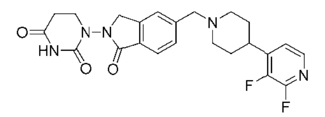 | 1-(5-((4-(2,3-difluoropyridin-4-yl)piperidin-1-yl)methyl)-1-oxoisoindolin-2-yl)dihydropyrimidine-2,4(1H,3H)-dione | 1-(5-((4-(2,3-difluoropyridin-4-yl)piperidin-1-yl)methyl)-1-oxoisoindolin-2-yl)dihydropyrimidine-2,4(1H,3H)-dione |
| | 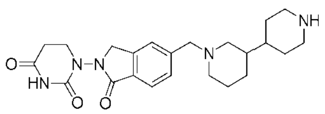 | 1-(5-([3,4'-bipiperidin]-1-ylmethyl)-1-oxoisoindolin-2-yl)dihydropyrimidine-2,4(1H,3H)-dione | 1-(5-([3,4'-bipiperidin]-1-ylmethyl)-1-oxoisoindolin-2-yl)dihydropyrimidine-2,4(1H,3H)-dione |
| GT-0639 2 | 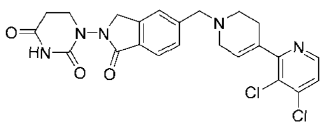 | 1-(5-((3,4-dichloro-3',6'-dihydro-[2,4'-bipyridin]-1'(2'H)-yl)methyl)-1-oxoisoindolin-2-yl)dihydropyrimidine-2,4(1H,3H)-dione | 1-(5-((3,4-dichloro-3',6'-dihydro-[2,4'-bipyridin]-1'(2'H)-yl)methyl)-1-oxoisoindolin-2-yl)dihydropyrimidine-2,4(1H,3H)-dione |
| GT-0639 3 | 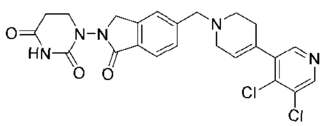 | 1-(5-((4,5-dichloro-3',6'-dihydro-[3,4'-bipyridin]-1'(2'H)-yl)methyl)-1-oxoisoindolin-2-yl)dihydropyrimidine-2,4(1H,3H)-dione | 1-(5-((4,5-dichloro-3',6'-dihydro-[3,4'-bipyridin]-1'(2'H)-yl)methyl)-1-oxoisoindolin-2-yl)dihydropyrimidine-2,4(1H,3H)-dione |
| GT-0639 4 | 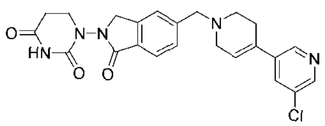 | 1-(5-((5-chloro-3',6'-dihydro-[3,4'-bipyridin]-1'(2'H)-yl)methyl)-1-oxoisoindolin-2-yl)dihydropyrimidine-2,4(1H,3H)-dione | 1-(5-((5-chloro-3',6'-dihydro-[3,4'-bipyridin]-1'(2'H)-yl)methyl)-1-oxoisoindolin-2-yl)dihydropyrimidine-2,4(1H,3H)-dione |
| GT-0743 2 | 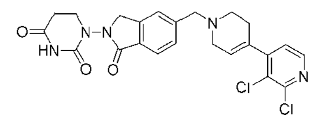 | 1-(5-((2',3'-dichloro-3,6-dihydro-[4,4'-bipyridin]-1(2H)-yl)methyl)-1-oxoisoindolin-2-yl)dihydropyrimidine-2,4(1H,3H)-dione | 1-(5-((2',3'-dichloro-3,6-dihydro-[4,4'-bipyridin]-1(2H)-yl)methyl)-1-oxoisoindolin-2-yl)dihydropyrimidine-2,4(1H,3H)-dione |
| GT-0639 5 | 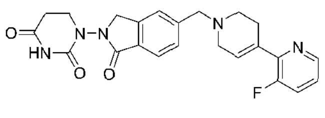 | 1-(5-((3-fluoro-3',6'-dihydro-[2,4'-bipyridin]-1'(2'H)-yl)methyl)-1-oxoisoindolin-2-yl)dihydropyrimidine-2,4(1H,3H)-dione | 1-(5-((3-fluoro-3',6'-dihydro-[2,4'-bipyridin]-1'(2'H)-yl)methyl)-1-oxoisoindolin-2-yl)dihydropyrimidine-2,4(1H,3H)-dione |
| | 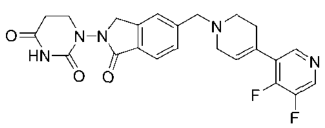 | 1-(5-((4,5-difluoro-3',6'-dihydro-[3,4'-bipyridin]-1'(2'H)-yl)methyl)-1-oxoisoindolin-2-yl)dihydropyrimidine-2,4(1H,3H)-dione | 1-(5-((4,5-difluoro-3',6'-dihydro-[3,4'-bipyridin]-1'(2'H)-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0639 7 | 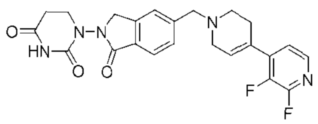 | 1-(5-((2',3'-difluoro-3,6-dihydro-[4,4'-bipyridin]-1(2H)-yl) methyl)-1-oxoisoindolin-2-yl) dihydropyrimidine-2,4(1H,3H)-dione | 1-(5-((2',3'-difluoro-3,6-dihydro-[4,4'-bipyridin]-1(2H)-yl) methyl)-1-oxoisoindolin-2-yl) dihydropyrimidine-2,4(1H,3H)-dione |
| GT-0516 2 | 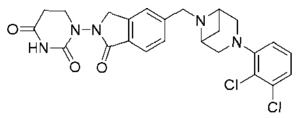 | 1-(5-((3-(2,3-dichlorophenyl)-3,6-diazabicyclo[3.1.1] heptan-6-yl)methyl)-1-oxoi-soindolin-2-yl)dihydropyrimidi-ne-2,4(1H,3H)-dione | 1-(5-((3-(2,3-dichlorophenyl)-3,6-diazabicyclo[3.1.1] heptan-6-yl)methyl)-1-oxoi-soindolin-2-yl)dihydropyrimidi-ne-2,4(1H,3H)-dione |
| GT-0742 6 | 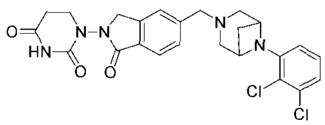 | 1-(5-((6-(2,3-dichlorophenyl)-3,6-diazabicyclo[3.1.1] heptan-3-yl)methyl)-1-oxoi-soindolin-2-yl)dihydropyrimidi-ne-2,4(1H,3H)-dione | 1-(5-((6-(2,3-dichlorophenyl)-3,6-diazabicyclo[3.1.1] heptan-3-yl)methyl)-1-oxoi-soindolin-2-yl)dihydropyrimidi-ne-2,4(1H,3H)-dione |
| GT-0742 7 | 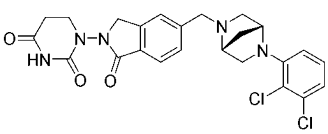 | 1-(5-(((1R,4R)-5-(2,3-dichlorophenyl)-2,5-diazabicyclo [2.2.1]heptan-2-yl)methyl)-1-oxoisoindolin-2-yl)dihydropyrimidine-2,4(1H,3H)-dione | 1-(5-(((1R,4R)-5-(2,3-dichlorophenyl)-2,5-diazabicyclo [2.2.1]heptan-2-yl)methyl)-1-oxoisoindolin-2-yl)dihydropyrimidine-2,4(1H,3H)-dione |
| GT-0516 3 | 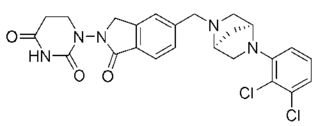 | 1-(5-(((1S,4S)-5-(2,3-dichlorophenyl)-2,5-diazabicyclo [2.2.1]heptan-2-yl)methyl)-1-oxoisoindolin-2-yl)dihydropyrimidine-2,4(1H,3H)-dione | 1-(5-(((1S,4S)-5-(2,3-dichlorophenyl)-2,5-diazabicyclo [2.2.1]heptan-2-yl)methyl)-1-oxoisoindolin-2-yl)dihydropyrimidine-2,4(1H,3H)-dione |
| GT-0742 8 | 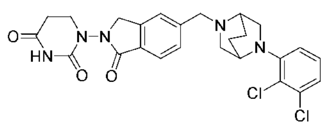 | 1-(5-((5-(2,3-dichlorophenyl)-2,5-diazabicyclo[2.2.2]octan-2-yl)methyl)-1-oxoisoindo-lin-2-yl)dihydropyrimidi-ne-2,4(1H,3H)-dione | 1-(5-((5-(2,3-dichlorophenyl)-2,5-diazabicyclo[2.2.2]octan-2-yl)methyl)-1-oxoisoindo-lin-2-yl)dihydropyrimidi-ne-2,4(1H,3H)-dione |
| GT-0742 9 | 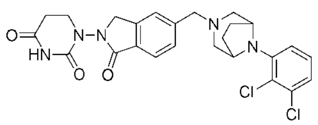 | 1-(5-((8-(2,3-dichlorophenyl)-3,8-diazabicyclo[3.2.1]octan-3-yl)methyl)-1-oxoisoindo-lin-2-yl)dihydropyrimidi-ne-2,4(1H,3H)-dione | 1-(5-((8-(2,3-dichlorophenyl)-3,8-diazabicyclo[3.2.1]octan-3-yl)methyl)-1-oxoisoindo-lin-2-yl)dihydropyrimidi-ne-2,4(1H,3H)-dione |
| GT-0743 0 | 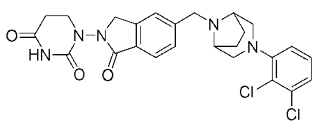 | 1-(5-((3-(2,3-dichlorophenyl)-3,8-diazabicyclo[3.2.1]octan-8-yl)methyl)-1-oxoisoindo-lin-2-yl)dihydropyrimidi-ne-2,4(1H,3H)-dione | 1-(5-((3-(2,3-dichlorophenyl)-3,8-diazabicyclo[3.2.1]octan-8-yl)methyl)-1-oxoisoindo-lin-2-yl)dihydropyrimidi-ne-2,4(1H,3H)-dione |
| GT-0743 1 | 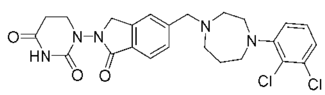 | 1-(5-((4-(2,3-dichlorophenyl)-1,4-diazepan-1-yl) methyl)-1-oxoisoindolin-2-yl) dihydropyrimidine-2,4(1H,3H)-dione | 1-(5-((4-(2,3-dichlorophenyl)-1,4-diazepan-1-yl) methyl)-1-oxoisoindolin-2-yl) dihydropyrimidine-2,4(1H,3H)-dione |
| GT-0486 4 | 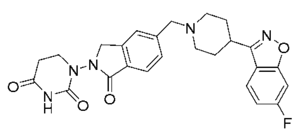 | 1-(5-((4-(6-fluorobenzo[d]isoxazol-3-yl)piperidin-1-yl) methyl)-1-oxoisoindolin-2-yl) dihydropyrimidine-2,4(1H,3H)-dione | 1-(5-((4-(6-fluorobenzo[d]isoxazol-3-yl)piperidin-1-yl) methyl)-1-oxoisoindolin-2-yl) dihydropyrimidine-2,4(1H,3H)-dione |

(continued)

| Compound No. | Structure of the compounds | The compounds' name | Chinese translation of the compounds' name |
|---|---|---|---|
| GT-0764 2 | 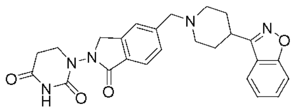 | 1-(5-((4-(benzo[d]isoxazol-3-yl)piperidin-1-yl)methyl)-1-oxoisoindolin-2-yl)dihydropyrimidine-2,4(1H,3H)-dione | 1-(5-((4-(benzo[d]isoxazol-3-yl)piperidin-1-yl)methyl)-1-oxoisoindolin-2-yl)dihydropyrimidine-2,4(1H,3H)-dione |
| GT-0749 5 | 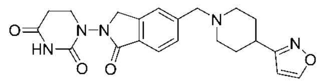 | 1-(5-((4-(isoxazol-3-yl)piperidin-1-yl)methyl)-1-oxoisoindolin-2-yl)dihydropyrimidine-2,4(1H,3H)-dione | 1-(5-((4-(isoxazol-3-yl)piperidin-1-yl)methyl)-1-oxoisoindolin-2-yl)dihydropyrimidine-2,4(1H,3H)-dione |
| GT-0749 6 | 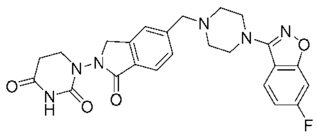 | 1-(5-((4-(6-fluorobenzo[d]isoxazol-3-yl)piperazin-1-yl)methyl)-1-oxoisoindolin-2-yl)dihydropyrimidine-2,4(1H,3H)-dione | 1-(5-((4-(6-fluorobenzo[d]isoxazol-3-yl)piperazin-1-yl)methyl)-1-oxoisoindolin-2-yl)dihydropyrimidine-2,4(1H,3H)-dione |
| GT-0749 7 | 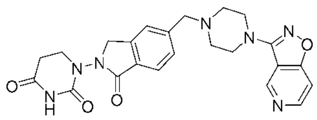 | 1-(5-((4-(isoxazolo[4,5-c]pyridin-3-yl)piperazin-1-yl)methyl)-1-oxoisoindolin-2-yl)dihydropyrimidine-2,4(1H,3H)-dione | 1-(5-((4-(isoxazolo[4,5-c]pyridin-3-yl)piperazin-1-yl)methyl)-1-oxoisoindolin-2-yl)dihydropyrimidine-2,4(1H,3H)-dione |
| GT-0750 0 | 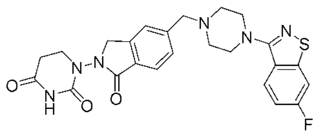 | 1-(5-((4-(6-fluorobenzo[d]isothiazol-3-yl)piperazin-1-yl)methyl)-1-oxoisoindolin-2-yl)dihydropyrimidine-2,4(1H,3H)-dione | 1-(5-((4-(6-fluorobenzo[d]isothiazol-3-yl)piperazin-1-yl)methyl)-1-oxoisoindolin-2-yl)dihydropyrimidine-2,4(1H,3H)-dione |
| GT-0754 5 | 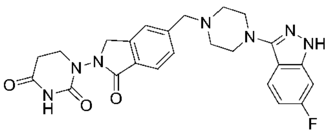 | 1-(5-((4-(6-fluoro-1H-indazol-3-yl)piperazin-1-yl)methyl)-1-oxoisoindolin-2-yl)dihydropyrimidine-2,4(1H,3H)-dione | 1-(5-((4-(6-fluoro-1H-indazol-3-yl)piperazin-1-yl)methyl)-1-oxoisoindolin-2-yl)dihydropyrimidine-2,4(1H,3H)-dione |
| GT-0750 2 | 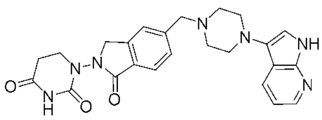 | 1-(5-((4-(1H-pyrrolo[2,3-b]pyridin-3-yl)piperazin-1-yl)methyl)-1-oxoisoindolin-2-yl)dihydropyrimidine-2,4(1H,3H)-dione | 1-(5-((4-(1H-pyrrolo[2,3-b]pyridin-3-yl)piperazin-1-yl)methyl)-1-oxoisoindolin-2-yl)dihydropyrimidine-2,4(1H,3H)-dione |
| GT-0516 4 | 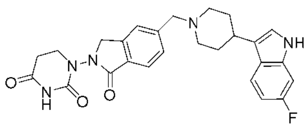 | 1-(5-((4-(6-fluoro-1H-indol-3-yl)piperidin-1-yl)methyl)-1-oxoisoindolin-2-yl)dihydropyrimidine-2,4(1H,3H)-dione | 1-(5-((4-(6-fluoro-1H-indol-3-yl)piperidin-1-yl)methyl)-1-oxoisoindolin-2-yl)dihydropyrimidine-2,4(1H,3H)-dione |
| GT-0750 8 | 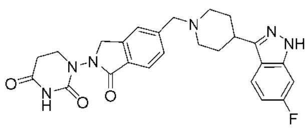 | 1-(5-((4-(6-fluoro-1H-indazol-3-yl)piperidin-1-yl)methyl)-1-oxoisoindolin-2-yl)dihydropyrimidine-2,4(1H,3H)-dione | 1-(5-((4-(6-fluoro-1H-indazol-3-yl)piperidin-1-yl)methyl)-1-oxoisoindolin-2-yl)dihydropyrimidine-2,4(1H,3H)-dione |
| GT-0757 5 | 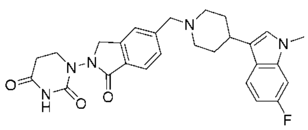 | 1-(5-((4-(6-fluoro-1-methyl-1H-indol-3-yl)piperidin-1-yl)methyl)-1-oxoisoindolin-2-yl)dihydropyrimidine-2,4(1H,3H)-dione | 1-(5-((4-(6-fluoro-1-methyl-1H-indol-3-yl)piperidin-1-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0516 5 | 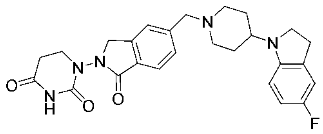 | 1-(5-((4-(5-fluoroindolin-1-yl) piperidin-1-yl)methyl)-1-oxoisoindolin-2-yl)dihydropyrimidine-2,4(1H,3H)-dione | 1-(5-((4-(5-fluoroindolin-1-yl) piperidin-1-yl)methyl)-1-oxoisoindolin-2-yl)dihydropyrimidine-2,4(1H,3H)-dione |
| GT-0750 3 | 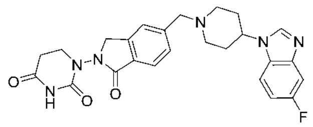 | 1-(5-((4-(5-fluoro-1H-benzo[d] imidazol-1-yl)piperidin-1-yl) methyl)-1-oxoisoindolin-2-yl) dihydropyrimidine-2,4(1H,3H)-dione | 1-(5-((4-(5-fluoro-1H-benzo[d] imidazol-1-yl)piperidin-1-yl) methyl)-1-oxoisoindolin-2-yl) dihydropyrimidine-2,4(1H,3H)-dione |
| GT-0757 9 | 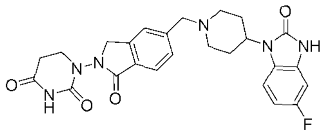 | 1-(5-((4-(5-fluoro-2-oxo-2,3-dihydro-1H-benzo[d]imidazol-1-yl)piperidin-1-yl) methyl)-1-oxoisoindolin-2-yl) dihydropyrimidine-2,4(1H,3H)-dione | 1-(5-((4-(5-fluoro-2-oxo-2,3-dihydro-1H-benzo[d]imidazol-1-yl)piperidin-1-yl) methyl)-1-oxoisoindolin-2-yl) dihydropyrimidine-2,4(1H,3H)-dione |
| GT-0757 6 | 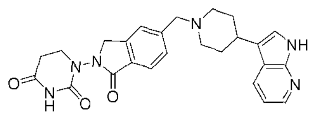 | 1-(5-((4-(1H-pyrrolo[2,3-b]pyridin-3-yl)piperidin-1-yl) methyl)-1-oxoisoindolin-2-yl) dihydropyrimidine-2,4(1H,3H)-dione | 1-(5-((4-(1H-pyrrolo[2,3-b]pyridin-3-yl)piperidin-1-yl) methyl)-1-oxoisoindolin-2-yl) dihydropyrimidine-2,4(1H,3H)-dione |
| GT-0757 7 | 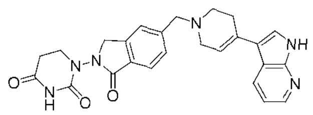 | 1-(5-((4-(1H-pyrrolo[2,3-b]pyridin-3-yl)-3,6-dihydropyridin-1(2H)-yl)methyl)-1-oxoisoindolin-2-yl)dihydropyrimidine-2,4(1H,3H)-dione | 1-(5-((4-(1H-pyrrolo[2,3-b]pyridin-3-yl)-3,6-dihydropyridin-1(2H)-yl)methyl)-1-oxoisoindolin-2-yl)dihydropyrimidine-2,4(1H,3H)-dione |
| GT-0757 4 | 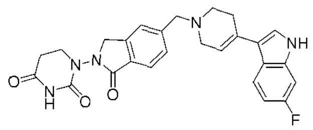 | 1-(5-((4-(6-fluoro-1H-indol-3-yl)-3,6-dihydropyridin-1(2H)-yl)methyl)-1-oxoisoindolin-2-yl)dihydropyrimidine-2,4(1H,3H)-dione | 1-(5-((4-(6-fluoro-1H-indol-3-yl)-3,6-dihydropyridin-1(2H)-yl)methyl)-1-oxoisoindolin-2-yl)dihydropyrimidine-2,4(1H,3H)-dione |
| GT-0516 7 | 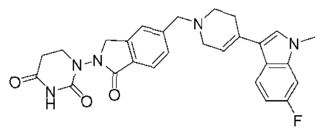 | 1-(5-((4-(6-fluoro-1-methyl-1H-indol-3-yl)-3,6-dihydropyridin-1(2H)-yl) methyl)-1-oxoisoindolin-2-yl) dihydropyrimidine-2,4(1H,3H)-dione | 1-(5-((4-(6-fluoro-1-methyl-1H-indol-3-yl)-3,6-dihydropyridin-1(2H)-yl) methyl)-1-oxoisoindolin-2-yl) dihydropyrimidine-2,4(1H,3H)-dione |
| GT-0757 8 | 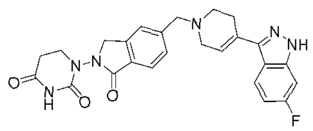 | 1-(5-((4-(6-fluoro-1H-indazol-3-yl)-3,6-dihydropyridin-1(2H)-yl)methyl)-1-oxoisoindolin-2-yl)dihydropyrimidine-2,4(1H,3H)-dione | 1-(5-((4-(6-fluoro-1H-indazol-3-yl)-3,6-dihydropyridin-1(2H)-yl)methyl)-1-oxoisoindolin-2-yl)dihydropyrimidine-2,4(1H,3H)-dione |
| GT-0758 4 | 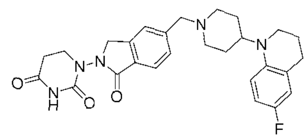 | 1-(5-((4-(6-fluoro-3,4-dihydroquinolin-1(2H)-yl)piperidin-1-yl)methyl)-1-oxoisoindolin-2-yl)dihydropyrimidine-2,4(1H,3H)-dione | 1-(5-((4-(6-fluoro-3,4-dihydroquinolin-1(2H)-yl)piperidin-1-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0758 5 | 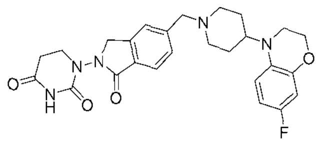 | 1-(5-((4-(7-fluoro-2,3-dihydro-4H-benzo[b][1,4]oxazin-4-yl)piperidin-1-yl)methyl)-1-oxoisoindolin-2-yl)dihydropyrimidine-2,4(1H,3H)-dione | 1-(5-((4-(7-fluoro-2,3-dihydro-4H-benzo[b][1,4]oxazin-4-yl)piperidin-1-yl)methyl)-1-oxoisoindolin-2-yl)dihydropyrimidine-2,4(1H,3H)-dione |
| GT-0758 6 | 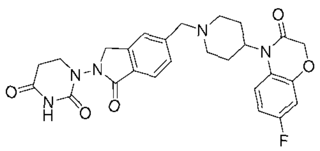 | 1-(5-((4-(7-fluoro-3-oxo-2,3-dihydro-4H-benzo[b][1,4]oxazin-4-yl)piperidin-1-yl) methyl)-1-oxoisoindolin-2-yl) dihydropyrimidine-2,4(1H,3H)-dione | 1-(5-((4-(7-fluoro-3-oxo-2,3-dihydro-4H-benzo[b][1,4]oxazin-4-yl)piperidin-1-yl) methyl)-1-oxoisoindolin-2-yl) dihydropyrimidine-2,4(1H,3H)-dione |
| GT-0758 7 | 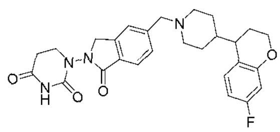 | 1-(5-((4-(7-fluorochroman-4-yl)piperidin-1-yl)methyl)-1-oxoisoindolin-2-yl)dihydropyrimidine-2,4(1H,3H)-dione | 1-(5-((4-(7-fluorochroman-4-yl)piperidin-1-yl)methyl)-1-oxoisoindolin-2-yl)dihydropyrimidine-2,4(1H,3H)-dione |
| GT-0758 8 | 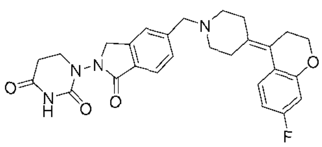 | 1-(5-((4-(7-fluorochroman-4-ylidene)piperidin-1-yl) methyl)-1-oxoisoindolin-2-yl) dihydropyrimidine-2,4(1H,3H)-dione | 1-(5-((4-(7-fluorochroman-4-ylidene)piperidin-1-yl) methyl)-1-oxoisoindolin-2-yl) dihydropyrimidine-2,4(1H,3H)-dione |
| GT-0758 9 | 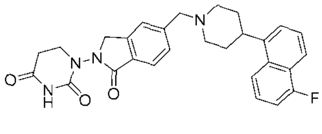 | 1-(5-((4-(5-fluoronaphthalen-1-yl)piperidin-1-yl) methyl)-1-oxoisoindolin-2-yl) dihydropyrimidine-2,4(1H,3H)-dione | 1-(5-((4-(5-fluoronaphthalen-1-yl)piperidin-1-yl) methyl)-1-oxoisoindolin-2-yl) dihydropyrimidine-2,4(1H,3H)-dione |
| GT-0764 5 | 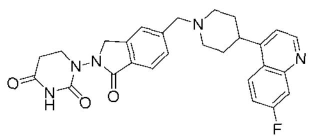 | 1-(5-((4-(7-fluoroquinolin-4-yl) piperidin-1-yl)methyl)-1-oxoisoindolin-2-yl)dihydropyrimidine-2,4(1H,3H)-dione | 1-(5-((4-(7-fluoroquinolin-4-yl) piperidin-1-yl)methyl)-1-oxoisoindolin-2-yl)dihydropyrimidine-2,4(1H,3H)-dione |
| GT-0764 4 | 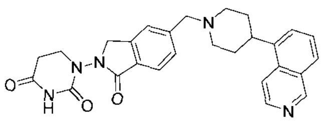 | 1-(5-((4-(isoquinolin-5-yl)piperidin-1-yl)methyl)-1-oxoisoindolin-2-yl)dihydropyrimidine-2,4(1H,3H)-dione | 1-(5-((4-(isoquinolin-5-yl)piperidin-1-yl)methyl)-1-oxoisoindolin-2-yl)dihydropyrimidine-2,4(1H,3H)-dione |
| GT-0759 0 | 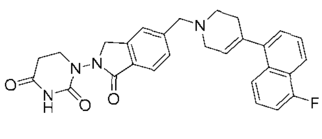 | 1-(5-((4-(5-fluoronaphthalen-1-yl)-3,6-dihydropyridin-1(2H)-yl)methyl)-1-oxoisoindolin-2-yl)dihydropyrimidine-2,4(1H,3H)-dione | 1-(5-((4-(5-fluoronaphthalen-1-yl)-3,6-dihydropyridin-1(2H)-yl)methyl)-1-oxoisoindolin-2-yl)dihydropyrimidine-2,4(1H,3H)-dione |
| GT-0516 6 | 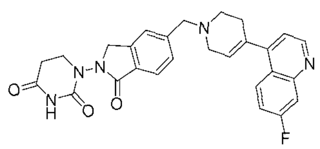 | 1-(5-((4-(7-fluoroquinolin-4-yl)-3,6-dihydropyridin-1(2H)-yl)methyl)-1-oxoisoindolin-2-yl)dihydropyrimidine-2,4(1H,3H)-dione | 1-(5-((4-(7-fluoroquinolin-4-yl)-3,6-dihydropyridin-1(2H)-yl)methyl)-1-oxoisoindolin-2-yl)dihydropyrimidine-2,4(1H,3H)-dione |
| | 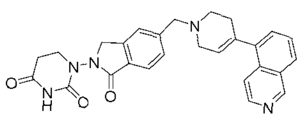 | 1-(5-((4-(isoquinolin-5-yl)-3,6-dihydropyridin-1(2H)-yl) methyl)-1-oxoisoindolin-2-yl) dihydropyrimidine-2,4(1H,3H)-dione | 1-(5-((4-(isoquinolin-5-yl)-3,6-dihydropyridin-1(2H)-yl) methyl)-1-oxoisoindolin-2-yl) dihydropyrimidine-2,4(1H,3H)-dione |

(continued)

| Compound No. | Structure of the compounds | The compounds' name | Chinese translation of the compounds' name |
|---|---|---|---|
| GT-0769 0 | 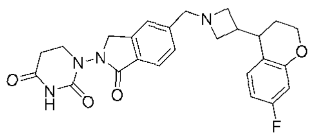 | 1-(5-((3-(7-fluorochroman-4-yl)azetidin-1-yl)methyl)-1-oxoisoindolin-2-yl)dihydropyrimidine-2,4(1H,3H)-dione | 1-(5-((3-(7-fluorochroman-4-yl)azetidin-1-yl)methyl)-1-oxoisoindolin-2-yl)dihydropyrimidine-2,4(1H,3H)-dione |
| GT-0516 8 | 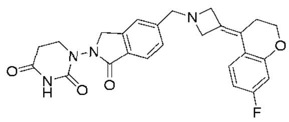 | 1-(5-((3-(7-fluorochroman-4-ylidene)azetidin-1-yl) methyl)-1-oxoisoindolin-2-yl) dihydropyrimidine-2,4(1H,3H)-dione | 1-(5-((3-(7-fluorochroman-4-ylidene)azetidin-1-yl) methyl)-1-oxoisoindolin-2-yl) dihydropyrimidine-2,4(1H,3H)-dione |
| GT-0771 1 | 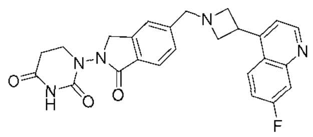 | 1-(5-((3-(7-fluoroquinolin-4-yl) azetidin-1-yl)methyl)-1-oxoisoindolin-2-yl)dihydropyrimidine-2,4(1H,3H)-dione | 1-(5-((3-(7-fluoroquinolin-4-yl) azetidin-1-yl)methyl)-1-oxoisoindolin-2-yl)dihydropyrimidine-2,4(1H,3H)-dione |
| GT-0516 9 | 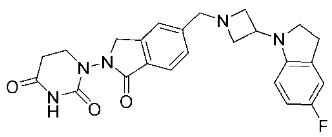 | 1-(5-((3-(5-fluoroindolin-1-yl) azetidin-1-yl)methyl)-1-oxoisoindolin-2-yl)dihydropyrimidine-2,4(1H,3H)-dione | 1-(5-((3-(5-fluoroindolin-1-yl) azetidin-1-yl)methyl)-1-oxoisoindolin-2-yl)dihydropyrimidine-2,4(1H,3H)-dione |
| GT-0772 3 | 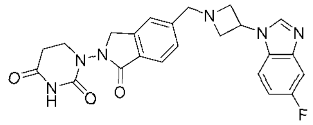 | 1-(5-((3-(5-fluoro-1H-benzo[d] imidazol-1-yl)azetidin-1-yl) methyl)-1-oxoisoindolin-2-yl) dihydropyrimidine-2,4(1H,3H)-dione | 1-(5-((3-(5-fluoro-1H-benzo[d] imidazol-1-yl)azetidin-1-yl) methyl)-1-oxoisoindolin-2-yl) dihydropyrimidine-2,4(1H,3H)-dione |
| GT-0805 7 | 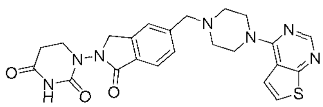 | 1-(1-oxo-5-((4-(thieno[2,3-d] pyrimidin-4-yl)piperazin-1-yl) methyl)isoindolin-2-yl)dihydropyrimidine-2,4(1H,3H)-dione | 1-(1-oxo-5-((4-(thieno[2,3-d] pyrimidin-4-yl)piperazin-1-yl) methyl)isoindolin-2-yl)dihydropyrimidine-2,4(1H,3H)-dione |
| GT-0805 8 | 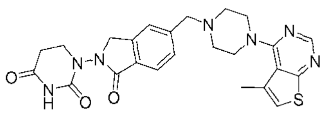 | 1-(5-((4-(5-methylthieno[2,3-d]pyrimidin-4-yl)piperazin-1-yl)methyl)-1-oxoisoindolin-2-yl)dihydropyrimidine-2,4(1H,3H)-dione | 1-(5-((4-(5-methylthieno[2,3-d]pyrimidin-4-yl)piperazin-1-yl)methyl)-1-oxoisoindolin-2-yl)dihydropyrimidine-2,4(1H,3H)-dione |
| GT-0805 9 | 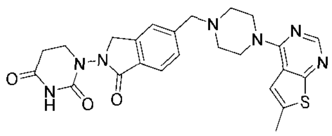 | 1-(5-((4-(6-methylthieno[2,3-d]pyrimidin-4-yl)piperazin-1-yl)methyl)-1-oxoisoindolin-2-yl)dihydropyrimidine-2,4(1H,3H)-dione | 1-(5-((4-(6-methylthieno[2,3-d]pyrimidin-4-yl)piperazin-1-yl)methyl)-1-oxoisoindolin-2-yl)dihydropyrimidine-2,4(1H,3H)-dione |
| GT-0806 0 | 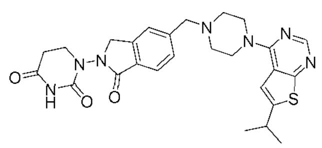 | 1-(5-((4-(6-isopropylthieno [2,3-d]pyrimidin-4-yl)piperazin-1-yl)methyl)-1-oxoisoindolin-2-yl)dihydropyrimidine-2,4(1H,3H)-dione | 1-(5-((4-(6-isopropylthieno [2,3-d]pyrimidin-4-yl)piperazin-1-yl)methyl)-1-oxoisoindolin-2-yl)dihydropyrimidine-2,4(1H,3H)-dione |
| GT-0806 1 | 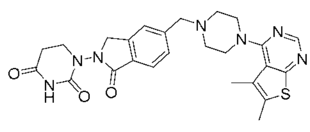 | 1-(5-((4-(5,6-dimethylthieno [2,3-d]pyrimidin-4-yl)piperazin-1-yl)methyl)-1-oxoisoindolin-2-yl)dihydropyrimidine-2,4(1H,3H)-dione | 1-(5-((4-(5,6-dimethylthieno [2,3-d]pyrimidin-4-yl)piperazin-1-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0806 4 | 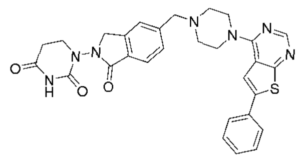 | 1-(1-oxo-5-((4-(6-phenylthieno[2,3-d]pyrimidin-4-yl)piperazin-1-yl)methyl)isoindolin-2-yl)dihydropyrimidine-2,4(1H,3H)-dione | 1-(1-oxo-5-((4-(6-phenylthieno[2,3-d]pyrimidin-4-yl)piperazin-1-yl)methyl)isoindolin-2-yl) dihydropyrimidine-2,4(1H,3H)-dione |
| GT-0806 3 | 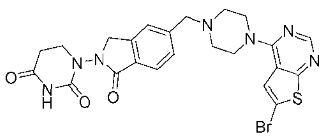 | 1-(5-((4-(6-bromothieno[2,3-d] pyrimidin-4-yl)piperazin-1-yl) methyl)-1-oxoisoindolin-2-yl) dihydropyrimidine-2,4(1H,3H)-dione | 1-(5-((4-(6-bromothieno[2,3-d] pyrimidin-4-yl)piperazin-1-yl) methyl)-1-oxoisoindolin-2-yl) dihydropyrimidine-2,4(1H,3H)-dione |
| GT-0806 5 | 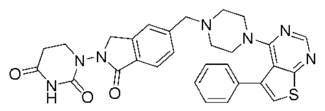 | 1-(1-oxo-5-((4-(5-phenylthieno[2,3-d]pyrimidin-4-yl)piperazin-1-yl)methyl)isoindolin-2-yl)dihydropyrimidine-2,4(1H,3H)-dione | 1-(1-oxo-5-((4-(5-phenylthieno[2,3-d]pyrimidin-4-yl)piperazin-1-yl)methyl)isoindolin-2-yl) dihydropyrimidine-2,4(1H,3H)-dione |
| GT-0807 7 | 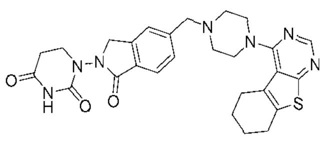 | 1-(1-oxo-5-((4-(5,6,7,8-tetrahydrobenzo[4,5]thieno[2 ,3-d] pyrimidin-4-yl)piperazin-1-yl) methyl)isoindolin-2-yl)dihydropyrimidine-2,4(1H,3H)-dione | 1-(1-oxo-5-((4-(5,6,7,8-tetrahydrobenzo[4,5]thieno[2 ,3-d] pyrimidin-4-yl)piperazin-1-yl) methyl)isoindolin-2-yl)dihydropyrimidine-2,4(1H,3H)-dione |
| GT-0807 9 | 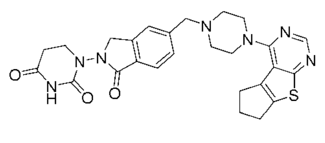 | 1-(5-((4-(6,7-dihydro-5H-cyclopenta[4,5]thieno[2,3-d]pyrimidin-4-yl)piperazin-1-yl) methyl)-1-oxoisoindolin-2-yl) dihydropyrimidine-2,4(1H,3H)-dione | 1-(5-((4-(6,7-dihydro-5H-cyclopenta[4,5]thieno[2,3-d]pyrimidin-4-yl)piperazin-1-yl) methyl)-1-oxoisoindolin-2-yl) dihydropyrimidine-2,4(1H,3H)-dione |
| GT-0806 6 | 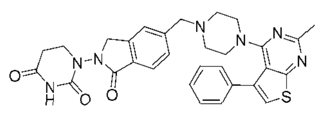 | 1-(5-((4-(2-methyl-5-phenylthieno[2,3-d]pyrimidin-4-yl) piperazin-1-yl)methyl)-1-oxoisoindolin-2-yl)dihydropyrimidine-2,4(1H,3H)-dione | 1-(5-((4-(2-methyl-5-phenylthieno[2,3-d]pyrimidin-4-yl) piperazin-1-yl)methyl)-1-oxoisoindolin-2-yl)dihydropyrimidine-2,4(1H,3H)-dione |
| GT-0806 8 | 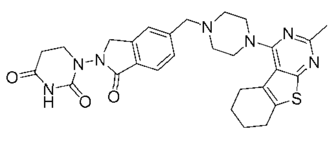 | 1-(5-((4-(2-methyl-5,6,7,8-tetrahydrobenzo[4,5]thieno[2 ,3-d]pyrimidin-4-yl)piperazin-1-yl)methyl)-1-oxoisoindolin-2-yl)dihydropyrimidine-2,4(1H,3H)-dione | 1-(5-((4-(2-methyl-5,6,7,8-tetrahydrobenzo[4,5]thieno[2 ,3-d]pyrimidin-4-yl)piperazin-1-yl)methyl)-1-oxoisoindolin-2-yl)dihydropyrimidine-2,4(1H,3H)-dione |
| GT-0808 0 | 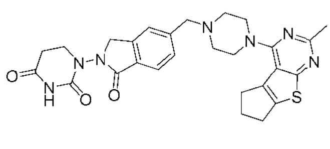 | 1-(5-((4-(2-methyl-6,7-dihydro-5H-cyclopenta[4,5]thieno [2,3-d]pyrimidin-4-yl)piperazin-1-yl)methyl)-1-oxoisoindolin-2-yl)dihydropyrimidine-2,4(1H,3H)-dione | 1-(5-((4-(2-methyl-6,7-dihydro-5H-cyclopenta[4,5]thieno [2,3-d]pyrimidin-4-yl)piperazin-1-yl)methyl)-1-oxoisoindolin-2-yl)dihydropyrimidine-2,4(1H,3H)-dione |
| GT-0806 7 | 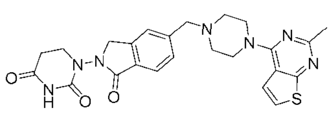 | 1-(5-((4-(2-methylthieno[2,3-d]pyrimidin-4-yl)piperazin-1-yl)methyl)-1-oxoisoindolin-2-yl)dihydropyrimidine-2,4(1H,3H)-dione | 1-(5-((4-(2-methylthieno[2,3-d]pyrimidin-4-yl)piperazin-1-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0808 1 | 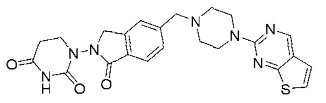 | 1-(1-oxo-5-((4-(thieno[2,3-d] pyrimidin-2-yl)piperazin-1-yl) methyl)isoindolin-2-yl)dihydropyrimidine-2,4(1H,3H)-dione | 1-(1-oxo-5-((4-(thieno[2,3-d] pyrimidin-2-yl)piperazin-1-yl) methyl)isoindolin-2-yl)dihydropyrimidine-2,4(1H,3H)-dione |
| GT-0806 2 | 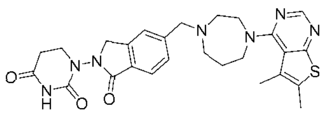 | 1-(5-((4-(5,6-dimethylthieno [2,3-d]pyrimidin-4-yl)-1,4-diazepan-1-yl)methyl)-1-oxoisoindolin-2-yl)dihydropyrimidine-2,4(1H,3H)-dione | 1-(5-((4-(5,6-dimethylthieno [2,3-d]pyrimidin-4-yl)-1,4-diazepan-1-yl)methyl)-1-oxoisoindolin-2-yl)dihydropyrimidine-2,4(1H,3H)-dione |
| GT-0808 2 | 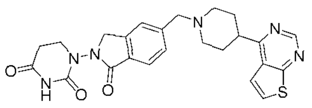 | 1-(1-oxo-5-((4-(thieno[2,3-d] pyrimidin-4-yl)piperidin-1-yl) methyl)isoindolin-2-yl)dihydropyrimidine-2,4(1H,3H)-dione | 1-(1-oxo-5-((4-(thieno[2,3-d] pyrimidin-4-yl)piperidin-1-yl) methyl)isoindolin-2-yl)dihydropyrimidine-2,4(1H,3H)-dione |
| GT-0808 3 | 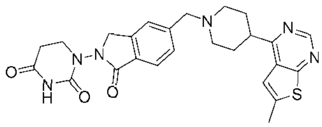 | 1-(5-((4-(6-methylthieno[2,3-d]pyrimidin-4-yl)piperidin-1-yl) methyl)-1-oxoisoindolin-2-yl) dihydropyrimidine-2,4(1H,3H)-dione | 1-(5-((4-(6-methylthieno[2,3-d]pyrimidin-4-yl)piperidin-1-yl) methyl)-1-oxoisoindolin-2-yl) dihydropyrimidine-2,4(1H,3H)-dione |
| GT-0808 4 | 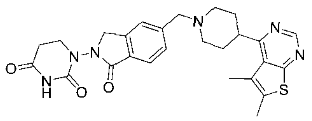 | 1-(5-((4-(5,6-dimethylthieno [2,3-d]pyrimidin-4-yl)piperidin-1-yl)methyl)-1-oxoisoindolin-2-yl)dihydropyrimidine-2,4(1H,3H)-dione | 1-(5-((4-(5,6-dimethylthieno [2,3-d]pyrimidin-4-yl)piperidin-1-yl)methyl)-1-oxoisoindolin-2-yl)dihydropyrimidine-2,4(1H,3H)-dione |
| GT-0808 5 | 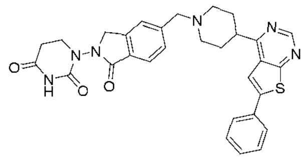 | 1-(1-oxo-5-((4-(6-phenylthieno[2,3-d]pyrimidin-4-yl)piperidin-1-yl)methyl)isoindolin-2-yl)dihydropyrimidine-2,4(1H,3H)-dione | 1-(1-oxo-5-((4-(6-phenylthieno[2,3-d]pyrimidin-4-yl)piperidin-1-yl)methyl)isoindolin-2-yl) dihydropyrimidine-2,4(1H,3H)-dione |
| GT-0808 6 | 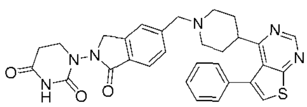 | 1-(1-oxo-5-((4-(5-phenylthieno[2,3-d]pyrimidin-4-yl)piperidin-1-yl)methyl)isoindolin-2-yl)dihydropyrimidine-2,4(1H,3H)-dione | 1-(1-oxo-5-((4-(5-phenylthieno[2,3-d]pyrimidin-4-yl)piperidin-1-yl)methyl)isoindolin-2-yl) dihydropyrimidine-2,4(1H,3H)-dione |
| GT-0811 2 | 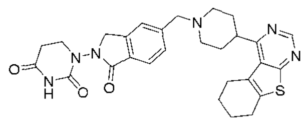 | 1-(1-oxo-5-((4-(5,6,7,8-tetrahydrobenzo[4,5]thieno[2 ,3-d] pyrimidin-4-yl)piperidin-1-yl) methyl)isoindolin-2-yl)dihydropyrimidine-2,4(1H,3H)-dione | 1-(1-oxo-5-((4-(5,6,7,8-tetrahydrobenzo[4,5]thieno[2 ,3-d] pyrimidin-4-yl)piperidin-1-yl) methyl)isoindolin-2-yl)dihydropyrimidine-2,4(1H,3H)-dione |
| GT-0811 3 | 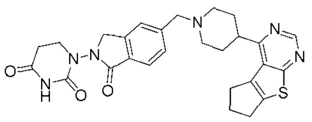 | 1-(5-((4-(6,7-dihydro-5H-cyclopenta[4,5]thieno[2,3-d]pyrimidin-4-yl)piperidin-1-yl) methyl)-1-oxoisoindolin-2-yl) dihydropyrimidine-2,4(1H,3H)-dione | 1-(5-((4-(6,7-dihydro-5H-cyclopenta[4,5]thieno[2,3-d]pyrimidin-4-yl)piperidin-1-yl) methyl)-1-oxoisoindolin-2-yl) dihydropyrimidine-2,4(1H,3H)-dione |

(continued)

| Compound No. | Structure of the compounds | The compounds' name | Chinese translation of the compounds' name |
|---|---|---|---|
| GT-0808 7 | 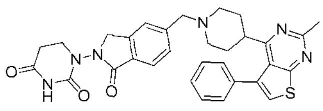 | 1-(5-((4-(2-methyl-5-phenylthieno[2,3-d]pyrimidin-4-yl) piperidin-1-yl)methyl)-1-oxoisoindolin-2-yl)dihydropyrimidine-2,4(1H,3H)-dione | 1-(5-((4-(2-methyl-5-phenylthieno[2,3-d]pyrimidin-4-yl) piperidin-1-yl)methyl)-1-oxoisoindolin-2-yl)dihydropyrimidine-2,4(1H,3H)-dione |
| GT-0808 9 | 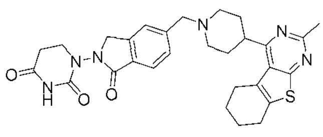 | 1-(5-((4-(2-methyl-5,6,7,8-tetrahydrobenzo[4,5]thieno[2 ,3-d]pyrimidin-4-yl)piperidin-1-yl) methyl)-1-oxoisoindolin-2-yl) dihydropyrimidine-2,4(1H,3H)-dione | 1-(5-((4-(2-methyl-5,6,7,8-tetrahydrobenzo[4,5]thieno[2 ,3-d]pyrimidin-4-yl)piperidin-1-yl) methyl)-1-oxoisoindolin-2-yl) dihydropyrimidine-2,4(1H,3H)-dione |
| GT-0811 4 | 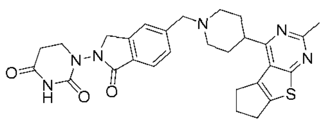 | 1-(5-((4-(2-methyl-6,7-dihydro-5H-cyclopenta[4,5]thieno [2,3-d]pyrimidin-4-yl)piperidin-1-yl)methyl)-1-oxoisoindolin-2-yl)dihydropyrimidine-2,4(1H,3H)-dione | 1-(5-((4-(2-methyl-6,7-dihydro-5H-cyclopenta[4,5]thieno [2,3-d]pyrimidin-4-yl)piperidin-1-yl)methyl)-1-oxoisoindolin-2-yl)dihydropyrimidine-2,4(1H,3H)-dione |
| GT-0808 8 | 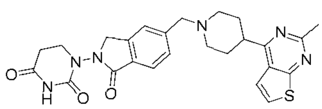 | 1-(5-((4-(2-methylthieno[2,3-d]pyrimidin-4-yl)piperidin-1-yl) methyl)-1-oxoisoindolin-2-yl) dihydropyrimidine-2,4(1H,3H)-dione | 1-(5-((4-(2-methylthieno[2,3-d]pyrimidin-4-yl)piperidin-1-yl) methyl)-1-oxoisoindolin-2-yl) dihydropyrimidine-2,4(1H,3H)-dione |
| GT-0811 5 | 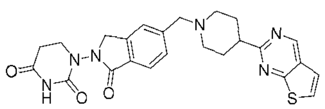 | 1-(1-oxo-5-((4-(thieno[2,3-d] pyrimidin-2-yl)piperidin-1-yl) methyl)isoindolin-2-yl)dihydropyrimidine-2,4(1H,3H)-dione | 1-(1-oxo-5-((4-(thieno[2,3-d] pyrimidin-2-yl)piperidin-1-yl) methyl)isoindolin-2-yl)dihydropyrimidine-2,4(1H,3H)-dione |
| | 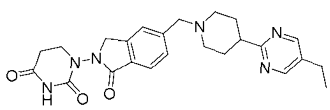 | 1-(5-((4-(5-ethylpyrimidin-2-yl) piperidin-1-yl)methyl)-1-oxoisoindolin-2-yl)dihydropyrimidine-2,4(1H,3H)-dione | 1-(5-((4-(5-ethylpyrimidin-2-yl) piperidin-1-yl)methyl)-1-oxoisoindolin-2-yl)dihydropyrimidine-2,4(1H,3H)-dione |
| GT-0811 6 | 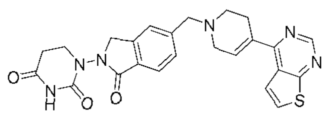 | 1-(1-oxo-5-((4-(thieno[2,3-d] pyrimidin-4-yl)-3,6-dihydropyridin-1(2H)-yl)methyl)isoindolin-2-yl)dihydropyrimidine-2,4(1H,3H)-dione | 1-(1-oxo-5-((4-(thieno[2,3-d] pyrimidin-4-yl)-3,6-dihydropyridin-1(2H)-yl)methyl)isoindolin-2-yl)dihydropyrimidine-2,4(1H,3H)-dione |
| GT-0811 7 | 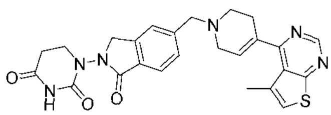 | 1-(5-((4-(5-methylthieno[2,3-d]pyrimidin-4-y1)-3,6-dihydropyridin-1(2H)-yl)methyl)-1-oxoisoindolin-2-yl)dihydropyrimidine-2,4(1H,3H)-dione | 1-(5-((4-(5-methylthieno[2,3-d]pyrimidin-4-yl)-3,6-dihydropyridin-1(2H)-yl)methyl)-1-oxoisoindolin-2-yl)dihydropyrimidine-2,4(1H,3H)-dione |
| GT-0815 7 | 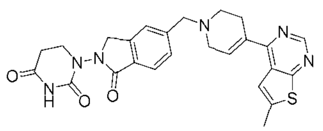 | 1-(5-((4-(6-methylthieno[2,3-d]pyrimidin-4-yl)-3,6-dihydropyridin-1(2H)-yl)methyl)-1-oxoisoindolin-2-yl)dihydropyrimidine-2,4(1H,3H)-dione | 1-(5-((4-(6-methylthieno[2,3-d]pyrimidin-4-yl)-3,6-dihydropyridin-1(2H)-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0815 8 | 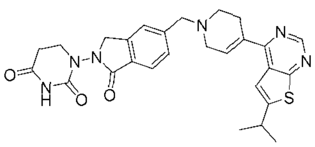 | 1-(5-((4-(6-isopropylthieno [2,3-d]pyrimidin-4-yl)-3,6-dihydropyridin-1(2H)-yl) methyl)-1-oxoisoindolin-2-yl) dihydropyrimidine-2,4(1H,3H)-dione | 1-(5-((4-(6-isopropylthieno [2,3-d]pyrimidin-4-yl)-3,6-dihydropyridin-1(2H)-yl)methyl)-1-oxoisoindolin-2-yl)dihydropyrimidine-2,4(1H,3H)-dione |
| GT-0815 9 | 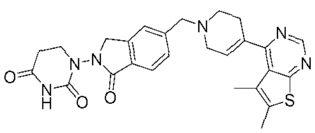 | 1-(5-((4-(5,6-dimethylthieno [2,3-d]pyrimidin-4-yl)-3,6-dihydropyridin-1(2H)-yl) methyl)-1-oxoisoindolin-2-yl) dihydropyrimidine-2,4(1H,3H)-dione | 1-(5-((4-(5,6-dimethylthieno [2,3-d]pyrimidin-4-yl)-3,6-dihydropyridin- 1(2H)-yl)methyl)-1-oxoisoindolin-2-yl)dihydropyrimidine-2,4(1H,3H)-dione |
| GT-0817 3 | 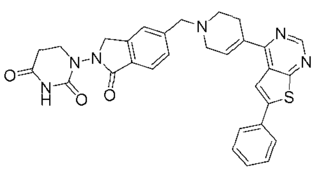 | 1-(1-oxo-5-((4-(6-phenylthieno[2,3-d]pyrimidin-4-yl)-3,6-dihydropyridin-1(2H)-yl) methyl)isoindolin-2-yl)dihydropyrimidine-2,4(1H,3H)-dione | 1-(1-oxo-5-((4-(6-phenylthieno[2,3-d]pyrimidin-4-yl)-3,6-dihydropyridin-1(2H)-yl) methyl)isoindolin-2-yl)dihydropyrimidine-2,4(1H,3H)-dione |
| GT-0817 4 | 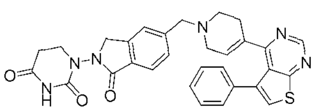 | 1-(1-oxo-5-((4-(5-phenylthieno[2,3-d]pyrimidin-4-yl)-3,6-dihydropyridin-1(2H)-yl) methyl)isoindolin-2-yl)dihydropyrimidine-2,4(1H,3H)-dione | 1-(1-oxo-5-((4-(5-phenylthieno[2,3-d]pyrimidin-4-yl)-3,6-dihydropyridin-1(2H)-yl) methyl)isoindolin-2-yl)dihydropyrimidine-2,4(1H,3H)-dione |
| GT-0817 8 | 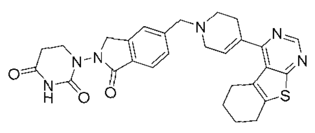 | 1-(1-oxo-5-((4-(5,6,7,8-tetrahydrobenzo[4,5]thieno[2 ,3-d] pyrimidin-4-yl)-3,6-dihydropyridin-1(2H)-yl)methyl)isoindolin-2-yl)dihydropyrimidine-2,4(1H,3H)-dione | 1-(1-oxo-5-((4-(5,6,7,8-tetrahydrobenzo[4,5]thieno[2 ,3-d] pyrimidin-4-yl)-3,6-dihydropyridin-1(2H)-yl)methyl)isoindolin-2-yl)dihydropyrimidine-2,4(1H,3H)-dione |
| GT-0843 6 | 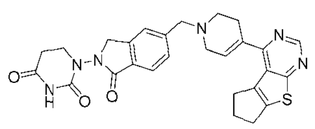 | 1-(5-((4-(6,7-dihydro-5H-cyclopenta[4,5]thieno[2,3-d]pyrimidin-4-yl)-3,6-dihydropyridin-1(2H)-yl)methyl)-1-oxoisoindolin-2-yl)dihydropyrimidine-2,4(1H,3H)-dione | 1-(5-((4-(6,7-dihydro-5H-cyclopenta[4,5]thieno[2,3-d]pyrimidin-4-yl)-3,6-dihydropyridin-1(2H)-yl)methyl)-1-oxoisoindolin-2-yl)dihydropyrimidine-2,4(1H,3H)-dione |
| GT-0817 6 | 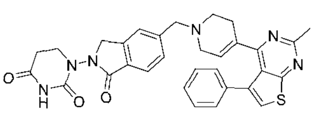 | 1-(5-((4-(2-methyl-5-phenylthieno[2,3-d]pyrimidin-4-yl)-3,6-dihydropyridin-1(2H)-yl)methyl)-1-oxoisoindolin-2-yl)dihydropyrimidine-2,4(1H,3H)-dione | 1-(5-((4-(2-methyl-5-phenylthieno[2,3-d]pyrimidin-4-yl)-3,6-dihydropyridin-1(2H)-yl)methyl)-1-oxoisoindolin-2-yl)dihydropyrimidine-2,4(1H,3H)-dione |
| GT-0817 7 | 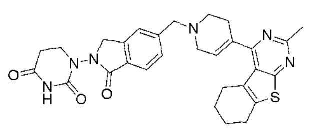 | 1-(5-((4-(2-methyl-5,6,7,8-tetrahydrobenzo[4,5]thieno[2 ,3-d]pyrimidin-4-yl)-3,6-dihydropyridin-1(2H)-yl)methyl)-1-oxoisoindolin-2-yl)dihydropyrimidine-2,4(1H,3H)-dione | 1-(5-((4-(2-methyl-5,6,7,8-tetrahydrobenzo[4,5]thieno[2 ,3-d]pyrimidin-4-yl)-3,6-dihydropyridin-1(2H)-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0843 7 | 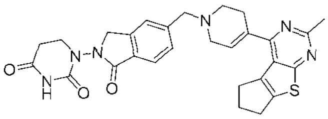 | 1-(5-((4-(2-methyl-6,7-dihydro-5H-cyclopenta[4,5]thieno [2,3-d]pyrimidin-4-yl)-3,6-dihydropyridin-1(2H)-yl) methyl)-1-oxoisoindolin-2-yl) dihydropyrimidine-2,4(1H,3H)-dione | 1-(5-((4-(2-methyl-6,7-dihydro-5H-cyclopenta[4,5]thieno [2,3-d]pyrimidin-4-yl)-3,6-dihydropyridin-1(2H)-yl)methyl)-1-oxoisoindolin-2-yl)dihydropyrimidine-2,4(1H,3H)-dione |
| GT-0817 5 | 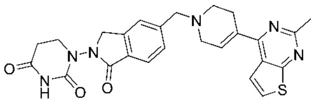 | 1-(5-((4-(2-methylthieno[2,3-d]pyrimidin-4-yl)-3,6-dihydropyridin-1(2H)-yl)methyl)-1-oxoisoindolin-2-yl)dihydropyrimidine-2,4(1H,3H)-dione | 1-(5-((4-(2-methylthieno[2,3-d]pyrimidin-4-yl)-3,6-dihydropyridin-1(2H)-yl)methyl)-1-oxoisoindolin-2-yl)dihydropyrimidine-2,4(1H,3H)-dione |
| GT-0843 8 | 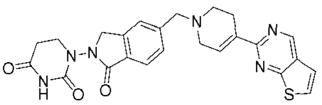 | 1-(1-oxo-5-((4-(thieno[2,3-d] pyrimidin-2-yl)-3,6-dihydropyridin-1(2H)-yl)methyl)isoindolin-2-yl)dihydropyrimidine-2,4(1H,3H)-dione | 1-(1-oxo-5-((4-(thieno[2,3-d] pyrimidin-2-yl)-3,6-dihydropyridin-1(2H)-yl)methyl)isoindolin-2-yl)dihydropyrimidine-2,4(1H,3H)-dione |
| GT-0843 9 | 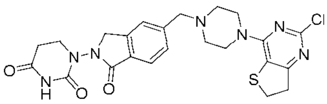 | 1-(5-((4-(2-chloro-6,7-dihydrothieno[3,2-d]pyrimidin-4-yl) piperazin-1-yl)methyl)-1-oxoisoindolin-2-yl)dihydropyrimidine-2,4(1H,3H)-dione | 1-(5-((4-(2-chloro-6,7-dihydrothieno[3,2-d]pyrimidin-4-yl) piperazin-1-yl)methyl)-1-oxoisoindolin-2-yl)dihydropyrimidine-2,4(1H,3H)-dione |
| GT-0844 1 | 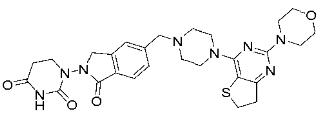 | 1-(5-((4-(2-morpholino-6,7-dihydrothieno[3,2-d]pyrimidin-4-yl)piperazin-1-yl)methyl)-1-oxoisoindolin-2-yl)dihydropyrimidine-2,4(1H,3H)-dione | 1-(5-((4-(2-morpholino-6,7-dihydrothieno[3,2-d]pyrimidin-4-yl)piperazin-1-yl)methyl)-1-oxoisoindolin-2-yl)dihydropyrimidine-2,4(1H,3H)-dione |
| GT-0894 8 | 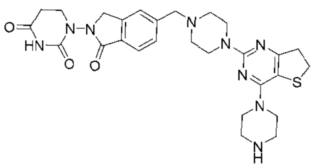 | 1-(1-oxo-5-((4-(4-(piperazin-1-yl)-6,7-dihydrothieno [3,2-d]pyrimidin-2-yl)piperazin-1-yl)methyl)isoindolin-2-yl) dihydropyrimidine-2,4(1H,3H)-dione | 1-(1-oxo-5-((4-(4-(piperazin-1-yl)-6,7-dihydrothieno[3,2-d] pyrimidin-2-yl)piperazin-1-yl) methyl)isoindolin-2-yl)dihydropyrimidine-2,4(1H,3H)-dione |
| GT-0844 2 | 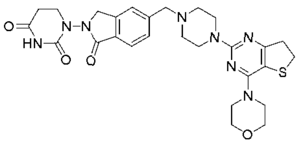 | 1-(5-((4-(4-morpholino-6,7-dihydrothieno[3,2-d]pyrimidin-2-yl)piperazin-1-yl)methyl)-1-oxoisoindolin-2-yl)dihydropyrimidine-2,4(1H,3H)-dione | 1-(5-((4-(4-morpholino-6,7-dihydrothieno[3,2-d]pyrimidin-2-yl)piperazin-1-yl)methyl)-1-oxoisoindolin-2-yl)dihydropyrimidine-2,4(1H,3H)-dione |
| | 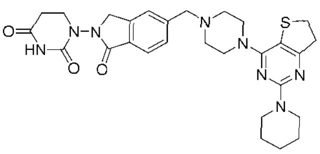 | 1-(1-oxo-5-((4-(2-(piperidin-1-yl)-6,7-dihydrothieno[3,2-d] pyrimidin-4-yl)piperazin-1-yl) methyl)isoindolin-2-yl)dihydropyrimidine-2,4(1H,3H)-dione | 1-(1-oxo-5-((4-(2-(piperidin-1-yl)-6,7-dihydrothieno[3,2-d] pyrimidin-4-yl)piperazin-1-yl) methyl)isoindolin-2-yl)dihydropyrimidine-2,4(1H,3H)-dione |
| | 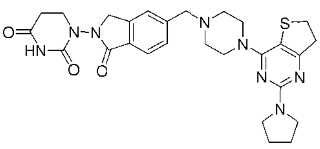 | 1-(1-oxo-5-((4-(2-(pyrrolidin-1-yl)-6,7-dihydrothieno [3,2-d]pyrimidin-4-yl)piperazin-1-yl)methyl)isoindolin-2-yl) dihydropyrimidine-2,4(1H,3H)-dione | 1-(1-oxo-5-((4-(2-(pyrrolidin-1-yl)-6,7-dihydrothieno[3,2-d] pyrimidin-4-yl)piperazin-1-yl) methyl)isoindolin-2-yl)dihydropyrimidine-2,4(1H,3H)-dione |

(continued)

| Compound No. | Structure of the compounds | The compounds' name | Chinese translation of the compounds' name |
|---|---|---|---|
| | 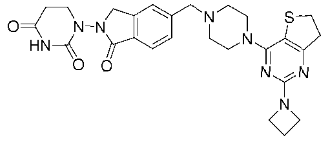 | 1-(5-((4-(2-(azetidin-1-yl)-6,7-dihydrothieno[3,2-d]pyrimidin-4-yl)piperazin-1-yl) methyl)-1-oxoisoindolin-2-yl) dihydropyrimidine-2,4(1H,3H)-dione | 1-(5-((4-(2-(azetidin-1-yl)-6,7-dihydrothieno[3,2-d]pyrimidin-4-yl)piperazin-1-yl) methyl)-1-oxoisoindolin-2-yl) dihydropyrimidine-2,4(1H,3H)-dione |
| GT-0844 3 | 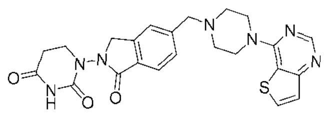 | 1-(1-oxo-5-((4-(thieno[3,2-d] pyrimidin-4-yl)piperazin-1-yl) methyl)isoindolin-2-yl)dihydropyrimidine-2,4(1H,3H)-dione | 1-(1-oxo-5-((4-(thieno[3,2-d] pyrimidin-4-yl)piperazin-1-yl) methyl)isoindolin-2-yl)dihydropyrimidine-2,4(1H,3H)-dione |
| GT-0844 4 | 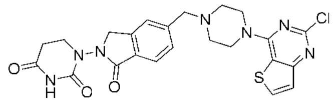 | 1-(5-((4-(2-chlorothieno[3,2-d] pyrimidin-4-yl)piperazin-1-yl) methyl)-1-oxoisoindolin-2-yl) dihydropyrimidine-2,4(1H,3H)-dione | 1-(5-((4-(2-chlorothieno[3,2-d] pyrimidin-4-yl)piperazin-1-yl) methyl)-1-oxoisoindolin-2-yl) dihydropyrimidine-2,4(1H,3H)-dione |
| GT-0844 6 | 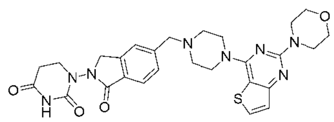 | 1-(5-((4-(2-morpholinothieno [3,2-d]pyrimidin-4-yl)piperazin-1-yl)methyl)-1-oxoisoindolin-2-yl)dihydropyrimidine-2,4(1H,3H)-dione | 1-(5-((4-(2-morpholinothieno [3,2-d]pyrimidin-4-yl)piperazin-1-yl)methyl)-1-oxoisoindolin-2-yl)dihydropyrimidine-2,4(1H,3H)-dione |
| GT-0894 9 | 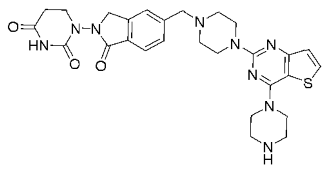 | 1-(1-oxo-5-((4-(4-(piperazin-1-yl)thieno[3,2-d]pyrimidin-2-yl)piperazin-1-yl)methyl) isoindolin-2-yl)dihydropyrimidine-2,4(1H,3H)-dione | 1-(1-oxo-5-((4-(4-(piperazin-1-yl)thieno[3,2-d]pyrimidin-2-yl) piperazin-1-yl)methyl)isoindolin-2-yl)dihydropyrimidine-2,4(1H,3H)-dione |
| GT-0844 7 | 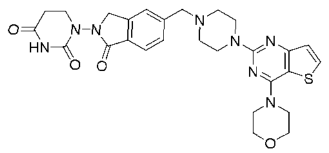 | 1-(5-((4-(4-morpholinothieno [3,2-d]pyrimidin-2-yl)piperazin-1-yl)methyl)-1-oxoisoindolin-2-yl)dihydropyrimidine-2,4(1H,3H)-dione | 1-(5-((4-(4-morpholinothieno [3,2-d]pyrimidin-2-yl)piperazin-1-yl)methyl)-1-oxoisoindolin-2-yl)dihydropyrimidine-2,4(1H,3H)-dione |
| GT-0486 6 | 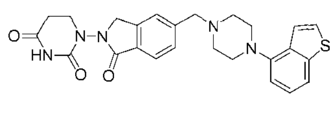 | 1-(5-((4-(benzo[b]thiophen-4-yl)piperazin-1-yl)methyl)-1-oxoisoindolin-2-yl)dihydropyrimidine-2,4(1H,3H)-dione | 1-(5-((4-(benzo[b]thiophen-4-yl)piperazin-1-yl)methyl)-1-oxoisoindolin-2-yl)dihydropyrimidine-2,4(1H,3H)-dione |
| GT-0771 9 | 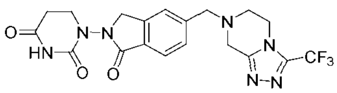 | 1-(1-oxo-5-((3-(trifluoromethyl)-5,6-dihydro-[1,2,4] triazolo[4,3-a]pyrazin-7(8H)-yl)methyl)isoindolin-2-yl)dihydropyrimidine-2,4(1H,3H)-dione | 1-(1-oxo-5-((3-(trifluoromethyl)-5,6-dihydro-[1,2,4] triazolo[4,3-a]pyrazin-7(8H)-yl)methyl)isoindolin-2-yl)dihydropyrimidine-2,4(1H,3H)-dione |
| GT-0486 7 | 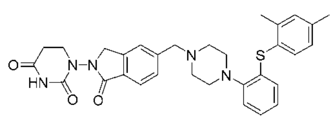 | 1-(5-((4-(2-((2,4-dimethylphenyl)thio)phenyl) piperazin-1-yl)methyl)-1-oxoisoindolin-2-yl)dihydropyrimidine-2,4(1H,3H)-dione | 1-(5-((4-(2-((2,4-dimethylphenyl)thio)phenyl) piperazin-1-yl) methyl)-1-oxoisoindolin-2-yl) dihydropyrimidine-2,4(1H,3H)-dione |
| GT-0772 0 | 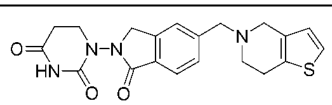 | 1-(5-((6,7-dihydrothieno[3,2-c] pyridin-5(4H)-yl)methyl)-1-oxoisoindolin-2-yl)dihydropyrimidine-2,4(1H,3H)-dione | 1-(5-((6,7-dihydrothieno[3,2-c] pyridin-5(4H)-yl)methyl)-1-oxoisoindolin-2-yl)dihydropyrimidine-2,4(1H,3H)-dione |

(continued)

| Compound No. | Structure of the compounds | The compounds' name | Chinese translation of the compounds' name |
|---|---|---|---|
| GT-0486 5 | 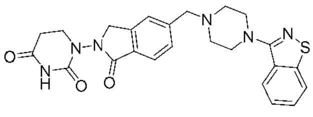 | 1-(5-((4-(benzo[d]isothiazol-3-yl)piperazin-1-yl)methyl)-1-oxoisoindolin-2-yl)dihydropyrimidine-2,4(1H,3H)-dione | 1-(5-((4-(benzo[d]isothiazol-3-yl)piperazin-1-yl)methyl)-1-oxoisoindolin-2-yl)dihydropyrimidine-2,4(1H,3H)-dione |
| GT-0772 1 | 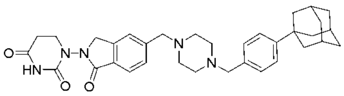 | 1-(5-((4-(4-((3r,5r,7r)-adamantan-1-yl)benzyl)piperazin-1-yl)methyl)-1-oxoisoindolin-2-yl)dihydropyrimidine-2,4(1H,3H)-dione | 1-(5-((4-(4-((3r,5r,7r)-adamantan-1-yl)benzyl)piperazin-1-yl)methyl)-1-oxoisoindolin-2-yl)dihydropyrimidine-2,4(1H,3H)-dione |
| GT-0780 6 | 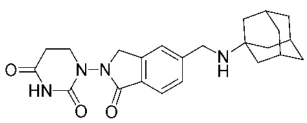 | 1-(5-((((3s,5s,7s)-adamantan-1-yl)amino)methyl)-1-oxoisoindolin-2-yl)dihydropyrimidine-2,4(1H,3H)-dione | 1-(5-((((3s,5s,7s)-adamantan-1-yl)amino)methyl)-1-oxoisoindolin-2-yl)dihydropyrimidine-2,4(1H,3H)-dione |
| GT-0778 4 | 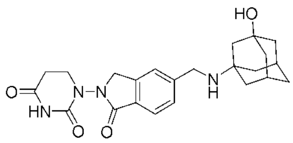 | 1-(5-((((1r,3s,5R,7S)-3-hydroxyadamantan-1-yl)amino)methyl)-1-oxoisoindolin-2-yl)dihydropyrimidine-2,4(1H,3H)-dione | 1-(5-((((1r,3s,5R,7S)-3-hydroxyadamantan-1-yl)amino)methyl)-1-oxoisoindolin-2-yl)dihydropyrimidine-2,4(1H,3H)-dione |
| GT-0813 3 | 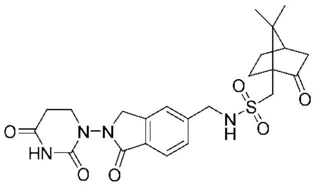 | 1-((1R,4R)-7,7-dimethyl-2-oxobicyclo[2.2.1]heptan-1-yl)-N-((2-(2,4-dioxotetrahydropyrimidin-1(2H)-yl)-1-oxoisoindolin-5-yl)methyl)methanesulfonami de | 1-((1R,4R)-7,7-dimethyl-2-oxobicyclo[2.2.1]heptan-1-yl)-N-((2-(2,4-dioxotetrahydropyrimidin-1 (2H)-yl)-1-oxoisoindolin-5-yl)methyl)methanesulfonami de |
| | 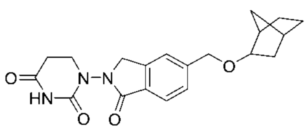 | 1-(5-((((1R,4S)-bicyclo[2.2.1]heptan-2-yl)oxy)methyl)-1-oxoisoindolin-2-yl)dihydropyrimidine-2,4(1H,3H)-dione | 1-(5-((((1R,4S)-bicyclo[2.2.1]heptan-2-yl)oxy)methyl)-1-oxoisoindolin-2-yl)dihydropyrimidine-2,4(1H,3H)-dione |
| GT-0526 7 | 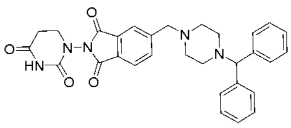 | 5-((4-benzhydrylpiperazin-1-yl)methyl)-2-(2,4-dioxotetrahydropyrimidin-1(2H)-yl)isoindoline-1,3-dione | 5-((4-benzhydrylpiperazin-1-yl)methyl)-2-(2,4-dioxotetrahydropyrimidin-1(2H)-yl)isoindoline-1,3-dione |
| GT-0517 3 | 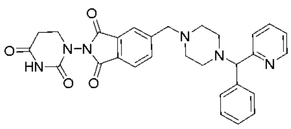 | 2-(2,4-dioxotetrahydropyrimidin-1(2H)-yl)-5-((4-(phenyl(pyridin-2-yl)methyl)piperazin-1-yl)methyl)isoindoline-1,3-dione | 2-(2,4-dioxotetrahydropyrimidin-1(2H)-yl)-5-((4-(phenyl(pyridin-2-yl)methyl)piperazin-1-yl)methyl)isoindoline-1,3-dione |
| GT-0637 7 | 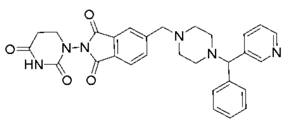 | 2-(2,4-dioxotetrahydropyrimidin-1(2H)-yl)-5-((4-(phenyl(pyridin-3-yl)methyl)piperazin-1-yl)methyl)isoindoline-1,3-dione | 2-(2,4-dioxotetrahydropyrimidin-1(2H)-yl)-5-((4-(phenyl(pyridin-3-yl)methyl)piperazin-1-yl)methyl)isoindoline-1,3-dione |
| GT-0637 8 | 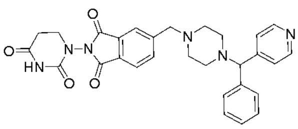 | 2-(2,4-dioxotetrahydropyrimidin-1(2H)-yl)-5-((4-(phenyl(pyridin-4-yl)methyl)piperazin-1-yl)methyl)isoindoline-1,3-dione | 2-(2,4-dioxotetrahydropyrimidin-1(2H)-yl)-5-((4-(phenyl(pyridin-4-yl)methyl)piperazin-1-yl)methyl)isoindoline-1,3-dione |

(continued)

| Compound No. | Structure of the compounds | The compounds' name | Chinese translation of the compounds' name |
|---|---|---|---|
| GT-0637 9 | 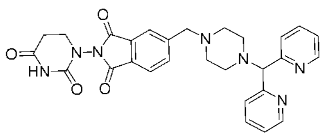 | 5-((4-(di(pyridin-2-yl)methyl) piperazin-1-yl)methyl)-2-(2,4-dioxotetrahydropyrimidin-1(2H)-yl)isoindoline-1,3-dione | 5-((4-(di(pyridin-2-yl)methyl) piperazin-1-yl)methyl)-2-(2,4-dioxotetrahydropyrimidin-1(2H)-yl)isoindoline-1,3-dione |
| | 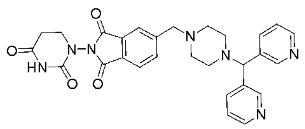 | 5-((4-(di(pyridin-3-yl)methyl) piperazin-1-yl)methyl)-2-(2,4-dioxotetrahydropyrimidin-1(2H)-yl)isoindoline-1,3-dione | 5-((4-(di(pyridin-3-yl)methyl) piperazin-1-yl)methyl)-2-(2,4-dioxotetrahydropyrimidin-1(2H)-yl)isoindoline-1,3-dione |
| | 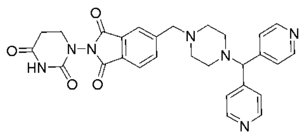 | 5-((4-(di(pyridin-4-yl)methyl) piperazin-1-yl)methyl)-2-(2,4-dioxotetrahydropyrimidin-1(2H)-yl)isoindoline-1,3-dione | 5-((4-(di(pyridin-4-yl)methyl) piperazin-1-yl)methyl)-2-(2,4-dioxotetrahydropyrimidin-1(2H)-yl)isoindoline-1,3-dione |
| | 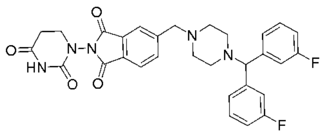 | 5-((4-(bis(3-fluorophenyl) methyl)piperazi n-1-yl) methyl)-2-(2,4-dioxotetrahy-dropyrimidin-1(2H)-yl)isoindo-line-1,3-dione | 5-((4-(bis(3-fluorophenyl) methyl)piperazi n-1-yl) methyl)-2-(2,4-dioxotetrahy-dropyrimidin-1(2H)-yl)isoindo-line-1,3-dione |
| GT-0526 8 | 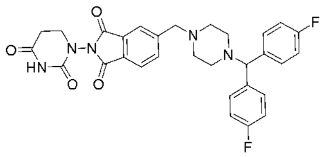 | 5-((4-(bis(4-fluorophenyl) methyl)piperazi n-1-yl) methyl)-2-(2,4-dioxotetrahy-dropyrimidin-1(2H)-yl)isoindo-line-1,3-dione | 5-((4-(bis(4-fluorophenyl) methyl)piperazi n-1-yl) methyl)-2-(2,4-dioxotetrahy-dropyrimidin-1(2H)-yl)isoindo-line-1,3-dione |
| GT-0526 9 | 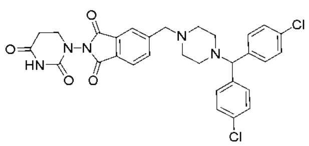 | 5-((4-(bis(4-chlorophenyl) methyl)piperaz in-1-yl) methyl)-2-(2,4-dioxotetrahy-dropyrimidin-1 (2H)-yl)isoin-doline-1,3-dione | 5-((4-(bis(4-chlorophenyl) methyl)piperaz in-1-yl) methyl)-2-(2,4-dioxotetrahy-dropyrimidin-1 (2H)-yl)isoin-doline-1,3-dione |
| GT-0638 1 | 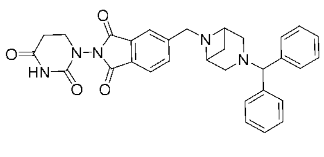 | 5-((3-benzhydryl-3,6-diazabicyclo[3.1.1]heptan-6-yl) methyl)-2-(2,4-dioxotetrahy-dropyrimidin-1(2H)-yl)isoindo-line-1,3-dione | 5-((3-benzhydryl-3,6-diazabicyclo[3.1.1]heptan-6-yl) methyl)-2-(2,4-dioxotetrahy-dropyrimidin-1(2H)-yl)isoindo-line-1,3-dione |
| GT-0527 4 | 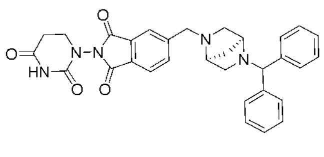 | 5-(((1S,4S)-5-benzhydryl-2,5-diazabicyclo[2.2.1]heptan-2-yl)methyl)-2-(2,4-dioxotetra-hydropyrimidin-1(2H)-yl)isoin-doline-1,3-dione | 5-(((1S,4S)-5-benzhydryl-2,5-diazabicyclo[2.2.1]heptan-2-yl)methyl)-2-(2,4-dioxotetra-hydropyrimidin-1(2H)-yl)isoin-doline-1,3-dione |
| GT-0638 3 | 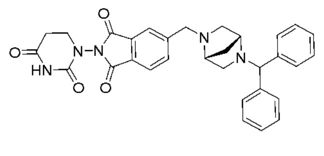 | 5-(((1R,4R)-5-benzhydryl-2,5-diazabicyclo[2.2.1]heptan-2-yl)methyl)-2-(2,4-dioxotetra-hydropyrimidin-1(2H)-yl)isoin-doline-1,3-dione | 5-(((1R,4R)-5-benzhydryl-2,5-diazabicyclo[2.2.1]heptan-2-yl)methyl)-2-(2,4-dioxotetra-hydropyrimidin-1(2H)-yl)isoin-doline-1,3-dione |
| GT-0645 3 | 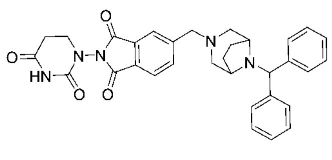 | 5-((8-benzhydryl-3,8-diazabicyclo[3.2.1]octan-3-yl) methyl)-2-(2,4-dioxotetrahy-dropyrimidin-1(2H)-yl)isoindo-line-1,3-dione | 5-((8-benzhydryl-3,8-diazabicyclo[3.2.1]octan-3-yl) methyl)-2-(2,4-dioxotetrahy-dropyrimidin-1(2H)-yl)isoindo-line-1,3-dione |

(continued)

| Compound No. | Structure of the compounds | The compounds' name | Chinese translation of the compounds' name |
|---|---|---|---|
| GT-0645 5 | 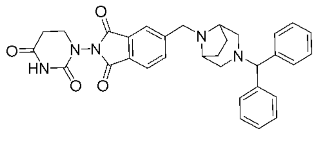 | 5-((3-benzhydryl-3,8-diazabicyclo[3.2.1]octan-8-yl) methyl)-2-(2,4-dioxotetrahydropyrimidin-1(2H)-yl)isoindoline-1,3-dione | 5-((3-benzhydryl-3,8-diazabicyclo[3.2.1]octan-8-yl) methyl)-2-(2,4-dioxotetrahydropyrimidin-1(2H)-yl)isoindoline-1,3-dione |
| GT-0645 1 | 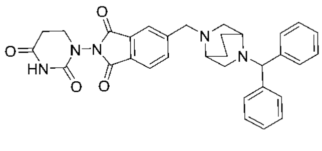 | 5-((5-benzhydryl-2,5-diazabicyclo[2.2.2]octan-2-yl) methyl)-2-(2,4-dioxotetrahydropyrimidin-1(2H)-yl)isoindoline-1,3-dione | 5-((5-benzhydryl-2,5-diazabicyclo[2.2.2]octan-2-yl) methyl)-2-(2,4-dioxotetrahydropyrimidin-1(2H)-yl)isoindoline-1,3-dione |
| GT-0729 5 | 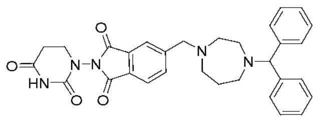 | 5-((4-benzhydryl-1,4-diazepan-1-yl)methyl)-2-(2,4-dioxotetrahydropyrimidin-1(2H)-yl) isoindoline-1,3-dione | 5-((4-benzhydryl-1,4-diazepan-1-yl)methyl)-2-(2,4-dioxotetrahydropyrimidin-1(2H)-yl) isoindoline-1,3-dione |
| GT-0643 0 | 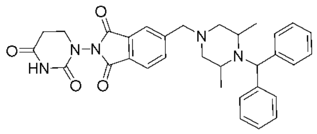 | 5-((4-benzhydryl-3,5-dimethylpiperazin-1-yl) methyl)-2-(2,4-dioxotetrahydropyrimidin-1(2H)-yl)isoindoline-1,3-dione | 5-((4-benzhydryl-3,5-dimethylpiperazin-1-yl) methyl)-2-(2,4-dioxotetrahydropyrimidin-1(2H)-yl)isoindoline-1,3-dione |
| | 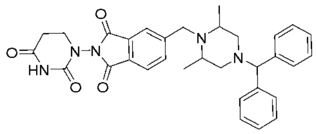 | 5-((4-benzhydryl-2,6-dimethylpiperazin-1-yl) methyl)-2-(2,4-dioxotetrahydropyrimidin-1(2H)-yl)isoindoline-1,3-dione | 5-((4-benzhydryl-2,6-dimethylpiperazin-1-yl) methyl)-2-(2,4-dioxotetrahydropyrimidin-1(2H)-yl)isoindoline-1,3-dione |
| GT-0638 5 | 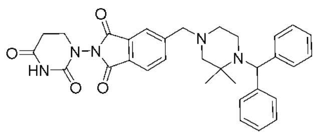 | 5-((4-benzhydryl-3,3-dimethylpiperazin-1-yl) methyl)-2-(2,4-dioxotetrahydropyrimidin-1(2H)-yl)isoindoline-1,3-dione | 5-((4-benzhydryl-3,3-dimethylpiperazin-1-yl) methyl)-2-(2,4-dioxotetrahydropyrimidin-1(2H)-yl)isoindoline-1,3-dione |
| | 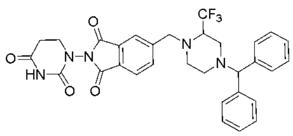 | 5-((4-benzhydryl-2-(trifluoromethyl)piperazin-1-yl) methyl)-2-(2,4-dioxotetrahydropyrimidin-1(2H)-yl)isoindoline-1,3-dione | 5-((4-benzhydryl-2-(trifluoromethyl)piperazin-1-yl) methyl)-2-(2,4-dioxotetrahydropyrimidin-1(2H)-yl)isoindoline-1,3-dione |
| GT-0644 9 | 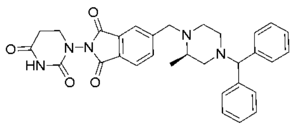 | (R)-5-((4-benzhydryl-2-methylpiperazin-1-yl) methyl)-2-(2,4-dioxotetrahydropyrimidin-1(2H)-yl)isoindoline-1,3-dione | (R)-5-((4-benzhydryl-2-methylpiperazin-1-yl) methyl)-2-(2,4-dioxotetrahydropyrimidin-1(2H)-yl)isoindoline-1,3-dione |
| | 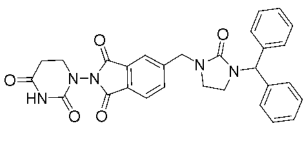 | 5-((3-benzhydryl-2-oxoimidazolidin-1-yl)methyl)-2-(2,4-dioxotetrahydropyrimidin-1(2H)-yl)isoindoline-1,3-dione | 5-((3-benzhydryl-2-oxoimidazolidin-1-yl)methyl)-2-(2,4-dioxotetrahydropyrimidin-1(2H)-yl)isoindoline-1,3-dione |
| GT-0517 7 | 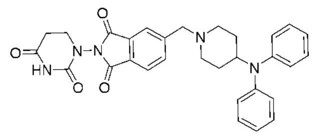 | 2-(2,4-dioxotetrahydropyrimidin-1(2H)-yl)-5-((4-(diphenylamino)piperidin-1-yl)methyl) isoindoline-1,3-dione | 2-(2,4-dioxotetrahydropyrimidin-1(2H)-yl)-5-((4-(diphenylamino)piperidin-1-yl)methyl) isoindoline-1,3-dione |

(continued)

| Compound No. | Structure of the compounds | The compounds' name | Chinese translation of the compounds' name |
|---|---|---|---|
| GT-0517 8 | 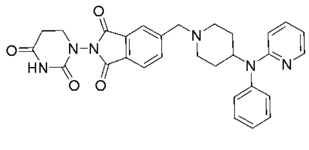 | 2-(2,4-dioxotetrahydropyrimidin-1(2H)-yl)-5-((4-(phenyl(pyridin-2-yl)amino)piperidin-1-yl)methyl)isoindoline-1,3-dione | 2-(2,4-dioxotetrahydropyrimidin-1(2H)-yl)-5-((4-(phenyl(pyridin-2-yl)amino)piperidin-1-yl)methyl)isoindoline-1,3-dione |
| GT-0518 0 | 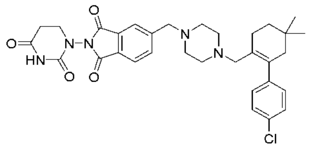 | 5-((4-((4'-chloro-5,5-dimethyl-3,4,5,6-tetrahydro-[1,1'-biphenyl]-2-yl)methyl)piperazin-1-yl)methyl)-2-(2,4-dioxotetrahydropyrimidin-1(2H)-yl)isoindoline-1,3-dione | 5-((4-((4'-chloro-5,5-dimethyl-3,4,5,6-tetrahydro-[1,1'-biphenyl]-2-yl)methyl)piperazin-1-yl)methyl)-2-(2,4-dioxotetrahydropyrimidin-1(2H)-yl)isoindoline-1,3-dione |
| GT-0645 7 | 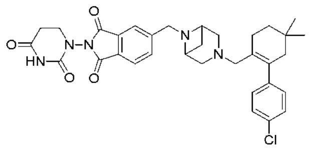 | 5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-2-(2,4-dioxotetrahydropyrimidin-1(2H)-yl)isoindoline-1,3-dione | 5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-2-(2,4-dioxotetrahydropyrimidin-1(2H)-yl)isoindoline-1,3-dione |
| GT-0645 9 | 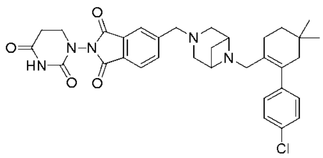 | 5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-2-(2,4-dioxotetrahydropyrimidin-1(2H)-yl)isoindoline-1,3-dione | 5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-2-(2,4-dioxotetrahydropyrimidin-1(2H)-yl)isoindoline-1,3-dione |
| | 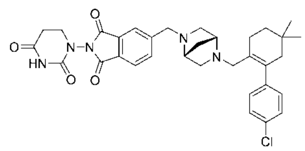 | 5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,4-dioxotetrahydropyrimidin-1(2H)-yl)isoindoline-1,3-dione | 5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,4-dioxotetrahydropyrimidin-1(2H)-yl)isoindoline-1,3-dione |
| | 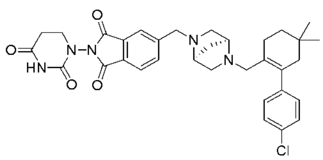 | 5-(((1S,4S)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,4-dioxotetrahydropyrimidin-1(2H)-yl)isoindoline-1,3-dione | 5-(((1S,4S)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,4-dioxotetrahydropyrimidin-1(2H)-yl)isoindoline-1,3-dione |
| GT-0733 4 | 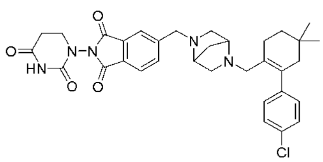 | 5-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,4-dioxotetrahydropyrimidin-1(2H)-yl)isoindoline-1,3-dione | 5-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,4-dioxotetrahydropyrimidin-1(2H)-yl)isoindoline-1,3-dione |

(continued)

| Compound No. | Structure of the compounds | The compounds' name | Chinese translation of the compounds' name |
|---|---|---|---|
| GT-0788 7 | 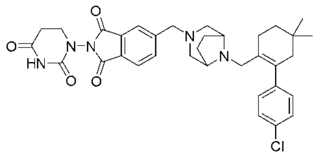 | 5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-2-(2,4-dioxotetrahydropyrimidin-1(2H)-yl)isoindoline-1,3-dione | 5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-2-(2,4-dioxotetrahydropyrimidin-1(2H)-yl)isoindoline-1,3-dione |
| GT-0733 3 | 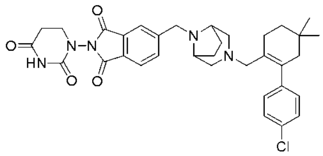 | 5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-2-(2,4-dioxotetrahydropyrimidin-1(2H)-yl)isoindoline-1,3-dione | 5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-2-(2,4-dioxotetrahydropyrimidin-1(2H)-yl)isoindoline-1,3-dione |
| GT-0730 0 | 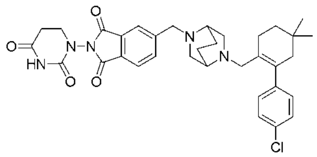 | 5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-2-(2,4-dioxotetrahydropyrimidin-1(2H)-yl)isoindoline-1,3-dione | 5-((5-((4'-chloro-5,5-dimethyl-3,4,5,6-tetrahydro-[1,1'-biphenyl]-2-yl)methyl)-2,5-diazabicyclo[2.2.2] octan-2-yl)methyl)-2-(2,4-dioxotetrahydropyrimidin-1(2H)-yl)isoindoline-1,3-dione |
| GT-0730 1 | 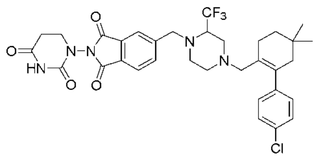 | 5-((4-((4'-chloro-5,5-dimethyl-3,4,5,6-tetrahydro-[1,1'-biphenyl]-2-yl)methyl)-2-(trifluoromethyl)piperazin-1-yl)methyl)-2-(2,4-dioxotetrahydropyrimidin-1(2H)-yl)isoindoline-1,3-dione | 5-((4-((4'-chloro-5,5-dimethyl-3,4,5,6-tetrahydro-[1,1'-biphenyl]-2-yl)methyl)-2-(trifluoromethyl)piperazin-1-yl)methyl)-2-(2,4-dioxotetrahydropyrimidin-1(2H)-yl)isoindoline-1,3-dione |
| GT-0730 2 | 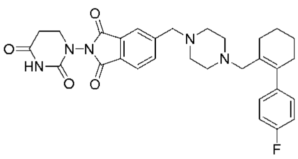 | 2-(2,4-dioxotetrahydropyrimidin-1(2H)-yl)-5-((4-((4'-fluoro-3,4,5,6-tetrahydro-[1,1'-biphenyl]-2-yl)methyl)piperazin-1-yl)methyl)isoindoline-1,3-dione | 2-(2,4-dioxotetrahydropyrimidin-1(2H)-yl)-5-((4-((4'-fluoro-3,4,5,6-tetrahydro-[1,1'-biphenyl]-2-yl)methyl)piperazin-1-yl)methyl)isoindoline-1,3-dione |
| GT-0730 3 | 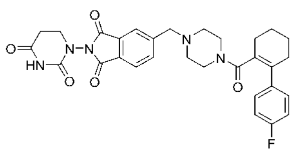 | 2-(2,4-dioxotetrahydropyrimidin-1(2H)-yl)-5-((4-(4'-fluoro-3,4,5,6-tetrahydro-[1,1'-biphenyl]-2-carbonyl)piperazin-1-yl)methyl)isoindoline-1,3-dione | 2-(2,4-dioxotetrahydropyrimidin-1 (2H)-yl)-5-((4-(4'-fluoro-3,4,5,6-tetrahydro-[1,1'-biphenyl]-2-carbonyl)piperazin-1-yl)methyl)isoindoline-1,3-dione |
| GT-0737 7 | 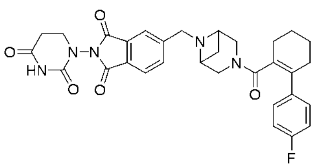 | 2-(2,4-dioxotetrahydropyrimidin-1(2H)-yl)-5-((3-(4'-fluoro-3,4,5,6-tetrahydro-[1,1'-biphenyl]-2-carbonyl)-3,6-diazabicyclo[3.1.1]heptan-6-yl)methyl)isoindoline-1,3-dione | 2-(2,4-dioxotetrahydropyrimidin-1(2H)-yl)-5-((3-(4'-fluoro-3,4,5,6-tetrahydro-[1,1'-biphenyl]-2-carbonyl)-3,6-diazabicyclo[3.1.1]heptan-6-yl)methyl)isoindoline-1,3-dione |

(continued)

| Compound No. | Structure of the compounds | The compounds' name | Chinese translation of the compounds' name |
|---|---|---|---|
| GT-0517 9 | 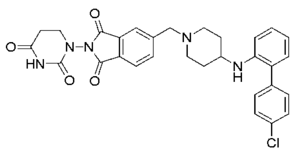 | 5-((4-((4'-chloro-[1,1'-biphenyl]-2-yl)amino)piperidin-1-yl)methyl)-2-(2,4-dioxotetrahydropyrimidin-1(2H)-yl)isoindoline-1,3-dione | 5-((4-((4'-chloro-[1,1'-biphenyl]-2-yl)amino)piperidin-1-yl)methyl)-2-(2,4-dioxotetrahydropyrimidin-1(2H)-yl)isoindoline-1,3-dione |
| GT-0643 2 | 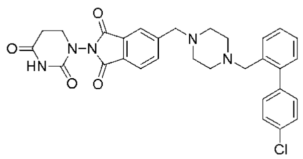 | 5-((4-((4'-chloro-[1,1'-biphenyl]-2-yl)methyl)piperazin-1-yl)methyl)-2-(2,4-dioxotetrahydropyrimidin-1(2H)-yl)isoindoline-1,3-dione | 5-((4-((4'-chloro-[1,1'-biphenyl]-2-yl)methyl)piperazin-1-yl)methyl)-2-(2,4-dioxotetrahydropyrimidin-1(2H)-yl)isoindoline-1,3-dione |
| GT-0730 4 | 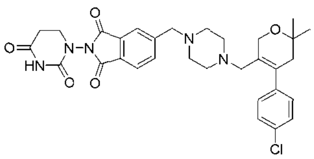 | 5-((4-((4-(4-chlorophenyl)-6,6-dimethyl-5,6-dihydro-2H-pyran-3-yl)methyl)piperazin-1-yl)methyl)-2-(2,4-dioxotetrahydropyrimidin-1(2H)-yl)isoindoline-1,3-dione | 5-((4-((4-(4-chlorophenyl)-6,6-dimethyl-5,6-dihydro-2H-pyran-3-yl)methyl)piperazin-1-yl)methyl)-2-(2,4-dioxotetrahydropyrimidin-1(2H)-yl)isoindoline-1,3-dione |
| | 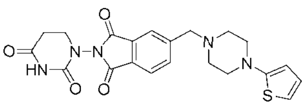 | 2-(2,4-dioxotetrahydropyrimidin-1(2H)-yl)-5-((4-(thiophen-2-yl)piperazin-1-yl)methyl)isoindoline-1,3-dione | 2-(2,4-dioxotetrahydropyrimidin-1(2H)-yl)-5-((4-(thiophen-2-yl)piperazin-1-yl)methyl)isoindoline-1,3-dione |
| GT-0530 9 | 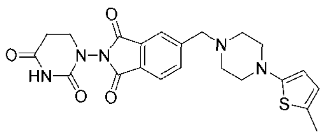 | 2-(2,4-dioxotetrahydropyrimidin-1(2H)-yl)-5-((4-(5-methylthiophen-2-yl)piperazin-1-yl)methyl)isoindoline-1,3-dione | 2-(2,4-dioxotetrahydropyrimidin-1(2H)-yl)-5-((4-(5-methylthiophen-2-yl)piperazin-1-yl)methyl)isoindoline-1,3-dione |
| | 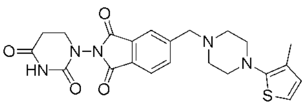 | 2-(2,4-dioxotetrahydropyrimidin-1(2H)-yl)-5-((4-(3-methylthiophen-2-yl)piperazin-1-yl)methyl)isoindoline-1,3-dione | 2-(2,4-dioxotetrahydropyrimidin-1(2H)-yl)-5-((4-(3-methylthiophen-2-yl)piperazin-1-yl)methyl)isoindoline-1,3-dione |
| GT-0531 3 | 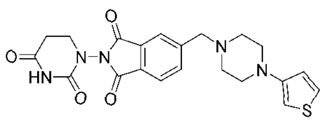 | 2-(2,4-dioxotetrahydropyrimidin-1(2H)-yl)-5-((4-(thiophen-3-yl)piperazin-1-yl)methyl)isoindoline-1,3-dione | 2-(2,4-dioxotetrahydropyrimidin-1(2H)-yl)-5-((4-(thiophen-3-yl)piperazin-1-yl)methyl)isoindoline-1,3-dione |
| | 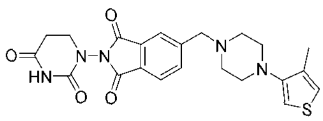 | 2-(2,4-dioxotetrahydropyrimidin-1(2H)-yl)-5-((4-(4-methylthiophen-3-yl)piperazin-1-yl)methyl)isoindoline-1,3-dione | 2-(2,4-dioxotetrahydropyrimidin-1(2H)-yl)-5-((4-(4-methylthiophen-3-yl)piperazin-1-yl)methyl)isoindoline-1,3-dione |
| GT-0531 2 | 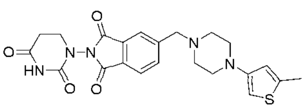 | 2-(2,4-dioxotetrahydropyrimidin-1(2H)-yl)-5-((4-(5-methylthiophen-3-yl)piperazin-1-yl)methyl)isoindoline-1,3-dione | 2-(2,4-dioxotetrahydropyrimidin-1(2H)-yl)-5-((4-(5-methylthiophen-3-yl)piperazin-1-yl)methyl)isoindoline-1,3-dione |
| | 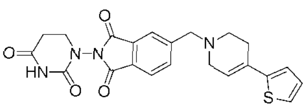 | 2-(2,4-dioxotetrahydropyrimidin-1(2H)-yl)-5-((4-(thiophen-2-yl)-3,6-dihydropyridin-1(2H)-yl)methyl)isoindoline-1,3-dione | 2-(2,4-dioxotetrahydropyrimidin-1(2H)-yl)-5-((4-(thiophen-2-yl)-3,6-dihydropyridin-1(2H)-yl)methyl)isoindoline-1,3-dione |

(continued)

| Compound No. | Structure of the compounds | The compounds' name | Chinese translation of the compounds' name |
|---|---|---|---|
| GT-0531 0 | 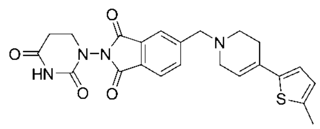 | 2-(2,4-dioxotetrahydropyrimidin-1(2H)-yl)-5-((4-(5-methylthiophen-2-yl)-3,6-dihydropyridin-1(2H)-yl)methyl) isoindoline-1,3-dione | 2-(2,4-dioxotetrahydropyrimidin-1(2H)-yl)-5-((4-(5-methylthiophen-2-yl)-3,6-dihydropyridin-1(2H)-yl)methyl) isoindoline-1,3-dione |
| GT-0643 4 | 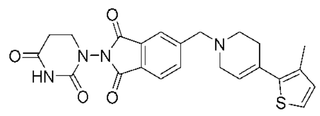 | 2-(2,4-dioxotetrahydropyrimidin-1(2H)-yl)-5-((4-(3-methylthiophen-2-yl)-3,6-dihydropyridin-1(2H)-yl)methyl) isoindoline-1,3-dione | 2-(2,4-dioxotetrahydropyrimidin-1(2H)-yl)-5-((4-(3-methylthiophen-2-yl)-3,6-dihydropyridin-1(2H)-yl)methyl) isoindoline-1,3-dione |
| GT-0643 5 | 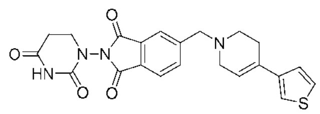 | 2-(2,4-dioxotetrahydropyrimidin-1(2H)-yl)-5-((4-(thiophen-3-yl)-3,6-dihydropyridin-1(2H)-yl)methyl)isoindoline-1,3-dione | 2-(2,4-dioxotetrahydropyrimidin-1(2H)-yl)-5-((4-(thiophen-3-yl)-3,6-dihydropyridin-1(2H)-yl)methyl)isoindoline-1,3-dione |
| GT-0531 6 | 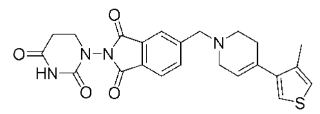 | 2-(2,4-dioxotetrahydropyrimidin-1(2H)-yl)-5-((4-(4-methylthiophen-3-yl)-3,6-dihydropyridin-1(2H)-yl)methyl) isoindoline-1,3-dione | 2-(2,4-dioxotetrahydropyrimidin-1(2H)-yl)-5-((4-(4-methylthiophen-3-yl)-3,6-dihydropyridin-1(2H)-yl)methyl) isoindoline-1,3-dione |
| GT-0742 3 | 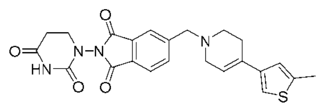 | 2-(2,4-dioxotetrahydropyrimidin-1(2H)-yl)-5-((4-(5-methylthiophen-3-yl)-3,6-dihydropyridin-1(2H)-yl)methyl) isoindoline-1,3-dione | 2-(2,4-dioxotetrahydropyrimidin-1(2H)-yl)-5-((4-(5-methylthiophen-3-yl)-3,6-dihydropyridin-1(2H)-yl)methyl) isoindoline-1,3-dione |
| GT-0531 7 | 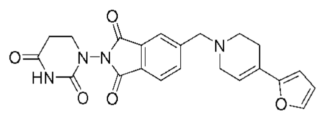 | 2-(2,4-dioxotetrahydropyrimidin-1(2H)-yl)-5-((4-(furan-2-yl)-3,6-dihydropyridin-1(2H)-yl)methyl)isoindoline-1,3-dione | 2-(2,4-dioxotetrahydropyrimidin-1(2H)-yl)-5-((4-(furan-2-yl)-3,6-dihydropyridin-1(2H)-yl)methyl)isoindoline-1,3-dione |
| GT-0744 2 | 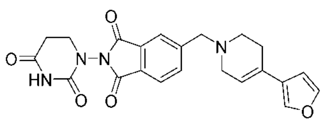 | 2-(2,4-dioxotetrahydropyrimidin-1(2H)-yl)-5-((4-(furan-3-yl)-3,6-dihydropyridin-1(2H)-yl)methyl)isoindoline-1,3-dione | 2-(2,4-dioxotetrahydropyrimidin-1(2H)-yl)-5-((4-(furan-3-yl)-3,6-dihydropyridin-1(2H)-yl)methyl)isoindoline-1,3-dione |
| GT-0742 4 | 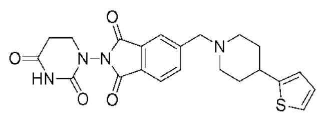 | 2-(2,4-dioxotetrahydropyrimidin-1(2H)-yl)-5-((4-(thiophen-2-yl)piperidin-1-yl) methyl)isoindoline-1,3-dione | 2-(2,4-dioxotetrahydropyrimidin-1(2H)-yl)-5-((4-(thiophen-2-yl)piperidin-1-yl) methyl)isoindoline-1,3-dione |
| GT-0531 1 | 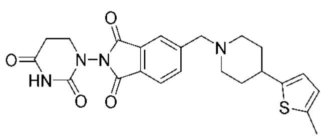 | 2-(2,4-dioxotetrahydropyrimidin-1(2H)-yl)-5-((4-(5-methylthiophen-2-yl)piperidin-1-yl)methyl)isoindoline-1,3-dione | 2-(2,4-dioxotetrahydropyrimidin-1(2H)-yl)-5-((4-(5-methylthiophen-2-yl)piperidin-1-yl)methyl)isoindoline-1,3-dione |
| GT-0730 6 | 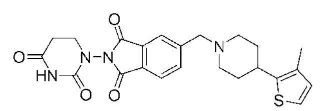 | 2-(2,4-dioxotetrahydropyrimidin-1(2H)-yl)-5-((4-(3-methylthiophen-2-yl)piperidin-1-yl)methyl)isoindoline-1,3-dione | 2-(2,4-dioxotetrahydropyrimidin-1(2H)-yl)-5-((4-(3-methylthiophen-2-yl)piperidin-1-yl)methyl)isoindoline-1,3-dione |

(continued)

| Compound No. | Structure of the compounds | The compounds' name | Chinese translation of the compounds' name |
|---|---|---|---|
| GT-0742 0 | 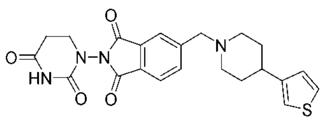 | 2-(2,4-dioxotetrahydropyrimi-din-1(2H)-yl)-5-((4-(thiophen-3-yl)piperidin-1-yl) methyl)isoindoline-1,3-dione | 2-(2,4-dioxotetrahydropyrimidin-1(2H)-yl)-5-((4-(thiophen-3-yl)piperidin-1-yl) methyl)isoindoline-1,3-dione |
| GT-0742 1 | 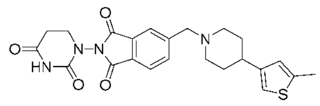 | 2-(2,4-dioxotetrahydropyrimi-din-1(2H)-yl)-5-((4-(5-methylthiophen-3-yl)piperi-din-1-yl)methyl)isoindo-line-1,3-dione | 2-(2,4-dioxotetrahydropyrimidin-1(2H)-yl)-5-((4-(5-methylthiophen-3-yl)piperi-din-1-yl)methyl)isoindo-line-1,3-dione |
| GT-0531 4 | 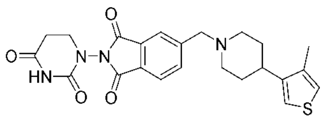 | 2-(2,4-dioxotetrahydropyrimi-din-1(2H)-yl)-5-((4-(4-methylthiophen-3-yl)piperi-din-1-yl)methyl)isoindo-line-1,3-dione | 2-(2,4-dioxotetrahydropyrimidin-1(2H)-yl)-5-((4-(4-methylthiophen-3-yl)piperi-din-1-yl)methyl)isoindo-line-1,3-dione |
| GT-0531 5 | 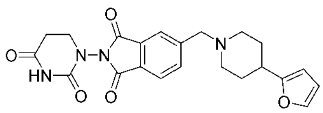 | 2-(2,4-dioxotetrahydropyrimi-din-1(2H)-yl)-5-((4-(furan-2-yl) piperidin-1-yl)methyl)isoindoline-1,3-dione | 2-(2,4-dioxotetrahydropyrimidin-1(2H)-yl)-5-((4-(furan-2-yl) piperidin-1-yl)methyl)isoindoline-1,3-dione |
| GT-0742 2 | 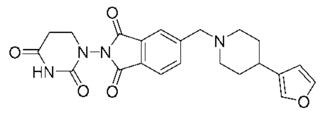 | 2-(2,4-dioxotetrahydropyrimi-din-1(2H)-yl)-5-((4-(furan-3-yl) piperidin-1-yl)methyl)isoindoline-1,3-dione | 2-(2,4-dioxotetrahydropyrimidin-1(2H)-yl)-5-((4-(furan-3-yl) piperidin-1-yl)methyl)isoindoline-1,3-dione |
| GT-0485 4 | 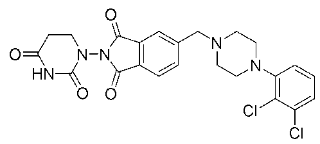 | 5-((4-(2,3-dichlorophenyl)piperazin-1-yl)methyl)-2-(2,4-di-oxotetrahydropyrimi-din-1(2H)-yl)isoindoline-1,3-dione | 5-((4-(2,3-dichlorophenyl)piperazin-1-yl)methyl)-2-(2,4-di-oxotetrahydropyrimi-din-1(2H)-yl)isoindoline-1,3-dione |
| GT-0671 9 | 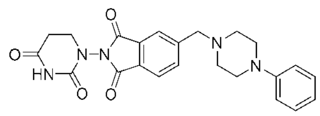 | 2-(2,4-dioxotetrahydropyrimi-din-1(2H)-yl)-5-((4-phenylpi-perazin-1-yl)methyl)isoindo-line-1,3-dione | 2-(2,4-dioxotetrahydropyrimidin-1(2H)-yl)-5-((4-phenylpi-perazin-1-yl)methyl)isoindo-line-1,3-dione |
| GT-0672 0 | 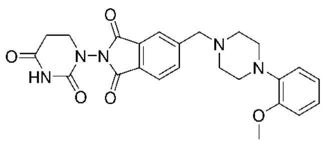 | 2-(2,4-dioxotetrahydropyrimi-din-1(2H)-yl)-5-((4-(2-methoxyphenyl)piperazin-1-yl) methyl)isoindoline-1,3-dione | 2-(2,4-dioxotetrahydropyrimidin-1(2H)-yl)-5-((4-(2-methoxyphenyl)piperazin-1-yl) methyl)isoindoline-1,3-dione |
| GT-0672 1 | 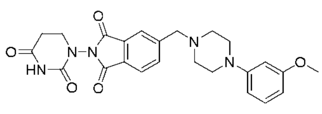 | 2-(2,4-dioxotetrahydropyrimi-din-1(2H)-yl)-5-((4-(3-methoxyphenyl)piperazin-1-yl) methyl)isoindoline-1,3-dione | 2-(2,4-dioxotetrahydropyrimidin-1(2H)-yl)-5-((4-(3-methoxyphenyl)piperazin-1-yl) methyl)isoindoline-1,3-dione |
| GT-0672 8 | 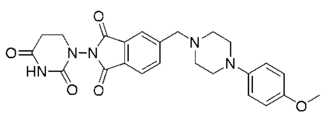 | 2-(2,4-dioxotetrahydropyrimi-din-1(2H)-yl)-5-((4-(4-methoxyphenyl)piperazin-1-yl) methyl)isoindoline-1,3-dione | 2-(2,4-dioxotetrahydropyrimidin-1(2H)-yl)-5-((4-(4-methoxyphenyl)piperazin-1-yl) methyl)isoindoline-1,3-dione |
| GT-0672 3 | 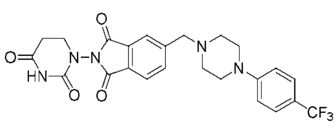 | 2-(2,4-dioxotetrahydropyrimi-din-1(2H)-yl)-5-((4-(4-(trifluoromethyl)phenyl)piper azin-1-yl)methyl)isoindoline-1,3-dione | 2-(2,4-dioxotetrahydropyrimidin-1(2H)-yl)-5-((4-(4-(trifluoromethyl)phenyl)piper azin-1-yl)methyl)isoindoline-1,3-dione |

(continued)

| Compound No. | Structure of the compounds | The compounds' name | Chinese translation of the compounds' name |
|---|---|---|---|
| GT-0672 4 | 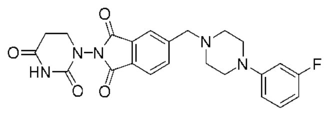 | 2-(2,4-dioxotetrahydropyrimidin-1(2H)-yl)-5-((4-(3-fluorophenyl)piperazin-1-yl)methyl) isoindoline-1,3-dione | 2-(2,4-dioxotetrahydropyrimidin-1(2H)-yl)-5-((4-(3-fluorophenyl)piperazin-1-yl)methyl) isoindoline-1,3-dione |
| GT-0672 5 | 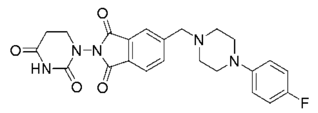 | 2-(2,4-dioxotetrahydropyrimidin-1(2H)-yl)-5-((4-(4-fluorophenyl)piperazin-1-yl)methyl) isoindoline-1,3-dione | 2-(2,4-dioxotetrahydropyrimidin-1(2H)-yl)-5-((4-(4-fluorophenyl)piperazin-1-yl)methyl) isoindoline-1,3-dione |
| GT-0672 6 | 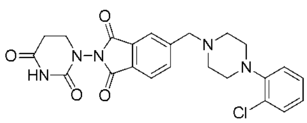 | 5-((4-(2-chlorophenyl)piperazin-1-yl)methyl)-2-(2,4-dioxotetrahydropyrimidin-1(2H)-yl) isoindoline-1,3-dione | 5-((4-(2-chlorophenyl)piperazin-1-yl)methyl)-2-(2,4-dioxotetrahydropyrimidin-1(2H)-yl) isoindoline-1,3-dione |
| GT-0678 3 | 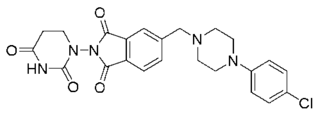 | 5-((4-(4-chlorophenyl)piperazin-1-yl)methyl)-2-(2,4-dioxotetrahydropyrimidin-1(2H)-yl) isoindoline-1,3-dione | 5-((4-(4-chlorophenyl)piperazin-1-yl)methyl)-2-(2,4-dioxotetrahydropyrimidin-1(2H)-yl) isoindoline-1,3-dione |
| GT-0672 7 | 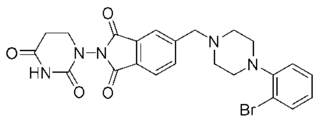 | 5-((4-(2-bromophenyl)piperazin-1-yl)methyl)-2-(2,4-dioxotetrahydropyrimidin-1(2H)-yl) isoindoline-1,3-dione | 5-((4-(2-bromophenyl)piperazin-1-yl)methyl)-2-(2,4-dioxotetrahydropyrimidin-1(2H)-yl) isoindoline-1,3-dione |
| GT-0672 2 | 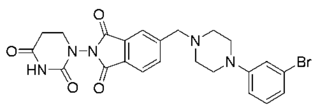 | 5-((4-(3-bromophenyl)piperazin-1-yl)methyl)-2-(2,4-dioxotetrahydropyrimidin-1(2H)-yl) isoindoline-1,3-dione | 5-((4-(3-bromophenyl)piperazin-1-yl)methyl)-2-(2,4-dioxotetrahydropyrimidin-1(2H)-yl) isoindoline-1,3-dione |
| GT-0678 4 | 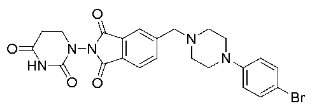 | 5-((4-(4-bromophenyl)piperazin-1-yl)methyl)-2-(2,4-dioxotetrahydropyrimidin-1(2H)-yl) isoindoline-1,3-dione | 5-((4-(4-bromophenyl)piperazin-1-yl)methyl)-2-(2,4-dioxotetrahydropyrimidin-1(2H)-yl) isoindoline-1,3-dione |
| GT-0678 5 | 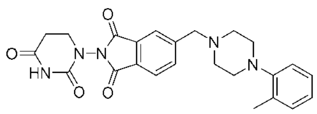 | 2-(2,4-dioxotetrahydropyrimidin-1(2H)-yl)-5-((4-(o-tolyl)piperazin-1-yl)methyl)isoindoline-1,3-dione | 2-(2,4-dioxotetrahydropyrimidin-1(2H)-yl)-5-((4-(o-tolyl)piperazin-1-yl)methyl)isoindoline-1,3-dione |
| GT-0678 6 | 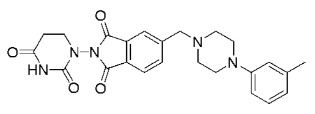 | 2-(2,4-dioxotetrahydropyrimidin-1(2H)-yl)-5-((4-(m-tolyl)piperazin-1-yl)methyl)isoindoline-1,3-dione | 2-(2,4-dioxotetrahydropyrimidin-1(2H)-yl)-5-((4-(m-tolyl)piperazin-1-yl)methyl)isoindoline-1,3-dione |
| GT-0678 7 | 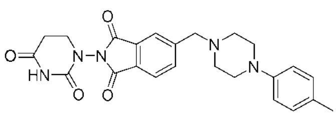 | 2-(2,4-dioxotetrahydropyrimidin-1(2H)-yl)-5-((4-(p-tolyl)piperazin-1-yl)methyl)isoindoline-1,3-dione | 2-(2,4-dioxotetrahydropyrimidin-1(2H)-yl)-5-((4-(p-tolyl)piperazin-1-yl)methyl)isoindoline-1,3-dione |
| GT-0678 8 | 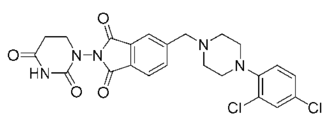 | 5-((4-(2,4-dichlorophenyl)piperazin-1-yl)methyl)-2-(2,4-dioxotetrahydropyrimidin-1(2H)-yl)isoindoline-1,3-dione | 5-((4-(2,4-dichlorophenyl)piperazin-1-yl)methyl)-2-(2,4-dioxotetrahydropyrimidin-1(2H)-yl)isoindoline-1,3-dione |

(continued)

| Compound No. | Structure of the compounds | The compounds' name | Chinese translation of the compounds' name |
|---|---|---|---|
| GT-0678 9 | 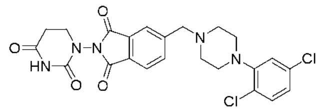 | 5-((4-(2,5-dichlorophenyl)piperazin-1-yl)methyl)-2-(2,4-dioxotetrahydropyrimidin-1(2H)-yl)isoindoline-1,3-dione | 5-((4-(2,5-dichlorophenyl)piperazin-1-yl)methyl)-2-(2,4-dioxotetrahydropyrimidin-1(2H)-yl)isoindoline-1,3-dione |
| GT-0679 0 | 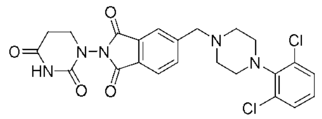 | 5-((4-(2,6-dichlorophenyl)piperazin-1-yl)methyl)-2-(2,4-dioxotetrahydropyrimidin-1(2H)-yl)isoindoline-1,3-dione | 5-((4-(2,6-dichlorophenyl)piperazin-1-yl)methyl)-2-(2,4-dioxotetrahydropyrimidin-1(2H)-yl)isoindoline-1,3-dione |
| GT-0679 1 | 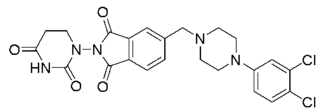 | 5-((4-(3,4-dichlorophenyl)piperazin-1-yl)methyl)-2-(2,4-dioxotetrahydropyrimidin-1(2H)-yl)isoindoline-1,3-dione | 5-((4-(3,4-dichlorophenyl)piperazin-1-yl)methyl)-2-(2,4-dioxotetrahydropyrimidin-1(2H)-yl)isoindoline-1,3-dione |
| GT-0679 2 | 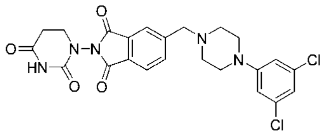 | 5-((4-(3,5-dichlorophenyl)piperazin-1-yl)methyl)-2-(2,4-dioxotetrahydropyrimidin-1(2H)-yl)isoindoline-1,3-dione | 5-((4-(3,5-dichlorophenyl)piperazin-1-yl)methyl)-2-(2,4-dioxotetrahydropyrimidin-1(2H)-yl)isoindoline-1,3-dione |
| GT-0679 3 | 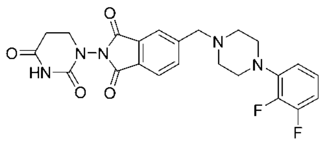 | 5-((4-(2,3-difluorophenyl)piperazin-1-yl)methyl)-2-(2,4-dioxotetrahydropyrimidin-1(2H)-yl)isoindoline-1,3-dione | 5-((4-(2,3-difluorophenyl)piperazin-1-yl)methyl)-2-(2,4-dioxotetrahydropyrimidin-1(2H)-yl)isoindoline-1,3-dione |
| GT-0679 4 | 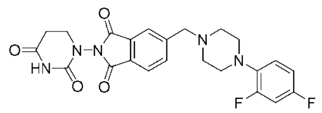 | 5-((4-(2,4-difluorophenyl)piperazin-1-yl)methyl)-2-(2,4-dioxotetrahydropyrimidin-1(2H)-yl)isoindoline-1,3-dione | 5-((4-(2,4-difluorophenyl)piperazin-1-yl)methyl)-2-(2,4-dioxotetrahydropyrimidin-1(2H)-yl)isoindoline-1,3-dione |
| GT-0679 5 | 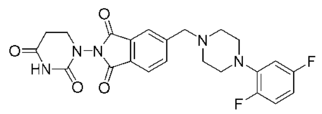 | 5-((4-(2,5-difluorophenyl)piperazin-1-yl)methyl)-2-(2,4-dioxotetrahydropyrimidin-1(2H)-yl)isoindoline-1,3-dione | 5-((4-(2,5-difluorophenyl)piperazin-1-yl)methyl)-2-(2,4-dioxotetrahydropyrimidin-1(2H)-yl)isoindoline-1,3-dione |
| GT-0679 6 | 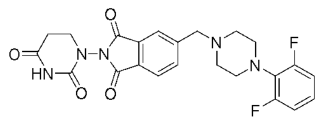 | 5-((4-(2,6-difluorophenyl)piperazin-1-yl)methyl)-2-(2,4-dioxotetrahydropyrimidin-1(2H)-yl)isoindoline-1,3-dione | 5-((4-(2,6-difluorophenyl)piperazin-1-yl)methyl)-2-(2,4-dioxotetrahydropyrimidin-1(2H)-yl)isoindoline-1,3-dione |
| GT-0679 7 | 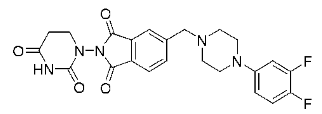 | 5-((4-(3,4-difluorophenyl)piperazin-1-yl)methyl)-2-(2,4-dioxotetrahydropyrimidin-1(2H)-yl)isoindoline-1,3-dione | 5-((4-(3,4-difluorophenyl)piperazin-1-yl)methyl)-2-(2,4-dioxotetrahydropyrimidin-1(2H)-yl)isoindoline-1,3-dione |
| GT-0679 8 | 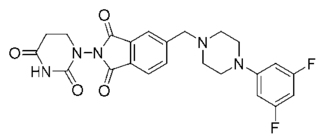 | 5-((4-(3,5-difluorophenyl)piperazin-1-yl)methyl)-2-(2,4-dioxotetrahydropyrimidin-1(2H)-yl)isoindoline-1,3-dione | 5-((4-(3,5-difluorophenyl)piperazin-1-yl)methyl)-2-(2,4-dioxotetrahydropyrimidin-1(2H)-yl)isoindoline-1,3-dione |

(continued)

| Compound No. | Structure of the compounds | The compounds' name | Chinese translation of the compounds' name |
|---|---|---|---|
| GT-0680 1 | 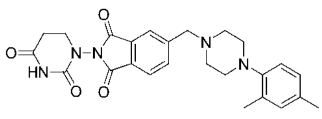 | 5-((4-(2,4-dimethylphenyl)piperazin-1-yl)methyl)-2-(2,4-dioxotetrahydropyrimidin-1(2H)-yl)isoindoline-1,3-dione | 5-((4-(2,4-dimethylphenyl)piperazin-1-yl)methyl)-2-(2,4-dioxotetrahydropyrimidin-1(2H)-yl)isoindoline-1,3-dione |
| GT-0680 2 | 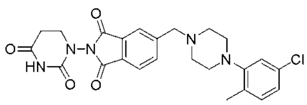 | 5-((4-(5-chloro-2-methylphenyl)piperazin-1-yl)methyl)-2-(2,4-dioxotetrahydropyrimidin-1(2H)-yl)isoindoline-1,3-dione | 5-((4-(5-chloro-2-methylphenyl)piperazin-1-yl)methyl)-2-(2,4-dioxotetrahydropyrimidin-1(2H)-yl)isoindoline-1,3-dione |
| GT-0680 3 | 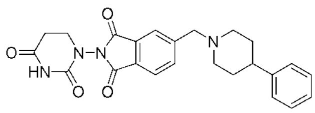 | 2-(2,4-dioxotetrahydropyrimidin-1(2H)-yl)-5-((4-phenylpiperidin-1-yl)methyl)isoindoline-1,3-dione | 2-(2,4-dioxotetrahydropyrimidin-1(2H)-yl)-5-((4-phenylpiperidin-1-yl)methyl)isoindoline-1,3-dione |
| GT-0680 4 | 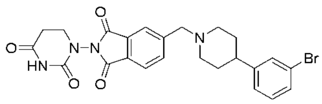 | 5-((4-(3-bromophenyl)piperidin-1-yl)methyl)-2-(2,4-dioxotetrahydropyrimidin-1(2H)-yl) isoindoline-1,3-dione | 5-((4-(3-bromophenyl)piperidin-1-yl)methyl)-2-(2,4-dioxotetrahydropyrimidin-1(2H)-yl) isoindoline-1,3-dione |
| GT-0680 5 | 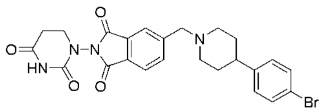 | 5-((4-(4-bromophenyl)piperidin-1-yl)methyl)-2-(2,4-dioxotetrahydropyrimidin-1(2H)-yl) isoindoline-1,3-dione | 5-((4-(4-bromophenyl)piperidin-1-yl)methyl)-2-(2,4-dioxotetrahydropyrimidin-1(2H)-yl) isoindoline-1,3-dione |
| GT-0687 5 | 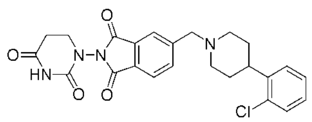 | 5-((4-(2-chlorophenyl)piperidin-1-yl)methyl)-2-(2,4-dioxotetrahydropyrimidin-1(2H)-yl) isoindoline-1,3-dione | 5-((4-(2-chlorophenyl)piperidin-1-yl)methyl)-2-(2,4-dioxotetrahydropyrimidin-1(2H)-yl) isoindoline-1,3-dione |
| GT-0687 6 | 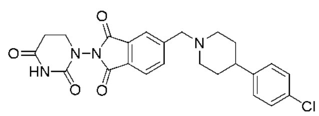 | 5-((4-(4-chlorophenyl)piperidin-1-yl)methyl)-2-(2,4-dioxotetrahydropyrimidin-1(2H)-yl) isoindoline-1,3-dione | 5-((4-(4-chlorophenyl)piperidin-1-yl)methyl)-2-(2,4-dioxotetrahydropyrimidin-1(2H)-yl) isoindoline-1,3-dione |
| GT-0687 7 | 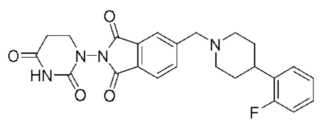 | 2-(2,4-dioxotetrahydropyrimidin-1(2H)-yl)-5-((4-(2-fluorophenyl)piperidin-1-yl)methyl) isoindoline-1,3-dione | 2-(2,4-dioxotetrahydropyrimidin-1(2H)-yl)-5-((4-(2-fluorophenyl)piperidin-1-yl)methyl) isoindoline-1,3-dione |
| GT-0687 8 | 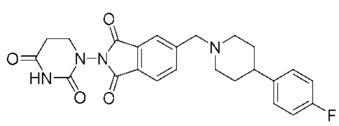 | 2-(2,4-dioxotetrahydropyrimidin-1(2H)-yl)-5-((4-(4-fluorophenyl)piperidin-1-yl)methyl) isoindoline-1,3-dione | 2-(2,4-dioxotetrahydropyrimidin-1(2H)-yl)-5-((4-(4-fluorophenyl)piperidin-1-yl)methyl) isoindoline-1,3-dione |
| GT-0687 9 | 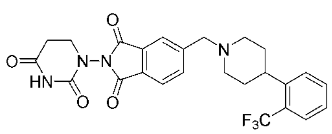 | 2-(2,4-dioxotetrahydropyrimidin-1(2H)-yl)-5-((4-(2-(trifluoromethyl)phenyl)piper idin-1-yl)methyl)isoindoline-1,3-dione | 2-(2,4-dioxotetrahydropyrimidin-1(2H)-yl)-5-((4-(2-(trifluoromethyl)phenyl)piper idin-1-yl) methyl)isoindoline-1,3-dione |
| GT-0688 0 | 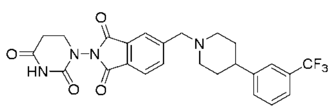 | 2-(2,4-dioxotetrahydropyrimidin-1(2H)-yl)-5-((4-(3-(trifluoromethyl)phenyl)piper idin-1-yl)methyl)isoindoline-1,3-dione | 2-(2,4-dioxotetrahydropyrimidin-1(2H)-yl)-5-((4-(3-(trifluoromethyl)phenyl)piper idin-1-yl) methyl)isoindoline-1,3-dione |

(continued)

| Compound No. | Structure of the compounds | The compounds' name | Chinese translation of the compounds' name |
|---|---|---|---|
| GT-0525 0 | 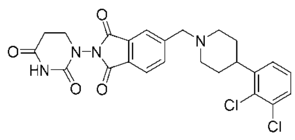 | 5-((4-(2,3-dichlorophenyl)piperidin-1-yl)methyl)-2-(2,4-dioxotetrahydropyrimidin-1(2H)-yl)isoindoline-1,3-dione | 5-((4-(2,3-dichlorophenyl)piperidin-1-yl)methyl)-2-(2,4-dioxotetrahydropyrimidin-1(2H)-yl)isoindoline-1,3-dione |
| GT-0689 5 | 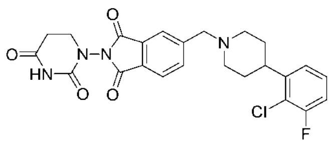 | 5-((4-(2-chloro-3-fluorophenyl)piperidin-1-yl)methyl)-2-(2,4-dioxotetrahydropyrimidin-1(2H)-yl)isoindoline-1,3-dione | 5-((4-(2-chloro-3-fluorophenyl)piperidin-1-yl)methyl)-2-(2,4-dioxotetrahydropyrimidin-1(2H)-yl)isoindoline-1,3-dione |
| GT-0689 7 | 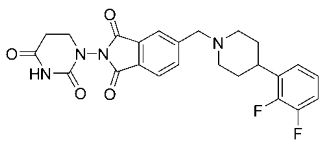 | 5-((4-(2,3-difluorophenyl)piperidin-1-yl)methyl)-2-(2,4-dioxotetrahydropyrimidin-1(2H)-yl)isoindoline-1,3-dione | 5-((4-(2,3-difluorophenyl)piperidin-1-yl)methyl)-2-(2,4-dioxotetrahydropyrimidin-1(2H)-yl)isoindoline-1,3-dione |
| GT-0524 9 | 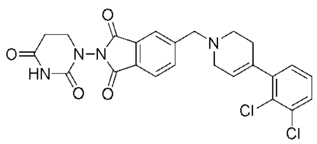 | 5-((4-(2,3-dichlorophenyl)-3,6-dihydropyridin-1(2H)-yl)methyl)-2-(2,4-dioxotetrahydropyrimidin-1(2H)-yl)isoindoline-1,3-dione | 5-((4-(2,3-dichlorophenyl)-3,6-dihydropyridin-1(2H)-yl)methyl)-2-(2,4-dioxotetrahydropyrimidin-1(2H)-yl)isoindoline-1,3-dione |
| GT-0689 6 | 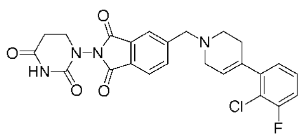 | 5-((4-(2-chloro-3-fluorophenyl)-3,6-dihydropyridin-1(2H)-yl)methyl)-2-(2,4-dioxotetrahydropyrimidin-1(2H)-yl)isoindoline-1,3-dione | 5-((4-(2-chloro-3-fluorophenyl)-3,6-dihydropyridin-1(2H)-yl)methyl)-2-(2,4-dioxotetrahydropyrimidin-1(2H)-yl)isoindoline-1,3-dione |
| GT-0535 1 | 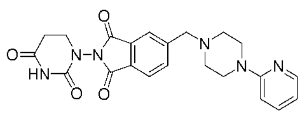 | 2-(2,4-dioxotetrahydropyrimidin-1(2H)-yl)-5-((4-(pyridin-2-yl)piperazin-1-yl)methyl)isoindoline-1,3-dione | 2-(2,4-dioxotetrahydropyrimidin-1(2H)-yl)-5-((4-(pyridin-2-yl)piperazin-1-yl)methyl)isoindoline-1,3-dione |
| GT-0535 2 | 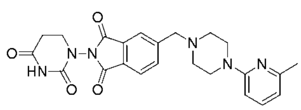 | 2-(2,4-dioxotetrahydropyrimidin-1(2H)-yl)-5-((4-(6-methylpyridin-2-yl)piperazin-1-yl)methyl)isoindoline-1,3-dione | 2-(2,4-dioxotetrahydropyrimidin-1(2H)-yl)-5-((4-(6-methylpyridin-2-yl)piperazin-1-yl)methyl)isoindoline-1,3-dione |
| GT-0535 3 | 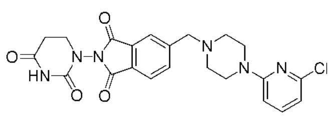 | 5-((4-(6-chloropyridin-2-yl)piperazin-1-yl)methyl)-2-(2,4-dioxotetrahydropyrimidin-1(2H)-yl)isoindoline-1,3-dione | 5-((4-(6-chloropyridin-2-yl)piperazin-1-yl)methyl)-2-(2,4-dioxotetrahydropyrimidin-1(2H)-yl)isoindoline-1,3-dione |
| GT-0535 0 | 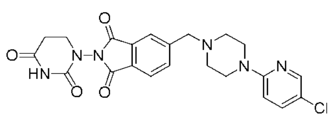 | 5-((4-(5-chloropyridin-2-yl)piperazin-1-yl)methyl)-2-(2,4-dioxotetrahydropyrimidin-1(2H)-yl)isoindoline-1,3-dione | 5-((4-(5-chloropyridin-2-yl)piperazin-1-yl)methyl)-2-(2,4-dioxotetrahydropyrimidin-1(2H)-yl)isoindoline-1,3-dione |
| GT-0535 4 | 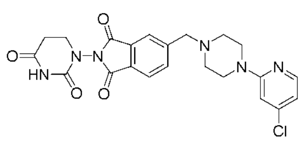 | 5-((4-(4-chloropyridin-2-yl)piperazin-1-yl)methyl)-2-(2,4-dioxotetrahydropyrimidin-1(2H)-yl)isoindoline-1,3-dione | 5-((4-(4-chloropyridin-2-yl)piperazin-1-yl)methyl)-2-(2,4-dioxotetrahydropyrimidin-1(2H)-yl)isoindoline-1,3-dione |

(continued)

| Compound No. | Structure of the compounds | The compounds' name | Chinese translation of the compounds' name |
|---|---|---|---|
| | 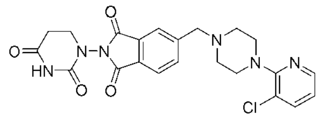 | 5-((4-(3-chloropyridin-2-yl)piperazin-1-yl)methyl)-2-(2,4-dioxotetrahydropyrimidin-1(2H)-yl)isoindoline-1,3-dione | 5-((4-(3-chloropyridin-2-yl)piperazin-1-yl)methyl)-2-(2,4-dioxotetrahydropyrimidin-1(2H)-yl)isoindoline-1,3-dione |
| GT-0535 5 | 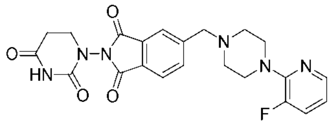 | 2-(2,4-dioxotetrahydropyrimidin-1(2H)-yl)-5-((4-(3-fluoropyridin-2-yl)piperazin-1-yl)methyl)isoindoline-1,3-dione | 2-(2,4-dioxotetrahydropyrimidin-1(2H)-yl)-5-((4-(3-fluoropyridin-2-yl)piperazin-1-yl)methyl)isoindoline-1,3-dione |
| GT-0696 4 | 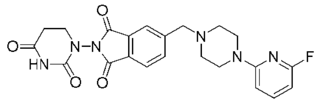 | 2-(2,4-dioxotetrahydropyrimidin-1(2H)-yl)-5-((4-(6-fluoropyridin-2-yl)piperazin-1-yl)methyl)isoindoline-1,3-dione | 2-(2,4-dioxotetrahydropyrimidin-1(2H)-yl)-5-((4-(6-fluoropyridin-2-yl)piperazin-1-yl)methyl)isoindoline-1,3-dione |
| GT-0696 5 | 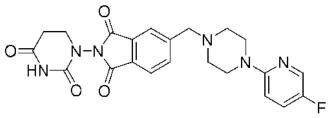 | 2-(2,4-dioxotetrahydropyrimidin-1(2H)-yl)-5-((4-(5-fluoropyridin-2-yl)piperazin-1-yl)methyl)isoindoline-1,3-dione | 2-(2,4-dioxotetrahydropyrimidin-1(2H)-yl)-5-((4-(5-fluoropyridin-2-yl)piperazin-1-yl)methyl)isoindoline-1,3-dione |
| GT-0696 6 | 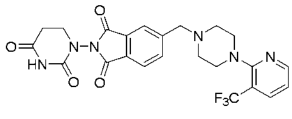 | 2-(2,4-dioxotetrahydropyrimidin-1(2H)-yl)-5-((4-(3-(trifluoromethyl)pyridin-2-yl)piperazin-1-yl)methyl)isoindoline-1,3-dione | 2-(2,4-dioxotetrahydropyrimidin-1(2H)-yl)-5-((4-(3-(trifluoromethyl)pyridin-2-yl)piperazin-1-yl)methyl)isoindoline-1,3-dione |
| GT-0696 7 | 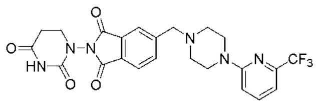 | 2-(2,4-dioxotetrahydropyrimidin-1(2H)-yl)-5-((4-(6-(trifluoromethyl)pyridin-2-yl)piperazin-1-yl)methyl)isoindoline-1,3-dione | 2-(2,4-dioxotetrahydropyrimidin-1(2H)-yl)-5-((4-(6-(trifluoromethyl)pyridin-2-yl)piperazin-1-yl)methyl)isoindoline-1,3-dione |
| GT-0696 8 | 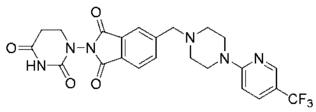 | 2-(2,4-dioxotetrahydropyrimidin-1(2H)-yl)-5-((4-(5-(trifluoromethyl)pyridin-2-yl)piperazin-1-yl)methyl)isoindoline-1,3-dione | 2-(2,4-dioxotetrahydropyrimidin-1(2H)-yl)-5-((4-(5-(trifluoromethyl)pyridin-2-yl)piperazin-1-yl)methyl)isoindoline-1,3-dione |
| | 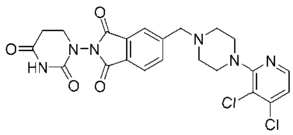 | 5-((4-(3,4-dichloropyridin-2-yl)piperazin-1-yl)methyl)-2-(2,4-dioxotetrahydropyrimidin-1(2H)-yl)isoindoline-1,3-dione | 5-((4-(3,4-dichloropyridin-2-yl)piperazin-1-yl)methyl)-2-(2,4-dioxotetrahydropyrimidin-1(2H)-yl)isoindoline-1,3-dione |
| GT-0696 9 | 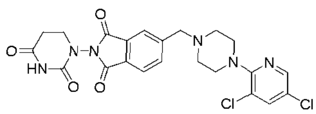 | 5-((4-(3,5-dichloropyridin-2-yl)piperazin-1-yl)methyl)-2-(2,4-dioxotetrahydropyrimidin-1(2H)-yl)isoindoline-1,3-dione | 5-((4-(3,5-dichloropyridin-2-yl)piperazin-1-yl)methyl)-2-(2,4-dioxotetrahydropyrimidin-1(2H)-yl)isoindoline-1,3-dione |
| | 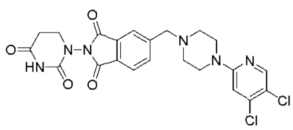 | 5-((4-(4,5-dichloropyridin-2-yl)piperazin-1-yl)methyl)-2-(2,4-dioxotetrahydropyrimidin-1(2H)-yl)isoindoline-1,3-dione | 5-((4-(4,5-dichloropyridin-2-yl)piperazin-1-yl)methyl)-2-(2,4-dioxotetrahydropyrimidin-1(2H)-yl)isoindoline-1,3-dione |

(continued)

| Compound No. | Structure of the compounds | The compounds' name | Chinese translation of the compounds' name |
|---|---|---|---|
| GT-0716 2 | 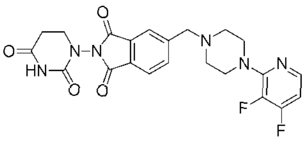 | 5-((4-(3,4-difluoropyridin-2-yl) piperazin-1-yl)methyl)-2-(2,4-dioxotetrahydropyrimidin-1(2H)-yl)isoindoline-1,3-dione | 5-((4-(3,4-difluoropyridin-2-yl) piperazin-1-yl)methyl)-2-(2,4-dioxotetrahydropyrimidin-1(2H)-yl)isoindoline-1,3-dione |
| GT-0819 0 | 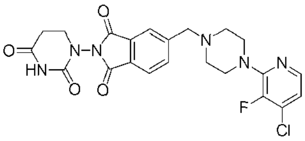 | 5-((4-(4-chloro-3-fluoropyridin-2-yl)piperazin-1-yl) methyl)-2-(2,4-dioxotetrahy-dropyrimidin-1(2H)-yl)isoindo-line-1,3-dione | 5-((4-(4-chloro-3-fluoropyridin-2-yl)piperazin-1-yl) methyl)-2-(2,4-dioxotetrahy-dropyrimidin-1(2H)-yl)isoindo-line-1,3-dione |
| GT-0697 0 | 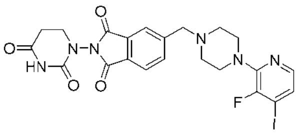 | 2-(2,4-dioxotetrahydropyrimi-din-1(2H)-yl)-5-((4-(3-fluoro-4-iodopyridin-2-yl)piperazin-1-yl)methyl)isoindoline-1,3-dione | 2-(2,4-dioxotetrahydropyrimidin-1(2H)-yl)-5-((4-(3-fluoro-4-iodopyridin-2-yl)piperazin-1-yl)methyl)isoindoline-1,3-dione |
| GT-0729 1 | 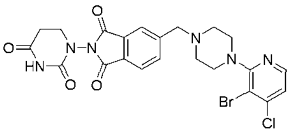 | 5-((4-(3-bromo-4-chloropyridin-2-yl)piperazin-1-yl) methyl)-2-(2,4-dioxotetrahy-dropyrimidin-1(2H)-yl)isoindo-line-1,3-dione | 5-((4-(3-bromo-4-chloropyridin-2-yl)piperazin-1-yl) methyl)-2-(2,4-dioxotetrahy-dropyrimidin-1(2H)-yl)isoindo-line-1,3-dione |
| GT-0702 6 | 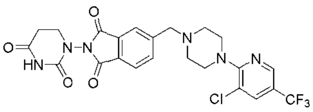 | 5-((4-(3-chloro-5-(trifluoro-methyl)pyridin-2-yl)pipera-zin-1-yl)methyl)-2-(2,4-dioxotetrahydropyrimidin-1(2H)-yl) isoindoline-1,3-dione | 5-((4-(3-chloro-5-(trifluoro-methyl)pyridin-2-yl)pipera-zin-1-yl)methyl)-2-(2,4-dioxotetrahydropyrimidin-1(2H)-yl) isoindoline-1,3-dione |
| | 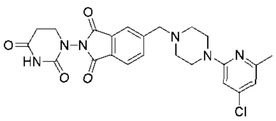 | 5-((4-(4-chloro-6-methylpyridin-2-yl)piperazin-1-yl) methyl)-2-(2,4-dioxotetrahy-dropyrimidin-1(2H)-yl)isoindo-line-1,3-dione | 5-((4-(4-chloro-6-methylpyridin-2-yl)piperazin-1-yl) methyl)-2-(2,4-dioxotetrahy-dropyrimidin-1(2H)-yl)isoindo-line-1,3-dione |
| GT-0697 1 | 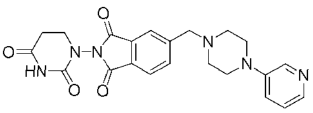 | 2-(2,4-dioxotetrahydropyrimi-din-1(2H)-yl)-5-((4-(pyridin-3-yl)piperazin-1-yl)methyl)isoindoline-1,3-dione | 2-(2,4-dioxotetrahydropyrimidin-1(2H)-yl)-5-((4-(pyridin-3-yl)piperazin-1-yl)methyl)isoindoline-1,3-dione |
| GT-0702 7 | 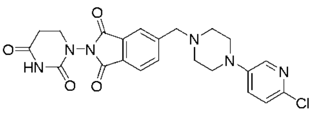 | 5-((4-(6-chloropyridin-3-yl)piperazin-1-yl)methyl)-2-(2,4-di-oxotetrahydropyrimi-din-1(2H)-yl)isoindoline-1,3-dione | 5-((4-(6-chloropyridin-3-yl)piperazin-1-yl)methyl)-2-(2,4-di-oxotetrahydropyrimi-din-1(2H)-yl)isoindoline-1,3-dione |
| GT-0697 2 | 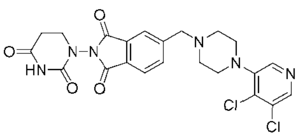 | 5-((4-(4,5-dichloropyridin-3-yl) piperazin-1-yl)methyl)-2-(2,4-dioxotetrahydropyrimidin-1(2H)-yl)isoindoline-1,3-dione | 5-((4-(4,5-dichloropyridin-3-yl) piperazin-1-yl)methyl)-2-(2,4-dioxotetrahydropyrimidin-1(2H)-yl)isoindoline-1,3-dione |
| GT-0697 3 | 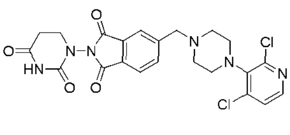 | 5-((4-(2,4-dichloropyridin-3-yl) piperazin-1-yl)methyl)-2-(2,4-dioxotetrahydropyrimidin-1(2H)-yl)isoindoline-1,3-dione | 5-((4-(2,4-dichloropyridin-3-yl) piperazin-1-yl)methyl)-2-(2,4-dioxotetrahydropyrimidin-1(2H)-yl)isoindoline-1,3-dione |

(continued)

| Compound No. | Structure of the compounds | The compounds' name | Chinese translation of the compounds' name |
|---|---|---|---|
| GT-0707 3 | 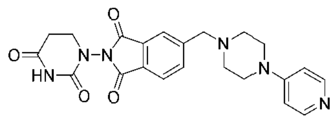 | 2-(2,4-dioxotetrahydropyrimidin-1(2H)-yl)-5-((4-(pyridin-4-yl)piperazin-1-yl)methyl)isoindoline-1,3-dione | 2-(2,4-dioxotetrahydropyrimidin-1(2H)-yl)-5-((4-(pyridin-4-yl)piperazin-1-yl)methyl)isoindoline-1,3-dione |
| GT-0716 1 | 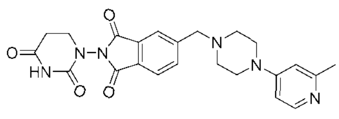 | 2-(2,4-dioxotetrahydropyrimidin-1(2H)-yl)-5-((4-(2-methylpyridin-4-yl)piperazin-1-yl)methyl)isoindoline-1,3-dione | 2-(2,4-dioxotetrahydropyrimidin-1(2H)-yl)-5-((4-(2-methylpyridin-4-yl)piperazin-1-yl)methyl)isoindoline-1,3-dione |
| GT-0716 3 | 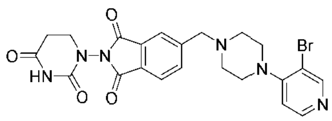 | 5-((4-(3-bromopyridin-4-yl)piperazin-1-yl)methyl)-2-(2,4-dioxotetrahydropyrimidin-1(2H)-yl)isoindoline-1,3-dione | 5-((4-(3-bromopyridin-4-yl)piperazin-1-yl)methyl)-2-(2,4-dioxotetrahydropyrimidin-1(2H)-yl)isoindoline-1,3-dione |
| GT-0716 4 | 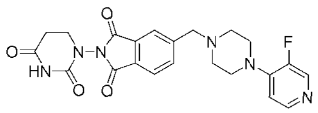 | 2-(2,4-dioxotetrahydropyrimidin-1(2H)-yl)-5-((4-(3-fluoropyridin-4-yl)piperazin-1-yl)methyl)isoindoline-1,3-dione | 2-(2,4-dioxotetrahydropyrimidin-1(2H)-yl)-5-((4-(3-fluoropyridin-4-yl)piperazin-1-yl)methyl)isoindoline-1,3-dione |
| GT-0716 5 | 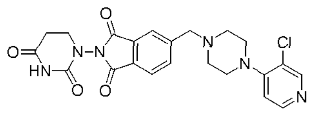 | 5-((4-(3-chloropyridin-4-yl)piperazin-1-yl)methyl)-2-(2,4-dioxotetrahydropyrimidin-1(2H)-yl)isoindoline-1,3-dione | 5-((4-(3-chloropyridin-4-yl)piperazin-1-yl)methyl)-2-(2,4-dioxotetrahydropyrimidin-1(2H)-yl)isoindoline-1,3-dione |
| | 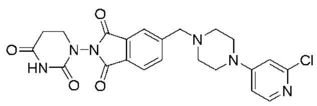 | 5-((4-(2-chloropyridin-4-yl)piperazin-1-yl)methyl)-2-(2,4-dioxotetrahydropyrimidin-1(2H)-yl)isoindoline-1,3-dione | 5-((4-(2-chloropyridin-4-yl)piperazin-1-yl)methyl)-2-(2,4-dioxotetrahydropyrimidin-1(2H)-yl)isoindoline-1,3-dione |
| GT-0716 6 | 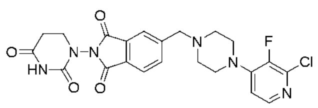 | 5-((4-(2-chloro-3-fluoropyridin-4-yl)piperazin-1-yl)methyl)-2-(2,4-dioxotetrahydropyrimidin-1(2H)-yl)isoindoline-1,3-dione | 5-((4-(2-chloro-3-fluoropyridin-4-yl)piperazin-1-yl)methyl)-2-(2,4-dioxotetrahydropyrimidin-1(2H)-yl)isoindoline-1,3-dione |
| GT-0716 7 | 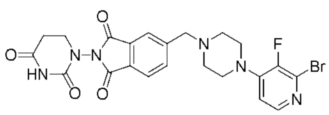 | 5-((4-(2-bromo-3-fluoropyridin-4-yl)piperazin-1-yl)methyl)-2-(2,4-dioxotetrahydropyrimidin-1(2H)-yl)isoindoline-1,3-dione | 5-((4-(2-bromo-3-fluoropyridin-4-yl)piperazin-1-yl)methyl)-2-(2,4-dioxotetrahydropyrimidin-1(2H)-yl)isoindoline-1,3-dione |
| GT-0716 8 | 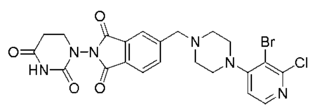 | 5-((4-(3-bromo-2-chloropyridin-4-yl)piperazin-1-yl)methyl)-2-(2,4-dioxotetrahydropyrimidin-1(2H)-yl)isoindoline-1,3-dione | 5-((4-(3-bromo-2-chloropyridin-4-yl)piperazin-1-yl)methyl)-2-(2,4-dioxotetrahydropyrimidin-1(2H)-yl)isoindoline-1,3-dione |
| GT-0728 7 | 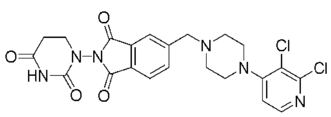 | 5-((4-(2,3-dichloropyridin-4-yl)piperazin-1-yl)methyl)-2-(2,4-dioxotetrahydropyrimidin-1(2H)-yl)isoindoline-1,3-dione | 5-((4-(2,3-dichloropyridin-4-yl)piperazin-1-yl)methyl)-2-(2,4-dioxotetrahydropyrimidin-1(2H)-yl)isoindoline-1,3-dione |

(continued)

| Compound No. | Structure of the compounds | The compounds' name | Chinese translation of the compounds' name |
|---|---|---|---|
| GT-0728 8 | 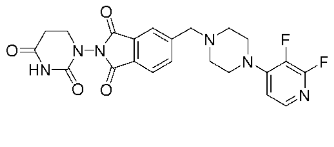 | 5-((4-(2,3-difluoropyridin-4-yl) piperazin-1-yl)methyl)-2-(2,4-dioxotetrahydropyrimidin-1(2H)-yl)isoindoline-1,3-dione | 5-((4-(2,3-difluoropyridin-4-yl) piperazin-1-yl)methyl)-2-(2,4-dioxotetrahydropyrimidin-1(2H)-yl)isoindoline-1,3-dione |
| | 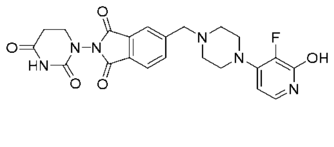 | 2-(2,4-dioxotetrahydropyrimidin-1 (2H)-yl)-5-((4-(3-fluoro-2-hydroxypyridin-4-yl) piperazin-1-yl)methyl)isoindoline-1,3-dione | 2-(2,4-dioxotetrahydropyrimidin-1(2H)-yl)-5-((4-(3-fluoro-2-hydroxypyridin-4-yl)piperazin-1-yl)methyl)isoindoline-1,3-dione |
| GT-0728 9 | 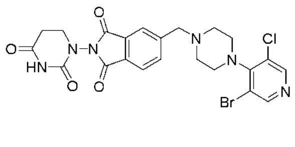 | 5-((4-(3-bromo-5-chloropyridin-4-yl)piperazin-1-yl) methyl)-2-(2,4-dioxotetrahydropyrimidin-1(2H)-yl)isoindoline-1,3-dione | 5-((4-(3-bromo-5-chloropyridin-4-yl)piperazin-1-yl) methyl)-2-(2,4-dioxotetrahydropyrimidin-1(2H)-yl)isoindoline-1,3-dione |
| GT-0729 0 | 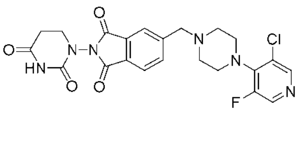 | 5-((4-(3-chloro-5-fluoropyridin-4-yl)piperazin-1-yl) methyl)-2-(2,4-dioxotetrahydropyrimidin-1(2H)-yl)isoindoline-1,3-dione | 5-((4-(3-chloro-5-fluoropyridin-4-yl)piperazin-1-yl) methyl)-2-(2,4-dioxotetrahydropyrimidin-1(2H)-yl)isoindoline-1,3-dione |
| GT-0703 8 | 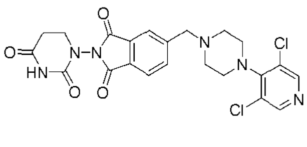 | 5-((4-(3,5-dichloropyridin-4-yl) piperazin-1-yl)methyl)-2-(2,4-dioxotetrahydropyrimidin-1(2H)-yl)isoindoline-1,3-dione | 5-((4-(3,5-dichloropyridin-4-yl) piperazin-1-yl)methyl)-2-(2,4-dioxotetrahydropyrimidin-1(2H)-yl)isoindoline-1,3-dione |
| GT-0703 9 | 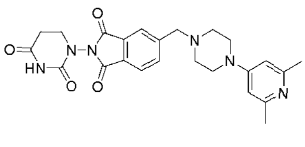 | 5-((4-(2,6-dimethylpyridin-4-yl)piperazin-1-yl) methyl)-2-(2,4-dioxotetrahydropyrimidin-1 (2H)-yl)isoindoline-1,3-dione | 5-((4-(2,6-dimethylpyridin-4-yl)piperazin-1-yl) methyl)-2-(2,4-dioxotetrahydropyrimidin-1(2H)-yl)isoindoline-1,3-dione |
| GT-0704 1 | 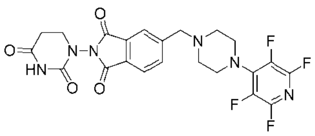 | 2-(2,4-dioxotetrahydropyrimidin-1(2H)-yl)-5-((4-(perfluoropyridin-4-yl)piperazin-1-yl) methyl)isoindoline-1,3-dione | 2-(2,4-dioxotetrahydropyrimidin-1(2H)-yl)-5-((4-(perfluoropyridin-4-yl)piperazin-1-yl) methyl)isoindoline-1,3-dione |
| | 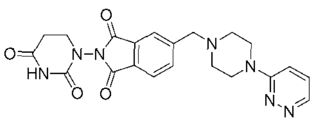 | 2-(2,4-dioxotetrahydropyrimidin-1(2H)-yl)-5-((4-(pyridazin-3-yl)piperazin-1-yl)methyl) isoindoline-1,3-dione | 2-(2,4-dioxotetrahydropyrimidin-1 (2H)-yl)-5-((4-(pyridazin-3-yl)piperazin-1-yl)methyl) isoindoline-1,3-dione |
| GT-0704 3 | 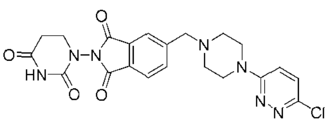 | 5-((4-(6-chloropyridazin-3-yl) piperazin-1-yl)methyl)-2-(2,4-dioxotetrahydropyrimidin-1(2H)-yl)isoindoline-1,3-dione | 5-((4-(6-chloropyridazin-3-yl) piperazin-1-yl)methyl)-2-(2,4-dioxotetrahydropyrimidin-1(2H)-yl)isoindoline-1,3-dione |
| GT-0728 5 | 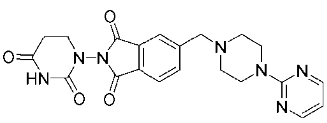 | 2-(2,4-dioxotetrahydropyrimidin-1(2H)-yl)-5-((4-(pyrimidin-2-yl)piperazin-1-yl)methyl) isoindoline-1,3-dione | 2-(2,4-dioxotetrahydropyrimidin-1(2H)-yl)-5-((4-(pyrimidin-2-yl)piperazin-1-yl)methyl) isoindoline-1,3-dione |

(continued)

| Compound No. | Structure of the compounds | The compounds' name | Chinese translation of the compounds' name |
|---|---|---|---|
| GT-0728 6 | 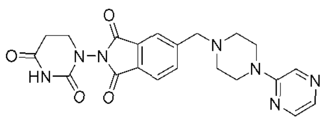 | 2-(2,4-dioxotetrahydropyrimidin-1(2H)-yl)-5-((4-(pyrazin-2-yl)piperazin-1-yl)methyl)isoindoline-1,3-dione | 2-(2,4-dioxotetrahydropyrimidin-1(2H)-yl)-5-((4-(pyrazin-2-yl)piperazin-1-yl)methyl)isoindoline-1,3-dione |
| GT-0703 4 | 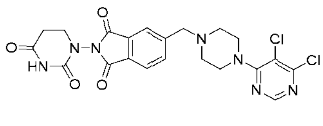 | 5-((4-(5,6-dichloropyrimidin-4-yl)piperazin-1-yl) methyl)-2-(2,4-dioxotetrahydropyrimidin-1(2H)-yl)isoindoline-1,3-dione | 5-((4-(5,6-dichloropyrimidin-4-yl)piperazin-1-yl) methyl)-2-(2,4-dioxotetrahydropyrimidin-1(2H)-yl)isoindoline-1,3-dione |
| GT-0703 5 | 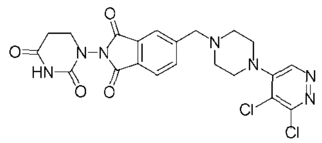 | 5-((4-(5,6-dichloropyridazin-4-yl)piperazin-1-yl) methyl)-2-(2,4-dioxotetrahydropyrimidin-1(2H)-yl)isoindoline-1,3-dione | 5-((4-(5,6-dichloropyridazin-4-yl)piperazin-1-yl) methyl)-2-(2,4-dioxotetrahydropyrimidin-1(2H)-yl)isoindoline-1,3-dione |
| GT-0703 6 | 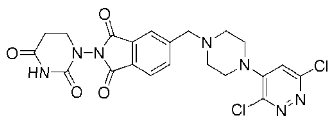 | 5-((4-(3,6-dichloropyridazin-4-yl)piperazin-1-yl) methyl)-2-(2,4-dioxotetrahydropyrimidin-1(2H)-yl)isoindoline-1,3-dione | 5-((4-(3,6-dichloropyridazin-4-yl)piperazin-1-yl) methyl)-2-(2,4-dioxotetrahydropyrimidin-1(2H)-yl)isoindoline-1,3-dione |
| GT-0703 7 | 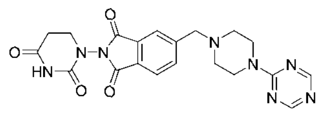 | 5-((4-(1,3,5-triazin-2-yl)piperazin-1-yl)methyl)-2-(2,4-dioxotetrahydropyrimidin-1(2H)-yl) isoindoline-1,3-dione | 5-((4-(1,3,5-triazin-2-yl)piperazin-1-yl)methyl)-2-(2,4-dioxotetrahydropyrimidin-1(2H)-yl) isoindoline-1,3-dione |
| GT-0638 9 | 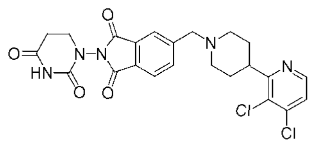 | 5-((4-(3,4-dichloropyridin-2-yl) piperidin-1-yl)methyl)-2-(2,4-dioxotetrahydropyrimidin-1(2H)-yl)isoindoline-1,3-dione | 5-((4-(3,4-dichloropyridin-2-yl) piperidin-1-yl)methyl)-2-(2,4-dioxotetrahydropyrimidin-1(2H)-yl)isoindoline-1,3-dione |
| GT-0639 1 | 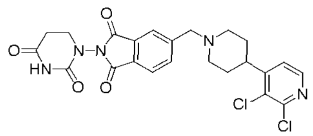 | 5-((4-(2,3-dichloropyridin-4-yl) piperidin-1-yl)methyl)-2-(2,4-dioxotetrahydropyrimidin-1(2H)-yl)isoindoline-1,3-dione | 5-((4-(2,3-dichloropyridin-4-yl) piperidin-1-yl)methyl)-2-(2,4-dioxotetrahydropyrimidin-1(2H)-yl)isoindoline-1,3-dione |
| GT-0749 3 | 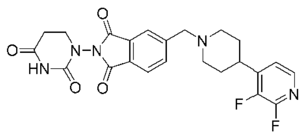 | 5-((4-(2,3-difluoropyridin-4-yl) piperidin-1-yl)methyl)-2-(2,4-dioxotetrahydropyrimidin-1(2H)-yl)isoindoline-1,3-dione | 5-((4-(2,3-difluoropyridin-4-yl) piperidin-1-yl)methyl)-2-(2,4-dioxotetrahydropyrimidin-1(2H)-yl)isoindoline-1,3-dione |
| | 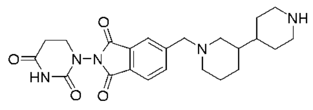 | 5-([3,4'-bipiperidin]-1-ylmethyl)-2-(2,4-dioxotetrahydropyrimidin-1(2H)-yl)isoindoline-1,3-dione | 5-([3,4'-bipiperidin]-1-ylmethyl)-2-(2,4-dioxotetrahydropyrimidin-1(2H)-yl)isoindoline-1,3-dione |
| | 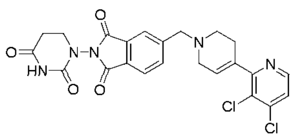 | 5-((3,4-dichloro-3',6'-dihydro-[2,4'-bipyridin]-1'(2'H)-yl) methyl)-2-(2,4-dioxotetrahydropyrimidin-1(2H)-yl)isoindoline-1,3-dione | 5-((3,4-dichloro-3',6'-dihydro-[2,4'-bipyridin]-1'(2'H)-yl) methyl)-2-(2,4-dioxotetrahydropyrimidin-1(2H)-yl)isoindoline-1,3-dione |

(continued)

| Compound No. | Structure of the compounds | The compounds' name | Chinese translation of the compounds' name |
|---|---|---|---|
| | 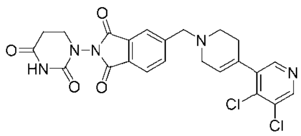 | 5-((4,5-dichloro-3',6'-dihydro-[3,4'-bipyridin]-1'(2'H)-yl)methyl)-2-(2,4-dioxotetrahydropyrimidin-1(2H)-yl)isoindoline-1,3-dione | 5-((4,5-dichloro-3',6'-dihydro-[3,4'-bipyridin]-1'(2'H)-yl)methyl)-2-(2,4-dioxotetrahydropyrimidin-1(2H)-yl)isoindoline-1,3-dione |
| GT-0749 1 | 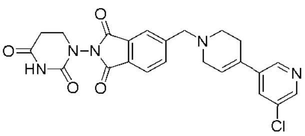 | 5-((5-chloro-3',6'-dihydro-[3,4'-bipyridin]-1'(2'H)-yl)methyl)-2-(2,4-dioxotetrahydropyrimidin-1(2H)-yl)isoindoline-1,3-dione | 5-((5-chloro-3',6'-dihydro-[3,4'-bipyridin]-1'(2'H)-yl)methyl)-2-(2,4-dioxotetrahydropyrimidin-1(2H)-yl)isoindoline-1,3-dione |
| GT-0744 0 | 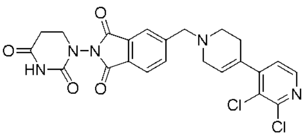 | 5-((2',3'-dichloro-3,6-dihydro-[4,4'-bipyridin]-1(2H)-yl)methyl)-2-(2,4-dioxotetrahydropyrimidin-1(2H)-yl)isoindoline-1,3-dione | 5-((2',3'-dichloro-3,6-dihydro-[4,4'-bipyridin]-1(2H)-yl)methyl)-2-(2,4-dioxotetrahydropyrimidin-1(2H)-yl)isoindoline-1,3-dione |
| GT-0749 2 | 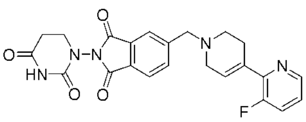 | 2-(2,4-dioxotetrahydropyrimidin-1(2H)-yl)-5-((3-fluoro-3',6'-dihydro-[2,4'-bipyridin]-1'(2'H)-yl)methyl)isoindoline-1,3-dione | 2-(2,4-dioxotetrahydropyrimidin-1(2H)-yl)-5-((3-fluoro-3',6'-dihydro-[2,4'-bipyridin]-1'(2'H)-yl)methyl)isoindoline-1,3-dione |
| GT-0749 4 | 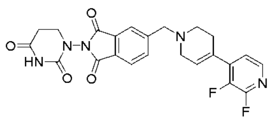 | 5-((2',3'-difluoro-3,6-dihydro-[4,4'-bipyridin]-1(2H)-yl)methyl)-2-(2,4-dioxotetrahydropyrimidin-1(2H)-yl)isoindoline-1,3-dione | 5-((2',3'-difluoro-3,6-dihydro-[4,4'-bipyridin]-1(2H)-yl)methyl)-2-(2,4-dioxotetrahydropyrimidin-1(2H)-yl)isoindoline-1,3-dione |
| GT-0525 1 | 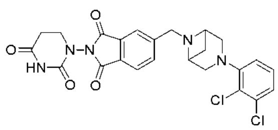 | 5-((3-(2,3-dichlorophenyl)-3,6-diazabicyclo[3.1.1]heptan-6-yl)methyl)-2-(2,4-dioxotetrahydropyrimidin-1(2H)-yl)isoindoline-1,3-dione | 5-((3-(2,3-dichlorophenyl)-3,6-diazabicyclo[3.1.1]heptan-6-yl)methyl)-2-(2,4-dioxotetrahydropyrimidin-1(2H)-yl)isoindoline-1,3-dione |
| GT-0743 4 | 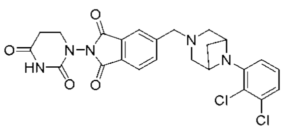 | 5-((6-(2,3-dichlorophenyl)-3,6-diazabicyclo[3.1.1]heptan-3-yl)methyl)-2-(2,4-dioxotetrahydropyrimidin-1(2H)-yl)isoindoline-1,3-dione | 5-((6-(2,3-dichlorophenyl)-3,6-diazabicyclo[3.1.1]heptan-3-yl)methyl)-2-(2,4-dioxotetrahydropyrimidin-1(2H)-yl)isoindoline-1,3-dione |
| GT-0743 5 | 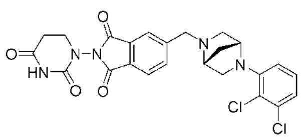 | 5-(((1R,4R)-5-(2,3-dichlorophenyl)-2,5-diazabicyclo[2.2.1]heptan-2-yl)methyl)-2-(2,4-dioxotetrahydropyrimidin-1(2H)-yl)isoindoline-1,3-dione | 5-(((1R,4R)-5-(2,3-dichlorophenyl)-2,5-diazabicyclo[2.2.1]heptan-2-yl)methyl)-2-(2,4-dioxotetrahydropyrimidin-1(2H)-yl)isoindoline-1,3-dione |
| GT-0525 2 | 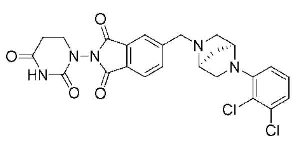 | 5-(((1S,4S)-5-(2,3-dichlorophenyl)-2,5-diazabicyclo[2.2.1]heptan-2-yl)methyl)-2-(2,4-dioxotetrahydropyrimidin-1(2H)-yl)isoindoline-1,3-dione | 5-(((1S,4S)-5-(2,3-dichlorophenyl)-2,5-diazabicyclo[2.2.1]heptan-2-yl)methyl)-2-(2,4-dioxotetrahydropyrimidin-1(2H)-yl)isoindoline-1,3-dione |

(continued)

| Compound No. | Structure of the compounds | The compounds' name | Chinese translation of the compounds' name |
|---|---|---|---|
| GT-0743 6 | 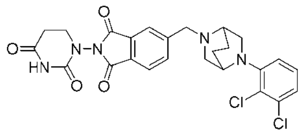 | 5-((5-(2,3-dichlorophenyl)-2,5-diazabicyclo[2.2.2]octan-2-yl)methyl)-2-(2,4-dioxotetrahydropyrimidin-1(2H)-yl) isoindoline-1,3-dione | 5-((5-(2,3-dichlorophenyl)-2,5-diazabicyclo [2.2.2] octan-2-yl) methyl)-2-(2,4-dioxotetrahydropyrimidin-1(2H)-yl)isoindoline-1,3-dione |
| GT-0743 7 | 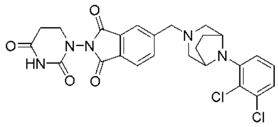 | 5-((8-(2,3-dichlorophenyl)-3,8-diazabicyclo[3.2.1]octan-3-yl)methyl)-2-(2,4-dioxotetrahydropyrimidin-1(2H)-yl) isoindoline-1,3-dione | 5-((8-(2,3-dichlorophenyl)-3,8-diazabicyclo[3.2.1]octan-3-yl) methyl)-2-(2,4-dioxotetrahydropyrimidin-1(2H)-yl)isoindoline-1,3-dione |
| GT-0743 8 | 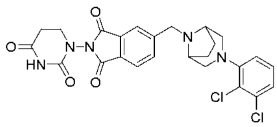 | 5-((3-(2,3-dichlorophenyl)-3,8-diazabicyclo[3.2.1]octan-8-yl)methyl)-2-(2,4-dioxotetrahydropyrimidin-1(2H)-yl) isoindoline-1,3-dione | 5-((3-(2,3-dichlorophenyl)-3,8-diazabicyclo[3.2.1]octan-8-yl) methyl)-2-(2,4-dioxotetrahydropyrimidin-1(2H)-yl)isoindoline-1,3-dione |
| GT-0743 9 | 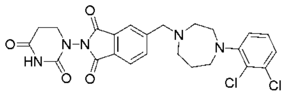 | 5-((4-(2,3-dichlorophenyl)-1,4-diazepan-1-yl) methyl)-2-(2,4-dioxotetrahydropyrimidin-1(2H)-yl)isoindoline-1,3-dione | 5-((4-(2,3-dichlorophenyl)-1,4-diazepan-1-yl)methyl)-2-(2,4-dioxotetrahydropyrimidin-1(2H)-yl)isoindoline-1,3-dione |
| GT-0517 0 | 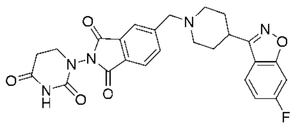 | 2-(2,4-dioxotetrahydropyrimidin-1(2H)-yl)-5-((4-(6-fluorobenzo[d]isoxazol-3-yl)piperidin-1-yl)methyl)isoindoline-1,3-dione | 2-(2,4-dioxotetrahydropyrimidin-1(2H)-yl)-5-((4-(6-fluorobenzo[d]isoxazol-3-yl)piperidin-1-yl)methyl)isoindoline-1,3-dione |
| GT-0752 2 | 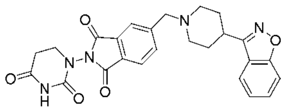 | 5-((4-(benzo[d]isoxazol-3-yl) piperidin-1-yl)methyl)-2-(2,4-dioxotetrahydropyrimidin-1(2H)-yl)isoindoline-1,3-dione | 5-((4-(benzo[d]isoxazol-3-yl) piperidin-1-yl)methyl)-2-(2,4-dioxotetrahydropyrimidin-1(2H)-yl)isoindoline-1,3-dione |
| GT-0752 3 | 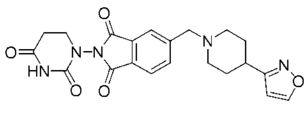 | 2-(2,4-dioxotetrahydropyrimidin-1(2H)-yl)-5-((4-(isoxazol-3-yl)piperidin-1-yl)methyl) isoindoline-1,3-dione | 2-(2,4-dioxotetrahydropyrimidin-1(2H)-yl)-5-((4-(isoxazol-3-yl)piperidin-1-yl)methyl) isoindoline-1,3-dione |
| GT-0752 4 | 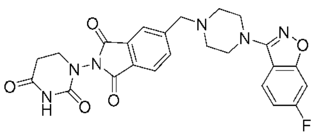 | 2-(2,4-dioxotetrahydropyrimidin-1(2H)-yl)-5-((4-(6-fluorobenzo[d]isoxazol-3-yl)piperazin-1-yl)methyl)isoindoline-1,3-dione | 2-(2,4-dioxotetrahydropyrimidin-1(2H)-yl)-5-((4-(6-fluorobenzo[d]isoxazol-3-yl)piperazin-1-yl)methyl)isoindoline-1,3-dione |
| GT-0752 5 | 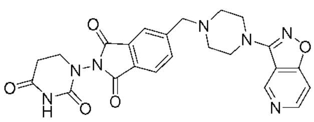 | 2-(2,4-dioxotetrahydropyrimidin-1(2H)-yl)-5-((4-(isoxazolo [4,5-c]pyridin-3-yl)piperazin-1-yl)methyl)isoindoline-1,3-dione | 2-(2,4-dioxotetrahydropyrimidin-1(2H)-yl)-5-((4-(isoxazolo [4,5-c]pyridin-3-yl)piperazin-1-yl)methyl)isoindoline-1,3-dione |
| GT-0752 6 | 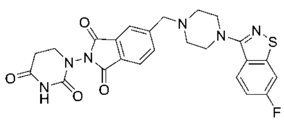 | 2-(2,4-dioxotetrahydropyrimidin-1(2H)-yl)-5-((4-(6-fluorobenzo[d]isothiazol-3-yl)piperazin-1-yl)methyl)isoindoline-1,3-dione | 2-(2,4-dioxotetrahydropyrimidin-1(2H)-yl)-5-((4-(6-fluorobenzo[d]isothiazol-3-yl)piperazin-1-yl)methyl)isoindoline-1,3-dione |

(continued)

| Compound No. | Structure of the compounds | The compounds' name | Chinese translation of the compounds' name |
|---|---|---|---|
| GT-0752 7 | 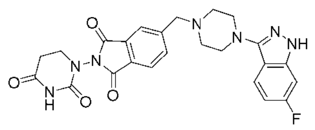 | 2-(2,4-dioxotetrahydropyrimidin-1(2H)-yl)-5-((4-(6-fluoro-1H-indazol-3-yl)piperazin-1-yl)methyl)isoindoline-1,3-dione | 2-(2,4-dioxotetrahydropyrimidin-1(2H)-yl)-5-((4-(6-fluoro-1H-indazol-3-yl)piperazin-1-yl)methyl)isoindoline-1,3-dione |
| GT-0752 8 | 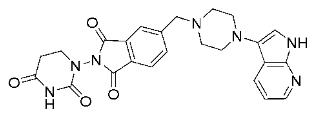 | 5-((4-(1H-pyrrolo[2,3-b]pyridin-3-yl)piperazin-1-yl)methyl)-2-(2,4-dioxotetrahydropyrimidin-1(2H)-yl)isoindoline-1,3-dione | 5-((4-(1H-pyrrolo[2,3-b]pyridin-3-yl)piperazin-1-yl)methyl)-2-(2,4-dioxotetrahydropyrimidin-1(2H)-yl)isoindoline-1,3-dione |
| GT-0525 9 | 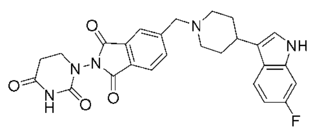 | 2-(2,4-dioxotetrahydropyrimidin-1(2H)-yl)-5-((4-(6-fluoro-1H-indol-3-yl)piperidin-1-yl)methyl)isoindoline-1,3-dione | 2-(2,4-dioxotetrahydropyrimidin-1(2H)-yl)-5-((4-(6-fluoro-1H-indol-3-yl)piperidin-1-yl)methyl)isoindoline-1,3-dione |
| GT-0753 0 | 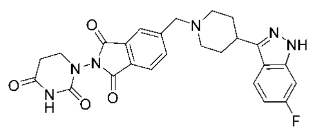 | 2-(2,4-dioxotetrahydropyrimidin-1(2H)-yl)-5-((4-(6-fluoro-1H-indazol-3-yl)piperidin-1-yl)methyl)isoindoline-1,3-dione | 2-(2,4-dioxotetrahydropyrimidin-1(2H)-yl)-5-((4-(6-fluoro-1H-indazol-3-yl)piperidin-1-yl)methyl)isoindoline-1,3-dione |
| GT-0758 1 | 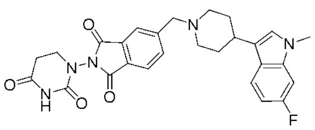 | 2-(2,4-dioxotetrahydropyrimidin-1(2H)-yl)-5-((4-(6-fluoro-1-methyl-1H-indol-3-yl)piperidin-1-yl)methyl)isoindoline-1,3-dione | 2-(2,4-dioxotetrahydropyrimidin-1 (2H)-yl)-5-((4-(6-fluoro-1-methyl-1H-indol-3-yl)piperidin-1-yl)methyl)isoindoline-1,3-dione |
| GT-0526 0 | 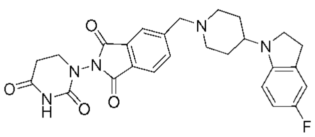 | 2-(2,4-dioxotetrahydropyrimidin-1(2H)-yl)-5-((4-(5-fluoroindolin-1-yl)piperidin-1-yl)methyl)isoindoline-1,3-dione | 2-(2,4-dioxotetrahydropyrimidin-1(2H)-yl)-5-((4-(5-fluoroindolin-1-yl)piperidin-1-yl)methyl)isoindoline-1,3-dione |
| GT-0752 9 | 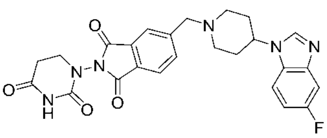 | 2-(2,4-dioxotetrahydropyrimidin-1(2H)-yl)-5-((4-(5-fluoro-1H-benzo[d]imidazol-1-yl)piperidin-1-yl)methyl)isoindoline-1,3-dione | 2-(2,4-dioxotetrahydropyrimidin-1(2H)-yl)-5-((4-(5-fluoro-1H-benzo[d]imidazol-1-yl)piperidin-1-yl)methyl)isoindoline-1,3-dione |
| GT-0758 3 | 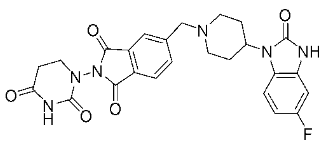 | 2-(2,4-dioxotetrahydropyrimidin-1(2H)-yl)-5-((4-(5-fluoro-2-oxo-2,3-dihydro-1H-benzo[d]imidazol-1-yl)piperidin-1-yl)methyl)isoindoline-1,3-dione | 2-(2,4-dioxotetrahydropyrimidin-1(2H)-yl)-5-((4-(5-fluoro-2-oxo-2,3-dihydro-1H-benzo[d]imidazol-1-yl)piperidin-1-yl)methyl)isoindoline-1,3-dione |
| GT-0758 2 | 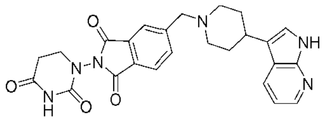 | 5-((4-(1H-pyrrolo[2,3-b]pyridin-3-yl)piperidin-1-yl)methyl)-2-(2,4-dioxotetrahydropyrimidin-1(2H)-yl)isoindoline-1,3-dione | 5-((4-(1H-pyrrolo[2,3-b]pyridin-3-yl)piperidin-1-yl)methyl)-2-(2,4-dioxotetrahydropyrimidin-1(2H)-yl)isoindoline-1,3-dione |
| GT-0518 1 | 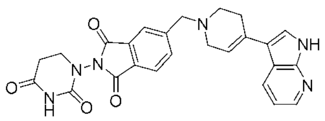 | 5-((4-(1H-pyrrolo[2,3-b]pyridin-3-yl)-3,6-dihydropyridin-1(2H)-yl)methyl)-2-(2,4-dioxotetrahydropyrimidin-1(2H)-yl)isoindoline-1,3-dione | 5-((4-(1H-pyrrolo[2,3-b]pyridin-3-yl)-3,6-dihydropyridin-1(2H)-yl)methyl)-2-(2,4-dioxotetrahydropyrimidin-1(2H)-yl)isoindoline-1,3-dione |

(continued)

| Compound No. | Structure of the compounds | The compounds' name | Chinese translation of the compounds' name |
|---|---|---|---|
| GT-0758 0 | 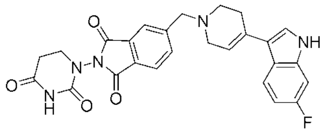 | 2-(2,4-dioxotetrahydropyrimidin-1(2H)-yl)-5-((4-(6-fluoro-1H-indol-3-yl)-3,6-dihydropyridin-1(2H)-yl)methyl)isoindoline-1,3-dione | 2-(2,4-dioxotetrahydropyrimidin-1(2H)-yl)-5-((4-(6-fluoro-1H-indol-3-yl)-3,6-dihydropyridin-1(2H)-yl)methyl)isoindoline-1,3-dione |
| GT-0527 6 | 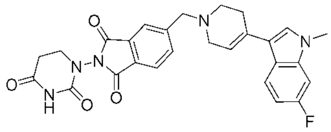 | 2-(2,4-dioxotetrahydropyrimidin-1(2H)-yl)-5-((4-(6-fluoro-1-methyl-1H-indol-3-yl)-3,6-dihydropyridin-1(2H)-yl)methyl) isoindoline-1,3-dione | 2-(2,4-dioxotetrahydropyrimidin-1(2H)-yl)-5-((4-(6-fluoro-1-methyl-1H-indol-3-yl)-3,6-dihydropyridin-1(2H)-yl)methyl) isoindoline-1,3-dione |
| GT-0764 3 | 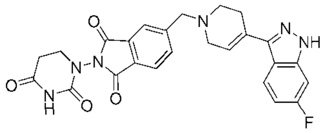 | 2-(2,4-dioxotetrahydropyrimidin-1(2H)-yl)-5-((4-(6-fluoro-1H-indazol-3-yl)-3,6-dihydropyridin-1(2H)-yl)methyl) isoindoline-1,3-dione | 2-(2,4-dioxotetrahydropyrimidin-1(2H)-yl)-5-((4-(6-fluoro-1H-indazol-3-yl)-3,6-dihydropyridin-1(2H)-yl)methyl) isoindoline-1,3-dione |
| GT-0768 1 | 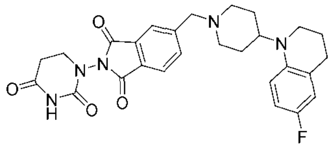 | 2-(2,4-dioxotetrahydropyrimidin-1(2H)-yl)-5-((4-(6-fluoro-3,4-dihydroquinolin-1(2H)-yl)piperidin-1-yl) methyl)isoindoline-1,3-dione | 2-(2,4-dioxotetrahydropyrimidin-1(2H)-yl)-5-((4-(6-fluoro-3,4-dihydroquinolin-1(2H)-yl)piperidin-1-yl) methyl)isoindoline-1,3-dione |
| GT-0768 2 | 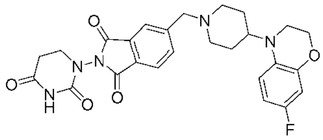 | 2-(2,4-dioxotetrahydropyrimidin-1(2H)-yl)-5-((4-(7-fluoro-2,3-dihydro-4H-benzo [b][1,4]oxazin-4-yl)piperidin-1-yl)methyl)isoindoline-1,3-dione | 2-(2,4-dioxotetrahydropyrimidin-1(2H)-yl)-5-((4-(7-fluoro-2,3-dihydro-4H-benzo [b][1,4]oxazin-4-yl)piperidin-1-yl)methyl)isoindoline-1,3-dione |
| GT-0768 3 | 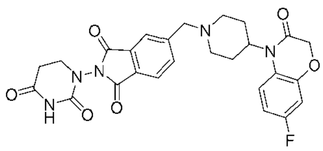 | 2-(2,4-dioxotetrahydropyrimidin-1(2H)-yl)-5-((4-(7-fluoro-3-oxo-2,3-dihydro-4H-benzo[b] [1,4]oxazin-4-yl)piperidin-1-yl) methyl)isoindoline-1,3-dione | 2-(2,4-dioxotetrahydropyrimidin-1(2H)-yl)-5-((4-(7-fluoro-3-oxo-2,3-dihydro-4H-benzo[b] [1,4]oxazin-4-yl)piperidin-1-yl) methyl)isoindoline-1,3-dione |
| GT-0768 4 | 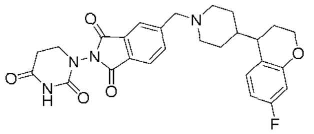 | 2-(2,4-dioxotetrahydropyrimidin-1(2H)-yl)-5-((4-(7-fluorochroman-4-yl)piperidin-1-yl) methyl)isoindoline-1,3-dione | 2-(2,4-dioxotetrahydropyrimidin-1(2H)-yl)-5-((4-(7-fluorochroman-4-yl)piperidin-1-yl) methyl)isoindoline-1,3-dione |
| GT-0768 5 | 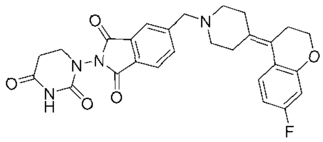 | 2-(2,4-dioxotetrahydropyrimidin-1(2H)-yl)-5-((4-(7-fluorochroman-4-ylidene)piperidin-1-yl)methyl)isoindoline-1,3-dione | 2-(2,4-dioxotetrahydropyrimidin-1(2H)-yl)-5-((4-(7-fluorochroman-4-ylidene)piperidin-1-yl)methyl)isoindoline-1,3-dione |
| GT-0768 6 | 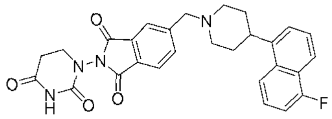 | 2-(2,4-dioxotetrahydropyrimidin-1(2H)-yl)-5-((4-(5-fluoronaphthalen-1-yl)piperidin-1-yl) methyl)isoindoline-1,3-dione | 2-(2,4-dioxotetrahydropyrimidin-1(2H)-yl)-5-((4-(5-fluoronaphthalen-1-yl)piperidin-1-yl) methyl)isoindoline-1,3-dione |
| GT-0768 9 | 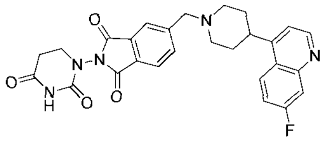 | 2-(2,4-dioxotetrahydropyrimidin-1(2H)-yl)-5-((4-(7-fluoroquinolin-4-yl)piperidin-1-yl) methyl)isoindoline-1,3-dione | 2-(2,4-dioxotetrahydropyrimidin-1(2H)-yl)-5-((4-(7-fluoroquinolin-4-yl)piperidin-1-yl) methyl)isoindoline-1,3-dione |

(continued)

| Compound No. | Structure of the compounds | The compounds' name | Chinese translation of the compounds' name |
|---|---|---|---|
| GT-0768 8 | 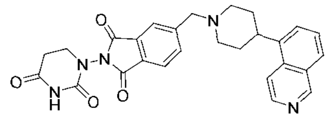 | 2-(2,4-dioxotetrahydropyrimidin-1(2H)-yl)-5-((4-(isoquinolin-5-yl)piperidin-1-yl)methyl) isoindoline-1,3-dione | 2-(2,4-dioxotetrahydropyrimidin-1(2H)-yl)-5-((4-(isoquinolin-5-yl)piperidin-1-yl)methyl) isoindoline-1,3-dione |
| GT-0768 7 | 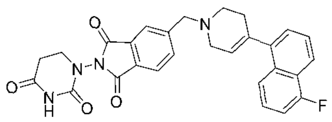 | 2-(2,4-dioxotetrahydropyrimidin-1(2H)-yl)-5-((4-(5-fluoronaphthalen-1-yl)-3,6-dihydropyridin-1(2H)-yl)methyl)isoindoline-1,3-dione | 2-(2,4-dioxotetrahydropyrimidin-1(2H)-yl)-5-((4-(5-fluoronaphthalen-1-yl)-3,6-dihydropyridin-1(2H)-yl)methyl)isoindoline-1,3-dione |
| GT-0526 1 | 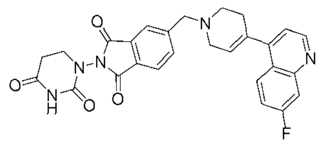 | 2-(2,4-dioxotetrahydropyrimidin-1(2H)-yl)-5-((4-(7-fluoroquinolin-4-yl)-3,6-dihydropyridin-1(2H)-yl)methyl)isoindoline-1,3-dione | 2-(2,4-dioxotetrahydropyrimidin-1(2H)-yl)-5-((4-(7-fluoroquinolin-4-yl)-3,6-dihydropyridin-1(2H)-yl)methyl)isoindoline-1,3-dione |
| GT-0527 5 | 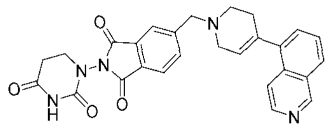 | 2-(2,4-dioxotetrahydropyrimidin-1(2H)-yl)-5-((4-(isoquinolin-5-yl)-3,6-dihydropyridin-1(2H)-yl)methyl)isoindoline-1,3-dione | 2-(2,4-dioxotetrahydropyrimidin-1(2H)-yl)-5-((4-(isoquinolin-5-yl)-3,6-dihydropyridin-1(2H)-yl)methyl)isoindoline-1,3-dione |
| GT-0771 5 | 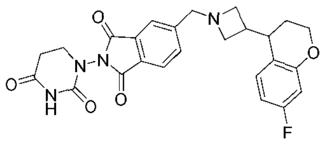 | 2-(2,4-dioxotetrahydropyrimidin-1(2H)-yl)-5-((3-(7-fluorochroman-4-yl)azetidin-1-yl) methyl)isoindoline-1,3-dione | 2-(2,4-dioxotetrahydropyrimidin-1(2H)-yl)-5-((3-(7-fluorochroman-4-yl)azetidin-1-yl) methyl)isoindoline-1,3-dione |
| GT-0788 8 | 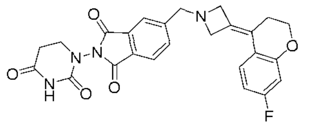 | 2-(2,4-dioxotetrahydropyrimidin-1(2H)-yl)-5-((3-(7-fluorochroman-4-ylidene)azetidin-1-yl)methyl)isoindoline-1,3-dione | 2-(2,4-dioxotetrahydropyrimidin-1(2H)-yl)-5-((3-(7-fluorochroman-4-ylidene)azetidin-1-yl)methyl)isoindoline-1,3-dione |
| GT-0771 8 | 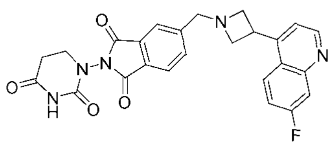 | 2-(2,4-dioxotetrahydropyrimidin-1(2H)-yl)-5-((3-(7-fluoroquinolin-4-yl)azetidin-1-yl) methyl)isoindoline-1,3-dione | 2-(2,4-dioxotetrahydropyrimidin-1(2H)-yl)-5-((3-(7-fluoroquinolin-4-yl)azetidin-1-yl) methyl)isoindoline-1,3-dione |
| GT-0527 8 | 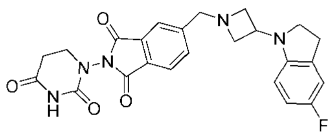 | 2-(2,4-dioxotetrahydropyrimidin-1(2H)-yl)-5-((3-(5-fluoroindolin-1-yl)azetidin-1-yl) methyl)isoindoline-1,3-dione | 2-(2,4-dioxotetrahydropyrimidin-1(2H)-yl)-5-((3-(5-fluoroindolin-1-yl)azetidin-1-yl) methyl)isoindoline-1,3-dione |
| | 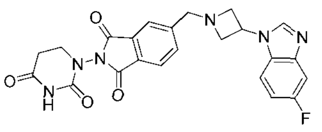 | 2-(2,4-dioxotetrahydropyrimidin-1(2H)-yl)-5-((3-(5-fluoro-1H-benzo[d]imidazol-1-yl) azetidin-1-yl)methyl)isoindoline-1,3-dione | 2-(2,4-dioxotetrahydropyrimidin-1(2H)-yl)-5-((3-(5-fluoro-1H-benzo[d]imidazol-1-yl) azetidin-1-yl)methyl)isoindoline-1,3-dione |
| GT-0809 0 | 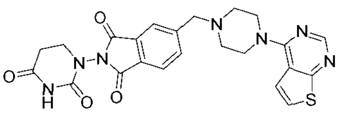 | 2-(2,4-dioxotetrahydropyrimidin-1(2H)-yl)-5-((4-(thieno [2,3-d]pyrimidin-4-yl)piperazin-1-yl)methyl)isoindoline-1,3-dione | 2-(2,4-dioxotetrahydropyrimidin-1(2H)-yl)-5-((4-(thieno [2,3-d]pyrimidin-4-yl)piperazin-1-yl)methyl)isoindoline-1,3-dione |

(continued)

| Compound No. | Structure of the compounds | The compounds' name | Chinese translation of the compounds' name |
|---|---|---|---|
| GT-0809 1 | 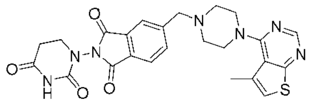 | 2-(2,4-dioxotetrahydropyrimidin-1(2H)-yl)-5-((4-(5-methylthieno[2,3-d]pyrimidin-4-yl)piperazin-1-yl)methyl) isoindoline-1,3-dione | 2-(2,4-dioxotetrahydropyrimidin-1(2H)-yl)-5-((4-(5-methylthieno[2,3-d]pyrimidin-4-yl)piperazin-1-yl)methyl) isoindoline-1,3-dione |
| GT-0809 2 | 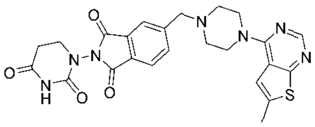 | 2-(2,4-dioxotetrahydropyrimidin-1(2H)-yl)-5-((4-(6-methylthieno[2,3-d]pyrimidin-4-yl)piperazin-1-yl)methyl) isoindoline-1,3-dione | 2-(2,4-dioxotetrahydropyrimidin-1(2H)-yl)-5-((4-(6-methylthieno[2,3-d]pyrimidin-4-yl)piperazin-1-yl)methyl) isoindoline-1,3-dione |
| GT-0809 3 | 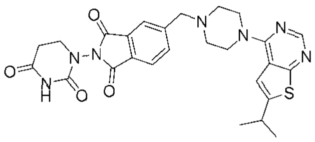 | 2-(2,4-dioxotetrahydropyrimidin-1(2H)-yl)-5-((4-(6-isopropylthieno[2,3-d]pyrimidin-4-yl) piperazin-1-yl)methyl)isoindoline-1,3-dione | 2-(2,4-dioxotetrahydropyrimidin-1(2H)-yl)-5-((4-(6-isopropylthieno[2,3-d]pyrimidin-4-yl) piperazin-1-yl)methyl)isoindoline-1,3-dione |
| GT-0809 4 | 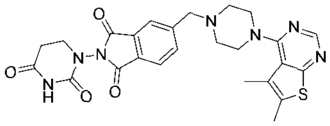 | 5-((4-(5,6-dimethylthieno[2,3-d]pyrimidin-4-yl)piperazin-1-yl)methyl)-2-(2,4-dioxotetrahydropyrimidin-1(2H)-yl)isoindoline-1,3-dione | 5-((4-(5,6-dimethylthieno[2,3-d]pyrimidin-4-yl)piperazin-1-yl)methyl)-2-(2,4-dioxotetrahydropyrimidin-1(2H)-yl)isoindoline-1,3-dione |
| GT-0809 7 | 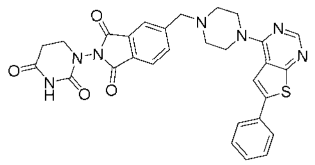 | 2-(2,4-dioxotetrahydropyrimidin-1(2H)-yl)-5-((4-(6-phenylthieno[2,3-d]pyrimidin-4-yl) piperazin-1-yl)methyl)isoindoline-1,3-dione | 2-(2,4-dioxotetrahydropyrimidin-1(2H)-yl)-5-((4-(6-phenylthieno[2,3-d]pyrimidin-4-yl) piperazin-1-yl)methyl)isoindoline-1,3-dione |
| GT-0809 6 | 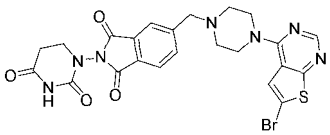 | 5-((4-(6-bromothieno[2,3-d] pyrimidin-4-yl)piperazin-1-yl) methyl)-2-(2,4-dioxotetrahydropyrimidin-1(2H)-yl)isoindoline-1,3-dione | 5-((4-(6-bromothieno[2,3-d] pyrimidin-4-yl)piperazin-1-yl) methyl)-2-(2,4-dioxotetrahydropyrimidin-1 (2H)-yl)isoindoline-1,3-dione |
| GT-0809 8 | 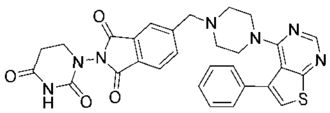 | 2-(2,4-dioxotetrahydropyrimidin-1(2H)-yl)-5-((4-(5-phenylthieno[2,3-d]pyrimidin-4-yl) piperazin-1-yl)methyl)isoindoline-1,3-dione | 2-(2,4-dioxotetrahydropyrimidin-1(2H)-yl)-5-((4-(5-phenylthieno[2,3-d]pyrimidin-4-yl) piperazin-1-yl)methyl)isoindoline-1,3-dione |
| GT-0810 2 | 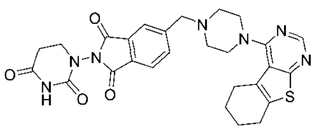 | 2-(2,4-dioxotetrahydropyrimidin-1(2H)-yl)-5-((4-(5,6,7,8-tetrahydrobenzo[4,5]thieno [2 ,3-d]pyrimidin-4-yl)piperazin-1-yl)methyl)isoindoline-1,3-dione | 2-(2,4-dioxotetrahydropyrimidin-1(2H)-yl)-5-((4-(5,6,7,8-tetrahydrobenzo[4,5]thieno [2 ,3-d]pyrimidin-4-yl)piperazin-1-yl)methyl)isoindoline-1,3-dione |
| GT-0810 3 | 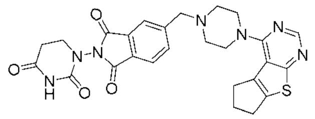 | 5-((4-(6,7-dihydro-5H-cyclopenta[4,5]thieno[2,3-d]pyrimidin-4-yl)piperazin-1-yl) methyl)-2-(2,4-dioxotetrahydropyrimidin-1 (2H)-yl)isoindoline-1,3-dione | 5-((4-(6,7-dihydro-5H-cyclopenta[4,5]thieno[2,3-d]pyrimidin-4-yl)piperazin-1-yl) methyl)-2-(2,4-dioxotetrahydropyrimidin-1(2H)-yl)isoindoline-1,3-dione |

(continued)

| Compound No. | Structure of the compounds | The compounds' name | Chinese translation of the compounds' name |
|---|---|---|---|
| GT-0809 9 | 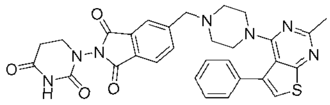 | 2-(2,4-dioxotetrahydropyrimidin-1(2H)-yl)-5-((4-(2-methyl-5-phenylthieno[2,3-d]pyrimidin-4-yl)piperazin-1-yl)methyl)isoindoline-1,3-dione | 2-(2,4-dioxotetrahydropyrimidin-1(2H)-yl)-5-((4-(2-methyl-5-phenylthieno[2,3-d]pyrimidin-4-yl)piperazin-1-yl)methyl)isoindoline-1,3-dione |
| GT-0810 1 | 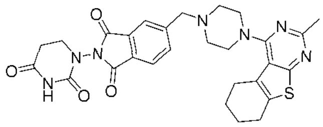 | 2-(2,4-dioxotetrahydropyrimidin-1(2H)-yl)-5-((4-(2-methyl-5,6,7,8-tetrahydrobenzo[4,5]thieno[2 ,3-d]pyrimidin-4-yl)piperazin-1-yl)methyl)isoindoline-1,3-dione | 2-(2,4-dioxotetrahydropyrimidin-1(2H)-yl)-5-((4-(2-methyl-5,6,7,8-tetrahydrobenzo[4,5]thieno[2 ,3-d]pyrimidin-4-yl)piperazin-1-yl)methyl)isoindoline-1,3-dione |
| GT-0810 4 | 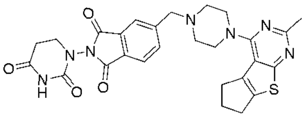 | 2-(2,4-dioxotetrahydropyrimidin-1(2H)-yl)-5-((4-(2-methyl-6,7-dihydro-5H-cyclopenta[4,5]thieno[2,3-d]pyrimidin-4-yl)piperazin-1-yl)methyl)isoindoline-1,3-dione | 2-(2,4-dioxotetrahydropyrimidin-1(2H)-yl)-5-((4-(2-methyl-6,7-dihydro-5H-cyclopenta[4,5]thieno[2,3-d]pyrimidin-4-yl)piperazin-1-yl)methyl)isoindoline-1,3-dione |
| GT-0810 0 | 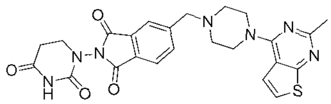 | 2-(2,4-dioxotetrahydropyrimidin-1(2H)-yl)-5-((4-(2-methylthieno[2,3-d]pyrimidin-4-yl)piperazin-1-yl)methyl)isoindoline-1,3-dione | 2-(2,4-dioxotetrahydropyrimidin-1(2H)-yl)-5-((4-(2-methylthieno[2,3-d]pyrimidin-4-yl)piperazin-1-yl)methyl)isoindoline-1,3-dione |
| GT-0810 5 | 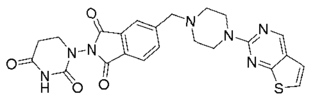 | 2-(2,4-dioxotetrahydropyrimidin-1(2H)-yl)-5-((4-(thieno[2,3-d]pyrimidin-2-yl)piperazin-1-yl)methyl)isoindoline-1,3-dione | 2-(2,4-dioxotetrahydropyrimidin-1(2H)-yl)-5-((4-(thieno[2,3-d]pyrimidin-2-yl)piperazin-1-yl)methyl)isoindoline-1,3-dione |
| GT-0809 5 | 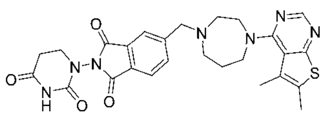 | 5-((4-(5,6-dimethylthieno[2,3-d]pyrimidin-4-yl)-1,4-diazepan-1-yl)methyl)-2-(2,4-dioxotetrahydropyrimidin-1(2H)-yl)isoindoline-1,3-dione | 5-((4-(5,6-dimethylthieno[2,3-d]pyrimidin-4-yl)-1,4-diazepan-1-yl)methyl)-2-(2,4-dioxotetrahydropyrimidin-1(2H)-yl)isoindoline-1,3-dione |
| GT-0810 6 | 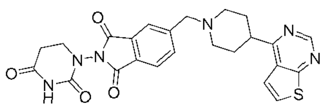 | 2-(2,4-dioxotetrahydropyrimidin-1(2H)-yl)-5-((4-(thieno[2,3-d]pyrimidin-4-yl)piperidin-1-yl)methyl)isoindoline-1,3-dione | 2-(2,4-dioxotetrahydropyrimidin-1(2H)-yl)-5-((4-(thieno[2,3-d]pyrimidin-4-yl)piperidin-1-yl)methyl)isoindoline-1,3-dione |
| GT-0810 7 | 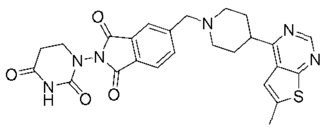 | 2-(2,4-dioxotetrahydropyrimidin-1(2H)-yl)-5-((4-(6-methylthieno[2,3-d]pyrimidin-4-yl)piperidin-1-yl)methyl)isoindoline-1,3-dione | 2-(2,4-dioxotetrahydropyrimidin-1(2H)-yl)-5-((4-(6-methylthieno[2,3-d]pyrimidin-4-yl)piperidin-1-yl)methyl)isoindoline-1,3-dione |
| | 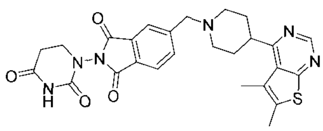 | 5-((4-(5,6-dimethylthieno[2,3-d]pyrimidin-4-yl)piperidin-1-yl)methyl)-2-(2,4-dioxotetrahydropyrimidin-1(2H)-yl)isoindoline-1,3-dione | 5-((4-(5,6-dimethylthieno[2,3-d]pyrimidin-4-yl)piperidin-1-yl)methyl)-2-(2,4-dioxotetrahydropyrimidin-1 (2H)-yl)isoindoline-1,3-dione |

(continued)

| Compound No. | Structure of the compounds | The compounds' name | Chinese translation of the compounds' name |
|---|---|---|---|
| GT-0810 8 | 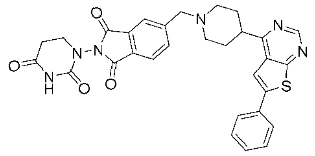 | 2-(2,4-dioxotetrahydropyrimidin-1(2H)-yl)-5-((4-(6-phenylthieno[2,3-d]pyrimidin-4-yl) piperidin-1-yl)methyl)isoindoline-1,3-dione | 2-(2,4-dioxotetrahydropyrimidin-1(2H)-yl)-5-((4-(6-phenylthieno[2,3-d]pyrimidin-4-yl) piperidin-1-yl)methyl)isoindoline-1,3-dione |
| GT-0810 9 | 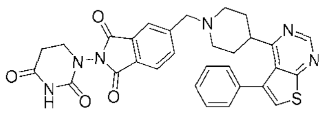 | 2-(2,4-dioxotetrahydropyrimidin-1(2H)-yl)-5-((4-(5-phenylthieno[2,3-d]pyrimidin-4-yl) piperidin-1-yl)methyl)isoindoline-1,3-dione | 2-(2,4-dioxotetrahydropyrimidin-1(2H)-yl)-5-((4-(5-phenylthieno[2,3-d]pyrimidin-4-yl) piperidin-1-yl)methyl)isoindoline-1,3-dione |
| GT-0842 4 | 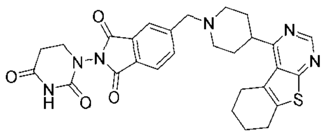 | 2-(2,4-dioxotetrahydropyrimidin-1(2H)-yl)-5-((4-(5,6,7,8-tetrahydrobenzo[4,5]thieno [2 ,3-d]pyrimidin-4-yl)piperidin-1-yl)methyl)isoindoline-1,3-dione | 2-(2,4-dioxotetrahydropyrimidin-1(2H)-yl)-5-((4-(5,6,7,8-tetrahydrobenzo[4,5]thieno [2 ,3-d]pyrimidin-4-yl)piperidin-1-yl)methyl)isoindoline-1,3-dione |
| GT-0842 5 | 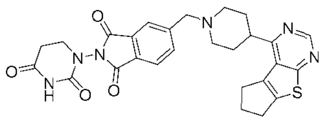 | 5-((4-(6,7-dihydro-5H-cyclopenta[4,5]thieno[2,3-d]pyrimidin-4-yl)piperidin-1-yl) methyl)-2-(2,4-dioxotetrahydropyrimidin-1 (2H)-yl)isoindoline-1,3-dione | 5-((4-(6,7-dihydro-5H-cyclopenta[4,5]thieno[2,3-d]pyrimidin-4-yl)piperidin-1-yl) methyl)-2-(2,4-dioxotetrahydropyrimidin-1 (2H)-yl)isoindoline-1,3-dione |
| GT-0811 0 | 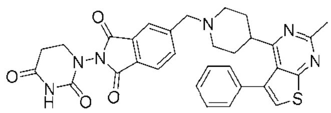 | 2-(2,4-dioxotetrahydropyrimidin-1 (2H)-yl)-5-((4-(2-methyl-5-phenylthieno[2,3-d] pyrimidin-4-yl)piperidin-1-yl) methyl)isoindoline-1,3-dione | 2-(2,4-dioxotetrahydropyrimidin-1(2H)-yl)-5-((4-(2-methyl-5-phenylthieno[2,3-d] pyrimidin-4-yl)piperidin-1-yl) methyl)isoindoline-1,3-dione |
| GT-0811 1 | 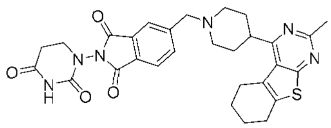 | 2-(2,4-dioxotetrahydropyrimidin-1(2H)-yl)-5-((4-(2-methyl-5,6,7,8-tetrahydrobenzo[4,5]thieno[2 ,3-d]pyrimidin-4-yl)piperidin-1-yl)methyl) isoindoline-1,3-dione | 2-(2,4-dioxotetrahydropyrimidin-1(2H)-yl)-5-((4-(2-methyl-5,6,7,8-tetrahydrobenzo[4,5]thieno[2 ,3-d]pyrimidin-4-yl)piperidin-1-yl)methyl) isoindoline-1,3-dione |
| GT-0842 6 | 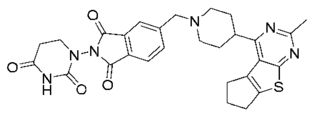 | 2-(2,4-dioxotetrahydropyrimidin-1(2H)-yl)-5-((4-(2-methyl-6,7-dihydro-5H-cyclopenta[4,5]thieno[2,3-d]pyrimidin-4-yl)piperidin-1-yl)methyl) isoindoline-1,3-dione | 2-(2,4-dioxotetrahydropyrimidin-1(2H)-yl)-5-((4-(2-methyl-6,7-dihydro-5H-cyclopenta[4,5]thieno[2,3-d]pyrimidin-4-yl)piperidin-1-yl)methyl) isoindoline-1,3-dione |
| GT-0527 2 | 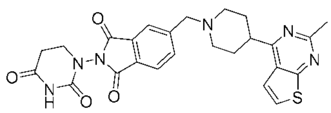 | 2-(2,4-dioxotetrahydropyrimidin-1(2H)-yl)-5-((4-(2-methylthieno[2,3-d]pyrimidin-4-yl)piperidin-1-yl)methyl) isoindoline-1,3-dione | 2-(2,4-dioxotetrahydropyrimidin-1(2H)-yl)-5-((4-(2-methylthieno[2,3-d]pyrimidin-4-yl)piperidin-1-yl)methyl) isoindoline-1,3-dione |
| GT-0842 7 | 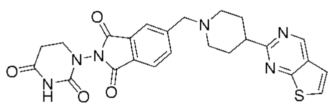 | 2-(2,4-dioxotetrahydropyrimidin-1(2H)-yl)-5-((4-(thieno [2,3-d]pyrimidin-2-yl)piperidin-1-yl)methyl)isoindoline-1,3-dione | 2-(2,4-dioxotetrahydropyrimidin-1(2H)-yl)-5-((4-(thieno [2,3-d]pyrimidin-2-yl)piperidin-1-yl)methyl)isoindoline-1,3-dione |

(continued)

| Compound No. | Structure of the compounds | The compounds' name | Chinese translation of the compounds' name |
|---|---|---|---|
| | 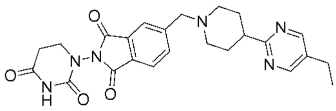 | 2-(2,4-dioxotetrahydropyrimidin-1(2H)-yl)-5-((4-(5-ethylpyrimidin-2-yl)piperidin-1-yl)methyl)isoindoline-1,3-dione | 2-(2,4-dioxotetrahydropyrimidin-1(2H)-yl)-5-((4-(5-ethylpyrimidin-2-yl)piperidin-1-yl)methyl)isoindoline-1,3-dione |
| GT-0527 9 | 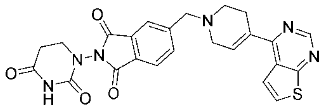 | 2-(2,4-dioxotetrahydropyrimidin-1(2H)-yl)-5-((4-(thieno [2,3-d]pyrimidin-4-yl)-3,6-dihydropyridin-1(2H)-yl)methyl) isoindoline-1,3-dione | 2-(2,4-dioxotetrahydropyrimidin-1(2H)-yl)-5-((4-(thieno [2,3-d]pyrimidin-4-yl)-3,6-dihydropyridin-1(2H)-yl)methyl) isoindoline-1,3-dione |
| GT-0842 8 | 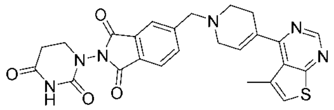 | 2-(2,4-dioxotetrahydropyrimidin-1(2H)-yl)-5-((4-(5-methylthieno[2,3-d]pyrimidin-4-yl)-3,6-dihydropyridin-1(2H)-yl)methyl)isoindoline-1,3-dione | 2-(2,4-dioxotetrahydropyrimidin-1(2H)-yl)-5-((4-(5-methylthieno[2,3-d]pyrimidin-4-yl)-3,6-dihydropyridin-1(2H)-yl)methyl)isoindoline-1,3-dione |
| GT-0528 0 | 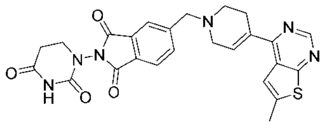 | 2-(2,4-dioxotetrahydropyrimidin-1(2H)-yl)-5-((4-(6-methylthieno[2,3-d]pyrimidin-4-yl)-3,6-dihydropyridin-1(2H)-yl)methyl)isoindoline-1,3-dione | 2-(2,4-dioxotetrahydropyrimidin-1(2H)-yl)-5-((4-(6-methylthieno[2,3-d]pyrimidin-4-yl)-3,6-dihydropyridin-1(2H)-yl)methyl)isoindoline-1,3-dione |
| GT-0842 9 | 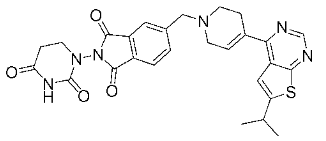 | 2-(2,4-dioxotetrahydropyrimidin-1(2H)-yl)-5-((4-(6-isopropylthieno[2,3-d]pyrimidin-4-yl)-3,6-dihydropyridin-1(2H)-yl)methyl)isoindoline-1,3-dione | 2-(2,4-dioxotetrahydropyrimidin-1(2H)-yl)-5-((4-(6-isopropylthieno[2,3-d]pyrimidin-4-yl)-3,6-dihydropyridin-1(2H)-yl)methyl)isoindoline-1,3-dione |
| GT-0527 3 | 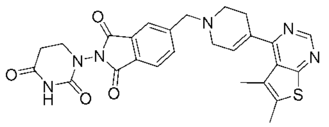 | 5-((4-(5,6-dimethylthieno[2,3-d]pyrimidin-4-yl)-3,6-dihydropyridin-1(2H)-yl) methyl)-2-(2,4-dioxotetrahydropyrimidin-1(2H)-yl)isoindoline-1,3-dione | 5-((4-(5,6-dimethylthieno[2,3-d]pyrimidin-4-yl)-3,6-dihydropyridin-1(2H)-yl) methyl)-2-(2,4-dioxotetrahydropyrimidin-1(2H)-yl)isoindoline-1,3-dione |
| GT-0843 0 | 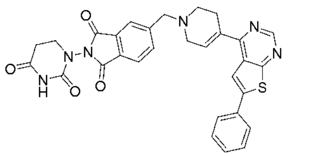 | 2-(2,4-dioxotetrahydropyrimidin-1(2H)-yl)-5-((4-(6-phenylthieno[2,3-d]pyrimidin-4-yl)-3,6-dihydropyridin-1(2H)-yl)methyl)isoindoline-1,3-dione | 2-(2,4-dioxotetrahydropyrimidin-1(2H)-yl)-5-((4-(6-phenylthieno[2,3-d]pyrimidin-4-yl)-3,6-dihydropyridin-1(2H)-yl)methyl)isoindoline-1,3-dione |
| GT-0843 1 | 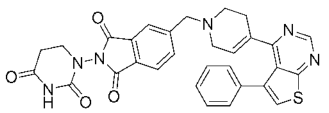 | 2-(2,4-dioxotetrahydropyrimidin-1(2H)-yl)-5-((4-(5-phenylthieno[2,3-d]pyrimidin-4-yl)-3,6-dihydropyridin-1(2H)-yl)methyl)isoindoline-1,3-dione | 2-(2,4-dioxotetrahydropyrimidin-1(2H)-yl)-5-((4-(5-phenylthieno[2,3-d]pyrimidin-4-yl)-3,6-dihydropyridin-1(2H)-yl)methyl)isoindoline-1,3-dione |
| GT-0843 3 | 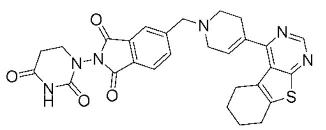 | 2-(2,4-dioxotetrahydropyrimidin-1(2H)-yl)-5-((4-(5,6,7,8-tetrahydrobenzo[4,5]thieno [2 ,3-d]pyrimidin-4-yl)-3,6-dihydropyridin-1(2H)-yl)methyl) isoindoline-1,3-dione | 2-(2,4-dioxotetrahydropyrimidin-1(2H)-yl)-5-((4-(5,6,7,8-tetrahydrobenzo[4,5]thieno [2 ,3-d]pyrimidin-4-yl)-3,6-dihydropyridin-1(2H)-yl)methyl) isoindoline-1,3-dione |

(continued)

| Compound No. | Structure of the compounds | The compounds' name | Chinese translation of the compounds' name |
|---|---|---|---|
| GT-0843 4 | 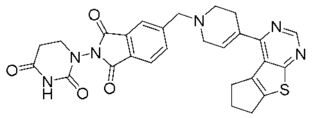 | 5-((4-(6,7-dihydro-5H-cyclopenta[4,5]thieno[2,3-d]pyrimidin-4-yl)-3,6-dihydropyridin-1(2H)-yl)methyl)-2-(2,4-dioxotetrahydropyrimidin-1(2H)-yl)isoindoline-1,3-dione | 5-((4-(6,7-dihydro-5H-cyclopenta[4,5]thieno[2,3-d]pyrimidin-4-yl)-3,6-dihydropyridin-1(2H)-yl)methyl)-2-(2,4-dioxotetrahydropyrimidin-1(2H)-yl)isoindoline-1,3-dione |
| GT-0848 4 | 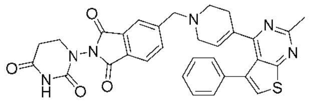 | 2-(2,4-dioxotetrahydropyrimidin-1(2H)-yl)-5-((4-(2-methyl-5-phenylthieno[2,3-d]pyrimidin-4-yl)-3,6-dihydropyridin-1(2H)-yl)methyl)isoindoline-1,3-dione | 2-(2,4-dioxotetrahydropyrimidin-1(2H)-yl)-5-((4-(2-methyl-5-phenylthieno[2,3-d]pyrimidin-4-yl)-3,6-dihydropyridin-1(2H)-yl)methyl)isoindoline-1,3-dione |
| GT-0843 2 | 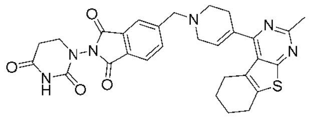 | 2-(2,4-dioxotetrahydropyrimidin-1(2H)-yl)-5-((4-(2-methyl-5,6,7,8-tetrahydrobenzo[4,5]thieno[2 ,3-d]pyrimidin-4-yl)-3,6-dihydropyridin-1(2H)-yl)methyl)isoindoline-1,3-dione | 2-(2,4-dioxotetrahydropyrimidin-1(2H)-yl)-5-((4-(2-methyl-5,6,7,8-tetrahydrobenzo[4,5]thieno[2 ,3-d]pyrimidin-4-yl)-3,6-dihydropyridin-1 (2H)-yl)methyl)isoindoline-1,3-dione |
| GT-0843 5 | 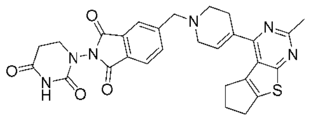 | 2-(2,4-dioxotetrahydropyrimidin-1(2H)-yl)-5-((4-(2-methyl-6,7-dihydro-5H-cyclopenta[4,5]thieno[2,3-d]pyrimidin-4-yl)-3,6-dihydropyridin-1(2H)-yl)methyl)isoindoline-1,3-dione | 2-(2,4-dioxotetrahydropyrimidin-1(2H)-yl)-5-((4-(2-methyl-6,7-dihydro-5H-cyclopenta[4,5]thieno[2,3-d]pyrimidin-4-yl)-3,6-dihydropyridin-1(2H)-yl)methyl)isoindoline-1,3-dione |
| GT-0530 7 | 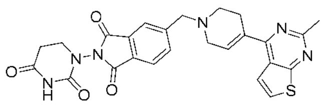 | 2-(2,4-dioxotetrahydropyrimidin-1(2H)-yl)-5-((4-(2-methylthieno[2,3-d]pyrimidin-4-yl)-3,6-dihydropyridin-1(2H)-yl)methyl)isoindoline-1,3-dione | 2-(2,4-dioxotetrahydropyrimidin-1(2H)-yl)-5-((4-(2-methylthieno[2,3-d]pyrimidin-4-yl)-3,6-dihydropyridin-1(2H)-yl)methyl)isoindoline-1,3-dione |
| GT-0530 8 | 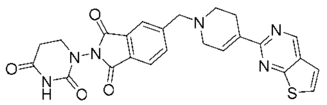 | 2-(2,4-dioxotetrahydropyrimidin-1(2H)-yl)-5-((4-(thieno [2,3-d]pyrimidin-2-yl)-3,6-dihydropyridin-1(2H)-yl)methyl) isoindoline-1,3-dione | 2-(2,4-dioxotetrahydropyrimidin-1(2H)-yl)-5-((4-(thieno [2,3-d]pyrimidin-2-yl)-3,6-dihydropyridin-1(2H)-yl)methyl) isoindoline-1,3-dione |
| GT-0844 8 | 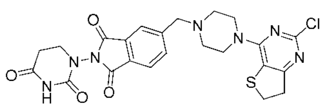 | 5-((4-(2-chloro-6,7-dihydrothieno[3,2-d]pyrimidin-4-yl) piperazin-1-yl)methyl)-2-(2,4-dioxotetrahydropyrimidin-1(2H)-yl)isoindoline-1,3-dione | 5-((4-(2-chloro-6,7-dihydrothieno[3,2-d]pyrimidin-4-yl) piperazin-1-yl)methyl)-2-(2,4-dioxotetrahydropyrimidin-1(2H)-yl)isoindoline-1,3-dione |
| GT-0845 0 | 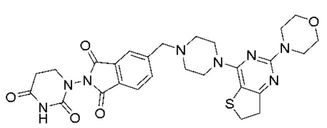 | 2-(2,4-dioxotetrahydropyrimidin-1(2H)-yl)-5-((4-(2-morpholino-6,7-dihydrothieno[3,2-d] pyrimidin-4-yl)piperazin-1-yl) methyl)isoindoline-1,3-dione | 2-(2,4-dioxotetrahydropyrimidin-1(2H)-yl)-5-((4-(2-morpholino-6,7-dihydrothieno[3,2-d] pyrimidin-4-yl)piperazin-1-yl) methyl)isoindoline-1,3-dione |

(continued)

| Compound No. | Structure of the compounds | The compounds' name | Chinese translation of the compounds' name |
|---|---|---|---|
| GT-0895 0 | 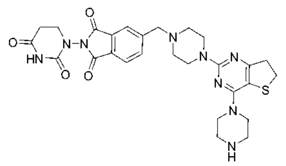 | 2-(2,4-dioxotetrahydropyrimidin-1(2H)-yl)-5-((4-(4-(piperazin-1-yl)-6,7-dihydrothieno [3,2-d]pyrimidin-2-yl)piperazin-1-yl)methyl)isoindoline-1,3-dione | 2-(2,4-dioxotetrahydropyrimidin-1(2H)-yl)-5-((4-(4-(piperazin-1-yl)-6,7-dihydrothieno [3,2-d]pyrimidin-2-yl)piperazin-1-yl)methyl)isoindoline-1,3-dione |
| GT-0845 1 | 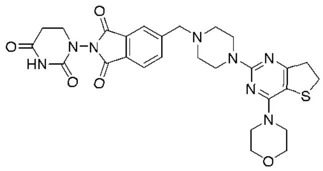 | 2-(2,4-dioxotetrahydropyrimidin-1(2H)-yl)-5-((4-(4-morpholino-6,7-dihydrothieno[3,2-d] pyrimidin-2-yl)piperazin-1-yl) methyl)isoindoline-1,3-dione | 2-(2,4-dioxotetrahydropyrimidin-1(2H)-yl)-5-((4-(4-morpholino-6,7-dihydrothieno[3,2-d] pyrimidin-2-yl)piperazin-1-yl) methyl)isoindoline-1,3-dione |
| | 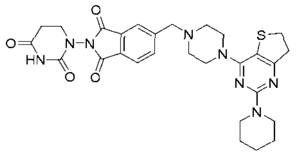 | 2-(2,4-dioxotetrahydropyrimidin-1(2H)-yl)-5-((4-(2-(piperidin-1-yl)-6,7-dihydrothieno [3,2-d]pyrimidin-4-yl)piperazin-1-yl)methyl)isoindoline-1,3-dione | 2-(2,4-dioxotetrahydropyrimidin-1(2H)-yl)-5-((4-(2-(piperidin-1-yl)-6,7-dihydrothieno [3,2-d]pyrimidin-4-yl)piperazin-1-yl)methyl)isoindoline-1,3-dione |
| | 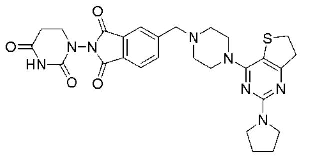 | 2-(2,4-dioxotetrahydropyrimidin-1(2H)-yl)-5-((4-(2-(pyrrolidin-1-yl)-6,7-dihydrothieno [3,2-d]pyrimidin-4-yl)piperazin-1-yl)methyl)isoindoline-1,3-dione | 2-(2,4-dioxotetrahydropyrimidin-1(2H)-yl)-5-((4-(2-(pyrrolidin-1-yl)-6,7-dihydrothieno [3,2-d]pyrimidin-4-yl)piperazin-1-yl)methyl)isoindoline-1,3-dione |
| | 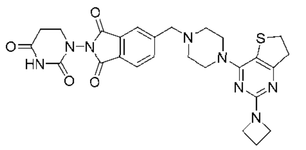 | 5-((4-(2-(azetidin-1-yl)-6,7-dihydrothieno[3,2-d]pyrimidin-4-yl)piperazin-1-yl) methyl)-2-(2,4-dioxotetrahydropyrimidin-1(2H)-yl)isoindoline-1,3-dione | 5-((4-(2-(azetidin-1-yl)-6,7-dihydrothieno[3,2-d]pyrimidin-4-yl)piperazin-1-yl) methyl)-2-(2,4-dioxotetrahydropyrimidin-1(2H)-yl)isoindoline-1,3-dione |
| GT-0845 2 | 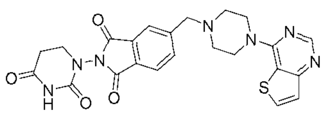 | 2-(2,4-dioxotetrahydropyrimidin-1(2H)-yl)-5-((4-(thieno [3,2-d]pyrimidin-4-yl)piperazin-1-yl)methyl)isoindoline-1,3-dione | 2-(2,4-dioxotetrahydropyrimidin-1(2H)-yl)-5-((4-(thieno [3,2-d]pyrimidin-4-yl)piperazin-1-yl)methyl)isoindoline-1,3-dione |
| GT-0845 3 | 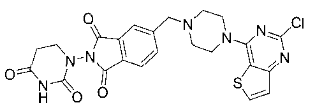 | 5-((4-(2-chlorothieno[3,2-d] pyrimidin-4-yl)piperazin-1-yl) methyl)-2-(2,4-dioxotetrahydropyrimidin-1(2H)-yl)isoindoline-1,3-dione | 5-((4-(2-chlorothieno[3,2-d] pyrimidin-4-yl)piperazin-1-yl) methyl)-2-(2,4-dioxotetrahydropyrimidin-1(2H)-yl)isoindoline-1,3-dione |
| GT-0845 5 | 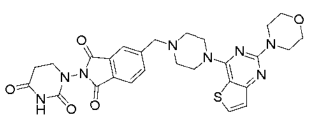 | 2-(2,4-dioxotetrahydropyrimidin-1(2H)-yl)-5-((4-(2-morpholinothieno[3,2-d]pyrimidin-4-yl)piperazin-1-yl)methyl)isoindoline-1,3-dione | 2-(2,4-dioxotetrahydropyrimidin-1(2H)-yl)-5-((4-(2-morpholinothieno[3,2-d]pyrimidin-4-yl)piperazin-1-yl)methyl)isoindoline-1,3-dione |
| GT-0895 1 | 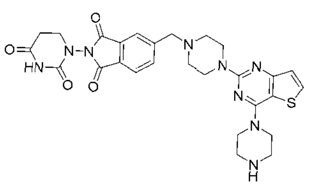 | 2-(2,4-dioxotetrahydropyrimidin-1(2H)-yl)-5-((4-(4-(piperazin-1-yl)thieno[3,2-d]pyrimidin-2-yl)piperazin-1-yl)methyl) isoindoline-1,3-dione | 2-(2,4-dioxotetrahydropyrimidin-1(2H)-yl)-5-((4-(4-(piperazin-1-yl)thieno[3,2-d]pyrimidin-2-yl)piperazin-1-yl)methyl) isoindoline-1,3-dione |

(continued)

| Compound No. | Structure of the compounds | The compounds' name | Chinese translation of the compounds' name |
|---|---|---|---|
| GT-0845 6 | 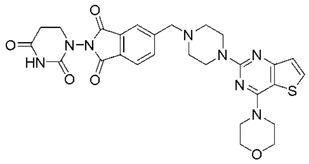 | 2-(2,4-dioxotetrahydropyrimidin-1(2H)-yl)-5-((4-(4-morpholinothieno[3,2-d]pyrimidin-2-yl)piperazin-1-yl)methyl)isoindoline-1,3-dione | 2-(2,4-dioxotetrahydropyrimidin-1(2H)-yl)-5-((4-(4-morpholinothieno[3,2-d]pyrimidin-2-yl)piperazin-1-yl)methyl)isoindoline-1,3-dione |
| GT-0517 2 | 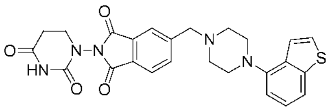 | 5-((4-(benzo[b]thiophen-4-yl) piperazin-1-yl)methyl)-2-(2,4-dioxotetrahydropyrimidin-1(2H)-yl)isoindoline-1,3-dione | 5-((4-(benzo[b]thiophen-4-yl) piperazin-1-yl)methyl)-2-(2,4-dioxotetrahydropyrimidin-1(2H)-yl)isoindoline-1,3-dione |
| GT-0525 3 | 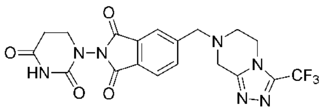 | 2-(2,4-dioxotetrahydropyrimidin-1(2H)-yl)-5-((3-(trifluoromethyl)-5,6-dihydro-[1,2,4] triazolo[4,3-a]pyrazin-7(8H)-yl)methyl)isoindoline-1,3-dione | 2-(2,4-dioxotetrahydropyrimidin-1(2H)-yl)-5-((3-(trifluoromethyl)-5,6-dihydro-[1,2,4] triazolo[4,3-a]pyrazin-7(8H)-yl)methyl)isoindoline-1,3-dione |
| GT-0516 1 | 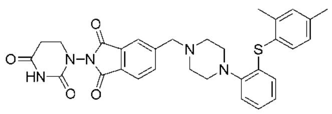 | 5-((4-(2-((2,4-dimethylphenyl) thio)phenyl) piperazin-1-yl) methyl)-2-(2,4-dioxotetrahydropyrimidin-1(2H)-yl)isoindoline-1,3-dione | 5-((4-(2-((2,4-dimethylphenyl) thio)phenyl) piperazin-1-yl) methyl)-2-(2,4-dioxotetrahydropyrimidin-1(2H)-yl)isoindoline-1,3-dione |
| GT-0525 4 | 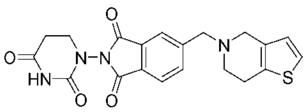 | 5-((6,7-dihydrothieno[3,2-c] pyridin-5(4H)-yl) methyl)-2-(2,4-dioxotetrahydropyrimidin-1(2H)-yl)isoindoline-1,3-dione | 5-((6,7-dihydrothieno[3,2-c] pyridin-5(4H)-yl) methyl)-2-(2,4-dioxotetrahydropyrimidin-1(2H)-yl)isoindoline-1,3-dione |
| GT-0517 1 | 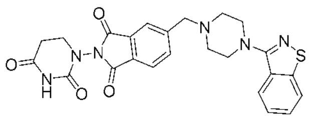 | 5-((4-(benzo[d]isothiazol-3-yl) piperazin-1-yl)methyl)-2-(2,4-dioxotetrahydropyrimidin-1(2H)-yl)isoindoline-1,3-dione | 5-((4-(benzo[d]isothiazol-3-yl) piperazin-1-yl)methyl)-2-(2,4-dioxotetrahydropyrimidin-1(2H)-yl)isoindoline-1,3-dione |
| GT-0772 2 | 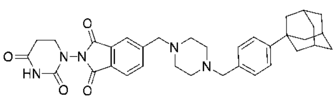 | 5-((4-(4-((3r,5r,7r)-adamantan-1-yl)benzyl)piperazin-1-yl) methyl)-2-(2,4-dioxotetrahydropyrimidin-1(2H)-yl)isoindoline-1,3-dione | 5-((4-(4-((3r,5r,7r)-adamantan-1-yl)benzyl)piperazin-1-yl) methyl)-2-(2,4-dioxotetrahydropyrimidin-1(2H)-yl)isoindoline-1,3-dione |
| GT-0527 7 | 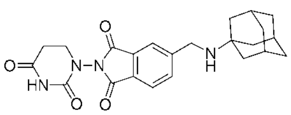 | 5-((((3s,5s,7s)-adamantan-1-yl)amino)methyl)-2-(2,4-dioxotetrahydropyrimidin-1(2H)-yl) isoindoline-1,3-dione | 5-((((3s,5s,7s)-adamantan-1-yl)amino)methyl)-2-(2,4-dioxotetrahydropyrimidin-1(2H)-yl) isoindoline-1,3-dione |
| GT-0778 6 | 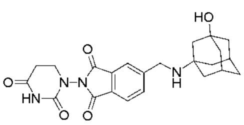 | 2-(2,4-dioxotetrahydropyrimidin-1(2H)-yl)-5-((((1r,3s,5R,7-S)-3-hydroxyadamantan-1-yl) amino)methyl)isoindoline-1,3-dione | 2-(2,4-dioxotetrahydropyrimidin-1(2H)-yl)-5-((((1r,3s,5R,7-S)-3-hydroxyadamantan-1-yl) amino)methyl)isoindoline-1,3-dione |

(continued)

| Compound No. | Structure of the compounds | The compounds' name | Chinese translation of the compounds' name |
|---|---|---|---|
| GT-0799 8 | 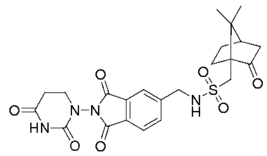 | 1-((1R,4R)-7,7-dimethyl-2-oxobicyclo[2.2.1]heptan-1-yl)-N-((2-(2,4-dioxotetrahydropyrimidin-1(2H)-yl)-1,3-dioxoisoindolin-5-yl)methyl)methanesulfonami de | 1-((1R,4R)-7,7-dimethyl-2-oxobicyclo[2.2.1]heptan-1-yl)-N-((2-(2,4-dioxotetrahydropyrimidin-1(2H)-yl)-1,3-dioxoisoindolin-5-yl)methyl)methanesulfonami de |
| | 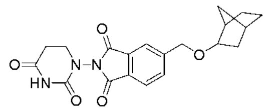 | 5-((((1R,4S)-bicyclo[2.2.1] heptan-2-yl)oxy) methyl)-2-(2,4-dioxotetrahydropyrimidin-1(2H)-yl)isoindoline-1,3-dione | 5-((((1R,4S)-bicyclo[2.2.1] heptan-2-yl)oxy) methyl)-2-(2,4-dioxotetrahydropyrimidin-1(2H)-yl)isoindoline-1,3-dione |
| GT-0613 2 | 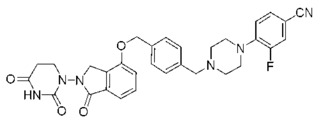 | 4-(4-(4-(((2-(2,4-dioxotetrahydropyrimidin-1(2H)-yl)-1-oxoisoindolin-4-yl)oxy)methyl) benzyl)pipera zin-1-yl)-3-fluorobenzonitrile | 4-(4-(4-(((2-(2,4-dioxotetrahydropyrimidin-1(2H)-yl)-1-oxoisoindolin-4-yl)oxy)methyl) benzyl)pipera zin-1-yl)-3-fluorobenzonitrile |

**II. Other Forms of Compounds** (including salts, enantiomers, stereoisomers, solvates, isotopically enriched analogs, prodrugs, or polymorphs of compounds)

The compounds of the present disclosure have the structures of any one of Formula (I), Formula (I-1), Formula (I-2), Formula (I-3), or Formula (I-4). Unless otherwise specified, all references to the compounds of the present disclosure also include compounds of any one of Formula (I), Formula (I-1), Formula (I-2), Formula (I-3), or Formula (I-4) and specific compounds within the scope of these general formulae.

It should be recognized that compounds of the present disclosure (including compounds of Formula (I), Formula (I-1), Formula (I-2), Formula (I-3), or Formula (I-4)) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorph forms or salts of the compounds of the present disclosure are also provided. Salts of the compounds of the present disclosure can be pharmaceutically acceptable salts including, but not limited to, hydrohalides hydrohalide (including hydrochloride, hydrobromide), sulfate, citrate, maleate, mesylate, lactate, lactobionate, L-tartrate, fumarate, L-malate, α-ketoglutarate, hippurate, D-glucuronate, D-gluconate, α-D-glucoheptonate, glycolate, mucate, L-ascorbate, orotate, picrate, glycinate, alaninate, arginate, cinnamate, laurate, pamoate, sebacate, besylate, methanesulfonate, ethanesulfonate, edisylate, formate, acetate, 2,2-dichloroacetate, trimethylacetate, propionate, valerate, palmitate, triphenylacetate, 2-ethyl-butane-1,4-dioate, iodate, nicotinate, L-pyroglutamate, L-prolinate, ferulate, 2-hydroxyethanesulfonate, nitrate, gentisate, cholate, salicylate, terephthalate, glutarate, adipate, stearate, oleate, undecylenate, camphorate, camsylate, dodecylsulfate, phosphate, thiocyanate, dihydrogen phosphate, pyrophosphate, metaphosphate, oxalate, malonate, benzoate, mandelate, succinate, pyruvate, 4-chlorobenzenesulfonate, 1,5-naphthalenedisulfonate, 3-hydroxy-2-naphthoate, 1-hydroxy-2-naphthoate, 2-naphthalenesulfonate, trifluoroacetate, glycolate, or 4-methylbenzenesulfonate of the compound of Formula (I). The compounds of the present disclosure can exist as non-solvated or solvated forms in pharmaceutically acceptable solvents such as water, ethanol, and the like. In some embodiments, the compounds of the present disclosure can be prepared as prodrugs or precursor drugs. Prodrugs can be converted into parent drugs in the body to play their role. In some embodiments, isotopically-labeled compounds of the present disclosure are also provided, examples of which include deuterium (D or $^2H$).

**III. Pharmaceutical Compositions/Formulations**

In some embodiments, the present disclosure provides a pharmaceutical composition comprising as active

ingredient the compound of the present disclosure or a pharmaceutically acceptable salt, solvate, isotopically enriched analog, polymorph, prodrug, stereoisomer (including enantiomer), or mixture of stereoisomers thereof, and at least one pharmaceutically acceptable carrier.

In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.

The pharmaceutical composition of the present disclosure can further comprise at least one second therapeutic agent, e.g., an anticancer agent. The second therapeutic agent may be used in combination with the compounds of Formula (I) described in the present disclosure to treat the diseases or disorders as disclosed herein. The second therapeutic agent includes, but is not limited to, chemotherapeutic agents, immunotherapeutic agents, gene therapy agents, and the like.

The pharmaceutical composition of the present disclosure comprising, as an active ingredient, the compounds of Formula (I) of the present disclosure or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of Formula (I) of the present disclosure into conventional, dispersible, chewable, orally disintegrating or rapidly dissolving formulations, or sustained-release capsules or controlled-release capsules as needed.

The compounds of Formula (I) of the present disclosure, as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of Formula (I) of the present disclosure or pharmaceutically acceptable salts thereof may be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

**IV. Kits/Packaged Products**

The compounds of Formula (I) of the present disclosure or a pharmaceutically acceptable salt, solvate, isotopically enriched analog, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

### V. Treatment Methods and Uses

The compounds of Formula (I) or a pharmaceutically acceptable salt, solvate, isotopically enriched analog, polymorph, prodrug, stereoisomer (including enantiomer), or mixture of stereoisomers thereof can be used as a medicament. Especially the compound of Formula (I) of the present disclosure or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of a disease or disorder associated with a cereblon protein. The disease or disorder associated with a cereblon protein comprises: tumors, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, or diabetes. In some embodiments, the disease or disorder associated with a cereblon protein include, but are not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including chronic myelogenous leukemia, B-cell chronic lymphocytic leukemia, leukemia-associated anemia, acute myeloid leukemia (AML), chronic myeloid leukemia (CML), monocytic leukemia, myelomonocytic leukemia, acute lymphoblastic leukemia (ALL), acute T-lymphocytic leukemia, T-lymphocytic leukemia, chronic lymphocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, and lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

The present disclosure provides a method for preventing and/or treating a disease or disorder associated with a cereblon protein in a subject, comprising administering to the subject a therapeutically effective amount of the compounds of Formula (I) of the present disclosure or a pharmaceutically acceptable salt thereof, or the pharmaceutical composition of the present disclosure comprising as an active ingredient the compounds of Formula (I) of the present disclosure or a pharmaceutically acceptable salt thereof. In some embodiments, the disease or disorder associated with a cereblon protein comprises: tumors, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, or diabetes. In some embodiments, the disease or disorder associated with a cereblon protein comprises: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including chronic myelogenous leukemia, B-cell chronic lymphocytic leukemia, leukemia-associated anemia, acute myeloid leukemia (AML), chronic myeloid leukemia (CML), monocytic leukemia, myelomonocytic leukemia, acute lymphoblastic leukemia (ALL), acute T-lymphocytic leukemia, T-lymphocytic leukemia, chronic lymphocytic leukemia;

lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, and lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

In the method for preventing and/or treating diseases or disorders associated with cereblon protein, the compound of Formula (I) of the present disclosure or the pharmaceutical composition comprising as an active ingredient the compound of Formula (I) of the present disclosure is administered to the subject through at least one mode of administration selected from the group consisting of nasal administration, inhalation administration, topical administration, oral administration, oral mucosal administration, rectal administration, pleural cavity administration, peritoneal administration, intramuscular administration, subcutaneous, transdermal, epidural, intrathecal, and intravenous administration.

The term "treatment" or "treating" refers to administering to a subject the compound of Formula (I) of the present disclosure, or a pharmaceutically acceptable salt thereof, or the pharmaceutical composition comprising, as an active ingredient, the compound of Formula (I) of the present disclosure or a pharmaceutically acceptable salt thereof, to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

## VI. Preparation Method

The compound of Formula (II) mentioned below is a sub-embodiment of the compound of Formula (I) of the present disclosure, and can be prepared by reacting a compound of Formula (M1) with a compound of Formula (M2):

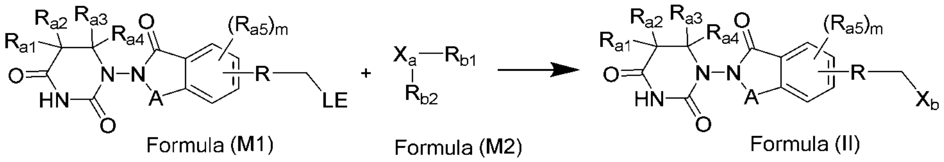

Formula (M1) Formula (M2) Formula (II)

wherein $R_{a1}$, $R_{a2}$, $R_{a3}$, $R_{a4}$, $(R_{a5})_m$, A, and R are as defined in the compound of Formula (I) and its various sub-embodiments in the present disclosure; and
wherein

(i) group LE represents Cl, Br, I, OMs, OTs, or ONs; group $X_a$ represents NH; group $R_{b1}$ represents $R_1$; group $R_{b2}$ represents $R_2$; and $X_b$ accordingly represents $NR_1R_2$, wherein $R_1$ and $R_2$ are as defined in the compound of Formula (I) and its various sub-embodiments in the present disclosure; or
(ii) group LE represents Cl, Br, I, OMs, OTs, or ONs; group $X_a$ represents NH; groups $R_{b1}$ and $R_{b2}$, together with the nitrogen atom to which they are attached, form an optionally substituted nitrogen-containing heterocycle $A_a$ represented by the following general formula:

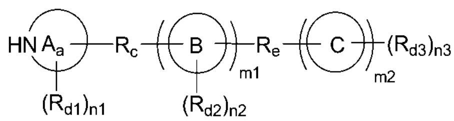

and $X_b$ of the compound of Formula (II) accordingly represents a structure represented by the following formula:

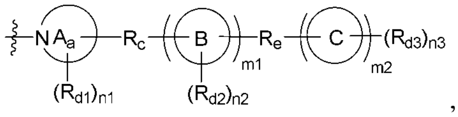

,

wherein nitrogen-containing heterocycle $A_a$ represents 4- to 30-membered nitrogen-containing heterocyclyl (e.g., 4- to 20-membered nitrogen-containing heterocyclyl, or 4- to 15-membered nitrogen-containing heterocyclyl); and $(R_{d1})_{n1}$, $R_c$, ring B, m1, $(R_{d2})_{n2}$, $R_c$, ring C, m2, and $(R_{d3})_{n3}$ are as defined in the compound of Formula (I) and its various sub-embodiments in the present disclosure; or
(iii) group LE represents C(O)Cl, COOH, or $S(O)_2Cl$; the compound of Formula (M2), $X_aR_{b1}R_{b2}$, is $NHR_3R_4$ or $NHR_{10}R_{11}$; and $X_b$ the compound of Formula (II) accordingly represents $C(O)NR_3R_4$ or $S(O)_2NR_{10}R_{11}$, wherein $R_3$, $R_4$, $R_{10}$, and $R_{11}$ are as defined in the compound of Formula (I) and its various sub-embodiments in the present disclosure; or
(iv) group LE represents $NH_2$; group $X_a$ represents C(O) or $S(O)_2$; group $R_{b1}$ represents OH or Cl; $R_{b2}$ is group $R_5$ or $R_{13}$ as defined in the compound of Formula (I) and its various sub-embodiments in the present disclosure; and $X_b$ of the compound of Formula (II) accordingly represents $NHC(O)R_5$ or $NHS(O)_2R_{13}$, wherein $R_5$ and $R_{13}$ are as defined in the compound of Formula (I) and its various sub-embodiments in the present disclosure; or
(v) group LE represents Cl, Br, or I; group $X_a$ represents O or S; group $R_{b1}$ represents H; $R_{b2}$ is group $R_8$ or $R_9$ as defined in the compound of Formula (I) and its various sub-embodiments in the present disclosure; and $X_b$ of the compound of Formula (II) accordingly represents $OR_8$ or $SR_9$, wherein $R_8$ and $R_9$ are as defined in the compound of Formula (I) and its various sub-embodiments in the present disclosure.

In sub-embodiments (i) and (ii) of the present disclosure, the compound of Formula (M1) undergoes an aminoalkylation reaction with the compound of Formula (M2). The aminoalkylation reaction can be conducted, for example, in the presence of an organic base and sodium iodide at a temperature ranging from room temperature to 80 °C (e.g., 40 °C to 60 °C, 40 °C to 50 °C, or 50 °C to 60 °C). Examples of the organic base include, but are not limited to, DIEA or triethylamine.
The molar ratio of the compound of Formula (M1) to the compound of Formula (M2) may be, for example, 1:1.1-2, 1:1.1-1.5, 1:1.1-1.2, or 1:1.2-1.3.
In some embodiments, when the group LE represents Cl, Br, or I, the compound of Formula (M1) may be prepared by a halogenation reaction of a compound of formula (M3) with a hydrohalic acid:

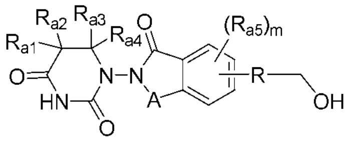

Formula (M3)

wherein $R_{a1}$, $R_{a2}$, $R_{a3}$, $R_{a4}$, $(R_{a5})_m$, and A are as defined in the compound of Formula (I) and its various sub-embodiments in the present disclosure, and R represents a bond.

In some embodiments, the halogenation reaction can be conducted at room temperature in the presence of, for example, a hydrohalic acid and acetic acid.

In some embodiments, R in the compound of Formula (M1), the compound of Formula (II), and the compound of Formula (M3) represents a bond.

In some embodiments, the compound of Formula (M3) (wherein R represents a bond) may be prepared by a coupling reaction of a compound of Formula (M4) with (tributylstannyl)methanol:

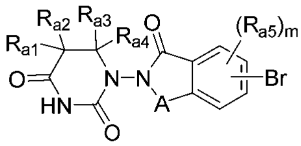

Formula (M4)

wherein $R_{a1}$, $R_{a2}$, $R_{a3}$, $R_{a4}$, $(R_{a5})_m$, and A are as defined in the compound of Formula (I) and its various sub-embodiments in the present disclosure.

In some embodiments, the coupling reaction can be conducted, for example, at a temperature ranging from 40 °C to 120 °C in the presence of tetrakis(triphenylphosphine)palladium(0) and 1,4-dioxane (or DMF).

In some embodiments, the compound of Formula (M4) may be prepared by an aminolysis reaction of an ester with an amine, wherein the ester is a compound of Formula (M6) and the amine is a compound of Formula (M5):

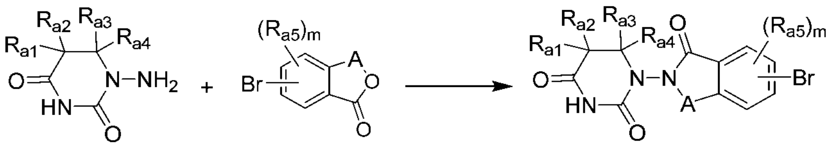

Formula (M5) Formula (M6) Formula (M4)

wherein $R_{a1}$, $R_{a2}$, $R_{a3}$, $R_{a4}$, $(R_{a5})_m$, and A are as defined in the compound of Formula (I) and its various sub-embodiments in the present disclosure.

In some embodiments, the aminolysis of the ester may be conducted at room temperature in the presence of $CH_3ONa$ and methanol. Alternatively, the reaction may be carried out at a temperature between 40°C and 90°C using $CH_3OLi$ or $Cs_2CO_3$ in methanol, or with DBU in THF within the same temperature range.

## VII. Definitions

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries

above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "R represents a bond" means that R is a bond linker. In other words, when R represents a bond, the L in the structure of Formula (I) is directly connected to the isoindolin ring in the structure of Formula (I). For example, the wording "$R_{w2}$ represents a bond" means that $R_{w2}$ is a bond linker. In other words, when $R_{w2}$ represents a bond, ring $A^5$ in the group L is directly linked to X.

In this document, the term "optionally substituted" used alone or in combination means that the referenced group may be unsubstituted or substituted with one or more substituents as defined here. Herein, the wording "optionally substituted with..." and "unsubstituted or substituted" can be used interchangeably. The term "substituted" generally represents the replacement of one or more hydrogen atoms in the referenced structure by identical or different specific substituents. The number of substituents is not theoretically limited in any way, or is automatically limited by the size of the building units (i.e., the total number of replaceable hydrogen atoms in the building units), or as clearly defined herein.

Herein, a bond interrupted by a wavy line shows the point of attachment of the depicted group to the rest of the molecule. For example, the monovalent group X depicted below

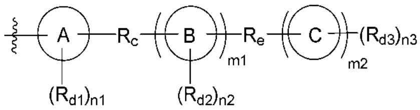

shows the point of attachment of ring A in said group X to group L in the structure of Formula (I). Herein, unless otherwise expressly specified, the two bonds interrupted by wavy lines in a depicted divalent group show the two points of attachment of the depicted group to the rest of the molecule. For example, in the structure of group L depicted below,

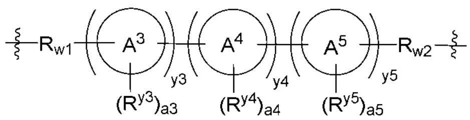

as defined herein, $R_{w1}$ is connected to R, and $R_{w2}$ is connected to X. For instance, the group represented by $R_{w1}$ is $-O-CH_2-$, either end of which may be connected to R of the compound of Formula (I), while the other end is connected to ring $A^3$. In other words, the O in the group $-O-CH_2-$ may be connected to R of the compound of Formula (I), and the $CH_2$ may be connected to ring $A^3$ of the compound of Formula (I), or vice versa. As another example, the divalent group R depicted below

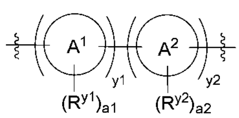

may have either end connected to the isoindolin ring of the compound of Formula (I), while the other end is connected to group L. In other words, ring $A^1$ of the divalent group R may be connected to the isoindolin ring of the compound of Formula (I), and the other end may be connected to group L, or vice versa.

As used herein, the term "oxo" or "oxo group" refers to =O.

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "$C_x$-$C_y$ alkyl" or "$C_{x-y}$ alkyl" (x and y each being an integer) refers to a linear or branched alkyl group containing from x to y carbon atoms. The term "$C_{1-6}$ alkyl" used alone or in combination in the present disclosure refers to a linear or branched alkyl group containing from 1 to 6 carbon atoms. Examples of the $C_{1-6}$ alkyl of the present disclosure may include a $C_{1-5}$ alkyl, $C_{1-4}$ alkyl, $C_{1-3}$ alkyl, and $C_1$-$C_2$ alkyl. Representative examples include methyl, ethyl, n-propyl, isopropyl, n-butyl, isobutyl, sec-butyl, tert-butyl, pentyl, isopentyl, neopentyl, tert-pentyl, and hexyl. The term "$C_{1-3}$ alkyl" or "$C_1$-$C_3$ alkyl" in the present disclosure refers to an alkyl group containing from 1 to 3 carbon atoms, and representative examples thereof include methyl, ethyl, n-propyl, and isopropyl. In the present disclosure, the "alkyl" is optionally substituted with one or more substituents optionally selected from the group consisting of halogen, hydroxyl, cyano, $C_{1-3}$ alkyl, $C_{1-3}$ alkoxy, halogenated $C_{1-6}$ alkoxy, halogenated $C_{1-6}$ alkyl (e.g., trifluoromethyl), $C_{3-6}$ cycloalkyl, 4- to 7-membered heterocyclyl, or a combination thereof.

As used herein, the term "halogenated alkyl", used alone or in combination, refers to a linear or branched alkyl

group substituted with one or more halogens, wherein one or more hydrogen atom(s) of the alkyl group are replaced with one or more halogens. The term "halogenated $C_x$-$C_y$ alkyl" or "halogenated $C_{x-y}$ alkyl" (x and y are each an integer) refers to a linear or branched alkyl containing from x to y carbon atoms substituted with one or more halogens. The term "halogenated $C_{1-10}$ alkyl" used alone or in combination in the present disclosure refers to a linear or branched alkyl group containing from 1 to 10 carbon atoms substituted with one or more halogens. Examples of the halogenated $C_{1-10}$ alkyl group of the present disclosure include halogenated $C_{1-9}$ alkyl group, e.g., halogenated $C_{1-8}$ alkyl group, halogenated $C_{2-8}$ alkyl group, halogenated $C_{1-7}$ alkyl group, halogenated $C_{1-6}$ alkyl, halogenated $C_{1-5}$ alkyl, or halogenated $C_{1-4}$ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halogenated $C_{1-3}$ alkyl" or "halogenated $C_1$-$C_3$ alkyl" of the present disclosure refers to an alkyl group containing from 1 to 3 carbon atoms substituted with one or more halogens, and its representative examples include halomethyl (e.g., trifluoromethyl), haloethyl, halo-n-propyl and haloisopropyl.

As used herein, the term "deuterated alkyl", used alone or in combination, refers to a linear or branched alkyl group substituted with one or more deuterium atoms, wherein one or more hydrogen atoms of the alkyl group are replaced with one or more deuterium atoms. The term "deuterated $C_x$-$C_y$ alkyl" or "deuterated $C_{x-y}$ alkyl" (x and y are each an integer) refers to a linear or branched alkyl containing from x to y carbon atoms substituted with one or more deuterium atoms. The term "deuterated $C_{1-6}$ alkyl" used alone or in combination in the present disclosure refers to a linear or branched alkyl group containing from 1 to 6 carbon atoms substituted with one or more deuterium atoms. Examples of the deuterated $C_{1-6}$ alkyl group of the present disclosure include deuterated $C_{1-5}$ alkyl, and deuterated $C_{1-4}$ alkyl. Representative examples include perdeuterated methyl ($CD_3$), perdeuterated ethyl ($CD_3CD_2$), perdeuterated n-propyl, perdeuterated isopropyl, perdeuterated n-butyl, perdeuterated isobutyl, perdeuterated sec-butyl, perdeuterated tert-butyl, perdeuterated pentyl, perdeuterated isopentyl, perdeuterated neopentyl, perdeuterated tert-pentyl, and perdeuterated hexyl. The term "deuterated $C_{1-3}$ alkyl" or "deuterated $C_1$-$C_3$ alkyl" of the present disclosure refers to an alkyl group containing from 1 to 3 carbon atoms substituted with one or more deuterium atoms, and its representative examples include perdeuterated methyl ($CD_3$) and perdeuterated ethyl ($CD_3CD_2$).

As used herein, the term "alkylene" (which is used interchangeably with "alkylene chain"), used alone or in combination, refers to a linear or branched divalent saturated hydrocarbon group composed of carbon and hydrogen atoms. The term "$C_x$-$C_y$ alkylene" or "$C_{x-y}$ alkylene" (x and y each being an integer) refers to a linear or branched alkylene group containing from x to y carbon atoms. The term "$C_1$-$C_6$ alkylene" as used herein, used alone or in combination, refers to a linear or branched alkylene group containing 1 to 6 carbon atoms, examples of which include $C_1$-$C_5$ alkylene, $C_1$-$C_4$ alkylene, $C_1$-$C_3$ alkylene, and $C_1$-$C_2$ alkylene. Representative examples include, but are not limited to, methylene (i.e., $-CH_2-$), ethylene (e.g., $-CH_2CH_2-$), propylene, isopropylene, butylene, isobutylene, sec-butylene, tert-butylene, n-pentylene, isopentylene, neopentylidene, tert-pentylene, and hexylene. In the present disclosure, the "alkylene" is optionally substituted with one or more substituents optionally selected from $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "alkoxy", used alone or in combination, refers to a linear or branched alkoxy group having structural formula of alkyl-O-. Optionally, the alkyl portion of the alkoxy group may contain 1-6 carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "$C_1$-$C_3$ alkoxy" or "$C_{1-3}$ alkoxy" refers to a linear or branched alkoxy group containing from 1 to 3 carbon atoms. Representative examples of $C_{1-3}$ alkoxy include, but are not limited to, methoxy, ethoxy, n-propoxy, and isopropoxy.

As used herein, the term "halogenated alkoxy", used alone or in combination, refers to an alkoxy group substituted with one or more halogen atoms. Optionally, the alkyl portion of the alkoxy group may contain 1-6 carbon atoms. Examples of "halogenated alkoxy" include halogenated $C_{1-6}$ alkoxy and halogenated $C_{1-4}$ alkoxy. Representative examples include, but are not limited to, $F_3C$-O-, $FCH_2$-O-, $F_2CHO$-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-.

As used herein, the term "heteroaryl", used alone or in combination, refers to a 5- to 20-membered (e.g., 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more heteroatoms independently selected from O, S and N, and the remaining rings which may be a saturated, partially unsaturated or aromatic ring and can be carbocyclic ring or contain one or more heteroatoms independently selected from O, S and N. Examples of monocyclic heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl,

triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo [2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, and imidazo[2,1-b] thiazolyl. Examples of tricyclic or polycyclic heteroaryl groups include, but are not limited to, acridinyl, benzindolyl, carbazolyl, dibenzofuranyl, and xanthyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "heteroarylene", used alone or in combination, refers to a 5- to 20-membered (e.g., 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) bivalent monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic or tetracyclic heteroarylene groups, which contain one aromatic ring having one or more heteroatoms independently selected from O, S and N, and the remaining ring(s) which may be a saturated, partially unsaturated or aromatic ring and can be carbocyclic ring or contain one or more heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3] oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene. Examples of tricyclic or polycyclic heteroarylene groups include, but are not limited to, acridinylene, benzindolylene, carbazolylene, dibenzofuranylene, and xanthylene. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "aryl", used alone or in combination, refers to a monovalent aromatic hydrocarbon group containing from 5 to 14 carbon atoms and optionally one or more fused rings, such as phenyl, naphthyl, or fluorenyl. As used herein, the "aryl" is optionally substituted. A substituted aryl group refers to an aryl group substituted one or more times (e.g., 1-4, 1-3, or 1-2 times) with a substituent(s). For example, aryl is mono-, di-, or tri-substituted with a substituent(s) optionally selected from e.g., $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "arylene", used alone or in combination, refers to a divalent aromatic hydrocarbon group containing from 5 to 14 carbon atoms and optionally one or more fused rings, such as phenylene (e.g.,

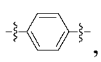, 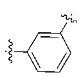, and 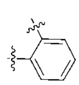

), naphthylene, or fluorenylene. As used herein, the "arylene" is optionally substituted. A substituted arylene refers to an arylene group substituted one or more times (e.g., 1-4, 1-3, or 1-2 times) with a substituent(s). For example, arylene is mono-, di-, or tri-substituted with a substituent(s) optionally selected from e.g., $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "cycloalkyl", used alone or in combination, refers to a saturated or partially unsaturated (i.e., containing one or more double bonds, but not having a fully conjugated π-electron system) monocyclic or bicyclic or tricyclic or polycyclic cyclic hydrocarbon radical, which in some embodiments contains from 3 to 20 carbon atoms (i.e.,

$C_{3-20}$ cycloalkyl), or from 3 to 15 carbon atoms (i.e., $C_{3-15}$ cycloalkyl), or from 3 to 12 carbon atoms (i.e., $C_{3-12}$ cycloalkyl), or from 3 to 11 carbon atoms (i.e., $C_{3-11}$ cycloalkyl), or from 3 to 10 carbon atoms (i.e., $C_{3-10}$ cycloalkyl), or from 3 to 8 carbon atoms ( i.e., $C_{3-8}$ cycloalkyl), or from 3 to 7 carbon atoms (i.e., $C_{3-7}$ cycloalkyl), or from 3 to 6 carbon atoms (i.e., $C_{3-6}$ cycloalkyl). The term "cycloalkyl" includes monocyclic, bicyclic, tricyclic, and polycyclic cyclic hydrocarbon radical having from 3 to 20 carbon atoms. Examples of the term "cycloalkyl" include, but are not limited to, monocyclic cycloalkyl, bridged cycloalkyl (e.g., $C_{5-15}$ bridged cycloalkyl or $C_{7-15}$ bridged cycloalkyl), fused cycloalkyl, and spiro-cycloalkyl (e.g., $C_{5-15}$ spiro-cycloalkyl). Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Examples of bridged cycloalkyl, fused cycloalkyl and spiro-cycloalkyl groups include, but are not limited to, decalinyl, octahydropentalenyl, octahydro-1H-indenyl, $C_{5-15}$ spiro-cycloalkyl, adamantanyl, noradamantanyl, bornyl, norbornyl (also named as bicyclo [2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) substituents selected from $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof. Examples of "$C_{3-6}$ cycloalkyl" include, but are not limited to, cyclopropyl, cyclobutyl, cyclopentyl, cyclopentenyl, and cyclohexyl.

As used herein, the term "$C_{x-y}$ spiro-cycloalkyl" (x and y each being an integer), used alone or in combination, refers to a spiro-cycloalkyl group containing from x to y carbon atoms. As used herein, the term "$C_{5-15}$ spiro-cycloalkyl", used alone or in combination, refers to a spiro-cycloalkyl group containing from 5 to 15 carbon atoms (e.g., 7-15, 5-11, 5-10, or 7-9 carbon atoms). The term "$C_{5-15}$ spirocycloalkyl" includes "$C_{7-15}$ spirocycloalkyl", and representative examples thereof include, but are not limited to, spiro[3.3]heptanyl, spiro[2.5]octanyl, spiro[3.5]nonanyl, spiro[4.4]nonanyl, spiro[4.5] decanyl, and spiro[5.5]undecanyl. The "$C_{5-15}$ spiro-cycloalkyl" is optionally further substituted with one or more substituents selected from the group consisting of $C_{1-3}$ alkyl, halogenated $C_{1-3}$ alkyl, $C_{1-3}$ alkoxy, deuterated $C_{1-3}$ alkoxy, $C_{1-3}$ alkylamino, amino, oxo, halogen, hydroxy, cyano, or any combination thereof.

As used herein, the term "$C_{x-y}$ bridged cycloalkyl" (where x and y are integers) used alone or in combination, refers to a bridged cycloalkyl group containing x to y carbon atoms. In the present invention, the term "$C_{5-15}$ bridged cycloalkyl" used alone or in combination, refers to a bridged cycloalkyl group containing 5 to 15 carbon atoms. Representative examples of the term "$C_{5-15}$ bridged cycloalkyl" include, but are not limited to, adamantanyl, noradamantanyl, norbornanyl (systematically named bicyclo[2.2.1]heptanyl), and cubanyl. Said "bridged cycloalkyl" may be optionally substituted with 1 to 10 substituents selected from: $C_{1-3}$ alkyl, deuterated $C_{1-3}$ alkyl, $C_{1-3}$ alkoxy, halogen, halogenated $C_{1-3}$ alkyl, $C_{1-3}$ alkylamino, amino, hydroxy, cyano, oxo, or any combination thereof.

As used herein, the term "cycloalkylene", used alone or in combination, refers to a saturated or partially unsaturated (i.e., containing one or more double bonds, but not having a fully conjugated $\pi$-electron system) monocyclic, bicyclic, tricyclic or polycyclic divalent cyclic hydrocarbon radical, which in some embodiments contains from 3 to 20 carbon atoms (i.e., $C_{3-20}$ cycloalkylene), or from 3 to 15 carbon atoms (i.e., $C_{3-15}$ cycloalkylene), or from 3 to 12 carbon atoms (i.e., $C_{3-12}$ cycloalkylene), or from 3 to 11 carbon atoms (i.e., $C_{3-11}$ cycloalkylene), or from 3 to 10 carbon atoms (i.e., $C_{3-10}$ cycloalkylene), or from 3 to 8 carbon atoms ( i.e., $C_{3-8}$ cycloalkylene), or from 3 to 7 carbon atoms (i.e., $C_{3-7}$ cycloalkylene), or from 3 to 6 carbon atoms (i.e., $C_{3-6}$ cycloalkylene). The term "cycloalkylene" includes monocyclic, bicyclic, tricyclic and polycyclic divalent cyclic hydrocarbon radical having from 3 to 20 carbon atoms. Examples of the term "cycloalkylene" include, but are not limited to, monocyclic cycloalkylene, bridged cycloalkylene (e.g., $C_{5-15}$ bridged cycloalkylene or $C_{7-15}$ bridged cycloalkylene), fused cycloalkylene (e.g., $C_{5-15}$ fused cycloalkylene), and spiro-cycloalkylene (e.g., $C_{5-15}$ spiro-cycloalkylene). Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Examples of bridged cycloalkylene, fused cycloalkylene and spiro-cycloalkylene groups include, but are not limited to, decalinylene, octahydropentalenylene, octahydro-1H-indenylene, 2,3-dihydro-1H-indenylene, spiro-cycloalkylene (e.g., $C_{5-15}$ spiro-cycloalkylene), adamantanylene, noradamantanylene, and norbornylene (also named as bicyclo[2.2.1]heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" can be optionally one or more (e.g., 1-5, 1-4, 1-3, 1-2, or 1) substituents selected from the group consisting of $C_1$-$C_3$ alkyl, $C_{3-6}$ cycloalkyl, hydroxy, amino, mercapto, halogen, $C_1$-$C_3$ alkoxy, $C_1$-$C_3$ alkylamino, halogenated $C_1$-$C_3$ alkyl, amino-substituted $C_{1-3}$ alkylene, $C_{1-3}$ alkyl-NHC(O)-, $C_{1-3}$ alkyl-C(O)NH-, cyano or any combination thereof.

As used herein, the term "$C_{x-y}$ spiro-cycloalkylene" (x and y each being an integer), used alone or in combination, refers to a spiro-cycloalkylene group containing from x to y carbon atoms. As used herein, the term "$C_{5-15}$ spiro-cycloalkylene", used alone or in combination, refers to a spiro-cycloalkylene group containing from 5 to 15 carbon atoms (e.g., 7-15, 5-11, 5-10, or 7-9 carbon atoms). Representative examples of "$C_{5-15}$ spiro-cycloalkylene" include, but are not limited to, spiro[3.3]heptylene, spiro[2.5]octylene, spiro[3.5]nonylene, spiro[4.4]nonylene, spiro[4.5]decylene, and spiro [5.5]undecylene. The "$C_{5-15}$ spiro-cycloalkylene" is optionally further substituted with one or more substituents selected

from the group consisting of $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "$C_{x-y}$ bridged cycloalkylene" (where x and y are integers) used alone or in combination, refers to a bridged cycloalkylene group containing x to y carbon atoms. In the present invention, the term "$C_{5-15}$ bridged cycloalkylene" used alone or in combination, refers to a bridged cycloalkylene group containing 5 to 15 (e.g., 7-15, 5-11, 5-10, or 7-9) carbon atoms. Representative examples of the term "$C_{5-15}$ bridged cycloalkylene" include, but are not limited to, adamantanylene, noradamantanylene, norbornanylene, bicyclo[2.2.1]heptanylene, 2-oxobicyclo[2.2.1]heptanylene and bicyclo[2.2.1]heptenylene. Said "$C_{5-15}$ bridged cycloalkylene" may be optionally substituted with one or more substituents selected from: $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "heterocyclyl" or "heterocyclic group", used alone or in combination, refers to a 4- to 20-membered saturated or partially unsaturated (i.e., containing one or more double bonds, but not having a fully conjugated $\pi$-electron system) monocyclic, bicyclic, tricyclic or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclyl" may refer to a 4- to 20-membered (e.g., 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, or 4- to 9-membered) saturated or partially unsaturated (i.e., containing one or more double bonds, but not having a fully conjugated $\pi$-electron system) monocyclic, bicyclic, tricyclic, or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclyl groups include, but not limited to, monocyclic heterocyclyl (e.g., 4- to 20-membered monocyclic heterocyclyl), bridged heterocyclyl (e.g., 5- to 20-membered bridged heterocyclyl, and 7- to 15-membered bridged heterocyclyl), fused heterocyclyl (e.g., 5-to 20-membered fused heterocyclyl), and spiro-heterocyclyl groups (e.g., 5- to 20-membered spiro-heterocyclyl). Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl) and diazacyclooctyl. Examples of bridged heterocyclyl, fused heterocyclyl and spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, and azaspirocycloalkyl (e.g., 5- to 20-membered azaspirocycloalkyl, such as 3-azaspiro[5.5]undecan-3-yl). The heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- and any combination thereof.

As used herein, the term "nitrogen-containing heterocyclyl", used alone or in combination, refers to 4- to 20-membered (e.g., 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, or 5- to 9-membered) saturated or partially unsaturated (i.e., containing one or more double bonds, but not having a fully conjugated $\pi$-electron system) monocyclic, bicyclic, tricyclic, or polycyclic cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the "nitrogen-containing heterocyclyl" include, but not limited to, nitrogen-containing monocyclic heterocyclyl (e.g., 4- to 20-membered nitrogen-containing monocyclic heterocyclyl), nitrogen-containing bridged heterocyclyl (e.g., 5- to 20-membered nitrogen-containing bridged heterocyclyl, and 7- to 15-membered nitrogen-containing bridged heterocyclyl), nitrogen-containing fused heterocyclyl (e.g., 5- to 20-membered nitrogen-containing fused heterocyclyl), and nitrogen-containing spiro-heterocyclyl (e.g., 5- to 20-membered nitrogen-containing spiro-heterocyclyl). Representative examples of the nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl (e.g., 1,4-diazacycloheptan-1-yl), and diazacyclooctyl. Examples of nitrogen-containing bridged heterocyclyl, nitrogen-containing fused heterocyclyl and nitrogen-containing spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, and azaspirocycloalkyl (e.g., 5- to 20-membered azaspirocycloalkyl, such as 3-azaspiro[5.5]undecan-3-yl). The nitrogen-containing heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen,

cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "heterocyclylene", used alone or in combination, refers to a 4- to 20-membered saturated or partially unsaturated (i.e., containing one or more double bonds, but not having a fully conjugated π-electron system) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclylene" may refer to e.g., a 4- to 15-membered (optionally 4- to 14-membered, 4- to 12-membered, 4- to 11-membered, 4- to 10-membered, 4- to 9-membered, 4- to 8-membered, 4- to 7-membered, 4- to 6-membered, or 4- to 5-membered) saturated or partially unsaturated (i.e., containing one or more double bonds, but not having a fully conjugated π-electron system) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclylene groups include, but are not limited to, monocyclic heterocyclylene (e.g., 4- to 20-membered monocyclic heterocyclylene), bridged heterocyclylene (e.g., 5- to 20-membered bridged heterocyclylene, and 7- to 15-membered bridged heterocyclylene), fused heterocyclylene (e.g., 5- to 20-membered fused heterocyclylene) and spiro-heterocyclylene groups (e.g., 5- to 20-membered spiro-heterocyclylene). Representative examples of the monocyclic heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptylene, azacyclooctylene, dioxacyclohexylene, diazacycloheptanylene (e.g., 1,4-diazacycloheptanylene, 4,5-diazacycloheptanylene, 1,3-diazacycloheptanylene), and diazacyclooctyl. Examples of the bridged heterocyclylene, fused heterocyclylene and spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). The heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "nitrogen-containing heterocyclylene", used alone or in combination, refers to 4- to 20-membered (e.g., 4- to 15-membered, 4- to 14-membered, 4- to 12-membered, 4- to 11-membered, 4- to 10-membered, 4- to 9-membered, 4- to 8-membered, 4- to 7-membered, 4- to 6-membered, or 4- to 5-membered) saturated or partially unsaturated (i.e., containing one or more double bonds, but not having a fully conjugated π-electron system) monocyclic, bicyclic, tricyclic or polycyclic divalent cyclic hydrocarbon radical containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing heterocyclylene groups include, but are not limited to, nitrogen-containing monocyclic heterocyclylene (e.g., 4- to 20-membered nitrogen-containing monocyclic heterocyclylene), nitrogen-containing bridged heterocyclylene (e.g., 5- to 20-membered nitrogen-containing bridged heterocyclylene, and 7- to 15-membered nitrogen-containing bridged heterocyclylene), nitrogen-containing fused heterocyclylene (e.g., 5- to 20-membered nitrogen-containing fused heterocyclylene) and nitrogen-containing spiro-heterocyclylene groups (e.g., 5- to 20-membered nitrogen-containing spiro-heterocyclylene). Representative examples of the nitrogen-containing monocyclic heterocyclylene include, but are not limited to, azetidinylene, pyrrolidinylene, imidazolidinylene, pyrazolidylene, oxazolidinylene, thiazolidinylene, piperidinylene, piperazinylene, morpholinylene, thiomorpholinylene, azacycloheptylene, azacyclooctylene, diazacycloheptanylene (e.g., 1,4-diazacycloheptanylene, 4,5-diazacycloheptanylene, 1,3-diazacycloheptanylene), and diazacyclooctylene. Examples of the nitrogen-containing bridged heterocyclylene, nitrogen-containing fused heterocyclylene and nitrogen-containing spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). The nitrogen-containing monocyclic heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "alkynylene", used alone or in combination, refers to a linear or branched bivalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynylene include, but are not

limited to, ethynylene, 1-propynylene, 1-butynylene, and 1,3-diynylene.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of $C_{2-6}$ alkynyl include, but are not limited to, ethynyl, 1-propynyl, 1-butynyl, and 1,3-diynyl.

As used herein, the term "alkenylene", used alone or in combination, refers to a linear or branched divalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenylene groups include, but are not limited to, vinylene (e.g., -CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of $C_{2-6}$ alkenyl group include, but are not limited to, vinyl (e.g., $CH_2$=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-enyl, 2-methyl-but-2-enyl, 3-methyl-but-2-enyl, 1-methyl-but-3-enyl, 2-methyl-but-3-enyl, 3-methyl-but-3-enyl, and hexenyl.

As used herein, "bornylane" or "bornane" (also known as 1,7,7-trimethylbicyclo[2.2.1]heptane; camphane; bornylane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

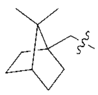 or 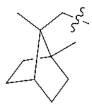 .

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en- 3-yl, or bicyclo[2.2.1]hept-5-en-7-yl.

As used herein, "adamantane" (also known as tricyclo[3.3.1.1$^{3,7}$]decane) has a definition known to those skilled in the art, and its structural formula is e.g., as follows:

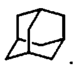 .

As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" has a definition known to those skilled in the art, and its structural formula is e.g., as follows:

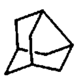 or 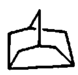 .

As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (corresponding English chemical name is adamantan-1-amine or Tricyclo[3.3.1.1$^{3,7}$]decan-1-amine; CAS No.: 768-94-5), with the following structural Formula:

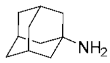.

Salts or pharmaceutically acceptable salts, enantiomers, stereoisomers, solvates, polymorphs of the compounds of Formula (I) of the present disclosure are also encompassed within the scope of the present invention.

In all embodiments of the present disclosure, the salts or pharmaceutically acceptable salts of the compounds of Formula (I) refer to non-toxic inorganic or organic acid and/or base addition salts. Examples include: sulfates, hydrohalides (including hydrochlorides and hydrobromates), maleates, sulfonates, citrates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, para-chlorobenzenesulfonates, 1,5-naphthalenedisulfonates, 3-hydroxy-2-naphthoates, 1-hydroxy-2-naphthoates, 2-naphthalenesulfonates, hydroxyacetates, or p-toluenesulfonates, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and the compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

As used herein, "p-menthane" has the definitions known to those skilled in the art, and its structural formula is e.g., as follows:

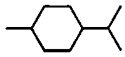

As used herein, "p-menthanyl" refers to a monovalent group of *p*-menthane, that is, the group remaining after any hydrogen in p-menthane is removed. Representative examples of "p-menthanyl" include, but are not limited to,

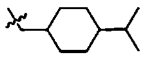, 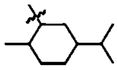, 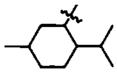, 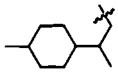,

or

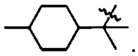.

As used herein, "*m*-menthane" has the definitions known to those skilled in the art, and its structural formula is e.g., as follows:

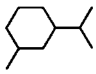

As used herein, "m-menthanyl" refers to a monovalent group of *m*-menthane, that is, the group remaining after any hydrogen in *m*-menthane is removed. Representative examples of "m-menthanyl" include, but are not limited to,

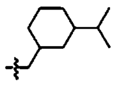, 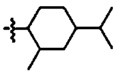, 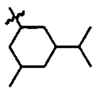, 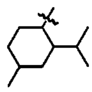,

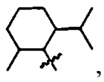, 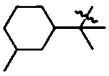, 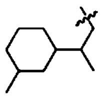, or 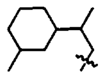.

As used herein, "Quinuclidine" (also known as 1-azabicyclo[2.2.2]octane) has the definitions known to those skilled in the art, and its structural formula is e.g., as follows:

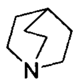

As used herein, "quinuclidinyl" refers to a monovalent group of Quinuclidine, that is, the group remaining after any hydrogen in Quinuclidine is removed. Representative examples of "quinuclidinyl" include, but are not limited to,

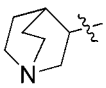, 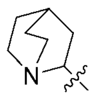, 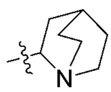, 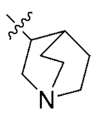, 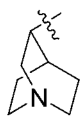, 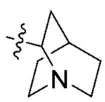, or 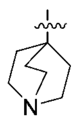.

## Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:

AcOH acetic acid

| | |
|---|---|
| NaOAc | sodium acetate |
| AIBN | 2,2'-azobis(2-methylpropionitrile) |
| Boc | t-Butyloxy carbonyl |
| Con. | Concentration |
| DBU | 1,8-diazabicyclo[5.4.0]undec-7-ene |
| DCM | dichloromethane |
| DIEA, or DIPEA | N, N-diisopropylethylamine |
| DMF | N,N-dimethylformamide |
| DMSO | dimethyl sulfoxide |
| EA | ethyl acetate |
| ESI | electrospray ionization |
| equiv | equivalent |
| EtOH | ethanol |
| EtOAc | ethyl acetate |
| HPLC | high performance liquid chromatography |
| HRMS | high resolution mass spectrometry |
| LC-MS | liquid chromatography-mass spectrometry |
| LRMS | low resolution mass spectrometry |
| LC | liquid chromatography |
| Me | methyl |
| MeCN | acetonitrile |
| MeOH | Methanol |
| MS | mass spectrometry |
| MsO- | Methanesulfonyloxy |
| $^1$H NMR | Proton nuclear magnetic resonance |
| MeO- | Methoxy |
| NBS | N-Bromosuccinimide |
| NMP | N-Methylpyrrolidone |
| ONs | o-Nitrobenzenesulfonyl |
| rt | room temperature |
| tBu | tert-butyl |
| TEA | triethylamine |
| TFA | trifluoroacetic acid |
| $T_fO$- | Trifluoromethanesulfonate |
| TLC | thin layer chromatography |
| TMS | tetramethylsilane |
| TsO- | Tosyloxy |
| Xantphos | 4,5-bis(diphenylphosphino)-9,9-dimethylxanthene |
| X-Phos | 2-dicyclohexylphosphino-2',4',6'-triisopropylbiphenyl |

In the present disclosure, the $^1$H NMR spectrum was recorded on a Bruker-500MHz nuclear magnetic resonance instrument, by using, as a solvent, $CD_3OD$ ($\delta$ = 3.31 ppm) containing 0.1% TMS (as an internal standard); or using, as a solvent, $CDCl_3$ ($\delta$ = 7.26 ppm) containing 0.1% TMS (as an internal standard); or using, as a solvent, DMSO-$d_6$ ($\delta$ = 2.50 ppm) containing 0.03% TMS (as an internal standard). LC-MS spectra were recorded on a Sciex API 2000 mass spectrometer equipped with an Agilent 1100 binary pump, DAD and ELSD detectors, or on an Agilent 1260-6125B single quadrupole LC-MS system equipped with an Agilent 1260 quaternary pump, DAD and ELSD detectors. HPLC preparative purification was performed using a SHIMADZU LC-20AP system. HPLC purity was determined using either a SHIMADZU LC-30AP or Waters 1525 system. All reactions were carried out under ambient atmosphere unless otherwise specified. Reaction progress was monitored by TLC or LC-MS.

Solvents and reagents are processed as follows: the solvents used in the reactions such as DCM, DMF, anhydrous EtOH, and anhydrous MeOH were all purchased from Sinopharm Group; HPLC preparation uses preparation grade $CH_3CN$ and deionized water; Other reaction substrates, reagents and drugs may be commercially available without special instructions, or may be synthesized using or according to methods known in the art.

Unless otherwise specified, the materials and reagents used in the following examples are commercially available and used directly, or can be synthesized by using methods known in the art.

**General synthesis methods**

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) of the present disclosure according to routine techniques in the art.

Scheme 1:

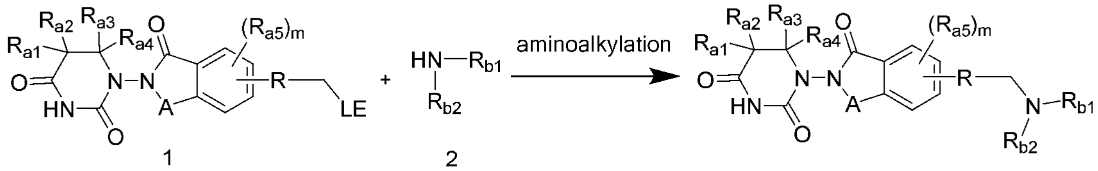


1 2

Scheme 1

In Scheme 1, $R_{a1}$, $R_{a2}$, $R_{a3}$, $R_{a4}$, $(R_{a5})_m$, A, and R are as defined in the compound of Formula (I) and its various sub-embodiments in the present disclosure. Group LE represents Cl, Br, I, OMs, OTs, or ONs. The substrate amine is a compound corresponding to the X moiety in the compound of Formula (I), wherein said X moiety contains an amino group or a nitrogen-containing heterocycle. In some embodiments, group $R_{b1}$ of the substrate amine is as defined for group $R_1$ of the compound of Formula (I) and its various sub-embodiments, and group $R_{b2}$ is as defined for group $R_2$ of the compound of Formula (I) and its various sub-embodiments. In some embodiments, groups $R_{b1}$ and $R_{b2}$ of the substrate amine, together with the nitrogen atom to which they are attached, form an optionally substituted nitrogen-containing heterocycle $A_a$ represented by the following general formula:

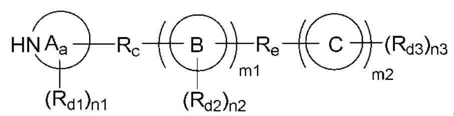

,

wherein nitrogen-containing heterocycle $A_a$ is as defined for ring A of the compound of Formula (I) and its various sub-embodiments; and $(R_{d1})_{n1}$, $R_c$, ring B, m1, $(R_{d2})_{n2}$, $R_c$, ring C, m2, and $(R_{d3})_{n3}$ are as defined for the compound of Formula (I) of the present disclosure and its various sub-embodiments.

The aminoalkylation reaction in Scheme 1 can be conducted, for example, in the presence of DIEA and sodium iodide, or triethylamine and sodium iodide, at a temperature ranging from room temperature to 80 °C (e.g., 40 °C to 60 °C, 40 °C to 50 °C, or 50 °C to 60 °C). The molar ratio of substrate 1 to substrate 2 may be, for example, 1:1.1-2, 1:1.1-1.5, 1:1.1-1.2, or 1:1.2-1.3.

For example, the specific procedure for Scheme 1 can be carried out as follows

To a solution of the mesylate substrate 1 (1.3 eq.) and the amine substrate 2 (1.0 eq.) in anhydrous DMF (3 mL) are added triethylamine (3.0 eq.) and sodium iodide (1.0 eq.). The reaction mixture is stirred at 50 °C for 2 hours. After monitoring the reaction via LCMS and confirming its completion, the reaction mixture is filtered, and the filtrate is separated and purified by preparative HPLC to give the target compound.

Scheme 2:

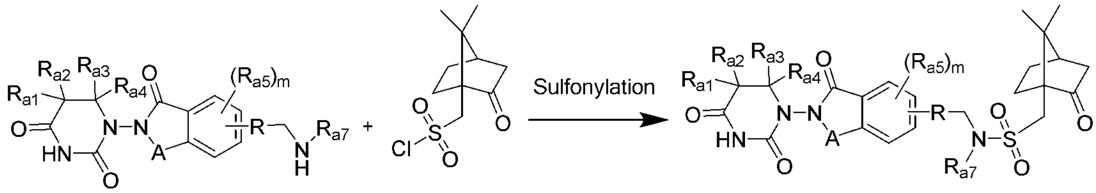


Scheme 2

In Scheme 2, $R_{a1}$, $R_{a2}$, $R_{a3}$, $R_{a4}$, $(R_{a5})_m$, A, R, and $R_{a7}$ are as defined in the compound of Formula (I) and its various sub-embodiments in the present disclosure.

To a solution of the amine substrate (1.0 eq.) and sulfonyl chloride substrate (1.1 eq.) in anhydrous DMF (3 mL) is added triethylamine (3.0 eq.). The reaction mixture is stirred at 25 °C for 2 hours. After monitoring the reaction via LCMS and confirming its completion, the reaction mixture is filtered, and the filtrate is separated and purified by preparative HPLC to give the target compound.

Scheme 3:

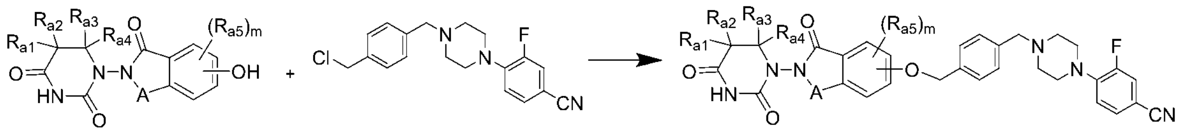

Scheme 3

In Scheme 3, $R_{a1}$, $R_{a2}$, $R_{a3}$, $R_{a4}$, $(R_{a5})_m$, and A are as defined in the compound of Formula (I) and its various sub-embodiments in the present disclosure.

To a solution of the alcohol substrate (1.0 eq.) and chloro substrate (1.2 eq.) in anhydrous DMF is added $K_2CO_3$ (5.0 eq.). The reaction mixture is stirred at 50-80 °C for 0.5-48 hours. After monitoring the reaction via LCMS and confirming its completion, the reaction mixture is filtered, and the filtrate is separated and purified by preparative HPLC to give the target compound.

Scheme I-1 for intermediate compounds:

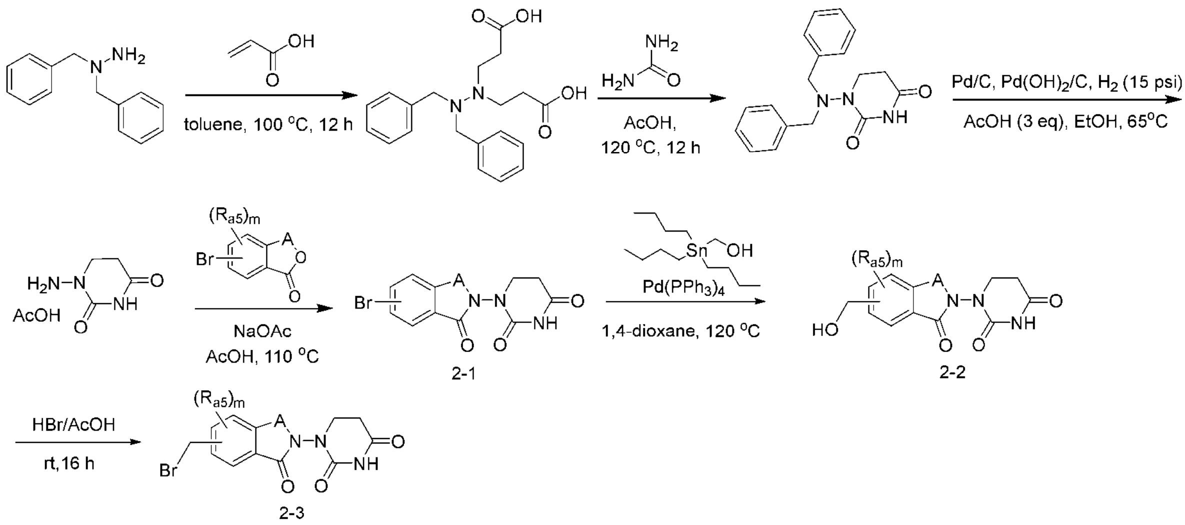


Scheme I-1

In Scheme I-1, the substituent Br on the substrate in Step 4 can be located at the 4-, 5-, 6-, or 7-position of the benzene ring of the isoindolinone moiety. Consequently, the Br substituent in the resulting intermediate 2-1, as well as the $-CH_2-OH$ and $-CH_2-Br$ substituents in the subsequent products 2-2 and 2-3, will correspondingly be located at the 4-, 5-, 6-, or 7-position of the benzene ring of the isoindolinone moiety. $(R_{a5})_m$ and A are as defined in the compound of Formula (I) of the present disclosure and its various sub-embodiments.

The coupling reaction in Step 5 of Scheme I-1 can be conducted, for example, at a temperature between 40 °C and 120 °C in the presence of tetrakis(triphenylphosphine)palladium(0) and 1,4-dioxane (or DMF). The halogenation reaction in Step 6 of Scheme I-1 can be performed at room temperature using, for example, a hydrohalic acid and acetic acid. Alternatively, depending on requirements, the halogenation reaction in Step 6 can be replaced with an esterification reaction to prepare the corresponding products containing OMs, OTs, or ONs groups.

For example, the procedures for Steps 5 and 6 of Scheme I-1 can be carried out as follows:

Step 5: to a solution of the bromo substrate 2-1 (1.0 eq.) and (tributylstannyl)methanol (1.5 eq.) in DMF is added tetrakis(triphenylphosphine)palladium(0) (0.05 eq.). Under a nitrogen ($N_2$) atmosphere, the reaction mixture is heated to 100 °C and stirred for 48 hours. Reaction completion is monitored by thin-layer chromatography (TLC). The reaction mixture is cooled to 60 °C, filtered hot to promptly remove the black solids, and the filtrate is concentrated under reduced pressure. Dichloromethane (DCM) is added to the residue, followed by filtration, to afford the solid intermediate compound 2-2.

Step 6: The solid intermediate compound 2-2 and HBr/AcOH are stirred at room temperature for 16 hours.

Reaction completion is confirmed by TLC. The reaction mixture is directly concentrated to obtain the target compound.

Scheme I-2 for intermediate compounds:

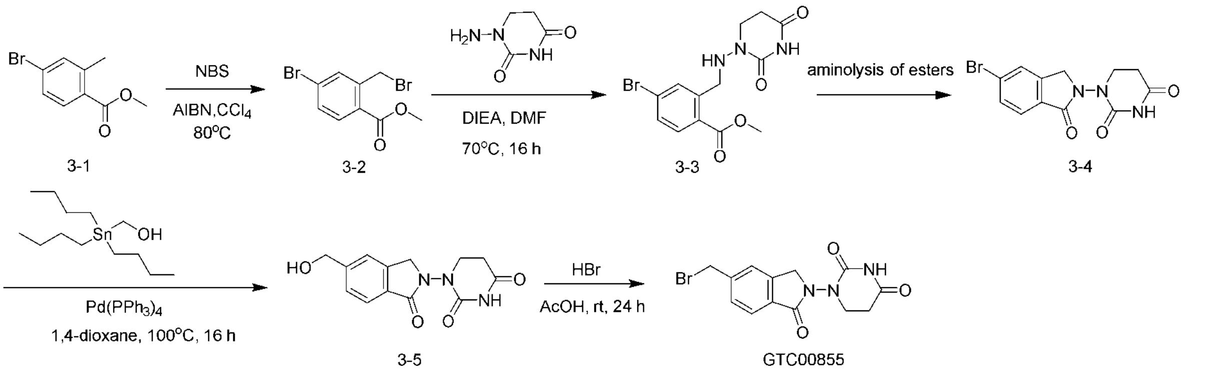


Scheme I-2

The aminolysis of the ester in Step 3 of Scheme I-2 can be conducted at room temperature in the presence of $CH_3ONa$ and methanol. Alternatively, the reaction can be performed at a temperature between 40 °C and 90 °C in the presence of $CH_3OLi$ or $Cs_2CO_3$ in methanol, or DBU in THF under the same temperature range.

Scheme I-3 for intermediate compounds:

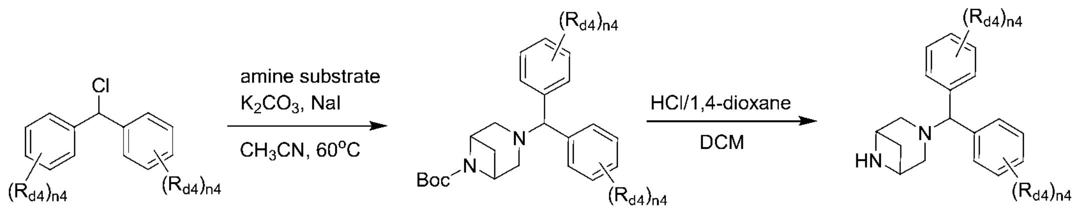


Scheme I-3

In Scheme I-3, $(R_{d4})_{n4}$ indicates that the benzene ring is substituted by n4 $R_{d4}$ groups, wherein each $R_{d4}$ is the same or different and independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl; and n4 represents an integer from 0 to 5.

The aminoalkylation reaction in Step 1 of Scheme I-3 may employ conventional techniques and methods well-known to those skilled in the art. For example, the reaction may proceed in the presence of N,N-diisopropylethylamine (DIEA) and sodium iodide, or triethylamine and sodium iodide, at temperatures ranging from room temperature to 80°C. The Boc deprotection reaction in Step 2 of Scheme I-3 may also employ conventional techniques and methods familiar to those skilled in the art, such as using a solution of HCl in 1,4-dioxane.

For illustration, Scheme I-3 may be performed as follows:

Step 1: To a solution of the halide substrate (1.0 equiv.) and amine substrate (1.5 equiv.) in acetonitrile are added potassium carbonate (3.0 equiv.) and sodium iodide (1.0 equiv.). The reaction mixture is heated to 60°C and stirred for 18 hours. After monitoring the reaction via TLC and confirming its completion, the reaction mixture is cooled to room temperature, filtered, and the filtrate is concentrated under reduced pressure. The residue is washed with water and extracted with dichloromethane. The organic phases are combined, dried over anhydrous sodium sulfate, filtered, and the filtrate is concentrated under reduced pressure. The residue is purified by column chromatography to obtain the solid intermediate.

Step 2: to a solution of the solid intermediate from Step 1 (1.0 equiv.) in dichloromethane is added a solution of HCl in 1,4-dioxane. The reaction mixture is stirred at room temperature for 1 hour. After monitoring the reaction via TLC and confirming its completion, the reaction mixture is concentrated under reduced pressure to obtain the target product.

Scheme I-4 for intermediate compounds:

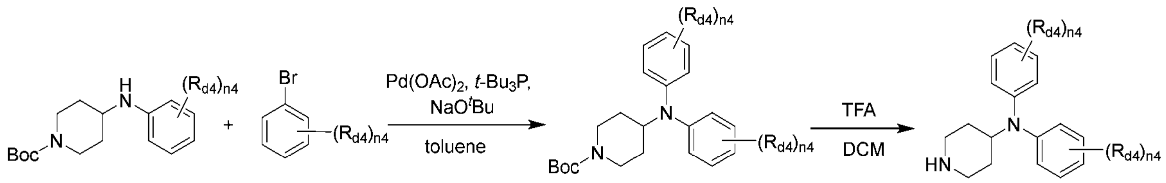


Scheme I-4

In Scheme I-4, $(R_{d4})_{n4}$ is as defined in Scheme I-3.

The coupling reaction in Step 1 of Scheme I-4 may be performed using conventional techniques and methods well-known to those skilled in the art, such as in the presence of $Pd_2(dba)_3$ and X-Phos, or $Pd(OAc)_2$ and Xantphos. The Boc deprotection reaction in Step 2 of Scheme I-4 may also employ conventional techniques and methods familiar to those skilled in the art, for example using a solution of HCl in 1,4-dioxane.

For illustration, Scheme I-4 may be executed as follows:

Step 1: to a solution of the halide substrate (1.0 equiv.) and amine substrate (1.0 equiv.) in anhydrous toluene are sequentially added $Pd(OAc)_2$ (0.1 eq.), t-$Bu_3P$ (0.5 eq.), and sodium tert-butoxide (2.25 equiv.). Under an $N_2$ atmosphere, the reaction mixture is heated to 100°C and stirred for 18 hours. After monitoring the reaction via TLC and confirming its completion, the reaction solution is cooled to room temperature, washed with water, and extracted with ethyl acetate. The organic phases are combined, dried over anhydrous $Na_2SO_4$, filtered, and the filtrate is concentrated under reduced pressure. The residue is purified by column chromatography to afford the solid intermediate.

Step 2: to a solution of the solid intermediate from Step 1 (1.0 equiv.) in dichloromethane is added trifluoroacetic acid (2 mL). The reaction mixture is stirred at room temperature for 1 hour. After monitoring the reaction via TLC and confirming its completion, the reaction solution is concentrated under reduced pressure to obtain the target product.

Scheme I-5 for intermediate compounds:

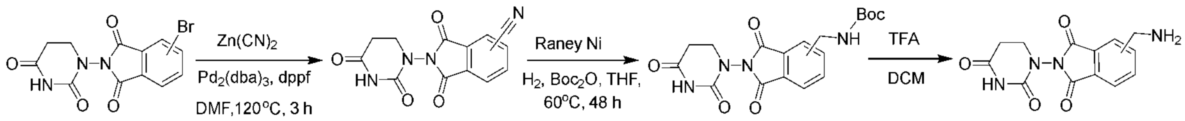


Scheme I-5

Scheme I-6 for intermediate compounds:

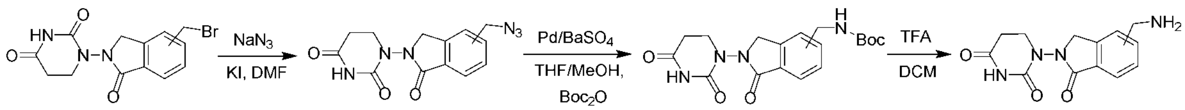


Scheme I-6

Depending upon the target compounds, the various schemes mentioned above and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

Examples

**Intermediate example 1: preparation of 5-(bromomethyl)-2-(2,4-dioxotetrahydropyrimidin-1(2H)-yl)isoindoline-1,3-dione (GTC00838)**

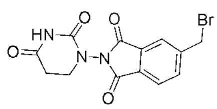

Referring to the method of Scheme I-1, the target product 5-(bromomethyl)-2-(2,4-dioxotetrahydropyrimidin-1(2H)-yl)isoindoline-1,3-dione was prepared as yellow solid (7.07 g, yield 96.79%). $^1$H NMR (400 MHz, DMSO) δ 10.90 (s, 1H), 8.08 (s, 1H), 7.99 (s, 2H), 4.90 (s, 2H), 3.81 (t, J = 6.8 Hz, 2H), 2.84 (t, J = 6.8 Hz, 2H). LCMS (ESI) calcd for $C_{13}H_{11}BrN_3O_4^+$ $[M+H]^+$: 351.99/353.99; found, 352.0/354.0.

**Intermediate example 2: preparation of 1-(5-(bromomethyl)-1-oxoisoindolin-2-yl)dihydropyrimidine-2,4(1H,3H)-dione (GTC00855)**

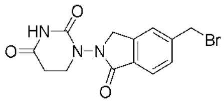

Referring to the method of Scheme I-2, the target product 1-(5-(bromomethyl)-1-oxoisoindolin-2-yl)dihydropyrimidine-2,4(1H,3H)-dione was prepared as yellow solid (7.07 g, yield 96.79%). $^1$H NMR (400 MHz, DMSO) δ 10.67 (s, 1H), 7.76 (d, *J* = 7.8 Hz, 1H), 7.71 (s, 1H), 7.62 (d, *J* = 7.9 Hz, 1H), 4.83 (s, 2H), 4.76 (d, *J* = 16.6 Hz, 1H), 4.53 (d, *J* = 16.3 Hz, 1H), 3.76 (d, *J* = 5.2 Hz, 2H), 2.93 - 2.81 (m, 1H), 2.76 (s, 1H). LCMS (ESI) calcd for $C_{13}H_{13}BrN_3O_3^+$ $[M+H]^+$ $[M+H]^+$, 338.01/340.01; found, 338.0/340.0.

**Intermediate example 3: preparation of 3-benzhydryl-3,6-diazabicyclo[3.1.1]heptane**

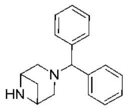

Referring to the method of Scheme I-3, the target product 3-benzhydryl-3,6-diazabicyclo[3.1.1]heptane was prepared as off-white solid (560 mg, yield 88.%). $^1$H NMR (400 MHz, DMSO) *δ* 9.18 (s, 1H), 7.71 (s, 1H), 7.52 (d, *J* = 7.2 Hz, 4H), 7.33 (t, *J* = 7.6 Hz, 4H), 7.22 (t, *J* = 7.2 Hz, 2H), 4.67 (s, 1H), 4.22 (s, 2H), 3.05 (d, *J* = 12.0 Hz, 2H), 2.81 (d, *J* = 12.0 Hz, 2H), 2.69 - 2.58 (m, 1H), 2.37 - 2.28 (m, 1H). LCMS (ESI) m/z: calcd for $C_{18}H_{21}N_2^+$ $[M+H]^+$, 265.17; found, 265.2.

**Intermediate example 4: preparation of (1S,4S)-2-benzhydryl-2,5-diazabicyclo[2.2.1]heptane**

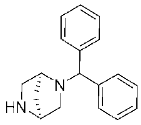

Referring to the method of Scheme I-3, the target product (1S,4S)-2-benzhydryl-2,5-diazabicyclo[2.2.1]heptane was prepared as brown solid (312 mg, yield 82%). $^1$H NMR (400 MHz, DMSO) δ 9.02 (brs, 1H), 8.84 (brs, 1H), 7.48 (d, *J* = 7.6 Hz, 4H), 7.31 (t, *J* = 7.6 Hz, 4H), 7.21 - 7.18 (m, 2H), 4.76 (s, 1H), 4.17 (s, 1H), 3.46 (s, 1H), 3.36 (s, 1H), 3.01 (brs, 1H), 2.68 - 2.64 (m, 2H), 2.12 (brs, 1H), 1.66 (d, *J* = 10.4 Hz, 1H). LCMS (ESI) m/z: calcd for $C_{18}H_{21}N_2^+$ $[M+H]^+$, 265.17; found, 265.5.

**Intermediate example 5: preparation of (1R,4R)-2-benzhydryl-2,5-diazabicyclo[2.2.1]heptane**

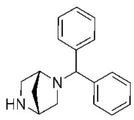

Referring to the method of Scheme I-3, the target product (1R,4R)-2-benzhydryl-2,5-diazabicyclo[2.2.1]heptane

was prepared as pale yellow solid (180 mg, yield 92%). LCMS (ESI) m/z: calcd for $C_{18}H_{21}N_2^+$ $[M+H]^+$, 265.17; found, 265.2.

**Intermediate example 6: preparation of 3-benzhydryl-3,8-diazabicyclo[3.2.1]octane**

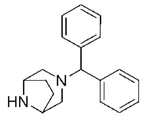

Referring to the method of Scheme I-3, the target product 3-benzhydryl-3,8-diazabicyclo[3.2.1]octanewas prepared as a white solid (133 mg, yield 66 %). $^1H$ NMR (400 MHz, CDCl3) δ 7.43(s, 1H), 9.17 (s, 1H), 7.40 - 7.29 (m, 4H), 7.27 - 7.25 (m, 4H), 7.20 - 7.16(m, 2H), 4.43 (s, 1H), 3.81 (s, 1H), 2.75 - 2.72 (m, 2H), 2.51 - 2.48 (m, 2H), 2.20 - 2.11 (m, 1H). LCMS (ESI) m/z: calcd for $C_{19}H_{23}N_2^+$ $[M+H]^+$, 279.19; found, 279.4.

**Intermediate example 7: preparation of 8-benzhydryl-3,8-diazabicyclo[3.2.1]octane**

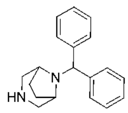

Referring to the method of Scheme I-3, the target product 8-benzhydryl-3,8-diazabicyclo[3.2.1]octane was prepared as a white solid (80 mg, yield 24%). $^1H$ NMR (400 MHz, DMSO) *δ* 8.99 (s, 1H), 8.40 (s, 1H), 7.55 - 7.51 (m, 4H), 7.33 - 7.29 (m, 4H), 7.22 - 7.18 (m, 2H), 4.54 (s, 1H), 3.20 - 2.99 (m, 6H), 2.11 (brs, 2H), 1.81 - 1.79 (m, 2H). LCMS (ESI) m/z: calcd for $C_{19}H_{23}N_2^+$ $[M+H]^+$, 279.19; found, 279.4.

**Intermediate example 8: preparation of (R)-1-benzhydryl-3-methylpiperazine**

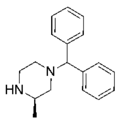

Referring to the method of Scheme I-3, the target product (R)-1-benzhydryl-3-methylpiperazine was prepared as pale yellow solid (167 mg, yield 89%). LCMS (ESI) m/z: calcd for $C_{18}H_{23}N_2^+$ $[M+H]^+$, 267.19; found, 267.2.

**Intermediate example 9: preparation of 1-benzhydryl-3,5-dimethylpiperazine**

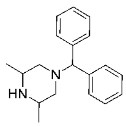

Referring to the method of Scheme I-3, the target product 1-benzhydryl-3,5-dimethylpiperazine was prepared as a white solid (295 mg, yield 91%). $^1H$ NMR (400 MHz, DMSO) δ 9.03 (s, 1H), 8.20 (s, 1H), 7.42 - 7.40 (m, 4H), 7.34 - 7.30 (m, 4H), 7.24 - 7.21 (m, 2H), 4.51 (s, 1H), 3.39 (brs, 2H), 2.85 (d, *J* = 11.6 Hz, 2H), 1.90 (t, *J* = 11.6 Hz, 2H), 1.11 (d, *J* = 6.8 Hz, 6H). LCMS (ESI) m/z: calcd for $C_{19}H_{25}N_2^+$ $[M+H]^+$, 281.20; found, 281.4.

**Intermediate example 10: preparation of 2-benzhydryl-2,5-diazabicyclo[2.2.2]octane**

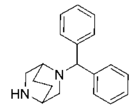

Referring to the method of Scheme I-3, the target product 2-benzhydryl-2,5-diazabicyclo[2.2.2]octane was prepared as a white solid (198 mg, yield 84%). $^1H$ NMR (400 MHz, MeOD) δ 7.57 - 7.54 (m, 4H), 7.34 - 7.30 (m, 1H), 7.25 - 7.21 (m, 2H), 4.97 (s, 1H), 3.78 - 3.64 (m, 1H), 3.61 (brs, 1H), 3.33 (brs, 1H), 3.16 (d, J = 12.4 Hz, 1H), 3.07 (s, 1H), 2.87 (d, J = 13.2 Hz, 1H), 2.39 - 2.25 (m, 1H), 2.05 - 2.01 (m, 1H), 1.77 - 1.64 (m, 1H). LCMS (ESI) m/z: calcd for $C_{19}H_{23}N_2^+$ [M+H]$^+$, 279.18; found, 279.2.

**Intermediate example 11: preparation** of **1-benzhydryl-2,2-dimethylpiperazine**

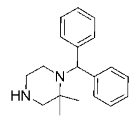

Referring to the method of Scheme I-3, the target product 1-benzhydryl-2,2-dimethylpiperazine was prepared as a white solid (170 mg, yield 82%). LCMS (ESI) m/z: calcd for $C_{19}H_{25}N_2^+$ [M+H]$^+$, 281.20; found, 281.2.

**Intermediate example 12: preparation of 1-(bis(3-fluorophenyl)methyl)piperazine**

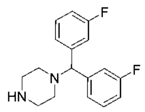

Referring to the method of Scheme I-3, the target product 1-(bis(3-fluorophenyl)methyl)piperazine was prepared as a white solid (700 mg, yield 97 %). $^1H$ NMR (400 MHz, $D_2O$) δ 7.50 - 7.38 (m, 6H), 7.21 - 7.17 (m, 2H), 5.35 (s, 1H), 3.58 - 3.55 (m, 4H), 3.42 - 3.40 (m, 1H). LCMS (ESI) m/z: calcd for $C_{17}H_{19}F_2N_2^+$ [M+H]$^+$, 289.15; found, 289.2.

**Intermediate example 13: preparation of N,N-diphenylpiperidin-4-amine**

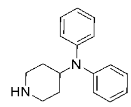

The target product N,N-diphenylpiperidin-4-amine was prepared by referring to the method of Scheme I-4,.

Step 1: to a solution of tert-butyl 4-(phenylamino)piperidine-1-carboxylate (5 g, 18.09 mmol) and bromobenzene (1.91 mL, 18.09 mmol) in anhydrous toluene (50 mL) were successively added $Pd(OAc)_2$ (0.41 g, 1.81 mmol), t-$Bu_3P$ (1.83 g, 9.046 mmol), and sodium tert-butoxide (3.91 g, 40.71 mmol). The reaction system was purged with $N_2$ three times, heated to 100 °C, and stirred for 18 hours. After monitoring the reaction via TLC and confirming its completion, the reaction mixture was cooled to room temperature, washed with water, extracted with ethyl acetate, and the combined organic phases were dried over anhydrous $Na_2SO_4$, and filtered. The filtrate was concentrated under reduced pressure. The residue was purified by column chromatography to afford tert-butyl 4-(diphenylamino)piperidine-1-carboxylate as a pale yellow solid (3 g, 47% yield). LCMS (ESI) m/z: calcd for $C_{22}H_{29}N_2O_2^+$ [M+H]$^+$, 353.22; found, 353.2.

Step 2: to a solution of tert-butyl 4-(diphenylamino)piperidine-1-carboxylate (2.9 g, 8.23 mmol) in dichloromethane (10 mL) was added trifluoroacetic acid (2 mL). The reaction mixture was stirred at room temperature for 1 hour. After monitoring the reaction via TLC and confirming its completion, the reaction mixture was concentrated under reduced pressure to afford N,N-diphenylpiperidin-4-amine trifluoroacetate as an off-white solid (1.7 g, 82% yield). $^1$H NMR (400 MHz, DMSO) $\delta$ 8.77 (brs, 1H), 8.16 (brs, 1H), 7.32 - 7.28 (m, 4H), 7.03 - 7.00 (m, 2H), 6.86 - 6.84 (m, 4H), 4.27 (t, *J* = 3.2 Hz, 1H), 3.31 (d, *J* = 12.4 Hz, 2H), 3.14 - 3.10 (m, 2H), 2.05 (d, *J* = 12.0 Hz, 2H), 1.51 - 1.47 (m, 2H). LCMS (ESI) m/z: calcd for $C_{17}H_{21}N_2^+$ [M+H]$^+$, 253.17; found, 253.2.

**Intermediate example 14: preparation of 5-(aminomethyl)-2-(2,4-dioxotetrahydropyrimidin-1(2H)-yl)isoindoline-1,3-dione (GT-07968)**

Referring to the method of Scheme I-5, the target product 5-(aminomethyl)-2-(2,4-dioxotetrahydropyrimidin-1(2H)-yl)isoindoline-1,3-dione was prepared as a white solid (0.89 g). $^1$H NMR (400 MHz, DMSO-d6) $\delta$ 10.92 (s, 1H), 8.44 (s, 3H), 8.12 (s, 1H), 8.07 (d, J = 7.7 Hz, 1H), 7.99 (d, J = 7.8 Hz, 1H), 4.29 (s, 2H), 3.83 (t, J = 6.8 Hz, 2H), 2.85 (t, J = 6.8 Hz, 2H). LCMS (ESI): $C_{13}H_{13}N_4O_4^+$ [M+H]$^+$, calcd for 289.09, found, 289.0.

**Intermediate example 15: preparation of 1-(5-(aminomethyl)-1-oxoisoindolin-2-yl)dihydropyrimidine-2,4(1H,3H)-dione (GT-08118)**

Referring to the method of Scheme I-6, the target product 1-(5-(aminomethyl)-1-oxoisoindolin-2-yl)dihydropyrimidine-2,4(1H,3H)-dione was prepared as a white solid (25 mg). $^1$H NMR (400 MHz, DMSO-d6) $\delta$ 10.67 (s, 1H), 8.25 (s, 3H), 7.84 (d, J = 7.8 Hz, 1H), 7.68 (s, 1H), 7.61 (d, J = 7.9 Hz, 1H), 4.79 (d, J = 17.0 Hz, 1H), 4.54 (d, J = 16.6 Hz, 1H), 4.18 (q, J = 5.6 Hz, 2H), 3.78 (s, 2H), 2.85 (d, J = 7.5 Hz, 1H), 2.76 (s, 1H). LCMS (ESI): calcd for $C_{13}H_{15}N_4O_3^+$ [M+H]$^+$, 275.11, found, 275.2.

**Example 1: preparation of 1-(5-((4-(2,3-dichlorophenyl)piperazin-1-yl)methyl)-1-oxoisoindolin-2-yl)dihydropyrimidine-2,4(1H,3H)-dione (GT-04853)**

Referring to the method of Scheme 1, the target product **GT-04853** was prepared as a white solid (22 mg, yield 37%). $^1$H NMR (400 MHz, MeOD) $\delta$ 7.88 (d, J = 7.8 Hz, 1H), 7.74 (s, 1H), 7.67 (d, J = 8.1 Hz, 1H), 7.25 - 7.16 (m, 2H), 7.06 (dd, J = 7.2, 2.3 Hz, 1H), 4.74 - 4.66 (m, 2H), 4.49 (s, 2H), 3.89 - 3.81 (m, 1H), 3.79 - 7.72 (m, 1H), 3.59 - 3.27 (m, 6H), 3.11 - 2.98 (m, 2H), 2.84 (t, J = 7.0 Hz, 2H). LCMS (ESI) calcd for $C_{23}H_{24}Cl_2N_5O_3^+$ [M+H]$^+$: 488.13, found, 488.1.

**Example 2: preparation of 1-(5-((4-(6-fluorobenzo[d]isoxazol-3-yl)piperidin-1-yl)methyl)-1-oxoisoindolin-2-yl)dihydropyrimidine-2,4(1H,3H)-dione (GT-04864)**

Referring to the method of Scheme 1, the target product **GT-04864** was prepared as a white solid (32 mg, yield 46%). $^1$H NMR (400 MHz, DMSO) $\delta$ 11.03 (brs, 1H), 10.69 (s, 1H), 8.17 (dd, J = 8.7, 5.2 Hz, 1H), 7.95 - 7.88 (m, 2H), 7.82 (d, J = 7.8 Hz, 1H), 7.72 (dd, J = 9.1, 1.9 Hz, 1H), 7.33 (td, J = 9.1, 2.0 Hz, 1H), 4.83 (d, J = 16.7 Hz, 1H), 4.60 (d, J = 16.7 Hz, 1H), 4.51 (d, J = 4.3 Hz, 2H), 3.80 (t, J = 6.8 Hz, 2H), 3.54 (d, J = 11.2 Hz, 2H), 3.21 - 3.08 (m, 3H), 2.89 - 2.84 (m, 1H), 2.78 - 2.73 (m, 1H), 2.38 - 2.32 (m, 3H), 2.26 - 2.13 (m, 2H). LCMS (ESI) calcd for $C_{25}H_{25}FN_5O_4^+$ [M+H]$^+$: 478.19, found, 478.2.

**Example 3: preparation of 1-(5-((4-(benzo[d]isothiazol-3-yl)piperazin-1-yl)methyl)-1-oxoisoindolin-2-yl)dihydropyrimidine-2,4(1H,3H)-dione (GT-04865)**

Referring to the method of Scheme 1, the target product **GT-04865** was prepared as a white solid (33 mg, yield 47%). $^1$H NMR (400 MHz, DMSO) $\delta$ 10.87 (brs, 1H), 10.69 (s, 1H), 8.12 (t, J = 8.5 Hz, 2H), 7.94 - 7.86 (m, 2H), 7.82 - 7.77 (m, 1H), 7.60 (t, J = 7.6 Hz, 1H), 7.47 (t, J = 7.6 Hz, 1H), 4.86 - 4.79 (m, 1H), 4.68 - 4.52 (m, 3H), 4.16 - 4.02 (m, 2H), 3.80 (t, J = 7.0 Hz, 2H), 3.47 - 3.44 (m, 5H), 2.96 - 2.72 (m, 2H), 1.06 (t, J = 7.0 Hz, 1H). LCMS (ESI) calcd for $C_{24}H_{25}N_6O_3S^+$ [M+H]$^+$: 477.17, found, 477.2.

**Example 4: preparation of 1-(5-((4-(benzo[b]thiophen-4-yl)piperazin-1-yl)methyl)-1-oxoisoindolin-2-yl)dihydropyrimidine-2,4(1H,3H)-dione (GT-04866)**

Referring to the method of Scheme 1, the target product **GT-04866** was prepared as a white solid (44 mg, yield 63%). $^1$H NMR (400 MHz, DMSO) $\delta$ 11.21 (brs, 1H), 10.69 (s, 1H), 7.94 (s, 1H), 7.91 (d, J = 7.8 Hz, 1H), 7.84 (d, J = 7.8 Hz, 1H), 7.76 (d, J = 5.5 Hz, 1H), 7.70 (d, J = 8.0 Hz, 1H), 7.48 (d, J = 5.5 Hz, 1H), 7.31 (t, J = 7.9 Hz, 1H), 6.95 (d, J = 7.7 Hz, 1H), 4.83 (d, J = 17.2 Hz, 1H), 4.62 - 4.58 (m, 3H), 3.80 (t, J = 6.8 Hz, 2H), 3.47 - 3.31 (m, 5H), 3.27 - 3.23 (m, 3H), 2.89 - 2.84 (m, 1H), 2.78 - 2.72 (m, 1H). LCMS (ESI) calcd for $C_{25}H_{26}N_5O_3S^+$ [M+H]$^+$: 476.18, found, 476.2.

**Example 5: preparation of 1-(5-((4-(2-((2,4-dimethylphenyl)thio)phenyl)piperazin-1-yl)methyl)-1-oxoisoindolin-2-yl)dihydropyrimidine-2,4(1H,3H)-dione (GT-04867)**

Referring to the method of Scheme 1, the target product **GT-04867** was prepared as a white solid (26 mg, yield 49%). $^1$H NMR (400 MHz, DMSO) δ 10.69 (s, 1H), 10.63 (s, 1H), 7.92 - 7.89 (m, 2H), 7.80 (d, J = 7.5 Hz, 1H), 7.33 (d, J = 7.8 Hz, 1H), 7.24 (d, J = 6.9 Hz, 1H), 7.16 (s, 2H), 7.10 (d, J = 7.8 Hz, 1H), 6.97 (t, J = 5.9 Hz, 1H), 6.42 (t, J = 7.0 Hz, 1H), 4.89 - 4.78 (m, 1H), 4.67 - 4.54 (m, 3H), 3.79 (t, J = 6.7 Hz, 2H), 3.55 - 3.38 (m, 5H), 3.24 - 3.18 (m, 3H), 2.91 - 2.83 (m, 1H), 2.81 - 2.71 (m, 1H), 2.32 (s, 3H), 2.23 (s, 3H). LCMS (ESI) calcd for $C_{31}H_{34}N_5O_3S^+$ [M+H]$^+$: 556.24, found, 556.3.

**Example 6: preparation of 1-(1-oxo-5-((4-(thiophen-2-yl)piperazin-1-yl)methyl)isoindolin-2-yl)dihydropyrimidine-2,4(1H,3H)-dione (GT-05073)**

Referring to the method of Scheme 1, the target product **GT-05073** was prepared as a white solid (46 mg, yield 59%). $^1$H NMR (400 MHz, DMSO-d6) δ 11.43 (s, 1H), 10.63 (s, 1H), 7.85 (s, 1H), 7.81 (d, *J* = 7.8 Hz, 1H), 7.75 (d, *J* = 7.8 Hz, 2H), 6.65 (d, *J* = 3.7 Hz, 1H), 6.06 (d, *J* = 3.8 Hz, 1H), 4.75 (d, *J* = 16.6 Hz, 1H), 4.51 (d, *J* = 16.9 Hz, 1H), 4.45 (s, 2H), 3.83 - 3.79 (m, 1H), 3.72 (t, *J* = 6.8 Hz, 2H), 3.66 (dd, *J* = 10.9, 6.0 Hz, 1H), 3.45 (brs, 2H), 3.16 (brs, 2H), 2.85 - 2.76 (m, 2H), 2.73 - 2.86 (m, 2H). LCMS (ESI) calcd for $C_{21}H_{24}N_5O_3S^+$ [M+H]$^+$: 426.16, found, 426.1.

**Example 7: preparation of 1-(5-((4-(5-methylthiophen-2-yl)piperazin-1-yl)methyl)-1-oxoisoindolin-2-yl)dihydropyrimidine-2,4(1H,3H)-dione (GT-05083)**

Referring to the method of Scheme 1, the target product **GT-05083** was prepared as a white solid (19 mg, yield 23%). LCMS (ESI) calcd for $C_{22}H_{26}N_5O_3S^+$ [M+H]$^+$: 440.18, found, 440.2.

**Example 8: preparation of 1-(5-((4-(3-methylthiophen-2-yl)piperazin-1-yl)methyl)-1-oxoisoindolin-2-yl)dihydropyrimidine-2,4(1H,3H)-dione (GT-05084)**

Referring to the method of Scheme 1, the target product **GT-05084** was prepared as a white solid (13 mg, yield 16%). $^1$H NMR (400 MHz, DMSO-d6) δ 10.70 (s, 1H), 10.53 (s, 1H), 7.91 (d, *J* = 8.1 Hz, 1H), 7.86 (s, 1H), 7.77 (d, *J* = 4.9 Hz, 1H), 7.09 (d, *J* = 5.1 Hz, 1H), 6.79 (d, *J* = 5.5 Hz, 1H), 4.82 (d, *J* = 14.9 Hz, 1H), 4.60 (d, *J* = 9.4 Hz, 1H), 4.55 (s, 2H), 3.80 (t, *J* = 6.7 Hz, 2H), 3.24 - 3.15 (m, 4H), 3.03 - 2.95 (m, 3H), 2.88 - 2.84 (m, 2H), 2.82 - 2.75 (m, 2H), 2.08 (s, 3H). LCMS (ESI) calcd for $C_{22}H_{26}N_5O_3S^+$ [M+H]$^+$: 440.18, found, 440.2.

**Example 9: preparation of 1-(1-oxo-5-((4-(thiophen-3-yl)piperazin-1-yl)methyl)isoindolin-2-yl)dihydropyrimidine-2,4(1H,3H)-dione (GT-05085)**

Referring to the method of Scheme 1, the target product **GT-05085** was prepared as a white solid (9 mg, yield 12%). $^1$H NMR (400 MHz, DMSO-d6) δ 10.91 (s, 1H), 10.70 (s, 1H), 7.90 (d, *J* = 8.0 Hz, 1H), 7.89 (s, 1H), 7.79 (d, *J* = 8.0 Hz, 1H), 7.51 - 7.42 (m, 1H), 6.98 (d, *J* = 5.3 Hz, 1H), 6.49 (d, *J* = 1.5 Hz, 1H), 4.82 (d, *J* = 16.6 Hz, 1H), 4.60 (d, *J* = 16.7 Hz, 1H), 4.53 (s, 2H), 3.80 (t, *J* = 6.7 Hz, 2H), 3.67 (d, *J* = 11.8 Hz, 2H), 3.29 - 3.14 (m, 4H), 3.19 - 3.03 (m, 2H), 2.94 - 2.82 (m, 1H), 2.80 - 2.74 (m, 1H). LCMS (ESI) calcd for $C_{21}H_{24}N_5O_3S^+$ [M+H]$^+$: 426.16, found, 426.2.

**Example 10: preparation of 1-(5-((4-(4-methylthiophen-3-yl)piperazin-1-yl)methyl)-1-oxoisoindolin-2-yl)dihydropyrimidine-2,4(1H,3H)-dione (GT-05086)**

Referring to the method of Scheme 1, the target product **GT-05086** was prepared as a white solid (57 mg, yield 70%). $^1$H NMR (400 MHz, DMSO-d6) δ 11.14 (s, 1H), 10.70 (s, 1H), 7.92 (s, 1H), 7.89 (d, *J* = 7.8 Hz, 1H), 7.83 (d, *J* = 7.9 Hz, 1H), 7.13 (d, *J* = 2.9 Hz, 1H), 6.81 (d, *J* = 3.2 Hz, 1H), 4.82 (d, *J* = 16.6 Hz, 1H), 4.59 (d, *J* = 16.9 Hz, 1H), 4.54 (s, 2H), 3.80 (t, *J* = 6.8 Hz, 2H), 3.44 - 3.36 (m, 2H), 3.30 (d, *J* = 13.7 Hz, 2H), 3.24 (d, *J* = 10.5 Hz, 2H), 3.04 - 2.98 (m, 2H), 2.90 - 2.84 (m, 1H), 2.80 - 2.74 (m, 1H), 2.10 (s, 3H). LCMS (ESI) calcd for $C_{22}H_{26}N_5O_3S^+$ [M+H]$^+$: 440.18, found, 440.2.

**Example 11: preparation of 1-(5-((4-(5-methylthiophen-3-yl)piperazin-1-yl)methyl)-1-oxoisoindolin-2-yl)dihydropyrimidine-2,4(1H,3H)-dione (GT-05087)**

Referring to the method of Scheme 1, the target product **GT-05087** was prepared as a white solid (54 mg, yield 67%). $^1$H NMR (400 MHz, DMSO-d6) δ 10.70 (s, 1H), 9.69 (s, 1H), 7.93 (s, 1H), 7.87 (d, *J* = 3.4 Hz, 1H), 7.86 (s, 1H), 7.82 (s, 1H), 6.68 (d, *J* = 3.4 Hz, 1H), 4.81 (d, *J* = 16.8 Hz, 1H), 4.59 (d, *J* = 16.8 Hz, 1H), 4.53 (s, 2H), 3.79 (t, *J* = 6.8 Hz, 2H), 3.64 - 3.61 (m, 2H), 3.31 - 3.20 (m, 4H), 3.25 - 3.03 (m, 2H), 2.92 - 2.85 (m, 1H), 2.78 - 2.74 (m, 1H), 2.37 (s, 3H). LCMS (ESI) calcd

for $C_{22}H_{26}N_5O_3S^+$ [M+H]$^+$: 440.18, found, 440.2.

**Example 12: preparation of 1-(1-oxo-5-((4-(thiophen-2-yl)-3,6-dihydropyridin-1(2H)-yl)methyl)isoindolin-2-yl) dihydropyrimidine-2,4(1H,3H)-dione (GT-04868)**

Referring to the method of Scheme 1, the target product **GT-04868** was prepared as a white solid (18 mg, yield 22%). $^1$H NMR (400 MHz, DMSO) δ 10.98 (brs, 1H), 10.69 (s, 1H), 7.92 - 7.88 (m, 2H), 7.82 - 7.78 (m, 1H), 7.48 (d, J = 4.9 Hz, 1H), 7.17 (s, 1H), 7.10 - 7.02 (m, 1H), 6.05 (s, 1H), 4.85 (d, J = 12.4 Hz, 1H), 4.67 - 4.46 (m, 3H), 3.88 - 3.69 (m, 4H), 3.66 - 3.59 (m, 1H), 2.98 - 2.65 (m, 5H). LCMS (ESI) calcd for $C_{22}H_{23}N_4O_3S^+$ [M+H]$^+$: 423.15, found, 423.2.

**Example 13: preparation of 1-(5-((4-(5-methylthiophen-2-yl)-3,6-dihydropyridin-1(2H)-yl)methyl)-1-oxoisoindolin-2-yl)dihydropyrimidine-2,4(1H,3H)-dione (GT-04869)**

Referring to the method of Scheme 1, the target product **GT-04869** was prepared as a white solid (43 mg, yield 53%). $^1$H NMR (400 MHz, DMSO) δ 10.74 (brs, 1H), 10.69 (s, 1H), 7.89 (d, J = 8.0 Hz, 2H), 7.78 (d, J = 7.2 Hz, 1H), 6.95 (s, 1H), 6.75 (s, 1H), 5.91 (s, 1H), 4.81 (d, J = 17.6 Hz, 1H), 4.60 - 4.49 (m, 3H), 3.83 - 3.78 (m, 4H), 3.66 - 3.57 (m, 2H), 2.79 - 2.72 (m, 4H), 2.41 (s, 3H). LCMS (ESI) calcd for $C_{23}H_{25}N_4O_3S^+$ [M+H]$^+$: 437.16, found, 437.2.

**Example 14: preparation of 1-(5-((4-(3-methylthiophen-2-yl)-3,6-dihydropyridin-1(2H)-yl)methyl)-1-oxoisoindolin-2-yl)dihydropyrimidine-2,4(1H,3H)-dione (GT-04870)**

Referring to the method of Scheme 1, the target product **GT-04870** was prepared as a white solid (38 mg, yield 47%). $^1$H NMR (400 MHz, DMSO) δ 10.89 (brs, 1H), 10.69 (s, 1H), 7.92 - 7.89 (m, 2H), 7.80 (d, J = 7.8 Hz, 1H), 7.39 (d, J = 5.0 Hz, 1H), 6.91 (d, J = 5.1 Hz, 1H), 5.83 (s, 1H), 4.82 (d, J = 17.1 Hz, 1H), 4.61 - 4.50 (m, 3H), 3.90 - 3.71 (m, 4H), 3.63 - 3.58 (m, 2H), 2.91 - 2.71 (m, 3H), 2.65 - 2.60 (m, 1H), 2.24 (s, 3H). LCMS (ESI) calcd for $C_{23}H_{25}N_4O_3S^+$ [M+H]$^+$: 437.16, found, 437.2.

**Example 15: preparation of 1-(1-oxo-5-((4-(thiophen-3-yl)-3,6-dihydropyridin-1(2H)-yl)methyl)isoindolin-2-yl) dihydropyrimidine-2,4(1H,3H)-dione (GT-04871)**

Referring to the method of Scheme 1, the target product **GT-04871** was prepared as a white solid (26 mg, yield 33%). $^1$H NMR (400 MHz, DMSO) δ 10.74 (brs, 1H), 10.69 (s, 1H), 7.92 - 7.88 (m, 2H), 7.82 - 7.78 (m, 1H), 7.61 - 7.51 (m, 2H), 7.37 (d, J = 4.9 Hz, 1H), 6.16 (s, 1H), 4.88 - 4.76 (m, 1H), 4.66 - 4.46 (m, 3H), 3.90 - 3.70 (m, 4H), 3.67 - 3.60 (m, 1H), 3.11 - 3.04(m, 1H), 2.94 - 2.70 (m, 4H). LCMS (ESI) calcd for $C_{22}H_{23}N_4O_3S^+$ [M+H]$^+$: 423.15, found, 423.1.

**Example 16: preparation of 1-(5-((4-(4-methylthiophen-3-yl)-3,6-dihydropyridin-1(2H)-yl)methyl)-1-oxoisoindolin-2-yl)dihydropyrimidine-2,4(1H,3H)-dione (GT-04872)**

Referring to the method of Scheme 1, the target product **GT-04872** was prepared as a white solid (27 mg, yield 34%). $^1$H NMR (400 MHz, DMSO) δ 11.01 (brs, 1H), 10.69 (s, 1H), 7.95 - 7.87 (m, 2H), 7.82 (d, J = 7.9 Hz, 1H), 7.42 (d, J = 3.2 Hz, 1H), 7.20 (d, J = 2.4 Hz, 1H), 5.79 (s, 1H), 4.82 (d, J = 16.6 Hz, 1H), 4.65 - 4.46 (m, 3H), 3.81 - 3.78 (m, 4H), 3.61 - 3.58 (m, 1H), 3.29 - 3.22 (m, 1H), 2.89 - 2.84 (m, 2H), 2.80 - 2.73 (m, 1H), 2.65 - 2.60 (m, 1H), 2.23 (s, 3H). LCMS (ESI) calcd for $C_{23}H_{25}N_4O_3S^+$ [M+H]$^+$: 437.16, found, 437.2.

**Example 17: preparation of 1-(5-((4-(5-methylthiophen-3-yl)-3,6-dihydropyridin-1(2H)-yl)methyl)-1-oxoisoindolin-2-yl)dihydropyrimidine-2,4(1H,3H)-dione (GT-04873)**

Referring to the method of Scheme 1, the target product **GT-04873** was prepared as a white solid (20 mg, yield 25%). $^1$H NMR (400 MHz, DMSO) δ 10.69 (brs, 1H), 10.51 (s, 1H), 7.90 (d, J = 7.9 Hz, 1H), 7.86 (s, 1H), 7.77 (d, J = 7.2 Hz, 1H), 7.28 (s, 1H), 7.05 (s, 1H), 6.06 (s, 1H), 4.86 - 4.75 (m, 1H), 4.61 - 4.49 (m, 3H), 3.81 - 3.75 (m, 4H), 3.61 - 3.58 (m, 1H), 3.25 - 3.20(m, 1H), 2.92 - 2.86 (m, 1H), 2.82 - 2.69 (m, 3H), 2.43 (s, 3H). LCMS (ESI) calcd for $C_{23}H_{25}N_4O_3S^+$ [M+H]$^+$: 437.16, found, 437.2.

**Example 18: preparation of 1-(5-((4-(furan-2-yl)-3,6-dihydropyridin-1(2H)-yl)methyl)-1-oxoisoindolin-2-yl)dihydropyrimidine-2,4(1H,3H)-dione (GT-04874)**

Referring to the method of Scheme 1, the target product **GT-04874** was prepared as a white solid (23 mg, yield 31%). $^1$H NMR (400 MHz, DMSO) δ 11.19 (brs, 1H), 10.69 (s, 1H), 7.93 (s, 1H), 7.88 (d, J = 7.8 Hz, 1H), 7.83 (d, J = 7.9 Hz,

1H), 7.68 (s, 1H), 6.57 - 6.49 (m, 2H), 6.08 (s, 1H), 4.82 (d, J = 16.7 Hz, 1H), 4.62 - 4.47 (m, 3H), 3.81 - 3.78 (m, 4H), 3.63 - 3.61 (m, 1H), 3.25 - 3.21 (m, 1H), 2.91 - 2.63 (m, 4H). LCMS (ESI) calcd for $C_{22}H_{23}N_4O_4^+$ [M+H]$^+$: 407.17, found, 407.2.

**Example 19: preparation of 1-(5-((4-(furan-3-yl)-3,6-dihydropyridin-1(2H)-yl)methyl)-1-oxoisoindolin-2-yl)dihydropyrimidine-2,4(1H,3H)-dione (GT-04875)**

Referring to the method of Scheme 1, the target product GT-04875 was prepared as a white solid (34 mg, yield 45%). $^1$H NMR (400 MHz, DMSO) δ 11.04 (brs, 1H), 10.69 (s, 1H), 7.91 (s, 1H), 7.88 (d, J = 7.8 Hz, 1H), 7.83 - 7.81 (m, 2H), 7.67 (t, J = 1.6 Hz, 1H), 6.75 (s, 1H), 5.98 (s, 1H), 4.82 (d, J = 16.4 Hz, 1H), 4.62 - 4.47 (m, 3H), 3.81 - 3.73 (m, 4H), 3.61 - 3.57 (m, 1H), 3.28 - 3.18 (m, 1H), 2.95 - 2.69 (m, 4H). LCMS (ESI) calcd for $C_{22}H_{23}N_4O_4^+$ [M+H]$^+$: 407.17, found, 407.2.

**Example 20: preparation of 1-(5-((4-benzhydrylpiperazin-1-yl)methyl)-1-oxoisoindolin-2-yl)dihydropyrimidine-2,4(1H,3H)-dione (GT-04889)**

Referring to the method of Scheme 1, the target product **GT-04889** was prepared as a white solid (33 mg, yield 59%). $^1$H NMR (400 MHz, DMSO) δ 10.68 (s, 1H), 7.90 - 7.78 (m, 2H), 7.72 (d, J = 7.7 Hz, 1H), 7.62 - 7.45 (m, 4H), 7.40 - 7.32 (m, 4H), 7.27 - 7.17 (m, 2H), 4.78 (d, J = 16.1 Hz, 1H), 4.55 (d, J = 16.1 Hz, 1H), 4.44 (brs, 2H), 3.78 (t, J = 7.2 Hz, 2H), 3.40 - 3.25 (m, 4H), 3.21 - 3.10 (m, 2H), 2.95 - 2.70 (m, 4H). LCMS (ESI) calcd for $C_{30}H_{32}N_5O_3^+$ [M+H]$^+$: 510.25, found, 510.3.

**Example 21: preparation of 1-(1-oxo-5-((4-(phenyl(pyridin-2-yl)methyl)piperazin-1-yl)methyl)isoindolin-2-yl)dihydropyrimidine-2,4(1H,3H)-dione (GT-04890)**

Referring to the method of Scheme 1, the target product **GT-04890** was prepared as a white solid (24 mg, yield 43%). $^1$H NMR (400 MHz, DMSO) δ 10.69 (s, 1H), 8.68 (d, J = 4.7 Hz, 1H), 8.05 (t, J = 7.4 Hz, 1H), 7.93 - 7.82 (m, 2H), 7.81 - 7.73 (m, 2H), 7.58 (d, J = 7.3 Hz, 2H), 7.55 - 7.49 (m, 1H), 7.40 (t, J = 7.3 Hz, 2H), 7.35 - 7.32 (m, 1H), 5.46 (brs, 1H), 4.80 (d, J = 16.8 Hz, 1H), 4.63 - 4.48 (m, 4H), 3.64 (brs, 7H), 3.11 - 2.94 (m, 4H). LCMS (ESI) calcd for $C_{29}H_{31}N_6O_3^+$ [M+H]$^+$: 511.25, found, 511.3.

**Example 22: preparation of 1-(1-oxo-5-((4-(phenyl(pyridin-4-yl)methyl)piperazin-1-yl)methyl)isoindolin-2-yl)dihydropyrimidine-2,4(1H,3H)-dione (GT-04891)**

Referring to the method of Scheme 1, the target product **GT-04891** was prepared as a white solid (28 mg, yield 50%). $^1$H NMR (400 MHz, DMSO) δ 10.69 (s, 1H), 8.99 (s, 1H), 8.80 (d, J = 5.2 Hz, 1H), 8.58 (d, J = 7.8 Hz, 1H), 7.97 (dd, J = 7.9, 5.6 Hz, 1H), 7.90 (s, 1H), 7.82 (dd, J = 18.9, 7.9 Hz, 2H), 7.49 (brs, 2H), 7.38 (t, J = 7.5 Hz, 2H), 7.30 (t, J = 7.3 Hz, 1H), 5.04 (s, 1H), 4.80 (d, J = 17.1 Hz, 1H), 4.55 (d, J = 17.1 Hz, 2H), 4.47 (s, 2H), 3.81 - 3.75 (m, 3H), 3.31 (brs, 3H), 3.21 - 3.13 (m, 2H), 2.97 - 2.81 (m, 3H). LCMS (ESI) calcd for $C_{29}H_{31}N_6O_3^+$ [M+H]$^+$: 511.25, found, 511.3.

**Example 23: preparation of 1-(5-((4-(di(pyridin-2-yl)methyl)piperazin-1-yl)methyl)-1-oxoisoindolin-2-yl)dihydropyrimidine-2,4(1H,3H)-dione (GT-04892)**

Referring to the method of Scheme 1, the target product **GT-04892** was prepared as a white solid (25 mg, yield 45%). $^1$H NMR (400 MHz, DMSO-d6) δ 10.68 (s, 1H), 8.65 (d, *J* = 4.4 Hz, 1H), 8.13 - 7.98 (m, 3H), 7.86 (d, *J* = 7.7 Hz, 2H), 7.77 (d, *J* = 7.8 Hz, 2H), 7.59 - 7.48 (m, 1H), 5.60 (s, 1H), 4.84 - 4.77 (m, 1H), 4.59 - 4.54 (m, 1H), 4.46 (brs, 2H), 3.82 - 3.76 (m, 3H), 3.43 - 3.24 (m, 4H), 3.02 - 2.81 (m, 4H), 2.79 - 2.76 (m, 1H). LCMS (ESI) calcd for $C_{28}H_{30}N_7O_3^+$ [M+H]$^+$: 512.24, found, 512.3.

**Example 24: preparation of 1-(5-((4-(di(pyridin-3-yl)methyl)piperazin-1-yl)methyl)-1-oxoisoindolin-2-yl)dihydropyrimidine-2,4(1H,3H)-dione (GT-04893)**

Referring to the method of Scheme 1, the target product **GT-04893** was prepared as a white solid (26 mg, yield 47%). $^1$H NMR (400 MHz, DMSO-d6) δ 10.68 (s, 1H), 10.17 (s, 1H), 9.52 (s, 1H), 9.34 (s, 2H), 9.19 (d, *J* = 6.0 Hz, 1H), 8.83 (s, 1H), 8.71 (d, *J* = 4.0 Hz, 1H), 8.65 (d, *J* = 8.1 Hz, 1H), 8.27 - 8.09 (m, 2H), 7.92 - 7.80 (m, 2H), 7.72 (dd, *J* = 13.4, 7.4 Hz, 2H), 6.08 (s, 2H), 5.31 (s, 1H), 4.81 (d, *J* = 15.3 Hz, 1H), 4.54 (d, *J* = 16.9 Hz, 1H), 3.78 (t, *J* = 6.8 Hz, 3H), 3.15 (brs, 4H), 2.94 - 2.81 (m, 1H), 2.77 - 2.73 (m, 1H), 2.59 (brs, 4H). LCMS (ESI) calcd for $C_{28}H_{30}N_7O_3^+$ [M+H]$^+$: 512.24, found, 512.2.

**Example 25: preparation of 1-(5-((4-(bis(4-fluorophenyl)methyl)piperazin-1-yl)methyl)-1-oxoisoindolin-2-yl)dihydropyrimidine-2,4(1H,3H)-dione (GT-04894)**

Referring to the method of Scheme 1, the target product **GT-04894** was prepared as a white solid (34 mg, yield 57%). $^1$H NMR (400 MHz, DMSO) δ 7.14 (d, J = 8.0 Hz, 1H), 6.98 (s, 1H), 6.91 (d, J = 7.4 Hz, 1H), 6.79 - 6.67 (m, 4H), 6.35 - 6.26 (m, 4H), 4.03 - 3.99 (m, 1H), 3.97 - 3.79 (m, 2H), 3.70 (brs, 2H), 3.23 - 2.95 (m, 2H), 2,.77 - 2.63 (m, 4H), 2.25 - 2.08 (m, 3H), 1.88 - 1.42 (m, 2H). LCMS (ESI) calcd for $C_{30}H_{30}F_2N_5O_3^+$ [M+H]$^+$: 546.23, found, 546.3.

**Example 26: preparation of 1-(5-((4-(bis(4-chlorophenyl)methyl)piperazin-1-yl)methyl)-1-oxoisoindolin-2-yl)dihydropyrimidine-2,4(1H,3H)-dione (GT-04895)**

Referring to the method of Scheme 1, the target product **GT-04895** was prepared as a white solid (39 mg, yield 62%). $^1$H NMR (400 MHz, DMSO) δ 7.14 (brs, 1H), 7.00 (brs, 1H), 6.93 (brs, 1H), 6.69 (brs, 4H), 6.57 (brs, 4H), 4.12 (s, 2H), 3.94 - 3.82 (m, 2H), 3.72 - 3.66 (m, 2H), 3.17 - 2.97 (m, 2H), 2.63 (brs, 4H), 2.38 - 2.21 (m, 1H), 2.14 (brs, 3H), 1.98 - 1.58 (m, 2H). LCMS (ESI) calcd for $C_{30}H_{30}Cl_2N_5O_3^+$ [M+H]$^+$: 578.17, found, 578.2.

**Example 27: preparation of 1-(5-(((1S,4S)-5-benzhydryl-2,5-diazabicyclo[2.2.1]heptan-2-yl)methyl)-1-oxoisoindolin-2-yl)dihydropyrimidine-2,4(1H,3H)-dione (GT-04938)**

Referring to the method of Scheme 1, the target product **GT-04938** was prepared as a white solid (40 mg, yield 53%). LCMS (ESI) calcd for $C_{31}H_{32}N_5O_3^+$ [M+H]$^+$: 522.25, found, 522.3.

**Example 28: preparation of 1-(5-((4-(diphenylamino)piperidin-1-yl)methyl)-1-oxoisoindolin-2-yl)dihydropyrimidine-2,4(1H,3H)-dione (GT-04939)**

Referring to the method of Scheme 1, the target product **GT-04939** was prepared as a white solid (28 mg, yield 38%). LCMS (ESI) calcd for $C_{30}H_{32}N_5O_3^+$ [M+H]$^+$: 510.25, found, 510.3.

**Example 29: preparation of 1-(1-oxo-5-((4-(phenyl(pyridin-2-yl)amino)piperidin-1-yl)methyl)isoindolin-2-yl)dihydropyrimidine-2,4(1H,3H)-dione (GT-04940)**

Referring to the method of Scheme 1, the target product **GT-04940** was prepared as a white solid (24 mg, yield 32%). LCMS (ESI) calcd for $C_{29}H_{31}N_6O_3^+$ [M+H]$^+$: 511.25, found, 511.2.

**Example 30: preparation of 1-(5-((4-((4'-chloro-[1,1'-biphenyl]-2-yl)amino)piperidin-1-yl)methyl)-1-oxoisoindolin-2-yl)dihydropyrimidine-2,4(1H,3H)-dione (GT-04943)**

Referring to the method of Scheme 1, the target product **GT-04943** was prepared as a white solid (29 mg, yield 37%). LCMS (ESI) calcd for $C_{30}H_{31}ClN_5O_3^+$ [M+H]$^+$: 544.21, found, 544.2.

**Example 31: preparation of 1-(5-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)dihydropyrimidine-2,4(1H,3H)-dione (GT-04944)**

Referring to the method of Scheme 1, the target product **GT-04944** was prepared as a white solid (59 mg, yield 71%). LCMS (ESI) calcd for $C_{32}H_{39}ClN_5O_3^+$ [M+H]$^+$: 576.27, found, 576.3.

**Example 32: preparation of 5-((4-(2,3-dichlorophenyl)piperazin-1-yl)methyl)-2-(2,4-dioxotetrahydropyrimidin-1(2H)-yl)isoindoline-1,3-dione (GT-04854)**

Referring to the method of Scheme 1, the target product **GT-04854** was prepared as a white solid (23 mg, yield 38%). $^1$H NMR (400 MHz, MeOD) δ 8.20 (s, 1H), 8.11 (s, 1H), 7.83 (d, J = 8.1 Hz, 1H), 7.35 - 7.30 (m, 2H), 7.18 - 7.16 (m, 1H), 4.59 (d, J = 11.7 Hz, 3H), 3.94 - 3.85 (m, 3H), 3.57 - 3.45 (m, 6H), 2.99 (t, J = 6.8 Hz, 2H). LCMS (ESI) calcd for $C_{23}H_{22}Cl_2N_5O_4^+$ [M+H]$^+$: 502.10, found, 502.1.

**Example 33: preparation of 1-(1-oxo-5-((4-(pyridin-2-yl)piperazin-1-yl)methyl)isoindolin-2-yl)dihydropyrimidine-2,4(1H,3H)-dione (GT-05124)**

Referring to the method of Scheme 1, the target product **GT-05124** was prepared as a white solid (51 mg, yield

66%). $^1$HNMR(400 MHz, DMSO-d6) δ 12.15 (s, 1H), 10.71 (s, 1H), 8.14 (d, *J* = 5.0, 1.3 Hz, 1H), 7.97 (s, 1H), 7.91 - 7.79 (m, 2H), 7.74 - 7.60 (m, 1H), 7.02 (d, *J* = 8.2 Hz, 1H), 6.80 (t, 1H), 4.82 (d, *J* = 16.7 Hz, 1H), 4.61 - 4.47 (m, 3H), 4.40 (d, *J* = 12.7 Hz, 2H), 3.80 (t, *J* = 6.8 Hz, 2H), 3.40 - 3.27 (m, 4H), 3.14 (s, 2H), 2.91 - 2.83 (m, 1H), 2.79 - 2.69 (m, 1H). LCMS (ESI) calcd for $C_{22}H_{25}N_6O_3^+$ $[M+H]^+$: 421.20, found, 421.2.

**Example 34: preparation of 1-(5-((4-(6-methylpyridin-2-yl)piperazin-1-yl)methyl)-1-oxoisoindolin-2-yl)dihydropyrimidine-2,4(1H,3H)-dione (GT-05125)**

Referring to the method of Scheme 1, the target product **GT-05125** was prepared as a white solid (35 mg, yield 46%). $^1$H NMR (400 MHz, DMSO) δ 11.45 (s, 1H), 10.69 (s, 1H), 7.89 (d, *J* = 8.4 Hz, 2H), 7.80 (d, *J* = 8.0 Hz, 1H), 7.73 - 7.61 (m, 1H), 6.91 (d, *J* = 7.8 Hz, 1H), 6.74 (d, *J* = 7.2 Hz, 1H), 4.82 (d, *J* = 16.7 Hz, 1H), 4.59 (d, *J* = 16.6 Hz, 1H), 4.51 (s, 2H), 4.46 - 4.26 (m, 2H), 3.80 (t, *J* = 6.9 Hz, 3H), 3.40 - 3.37 (m, 2H), 3.15 (s, 3H), 2.93 - 2.83 (m, 1H), 2.81 - 2.73 (m, 1H), 2.43 (s, 3H). LCMS (ESI) calcd for $C_{23}H_{27}N_6O_3^+$ $[M+H]^+$: 435.21, found, 435.3.

**Example 35: preparation of 1-(5-((4-(6-chloropyridin-2-yl)piperazin-1-yl)methyl)-1-oxoisoindolin-2-yl)dihydropyrimidine-2,4(1H,3H)-dione (GT-05126)**

Referring to the method of Scheme 1, the target product **GT-05126** was prepared as a white solid (59 mg, yield 74%). $^1$H NMR (400 MHz, DMSO) δ 11.22 (s, 1H), 10.69 (s, 1H), 7.89 (d, *J* = 7.4 Hz, 2H), 7.78 (d, *J* = 8.2 Hz, 1H), 7.65 (t, *J* = 8.0 Hz, 1H), 6.89 (d, *J* = 8.4 Hz, 1H), 6.79 (d, *J* = 7.5 Hz, 1H), 4.82 (d, *J* = 16.5 Hz, 1H), 4.59 (d, *J* = 16.7 Hz, 1H), 4.50 (s, 2H), 4.34 (d, *J* = 13.5 Hz, 2H), 3.80 (t, *J* = 6.9 Hz, 2H), 3.39 (s, 2H), 3.35 - 3.31 (m, 2H), 3.12 (s, 2H), 2.95 - 2.82 (m, 1H), 2.82 - 2.70 (m, 1H). LCMS (ESI) calcd for $C_{22}H_{24}ClN_6O_3^+$ $[M+H]^+$: 455.16, found, 455.1.

**Example 36: preparation of 1-(5-((4-(5-chloropyridin-2-yl)piperazin-1-yl)methyl)-1-oxoisoindolin-2-yl)dihydropyrimidine-2,4(1H,3H)-dione (GT-05127)**

Referring to the method of Scheme 1, the target product **GT-05127** was prepared as a white solid (66 mg, yield 83%). $^1$H NMR (400 MHz, DMSO) δ 11.18 (s, 1H), 10.69 (s, 1H), 8.18 (d, *J* = 2.6 Hz, 1H), 7.89 (d, *J* = 7.3 Hz, 2H), 7.78 (d, *J* = 8.0 Hz, 1H), 7.71 (dd, *J* = 9.1, 2.7 Hz, 1H), 6.98 (d, *J* = 9.1 Hz, 1H), 4.82 (d, *J* = 17.1 Hz, 1H), 4.59 (d, *J* = 17.0 Hz, 1H), 4.50 (s, 2H), 4.35 (d, *J* = 13.7 Hz, 2H), 3.88 - 3.73 (m, 2H), 3.44 (s, 2H), 3.35 - 3.27 (m, 2H), 3.12 (s, 2H), 2.94 - 2.82 (m, 1H), 2.82 - 2.70 (m, 1H). LCMS (ESI) calcd for $C_{22}H_{24}ClN_6O_3^+$ $[M+H]^+$: 455.16, found, 455.2.

**Example 37: preparation of 1-(5-((4-(4-chloropyridin-2-yl)piperazin-1-yl)methyl)-1-oxoisoindolin-2-yl)dihydropyrimidine-2,4(1H,3H)-dione (GT-05128)**

Referring to the method of Scheme 1, the target product **GT-05128** was prepared as a white solid (54 mg, yield 68%). $^1$H NMR (400 MHz, DMSO) δ 11.35 (s, 1H), 10.69 (s, 1H), 8.13 (d, *J* = 5.4 Hz, 1H), 7.88 (d, *J* = 8.0 Hz, 2H), 7.79 (d, *J* = 8.2 Hz, 1H), 7.09 (s, 1H), 6.84 (dd, *J* = *5.4,* 1.5 Hz, 1H), 4.82 (d, *J* = 16.7 Hz, 1H), 4.59 (d, *J* = 16.6 Hz, 1H), 4.50 (s, 2H), 4.43 (d, *J* = 13.3 Hz, 2H), 3.80 (t, *J* = 6.8 Hz, 2H), 3.42 - 3.28 (m, 4H), 3.10 (s, 2H), 2.96 - 2.81 (m, 1H), 2.82 - 2.68 (m, 1H). LCMS (ESI) calcd for $C_{22}H_{24}ClN_6O_3^+$ $[M+H]^+$: 455.16, found, 455.1.

**Example 38: preparation of 1-(5-((4-(3-fluoropyridin-2-yl)piperazin-1-yl)methyl)-1-oxoisoindolin-2-yl)dihydropyrimidine-2,4(1H,3H)-dione (GT-05129)**

Referring to the method of Scheme 1, the target product **GT-05129** was prepared as a white solid (40 mg, yield 52%). $^1$H NMR (400 MHz, DMSO) δ 11.58 (s, 1H), 10.69 (s, 1H), 8.07 (d, *J* = 4.8 Hz, 1H), 7.93 (s, 1H), 7.85 (dd, *J* = 18.5, 7.9 Hz, 2H), 7.60 (ddd, *J* = 13.5, 7.9, 1.3 Hz, 1H), 7.04 - 6.94 (m, 1H), 4.82 (d, *J* = 16.5 Hz, 1H), 4.58 (d, *J* = 16.7 Hz, 1H), 4.51 (s, 2H), 4.04 (d, *J* = 13.4 Hz, 2H), 3.80 (t, *J* = 6.8 Hz, 2H), 3.50 - 3.34 (m, 4H), 3.22 (s, 2H), 2.92 - 2.81 (m, 1H), 2.80 - 2.68 (m, 1H). LCMS (ESI) calcd for $C_{22}H_{24}FN_6O_3^+$ $[M+H]^+$: 439.19, found, 439.3.

**Example 39: preparation of 1-(5-((4-(6-fluoropyridin-2-yl)piperazin-1-yl)methyl)-1-oxoisoindolin-2-yl)dihydropyrimidine-2,4(1H,3H)-dione (GT-05130)**

Referring to the method of Scheme 1, the target product **GT-05130** was prepared as a white solid (45 mg, yield 58%). $^1$H NMR (400 MHz, DMSO) δ 11.18 (s, 1H), 10.69 (s, 1H), 7.89 (d, *J* = 7.9 Hz, 2H), 7.83 - 7.65 (m, 2H), 6.79 (dd, *J* = 8.2, 2.1 Hz, 1H), 6.40 (dd, *J* = 7.8, 2.4 Hz, 1H), 4.82 (d, *J* = 16.4 Hz, 1H), 4.59 (d, *J* = 16.6 Hz, 1H), 4.50 (s, 2H), 4.32 (d, *J* = 11.3 Hz, 2H), 3.80 (t, *J* = 6.8 Hz, 2H), 3.40 (s, 2H), 3.33 - 3.25 (m, 2H), 3.12 (s, 2H), 2.94 - 2.82 (m, 1H), 2.81 - 2.67 (m, 1H).LCMS (ESI) calcd for $C_{22}H_{24}FN_6O_3^+$ $[M+H]^+$: 439.19, found, 439.3.

**Example 40: preparation of 1-(5-((4-(5-fluoropyridin-2-yl)piperazin-1-yl)methyl)-1-oxoisoindolin-2-yl)dihydropyrimidine-2,4(1H,3H)-dione (GT-05131)**

Referring to the method of Scheme 1, the target product **GT-05131** was prepared as a white solid (45 mg, yield 56%). $^1$H NMR (400 MHz, DMSO) δ 11.40 (s, 1H), 10.69 (s, 1H), 8.15 (d, *J* = 3.0 Hz, 1H), 7.95 - 7.84 (m, 2H), 7.81 (d, *J* = 7.9 Hz, 1H), 7.64 - 7.55 (m, 1H), 6.99 (dd, *J* = 9.3, 3.4 Hz, 1H), 4.81 (d, *J* = 16.7 Hz, 1H), 4.58 (d, *J* = 16.6 Hz, 1H), 4.50 (s, 2H), 4.28 (d, *J* = 13.2 Hz, 2H), 3.79 (t, *J* = 6.8 Hz, 2H), 3.39 (d, *J* = 10.1 Hz, 2H), 3.31 (t, *J* = 12.9 Hz, 2H), 3.15 (d, *J* = 19.1 Hz, 2H), 2.93 - 2.82 (m, 1H), 2.82 - 2.69 (m, 1H). LCMS (ESI) calcd for $C_{22}H_{24}FN_6O_3^+$ [M+H]$^+$: 439.19, found, 439.2.

**Example 41: preparation of 1-(1-oxo-5-((4-(3-(trifluoromethyl)pyridin-2-yl)piperazin-1-yl)methyl)isoindolin-2-yl) dihydropyrimidine-2,4(1H,3H)-dione (GT-05174)**

Referring to the method of Scheme 1, the target product **GT-05174** was prepared as a white solid (19 mg, yield 27%). $^1$H NMR (400 MHz, DMSO) δ 11.02 (s, 1H), 10.70 (s, 1H), 8.60 (d, *J* = 3.7 Hz, 1H), 8.15 (dd, *J* = 7.8, 1.6 Hz, 1H), 7.93 - 7.85 (m, 2H), 7.82 (d, *J* = 7.9 Hz, 1H), 7.33 (dd, *J* = 7.6, 4.9 Hz, 1H), 4.82 (d, *J* = 16.6 Hz, 1H), 4.59 (d, *J* = 22.8 Hz, 3H), 3.80 (t, *J* = 6.8 Hz, 2H), 3.51 (s, 3H), 3.36 (d, *J* = 5.0 Hz, 3H), 3.23 (s, 2H), 2.94 - 2.83 (m, 1H), 2.81 - 2.72 (m, 1H). LCMS (ESI) calcd for $C_{23}H_{24}F_3N_6O_3^+$ [M+H]$^+$: 489.19, found, 489.2.

**Example 42: preparation of 1-(1-oxo-5-((4-(6-(trifluoromethyl)pyridin-2-yl)piperazin-1-yl)methyl)isoindolin-2-yl) dihydropyrimidine-2,4(1H,3H)-dione (GT-05175)**

Referring to the method of Scheme 1, the target product **GT-05175** was prepared as a white solid (25 mg, yield 35%). $^1$H NMR (400 MHz, DMSO) δ 11.16 (s, 1H), 10.69 (s, 1H), 7.94 - 7.79 (m, 3H), 7.77 (d, *J* = 7.6 Hz, 1H), 7.19 (dd, *J* = 25.0, 8.0 Hz, 2H), 4.81 (d, *J* = 16.5 Hz, 1H), 4.59 (d, *J* = 17.0 Hz, 1H), 4.53 - 4.39 (m, 3H), 3.79 (t, *J* = 6.9 Hz, 2H), 3.47 - 3.38 (m, 3H), 3.14 (s, 1H), 2.90 - 2.82 (m, 1H), 2.81 - 2.71 (m, 1H). LCMS (ESI) calcd for $C_{23}H_{24}F_3N_6O_3^+$ [M+H]$^+$: 489.19, found, 489.3.

**Example 43: preparation of 1-(1-oxo-5-((4-(5-(trifluoromethyl)pyridin-2-yl)piperazin-1-yl)methyl)isoindolin-2-yl) dihydropyrimidine-2,4(1H,3H)-dione (GT-05176)**

Referring to the method of Scheme 1, the target product **GT-05176** was prepared as a white solid (25 mg, yield 35%). $^1$H NMR (400 MHz, DMSO) δ 11.54 (s, 1H), 10.69 (s, 1H), 8.48 (s, 1H), 7.96 - 7.85 (m, 3H), 7.79 (d, *J* = 7.9 Hz, 1H), 7.07 (d, *J* = 9.0 Hz, 1H), 4.81 (d, *J* = 16.6 Hz, 1H), 4.57 (d, *J* = 21.4 Hz, 2H), 4.49 (s, 3H), 3.79 (t, *J* = 6.8 Hz, 2H), 3.49 - 3.34 (m, 4H), 3.12 (s, 2H), 2.92 - 2.82 (m, 1H), 2.81 - 2.71 (m, 1H). LCMS (ESI) calcd for $C_{23}H_{24}F_3N_6O_3^+$ [M+H]$^+$: 489.19, found, 489.3.

**Example 44: preparation of 5-((4-(2-((2,4-dimethylphenyl)thio)phenyl)piperazin-1-yl)methyl)-2-(2,4-dioxotetrahydropyrimidin-1(2H)-yl)isoindoline-1,3-dione (GT-05161)**

Referring to the method of Scheme 1, the target product was prepared as a white solid GT-05161(40 mg, yield 46%). $^1$H NMR (400 MHz, DMSO-d6) δ 11.07 (s, 1H), 10.94 (s, 1H), 8.33 (s, 1H), 8.20 (d, *J* = 7.2 Hz, 1H), 8.13 (d, *J* = 7.7 Hz, 1H), 7.33 (d, *J* = 7.8 Hz, 1H), 7.24 (s, 1H), 7.16 (t, *J* = 5.8 Hz, 2H), 7.11 (d, *J* = 7.9 Hz, 1H), 7.00 - 6.96 (m, 1H), 6.41 (d, *J* = 7.8 Hz, 1H), 4.66 (brs, 2H), 3.83 (t, *J* = 6.7 Hz, 2H), 3.58 - 3.44 (m, 4H), 3.27 - 3.14 (m, 4H), 2.87 (t, *J* = 6.7 Hz, 2H), 2.33 (s, 3H), 2.23 (s, 3H). LCMS (ESI) calcd for $C_{31}H_{32}N_5O_4S^+$ [M+H]$^+$: 570.22, found, 570.3.

**Example 45: preparation of 1-(5-((3-(2,3-dichlorophenyl)-3,6-diazabicyclo[3.1.1]heptan-6-yl)methyl)-1-oxoisoindolin-2-yl)dihydropyrimidine-2,4(1H,3H)-dione (GT-05162)**

Referring to the method of Scheme 1, the target product GT-05162 was prepared as a white solid (37 mg, yield 47%). $^1$H NMR (400 MHz, DMSO-d6) δ 10.67 (s, 1H), 7.74 (s, 1H), 7.68 (s, 1H), 7.59 (s, 1H), 7.39 - 7.28 (m, 2H), 7.24 - 7.12 (m, 1H), 4.82 - 4.64 (m, 2H), 4.53 (d, *J* = 15.6 Hz, 1H), 4.19 - 3.94 (m, 1H), 3.86 - 3.70 (m, 3H), 3.34 - 3.28 (m, 3H), 3.21 - 3.13 (m, 1H), 2.96 - 2.80 (m, 2H), 2.78 - 2.73 (m, 1H), 2.29 - 1.97 (m, 2H). LCMS (ESI) calcd for $C_{24}H_{24}Cl_2N_5O_3^+$ [M+H]$^+$: 500.13, found, 500.1.

**Example 46: preparation of 1-(5-(((1S,4S)-5-(2,3-dichlorophenyl)-2,5-diazabicyclo[2.2.1]heptan-2-yl)methyl)-1-oxoisoindolin-2-yl)dihydropyrimidine-2,4(1H,3H)-dione (GT-05163)**

Referring to the method of Scheme 1, the target product GT-05163 was prepared as a white solid (39 mg, yield

49%). $^1$H NMR (400 MHz, DMSO-d6) δ 10.62 (s, 1H), 10.19 (s, 1H), 7.86 (s, 1H), 7.81 (d, *J* = 7.8 Hz, 1H), 7.75 (d, *J* = 8.0 Hz, 1H), 7.10 (d, *J* = 8.0 Hz, 1H), 7.04 (d, *J* = 7.6 Hz, 1H), 6.91 (d, *J* = 8.1 Hz, 1H), 4.73 (d, *J* = 15.9 Hz, 1H), 4.63 (d, *J* = 12.4 Hz, 1H), 4.50 (d, *J* = 20.9 Hz, 2H), 4.45 - 4.35 (m, 1H), 4.32 (s, 1H), 3.86 (d, *J* = 11.2 Hz, 1H), 3.72 (t, *J* = 6.9 Hz, 2H), 3.66 (d, *J* = 11.1 Hz, 1H), 3.59 - 3.51 (m, 1H), 3.18 (d, *J* = 12.4 Hz, 1H), 2.88 - 2.75 (m, 1H), 2.72 - 2.67 (m, 1H), 2.51 (d, *J* = 11.0 Hz, 1H), 2.10 (d, *J* = 11.6 Hz, 1H). LCMS (ESI) calcd for $C_{24}H_{24}Cl_2N_5O_3^+$ [M+H]$^+$: 500.13, found, 500.1.

**Example 47: preparation of 1-(5-((4-(6-fluoro-1H-indol-3-yl)piperidin-1-yl)methyl)-1-oxoisoindolin-2-yl)dihydropyrimidine-2,4(1H,3H)-dione (GT-05164)**

Referring to the method of Scheme 1, the target product GT-05164 was prepared as a white solid (33 mg, yield 644%). $^1$H NMR (400 MHz, DMSO-d6) δ 10.96 (s, 1H), 10.70 (s, 1H), 10.39 (s, 1H), 7.96 - 7.85 (m, 2H), 7.80 (d, *J* = 7.9 Hz, 1H), 7.65 (dd, *J* = 8.7, 5.5 Hz, 1H), 7.14 - 7.11 (m, 2H), 6.87 - 2.82 (m, 1H), 4.84 (d, *J* = 16.2 Hz, 1H), 4.61 (d, *J* = 16.4 Hz, 1H), 4.50 (d, *J* = 4.3 Hz, 2H), 3.80 (t, *J* = 6.6 Hz, 2H), 3.55 - 3.46 (m, 2H), 3.22 - 3.09 (m, 2H), 3.09 - 2.97 (m, 1H), 2.96 - 2.83 (m, 1H), 2.79 (t, *J* = 5.6 Hz, 1H), 2.18 - 2.02 (m, 4H). LCMS (ESI) calcd for $C_{26}H_{27}FN_5O_3^+$ [M+H]$^+$: 476.21, found, 476.2.

**Example 48: preparation of 1-(5-((4-(5-fluoroindolin-1-yl)piperidin-1-yl)methyl)-1-oxoisoindolin-2-yl)dihydropyrimidine-2,4(1H,3H)-dione (GT-05165)**

Referring to the method of Scheme 1, the target product GT-05165 was prepared as a white solid (11 mg, yield 14%). $^1$H NMR (400 MHz, DMSO-d6) δ 10.70 (s, 1H), 10.47 (s, 1H), 7.91 - 7.85 (m, 2H), 7.76 (d, *J* = 7.8 Hz, 1H), 6.91 (dd, *J* = 8.5, 2.5 Hz, 1H), 6.80 (td, *J* = 9.0, 2.3 Hz, 1H), 6.48 (dd, *J* = 8.6, 4.2 Hz, 1H), 4.82 (d, *J* = 16.9 Hz, 1H), 4.59 (d, *J* = 16.5 Hz, 1H), 4.44 (d, *J* = 4.0 Hz, 2H), 3.80 (t, *J* = 6.9 Hz, 2H), 3.67 - 3.63 (m, 1H), 3.52 - 3.48 (m, 2H), 3.30 (t, *J* = 8.4 Hz, 3H), 3.13 - 3.00 (m, 2H), 2.88 (t, *J* = 8.1 Hz, 2H), 2.79 (t, *J* = 5.8 Hz, 1H), 2.03 - 1.95 (m, 2H), 1.84 (d, *J* = 12.8 Hz, 2H). LCMS (ESI) calcd for $C_{26}H_{29}FN_5O_3^+$ [M+H]$^+$: 478.22, found, 478.2.

**Example 49: preparation of 1-(5-((4-(7-fluoroquinolin-4-yl)-3,6-dihydropyridin-1(2H)-yl)methyl)-1-oxoisoindolin-2-yl)dihydropyrimidine-2,4(1H,3H)-dione (GT-05166)**

Referring to the method of Scheme 1, the target product GT-05166 was prepared as a white solid (20 mg, yield 26%). $^1$H NMR (400 MHz, DMSO-d6) δ 11.63 (s, 1H), 10.71 (s, 1H), 9.04 (d, *J* = 4.8 Hz, 1H), 8.44 (dd, *J* = 9.3, 6.2 Hz, 1H), 8.00 (s, 1H), 7.95 - 7.87 (m, 3H), 7.71 - 7.61 (m, 1H), 7.54 (d, *J* = 4.8 Hz, 1H), 5.91 (s, 1H), 4.85 (d, *J* = 16.8 Hz, 1H), 4.73 - 4.55 (m, 3H), 3.88 (brs, 2H), 3.81 (t, *J* = 6.8 Hz, 2H), 3.76 - 3.67 (m, 2H), 3.13 - 2.99 (m, 1H), 2.92 - 2.75 (m, 2H), 2.75 - 2.62 (m, 1H). LCMS (ESI) calcd for $C_{27}H_{25}FN_5O_3^+$ [M+H]$^+$: 486.19, found, 486.2.

**Example 50: preparation of 1-(5-((4-(6-fluoro-1-methyl-1H-indol-3-yl)-3,6-dihydropyridin-1(2H)-yl)methyl)-1-oxoisoindolin-2-yl)dihydropyrimidine-2,4(1H,3H)-dione (GT-05167)**

Referring to the method of Scheme 1, the target product GT-05167 was prepared as a white solid (30 mg, yield 39%). $^1$H NMR (400 MHz, DMSO-d6) δ 10.71 (s, 1H), 10.58 (s, 1H), 7.97 - 7.88 (m, 2H), 7.87 - 7.75 (m, 2H), 7.54 (s, 1H), 7.36 (dd, *J* = 10.1, 2.2 Hz, 1H), 6.97 (t, *J* = 8.2 Hz, 1H), 6.14 (s, 1H), 4.83 (d, *J* = 15.4 Hz, 1H), 4.68 - 4.53 (m, 2H), 3.88 - 3.77 (m, 4H), 3.75 (s, 3H), 3.68 - 3.64 (m, 1H), 3.23 - 3.16 (m, 2H), 2.90 - 2.76 (m, 4H). LCMS (ESI) calcd for $C_{27}H_{27}FN_5O_3^+$ [M+H]$^+$: 488.21, found, 488.2.

**Example 51: preparation of 1-(5-((3-(7-fluorochroman-4-ylidene)azetidin-1-yl)methyl)-1-oxoisoindolin-2-yl)dihydropyrimidine-2,4(1H,3H)-dione (GT-05168)**

Referring to the method of Scheme 1, the target product GT-05168 was prepared as a white solid (13 mg, yield 18%). $^1$H NMR (400 MHz, DMSO-d6) δ 11.53 (s, 1H), 10.70 (s, 1H), 7.91 - 7.85 (m, 2H), 7.78 (d, *J* = 8.1 Hz, 1H), 7.06 - 7.02 (m, 1H), 6.82 - 6.73 (m, 2H), 5.10 (s, 2H), 5.02 - 4.77 (m, 3H), 4.68 (d, *J* = 4.3 Hz, 2H), 4.60 - 4.56 (m, 1H), 4.25 - 4.13 (m, 2H), 3.80 (t, *J* = 6.8 Hz, 2H), 2.95 - 2.83 (m, 1H), 2.80 - 2.75 (m, 1H), 2.48 - 2.42 (m, 2H). LCMS (ESI) calcd for $C_{25}H_{24}FN_4O_4^+$ [M+H]$^+$: 463.18, found, 463.2.

**Example 52: preparation of 1-(5-((3-(5-fluoroindolin-1-yl)azetidin-1-yl)methyl)-1-oxoisoindolin-2-yl)dihydropyrimidine-2,4(1H,3H)-dione (GT-05169)**

Referring to the method of Scheme 1, the target product GT-05169 was prepared as a white solid (14 mg, yield 19%). $^1$H NMR (400 MHz, DMSO-d6) δ 11.66 (s, 1H), 10.70 (s, 1H), 7.88 (s, 1H), 7.85 (d, *J* = 8.0 Hz, 1H), 7.77 (d, *J* = 7.9 Hz, 1H), 6.97 (d, *J* = 8.5 Hz, 1H), 6.84 - 6.78 (m, 1H), 6.47 - 6.37 (m, 1H), 4.81 (d, *J* = 16.6 Hz, 1H), 4.65 - 4.55 (m, 4H), 4.35 - 4.21

(m, 4H), 3.79 (t, $J$ = 6.8 Hz, 2H), 3.58 - 3.48 (m, 2H), 2.93 (t, $J$ = 8.0 Hz, 2H), 2.88 - 2.76 (m, 1H). LCMS (ESI) calcd for $C_{24}H_{25}FN_5O_3^+$ [M+H]$^+$: 450.19, found, 450.2.

**Example 53: preparation of 2-(2,4-dioxotetrahydropyrimidin-1(2H)-yl)-5-((4-(6-fluorobenzo[d]isoxazol-3-yl)piperidin-1-yl)methyl)isoindoline-1,3-dione (GT-05170)**

Referring to the method of Scheme 1, the target product GT-05170 was prepared as a white solid (26 mg, yield 35%). $^1$H NMR (400 MHz, DMSO-d6) δ 10.99 (s, 1H), 10.94 (s, 1H), 8.33 (s, 1H), 8.20 (d, $J$ = 7.9 Hz, 1H), 8.15 (t, $J$ = 6.8 Hz, 2H), 7.73 (dd, $J$ = 9.0, 1.8 Hz, 1H), 7.35 (td, $J$ = 9.1, 2.0 Hz, 1H), 4.61 (s, 2H), 3.84 (t, $J$ = 6.7 Hz, 2H), 3.64 - 3.52 (m, 2H), 3.26 - 3.10 (m, 3H), 2.87 (t, $J$ = 6.7 Hz, 2H), 2.35 - 2.18 (m, 4H). LCMS (ESI) calcd for $C_{25}H_{23}FN_5O_5^+$ [M+H]$^+$: 492.17, found, 492.2.

**Example 54: preparation of 5-((4-(benzo[d]isothiazol-3-yl)piperazin-1-yl)methyl)-2-(2,4-dioxotetrahydropyrimidin-1(2H)-yl)isoindoline-1,3-dione (GT-05171)**

Referring to the method of Scheme 1, the target product GT-05171 was prepared as a white solid (18 mg, yield 24%). $^1$H NMR (400 MHz, DMSO-d6) δ 11.10 (s, 1H), 10.94 (s, 1H), 8.31 (s, 1H), 8.18 - 8.09 (m, 4H), 7.60 (t, $J$ = 7.6 Hz, 1H), 7.47 (t, $J$ = 7.4 Hz, 1H), 4.67 (s, 2H), 4.10 (s, 2H), 3.84 (t, $J$ = 6.8 Hz, 2H), 3.57 - 3.44 (m, 6H), 2.87 (t, $J$ = 6.8 Hz, 2H). LCMS (ESI) calcd for $C_{24}H_{23}N_6O_4S^+$ [M+H]$^+$: 491.15, found, 491.2.

**Example 55: preparation of 5-((4-(benzo[b]thiophen-4-yl)piperazin-1-yl)methyl)-2-(2,4-dioxotetrahydropyrimidin-1(2H)-yl)isoindoline-1,3-dione (GT-05172)**

Referring to the method of Scheme 1, the target product GT-05172 was prepared as a white solid (37 mg, yield 50%). $^1$H NMR (400 MHz, DMSO-d6) δ 11.31 (s, 1H), 10.94 (s, 1H), 8.36 (s, 1H), 8.23 (d, $J$ = 7.9 Hz, 1H), 8.15 (d, $J$ = 7.7 Hz, 1H), 7.76 (d, $J$ = 5.5 Hz, 1H), 7.71 (d, $J$ = 8.1 Hz, 1H), 7.50 (d, $J$ = 5.6 Hz, 1H), 7.32 *(t,* $J$ = 7.9 Hz, 1H), 6.96 (d, $J$ = 7.6 Hz, 1H), 4.68 (s, 2H), 3.84 (t, $J$ = 6.8 Hz, 2H), 3.63 - 3.54 (m, 6H), 3.27 - 3.17 (m, 2H), 2.87 (t, $J$ = 6.8 Hz, 2H). LCMS (ESI) calcd for $C_{25}H_{24}N_5O_4S^+$ [M+H]$^+$: 490.15, found, 490.1.

**Example 56: preparation of 2-(2,4-dioxotetrahydropyrimidin-1(2H)-yl)-5-((4-(phenyl(pyridin-2-yl)methyl)piperazin-1-yl)methyl)isoindoline-1,3-dione (GT-05173)**

Referring to the method of Scheme 1, the target product GT-05173 was prepared as a white solid (33 mg, yield 41%). $^1$H NMR (400 MHz, DMSO-d6) δ 10.92 (s, 1H), 8.67 (d, $J$ = 4.3 Hz, 1H), 8.11 - 7.98 (m, 2H), 7.72 (d, $J$ = 3.5 Hz, 1H), 7.58 - 7.50 (m, 4H), 7.42 - 7.34 (m, 4H), 5.38 (s, 1H), 4.47 - 4.42 (m, 2H), 3.82 (t, $J$ = 6.8 Hz, 2H), 3.27 (s, 4H), 2.96 (s, 4H), 2.86 (t, $J$ = 6.8 Hz, 2H). LCMS (ESI) calcd for $C_{29}H_{29}N_6O_4^+$ [M+H]$^+$: 525.22, found, 525.2.

**Example 57: preparation of 2-(2,4-dioxotetrahydropyrimidin-1(2H)-yl)-5-((4-(diphenylamino)piperidin-1-yl)methyl)isoindoline-1,3-dione (GT-05177)**

Referring to the method of Scheme 1, the target product GT-05177 was prepared as a white solid (19 mg, yield 24%). $^1$H NMR (400 MHz, DMSO-d6) δ 10.93 (s, 1H), 9.82 (s, 1H), 8.19 (s, 1H), 8.11 (d, $J$ = 7.8 Hz, 1H), 8.06 (d, $J$ = 7.6 Hz, 1H), 7.32 - 7.21 (m, 4H), 7.02 (t, $J$ = 7.3 Hz, 2H), 6.82 (d, $J$ = 8.1 Hz, 4H), 4.49 (s, 2H), 4.25 - 4.21 (m, 1H), 3.87 - 3.76 (m, 2H), 3.25 - 3.17 (m, 4H), 2.88 - 2.83 (m, 2H), 2.10 (d, $J$= 13.9 Hz, 2H), 1.68 - 1.54 (m, 2H). LCMS (ESI) calcd for $C_{30}H_{30}N_5O_4^+$ [M+H]$^+$: 524.23, found, 524.2.

**Example 58: preparation of 2-(2,4-dioxotetrahydropyrimidin-1(2H)-yl)-5-((4-(phenyl(pyridin-2-yl)amino)piperidin-1-yl)methyl)isoindoline-1,3-dione (GT-05178)**

Referring to the method of Scheme 1, the target product GT-05178 was prepared as a white solid (42 mg, yield 53%). $^1$H NMR (400 MHz, DMSO-d6) δ 10.93 (s, 1H), 10.33 (s, 1H), 8.23 (s, 1H), 8.14 - 8.08 (m, 3H), 7.64 - 7.57 (m, 3H), 7.53 - 7.50 (m, 1H), 7.27 (d, $J$ = 7.5 Hz, 2H), 6.82 (brs, 1H), 6.19 (brs, 1H), 4.95 (t, $J$ = 13.7 Hz, 1H), 4.51 (s, 2H), 3.82 (t, $J$ = 6.8 Hz, 2H), 3.27 - 3.15 (m, 4H), 2.86 (t, $J$ = 6.8 Hz, 2H), 2.13 (d, $J$ = 12.5 Hz, 3H), 1.75 - 1.65 (m, 3H). LCMS (ESI) calcd for $C_{29}H_{29}N_6O_4^+$ [M+H]$^+$: 525.22, found, 525.3.

**Example 59: preparation of 5-((4-((4'-chloro-[1,1'-biphenyl]-2-yl)amino)piperidin-1-yl)methyl)-2-(2,4-dioxotetrahydropyrimidin-1(2H)-yl)isoindoline-1,3-dione (GT-05179)**

Referring to the method of Scheme 1, the target product GT-05179 was prepared as a white solid (14 mg, yield 17%). $^1H$ NMR (400 MHz, DMSO-d6) δ 10.93 (s, 1H), 10.55 (s, 1H), 8.27 (s, 1H), 8.12 (s, 2H), 7.50 (d, *J* = 8.4 Hz, 2H), 7.40 (d, *J* = 8.3 Hz, 2H), 7.19 (d, *J* = 8.0 Hz, 1H), 6.99 (d, *J* = 7.5 Hz, 1H), 6.82 (d, *J* = 8.4 Hz, 1H), 6.74 - 6.70 (m, 1H), 4.59 - 4.40 (m, 3H), 3.83 (t, *J* = 6.7 Hz, 3H), 3.09 - 3.01 (m, 3H), 2.86 (t, *J* = 6.7 Hz, 2H), 2.08 (d, *J* = 11.4 Hz, 2H), 1.98 (s, 1H), 1.67 - 1.58 (m, 2H). LCMS (ESI) calcd for $C_{30}H_{29}ClN_5O_4^+$ [M+H]$^+$: 558.19, found, 558.2.

**Example 60: preparation of 5-((4-((4'-chloro-5,5-dimethyl-3,4,5,6-tetrahydro-[1,1'-biphenyl]-2-yl)methyl)piperazin-1-yl)methyl)-2-(2,4-dioxotetrahydropyrimidin-1(2H)-yl)isoindoline-1,3-dione (GT-05180)**

Referring to the method of Scheme 1, the target product GT-05180 was prepared as a white solid (40 mg, yield 45%). $^1H$ NMR (400 MHz, DMSO-d6) δ 10.92 (s, 1H), 8.10 - 8.02 (m, 2H), 7.97 - 7.94 (m, 1H), 7.43 (d, *J* = 8.3 Hz, 2H), 7.12 (d, *J* = 8.2 Hz, 2H), 3.82 (t, *J* = 6.8 Hz, 2H), 3.61 - 3.46 (m, 4H), 3.29 - 3.22 (m, 4H), 3.17 - 2.91 (m, 4H), 2.86 (t, *J* = 6.8 Hz, 2H), 2.27 (s, 2H), 2.02 (s, 2H), 1.46 (t, *J* = 5.8 Hz, 2H), 0.96 (s, 6H). LCMS (ESI) calcd for $C_{32}H_{37}ClN_5O_4^+$ [M+H]$^+$: 590.25, found, 590.3.

**Example 61: preparation of 5-((4-(1H-pyrrolo[2,3-b]pyridin-3-yl)-3,6-dihydropyridin-1(2H)-yl)methyl)-2-(2,4-dioxotetrahydropyrimidin-1(2H)-yl)isoindoline-1,3-dione (GT-05181)**

Referring to the method of Scheme 1, the target product GT-05181 was prepared as a white solid (29 mg, yield 40%). $^1H$ NMR (400 MHz, DMSO-d6) δ 12.02 (s, 1H), 10.99 (s, 1H), 10.94 (s, 1H), 8.36 (d, *J* = *4.1* Hz, 1H), 8.31 - 8.28 (m, 2H), 8.25 - 8.18 (m, 1H), 8.14 (d, *J* = 7.7 Hz, 1H), 7.71 (s, 1H), 7.18 (dd, *J* = 7.9, 4.9 Hz, 1H), 6.19 (s, 1H), 4.75 - 4.63 (m, 2H), 3.94 - 3.81 (m, 4H), 3.72 - 3.66 (m, 2H), 3.38 - 3.27 (m, 2H), 2.88 (t, *J* = 6.7 Hz, 2H). LCMS (ESI) calcd for $C_{25}H_{23}N_6O_4^+$ [M+H]$^+$: 471.18, found, 471.2.

**Example 62: preparation of 5-((4-(2,3-dichlorophenyl)-3,6-dihydropyridin-1(2H)-yl)methyl)-2-(2,4-dioxotetrahydropyrimidin-1(2H)-yl)isoindoline-1,3-dione (GT-05249)**

Referring to the method of Scheme 1, the target product GT-05249 was prepared as a white solid (26 mg, yield 34%). $^1H$ NMR (400 MHz, DMSO-d6) δ 11.22 (s, 1H), 10.94 (s, 1H), 8.34 (s, 1H), 8.21 (d, *J* = 6.6 Hz, 1H), 8.13 (d, *J* = 7.6 Hz, 1H), 7.63 (dd, *J* = 8.0, 1.3 Hz, 1H), 7.40 (t, *J* = 7.9 Hz, 1H), 7.26 (dd, *J* = 7.7, 1.4 Hz, 1H), 5.75 (s, 1H), 4.67 (brs, 2H), 3.85 - 3.80 (m, 4H), 3.71 - 3.58 (m, 2H), 2.88 - 2.76 (m, 3H), 2.61 - 2.53 (m, 1H). LCMS (ESI) calcd for $C_{24}H_{21}Cl_2N_4O_4^+$ [M+H]$^+$: 499.09, found, 499.1.

**Example 63: preparation of 5-((4-(2,3-dichlorophenyl)piperidin-1-yl)methyl)-2-(2,4-dioxotetrahydropyrimidin-1(2H)-yl)isoindoline-1,3-dione (GT-05250)**

Referring to the method of Scheme 1, the target product GT-05250 was prepared as a white solid (29 mg, yield 38%). $^1H$ NMR (400 MHz, DMSO-d6) δ 10.94 (s, 1H), 10.82 (s, 1H), 8.30 (s, 1H), 8.17 - 8.11 (m, 2H), 7.56 (d, *J* = 7.9 Hz, 1H), 7.41 (t, *J* = 7.9 Hz, 1H), 7.31 (d, *J* = 7.6 Hz, 1H), 4.57 (s, 2H), 3.85 - 3.81 (m, 3H), 3.59 - 3.46 (m, 3H), 3.23 - 3.15 (m, 2H), 2.87 (t, *J* = 6.8 Hz, 2H), 2.05 - 1.96 (m, 4H). LCMS (ESI) calcd for $C_{24}H_{23}Cl_2N_4O_4^+$ [M+H]$^+$: 501.11, found, 501.1.

**Example 64: preparation of 5-((3-(2,3-dichlorophenyl)-3,6-diazabicyclo[3.1.1]heptan-6-yl)methyl)-2-(2,4-dioxotetrahydropyrimidin-1(2H)-yl)isoindoline-1,3-dione (GT-05251)**

Referring to the method of Scheme 1, the target product GT-05251 was prepared as a white solid (23 mg, yield 29%). $^1H$ NMR (400 MHz, DMSO-d6) δ 10.91 (s, 1H), 8.18 - 8.11 (m, 1H), 8.06 - 7.95 (m, 2H), 7.39 - 7.33 (m, 2H), 7.25 - 7.14 (m, 1H), 4.74 (s, 1H), 4.43 - 4.14 (m, 2H), 3.88 - 3.77 (m, 3H), 3.63 - 3.53 (m, 1H), 3.60 - 3.46 (m, 3H), 3.26 - 3.14 (m, 2H), 2.86 (t, *J* = 6.7 Hz, 2H). LCMS (ESI) calcd for $C_{24}H_{22}Cl_2N_5O_4^+$ [M+H]$^+$: 514.10, found, 514.1.

**Example 65: preparation of 5-(((1S,4S)-5-(2,3-dichlorophenyl)-2,5-diazabicyclo[2.2.1]heptan-2-yl)methyl)-2-(2,4-dioxotetrahydropyrimidin-1(2H)-yl)isoindoline-1,3-dione (GT-05252)**

Referring to the method of Scheme 1, the target product GT-05252 was prepared as a white solid (26 mg, yield 33%). $^1H$ NMR (400 MHz, DMSO-d6) δ 10.93 (s, 1H), 10.03 (s, 1H), 8.34 (s, 1H), 8.20 (d, *J* = 7.1 Hz, 1H), 8.12 (d, *J* = *7.7* Hz, 1H), 7.21 - 7.14 (m, 1H), 7.11 (d, *J* = 7.3 Hz, 1H), 6.97 (d, *J* = 8.2 Hz, 1H), 4.79 (d, *J* = 9.4 Hz, 1H), 4.54 (d, *J* = 5.4 Hz, 1H),

4.44 (s, 1H), 3.97 (d, $J$ = 9.9 Hz, 1H), 3.82 (t, $J$ = 6.7 Hz, 2H), 3.66 - 3.60 (m, 2H), 3.30 - 3.24 (m, 2H), 2.86 (t, $J$ = 6.7 Hz, 3H), 2.59 (d, $J$ = 12.1 Hz, 1H), 2.20 (d, $J$ = 11.2 Hz, 1H). LCMS (ESI) calcd for $C_{24}H_{22}Cl_2N_5O_4^+$ [M+H]$^+$: 514.10, found, 514.1.

**Example 66: preparation of 2-(2,4-dioxotetrahydropyrimidin-1(2H)-yl)-5-((3-(trifluoromethyl)-5,6-dihydro-[1,2,4] triazolo[4,3-a]pyrazin-7(8H)-yl)methyl)isoindoline-1,3-dione (GT-05253)**

Referring to the method of Scheme 1, the target product GT-05253 was prepared as a white solid (11 mg, yield 16%). $^1$H NMR (400 MHz, DMSO-d6) δ 10.91 (s, 1H), 8.04 (s, 1H), 8.00 (s, 1H), 7.98 (s, 1H), 4.21 (t, $J$ = 5.3 Hz, 2H), 4.07 (s, 1H), 3.99 (s, 2H), 3.84 - 3.80 (m, 2H), 3.70 - 3.62 (m, 1H), 3.15 - 3.07 (m, 1H), 3.09 - 3.04 (m, 1H), 2.87 - 2.84 (m, 2H). LCMS (ESI) calcd for $C_{19}H_{17}F_3N_7O_4^+$ [M+H]$^+$: 464.13, found, 464.1.

**Example 67: preparation of 5-((6,7-dihydrothieno[3,2-c]pyridin-5(4H)-yl)methyl)-2-(2,4-dioxotetrahydropyrimidin-1(2H)-yl)isoindoline-1,3-dione (GT-05254)**

Referring to the method of Scheme 1, the target product GT-05254 was prepared as a white solid (18 mg, yield 28%). $^1$H NMR (400 MHz, DMSO-d6) δ 11.25 (s, 1H), 10.94 (s, 1H), 8.32 (s, 1H), 8.20 (d, $J$ = 5.1 Hz, 1H), 8.23 - 8.19 (m, 1H), 7.46 (d, $J$ = 5.2 Hz, 1H), 6.89 (s, 1H), 4.75 - 4.67 (m, 2H), 4.24 (s, 2H), 3.83 (t, $J$ = 6.8 Hz, 2H), 3.30 - 3.07 (m, 4H), 2.91 - 2.82 (m, 2H). LCMS (ESI) calcd for $C_{20}H_{10}N_4O_4S^+$ [M+H]$^+$: 411.11, found, 411.1.

**Example 68: preparation of 2-(2,4-dioxotetrahydropyrimidin-1(2H)-yl)-5-((4-(6-fluoro-1H-indol-3-yl)piperidin-1-yl)methyl)isoindoline-1,3-dione (GT-05259)**

Referring to the method of Scheme 1, the target product GT-05259 was prepared as a white solid (25 mg, yield 33%). $^1$H NMR (400 MHz, DMSO-d6) δ 10.97 (s, 1H), 10.94 (s, 1H), 8.31 (s, 1H), 8.19 - 8.13 (m, 2H), 7.64 (dd, $J$ = 8.6, 5.6 Hz, 1H), 7.17 - 7.08 (m, 2H), 6.87 - 6.82 (m, 1H), 4.59 (s, 2H), 3.84 (t, $J$ = 6.8 Hz, 2H), 3.49 (d, $J$ = 10.9 Hz, 2H), 3.23 - 3.12 (m, 2H), 3.03 (t, $J$ = 13.8 Hz, 1H), 2.87 (t, $J$ = 6.8 Hz, 2H), 2.19 - 1.98 (m, 4H). LCMS (ESI) calcd for $C_{26}H_{25}FN_5O_4^+$ [M+H]$^+$: 490.19, found, 490.2.

**Example 69: preparation of 2-(2,4-dioxotetrahydropyrimidin-1(2H)-yl)-5-((4-(5-fluoroindolin-1-yl)piperidin-1-yl) methyl)isoindoline-1,3-dione (GT-05260)**

Referring to the method of Scheme 1, the target product GT-05260 was prepared as a white solid (15 mg, yield 20%). $^1$H NMR (400 MHz, DMSO-d6) δ 10.93 (s, 1H), 8.25 - 8.12 (m, 1H), 7.98 - 7.83 (m, 1H), 7.74 - 7.70 (m, 1H), 6.92 (d, $J$ = 8.5 Hz, 1H), 6.82 (t, $J$ = 9.0 Hz, 1H), 6.58 - 6.52 (m, 1H), 4.56 - 4.38 (m, 2H), 3.50 - 3.36 (m, 4H), 3.35 - 3.29 (m, 2H), 3.15 - 3.00 (m, 3H), 2.91 - 2.83 (m, 4H), 2.18 - 2.00 (m, 2H), 1.91 - 1.78 (m, 2H). LCMS (ESI) calcd for $C_{26}H_{27}FN_5O_4^+$ [M+H]$^+$: 492.20, found, 492.2.

**Example 70: preparation of 2-(2,4-dioxotetrahydropyrimidin-1(2H)-yl)-5-((4-(7-fluoroquinolin-4-yl)-3,6-dihydropyridin-1(2H)-yl)methyl)isoindoline-1,3-dione (GT-05261)**

Referring to the method of Scheme 1, the target product GT-05261 was prepared as a white solid (20 mg, yield 26%). $^1$H NMR (400 MHz, DMSO-d6) δ 11.64 (s, 1H), 10.94 (s, 1H), 9.02 (d, $J$ = 4.7 Hz, 1H), 8.42 - 8.38 (m, 2H), 8.28 (d, $J$ = 7.5 Hz, 1H), 8.16 (d, $J$ = 7.7 Hz, 1H), 7.88 (dd, $J$ = 10.0, 2.6 Hz, 1H), 7.68 - 7.60 (m, 1H), 7.52 (d, $J$ = 4.7 Hz, 1H), 5.91 (s, 1H), 4.84 - 4.60 (m, 2H), 3.98 - 3.87 (m, 2H), 3.84 (t, $J$ = *6.8* Hz, 2H), 3.79 - 3.65 (m, 1H), 3.12 - 2.96 (m, 1H), 2.87 (t, $J$ = 6.8 Hz, 2H), 2.75 - 2.66 (m, 2H). LCMS (ESI) calcd for $C_{27}H_{23}FN_5O_4^+$ [M+H]$^+$: 500.17, found, 500.2.

**Example 71: preparation of 5-((4-benzhydrylpiperazin-1-yl)methyl)-2-(2,4-dioxotetrahydropyrimidin-1(2H)-yl) isoindoline-1,3-dione (GT-05267)**

Referring to the method of Scheme 1, the target product GT-05267 was prepared as a white solid (38 mg, yield 48%). $^1$H NMR (400 MHz, DMSO-d6) δ 10.85 (s, 1H), 8.14 (s, 1H), 8.01 (s, 2H), 7.51 - 7.37 (m, 4H), 7.32 - 7.23 (m, 4H), 7.22 - 7.16 (m, 2H), 4.53 - 4.31 (m, 3H), 3.74 (t, $J$ = 6.7 Hz, 2H), 3.19 - 3.02 (m, 5H), 2.91 - 2.71 (m, 5H). LCMS (ESI) calcd for $C_{30}H_{30}N_5O_4^+$ [M+H]$^+$: 524.23, found, 524.2.

**Example 72: preparation of 5-((4-(bis(4-fluorophenyl)methyl)piperazin-1-yl)methyl)-2-(2,4-dioxotetrahydropyrimidin-1(2H)-yl)isoindoline-1,3-dione (GT-05268)**

Referring to the method of Scheme 1, the target product GT-05268 was prepared as a white solid (33 mg, yield

39%). $^1$H NMR (400 MHz, DMSO-d6) δ 10.92 (s, 1H), 8.24 (s, 1H), 8.14 - 8.05 (m, 2H), 7.56 - 7.42 (m, 4H), 7.20 - 7.16 (m, 4H), 4.57 - 4.44 (m, 3H), 3.82 (t, *J* = 6.8 Hz, 2H), 3.23 - 3.07 (m, 5H), 2.92 - 2.79 (m, 5H). LCMS (ESI) calcd for $C_{30}H_{28}F_2N_5O_4^+$ [M+H]$^+$: 560.21, found, 560.2.

**Example 73: preparation of 5-((4-(bis(4-chlorophenyl)methyl)piperazin-1-yl)methyl)-2-(2,4-dioxotetrahydropyrimidin-1(2H)-yl)isoindoline-1,3-dione (GT-05269)**

Referring to the method of Scheme 1, the target product GT-05269 was prepared as a white solid (29 mg, yield 32%). $^1$H NMR (400 MHz, DMSO-d6) δ 10.93 (s, 1H), 8.25 (s, 1H), 8.14 - 8.05 (m, 2H), 7.53 - 7.46 (m, 4H), 7.42 - 7.38 (m, 4H), 4.71 - 4.64 (m, 1H), 4.56 - 4.48 (m, 2H), 3.82 (t, *J* = 6.7 Hz, 2H), 3.18 - 3.08 (m, 5H), 2.92 - 2.79 (m, 5H). LCMS (ESI) calcd for $C_{30}H_{28}Cl_2N_5O_4^+$ [M+H]$^+$: 592.15, found, 592.2.

**Example 74: preparation of 2-(2,4-dioxotetrahydropyrimidin-1(2H)-yl)-5-((4-(2-methylthieno[2,3-d]pyrimidin-4-yl)piperidin-1-yl)methyl)isoindoline-1,3-dione (GT-05272)**

Referring to the method of Scheme 1, the target product GT-05272 was prepared as a white solid (14 mg, yield 18%). $^1$H NMR (400 MHz, DMSO-d6) δ 10.94 (s, 1H), 10.67 (s, 1H), 8.33 (s, 1H), 8.19 (d, *J* = 7.6 Hz, 1H), 8.15 (d, *J* = 7.6 Hz, 1H), 7.87 (d, *J* = 6.0 Hz, 1H), 7.74 (d, *J* = 6.0 Hz, 1H), 4.68 - 4.55 (m, 2H), 3.84 (t, *J* = 6.7 Hz, 2H), 3.60 - 3.54 (m, 2H), 3.20 - 3.13 (m, 2H), 2.86 (t, *J* = 6.8 Hz, 2H), 2.70 (s, 3H), 2.33 - 2.26 (m, 3H), 2.05 (d, *J* = 13.3 Hz, 2H). LCMS (ESI) calcd for $C_{25}H_{25}N_6O_4S^+$ [M+H]$^+$: 505.17, found, 505.2.

**Example 75: preparation of 5-((4-(5,6-dimethylthieno[2,3-d]pyrimidin-4-yl)-3,6-dihydropyridin-1(2H)-yl) methyl)-2-(2,4-dioxotetrahydropyrimidin-1(2H)-yl)isoindoline-1,3-dione (GT-05273)**

Referring to the method of Scheme 1, the target product GT-05273 was prepared as a white solid (18 mg, yield 23%). $^1$H NMR (400 MHz, DMSO-d6) δ 11.67 (s, 1H), 10.94 (s, 1H), 8.95 (s, 1H), 8.40 (s, 1H), 8.31 - 8.23 (m, 1H), 8.14 (d, *J* = 7.7 Hz, 1H), 5.85 (s, 1H), 4.81 - 4.62 (m, 2H), 4.02 - 3.91 (m, 1H), 3.85 *(t, J* = 6.7 Hz, 2H), 3.73 - 3.70 (m, 3H), 3.38 (brs, 1H), 3.05 - 2.93 (m, 1H), 2.87 (t, *J* = 6.8 Hz, 2H), 2.52 (s, 3H), 2.29 (s, 3H). LCMS (ESI) calcd for $C_{26}H_{25}N_6O_4S^+$ [M+H]$^+$: 517.17, found, 517.2.

**Example 76: preparation of 5-(((1S,4S)-5-benzhydryl-2,5-diazabicyclo[2.2.1]heptan-2-yl)methyl)-2-(2,4-dioxotetrahydropyrimidin-1(2H)-yl)isoindoline-1,3-dione (GT-05274)**

Referring to the method of Scheme 1, the target product GT-05274 was prepared as a white solid (19 mg, yield 23%). $^1$H NMR (400 MHz, DMSO-d6) δ 10.93 (s, 1H), 8.43 - 8.31 (m, 1H), 8.31 - 8.20 (m, 1H), 8.10 - 8.09 (m, 1H), 7.74 - 7.49 (m, 3H), 7.42 - 7.20 (m, 7H), 4.71 (brs, 1H), 4.45 (brs, 1H), 4.23 (brs, 1H), 3.83 (t, *J* = 6.6 Hz, 2H), 3.27 - 3.18 (m, 5H), 3.18 - 3.00 (m, 1H), 2.86 (t, *J* = 6.7 Hz, 3H), 2.45 - 2.27 (m, 1H). LCMS (ESI) calcd for $C_{31}H_{30}N_5O_4^+$ [M+H]$^+$: 536.23, found, 536.2.

**Example 77: preparation of 2-(2,4-dioxotetrahydropyrimidin-1(2H)-yl)-5-((4-(isoquinolin-5-yl)-3,6-dihydropyridin-1(2H)-yl)methyl)isoindoline-1,3-dione (GT-05275)**

Referring to the method of Scheme 1, the target product GT-05275 was prepared as a white solid (24 mg, yield 33%). H NMR (400 MHz, DMSO-d6) δ 10.94 (s, 1H), 9.72 (s, 1H), 8.63 (d, *J* = 6.4 Hz, 1H), 8.55 - 8.42 (m, 2H), 8.42 - 8.28 (m, 2H), 8.15 (d, *J* = 7.6 Hz, 1H), 7.92 (d, *J* = 5.1 Hz, 2H), 5.82 (s, 1H), 4.81 - 4.64 (m, 2H), 3.92 - 3.83 (m, 4H), 3.13 - 2.99 (m, 2H), 2.87 (t, *J* = 6.8 Hz, 2H), 2.82 - 2.74 (m, 1H), 2.73 - 2.62 (m, 1H). LCMS (ESI) calcd for $C_{27}H_{24}N_5O_4^+$ [M+H]$^+$: 482.18, found, 482.2.

**Example 78: preparation of 2-(2,4-dioxotetrahydropyrimidin-1(2H)-yl)-5-((4-(6-fluoro-1-methyl-1H-indol-3-yl)-3,6-dihydropyridin-1(2H)-yl)methyl)isoindoline-1,3-dione (GT-05276)**

Referring to the method of Scheme 1, the target product GT-05276 was prepared as a white solid (19 mg, yield 25%). $^1$H NMR (400 MHz, DMSO-d6) δ 10.94 (s, 1H), 8.32 (s, 1H), 8.17 - 8.12 (m, 2H), 7.84 - 7.80 (m, 1H), 7.55 (s, 1H), 7.36 (dd, *J* = 10.1, 2.3 Hz, 1H), 6.97 (td, *J* = 9.3, 2.4 Hz, 1H), 6.14 (s, 1H), 4.77 - 4.60 (m, 2H), 3.88 - 3.78 (m, 5H), 3.76 (s, 3H), 3.70 - 3.64 (m, 2H), 2.89 - 2.81 (m, 4H). LCMS (ESI) calcd for $C_{27}H_{25}FN_5O_4^+$ [M+H]$^+$: 502.19, found, 502.2.

**Example 79: preparation of 5-((((3s,5s,7s)-adamantan-1-yl)amino)methyl)-2-(2,4-dioxotetrahydropyrimidin-1(2H)-yl)isoindoline-1,3-dione (GT-05277)**

Referring to the method of Scheme 1, the target product GT-05277 was prepared as a white solid (8 mg, yield 12%). $^1H$ NMR (400 MHz, DMSO-d6) δ 10.93 (s, 1H), 8.21 (brs, 1H), 8.08 (brs, 2H), 4.37 (brs, 1H), 3.86 - 3.81 (m, 2H), 3.50 - 3.43 (m, 2H), 2.87 (t, *J* = 6.7 Hz, 2H), 2.18 (s, 3H), 1.95 (s, 6H), 1.85 - 1.62 (m,6H). LCMS (ESI) calcd for $C_{23}H_{27}N_4O_4^+$ $[M+H]^+$: 423.20, found, 423.2.

**Example 80: preparation of 2-(2,4-dioxotetrahydropyrimidin-1(2H)-yl)-5-((3-(5-fluoroindolin-1-yl)azetidin-1-yl) methyl)isoindoline-1,3-dione (GT-05278)**

Referring to the method of Scheme 1, the target product GT-05278 was prepared as a white solid (13 mg, yield 18%). $^1H$ NMR (400 MHz, DMSO-d6) δ 10.93 (s, 1H), 8.14 - 8.05 (m, 1H), 7.96 - 7.83 (m, 1H), 7.76 - 7.67 (m, 1H), 6.98 (d, *J* = 7.5 Hz, 1H), 6.84 - 6.79 (m, 1H), 6.48 - 6.37 (m, 1H), 4.64 (s, 1H), 4.33 - 4.24 (m, 3H), 4.16 - 4.13 (m, 1H), 3.83 (t, *J* = 6.9 Hz, 2H), 3.71 - 3.62 (m, 3H), 2.99 - 2.90 (m, 3H), 2.86 (t, *J* = 6.5 Hz, 2H). LCMS (ESI) calcd for $C_{24}H_{23}FN_5O_4^+$ $[M+H]^+$: 464.17, found, 464.1.

**Example 81: preparation of 2-(2,4-dioxotetrahydropyrimidin-1(2H)-yl)-5-((4-(thieno[2,3-d]pyrimidin-4-yl)-3,6-dihydropyridin-1(2H)-yl)methyl)isoindoline-1,3-dione (GT-05279)**

Referring to the method of Scheme 1, the target product GT-05279 was prepared as a white solid (22 mg, yield 30%). LCMS (ESI) calcd for $C_{24}H_{21}N_6O_4S^+$ $[M+H]^+$: 489.13, found, 489.1.

**Example 82: preparation of 2-(2,4-dioxotetrahydropyrimidin-1(2H)-yl)-5-((4-(6-methylthieno[2,3-d]pyrimidin-4-yl)-3,6-dihydropyridin-1(2H)-yl)methyl)isoindoline-1,3-dione (GT-05280)**

Referring to the method of Scheme 1, the target product GT-05280 was prepared as a white solid (17 mg, yield 22%). LCMS (ESI) calcd for $C_{25}H_{23}N_6O_4S^+$ $[M+H]^+$: 503.15, found, 503.2.

**Example 83: preparation of 2-(2,4-dioxotetrahydropyrimidin-1(2H)-yl)-5-((4-(2-methylthieno[2,3-d]pyrimidin-4-yl)-3,6-dihydropyridin-1(2H)-yl)methyl)isoindoline-1,3-dione (GT-05307)**

Referring to the method of Scheme 1, the target product GT-05307 was prepared as a white solid (18 mg, yield 24%). $^1H$ NMR (400 MHz, DMSO-d6) δ 11.28 (s, 1H), 10.94 (s, 1H), 8.37 (s, 1H), 8.27 - 8.24 (m, 2H), 8.15 (d, *J* = 6.9 Hz, 1H), 7.92 (d, *J* = 6.1 Hz, 1H), 7.69 (d, *J* = 6.3 Hz, 1H), 6.61 (s, 1H), 4.76 - 4.67 (m, 2H), 4.05 - 3.92 (m, 2H), 3.84 (t, *J* = 6.8 Hz, 2H), 3.77 - 3.68 (m, 2H), 3.04 (s, 3H), 2.86 (t, *J* = 6.8 Hz, 2H), 2.72 (s, 3H). LCMS (ESI) calcd for $C_{25}H_{23}N_6O_4S^+$ $[M+H]^+$: 503.15, found, 503.2.

**Example 84: preparation of 2-(2,4-dioxotetrahydropyrimidin-1(2H)-yl)-5-((4-(thieno[2,3-d]pyrimidin-2-yl)-3,6-dihydropyridin-1(2H)-yl)methyl)isoindoline-1,3-dione (GT-05308)**

Referring to the method of Scheme 1, the target product GT-05308 was prepared as a white solid (18 mg, yield 24%). $^1H$ NMR (400 MHz, DMSO-d6) δ 11.05 (s, 1H), 10.94 (s, 1H), 9.33 (s, 1H), 8.35 (s, 1H), 8.24 - 8.20 (m, 1H), 8.15 (d, *J* = 7.8 Hz, 1H), 7.97 (d, *J* = 5.9 Hz, 1H), 7.60 (d, *J* = 5.9 Hz, 1H), 7.21 (s, 1H), 4.79 - 4.61 (m, 2H), 3.97 (s, 2H), 3.84 (t, *J* = 6.7 Hz, 2H), 3.78 - 3.68 (m, 1H), 3.27 - 3.23 (m, 1H), 3.14 - 3.09 (m, 1H), 3.01 - 2.95 (m, 1H), 2.87 (t, *J* = 6.8 Hz, 2H). LCMS (ESI) calcd for $C_{24}H_{21}N_6O_4S^+$ $[M+H]^+$: 489.13, found, 489.1.

**Example 85: preparation of 2-(2,4-dioxotetrahydropyrimidin-1(2H)-yl)-5-((4-(5-methylthiophen-2-yl)piperazin-1-yl)methyl)isoindoline-1,3-dione (GT-05309)**

Referring to the method of Scheme 1, the target product GT-05309 was prepared as a white solid (12 mg, yield 17%). LCMS (ESI) calcd for $C_{22}H_{24}N_5O_4S^+$ $[M+H]^+$: 454.15, found, 454.2.

**Example 86: preparation of 2-(2,4-dioxotetrahydropyrimidin-1(2H)-yl)-5-((4-(5-methylthiophen-2-yl)-3,6-dihydropyridin-1(2H)-yl)methyl)isoindoline-1,3-dione (GT-05310)**

Referring to the method of Scheme 1, the target product GT-05310 was prepared as a white solid (20 mg, yield 29%). $^1H$ NMR (400 MHz, DMSO-d6) δ 10.93 (s, 1H), 10.72 (s, 1H), 8.29 (s, 1H), 8.12 (s, 2H), 6.96 (d, *J* = 3.1 Hz, 1H), 6.75

(d, *J* = 2.5 Hz, 1H), 5.91 (s, 1H), 4.71 - 4.59 (m, 2H), 3.88 - 3.71 (m, 5H), 3.66 - 3.61 (m, 1H), 2.87 (t, *J* = 6.8 Hz, 2H), 2.81 - 2.75 (m, 2H), 2.42 (s, 3H). LCMS (ESI) calcd for $C_{23}H_{23}N_4O_4S^+$ $[M+H]^+$: 451.14, found, 451.2.

**Example 87: preparation of 2-(2,4-dioxotetrahydropyrimidin-1(2H)-yl)-5-((4-(5-methylthiophen-2-yl)piperidin-1-yl)methyl)isoindoline-1,3-dione (GT-05311)**

Referring to the method of Scheme 1, the target product GT-05311 was prepared as a white solid (22 mg, yield 32%). $^1H$ NMR (400 MHz, DMSO-d6) δ 10.93 (s, 1H), 10.57 (s, 1H), 8.28 (s, 1H), 8.18 - 8.07 (m, 2H), 6.66 (d, *J* = 3.3 Hz, 1H), 6.63 (d, *J* = 3.3 Hz, 1H), 4.54 (s, 2H), 3.82 (t, *J* = 6.8 Hz, 2H), 3.50 - 3.43 (m, 2H), 3.17 - 2.99 (m, 3H), 2.86 (t, *J* = 6.8 Hz, 2H), 2.39 (s, 3H), 2.10 - 2.08 (m, 2H), 1.95 - 1.85 (m, 2H). LCMS (ESI) calcd for $C_{23}H_{25}N_4O_4S^+$ $[M+H]^+$: 453.16, found, 453.2.

**Example 88: preparation of 2-(2,4-dioxotetrahydropyrimidin-1(2H)-yl)-5-((4-(5-methylthiophen-3-yl)piperazin-1-yl)methyl)isoindoline-1,3-dione (GT-05312)**

Referring to the method of Scheme 1, the target product GT-05312 was prepared as a white solid (15 mg, yield 22%). LCMS (ESI) calcd for $C_{22}H_{24}N_5O_4S^+$ $[M+H]^+$: 454.15, found, 454.2.

**Example 89: preparation of 2-(2,4-dioxotetrahydropyrimidin-1(2H)-yl)-5-((4-(thiophen-3-yl)piperazin-1-yl) methyl)isoindoline-1,3-dione (GT-05313)**

Referring to the method of Scheme 1, the target product GT-05313 was prepared as a white solid (7 mg, yield 10%). LCMS (ESI) calcd for $C_{21}H_{22}N_5O_4S^+$ $[M+H]^+$: 440.14, found, 440.1.

**Example 90: preparation of 2-(2,4-dioxotetrahydropyrimidin-1(2H)-yl)-5-((4-(4-methylthiophen-3-yl)piperidin-1-yl)methyl)isoindoline-1,3-dione (GT-05314)**

Referring to the method of Scheme 1, the target product GT-05314 was prepared as a white solid (17 mg, yield 24%). $^1H$ NMR (400 MHz, DMSO-d6) δ 10.86 (s, 1H), 10.36 (s, 1H), 8.20 (s, 1H), 8.06 (s, 2H), 7.06 (s, 3H), 4.48 (s, 2H), 3.76 (t, *J* = 6.8 Hz, 2H), 3.46 - 3.36 (m, 2H), 3.08 - 3.00 (m, 2H), 2.79 (t, *J* = 6.8 Hz, 3H), 2.75 - 2.67 (m, 1H), 2.09 (s, 3H), 1.94 - 1.91 (m, 2H), 1.84 - 1.74 (m, 2H). LCMS (ESI) calcd for $C_{23}H_{25}N_4O_4S^+$ $[M+H]^+$: 453.16, found, 453.2.

**Example 91: preparation of 2-(2,4-dioxotetrahydropyrimidin-1(2H)-yl)-5-((4-(furan-2-yl)piperidin-1-yl)methyl) isoindoline-1,3-dione (GT-05315)**

Referring to the method of Scheme 1, the target product GT-05315 was prepared as a white solid (15 mg, yield 23%). $^1H$ NMR (400 MHz, DMSO-d6) δ 10.93 (s, 1H), 10.60 (s, 1H), 8.29 (s, 1H), 8.16 - 8.11 (m, 2H), 7.56 (s, 1H), 6.39 (s, 1H), 6.15 (s, 1H), 4.54 (s, 2H), 3.83 (t, *J* = 6.8 Hz, 2H), 3.52 - 3.43 (m, 2H), 3.12 - 3.07 (m, 2H), 2.86 (t, *J* = 6.8 Hz, 2H), 2.15 - 2.11 (m, 3H), 1.93 - 1.83 (m, 2H). LCMS (ESI) calcd for $C_{22}H_{23}N_4O_5^+$ $[M+H]^+$: 423.17, found, 423.2.

**Example 92: preparation of 2-(2,4-dioxotetrahydropyrimidin-1(2H)-yl)-5-((4-(4-methylthiophen-3-yl)-3,6-dihydropyridin-1(2H)-yl)methyl)isoindoline-1,3-dione (GT-05316)**

Referring to the method of Scheme 1, the target product GT-05316 was prepared as a white solid (18 mg, yield 26%). $^1H$ NMR (400 MHz, DMSO-d6) δ 10.94 (s, 1H), 10.78 (s, 1H), 8.31 (s, 1H), 8.18 - 8.12 (m, 2H), 7.44 (d, *J* = 2.6 Hz, 1H), 7.21 (d, *J* = 2.2 Hz, 1H), 5.80 (s, 1H), 4.73 - 4.67 (m, 2H), 3.85 - 3.80 (m, 4H), 3.73 - 3.53 (m, 2H), 2.87 (t, *J* = 6.8 Hz, 2H), 2.78 - 2.75(m, 1H), 2.68 - 2.64 (m, 1H), 2.24 (s, 3H). LCMS (ESI) calcd for $C_{23}H_{23}N_4O_4S^+$ $[M+H]^+$: 451.14, found, 451.1.

**Example 93: preparation of 2-(2,4-dioxotetrahydropyrimidin-1(2H)-yl)-5-((4-(furan-2-yl)-3,6-dihydropyridin-1(2H)-yl)methyl)isoindoline-1,3-dione (GT-05317)**

Referring to the method of Scheme 1, the target product GT-05317 was prepared as a white solid (15 mg, yield 23%). $^1H$ NMR (400 MHz, DMSO-d6) δ 11.14 (s, 1H), 10.86 (s, 1H), 8.26 (s, 1H), 8.12 (s, 1H), 8.04 (d, *J* = 7.5 Hz, 1H), 7.61 (s, 1H), 6.47 - 6.45 (m, 2H), 6.01 (s, 1H), 4.63 - 4.45 (m, 2H), 3.77 (t, *J* = 6.8 Hz, 2H), 3.72 - 3.65 (m, 1H), 3.61 - 3.57 (m, 2H), 2.79 (t, *J* = 6.8 Hz, 2H), 2.70 - 2.65 (m, 1H), 2.64 - 2.57 (m, 2H). LCMS (ESI) calcd for $C_{22}H_{21}N_4O_5^+$ $[M+H]^+$: 421.15, found, 421.2.

**Example 94: preparation of 1-(5-((4-(3,4-dichloropyridin-2-yl)piperidin-1-yl)methyl)-1-oxoisoindolin-2-yl)dihydropyrimidine-2,4(1H,3H)-dione (GT-06388)**

Referring to the method of Scheme 1, the target product GT-06388 was prepared as a white solid (37 mg, yield 60%). $^1$H NMR (400 MHz, DMSO-d6) δ 11.13 (s, 1H), 10.69 (s, 1H), 8.48 (d, *J* = 5.2 Hz, 1H), 7.92 (s, 1H), 7.87 (d, *J* = 7.8 Hz, 1H), 7.82 (d, *J* = 7.7 Hz, 1H), 7.65 (d, *J* = 5.2 Hz, 1H), 4.82 (d, *J* = 16.6 Hz, 1H), 4.59 (d, *J* = 16.8 Hz, 1H), 4.45 (d, *J* = 4.6 Hz, 2H), 3.80 (t, *J* = 6.8 Hz, 2H), 3.53 - 3.41 (m, 3H), 3.20 - 3.11 (m, 2H), 2.90 - 2.84 (m, 1H), 2.82 - 2.71 (m, 1H), 2.25 - 2.17 (m, 2H), 1.97 (d, *J* = 14.1 Hz, 2H). LCMS (ESI) calcd for $C_{23}H_{24}Cl_2N_5O_3^+$ [M+H]$^+$: 488.13, found, 488.2.

**Example 95: preparation of 5-((4-(3,4-dichloropyridin-2-yl)piperidin-1-yl)methyl)-2-(2,4-dioxotetrahydropyrimidin-1(2H)-yl)isoindoline-1,3-dione (GT-06389)**

Referring to the method of Scheme 1, the target product GT-06389 was prepared as a white solid (38 mg, yield 62%). $^1$H NMR (400 MHz, DMSO-d6) δ 11.27 (s, 1H), 10.93 (s, 1H), 8.48 (d, *J* = 5.2 Hz, 1H), 8.34 (s, 1H), 8.20 (dd, *J* = 7.8, 1.2 Hz, 1H), 8.11 (d, *J* = 7.7 Hz, 1H), 7.66 (d, *J* = 5.2 Hz, 1H), 4.54 (s, 2H), 3.83 (t, *J* = 6.8 Hz, 2H), 3.49 - 3.44 (m, 3H), 3.21 - 3.06 (m, 2H), 2.86 (t, *J* = 6.8 Hz, 2H), 2.25 - 2.15 (m, 2H), 1.98 (d, *J* = 12.7 Hz, 2H). LCMS (ESI) calcd for $C_{23}H_{22}Cl_2N_5O_4^+$ [M+H]$^+$: 502.10, found, 502.1.

**Example 96: preparation of 1-(5-((4-(2,3-dichloropyridin-4-yl)piperidin-1-yl)methyl)-1-oxoisoindolin-2-yl)dihydropyrimidine-2,4(1H,3H)-dione (GT-06390)**

Referring to the method of Scheme 1, the target product GT-06390 was prepared as a white solid (39 mg, yield 63%). $^1$H NMR (400 MHz, DMSO-d6) δ 11.13 (s, 1H), 10.69 (s, 1H), 8.39 (d, *J* = 5.0 Hz, 1H), 7.93 - 7.85 (m, 2H), 7.80 (d, *J* = 7.8 Hz, 1H), 7.37 (d, *J* = 5.1 Hz, 1H), 4.82 (d, *J* = 16.5 Hz, 1H), 4.59 (d, *J* = 16.6 Hz, 1H), 4.46 (d, *J* = 4.7 Hz, 2H), 3.79 (t, *J* = 6.8 Hz, 2H), 3.48 (d, *J* = 11.3 Hz, 2H), 3.30 - 3.27 (m, 1H), 3.21 - 3.12 (m, 2H), 2.88 - 2.84 (m, 1H), 2.79 - 2.73 (m, 1H), 2.16 - 2.08 (m, 2H), 1.99 (d, *J* = 12.4 Hz, 2H). LCMS (ESI) calcd for $C_{23}H_{24}Cl_2N_5O_3^+$ [M+H]$^+$: 488.13, found, 488.2.

**Example 97: preparation of 5-((4-(2,3-dichloropyridin-4-yl)piperidin-1-yl)methyl)-2-(2,4-dioxotetrahydropyrimidin-1(2H)-yl)isoindoline-1,3-dione (GT-06391)**

Referring to the method of Scheme 1, the target product GT-06391 was prepared as a white solid (42 mg, yield 68%). $^1$H NMR (400 MHz, DMSO-d6) δ 11.48 (s, 1H), 10.93 (s, 1H), 8.39 (d, *J* = 5.0 Hz, 1H), 8.33 (s, 1H), 8.20 (d, *J* = 7.6 Hz, 1H), 8.11 (d, *J* = 7.7 Hz, 1H), 7.37 (d, *J* = 5.0 Hz, 1H), 4.56 (s, 2H), 3.83 (t, *J* = 6.8 Hz, 2H), 3.51 - 3.48 (m, 2H), 3.30 - 3.27(m, 1H), 3.21 - 3.13 (m, 2H), 2.87 (dd, *J* = 12.8, 6.1 Hz, 2H), 2.19 - 2.03 (m, 2H), 1.99 (d, *J* = 12.4 Hz, 2H). LCMS (ESI) calcd for $C_{23}H_{22}Cl_2N_5O_4^+$ [M+H]$^+$: 502.10, found, 502.2.

**Example 98: preparation of 1-(5-((3,4-dichloro-3',6'-dihydro-[2,4'-bipyridin]-1'(2'H)-yl)methyl)-1-oxoisoindolin-2-yl)dihydropyrimidine-2,4(1H,3H)-dione (GT-06392)**

Referring to the method of Scheme 1, the target product GT-06392 was prepared as a white solid (30 mg, yield 49%). $^1$H NMR (400 MHz, DMSO-d6) δ 11.18 (s, 1H), 10.69 (s, 1H), 8.50 (d, *J* = 5.2 Hz, 1H), 7.93 (s, 1H), 7.90 (d, *J* = 7.8 Hz, 1H), 7.84 (d, *J* = 7.9 Hz, 1H), 7.72 (d, *J* = 5.2 Hz, 1H), 6.20 (s, 1H), 4.83 (d, *J = 16.6* Hz, 1H), 4.61 - 4.54 (m, 3H), 3.81 - 3.78 (m, 3H), 3.66 - 3.63 (m, 1H), 3.30 - 3.24 (m, 1H), 3.01 - 2.97 (m, 1H), 2.86 (t, *J* = 7.8 Hz, 1H), 2.77 (t, *J* = 5.6 Hz, 1H), 2.71 (d, *J* = 19.2 Hz, 2H). LCMS (ESI) calcd for $C_{23}H_{22}Cl_2N_5O_3^+$ [M+H]$^+$: 486.11, found, 486.2.

**Example 99: preparation of 1-(5-((4,5-dichloro-3',6'-dihydro-[3,4'-bipyridin]-1'(2'H)-yl)methyl)-1-oxoisoindolin-2-yl)dihydropyrimidine-2,4(1H,3H)-dione (GT-06393)**

Referring to the method of Scheme 1, the target product GT-06393 was prepared as a white solid (21 mg, yield 34%). $^1$H NMR (400 MHz, DMSO-d6) δ 11.36 (s, 1H), 10.69 (s, 1H), 8.76 (s, 1H), 8.40 (s, 1H), 7.94 (s, 1H), 7.90 (d, *J* = 7.8 Hz, 1H), 7.85 (d, *J* = 8.0 Hz, 1H), 5.91 (s, 1H), 4.83 (d, *J* = 16.6 Hz, 1H), 4.64 - 4.48 (m, 3H), 3.80 - 3.71 (m, 3H), 3.63 - 3.57 (m, 1H), 3.31 - 3.22 (m, 1H), 3.00 - 2.82 (m, 2H), 2.77 *(t, J= 12.3* Hz, 2H), 2.60 (d, *J* = 17.2 Hz, 1H). LCMS (ESI) calcd for $C_{23}H_{22}Cl_2N_5O_3^+$ [M+H]$^+$: 486.11, found, 486.2.

**Example 100: preparation of 1-(5-((5-chloro-3',6'-dihydro-[3,4'-bipyridin]-1'(2'H)-yl)methyl)-1-oxoisoindolin-2-yl)dihydropyrimidine-2,4(1H,3H)-dione (GT-06394)**

Referring to the method of Scheme 1, the target product GT-06394 was prepared as a white solid (26 mg, yield

45%). $^{1}$H NMR (400 MHz, DMSO-d6) δ 11.39 (s, 1H), 10.69 (s, 1H), 8.68 (d, *J* = 1.9 Hz, 1H), 8.59 (d, *J* = 2.2 Hz, 1H), 8.06 (t, *J* = 2.1 Hz, 1H), 7.95 (s, 1H), 7.87 (d, *J* = 4.9 Hz, 2H), 6.40 (s, 1H), 4.82 (d, *J = 16.3* Hz, 1H), 4.61 - 4.53 (m, 3H), 3.87 - 3.78 (m, 4H), 3.66 - 3.62 (m, 1H), 3.31 - 3.22 (m, 1H), 2.96 - 2.75 (m, 4H). LCMS (ESI) calcd for $C_{23}H_{23}ClN_5O_3^+$ [M+H]$^+$: 452.15, found, 452.2.

**Example 101: preparation of 1-(5-((3-fluoro-3',6'-dihydro-[2,4'-bipyridin]-1'(2'H)-yl)methyl)-1-oxoisoindolin-2-yl)dihydropyrimidine-2,4(1H,3H)-dione (GT-06395)**

Referring to the method of Scheme 1, the target product GT-06395 was prepared as a white solid (34 mg, yield 61%). $^{1}$H NMR (400 MHz, DMSO-d6) δ 11.60 (s, 1H), 10.70 (s, 1H), 8.46 (d, *J* = 4.5 Hz, 1H), 7.98 (s, 1H), 7.88 (s, 2H), 7.77 (dd, *J* = 11.8, 7.9 Hz, 1H), 7.45 - 7.42 (m, 1H), 6.56 (s, 1H), 4.83 (d, *J* = 16.7 Hz, 1H), 4.67 - 4.51 (m, 3H), 3.91 - 3.75 (m, 4H), 3.65 - 3.62 (m, 1H), 3.35 - 3.19 (m, 1H), 3.12 - 2.97 (m, 1H), 2.98 - 2.83 (m, 2H), 2.80 - 2.74 (m, 1H). LCMS (ESI) calcd for $C_{23}H_{23}FN_5O_3^+$ [M+H]$^+$: 436.18, found, 436.2.

**Example 102: preparation of 1-(5-((4-(2,3-difluoropyridin-4-yl)piperidin-1-yl)methyl)-1-oxoisoindolin-2-yl)dihydropyrimidine-2,4(1H,3H)-dione (GT-06396)**

Referring to the method of Scheme 1, the target product GT-06396 was prepared as a white solid (27 mg, yield 47%). $^{1}$H NMR (400 MHz, DMSO-d6) δ 11.25 (s, 1H), 10.69 (s, 1H), 8.04 (d, *J* = 5.0 Hz, 1H), 7.92 (s, 1H), 7.88 (d, *J* = 7.8 Hz, 1H), 7.82 (d, *J* = 7.8 Hz, 1H), 7.29 (t, *J* = 4.8 Hz, 1H), 4.82 (d, *J* = 16.5 Hz, 1H), 4.59 (d, *J* = 16.6 Hz, 1H), 4.46 (d, *J* = 4.7 Hz, 2H), 3.80 (t, *J* = 6.8 Hz, 2H), 3.47 (d, *J* = 11.2 Hz, 2H), 3.25 (t, *J* = 12.3 Hz, 1H), 3.18 - 3.09 (m, 2H), 2.96 - 2.70 (m, 2H), 2.25 - 2.16 (m, 2H), 1.98 (d, *J* = 13.2 Hz, 2H). LCMS (ESI) calcd for $C_{23}H_{24}F_2N_5O_3^+$ [M+H]$^+$: 456.18, found, 456.2.

**Example 103: preparation of 1-(5-((2',3'-difluoro-3,6-dihydro-[4,4'-bipyridin]-1(2H)-yl)methyl)-1-oxoisoindolin-2-yl)dihydropyrimidine-2,4(1H,3H)-dione (GT-06397)**

Referring to the method of Scheme 1, the target product GT-06397 was prepared as a white solid (39 mg, yield 68%). $^{1}$H NMR (400 MHz, DMSO-d6) δ 11.18 (s, 1H), 10.69 (s, 1H), 8.05 (d, *J* = 5.1 Hz, 1H), 7.96 - 7.87 (m, 2H), 7.82 (d, *J* = 7.2 Hz, 1H), 7.41 (t, *J* = 5.1 Hz, 1H), 6.34 (s, 1H), 4.82 (d, *J* = 16.6 Hz, 1H), 4.61 - 4.57 (m, 3H), 3.94 - 3.83 (m, 2H), 3.80 (t, *J* = 6.9 Hz, 2H), 3.70 - 3.60 (m, 1H), 3.31 - 3.23 (m, 1H), 3.05 - 2.95 (m, 1H), 2.88 - 2.84 (m, 1H), 2.78 (t, *J* = 5.6 Hz, 1H), 2.73 - 2.64 (m, 2H). LCMS (ESI) calcd for $C_{23}H_{22}F_2N_5O_3^+$ [M+H]$^+$: 454.16, found, 454.2.

**Example 104: preparation of 1-(5-((4-(bis(3-fluorophenyl)methyl)piperazin-1-yl)methyl)-1-oxoisoindolin-2-yl)dihydropyrimidine-2,4(1H,3H)-dione (GT-07293)**

Referring to the method of Scheme 1, the target product GT-07293 was prepared as a white solid (9 mg, yield 50%). $^{1}$H NMR (400 MHz, DMSO-d6) δ 10.68 (s, 1H), 10.58 (s, 1H), 7.90 - 7.79 (m, 2H), 7.73 (d, *J* = 7.7 Hz, 1H), 7.40 - 7.35 (m, 2H), 7.29 - 7.25 (m, 4H), 7.07 (t, *J* = 7.8 Hz, 2H), 4.79 (d, *J* = 16.9 Hz, 1H), 4.65 - 4.52 (m, 2H), 4.45 (s, 2H), 3.78 (t, *J* = 6.9 Hz, 2H), 3.35 - 3.28 (m, 2H), 3.24 - 3.08 (m, 3H), 2.87 - 2.75 (m, 3H), 2.41 - 2.33 (m, 2H). LCMS (ESI) calcd for $C_{30}H_{30}F_2N_5O_3^+$ [M+H]$^+$: 546.23, found, 546.3.

**Example 105: preparation of 1-(5-((4-benzhydryl-1,4-diazepan-1-yl)methyl)-1-oxoisoindolin-2-yl)dihydropyrimidine-2,4(1H,3H)-dione (GT-07294)**

Referring to the method of Scheme 1, the target product GT-07294 was prepared as a white solid (12 mg, yield 40%). $^{1}$H NMR (400 MHz, DMSO-d6) δ 10.68 (s, 1H), 8.02 - 7.81 (m, 5H), 7.79 - 7.84 (m, 1H), 7.63 - 7.51 (m, 1H), 7.48 - 7.23 (m, 6H), 4.79 (d, *J* = 16.8 Hz, 1H), 4.56 (d, *J* = 16.7 Hz, 1H), 4.46 (s, 2H), 4.05 - 3.97 (m, 1H), 3.78 (t, *J* = 6.8 Hz, 2H), 3.40 - 3.31 (m, 9H), 3.31 - 3.17 (m, 2H), 2.90 - 2.74 (m, 2H). LCMS (ESI) calcd for $C_{31}H_{34}N_5O_3^+$ [M+H]$^+$: 524.27, found, 524.3.

**Example 106: preparation of 5-((4-benzhydryl-1,4-diazepan-1-yl)methyl)-2-(2,4-dioxotetrahydropyrimidin-1(2H)-yl)isoindoline-1,3-dione (GT-07295)**

Referring to the method of Scheme 1, the target product GT-07295 was prepared as a white solid (12 mg, yield 39%). $^{1}$H NMR (400 MHz, DMSO-d6) δ 10.92 (s, 1H), 8.26 (s, 1H), 8.13 (d, *J* = 6.6 Hz, 1H), 8.08 (d, *J* = 7.7 Hz, 1H), 8.01 - 7.95 (m, 1H), 7.91 - 7.83 (m, 1H), 7.65 - 7.52 (m, 1H), 7.50 - 7.20 (m, 6H), 4.68 - 4.46 (m, 1H), 3.81 (t, *J* = 6.7 Hz, 2H), 3.48 - 3.36 (m, 7H), 3.37 - 3.13 (m, 3H), 2.85 (t, *J* = 6.7 Hz, 2H), 2.08 - 2.01 (m, 2H). LCMS (ESI) calcd for $C_{31}H_{32}N_5O_4^+$ [M+H]$^+$: 538.24, found, 538.3.

**Example 107: preparation of 1-(5-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)dihydropyrimidine-2,4(1H,3H)-dione (GT-07329)**

Referring to the method of Scheme 1, the target product GT-07329 was prepared as a white solid (20 mg, yield 39%). $^1$H NMR (400 MHz, DMSO-d6) δ 10.68 (s, 1H), 7.99 - 7.68 (m, 3H), 7.46 (d, *J* = 8.0 Hz, 2H), 7.18 (d, *J* = 8.2 Hz, 2H), 4.78 (d, *J* = 16.3 Hz, 1H), 4.55 (d, *J* = 14.2 Hz, 1H), 4.47 - 4.31 (m, 1H), 4.31 - 3.94 (m, 3H), 3.78 (t, *J* = 6.9 Hz, 2H), 3.68 - 3.59 (m, 2H), 3.34 - 3.27 (m, 5H), 2.94 - 2.72 (m, 3H), 2.30 - 2.14 (m, 1H), 2.12 - 1.99 (m, 3H), 1.47 (s, 2H), 0.96 (s, 6H). LCMS (ESI) calcd for $C_{33}H_{39}ClN_5O_3^+$ [M+H]$^+$: 588.27, found, 588.3.

**Example 108: preparation of 1-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-oxoisoindolin-2-yl)dihydropyrimidine-2,4(1H,3H)-dione (GT-07330)**

Referring to the method of Scheme 1, the target product GT-07330 was prepared as a white solid (22 mg, yield 53%). $^1$H NMR (400 MHz, DMSO-d6) δ 10.69 (s, 1H), 9.89 (s, 1H), 7.86 (d, *J* = 7.5 Hz, 2H), 7.77 (d, *J* = 8.0 Hz, 1H), 7.37 (d, *J* = 8.3 Hz, 2H), 7.05 (d, *J* = 8.2 Hz, 2H), 4.79 (d, *J* = 16.0 Hz, 1H), 4.56 (d, *J* = 16.7 Hz, 1H), 4.27 (d, *J* = 4.9 Hz, 2H), 3.78 (t, *J* = 6.8 Hz, 4H), 2.84 - 2.72 (m, 4H), 2.61 - 2.57 (m, 2H), 2.33 - 2.17 (m, 5H), 2.02 - 1.89 (m, 5H), 1.42 (t, *J* = 6.2 Hz, 2H), 0.94 (s, 6H). LCMS (ESI) calcd for $C_{34}H_{41}ClN_5O_3^+$ [M+H]$^+$: 602.29, found, 602.3.

**Example 109: preparation of 1-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)dihydropyrimidine-2,4(1H,3H)-dione (GT-07331)**

Referring to the method of Scheme 1, the target product GT-07331 was prepared as a white solid (16 mg, yield 39%). $^1$H NMR (400 MHz, DMSO-d6) δ 10.69 (s, 1H), 8.11 - 7.55 (m, 3H), 7.45 (d, *J* = 6.9 Hz, 2H), 7.19 (d, *J* = 7.9 Hz, 2H), 4.87 - 4.71 (m, 1H), 4.67 - 4.28 (m, 2H), 3.91 - 3.72 (m, 4H), 3.35 - 3.21 (m, 6H), 2.92 - 2.76 (m, 3H), 2.40 - 2.20 (m, 3H), 2.12 - 1.97 (m, 3H), 1.46 (s, 2H), 0.96 (s, 6H). LCMS (ESI) calcd for $C_{34}H_{41}ClN_5O_3^+$ [M+H]$^+$: 602.29, found, 602.3.

**Example 110: preparation of 1-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-1-oxoisoindolin-2-yl)dihydropyrimidine-2,4(1H,3H)-dione (GT-07296)**

Referring to the method of Scheme 1, the target product GT-07296 was prepared as a white solid (17 mg, yield 42%). $^1$H NMR (400 MHz, DMSO-d6) δ 10.66 (s, 1H), 7.73 (d, *J* = 7.5 Hz, 1H), 7.55 (s, 1H), 7.46 (d, *J* = 7.7 Hz, 1H), 7.42 (d, *J* = 6.0 Hz, 2H), 7.09 (d, *J* = 7.2 Hz, 2H), 4.73 (d, *J* = 16.4 Hz, 1H), 4.50 (d, *J* = 16.6 Hz, 1H), 4.25 - 3.87 (m, 2H), 3.79 - 3.74 (m, 2H), 3.70 - 3.49 (m, 4H), 3.25 - 3.00 (m, 2H), 2.95 - 2.69 (m, 4H), 2.44 - 2.17 (m, 2H), 2.12 - 1.95 (m, 3H), 1.44 (s, 2H), 0.94 (s, 6H). LCMS (ESI) calcd for $C_{33}H_{38}ClF_3N_5O_3^+$ [M+H]$^+$: 644.26, found, 644.3.

**Example 111: preparation of 1-(5-((4-((4'-fluoro-3,4,5,6-tetrahydro-[1,1'-biphenyl]-2-yl)methyl)piperazin-1-yl)methyl)-1-oxoisoindolin-2-yl)dihydropyrimidine-2,4(1H,3H)-dione (GT-07297)**

Referring to the method of Scheme 1, the target product GT-07297 was prepared as a white solid (24 mg, yield 55%). $^1$H NMR (400 MHz, DMSO-d6) δ 10.68 (s, 1H), 7.80 (s, 1H), 7.74 - 7.53 (m, 2H), 7.24 - 7.06 (m, 4H), 4.77 (d, *J* = 16.4 Hz, 1H), 4.53 (d, *J* = 16.6 Hz, 1H), 3.78 (t, *J* = 7.3 Hz, 2H), 3.48 - 3.31 (m, 10H), 2.96 - 2.81 (m, 2H), 2.79 - 2.74 (m, 2H), 2.23 (s, 4H), 1.67 (s, 4H). LCMS (ESI) calcd for $C_{30}H_{35}FN_5O_3^+$ [M+H]$^+$: 532.27, found, 532.3.

**Example 112: preparation of 1-(5-((4-(4'-fluoro-3,4,5,6-tetrahydro-[1,1'-biphenyl]-2-carbonyl)piperazin-1-yl)methyl)-1-oxoisoindolin-2-yl)dihydropyrimidine-2,4(1H,3H)-dione (GT-07299)**

Referring to the method of Scheme 1, the target product GT-07299 was prepared as a white solid (27 mg, yield 62%). $^1$H NMR (400 MHz, DMSO-d6) δ 10.69 (s, 1H), 7.85 (d, *J* = 7.8 Hz, 1H), 7.84 - 7.63 (m, 2H), 7.26 - 7.06 (m, 4H), 4.79 (d, *J* = 15.4 Hz, 1H), 4.56 (d, *J* = 15.4 Hz, 1H), 4.40 (s, 1H), 4.34 - 4.21 (m, 2H), 3.80 - 3.74 (m, 3H), 3.21 - 3.07 (m, 2H), 3.01 - 2.71 (m, 4H), 2.69 - 2.56 (m, 2H), 2.46 - 2.28 (m, 1H), 2.18 - 1.97 (m, 3H), 1.72 (s, 4H). LCMS (ESI) calcd for $C_{30}H_{33}FN_5O_4^+$ [M+H]$^+$: 546.25, found, 546.3.

**Example 113: preparation of 1-(5-((3-(4'-fluoro-3,4,5,6-tetrahydro-[1,1'-biphenyl]-2-carbonyl)-3,6-diazabicyclo [3.1.1]heptan-6-yl)methyl)-1-oxoisoindolin-2-yl)dihydropyrimidine-2,4(1H,3H)-dione (GT-07376)**

Referring to the method of Scheme 1, the target product GT-07376 was prepared as a white solid (26 mg, yield 93%). $^1$H NMR (400 MHz, DMSO-d6) δ 10.76 (s, 1H), 10.69 (s, 1H), 7.85 (d, *J* = 7.7 Hz, 1H), 7.77 - 7.67 (m, 2H), 7.21 - 7.11 (m, 4H), 4.79 (d, *J* = 15.4 Hz, 1H), 4.56 (d, *J* = 15.8 Hz, 1H), 4.39 (s, 1H), 4.36 - 4.14 (m, 2H), 3.80 - 3.77 (m, 3H), 3.29 - 3.01 (m, 2H), 2.95 - 2.73 (m, 4H), 2.64 - 2.55 (m, 4H), 2.15 - 2.02 (m, 2H), 1.68 (s, 4H). LCMS (ESI) calcd for $C_{30}H_{33}FN_5O_4^+$ $[M+H]^+$: 546.25, found, 546.3.

**Example 114: preparation of 1-(5-((4-((4-(4-chlorophenyl)-6,6-dimethyl-5,6-dihydro-2H-pyran-3-yl)methyl)piperazin-1-yl)methyl)-1-oxoisoindolin-2-yl)dihydropyrimidine-2,4(1H,3H)-dione (GT-07332)**

Referring to the method of Scheme 1, the target product GT-07332 was prepared as a white solid (29 mg, yield 68%). $^1$H NMR (400 MHz, DMSO-d6) δ 10.69 (s, 1H), 7.83 (d, *J* = 7.3 Hz, 1H), 7.77 (brs, 1H), 7.68 (brs, 1H), 7.44 (d, *J* = 8.2 Hz, 2H), 7.18 (d, *J* = 8.0 Hz, 2H), 4.78 (d, *J* = 16.2 Hz, 1H), 4.55 (d, *J* = 16.5 Hz, 1H), 4.48 - 4.08 (m, 4H), 3.78 (t, *J* = 7.1 Hz, 2H), 3.47 - 3.42 (m, 3H), 3.36 - 2.98 (m, 6H), 2.94 - 2.72 (m, 3H), 2.19 (s, 2H), 1.21 (s, 6H). LCMS (ESI) calcd for $C_{31}H_{37}ClN_5O_4^+$ $[M+H]^+$: 578.25, found, 578.3.

**Example 115: preparation of 5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-2-(2,4-dioxotetrahydropyrimidin-1(2H)-yl)isoindoline-1,3-dione (GT-07333)**

Referring to the method of Scheme 1, the target product GT-07333 was prepared as a white solid (13 mg, yield 31%). $^1$H NMR (400 MHz, DMSO-d6) δ 10.93 (s, 1H), 9.80 (s, 1H), 8.26 (s, 1H), 8.15 (d, *J* = 8.1 Hz, 1H), 8.10 (d, *J* = 7.7 Hz, 1H), 7.37 (d, *J* = 8.3 Hz, 2H), 7.05 (d, *J* = 8.4 Hz, 2H), 4.37 (d, *J* = 5.2 Hz, 2H), 3.84 - 3.80 (m, 4H), 2.86 (t, *J* = 6.8 Hz, 2H), 2.79 (s, 2H), 2.60 (d, *J* = 12.9 Hz, 2H), 2.33 - 2.18 (m, 4H), 2.18 - 2.13 (m, 2H), 1.99 - 1.93 (m, 4H), 1.42 (t, *J* = 6.1 Hz, 2H), 0.94 (s, 6H). LCMS (ESI) calcd for $C_{34}H_{39}ClN_5O_4^+$ $[M+H]^+$: 616.27, found, 616.3.

**Example 116: preparation of 5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-2-(2,4-dioxotetrahydropyrimidin-1(2H)-yl)isoindoline-1,3-dione (GT-07300)**

Referring to the method of Scheme 1, the target product GT-07300 was prepared as a white solid (26 mg, yield 61%). $^1$H NMR (400 MHz, DMSO-d6) δ 10.92 (s, 1H), 8.20 - 7.83 (m, 3H), 7.51 - 7.37 (m, 2H), 7.19 (d, *J* = 4.6 Hz, 2H), 3.83 (d, *J* = 6.7 Hz, 2H), 3.79 - 3.66 (m, 2H), 3.35- 3.27 (m, 9H), 2.86 (t, *J* = 6.6 Hz, 2H), 2.33 - 2.20 (m, 3H), 2.14 - 1.96 (m, 3H), 1.51 - 1.43 (m, 2H), 0.96 (s, 6H). LCMS (ESI) calcd for $C_{34}H_{39}ClN_5O_4^+$ $[M+H]^+$: 616.27, found, 616.3.

**Example 117: preparation of 5-((4-((4'-chloro-5,5-dimethyl-3,4,5,6-tetrahydro-[1,1'-biphenyl]-2-yl)methyl)-2-(trifluoromethyl)piperazin-1-yl)methyl)-2-(2,4-dioxotetrahydropyrimidin-1(2H)-yl)isoindoline-1,3-dione (GT-07301)**

Referring to the method of Scheme 1, the target product GT-07301 was prepared as a white solid (12 mg, yield 29%). $^1$H NMR (400 MHz, DMSO-d6) δ 10.90 (s, 1H), 7.99 - 7.93 (m, 1H), 7.89 (s, 1H), 7.85 (d, *J* = 6.8 Hz, 1H), 7.41 (brs, 2H), 7.09 (d, *J* = 6.9 Hz, 2H), 4.27 - 3.95 (m, 2H), 3.80 (t, *J* = 6.8 Hz, 3H), 3.45 - 3.31 (m, 7H), 3.26 - 3.06 (m, 1H), 2.86 - 2.78 (m, 4H), 2.40 - 2.23 (m, 2H), 2.01 (brs, 3H), 1.43 (brs, 2H), 0.95 (s, 6H). LCMS (ESI) calcd for $C_{33}H_{36}ClF_3N_5O_4^+$ $[M+H]^+$: 658.24, found, 658.3.

**Example 118: preparation of 2-(2,4-dioxotetrahydropyrimidin-1(2H)-yl)-5-((4-((4'-fluoro-3,4,5,6-tetrahydro-[1,1'-biphenyl]-2-yl)methyl)piperazin-1-yl)methyl)isoindoline-1,3-dione (GT-07302)**

Referring to the method of Scheme 1, the target product GT-07302 was prepared as a white solid (31 mg, yield 69%). $^1$H NMR (400 MHz, DMSO-d6) δ 10.91 (s, 1H), 8.10 (s, 1H), 8.04 - 7.97 (m, 2H), 7.24 - 7.11 (m, 4H), 4.42 - 3.98 (m, 2H), 3.81 (t, *J* = 6.8 Hz, 2H), 3.47 - 3.28 (m, 5H), 3.18 - 2.97 (m, 4H), 2.87 - 2.83 (m, 3H), 2.24 (s, 4H), 1.68 (s, 4H). LCMS (ESI) calcd for $C_{30}H_{33}FN_5O_4^+$ $[M+H]^+$: 546.25, found, 546.3.

**Example 119: preparation of 2-(2,4-dioxotetrahydropyrimidin-1(2H)-yl)-5-((4-(4'-fluoro-3,4,5,6-tetrahydro-[1,1'-biphenyl]-2-carbonyl)piperazin-1-yl)methyl)isoindoline-1,3-dione (GT-07303)**

Referring to the method of Scheme 1, the target product GT-07303 was prepared as a white solid (25 mg, yield

56%). $^1H$ NMR (400 MHz, DMSO-d6) δ 10.93 (s, 1H), 8.20 - 8.16 (m, 1H), 8.08 - 8.00 (m, 2H), 7.27 - 7.18 (m, 2H), 7.12 - 7.05 (m, 2H), 4.57 - 4.45 (m, 1H), 4.39 - 4.26 (m, 2H), 3.82 (t, *J* = 6.7 Hz, 2H), 3.77 - 3.71 (m, 1H), 3.13 - 2.94 (m, 3H), 2.86 (t, *J* = 6.7 Hz, 2H), 2.69 - 2.52 (m, 3H), 2.42 - 2.30 (m, 1H), 2.17 - 2.03 (m, 3H), 1.77 - 1.60 (m, 5H). LCMS (ESI) calcd for $C_{30}H_{31}FN_5O_5^+$ [M+H]$^+$: 560.23, found, 560.3.

**Example 120: preparation of 2-(2,4-dioxotetrahydropyrimidin-1(2H)-yl)-5-((3-(4'-fluoro-3,4,5,6-tetrahydro-[1,1'-biphenyl]-2-carbonyl)-3,6-diazabicyclo[3.1.1]heptan-6-yl)methyl)isoindoline-1,3-dione (GT-07377)**

Referring to the method of Scheme 1, the target product GT-07377 was prepared as a white solid (19 mg, yield 66%). $^1H$ NMR (400 MHz, DMSO-d6) δ 10.92 (s, 1H), 8.15 - 7.99 (m, 3H), 7.22 (brs, 2H), 7.13 (brs, 2H), 4.57 - 4.47 (m, 1H), 4.40 - 4.17 (m, 1H), 3.83 - 3.74 (m, 3H), 3.31 - 3.22 (m, 4H), 3.17 - 2.92 (m, 2H), 2.86 (t, *J* = 6.8 Hz, 2H), 2.65 - 2.57 (m, 2H), 2.43 - 2.28 (m, 1H), 2.19 - 2.00 (m, 2H), 1.72 - 1.64 (m, 4H). LCMS (ESI) calcd for $C_{30}H_{31}FN_5O_5^+$ [M+H]$^+$: 560.23, found, 560.3.

**Example 121: preparation of 5-((4-((4-(4-chlorophenyl)-6,6-dimethyl-5,6-dihydro-2H-pyran-3-yl)methyl)piperazin-1-yl)methyl)-2-(2,4-dioxotetrahydropyrimidin-1(2H)-yl)isoindoline-1,3-dione (GT-07304)**

Referring to the method of Scheme 1, the target product GT-07304 was prepared as a white solid (32 mg, yield 74%). $^1H$ NMR (400 MHz, DMSO-d6) δ 10.92 (s, 1H), 8.13 (s, 1H), 8.05 - 7.99 (m, 2H), 7.44 (d, *J* = 8.2 Hz, 2H), 7.19 (d, *J* = 8.1 Hz, 2H), 4.42 - 4.18 (m, 3H), 3.81 (t, *J* = 6.8 Hz, 2H), 3.52 - 3.44 (m, 5H), 3.36 - 2.93 (m, 6H), 2.85 (t, *J* = 6.8 Hz, 2H), 2.20 (s, 2H), 1.21 (s, 6H). LCMS (ESI) calcd for $C_{31}H_{35}ClN_5O_5^+$ [M+H]$^+$: 592.23, found, 592.3.

**Example 122: preparation of 5-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,4-dioxotetrahydropyrimidin-1(2H)-yl)isoindoline-1,3-dione (GT-07334)**

Referring to the method of Scheme 1, the target product GT-07334 was prepared as a white solid (26 mg, yield 50%). $^1H$ NMR (400 MHz, DMSO-d6) δ 10.92 (s, 1H), 8.20 (s, 1H), 8.05 - 7.97 (m, 2H), 7.46 (d, *J* = 7.9 Hz, 2H), 7.18 (d, *J* = 7.9 Hz, 2H), 4.54 - 4.33 (m, 1H), 4.28 - 3.96 (m, 2H), 3.82 (t, *J* = 6.7 Hz, 2H), 3.61 (brs, 2H), 3.31 - 3.29 (m, 5H), 3.26 - 3.02 (m, 2H), 2.85 (t, *J* = 6.7 Hz, 2H), 2.48 - 2.37 (m, 1H), 2.30 - 2.15 (m, 1H), 2.13 - 2.01 (m, 2H), 1.47 (s, 2H), 0.96 (s, 6H). LCMS (ESI) calcd for $C_{33}H_{37}ClN_5O_4^+$ [M+H]$^+$: 602.25, found, 602.3.

**Example 123: preparation of 1-(5-((4-(3-methylthiophen-2-yl)piperidin-1-yl)methyl)-1-oxoisoindolin-2-yl)dihydropyrimidine-2,4(1H,3H)-dione (GT-07305)**

Referring to the method of Scheme 1, the target product GT-07305 was prepared as a white solid (10 mg, yield 15%). $^1H$ NMR (400 MHz, DMSO-d6) δ 10.70 (s, 1H), 10.49 (s, 1H), 7.89 (d, *J* = 7.8 Hz, 1H), 7.86 (s, 1H), 7.76 (d, *J* = 7.8 Hz, 1H), 7.26 (d, *J* = 5.0 Hz, 1H), 6.82 (d, *J* = 5.0 Hz, 1H), 4.82 (d, *J* = 16.1 Hz, 1H), 4.59 (d, *J* = 16.8 Hz, 1H), 4.45 (d, *J* = 4.5 Hz, 2H), 3.79 (t, *J* = 6.8 Hz, 2H), 3.44 (d, *J* = 11.9 Hz, 2H), 3.19 - 3.01 (m, 3H), 2.91 - 2.74 (m, 2H), 2.14 (s, 3H), 2.01 - 1.90 (m, 4H). LCMS (ESI) calcd for $C_{23}H_{27}N_4O_3S^+$ [M+H]$^+$: 439.18, found, 439.3.

**Example 124: preparation of 2-(2,4-dioxotetrahydropyrimidin-1(2H)-yl)-5-((4-(3-methylthiophen-2-yl)piperidin-1-yl)methyl)isoindoline-1,3-dione (GT-07306)**

Referring to the method of Scheme 1, the target product GT-07306 was prepared as a white solid (21 mg, yield 31%). $^1H$ NMR (400 MHz, DMSO-d6) δ 10.93 (s, 1H), 10.55 (s, 1H), 8.28 (s, 1H), 8.13 (s, 2H), 7.27 (d, *J* = 5.0 Hz, 1H), 6.83 (d, *J* = 5.0 Hz, 1H), 4.55 (s, 2H), 3.83 (t, *J* = 6.8 Hz, 2H), 3.46 (d, *J* = 11.1 Hz, 2H), 3.19 - 3.06 (m, 3H), 2.86 (t, *J* = 6.8 Hz, 2H), 2.14 (s, 3H), 2.07 - 1.89 (m, 4H). LCMS (ESI) calcd for $C_{23}H_{25}N_4O_4S^+$ [M+H]$^+$: 453.16, found, 453.3.

**Example 125: preparation of 1-(1-oxo-5-((4-(thiophen-3-yl)piperidin-1-yl)methyl)isoindolin-2-yl)dihydropyrimidine-2,4(1H,3H)-dione (GT-07371)**

Referring to the method of Scheme 1, the target product GT-07371 was prepared as a white solid (11 mg, yield 24%). $^1H$ NMR (400 MHz, DMSO-d6) δ 10.74 (s, 1H), 10.69 (s, 1H), 7.92 - 7.86 (m, 2H), 7.79 (d, *J* = 7.9 Hz, 1H), 7.50 (dd, *J* = 5.0, 2.9 Hz, 1H), 7.22 (d, *J* = 2.6 Hz, 1H), 7.04 (dd, *J* = 5.0, 1.2 Hz, 1H), 4.82 (d, *J* = 16.4 Hz, 1H), 4.59 (d, *J* = 16.5 Hz, 1H), 4.45 (d, *J* = 5.0 Hz, 2H), 3.79 (t, *J* = 6.9 Hz, 2H), 3.10 - 3.01 (m, 3H), 2.90 - 2.78 (m, 4H), 2.07 (d, *J* = 13.3 Hz, 2H), 2.00 - 1.91 (m, 2H). LCMS (ESI) calcd for $C_{22}H_{25}N_4O_3S^+$ [M+H]$^+$: 425.16, found, 425.2.

**Example 126: preparation of 1-(5-((4-(4-methylthiophen-3-yl)piperidin-1-yl)methyl)-1-oxoisoindolin-2-yl)dihydropyrimidine-2,4(1H,3H)-dione (GT-07372)**

Referring to the method of Scheme 1, the target product GT-07372 was prepared as a white solid (13 mg, yield 30%). $^1H$ NMR (400 MHz, DMSO-d6) δ 10.69 (s, 1H), 10.64 (s, 1H), 7.90 - 7.86 (m, 2H), 7.78 (d, *J* = 8.2 Hz, 1H), 7.15 - 7.09 (m, 2H), 4.82 (d, *J* = 16.7 Hz, 1H), 4.59 (d, *J* = 16.8 Hz, 1H), 4.45 (d, *J* = 4.6 Hz, 2H), 3.79 (t, *J* = 6.8 Hz, 2H), 3.18 - 2.99 (m, 2H), 2.86 - 2.75 (m, 3H), 2.50 - 2.40 (m, 2H), 2.17 (s, 3H), 2.04 - 1.87 (m, 4H). LCMS (ESI) calcd for $C_{23}H_{27}N_4O_3S^+$ [M+H]$^+$: 439.18, found, 439.2.

**Example 127: preparation of 1-(5-((4-(5-methylthiophen-3-yl)piperidin-1-yl)methyl)-1-oxoisoindolin-2-yl)dihydropyrimidine-2,4(1H,3H)-dione (GT-07373)**

Referring to the method of Scheme 1, the target product GT-07373 was prepared as a white solid (12 mg, yield 27%). $^1H$ NMR (400 MHz, DMSO-d6) δ 10.91 (s, 1H), 10.69 (s, 1H), 7.91 (s, 1H), 7.87 (d, *J* = 7.8 Hz, 1H), 7.80 (d, *J* = 7.8 Hz, 1H), 6.91 (s, 1H), 6.71 (s, 1H), 4.82 (d, *J* = 16.6 Hz, 1H), 4.58 (d, *J* = 16.8 Hz, 1H), 4.44 (d, *J* = 5.0 Hz, 2H), 3.79 (t, *J* = 6.8 Hz, 2H), 3.41 (d, *J* = 11.5 Hz, 2H), 3.07 - 2.98 (m, 2H), 2.87 - 2.73 (m, 3H), 2.40 (s, 3H), 2,04 - 1.92 (m, 4H). LCMS (ESI) calcd for $C_{23}H_{27}N_4O_3S^+$ [M+H]$^+$: 439.18, found, 439.2.

**Example 128: preparation of 1-(5-((4-(furan-3-yl)piperidin-1-yl)methyl)-1-oxoisoindolin-2-yl)dihydropyrimidine-2,4(1H,3H)-dione (GT-07375)**

Referring to the method of Scheme 1, the target product GT-07375 was prepared as a white solid (12 mg, yield 25%). $^1H$ NMR (400 MHz, DMSO-d6) δ 10.69 (s, 1H), 10.63 (s, 1H), 7.90 - 7.85 (m, 2H), 7.78 (d, *J* = 8.0 Hz, 1H), 7.60 (t, *J* = 1.5 Hz, 1H), 7.49 (s, 1H), 6.42 (d, *J* = 0.9 Hz, 1H), 4.81 (d, *J* = 16.5 Hz, 1H), 4.58 (d, *J* = 17.0 Hz, 1H), 4.44 (d, *J* = 4.4 Hz, 2H), 3.79 (t, *J* = 6.7 Hz, 2H), 3.41 (d, *J* = 11.0 Hz, 2H), 3.14 - 2.97 (m, 2H), 2.93 - 2.75 (m, 2H), 2.72 - 2.65 (m, 1H), 2.04 (d, *J* = 13.7 Hz, 2H), 1.89 - 1.81 (m, 2H). LCMS (ESI) calcd for $C_{22}H_{25}N_4O_4^+$ [M+H]$^+$: 409.19, found, 409.2.

**Example 129: preparation of 2-(2,4-dioxotetrahydropyrimidin-1(2H)-yl)-5-((4-(thiophen-3-yl)piperidin-1-yl)methyl)isoindoline-1,3-dione (GT-07420)**

Referring to the method of Scheme 1, the target product GT-07420 was prepared as a white solid (16 mg, yield 34%). $^1H$ NMR (400 MHz, DMSO-d6) δ 11.01 (s, 1H), 10.93 (s, 1H), 8.32 (s, 1H), 8.19 (d, *J* = 7.6 Hz, 1H), 8.11 (d, J= 7.6 Hz, 1H), 7.50 (dd, *J* = 4.8, 2.9 Hz, 1H), 7.22 (s, 1H), 7.04 (d, *J* = 4.2 Hz, 1H), 4.61 - 4.48 (m, 2H), 3.68 - 3.63 (m, 1H), 3.44 - 3.42 (m, 3H), 3.13 - 3.03 (m, 3H), 2.75 (t, *J* = 6.0 Hz, 1H), 2.14 - 2.07 (m, 3H), 2.02 - 1.90 (m, 2H). LCMS (ESI) calcd for $C_{22}H_{23}N_4O_4S^+$ [M+H]$^+$: 439.14, found, 439.2.

**Example 130: preparation of 2-(2,4-dioxotetrahydropyrimidin-1(2H)-yl)-5-((4-(5-methylthiophen-3-yl)piperidin-1-yl)methyl)isoindoline-1,3-dione (GT-07421)**

Referring to the method of Scheme 1, the target product GT-07421 was prepared as a white solid (19 mg, yield 42%). $^1H$ NMR (400 MHz, DMSO-d6) δ 10.93 (s, 1H), 10.75 (s, 1H), 8.30 (s, 1H), 8.16 (d, *J* = 7.4 Hz, 1H), 8.11 (d, *J*= 7.5 Hz, 1H), 6.92 (s, 1H), 6.71 (s, 1H), 4.60 - 4.49 (m, 2H), 3.71 - 3.59 (m, 1H), 3.49 - 3.39 (m, 2H), 3.13 - 2.98 (m, 3H), 2.79 - 2.73 (m, 2H), 2.40 (s, 3H), 2.09 - 2.01 (m, 3H), 1.94 - 1.83 (m, 2H). LCMS (ESI) calcd for $C_{23}H_{25}N_4O_4S^+$ [M+H]$^+$: 453.16, found, 453.2.

**Example 131: preparation of 2-(2,4-dioxotetrahydropyrimidin-1(2H)-yl)-5-((4-(furan-3-yl)piperidin-1-yl)methyl)isoindoline-1,3-dione (GT-07422)**

Referring to the method of Scheme 1, the target product GT-07422 was prepared as a white solid (25 mg, yield 51%). $^1H$ NMR (400 MHz, DMSO-d6) δ 11.08 (s, 1H), 10.93 (s, 1H), 8.33 (s, 1H), 8.19 (d, *J* = 7.8 Hz, 1H), 8.10 (d, *J* = 7.7 Hz, 1H), 7.60 (s, 1H), 7.49 (s, 1H), 6.42 (s, 1H), 4.59 - 4.50 (m, 2H), 4.47 - 4.36 (m, 1H), 3.68 - 3.64 (m, 1H), 3.28 - 3.17 (m, 1H), 3.14 - 3.00 (m, 3H), 2.75 (t, *J* = 6.1 Hz, 1H), 2.71 - 2.67 (m, 1H), 2.10 - 2.01 (m, 3H), 1.92 - 1.85 (m, 2H). LCMS (ESI) calcd for $C_{22}H_{23}N_4O_5^+$ [M+H]$^+$: 423.17, found, 423.2.

**Example 132: preparation of 2-(2,4-dioxotetrahydropyrimidin-1(2H)-yl)-5-((4-(5-methylthiophen-3-yl)-3,6-dihydropyridin-1(2H)-yl)methyl)isoindoline-1,3-dione (GT-07423)**

Referring to the method of Scheme 1, the target product GT-07423 was prepared as a white solid (20 mg, yield

44%). $^1$H NMR (400 MHz, DMSO) δ 11.11 (s, 1H), 10.93 (s, 1H), 8.33 (s, 1H), 8.19 (d, *J* = 6.8 Hz, 1H), 8.11 (d, *J* = 7.7 Hz, 1H), 7.27 (s, 1H), 7.05 (s, 1H), 6.05 (s, 1H), 4.71 - 4.55 (m, 2H), 3.83 (t, *J* = 6.8 Hz, 2H), 3.80 - 3.55 (m, 3H), 3.25 - 3.18 (m, 1H), 2.86 (t, *J* = 6.7 Hz, 2H), 2.77 - 2.73 (m, 2H), 2.42 (s, 3H). LCMS (ESI) calcd for $C_{23}H_{23}N_4O_4S^+$ [M+H]$^+$: 451.14, found, 451.2.

**Example 133: preparation of 2-(2,4-dioxotetrahydropyrimidin-1(2H)-yl)-5-((4-(furan-3-yl)-3,6-dihydropyridin-1(2H)-yl)methyl)isoindoline-1,3-dione (GT-07442)**

Referring to the method of Scheme 1, the target product GT-07442 was prepared as a white solid (23 mg, yield 38%). $^1$H NMR (400 MHz, DMSO) δ 11.14 (s, 1H), 10.93 (s, 1H), 8.33 (s, 1H), 8.20 (d, *J* = 5.5 Hz, 1H), 8.11 (d, *J* = 7.9 Hz, 1H), 7.83 (s, 1H), 7.67 (s, 1H), 6.75 (s, 1H), 5.98 (s, 1H), 4.70 - 4.57 (m, 2H), 3.83 (t, *J* = 6.8 Hz, 2H), 3.76 - 3.71 (m, 2H), 3.67 - 3.55 (m, 1H), 3.26 - 3.18 (m, 1H), 2.87 (t, *J* = 6.7 Hz, 2H), 2.77 - 2.73 (m, 1H), 2.63 - 2.58 (m, 1H). LCMS (ESI) calcd for $C_{22}H_{21}N_4O_5^+$ [M+H]$^+$: 421.15, found, 421.1.

**Example 134: preparation of 2-(2,4-dioxotetrahydropyrimidin-1(2H)-yl)-5-((4-(thiophen-2-yl)piperidin-1-yl) methyl)isoindoline-1,3-dione (GT-07424)**

Referring to the method of Scheme 1, the target product GT-07424 was prepared as a white solid (25 mg, yield 54%). $^1$H NMR (400 MHz, DMSO-d6) δ 11.11 (s, 1H), 10.93 (s, 1H), 8.33 (s, 1H), 8.19 (d, *J* = 7.7 Hz, 1H), 8.11 (d, *J* = 7.6 Hz, 1H), 7.38 (dd, *J* = 5.1, 0.9 Hz, 1H), 6.97 (dd, *J* = 5.0, 3.5 Hz, 1H), 6.90 (d, *J* = 3.4 Hz, 1H), 4.55 (brs, 2H), 3.49 - 3.42 (m, 2H), 3.40 - 3.37 (m, 2H), 3.16 - 3.02 (m, 3H), 2.25 - 2.08 (m, 3H), 2.04 - 1.98 (m, 2H). LCMS (ESI) calcd for $C_{22}H_{23}N_4O_4S^+$ [M+H]$^+$: 439.14, found, 439.2.

**Example 135: preparation of 1-(5-((4-(5-methylthiophen-2-yl)piperidin-1-yl)methyl)-1-oxoisoindolin-2-yl)dihydropyrimidine-2,4(1H,3H)-dione (GT-07425)**

Referring to the method of Scheme 1, the target product GT-07425 was prepared as a white solid (21 mg, yield 48%). $^1$H NMR (400 MHz, DMSO-d6) δ 10.78 (s, 1H), 10.69 (s, 1H), 7.88 (d, *J* = 8.1 Hz, 2H), 7.78 (d, *J* = 7.7 Hz, 1H), 6.65 (d, *J* = 3.5 Hz, 1H), 6.62 (dd, *J* = 3.4, 1.1 Hz, 1H), 4.81 (d, *J* = 16.4 Hz, 1H), 4.58 (d, *J* = 16.6 Hz, 1H), 4.44 (d, *J* = 5.0 Hz, 2H), 3.79 (t, *J* = 6.9 Hz, 2H), 3.45 - 3.40 (m, 2H), 3.14 - 2.96 (m, 3H), 2.90 - 2.85 (m, 1H), 2.79 - 2.74 (m, 1H), 2.38 (s, 3H), 2.12 - 2.03 (m, 2H), 2.01 - 1.94 (m, 2H). LCMS (ESI) calcd for $C_{23}H_{27}N_4O_3S^+$ [M+H]$^+$: 439.18, found, 439.2.

**Example 136: preparation of 1-(5-((6-(2,3-dichlorophenyl)-3,6-diazabicyclo[3.1.1]heptan-3-yl)methyl)-1-oxoisoindolin-2-yl)dihydropyrimidine-2,4(1H,3H)-dione (GT-07426)**

Referring to the method of Scheme 1, the target product GT-07426 was prepared as a white solid (8 mg, yield 21%). $^1$H NMR (400 MHz, DMSO-d6) δ 12.07 (s, 1H), 10.71 (s, 1H), 7.69 (t, *J* = 9.1 Hz, 1H), 7.67 - 7.60 (m, 2H), 7.02 (t, *J* = 8.1 Hz, 1H), 6.88 (d, *J* = 7.8 Hz, 1H), 6.48 (d, *J* = 8.3 Hz, 1H), 4.72 - 4.42 (m, 5H), 4.26 (brs, 2H), 3.82 (t, *J* = 6.7 Hz, 2H), 3.61 - 3.50 (m, 3H), 2.93 - 2.71 (m, 4H). LCMS (ESI) calcd for $C_{24}H_{24}Cl_2N_5O_3^+$ [M+H]$^+$: 500.13, found, 500.2.

**Example 137: preparation of 1-(5-(((1R,4R)-5-(2,3-dichlorophenyl)-2,5-diazabicyclo[2.2.1]heptan-2-yl) methyl)-1-oxoisoindolin-2-yl)dihydropyrimidine-2,4(1H,3H)-dione (GT-07427)**

Referring to the method of Scheme 1, the target product GT-07427 was prepared as a white solid (22 mg, yield 57%). $^1$H NMR (400 MHz, DMSO-d6) δ 10.73 (s, 1H), 10.69 (s, 1H), 7.98 (s, 1H), 7.86 (s, 2H), 7.19 - 7.14 (m, 1H), 7.11 (dd, *J* = 7.9, 1.4 Hz, 1H), 6.99 (d, *J* = 8.2 Hz, 1H), 4.80 (d, *J* = 15.9 Hz, 1H), 4.69 (d, *J* = 9.0 Hz, 1H), 4.56 (d, *J* = 20.2 Hz, 2H), 4.52 - 4.41 (m, 1H), 4.37 (s, 1H), 3.94 - 3.81 (m, 2H), 3.81 - 3.75 (m, 2H), 3.69 - 3.56 (m, 1H), 3.23 (d, *J* = 12.8 Hz, 1H), 2.88 - 2.83 (m, 1H), 2.79 - 2.73 (m, 1H), 2.58 (d, *J* = 11.3 Hz, 1H), 2.15 (d, *J* = 11.7 Hz, 1H). LCMS (ESI) calcd for $C_{24}H_{24}Cl_2N_5O_3^+$ [M+H]$^+$: 500.13, found, 500.2.

**Example 138: preparation of 1-(5-((5-(2,3-dichlorophenyl)-2,5-diazabicyclo[2.2.2]octan-2-yl)methyl)-1-oxoisoindolin-2-yl)dihydropyrimidine-2,4(1H,3H)-dione (GT-07428)**

Referring to the method of Scheme 1, the target product GT-07428 was prepared as a white solid (21 mg, yield 56%). $^1$H NMR (400 MHz, DMSO-d6) δ 11.31 (s, 1H), 10.69 (s, 1H), 8.06 (d, *J* = 5.8 Hz, 1H), 7.97 (dd, *J* = 11.8, 8.1 Hz, 1H), 7.87 (dd, *J* = 7.8, 5.6 Hz, 1H), 7.38 - 7.32 (m, 1H), 7.30 - 7.20 (m, 2H), 4.81 (d, *J* = 16.5 Hz, 1H), 4.75 - 4.51 (m, 3H), 4.06 (dd, *J* = 60.2, 11.5 Hz, 1H), 3.81 - 3.76 (m, 4H), 3.69 - 3.53 (m, 2H), 3.37 - 3.21 (m, 1H), 2.89 - 2.83 (m, 1H), 2.77 - 2.67 (m, 1H), 2.47 - 2.30 (m, 1H), 2.18 - 2.04(m, 1H), 1.97 - 1.80 (m, 2H). LCMS (ESI) calcd for $C_{25}H_{26}Cl_2N_5O_3^+$ [M+H]$^+$: 514.14, found,

514.2.

**Example 139: preparation of 1-(5-((8-(2,3-dichlorophenyl)-3,8-diazabicyclo[3.2.1]octan-3-yl)methyl)-1-oxoisoindolin-2-yl)dihydropyrimidine-2,4(1H,3H)-dione (GT-07429)**

Referring to the method of Scheme 1, the target product GT-07429 was prepared as a white solid (20 mg, yield 53%). $^1$H NMR (400 MHz, DMSO-d6) δ 10.69 (s, 1H), 10.58 (s, 1H), 8.05 (s, 1H), 7.94 (d, *J* = 7.8 Hz, 1H), 7.86 (d, *J* = 7.8 Hz, 1H), 7.27 - 7.23 (m, 2H), 7.08 - 7.04 (m, 1H), 4.81 (d, *J* = 16.5 Hz, 1H), 4.58 (d, *J* = 16.5 Hz, 1H), 4.51 (d, *J* = 3.9 Hz, 2H), 4.16 (brs, 2H), 3.79 (t, *J* = 7.0 Hz, 2H), 3.37 - 3.30 (m, 4H), 2.90 - 2.83 (m, 1H), 2.79 - 274 (m, 1H), 2.23 (d, *J* = 8.5 Hz, 2H), 2.00 - 1.94 (m, 2H). LCMS (ESI) calcd for $C_{25}H_{26}Cl_2N_5O_3^+$ [M+H]$^+$: 514.14, found, 514.2.

**Example 140: preparation of 1-(5-((3-(2,3-dichlorophenyl)-3,8-diazabicyclo[3.2.1]octan-8-yl)methyl)-1-oxoisoindolin-2-yl)dihydropyrimidine-2,4(1H,3H)-dione (GT-07430)**

Referring to the method of Scheme 1, the target product GT-07430 was prepared as a white solid (23 mg, yield 61%). $^1$H NMR (400 MHz, DMSO-d6) δ 11.38 (s, 1H), 10.69 (s, 1H), 8.08 (s, 1H), 7.98 (d, *J* = 8.0 Hz, 1H), 7.88 (d, *J* = 7.9 Hz, 1H), 7.39 - 7.32 (m, 2H), 7.19 - 7.16 (m, 1H), 4.83 (d, *J* = 16.6 Hz, 1H), 4.59 (d, *J* = 16.5 Hz, 1H), 4.40 (d, *J* = 5.9 Hz, 2H), 3.97 (s, 2H), 3.80 (t, *J* = 6.8 Hz, 2H), 3.62 (t, *J* = 10.6 Hz, 2H), 3.25 - 3.14 (m, 3H), 2.93 - 2.73 (m, 2H), 2.45 - 2.25 (m, 4H). LCMS (ESI) calcd for $C_{25}H_{26}Cl_2N_5O_3^+$ [M+H]$^+$: 514.14, found, 514.2.

**Example 141: preparation of 1-(5-((4-(2,3-dichlorophenyl)-1,4-diazepan-1-yl)methyl)-1-oxoisoindolin-2-yl)dihydropyrimidine-2,4(1H,3H)-dione (GT-07431)**

Referring to the method of Scheme 1, the target product GT-07431 was prepared as a white solid (21 mg, yield 55%). $^1$H NMR (400 MHz, DMSO-d6) δ 11.60 (s, 1H), 10.69 (s, 1H), 8.00 (s, 1H), 7.90 (d, *J* = 8.2 Hz, 1H), 7.86 (d, *J* = 7.8 Hz, 1H), 7.33 - 7.29 (m, 2H), 7.23 (dd, *J* = 10.0, 5.3 Hz, 1H), 4.82 (d, *J* = 16.6 Hz, 1H), 4.64 - 4.51 (m, 3H), 3.80 (t, *J* = 6.8 Hz, 2H), 3.63 - 3.57 (m, 1H), 3.47 - 3.38 (m, 2H), 3.34 - 3.22 (m, 4H), 2.88 - 2.74 (m, 2H), 2.41 - 2.25 (m, 1H), 2.25 - 2.05 (m, 2H). LCMS (ESI) calcd for $C_{24}H_{26}Cl_2N_5O_3^+$ [M+H]$^+$: 502.14, found, 502.2.

**Example 142: preparation of 1-(5-((2',3'-dichloro-3,6-dihydro-[4,4'-bipyridin]-1(2H)-yl)methyl)-1-oxoisoindolin-2-yl)dihydropyrimidine-2,4(1H,3H)-dione (GT-07432)**

Referring to the method of Scheme 1, the target product GT-07432 was prepared as a white solid (8 mg, yield 20%). $^1$H NMR (400 MHz, DMSO-d6) δ 11.19 (s, 1H), 10.69 (s, 1H), 8.40 (d, *J* = 4.9 Hz, 1H), 7.95 - 7.86 (m, 2H), 7.83 (d, *J* = 7.6 Hz, 1H), 7.78 - 7.71 (m, 1H), 7.67 - 7.50 (m, 1H), 7.36 (d, *J* = 4.9 Hz, 1H), 5.92 (s, 1H), 4.80 (t, *J* = 20.1 Hz, 1H), 4.61 - 4.57 (m, 3H), 3.81 - 3.77 (m, 4H), 3.68 - 3.58 (m, 1H), 3.29 - 3.26 (m, 1H), 2.92 - 2.84 (m, 2H), 2.78 - 2.72 (m, 1H), 2.60 - 2.56 (m, 1H). LCMS (ESI) calcd for $C_{23}H_{22}Cl_2N_5O_3^+$ [M+H]$^+$: 486.11, found, 486.2.

**Example 143: preparation of 1-(5-((4-benzhydryl-2,6-dimethylpiperazin-1-yl)methyl)-1-oxoisoindolin-2-yl)dihydropyrimidine-2,4(1H,3H)-dione (GT-07433)**

Referring to the method of Scheme 1, the target product GT-07433 was prepared as a white solid (8 mg, yield 30%). $^1$H NMR (400 MHz, DMSO-d6) δ 10.70 (s, 1H), 7.90 - 7.82 (m, 2H), 7.74 - 7.68 (m, 1H), 7.39 - 7.20 (m, 10H), 4.85 - 4.79 (m, 1H), 4.73 - 4.51 (m, 3H), 3.45 - 3.21 (m, 2H), 2.96 - 2.72 (m, 5H), 1.56 - 1.30 (m, 5H), 1.26 - 1.10 (m, 5H). LCMS (ESI) calcd for $C_{32}H_{36}N_5O_3^+$ [M+H]$^+$: 538.28, found, 538.3.

**Example 144: preparation of 5-((6-(2,3-dichlorophenyl)-3,6-diazabicyclo[3.1.1]heptan-3-yl)methyl)-2-(2,4-dioxotetrahydropyrimidin-1(2H)-yl)isoindoline-1,3-dione (GT-07434)**

Referring to the method of Scheme 1, the target product GT-07434 was prepared as a white solid (27 mg, yield 68%). $^1$H NMR (400 MHz, DMSO-d6) δ 12.21 (s, 1H), 10.95 (s, 1H), 8.14 (s, 1H), 8.07 (d, *J* = 5.5 Hz, 1H), 7.92 (d, *J* = 7.0 Hz, 1H), 6.96 (t, *J* = 8.1 Hz, 2H), 6.56 (d, *J* = 6.6 Hz, 1H), 4.77 - 4.63 (m, 1H), 4.59 (s, 1H), 4.46 - 4.29 (m, 2H), 3.84 (t, *J* = 6.8 Hz, 2H), 3.66 - 3.49 (m, 3H), 3.39 - 3.30 (m, 2H), 2.87 (t, *J*= 6.8 Hz, 2H), 2.77 - 2.69 (m, 1H). LCMS (ESI) calcd for $C_{24}H_{22}Cl_2N_5O_4^+$ [M+H]$^+$: 514.10, found, 514.1.

**Example 145: preparation of 5-(((1R,4R)-5-(2,3-dichlorophenyl)-2,5-diazabicyclo[2.2.1]heptan-2-yl) methyl)-2-(2,4-dioxotetrahydropyrimidin-1(2H)-yl)isoindoline-1,3-dione (GT-07435)**

Referring to the method of Scheme 1, the target product GT-07435 was prepared as a white solid (24 mg, yield 61%). $^1$H NMR (400 MHz, DMSO-d6) δ 10.92 (s, 1H), 10.40 (s, 1H), 8.37 (s, 1H), 8.24 (d, *J* = 7.3 Hz, 1H), 8.11 (d, *J* = 7.7 Hz, 1H), 7.15 (dd, *J* = 8.0, 2.5 Hz, 1H), 7.11 (d, *J* = 7.1 Hz, 1H), 6.97 (d, *J* = 8.0 Hz, 1H), 4.78 (dd, *J* = 12.5, 4.3 Hz, 1H), 4.58 - 4.47 (m, 2H), 4.42 (s, 1H), 3.95 (d, *J* = 10.8 Hz, 1H), 3.82 (t, *J*= 6.7 Hz, 3H), 3.74 (dd, *J* = 11.2, 3.9 Hz, 1H), 3.69 - 3.56 (m, 1H), 3.26 (d, *J* = 12.3 Hz, 1H), 2.85 (t, *J* = 6.8 Hz, 3H), 2.59 (d, *J* = 11.6 Hz, 1H), 2.18 (d, *J* = 10.9 Hz, 1H). LCMS (ESI) calcd for $C_{24}H_{22}Cl_2N_5O_4^+$ [M+H]$^+$: 514.10, found, 514.1.

**Example 146: preparation of 5-((5-(2,3-dichlorophenyl)-2,5-diazabicyclo[2.2.2]octan-2-yl)methyl)-2-(2,4-dioxotetrahydropyrimidin-1(2H)-yl)isoindoline-1,3-dione (GT-07436)**

Referring to the method of Scheme 1, the target product GT-07436 was prepared as a white solid (21 mg, yield 55%). $^1$H NMR (400 MHz, DMSO-d6) δ 11.26 (s, 1H), 10.93 (s, 1H), 8.50 - 8.45 (m, 1H), 8.42 - 8.29 (m, 1H), 8.11 (t, *J* = 7.5 Hz, 1H), 7.35 - 7.31 (m, 1H), 7.30 - 7.18 (m, 2H), 4.84 - 4.63 (m, 2H), 4.06 (dd, *J* = 84.4, 11.9 Hz, 1H), 3.83 (t, *J* = 6.8 Hz, 3H), 3.73 - 3.63 (m, 2H), 3.57 - 3.47 (m, 1H), 3.28 - 3.21 (m, 1H), 2.86 (t, *J* = 6.8 Hz, 2H), 2.47 - 2.28 (m, 1H), 2.27 - 2.07 (m, 1H), 2.07 - 1.78 (m, 2H). LCMS (ESI) calcd for $C_{25}H_{24}Cl_2N_5O_4^+$ [M+H]$^+$: 528.12, found, 528.2.

**Example 147: preparation of 5-((8-(2,3-dichlorophenyl)-3,8-diazabicyclo[3.2.1]octan-3-yl)methyl)-2-(2,4-dioxotetrahydropyrimidin-1(2H)-yl)isoindoline-1,3-dione (GT-07437)**

Referring to the method of Scheme 1, the target product GT-07437 was prepared as a white solid (27 mg, yield 70%). $^1$H NMR (400 MHz, DMSO-d6) δ 10.92 (s, 1H), 10.54 (s, 1H), 8.47 (s, 1H), 8.32 (d, *J* = 7.2 Hz, 1H), 8.10 (d, *J* = 7.2 Hz, 1H), 7.27 - 7.23 (m, 2H), 7.06 (d, *J* = 4.6 Hz, 1H), 4.60 (brs, 2H), 4.18 (brs, 2H), 3.82 (t, *J* = 6.8 Hz, 3H), 3.48 - 3.34 (m, 2H), 2.86 (t, *J* = 6.8 Hz, 3H), 2.23 - 2.18 (m, 2H), 2.03 - 1.98 (m, 2H). LCMS (ESI) calcd for $C_{25}H_{24}Cl_2N_5O_4^+$ [M+H]$^+$: 528.12, found, 528.2.

**Example 148: preparation of 5-((3-(2,3-dichlorophenyl)-3,8-diazabicyclo[3.2.1]octan-8-yl)methyl)-2-(2,4-dioxotetrahydropyrimidin-1(2H)-yl)isoindoline-1,3-dione (GT-07438)**

Referring to the method of Scheme 1, the target product GT-07438 was prepared as a white solid (23 mg, yield 60%). $^1$H NMR (400 MHz, DMSO-d6) δ 11.25 (s, 1H), 10.93 (s, 1H), 8.46 (s, 1H), 8.35 (d, *J* = 7.6 Hz, 1H), 8.13 (d, *J* = 7.7 Hz, 1H), 7.40 - 7.34 (m, 2H), 7.18 (dd, *J* = 7.7, 1.7 Hz, 1H), 4.50 (d, *J* = 5.6 Hz, 2H), 4.00 (brs, 2H), 3.83 (t, *J* = 6.8 Hz, 2H), 3.61 - 3.53 (m, 2H), 3.25 - 3.21 (m, 2H), 2.86 (t, *J* = 6.8 Hz, 2H), 2.43 - 2.25 (m, 4H). LCMS (ESI) calcd for $C_{25}H_{24}Cl_2N_5O_4^+$ [M+H]$^+$: 528.12, found, 528.2.

**Example 149: preparation of 5-((4-(2,3-dichlorophenyl)-1,4-diazepan-1-yl)methyl)-2-(2,4-dioxotetrahydropyrimidin-1(2H)-yl)isoindoline-1,3-dione (GT-07439)**

Referring to the method of Scheme 1, the target product GT-07439 was prepared as a white solid (25 mg, yield 64%). $^1$H NMR (400 MHz, DMSO-d6) δ 11.26 (s, 1H), 10.93 (s, 1H), 8.38 (s, 1H), 8.24 (d, *J* = 7.8 Hz, 1H), 8.11 (d, *J* = 7.7 Hz, 1H), 7.31 (d, *J* = 4.9 Hz, 2H), 7.24 - 7.21 (m, 1H), 4.65 - 4.63 (m, 2H), 3.83 (t, *J* = 6.8 Hz, 2H), 3.55 - 3.50 (m, 3H), 3.45 - 3.39 (m, 2H), 3.31 - 3.19 (m, 3H), 2.86 (t, *J* = 6.8 Hz, 2H), 2.28 - 2.12 (m, 2H). LCMS (ESI) calcd for $C_{24}H_{24}Cl_2N_5O_4^+$ [M+H]$^+$: 516.12, found, 516.1.

**Example 150: preparation of 5-((2',3'-dichloro-3,6-dihydro-[4,4'-bipyridin]-1(2H)-yl)methyl)-2-(2,4-dioxotetrahydropyrimidin-1(2H)-yl)isoindoline-1,3-dione (GT-07440)**

Referring to the method of Scheme 1, the target product GT-07440 was prepared as a white solid (20 mg, yield 50%). $^1$H NMR (400 MHz, DMSO-d6) δ 11.65 (s, 1H), 10.93 (s, 1H), 8.40 (d, *J* = 4.9 Hz, 1H), 8.37 (s, 1H), 8.28 - 8.21 (m, 1H), 8.12 (d, *J* = 7.7 Hz, 1H), 7.36 (d, *J* = 4.9 Hz, 1H), 5.92 (s, 1H), 4.67 (s, 2H), 3.94 - 3.73 (m, 4H), 3.66 - 3.63 (m, 1H), 3.28 (brs, 1H), 3.03 - 2.89 (m, 1H), 2.91 - 2.81 (m, 2H), 2.58 (d, *J* = 17.5 Hz, 1H). LCMS (ESI) calcd for $C_{23}H_{20}Cl_2N_5O_4^+$ [M+H]$^+$: 500.09, found, 500.1.

**Example 151: preparation of 5-((5-chloro-3',6'-dihydro-[3,4'-bipyridin]-1'(2'H)-yl)methyl)-2-(2,4-dioxotetrahydropyrimidin-1(2H)-yl)isoindoline-1,3-dione (GT-07491)**

Referring to the method of Scheme 1, the target product GT-07491 was prepared as a white solid (13 mg, yield 23%). $^1$H NMR (400 MHz, DMSO-d6) δ 11.37 (s, 1H), 10.93 (s, 1H), 8.69 (d, *J* = 1.7 Hz, 1H), 8.59 (d, *J* = 2.2 Hz, 1H), 8.36 (s, 1H), 8.23 (d, *J* = 7.5 Hz, 1H), 8.12 (d, *J* = 7.7 Hz, 1H), 8.06 (s, 1H), 6.40 (s, 1H), 4.75 - 4.59 (m, 2H), 3.91 - 3.80 (m, 4H), 3.67 - 3.60 (m, 1H), 3.38 - 3.18 (m, 1H), 2.94 - 2.85 (m, 4H). LCMS (ESI) calcd for $C_{23}H_{21}ClN_5O_4^+$ [M+H]$^+$: 466.13, found, 466.2.

**Example 152: preparation of 2-(2,4-dioxotetrahydropyrimidin-1(2H)-yl)-5-((3-fluoro-3',6'-dihydro-[2,4'-bipyridin]-1'(2'H)-yl)methyl)isoindoline-1,3-dione (GT-07492)**

Referring to the method of Scheme 1, the target product GT-07492 was prepared as a white solid (22 mg, yield 40%). $^1$H NMR (400 MHz, DMSO-d6) δ 11.87 (s, 1H), 10.93 (s, 1H), 8.50 - 8.44 (m, 1H), 8.42 (d, *J* = 7.1 Hz, 1H), 8.29 (t, *J* = 8.5 Hz, 1H), 8.11 (d, *J* = 7.7 Hz, 1H), 7.81 - 7.75 (m, 1H), 7.48 - 7.39 (m, 1H), 6.56 (s, 1H), 4.80 - 4.60 (m, 2H), 3.97 - 3.91 (m, 1H), 3.83 (t, *J* = 12.9 Hz, 3H), 3.67 - 3.60 (m, 1H), 3.33 - 3.26 (m, 1H), 3.12 - 2.90 (m, 2H), 2.92 - 2.83 (m, 2H). LCMS (ESI) calcd for $C_{23}H_{21}FN_5O_4^+$ [M+H]$^+$: 450.16, found, 450.2.

**Example 153: preparation of 5-((4-(2,3-difluoropyridin-4-yl)piperidin-1-yl)methyl)-2-(2,4-dioxotetrahydropyrimidin-1(2H)-yl)isoindoline-1,3-dione (GT-07493)**

Referring to the method of Scheme 1, the target product GT-07493 was prepared as a white solid (16 mg, yield 28%). $^1$H NMR (400 MHz, DMSO-d6) δ 11.23 (s, 1H), 10.93 (s, 1H), 8.33 (s, 1H), 8.19 (d, *J* = 7.7 Hz, 1H), 8.12 (d, *J* = 7.6 Hz, 1H), 8.04 (d, *J* = 4.9 Hz, 1H), 7.29 (t, *J* = 4.8 Hz, 1H), 4.64 - 4.48 (m, 2H), 3.82 (dd, *J* = 8.8, 4.8 Hz, 2H), 3.49 (d, *J* = 11.6 Hz, 2H), 3.25 (t, *J* = 12.2 Hz, 1H), 3.16 - 3.10 (m, 2H), 2.86 (t, *J* = 6.8 Hz, 2H), 2.21 - 2.12 (m, 2H), 1.99 (d, *J* = 13.2 Hz, 2H). LCMS (ESI) calcd for $C_{23}H_{22}F_2N_5O_4^+$ [M+H]$^+$: 470.16, found, 470.2.

**Example 154: preparation of 5-((2',3'-difluoro-3,6-dihydro-[4,4'-bipyridin]-1(2H)-yl)methyl)-2-(2,4-dioxotetrahydropyrimidin-1(2H)-yl)isoindoline-1,3-dione (GT-07494)**

Referring to the method of Scheme 1, the target product GT-07494 was prepared as a white solid (8 mg, yield 14%). $^1$H NMR (400 MHz, DMSO-d6) δ 11.38 (s, 1H), 10.93 (s, 1H), 8.34 (s, 1H), 8.25 - 8.17 (m, 1H), 8.12 (d, *J* = 7.5 Hz, 1H), 8.05 (d, *J* = 5.1 Hz, 1H), 7.42 (t, *J* = 5.1 Hz, 1H), 6.34 (s, 1H), 4.72 - 4.62 (m, 2H), 3.94 - 3.81 (m, 4H), 3.69 - 3.62 (m, 1H), 3.31 - 3.24 (m, 1H), 3.06 - 2.91 (m, 1H), 2.86 (t, *J* = 6.8 Hz, 2H), 2.75 - 2.67 (m, 1H). LCMS (ESI) calcd for $C_{23}H_{20}F_2N_5O_4^+$ [M+H]$^+$: 468.15, found, 468.2.

**Example 155: preparation of 1-(5-((4-(isoxazol-3-yl)piperidin-1-yl)methyl)-1-oxoisoindolin-2-yl)dihydropyrimidine-2,4(1H,3H)-dione (GT-07495)**

Referring to the method of Scheme 1, the target product GT-07495 was prepared as a white solid (10 mg, yield 19%). $^1$H NMR (400 MHz, DMSO-d6) δ 10.93 (s, 1H), 10.69 (s, 1H), 8.85 (d, *J* = 1.7 Hz, 1H), 7.93 - 7.89 (m, 2H), 7.88 - 7.82 (m, 1H), 7.80 (d, *J* = 7.8 Hz, 1H), 6.53 (d, *J* = 1.7 Hz, 1H), 4.82 (d, *J* = 16.7 Hz, 1H), 4.58 (d, *J* = 16.7 Hz, 1H), 4.46 (d, *J* = 4.9 Hz, 2H), 3.79 (t, *J* = 6.9 Hz, 2H), 3.45 (d, *J* = 11.3 Hz, 2H), 3.10 - 3.01 (m, 3H), 2.93 - 2.75 (m, 2H), 2.22 - 2.15 (m, 2H), 2.07 - 1.98 (m, 2H). LCMS (ESI) calcd for $C_{21}H_{24}N_5O_4^+$ [M+H]$^+$: 410.18, found, 410.2.

**Example 156: preparation of 1-(5-((4-(6-fluorobenzo[d]isoxazol-3-yl)piperazin-1-yl)methyl)-1-oxoisoindolin-2-yl)dihydropyrimidine-2,4(1H,3H)-dione (GT-07496)**

Referring to the method of Scheme 1, the target product GT-07496 was prepared as a white solid (9 mg, yield 15%). $^1$H NMR (400 MHz, DMSO) δ 11.54 (s, 1H), 10.70 (s, 1H), 8.07 (dd, *J* = 8.9, 5.2 Hz, 1H), 7.96 - 7.86 (m, 2H), 7.83 - 7.80 (m, 1H), 7.61 (dd, *J* = 9.1, 1.9 Hz, 1H), 7.25 (t, *J* = 9.0 Hz, 1H), 4.82 (d, *J* = 16.8 Hz, 1H), 4.65 - 4.56 (m, 3H), 4.11 (d, *J* = 12.1 Hz, 2H), 3.80 (t, *J* = 6.9 Hz, 2H), 3.54 (t, *J* = 12.1 Hz, 2H), 3.47 - 3.41 (m, 4H), 2.92 - 2.70 (m, 2H). LCMS (ESI) calcd for $C_{24}H_{24}FN_6O_4^+$ [M+H]$^+$: 479.18, found, 479.2.

**Example 157: preparation of 1-(5-((4-(isoxazolo[4,5-c]pyridin-3-yl)piperazin-1-yl)methyl)-1-oxoisoindolin-2-yl) dihydropyrimidine-2,4(1H,3H)-dione (GT-07497)**

Referring to the method of Scheme 1, the target product GT-07497 was prepared as a white solid (25 mg, yield 43%). $^1$H NMR (400 MHz, DMSO) δ 12.22 (s, 1H), 10.70 (s, 1H), 9.75 - 9.65 (m, 1H), 8.85 - 8.81 (m, 1H), 8.13 - 8.04 (m, 1H),

7.97 (s, 1H), 7.92 - 7.82 (m, 2H), 4.83 (d, J = 16.7 Hz, 1H), 4.71 - 4.52 (m, 5H), 3.80 (t, J = 6.8 Hz, 2H), 3.74 (s, 2H), 3.45 (s, 2H), 3.32 (s, 2H), 2.95 - 2.70 (m, 2H). LCMS (ESI) calcd for $C_{23}H_{24}N_7O_4^+$ $[M+H]^+$: 462.19, found, 462.2.

**Example 158: preparation of 1-(5-((4-(6-fluorobenzo[d]isothiazol-3-yl)piperazin-1-yl)methyl)-1-oxoisoindolin-2-yl)dihydropyrimidine-2,4(1H,3H)-dione (GT-07500)**

Referring to the method of Scheme 1, the target product GT-07500 was prepared as a white solid (23 mg, yield 37%). $^1H$ NMR (400 MHz, DMSO) δ 11.22 (s, 1H), 10.70 (s, 1H), 8.18 (dd, J = 8.9, 4.9 Hz, 1H), 8.01 (dd, J = 8.9, 2.3 Hz, 1H), 7.90 (d, J = 7.6 Hz, 2H), 7.81 (d, J = 8.1 Hz, 1H), 7.35 (td, J = 8.8, 2.3 Hz, 1H), 4.82 (d, J = 17.0 Hz, 1H), 4.61 - 4.57 (m, 3H), 4.05 (d, J = 13.0 Hz, 2H), 3.80 (t, J = 6.9 Hz, 2H), 3.49 - 3.43 (m, 4H), 3.30 - 3.25 (m, 2H), 2.90 - 2.74 (m, 2H). LCMS (ESI) calcd for $C_{24}H_{24}FN_6O_3S^+$ $[M+H]^+$: 495.16, found, 495.2.

**Example 159: preparation of 1-(5-((4-(6-fluoro-1H-indazol-3-yl)piperazin-1-yl)methyl)-1-oxoisoindolin-2-yl)dihydropyrimidine-2,4(1H,3H)-dione (GT-07545)**

Referring to the method of Scheme 1, the target product GT-07545 was prepared as a white solid (12 mg, yield 20%). $^1H$ NMR (400 MHz, DMSO) δ 12.28 (s, 1H), 11.00 (s, 1H), 10.70 (s, 1H), 7.91 - 7.89 (m, 2H), 7.86 - 7.78 (m, 2H), 7.16 (dd, J = 9.7, 2.2 Hz, 1H), 6.89 (td, J = 9.2, 2.2 Hz, 1H), 4.82 (d, J = 16.9 Hz, 1H), 4.65 - 4.50 (m, 3H), 3.94 (d, J = 9.1 Hz, 2H), 3.80 (t, J = 7.1 Hz, 2H), 3.35 - 3.33 (m, 2H), 3.31 (d, J = 8.7 Hz, 4H), 2.94 - 2.73 (m, 2H). LCMS (ESI) calcd for $C_{24}H_{25}FN_7O_3^+$ $[M+H]^+$: 478.20, found, 478.2.

**Example 160: preparation of 1-(5-((4-(1H-pyrrolo[2,3-b]pyridin-3-yl)piperazin-1-yl)methyl)-1-oxoisoindolin-2-yl) dihydropyrimidine-2,4(1H,3H)-dione (GT-07502)**

Referring to the method of Scheme 1, the target product GT-07502 was prepared as a white solid (5 mg, yield 9%). $^1H$ NMR (400 MHz, DMSO) δ 10.70 (s, 1H), 8.26 (d, J = 4.9 Hz, 1H), 8.17 (d, J = 8.2 Hz, 1H), 7.92 (d, J = 6.8 Hz, 1H), 7.89 (s, 1H), 7.83 (d, J = 7.9 Hz, 1H), 7.13 - 7.10 (m, 1H), 4.83 (d, J = 17.2 Hz, 1H), 4.66 - 4.49 (m, 3H), 3.81 - 3.78 (m, 2H), 3.59 - 3.52 (m, 4H), 3.36 - 3.31 (m, 2H), 3.22 - 3.20 (m, 1H), 3.13 - 3.06 (m, 1H), 2.94 - 2.72 (m, 2H). LCMS (ESI) calcd for $C_{24}H_{26}N_7O_3^+$ $[M+H]^+$: 460.21, found, 460.3.

**Example 161: preparation of 1-(5-((4-(5-fluoro-1H-benzo[d]imidazol-1-yl)piperidin-1-yl)methyl)-1-oxoisoindolin-2-yl)dihydropyrimidine-2,4(1H,3H)-dione (GT-07503)**

Referring to the method of Scheme 1, the target product GT-07503 was prepared as a white solid (33 mg, yield 55%). $^1H$ NMR (400 MHz, DMSO) δ 10.70 (s, 1H), 9.41 (s, 1H), 8.33 (s, 1H), 7.98 (d, J = 5.6 Hz, 1H), 7.91 - 7.89 (m, 2H), 7.75 - 7.64 (m, 1H), 7.46 - 7.40 (m, 1H), 5.00 - 4.91 (m, 1H), 4.84 (d, J = 16.4 Hz, 2H), 4.69 - 4.50 (m, 4H), 4.05 - 3.94 (m, 2H), 3.60 (d, J = 11.2 Hz, 2H), 3.24 - 3.16 (m, 2H), 2.90 - 2.84 (m, 1H), 2.82 - 2.64 (m, 3H), 2.32 (d, J = 11.5 Hz, 2H). LCMS (ESI) calcd for $C_{25}H_{26}FN_6O_3^+$ $[M+H]^+$: 477.20, found, 477.2.

**Example 162: preparation of 1-(1-oxo-5-((4-(thiophen-2-yl)piperidin-1-yl)methyl)isoindolin-2-yl)dihydropyrimidine-2,4(1H,3H)-dione (GT-07511)**

Referring to the method of Scheme 1, the target product GT-07511 was prepared as a white solid (6 mg, yield 13%). $^1H$ NMR (400 MHz, DMSO-d6) δ 10.91 (s, 1H), 10.69 (s, 1H), 7.91 (s, 1H), 7.88 (d, J = 7.8 Hz, 1H), 7.80 (d, J = 7.9 Hz, 1H), 7.38 (dd, J = 5.1, 1.2 Hz, 1H), 6.97 (dd, J = 5.1, 3.5 Hz, 1H), 6.90 (d, J= 3.4 Hz, 1H), 4.82 (d, J = 16.4 Hz, 1H), 4.58 (d, J = 17.6 Hz, 1H), 4.45 (d, J = 5.0 Hz, 2H), 3.79 (t, J = 6.9 Hz, 3H), 3.44 - 3.41 (m, 2H), 3.16 - 3.04 (m, 3H), 2.90 - 2.76 (m, 2H), 2.21 - 2.10 (m, 2H), 2.07 - 2.01 (m, 2H). LCMS (ESI) calcd for $C_{22}H_{25}N_4O_3S^+$ $[M+H]^+$: 425.16, found, 425.2.

**Example 163: preparation of 5-((4-(benzo[d]isoxazol-3-yl)piperidin-1-yl)methyl)-2-(2,4-dioxotetrahydropyrimidin-1(2H)-yl)isoindoline-1,3-dione (GT-07522)**

Referring to the method of Scheme 1, the target product GT-07522 was prepared as a white solid (34 mg, yield 58%). $^1H$ NMR (400 MHz, DMSO-d6) δ 11.36 (s, 1H), 10.93 (s, 1H), 8.36 (s, 1H), 8.23 (d, J = 7.6 Hz, 1H), 8.13 (d, J = 7.8 Hz, 2H), 7.74 (d, J = 8.4 Hz, 1H), 7.66 (t, J = 7.7 Hz, 1H), 7.41 (t, J = 7.4 Hz, 1H), 4.61 (s, 2H), 3.83 (t, J = 6.8 Hz, 2H), 3.55 (d, J = 10.6 Hz, 2H), 3.50 - 3.43 (m, 1H), 3.21 - 3.14 (m, 2H), 2.87 (t, J = 6.8 Hz, 2H), 2.44 - 2.32 (m, 2H), 2.23 (d, J = 12.9 Hz, 2H). LCMS (ESI) calcd for $C_{25}H_{24}N_5O_5^+$ $[M+H]^+$: 474.18, found, 474.2.

**Example 164: preparation of 2-(2,4-dioxotetrahydropyrimidin-1(2H)-yl)-5-((4-(isoxazol-3-yl)piperidin-1-yl) methyl)isoindoline-1,3-dione (GT-07523)**

Referring to the method of Scheme 1, the target product GT-07523 was prepared as a white solid (20 mg, yield 38%). $^1$H NMR (400 MHz, DMSO-d6) δ 11.28 (s, 1H), 10.93 (s, 1H), 8.85 (d, *J* = 1.6 Hz, 1H), 8.34 (s, 1H), 8.20 (dd, *J* = 7.8, 1.2 Hz, 1H), 8.11 (d, *J* = 7.8 Hz, 1H), 6.53 (d, *J* = 1.7 Hz, 1H), 4.55 (s, 2H), 3.83 (t, *J* = 6.8 Hz, 2H), 3.70 - 3.62 (m, 1H), 3.33 - 3.26 (m, 1H), 3.12 - 3.00 (m, 3H), 2.86 (t, *J* = 6.8 Hz, 2H), 2.23 - 2.13 (m, 2H), 2.06 - 2.01 (m, 2H). LCMS (ESI) calcd for $C_{21}H_{22}N_5O_5^+$ [M+H]$^+$: 424.16, found, 424.2.

**Example 165: preparation of 2-(2,4-dioxotetrahydropyrimidin-1(2H)-yl)-5-((4-(6-fluorobenzo[d]isoxazol-3-yl)piperazin-1-yl)methyl)isoindoline-1,3-dione (GT-07524)**

Referring to the method of Scheme 1, the target product GT-07524 was prepared as a white solid (34 mg, yield 56%). $^1$H NMR (400 MHz, DMSO) δ 11.51 (s, 1H), 10.93 (s, 1H), 8.30 (s, 1H), 8.13 (t, *J* = 8.3 Hz, 2H), 8.08 (dd, *J* = 8.9, 5.2 Hz, 1H), 7.61 (dd, *J* = 9.0, 1.6 Hz, 1H), 7.25 (t, *J* = 9.0 Hz, 1H), 4.64 (s, 2H), 4.11 (s, 2H), 3.83 (t, *J* = 6.7, 2H), 3.55 - 3.48 (m, 4H), 3.31 - 3.24(m, 2H), 2.86 (t, *J* = 6.7 Hz, 2H). LCMS (ESI) calcd for $C_{24}H_{22}FN_6O_5^+$ [M+H]$^+$: 493.16, found, 493.2.

**Example 166: preparation of 2-(2,4-dioxotetrahydropyrimidin-1(2H)-yl)-5-((4-(isoxazolo[4,5-c]pyridin-3-yl)piperazin-1-yl)methyl)isoindoline-1,3-dione (GT-07525)**

Referring to the method of Scheme 1, the target product GT-07525 was prepared as a white solid (32 mg, yield 55%). $^1$H NMR (400 MHz, DMSO) δ 10.93 (s, 1H), 9.66 (s, 1H), 8.82 (d, *J* = 6.4 Hz, 1H), 8.37 (s, 1H), 8.23 (dd, *J* = 7.8, 1.1 Hz, 1H), 8.13 (d, *J* = 7.7 Hz, 1H), 8.04 (d, *J* = 6.4 Hz, 1H), 4.67 (s, 2H), 4.45 - 4.41 (m, 2H), 3.83 (t, *J* = 6.8 Hz, 2H), 3.71 (s, 2H), 3.47 (s, 2H), 3.34 (s, 2H), 2.86 (t, *J* = 6.8 Hz, 2H). LCMS (ESI) calcd for $C_{23}H_{22}N_7O_5^+$ [M+H]$^+$: 476.17, found, 476.2.

**Example 167: preparation of 2-(2,4-dioxotetrahydropyrimidin-1(2H)-yl)-5-((4-(6-fluorobenzo[d]isothiazol-3-yl) piperazin-1-yl)methyl)isoindoline-1,3-dione (GT-07526)**

Referring to the method of Scheme 1, the target product GT-07526 was prepared as a white solid (27 mg, yield 43%). $^1$H NMR (400 MHz, DMSO) δ 11.65 (s, 1H), 10.93 (s, 1H), 8.34 (s, 1H), 8.20 - 8.17 (M, 2H), 8.13 (d, *J* = 7.6 Hz, 1H), 8.01 (dd, *J* = 8.9, 2.3 Hz, 1H), 7.35 (t, *J* = 8.8 Hz, 1H), 4.66 (s, 2H), 4.04 (brs, 2H), 3.83 (t, *J* = 6.8 Hz, 2H), 3.52 - 3.43 (m, 6H), 2.86 (t, *J* = 6.8 Hz, 2H). LCMS (ESI) calcd for $C_{24}H_{22}FN_6O_4S^+$ [M+H]$^+$: 509.14, found, 509.2.

**Example 168: preparation of 2-(2,4-dioxotetrahydropyrimidin-1(2H)-yl)-5-((4-(6-fluoro-1H-indazol-3-yl)piperazin-1-yl)methyl)isoindoline-1,3-dione (GT-07527)**

Referring to the method of Scheme 1, the target product GT-07527 was prepared as a white solid (30 mg, yield 50%). $^1$H NMR (400 MHz, DMSO) δ 12.28 (s, 1H), 10.93 (s, 1H), 8.32 (s, 1H), 8.19 (d, *J* = 7.8 Hz, 1H), 8.13 (d, *J* = 7.7 Hz, 1H), 7.85 - 7.82 (m, 1H), 7.16 (dd, *J* = 9.7, 2.2 Hz, 1H), 6.89 (td, *J* = 9.2, 2.2 Hz, 1H), 4.64 (s, 2H), 3.95 (s, 2H), 3.83 (t, *J* = 6.8 Hz, 2H), 3.35 - 3.25 (m, 6H), 2.86 (t, *J* = 6.8 Hz, 2H). LCMS (ESI) calcd for $C_{24}H_{23}FN_7O_4^+$ [M+H]$^+$: 492.18, found, 492.2.

**Example 169: preparation of 5-((4-(1H-pyrrolo[2,3-b]pyridin-3-yl)piperazin-1-yl)methyl)-2-(2,4-dioxotetrahydropyrimidin-1(2H)-yl)isoindoline-1,3-dione (GT-07528)**

Referring to the method of Scheme 1, the target product GT-07528 was prepared as a white solid (10 mg, yield 17%). $^1$H NMR (400 MHz, DMSO) δ 11.61 (s, 1H), 10.93 (s, 1H), 8.36 (s, 1H), 8.27 (d, *J* = 4.4 Hz, 1H), 8.23 (d, *J* = 7.3 Hz, 2H), 8.13 (d, *J* = 7.7 Hz, 1H), 7.16 - 7.13 (m, 1H), 4.65 (s, 2H), 3.83 (t, *J* = 6.8 Hz, 2H), 3.42 - 3.30 (m, 8H), 2.86 (t, *J* = 6.8 Hz, 2H). LCMS (ESI) calcd for $C_{24}H_{24}N_7O_4^+$ [M+H]$^+$: 474.19, found, 474.2.

**Example 170: preparation of 2-(2,4-dioxotetrahydropyrimidin-1(2H)-yl)-5-((4-(5-fluoro-1H-benzo[d]imidazol-1-yl)piperidin-1-yl)methyl)isoindoline-1,3-dione (GT-07529)**

Referring to the method of Scheme 1, the target product GT-07529 was prepared as a white solid (45 mg, yield 75%). $^1$H NMR (400 MHz, DMSO) δ 12.17 (s, 1H), 10.93 (s, 1H), 9.36 (s, 1H), 8.40 (s, 1H), 8.34 - 8.22 (m, 2H), 8.14 (d, *J* = 7.8 Hz, 1H), 7.70 - 7.65 (m, 1H), 7.47 - 7.41 (m, 1H), 4.97 - 4.92 (m, 1H), 4.63 (s, 2H), 3.33 - 3.15 (m, 4H), 2.86 (t, *J* = 6.8 Hz, 2H), 2.78 - 2.67 (m, 2H), 2.33 (d, *J* = 12.0 Hz, 2H), 1.35 - 1.30 (m, 2H). LCMS (ESI) calcd for $C_{25}H_{24}FN_6O_4^+$ [M+H]$^+$: 491.18, found, 491.2.

**Example 171: preparation of 1-(5-((4-(6-fluoro-1H-indol-3-yl)-3,6-dihydropyridin-1(2H)-yl)methyl)-1-oxoisoindolin-2-yl)dihydropyrimidine-2,4(1H,3H)-dione (GT-07574)**

Referring to the method of Scheme 1, the target product GT-07574 was prepared as a white solid (35 mg, yield 58%). LCMS (ESI) calcd for $C_{26}H_{25}FN_5O_3^+$ $[M+H]^+$: 474.19, found, 474.2.

**Example 172: preparation of 1-(5-((4-(6-fluoro-1-methyl-1H-indol-3-yl)piperidin-1-yl)methyl)-1-oxoisoindolin-2-yl)dihydropyrimidine-2,4(1H,3H)-dione (GT-07575)**

Referring to the method of Scheme 1, the target product GT-07575 was prepared as a white solid (45 mg, yield 73%). $^1H$ NMR (400 MHz, DMSO) δ 10.99 (s, 1H), 10.70 (s, 1H), 7.94 (s, 1H), 7.89 (d, *J* = 7.8 Hz, 1H), 7.83 (d, *J* = 8.0 Hz, 1H), 7.68 (dd, *J* = 8.8, 5.5 Hz, 1H), 7.25 (dd, *J* = 10.4, 2.4 Hz, 1H), 7.11 (s, 1H), 6.91 - 6.83 (m, 1H), 4.83 (d, *J* = 16.5 Hz, 1H), 4.59 (d, *J* = 16.3 Hz, 1H), 4.48 (d, *J* = 4.7 Hz, 2H), 3.80 (t, *J* = 6.9 Hz, 2H), 3.69 (s, 3H), 3.46 (d, *J* = 11.2 Hz, 2H), 3.18 - 2.98 (m, 4H), 2.90 - 2.73 (m, 2H), 2.15 - 2.08 (m, 3H). LCMS (ESI) calcd for $C_{27}H_{29}FN_5O_3^+$ $[M+H]^+$: 490.22, found, 490.3.

**Example 173: preparation of 1-(5-((4-(1H-pyrrolo[2,3-b]pyridin-3-yl)piperidin-1-yl)methyl)-1-oxoisoindolin-2-yl) dihydropyrimidine-2,4(1H,3H)-dione (GT-07576)**

Referring to the method of Scheme 1, the target product GT-07576 was prepared as a white solid (25 mg, yield 43%). $^1H$ NMR (400 MHz, DMSO) δ 12.38 (s, 1H), 11.51 (s, 1H), 10.70 (s, 1H), 8.69 (d, *J* = 7.9 Hz, 1H), 8.38 (d, *J* = 5.4 Hz, 1H), 7.99 (s, 1H), 7.88 (s, 2H), 7.46 (d, *J* = 2.2 Hz, 1H), 7.36 (dd, *J* = 7.9, 5.4 Hz, 1H), 4.83 (d, *J* = 16.6 Hz, 1H), 4.62 (d, *J* = 16.6 Hz, 2H), 4.50 (d, *J* = 4.5 Hz, 2H), 3.46 (d, *J* = 11.1 Hz, 2H), 3.23 - 3.21 (m, 1H), 3.18 - 3.04 (m, 4H), 2.91 - 2.74 (m 2H), 2.35 - 2.25 (m, 2H), 2.08 (d, *J* = 12.9 Hz, 2H). LCMS (ESI) calcd for $C_{25}H_{27}N_6O_3^+$ $[M+H]^+$: 459.21, found, 459.2.

**Example 174: preparation of 1-(5-((4-(1H-pyrrolo[2,3-b]pyridin-3-yl)-3,6-dihydropyridin-1(2H)-yl)methyl)-1-oxoisoindolin-2-yl)dihydropyrimidine-2,4(1H,3H)-dione (GT-07577)**

Referring to the method of Scheme 1, the target product GT-07577 was prepared as a white solid (16 mg, yield 28%). $^1H$ NMR (400 MHz, DMSO) δ 12.21 (s, 1H), 11.28 (s, 1H), 10.70 (s, 1H), 8.39 (d, *J* = 7.8 Hz, 1H), 8.32 (d, *J* = 4.1 Hz, 1H), 7.97 (s, 1H), 7.92 - 7.85 (m, 2H), 7.73 (d, *J* = 2.4 Hz, 1H), 7.24 (dd, *J* = 7.9, 5.0 Hz, 1H), 6.20 (s, 1H), 4.83 (d, *J* = 16.8 Hz, 1H), 4.64 - 4.52 (m, 3H), 4.08 - 3.94 (m, 4H), 3.65 (d, *J*= 11.2 Hz, 1H), 3.33 - 3.27 (m, 1H), 3.02 - 2.93 (m, 1H), 2.90 - 2.71 (m, 3H). LCMS (ESI) calcd for $C_{25}H_{25}N_6O_3^+$ $[M+H]^+$: 457.20, found, 457.2.

**Example 175: preparation of 1-(5-((4-(5-fluoro-2-oxo-2,3-dihydro-1H-benzo[d]imidazol-1-yl)piperidin-1-yl) methyl)-1-oxoisoindolin-2-yl)dihydropyrimidine-2,4(1H,3H)-dione (GT-07579)**

Referring to the method of Scheme 1, the target product GT-07579 was prepared as a white solid (30 mg, yield 48%). $^1H$ NMR (400 MHz, DMSO) δ 11.25 (s, 1H), 11.12 (s, 1H), 10.70 (s, 1H), 7.93 (s, 1H), 7.89 (d, *J* = 7.8 Hz, 1H), 7.83 (d, *J* = 7.9 Hz, 1H), 7.58 - 7.54 (m, 1H), 6.89 - 6.78 (m, 2H), 4.83 (d, *J* = 16.4 Hz, 1H), 4.61 - 4.56 (m, 2H), 4.48 (d, *J* = 4.5 Hz, 2H), 3.80 (t, *J* = 6.8 Hz, 2H), 3.50 (d, *J* = 11.1 Hz, 2H), 3.26 - 3.10 (m, 2H), 2.93 - 2.71 (m, 4H), 1.87 *(d, J*= 12.0 Hz, 2H) LCMS (ESI) calcd for $C_{25}H_{26}FN_6O_4^+$ $[M+H]^+$: 493.20, found, 493.2.

**Example 176: preparation of 1-(5-((4-(benzo[d]isoxazol-3-yl)piperidin-1-yl)methyl)-1-oxoisoindolin-2-yl)dihydropyrimidine-2,4(1H,3H)-dione (GT-07642)**

Referring to the method of Scheme 1, the target product GT-07642 was prepared as a white solid (15 mg, yield 26%). $^1H$ NMR (400 MHz, DMSO-d6) δ 10.80 (s, 1H), 10.70 (s, 1H), 8.10 (d, *J* = 8.0 Hz, 1H), 7.96 - 7.89 (m, 2H), 7.81 (d, *J* = 7.9 Hz, 1H), 7.74 (d, *J* = 8.4 Hz, 1H), 7.66 (t, *J* = 7.7 Hz, 1H), 7.41 (t, *J* = 7.3 Hz, 1H), 4.83 *(d, J* = 15.9 Hz, 1H), 4.60 (d, *J* = 16.4 Hz, 1H), 4.52 (d, *J* = 4.9 Hz, 2H), 3.80 (t, *J* = 7.1 Hz, 2H), 3.55 (d, *J* = 10.8 Hz, 2H), 3.50 - 3.44 (m, 1H), 3.21 - 3.13 (m, 2H), 2.88 - 2.74 (m, 2H), 2.36 - 2.22 (m, 4H). LCMS (ESI) calcd for $C_{25}H_{26}N_5O_4^+$ $[M+H]^+$: 460.20, found, 460.2.

**Example 177: preparation of 2-(2,4-dioxotetrahydropyrimidin-1(2H)-yl)-5-((4-(6-fluoro-1H-indol-3-yl)-3,6-dihydropyridin-1(2H)-yl)methyl)isoindoline-1,3-dione (GT-07580)**

Referring to the method of Scheme 1, the target product GT-07580 was prepared as a white solid (31 mg, yield 52%). $^1H$ NMR (400 MHz, DMSO) δ 11.39 (s, 1H), 10.93 (s, 1H), 8.34 (s, 1H), 8.17 (d, *J* = 7.7 Hz, 1H), 8.09 (d, *J* = 7.6 Hz, 1H), 8.04 - 7.92 (m, 1H), 7.24 - 7.06 (m, 1H), 6.89 (t, *J* = 8.2 Hz, 1H), 6.77 - 6.73 (m, 1H), 4.66 - 4.35 (m, 2H), 3.85 - 3.80 (m, 3H), 3.73 - 3.61 (m, 1H), 3.27 - 3.23 (m, 2H), 3.12 - 2.97 (m, 1H), 2.87 - 2.83 (m, 2H), 2.80 - 2.66 (m, 2H). LCMS (ESI) calcd

for $C_{26}H_{23}FN_5O_4^+$ [M+H]$^+$: 488.17, found, 488.2.

**Example 178: preparation of 2-(2,4-dioxotetrahydropyrimidin-1(2H)-yl)-5-((4-(6-fluoro-1-methyl-1H-indol-3-yl) piperidin-1-yl)methyl)isoindoline-1,3-dione (GT-07581)**

Referring to the method of Scheme 1, the target product GT-07581 was prepared as a white solid (38 mg, yield 62%). $^1$H NMR (400 MHz, DMSO) δ 11.10 (s, 1H), 10.93 (s, 1H), 8.35 (s, 1H), 8.21 (d, *J* = 7.8 Hz, 1H), 8.12 (d, *J* = 7.6 Hz, 1H), 7.67 (dd, *J* = 8.5, 5.5 Hz, 1H), 7.25 (dd, *J* = 10.4, 2.2 Hz, 1H), 7.11 (s, 1H), 6.93 - 6.79 (m, 1H), 4.57 (s, 2H), 3.83 (t, *J* = 6.7 Hz, 2H), 3.70 (s, 3H), 3.47 (d, *J* = 10.8 Hz, 2H), 3.18 - 3.08 (m, 2H), 3.03 - 2.97 (m, 1H), 2.87 (t, *J* = 6.7 Hz, 2H), 2.12 - 2.02 (m, 4H). LCMS (ESI) calcd for $C_{27}H_{27}FN_5O_4^+$ [M+H]$^+$: 504.20, found, 504.1.

**Example 179: preparation of 5-((4-(1H-pyrrolo[2,3-b]pyridin-3-yl)piperidin-1-yl)methyl)-2-(2,4-dioxotetrahydropyrimidin-1(2H)-yl)isoindoline-1,3-dione (GT-07582)**

Referring to the method of Scheme 1, the target product GT-07582 was prepared as a white solid (35 mg, yield 60%). $^1$H NMR (400 MHz, DMSO) δ 12.15 (s, 1H), 11.59 (s, 1H), 10.93 (s, 1H), 8.57 - 8.52 (m, 1H), 8.39 (s, 1H), 8.33 (d, *J* = 5.2 Hz, 1H), 8.26 (d, *J* = 7.8 Hz, 1H), 8.12 (d, *J* = 7.6 Hz, 1H), 7.41 (s, 1H), 7.28 (dd, *J* = 7.7, 5.2 Hz, 1H), 4.59 (d, *J* = 4.1 Hz, 2H), 3.84 (d, *J* = 6.7 Hz, 2H), 3.47 (d, *J* = 11.4 Hz, 2H), 3.18 - 3.02 (m, 3H), 2.86 (t, *J* = 6.8 Hz, 2H), 2.32 - 2.21 (m, 2H), 2.09 (d, *J* = 13.4 Hz, 2H). LCMS (ESI) calcd for $C_{25}H_{25}N_6O_4^+$ [M+H]$^+$: 473.19, found, 473.1.

**Example 180: preparation of 2-(2,4-dioxotetrahydropyrimidin-1(2H)-yl)-5-((4-(5-fluoro-2-oxo-2,3-dihydro-1H-benzo[d]imidazol-1-yl)piperidin-1-yl)methyl)isoindoline-1,3-dione (GT-07583)**

Referring to the method of Scheme 1, the target product GT-07583 was prepared as a white solid (34 mg, yield 55%). $^1$H NMR (400 MHz, DMSO) δ 11.28 (s, 1H), 11.11 (s, 1H), 10.93 (s, 1H), 8.33 (s, 1H), 8.20 (d, *J* = 7.7 Hz, 1H), 8.12 (d, *J* = 7.7 Hz, 1H), 7.52 (s, 1H), 6.89 - 6.81 (m, 2H), 4.58 - 4.52 (m, 3H), 3.83 (t, *J* = 6.8 Hz, 2H), 3.51 (brs, 2H), 3.25 - 3.17 (m, 2H), 2.86 (t, *J* = 6.8 Hz, 3H), 2.79 (d, *J* = 12.9 Hz, 2H), 1.88 (d, *J* = 12.0 Hz, 2H). LCMS (ESI) calcd for $C_{25}H_{24}FN_6O_5^+$ [M+H]$^+$: 507.18, found, 507.1.

**Example 181: preparation of 1-(5-((4-(6-fluoro-3,4-dihydroquinolin-1(2H)-yl)piperidin-1-yl)methyl)-1-oxoisoindolin-2-yl)dihydropyrimidine-2,4(1H,3H)-dione (GT-07584)**

Referring to the method of Scheme 1, the target product GT-07584 was prepared as a white solid (25 mg, yield 53%). $^1$H NMR (400 MHz, DMSO) δ 10.69 (s, 1H), 8.43 - 8.22 (m, 1H), 8.03 - 7.72 (m, 4H), 6.83 - 6.67 (m, 1H), 4.89 - 4.73 (m, 1H), 4.67 - 4.48 (m, 2H), 4.48 - 4.35 (m, 1H), 3.82 - 3.78 (m, 2H), 3.75 - 3.63 (m, 1H), 3.45 - 3.42 (m, 2H), 3.34 - 3.21 (m, 3H), 3.14 - 2.99 (m, 2H), 2.92 - 2.84 (m, 3H), 2.71 - 2.63 (m, 2H), 2.48 - 2.39 (m, 1H), 2.25 - 2.17 (m, 1H), 1.86 - 1.70 (m, 2H). LCMS (ESI) calcd for $C_{27}H_{31}FN_5O_3^+$ [M+H]$^+$: 492.24, found, 492.3.

**Example 182: preparation of 1-(5-((4-(7-fluoro-2,3-dihydro-4H-benzo[b][1,4]oxazin-4-yl)piperidin-1-yl) methyl)-1-oxoisoindolin-2-yl)dihydropyrimidine-2,4(1H,3H)-dione (GT-07585)**

Referring to the method of Scheme 1, the target product GT-07585 was prepared as a white solid (19 mg, yield 40%). $^1$H NMR (400 MHz, DMSO) δ 10.75 (s, 1H), 10.70 (s, 1H), 7.90 - 7.88 (m, 2H), 7.77 (d, *J* = 8.0 Hz, 1H), 6.83 (dd, *J* = 8.7, 5.8 Hz, 1H), 6.62 - 6.57 (m, 2H), 4.82 (d, *J* = 17.0 Hz, 1H), 4.59 (d, *J* = 16.2 Hz, 1H), 4.43 (d, *J* = 4.4 Hz, 2H), 4.21 - 4.13 (m, 2H), 3.95 - 3.91 (m, 1H), 3.79 (t, *J* = 7.0 Hz, 2H), 3.47 (d, *J* = 11.1 Hz, 2H), 3.19 - 3.13 (m, 2H), 3.09 - 3.03 (m, 2H), 2.90 - 2.74 (m, 2H), 2.15 - 2.05 (m, 2H), 1.80 (d, *J* = 11.9 Hz, 2H). LCMS (ESI) calcd for $C_{26}H_{29}FN_5O_4^+$ [M+H]$^+$: 494.22, found, 494.3.

**Example 183: preparation of 1-(5-((4-(7-fluoro-3-oxo-2,3-dihydro-4H-benzo[b][1,4]oxazin-4-yl)piperidin-1-yl) methyl)-1-oxoisoindolin-2-yl)dihydropyrimidine-2,4(1H,3H)-dione (GT-07586)**

Referring to the method of Scheme 1, the target product GT-07586 was prepared as a white solid (35 mg, yield 72%). $^1$H NMR (400 MHz, DMSO) δ 10.70 (s, 1H), 10.62 (s, 1H), 7.90 (d, *J* = 7.8 Hz, 1H), 7.86 (s, 1H), 7.78 - 7.75 (m, 1H), 7.57 - 7.52 (m, 1H), 7.00 (dd, *J* = 9.2, 2.9 Hz, 1H), 6.91 (td, *J* = 8.6, 2.8 Hz, 1H), 4.82 (d, *J* = 16.7 Hz, 1H), 4.65 - 4.55 (m, 3H), 4.45 (d, *J* = 4.0 Hz, 2H), 3.79 (t, *J* = 6.8 Hz, 2H), 3.49 (d, *J* = 12.1 Hz, 2H), 3.21 - 3.10 (m, 2H), 2.97 - 2.65 (m, 5H), 1.91 (d, *J* = 12.4 Hz, 2H). LCMS (ESI) calcd for $C_{26}H_{27}FN_5O_5^+$ [M+H]$^+$: 508.20, found, 508.3.

**Example 184: preparation of 1-(5-((4-(7-fluorochroman-4-yl)piperidin-1-yl)methyl)-1-oxoisoindolin-2-yl)dihydropyrimidine-2,4(1H,3H)-dione (GT-07587)**

Referring to the method of Scheme 1, the target product GT-07587 was prepared as a white solid (13 mg, yield 28%). $^1H$ NMR (400 MHz, DMSO) δ 10.78 (s, 1H), 10.69 (s, 1H), 7.87 - 7.84 (m, 2H), 7.76 (d, *J* = 7.9 Hz, 1H), 7.16 - 7.09 (m, 1H), 6.70 - 6.65 (m, 1H), 6.62 - 6.57 (m, 1H), 4.80 (d, *J* = 16.5 Hz, 1H), 4.57 (d, *J* = 16.6 Hz, 1H), 4.39 (d, *J* = 4.2 Hz, 2H), 4.20 - 4.11 (m, 2H), 3.79 (t, *J* = 6.9 Hz, 2H), 3.45 - 3.35 (m, 3H), 2.89 - 2.82 (m, 2H), 2.73 - 2.64 (m, 1H), 1.95 (brs, 1H), 1.92 - 1.78 (m, 4H), 1.66 - 1.59 (m, 3H). LCMS (ESI) calcd for $C_{27}H_{30}FN_4O_4^+$ $[M+H]^+$: 493.22, found, 493.3.

**Example 185: preparation of 1-(5-((4-(7-fluorochroman-4-ylidene)piperidin-1-yl)methyl)-1-oxoisoindolin-2-yl) dihydropyrimidine-2,4(1H,3H)-dione (GT-07588)**

Referring to the method of Scheme 1, the target product GT-07588 was prepared as a white solid (8 mg, yield 17%). $^1H$ NMR (400 MHz, DMSO) δ 11.06 (s, 1H), 10.69 (s, 1H), 7.92 - 7.83 (m, 2H), 7.77 (d, *J* = 7.8 Hz, 1H), 7.26 - 7.14 (m, 1H), 6.75 - 6.62 (m, 2H), 4.81 (d, *J* = 16.7 Hz, 1H), 4.58 (d, *J* = 16.3 Hz, 1H), 4.46 (d, *J* = 4.0 Hz, 2H), 4.31 (d, *J* = 5.1 Hz, 1H), 4.23 - 4.19 (m, 1H), 3.79 (t, *J* = 6.9 Hz, 2H), 3.47 - 3.42 (m, 2H), 3.28 - 3.24 (m, 3H), 3.01 - 2.78 (m, 5H), 2.75 - 2.64 (m, 2H). LCMS (ESI) calcd for $C_{27}H_{28}FN_4O_4^+$ $[M+H]^+$: 491.21, found, 491.3.

**Example 186: preparation of 1-(5-((4-(5-fluoronaphthalen-1-yl)piperidin-1-yl)methyl)-1-oxoisoindolin-2-yl)dihydropyrimidine-2,4(1H,3H)-dione (GT-07589)**

Referring to the method of Scheme 1, the target product GT-07589 was prepared as a white solid (30 mg, yield 64%). $^1H$ NMR (400 MHz, DMSO) δ 11.29 (s, 1H), 10.70 (s, 1H), 8.06 (d, *J* = 8.7 Hz, 1H), 7.99 - 7.95 (m, 2H), 7.90 (d, *J* = 7.7 Hz, 1H), 7.85 (d, *J* = 7.3 Hz, 1H), 7.62 (t, *J* = 7.8 Hz, 1H), 7.55 (dd, *J* = 14.6, 7.8 Hz, 1H), 7.48 (d, *J* = 7.2 Hz, 1H), 7.42 - 7.32 (m, 1H), 4.84 (d, *J* = 16.5 Hz, 1H), 4.61 (d, *J* = 16.6 Hz, 1H), 4.51 (s, 2H), 3.80 (t, *J* = 6.8 Hz, 2H), 3.68 (t, *J* = 11.8 Hz, 1H), 3.53 (d, *J* = 9.7 Hz, 2H), 3.25 - 3.18 (m, 2H), 2.95 - 2.71 (m, 2H), 2.33 - 2.22 (m, 2H), 2.07 - 2.04 (m, 2H). LCMS (ESI) calcd for $C_{28}H_{28}FN_4O_3^+$ $[M+H]^+$: 487.21, found, 487.3.

**Example 187: preparation of 1-(5-((4-(5-fluoronaphthalen-1-yl)-3,6-dihydropyridin-1(2H)-yl)methyl)-1-oxoisoindolin-2-yl)dihydropyrimidine-2,4(1H,3H)-dione (GT-07590)**

Referring to the method of Scheme 1, the target product GT-07590 was prepared as a white solid (31 mg, yield 67%). $^1H$ NMR (400 MHz, DMSO) δ 11.61 (s, 1H), 10.70 (s, 1H), 8.04 - 8.00 (m, 3H), 7.92 (s, 2H), 7.63 (t, *J* = 7.7 Hz, 1H), 7.55 - 7.48 (m, 1H), 7.45 (d, *J* = 7.0 Hz, 1H), 7.40 - 7.32 (m, 1H), 5.75 (s, 1H), 4.84 (d, *J* = 16.6 Hz, 1H), 4.67 - 4.61 (m, 3H), 3.96 - 3.74 (m, 4H), 3.72 - 3.67 (m, 1H), 3.46 - 3.44 (m, 1H), 3.15 - 2.97 (m, 1H), 2.94 - 2.73 (m, 2H), 2.71 - 2.55 (m, 1H). LCMS (ESI) calcd for $C_{28}H_{26}FN_4O_3^+$ $[M+H]^+$: 485.20, found, 485.3.

**Example 188: preparation of 1-(5-((4-(isoquinolin-5-yl)piperidin-1-yl)methyl)-1-oxoisoindolin-2-yl)dihydropyrimidine-2,4(1H,3H)-dione (GT-07644)**

Referring to the method of Scheme 1, the target product GT-07644 was prepared as a white solid (14 mg, yield 31%). $^1H$ NMR (400 MHz, DMSO) δ 11.24 (s, 1H), 10.70 (s, 1H), 9.72 (s, 1H), 8.68 (d, *J* = 6.6 Hz, 1H), 8.52 (d, *J* = 6.6 Hz, 1H), 8.31 (d, *J* = 6.6 Hz, 1H), 7.96 (s, 1H), 7.94 - 7.88 (m, 3H), 7.85 (d, *J* = 8.4 Hz, 1H), 4.84 (d, *J* = 16.8 Hz, 1H), 4.60 (d, *J* = 16.3 Hz, 1H), 4.52 (d, *J* = 3.7 Hz, 2H), 3.80 (t, *J* = 6.8 Hz, 2H), 3.28 - 3.19 (m, 4H), 2.82 - 276 (m, 3H), 2.36 - 2.24 (m, 2H), 2.05 (d, *J* = 13.8 Hz, 2H). LCMS (ESI) calcd for $C_{27}H_{28}N_5O_3^+$ $[M+H]^+$: 470.22, found, 470.2.

**Example 189: preparation of 1-(5-((4-(7-fluoroquinolin-4-yl)piperidin-1-yl)methyl)-1-oxoisoindolin-2-yl)dihydropyrimidine-2,4(1H,3H)-dione (GT-07645)**

Referring to the method of Scheme 1, the target product GT-07645 was prepared as a white solid (18 mg, yield 39%). $^1H$ NMR (400 MHz, DMSO) δ 11.76 (s, 1H), 10.70 (s, 1H), 9.15 (t, *J* = 4.0 Hz, 1H), 8.65 - 8.61 (m, 1H), 8.07 - 8.05(m, 1H), 7.99 (s, 1H), 7.83 (s, 2H), 7.81 (t, *J* = 8.7 Hz, 1H), 7.66 (t, *J* = 8.2 Hz, 1H), 4.85 (d, *J* = 16.6 Hz, 1H), 4.60 (d, *J* = 16.7 Hz, 1H), 4.52 (d, *J* = 3.8 Hz, 2H), 3.81 (t, *J* = 6.8 Hz, 3H), 3.57 (d, *J* = 11.2 Hz, 2H), 3.24 (brs, 2H), 2.96 - 2.72 (m, 2H), 2.47 - 2.30 (m, 2H), 2.06 (d, *J* = 13.2 Hz, 2H). LCMS (ESI) calcd for $C_{27}H_{27}FN_5O_3^+$ $[M+H]^+$: 488.21, found, 488.2.

**Example 190: preparation of 2-(2,4-dioxotetrahydropyrimidin-1(2H)-yl)-5-((4-(6-fluoro-3,4-dihydroquinolin-1(2H)-yl)piperidin-1-yl)methyl)isoindoline-1,3-dione (GT-07681)**

Referring to the method of Scheme 1, the target product GT-07681 was prepared as a white solid (29 mg, yield 63%). $^1$H NMR (400 MHz, DMSO) δ 11.45 (s, 1H), 10.93 (s, 1H), 8.33 (s, 1H), 8.20 (d, *J* = 7.8 Hz, 1H), 8.11 (d, *J* = 7.7 Hz, 1H), 6.80 - 6.72 (m, 3H), 4.51 (s, 2H), 3.68 - 3.64(m, 1H), 3.48 - 3.45 (m, 2H), 3.14 - 3.07 (m, 5H), 2.86 (t, *J* = 6.7 Hz, 2H), 2.65 (t, *J* = 6.3 Hz, 2H), 2.28 - 2.18 (m, 2H), 1.84 - 1.77 (m, 5H). LCMS (ESI) calcd for $C_{27}H_{29}FN_5O_4^+$ $[M+H]^+$: 506.22, found, 506.2.

**Example 191: preparation of 2-(2,4-dioxotetrahydropyrimidin-1(2H)-yl)-5-((4-(7-fluoro-2,3-dihydro-4H-benzo[b][1,4]oxazin-4-yl)piperidin-1-yl)methyl)isoindoline-1,3-dione (GT-07682)**

Referring to the method of Scheme 1, the target product GT-07682 was prepared as a white solid (27 mg, yield 58%). $^1$H NMR (400 MHz, DMSO) δ 11.54 (s, 1H), 10.93 (s, 1H), 8.34 (s, 1H), 8.21 (dd, *J* = 7.8, 1.1 Hz, 1H), 8.11 (d, *J* = 7.6 Hz, 1H), 6.87 - 6.79 (m, 1H), 6.59 - 6.57 (m, 2H), 4.52 (s, 2H), 4.18 (t, *J* = 3.7 Hz, 2H), 3.84 (t, *J* = 6.8 Hz, 2H), 3.47 (d, *J* = 11.1 Hz, 2H), 3.15 - 3.13 (m, 2H), 3.09 (d, *J* = 9.9 Hz, 2H), 2.86 (t, *J* = 6.8 Hz, 2H), 2.24 - 2.15 (m, 3H), 1.79 (d, *J* = 11.8 Hz, 2H). LCMS (ESI) calcd for $C_{26}H_{27}FN_5O_5^+$ $[M+H]^+$: 508.20, found, 508.1.

**Example 192: preparation of 2-(2,4-dioxotetrahydropyrimidin-1(2H)-yl)-5-((4-(7-fluoro-3-oxo-2,3-dihydro-4H-benzo[b][1,4]oxazin-4-yl)piperidin-1-yl)methyl)isoindoline-1,3-dione (GT-07683)**

Referring to the method of Scheme 1, the target product GT-07683 was prepared as a white solid (22 mg, yield 46%). $^1$H NMR (400 MHz, DMSO-d6) δ 10.93 (s, 1H), 10.78 (s, 1H), 8.29 (s, 1H), 8.16 - 8.11 (m, 2H), 7.56 - 7.52 (m, 1H), 6.99 (dd, *J* = 9.2, 2.8 Hz, 1H), 6.94 - 4.83 (m, 1H), 4.58 (s, 2H), 4.54 (s, 1H), 4.43 (s, 1H), 3.83 (t, *J* = 6.8 Hz, 2H), 3.69 - 3.64 (m, 1H), 3.49 (brs, 2H), 3.15 (brs, 2H), 2.91 - 2.85 (m, 4H), 1.99 - 1.91 (m, 2H). LCMS (ESI) calcd for $C_{26}H_{25}FN_5O_6^+$ $[M+H]^+$: 522.18, found, 522.1.

**Example 193: preparation of 2-(2,4-dioxotetrahydropyrimidin-1(2H)-yl)-5-((4-(7-fluorochroman-4-yl)piperidin-1-yl)methyl)isoindoline-1,3-dione (GT-07684)**

Referring to the method of Scheme 1, the target product GT-07684 was prepared as a white solid (13 mg, yield 28%). $^1$H NMR (400 MHz, DMSO-d6) δ 10.92 (s, 1H), 10.82 (s, 1H), 8.27 (s, 1H), 8.12 - 8.07 (m, 2H), 7.13 (t, *J* = 7.6 Hz, 1H), 6.67 (td, *J* = 8.5, 2.6 Hz, 1H), 6.59 (dd, *J* = 10.5, 2.5 Hz, 1H), 4.47 (s, 2H), 4.25 - 4.05 (m, 3H), 3.82 (t, *J* = 6.8 Hz, 2H), 2.94 - 2.84 (m, 4H), 2.73 - 2.67 (m, 2H), 1.95 (s, 2H), 1.86 - 1.80 (m, 4H), 1.69 (d, *J* = 17.2 Hz, 2H). LCMS (ESI) calcd for $C_{27}H_{28}FN_4O_5^+$ $[M+H]^+$: 507.20, found, 507.1.

**Example 194: preparation of 2-(2,4-dioxotetrahydropyrimidin-1(2H)-yl)-5-((4-(7-fluorochroman-4-ylidene)piperidin-1-yl)methyl)isoindoline-1,3-dione (GT-07685)**

Referring to the method of Scheme 1, the target product GT-07685 was prepared as a white solid (7 mg, yield 15%). $^1$H NMR (400 MHz, DMSO-d6) δ 11.17 (s, 1H), 10.92 (s, 1H), 8.28 (s, 1H), 8.19 - 8.05 (m, 2H), 7.25 - 7.16 (m, 1H), 6.72 - 6.64 (m, 2H), 4.55 (s, 2H), 4.30 - 4.22 (m, 3H), 3.83 (t, *J* = 4.9 Hz, 2H), 3.51 - 3.47 (m, 2H), 3.05 - 2.94 (m, 4H), 2.86 (t, *J* = 5.5 Hz, 2H), 2.74 - 2.64 (m, 3H). LCMS (ESI) calcd for $C_{27}H_{26}FN_4O_5^+$ $[M+H]^+$: 505.19, found, 505.1.

**Example 195: preparation of 2-(2,4-dioxotetrahydropyrimidin-1(2H)-yl)-5-((4-(5-fluoronaphthalen-1-yl)piperidin-1-yl)methyl)isoindoline-1,3-dione (GT-07686)**

Referring to the method of Scheme 1, the target product GT-07686 was prepared as a white solid (21 mg, yield 46%). $^1$H NMR (400 MHz, DMSO-d6) δ 11.01 (s, 1H), 10.93 (s, 1H), 8.35 (s, 1H), 8.21 (d, *J* = 7.6 Hz, 1H), 8.14 (d, *J* = 7.6 Hz, 1H), 8.06 (d, *J* = 8.7 Hz, 1H), 7.98 (d, *J* = 8.4 Hz, 1H), 7.66 - 7.60 (m, 1H), 7.58 - 7.53 (m, 1H), 7.48 (d, *J* = 7.2 Hz, 1H), 7.36 (dd, *J* = 10.7, 7.8 Hz, 1H), 4.60 (s, 2H), 3.84 (t, *J* = 6.8 Hz, 2H), 3.72 - 3.64 (m, 2H), 3.62 - 3.46 (m, 3H), 2.87 (t, *J* = 6.8 Hz, 2H), 2.24 - 2.19 (m, 2H), 2.13 - 2.04 (m, 2H). LCMS (ESI) calcd for $C_{28}H_{26}FN_4O_4^+$ $[M+H]^+$: 501.19, found, 501.1.

**Example 196: preparation of 2-(2,4-dioxotetrahydropyrimidin-1(2H)-yl)-5-((4-(5-fluoronaphthalen-1-yl)-3,6-dihydropyridin-1(2H)-yl)methyl)isoindoline-1,3-dione (GT-07687)**

Referring to the method of Scheme 1, the target product GT-07687 was prepared as a white solid (21 mg, yield 46%). $^1$H NMR (400 MHz, DMSO-d6) δ 11.47 (s, 1H), 10.93 (s, 1H), 8.40 (s, 1H), 8.27 (s, 1H), 8.15 (d, *J* = 7.6 Hz, 1H), 8.03

(d, *J* = 8.4 Hz, 2H), 7.98 (d, *J* = 8.2 Hz, 2H), 7.67 - 7.60 (m, 1H), 7.55 - 7.48 (m, 1H), 7.47 (d, *J* = 5.1 Hz, 1H), 7.37 (dd, *J* = 10.6, 7.8 Hz, 1H), 5.75 (s, 1H), 4.81 - 4.66 (m, 2H), 3.94 - 3.82 (m, 4H), 3.75 - 3.61 (m, 2H), 3.00 (brs, 1H), 2.87 (t, *J* = 6.8 Hz, 3H), 2.62 (d, *J* = 17.6 Hz, 1H). LCMS (ESI) calcd for $C_{28}H_{24}FN_4O_4^+$ [M+H]$^+$: 499.18, found, 499.1.

**Example 197: preparation of 2-(2,4-dioxotetrahydropyrimidin-1(2H)-yl)-5-((4-(isoquinolin-5-yl)piperidin-1-yl)methyl)isoindoline-1,3-dione (GT-07688)**

Referring to the method of Scheme 1, the target product GT-07688 was prepared as a white solid (18 mg, yield 41%). $^1$H NMR (400 MHz, DMSO-d6) δ 11.80 (s, 1H), 10.94 (s, 1H), 9.79 (s, 1H), 8.70 (d, *J* = 6.6 Hz, 1H), 8.59 (d, *J* = 6.7 Hz, 1H), 8.41 (s, 1H), 8.37 - 8.34 (m, 1H), 8.28 (dd, *J* = 7.8, 1.1 Hz, 1H), 8.14 (d, *J* = 7.7 Hz, 1H), 7.98 - 7.92 (m, 2H), 4.62 (s, 2H), 3.84 (t, *J* = 6.8 Hz, 2H), 3.77 - 3.63 (m, 5H), 2.87 (t, *J* = 6.8 Hz, 2H), 2.42 - 2.28 (m, 2H), 2.05 (d, *J* = 12.9 Hz, 2H). LCMS (ESI) calcd for $C_{27}H_{26}N_5O_4^+$ [M+H]$^+$: 484.20, found, 484.1.

**Example 198: preparation of 2-(2,4-dioxotetrahydropyrimidin-1(2H)-yl)-5-((4-(7-fluoroquinolin-4-yl)piperidin-1-yl)methyl)isoindoline-1,3-dione (GT-07689)**

Referring to the method of Scheme 1, the target product GT-07689 was prepared as a white solid (31 mg, yield 68%). $^1$H NMR (400 MHz, DMSO-d6) δ 11.76 (s, 1H), 10.94 (s, 1H), 9.09 (t, *J* = 5.7 Hz, 1H), 8.63 - 8.53 (m, 1H), 8.40 (s, 1H), 8.27 (d, *J* = 7.7 Hz, 1H), 8.14 (d, *J* = 7.7 Hz, 1H), 8.01 - 7.97 (m, 1H), 7.82 - 7.72 (m, 1H), 7.61 - 7.58 (m, 1H), 4.62 (s, 2H), 3.89 - 3.82 (m, 3H), 3.58 (d, *J* = 11.6 Hz, 2H), 3.32 - 3.19 (m, 2H), 2.87 (t, *J* = 6.8 Hz, 2H), 2.44 - 2.27 (m, 2H), 2.07 (d, *J* = 12.9 Hz, 2H). LCMS (ESI) calcd for $C_{27}H_{25}FN_5O_4^+$ [M+H]$^+$: 502.19, found, 502.1.

**Example 199: preparation of 1-(5-((3-(7-fluorochroman-4-yl)azetidin-1-yl)methyl)-1-oxoisoindolin-2-yl)dihydropyrimidine-2,4(1H,3H)-dione (GT-07690)**

Referring to the method of Scheme 1, the target product GT-07690 was prepared as a white solid (3 mg, yield 7%). $^1$H NMR (400 MHz, DMSO-d6) δ 10.93 (s, 1H), 10.69 (s, 1H), 7.86 (d, *J* = 7.8 Hz, 1H), 7.81 (d, *J* = 10.2 Hz, 1H), 7.70 (t, *J* = 8.0 Hz, 1H), 7.09 - 7.02 (m, 1H), 6.68 - 6.60 (m, 2H), 4.80 (d, *J* = 16.0 Hz, 2H), 4.63 - 4.46 (m, 3H), 4.20 - 4.10 (m, 2H), 4.07 - 3.99 (m, 3H), 3.79 (t, *J* = 6.8 Hz, 2H), 3.24 - 3.14 (m, 1H), 2.98 - 2.73 (m, 3H), 2.01 - 1.88 (m, 1H), 1.70 - 1.64 (m, 1H). LCMS (ESI) calcd for $C_{25}H_{26}FN_4O_4^+$ [M+H]$^+$: 465.19, found, 465.1.

**Example 200: preparation of 1-(5-((3-(7-fluoroquinolin-4-yl)azetidin-1-yl)methyl)-1-oxoisoindolin-2-yl)dihydropyrimidine-2,4(1H,3H)-dione (GT-07711)**

Referring to the method of Scheme 1, the target product GT-07711 was prepared as a white solid (4 mg, yield 9%). $^1$H NMR (400 MHz, DMSO-d6) δ 11.90 (s, 1H), 10.69 (s, 1H), 9.05 (d, *J* = 4.6 Hz, 1H), 7.96 (dd, *J* = 9.3, 6.0 Hz, 1H), 7.91 (s, 1H), 7.88 (dd, *J* = 7.1, 4.4 Hz, 1H), 7.84 (s, 1H), 7.81 (d, *J* = 5.4 Hz, 1H), 7.75 (d, *J* = 8.4 Hz, 1H), 7.63 (d, *J* = 4.4 Hz, 1H), 4.82 - 4.73 (m, 3H), 4.64 - 4.35 (m, 4H), 4.50 - 4.43 (m, 2H), 3.79 (t, *J* = 6.8 Hz, 2H), 2.94 - 2.74 (m, 2H). LCMS (ESI) calcd for $C_{25}H_{23}FN_5O_3^+$ [M+H]$^+$: 460.18, found, 460.1.

**Example 201: preparation of 2-(2,4-dioxotetrahydropyrimidin-1(2H)-yl)-5-((3-(7-fluorochroman-4-yl)azetidin-1-yl)methyl)isoindoline-1,3-dione (GT-07715)**

Referring to the method of Scheme 1, the target product GT-07715 was prepared as a white solid (10 mg, yield 23%). $^1$H NMR (400 MHz, DMSO-d6) δ 11.46 (s, 1H), 10.92 (s, 1H), 8.22 (s, 1H), 8.12 - 8.07 (m, 2H), 7.07 (brs, 1H), 6.68 - 6.60 (m, 2H), 4.57 (s, 2H), 4.15 - 4.12 (m, 2H), 4.03 (brs, 3H), 3.83 (t, *J* = 6.8 Hz, 2H), 3.19 (brs, 2H), 2.97 - 2.92 (dm, 1H), 2.86 (t, *J* = 6.8 Hz, 3H), 2.02 - 1.87 (m, 1H), 1.75 - 1.59 (m, 1H). LCMS (ESI) calcd for $C_{25}H_{24}FN_4O_5^+$ [M+H]$^+$: 479.17, found, 479.1.

**Example 202: preparation of 2-(2,4-dioxotetrahydropyrimidin-1(2H)-yl)-5-((3-(7-fluoroquinolin-4-yl)azetidin-1-yl)methyl)isoindoline-1,3-dione (GT-07718)**

Referring to the method of Scheme 1, the target product GT-07718 was prepared as a white solid (25 mg, yield 58%). $^1$H NMR (400 MHz, DMSO-d6) δ 10.93 (s, 1H), 9.23 - 9.18 (m, 1H), 8.29 (s, 1H), 8.18 (d, *J* = 7.9 Hz, 1H), 8.14 - 8.00 (m, 3H), 7.88 - 7.82 (m, 1H), 7.72 - 7.67 (m, 1H), 4.85 - 7.78 (m, 1H), 4.66 (d, *J* = 7.1 Hz, 2H), 4.59 - 4.45 (m, 4H), 3.83 (t, *J* = 6.8 Hz, 2H), 2.86 (t, *J* = 6.8 Hz, 2H). LCMS (ESI) calcd for $C_{25}H_{21}FN_5O_4^+$ [M+H]$^+$: 474.16, found, 474.1.

**Example 203: preparation of 1-(1-oxo-5-((3-(trifluoromethyl)-5,6-dihydro-[1,2,4]triazolo[4,3-a]pyrazin-7(8H)-yl) methyl)isoindolin-2-yl)dihydropyrimidine-2,4(1H,3H)-dione (GT-07719)**

Referring to the method of Scheme 1, the target product GT-07719 was prepared as a white solid (6 mg, yield 19%). $^1$H NMR (400 MHz, DMSO-d6) δ 10.67 (s, 1H), 7.80 (d, *J* = 7.8 Hz, 1H), 7.74 (s, 1H), 7.63 (d, *J* = 7.6 Hz, 1H), 4.77 (d, *J* = 16.4 Hz, 1H), 4.55 (d, *J* = 16.4 Hz, 1H), 4.27 (t, *J* = 4.9 Hz, 2H), 4.13 (d, *J* = 6.6 Hz, 4H), 3.90 - 3.85 (m, 2H), 3.18 (brs, 2H), 2.88 - 2.72 (m, 2H). LCMS (ESI) calcd for $C_{19}H_{19}F_3N_7O_3^+$ [M+H]$^+$: 450.15, found, 450.1.

**Example 204: preparation of 1-(5-((6,7-dihydrothieno[3,2-c]pyridin-5(4H)-yl)methyl)-1-oxoisoindolin-2-yl)dihydropyrimidine-2,4(1H,3H)-dione (GT-07720)**

Referring to the method of Scheme 1, the target product GT-07720 was prepared as a white solid (25 mg, yield 65%). $^1$H NMR (400 MHz, DMSO-d6) δ 11.21 (s, 1H), 10.69 (s, 1H), 7.93 - 7.88 (m, 2H), 7.83 (s, 1H), 7.46 (d, *J* = 4.9 Hz, 1H), 6.89 (d, *J* = 5.0 Hz, 1H), 4.82 (d, *J* = 16.5 Hz, 1H), 4.67 (d, *J* = 12.9 Hz, 1H), 4.61 - 4.54 (m, 2H), 4.22 (brs, 2H), 3.84 - 3.71 (m, 3H), 3.46 - 3.42 (m, 1H), 3.28 - 3.21 (m, 1H), 3.13 (d, *J* = 14.6 Hz, 1H), 2.90 - 2.74 (m, 2H). LCMS (ESI) calcd for $C_{20}H_{21}N_4O_3S^+$ [M+H]$^+$: 397.13, found, 397.1.

**Example 205: preparation of 1-(5-((4-(4-((3r,5r,7r)-adamantan-1-yl)benzyl)piperazin-1-yl)methyl)-1-oxoisoindolin-2-yl)dihydropyrimidine-2,4(1H,3H)-dione (GT-07721)**

Referring to the method of Scheme 1, the target product GT-07721 was prepared as a white solid (4 mg, yield 7%). $^1$H NMR (400 MHz, DMSO-d6) δ 10.68 (s, 1H), 7.81 (d, *J* = 7.9 Hz, 1H), 7.79 - 7.69 (m, 1H), 7.68 - 7.63 (m, 1H), 7.52 - 7.48 (m, 2H), 7.43 (d, *J* = 8.1 Hz, 2H), 4.77 (d, *J* = 16.9 Hz, 1H), 4.54 (d, *J* = 16.4 Hz, 1H), 4.27 (brs, 2H), 3.80 - 3.76 (m, 2H), 3.41 - 3.34 (m, 8H), 3.23 - 2.09 (m, 2H), 2.88 - 2.71 (m, 2H), 2.06 (s, 3H), 1.86 (s, 6H), 1.74 (s, 6H). LCMS (ESI) calcd for $C_{34}H_{42}N_5O_3^+$ [M+H]$^+$: 568.33, found, 568.3.

**Example 206: preparation of 5-((4-(4-((3r,5r,7r)-adamantan-1-yl)benzyl)piperazin-1-yl)methyl)-2-(2,4-dioxotetrahydropyrimidin-1(2H)-yl)isoindoline-1,3-dione (GT-07722)**

Referring to the method of Scheme 1, the target product GT-07722 was prepared as a white solid (7 mg, yield 13%). $^1$H NMR (400 MHz, DMSO-d6) δ 10.91 (s, 1H), 8.11 (s, 1H), 8.02 (s, 2H), 7.51 (d, *J* = 7.7 Hz, 2H), 7.43 (d, *J* = 8.3 Hz, 2H), 4.27 (s, 2H), 3.82 (t, *J* = 6.8 Hz, 2H), 3.64 - 3.59 (m, 2H), 3.38 (brs, 4H), 3.18 (brs, 4H), 2.85 (t, *J* = 6.8 Hz, 2H), 2.06 (s, 3H), 1.86 (s, 6H), 1.74 (s, 6H). LCMS (ESI) calcd for $C_{34}H_{40}N_5O_4^+$ [M+H]$^+$: 582.31, found, 582.2.

**Example 207: preparation of 1-(5-((3-(5-fluoro-1H-benzo[d]imidazol-1-yl)azetidin-1-yl)methyl)-1-oxoisoindolin-2-yl)dihydropyrimidine-2,4(1H,3H)-dione (GT-07723)**

Referring to the method of Scheme 1, the target product GT-07723 was prepared as a white solid (15 mg, yield 35%). $^1$H NMR (400 MHz, DMSO-d6) δ 10.68 (s, 1H), 9.01 (s, 1H), 7.85 (d, *J* = 7.9 Hz, 1H), 7.83 (s, 1H), 7.77 (d, *J* = 7.5 Hz, 1H), 7.73 (d, *J* = 8.6 Hz, 1H), 7.69 (dd, *J* = 7.4, 1.0 Hz, 1H), 7.64 (d, *J* = 7.5 Hz, 1H), 7.55 (t, *J* = 7.4 Hz, 1H), 4.88 - 4.69 (m, 1H), 4.58 - 4.52 (m, 1H), 4.25 (s, 1H), 3.82 - 3.77 (m, 2H), 2.95 - 2.70 (m, 2H), 2.17 (s, 1H), 1.98 (s, 2H), 1.72 - 1.61 (m, 3H). LCMS (ESI) calcd for $C_{23}H_{22}FN_6O_3^+$ [M+H]$^+$: 449.17, found, 449.1.

**Example 208: preparation of 1-(5-((8-((4'-chloro-5,5-dimethyl-3,4,5,6-tetrahydro-[1,1'-biphenyl]-2-yl) methyl)-3,8-diazabicyclo[3.2.1]octan-3-yl)methyl)-1-oxoisoindolin-2-yl)dihydropyrimidine-2,4(1H,3H)-dione (GT-07886)**

Referring to the method of Scheme 1, the target product GT-07886 was prepared as a white solid (32 mg, yield 48%). $^1$H NMR (400 MHz, DMSO-d6) δ 10.67 (s, 1H), 7.75 (d, *J* = 7.3 Hz, 1H), 7.66 - 7.55 (m, 2H), 7.44 (d, *J* = 8.4 Hz, 2H), 7.20 (d, *J* = 8.3 Hz, 2H), 4.74 (d, *J* = 16.2 Hz, 1H), 4.51 (d, *J* = 16.3 Hz, 1H), 3.87 - 3.75 (m, 9H), 3.49 (s, 2H), 3.10 - 3.04 (m, 1H), 2.88 - 2.80 (m, 1H), 2.80 - 2.74 (m, 1H), 2.43 - 2.32 (m, 2H), 2.07 (s, 2H), 1.92 - 1.80 (m, 2H), 1.47 (t, *J* = 6.2 Hz, 2H), 1.28 - 1.19 (m, 2H), 0.96 (s, 6H). LCMS (ESI) calcd for $C_{34}H_{41}ClN_5O_3^+$ [M+H]$^+$: 602.29, found, 602.2.

**Example 209: preparation of 5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-2-(2,4-dioxotetrahydropyrimidin-1(2H)-yl)isoindoline-1,3-dione (GT-07887)**

Referring to the method of Scheme 1, the target product GT-07887 was prepared as a white solid (33 mg, yield

48%). $^1$H NMR (400 MHz, DMSO-d6) δ 10.91 (s, 1H), 7.98 - 7.91 (m, 3H), 7.44 (d, *J* = 8.4 Hz, 2H), 7.20 (d, *J* = 8.4 Hz, 2H), 3.81 (t, *J* = 6.8 Hz, 4H), 3.73 (brs, 4H), 3.48 (s, 2H), 2.85 (t, *J* = 6.8 Hz, 2H), 2.76 - 2.67 (m, 2H), 2.36 (s, 2H), 2.07 (s, 2H), 1.83 (s, 2H), 1.47 (t, *J* = 6.2 Hz, 2H), 1.22 (s, 2H), 0.96 (s, 6H). LCMS (ESI) calcd for $C_{34}H_{39}ClN_5O_4^+$ [M+H]$^+$: 616.27, found, 616.2.

**Example 210: preparation of 2-(2,4-dioxotetrahydropyrimidin-1(2H)-yl)-5-((3-(7-fluorochroman-4-ylidene)azetidin-1-yl)methyl)isoindoline-1,3-dione (GT-07888)**

Referring to the method of Scheme 1, the target product GT-07888 was prepared as a white solid (16 mg, yield 27%). $^1$H NMR (400 MHz, DMSO-d6) δ 11.80 (s, 1H), 10.93 (s, 1H), 8.30 (s, 1H), 8.19 (d, *J* = 7.7 Hz, 1H), 8.11 (d, *J* = 7.7 Hz, 1H), 7.05 - 7.01 (m, 1H), 6.82 - 6.73 (m, 2H), 5.20 - 5.04 (m, 2H), 4.94 - 4.89 (m, 2H), 4.75 (s, 2H), 4.22 - 4.16 (m, 2H), 3.83 (t, *J* = 6.7 Hz, 2H), 2.86 (t, *J* = 6.7 Hz, 2H), 2.45 - 2.41 (m, 2H). LCMS (ESI) calcd for $C_{25}H_{22}FN_4O_5^+$ [M+H]$^+$: 477.16, found, 477.1.

**Example 211: preparation of 1-((1R,4R)-7,7-dimethyl-2-oxobicyclo[2.2.1]heptan-1-yl)-N-((2-(2,4-dioxotetrahydropyrimidin-1(2H)-yl)-1,3-dioxoisoindolin-5-yl)methyl)methanesulfonamide (GT-07998)**

Referring to the method of Scheme 2, the target product GT-07998 was prepared as a white solid (33 mg, yield 66%). $^1$H NMR (400 MHz, DMSO-d6) δ 10.90 (s, 1H), 7.99 (d, *J* = 7.7 Hz, 1H), 7.96 (s, 1H), 7.91 (d, *J* = 7.8 Hz, 1H), 7.85 (t, *J* = 5.9 Hz, 1H), 4.45 (d, *J* = 6.2 Hz, 2H), 3.82 (t, *J* = 6.8 Hz, 2H), 3.37 (dd, *J* = 14.9, 2.8 Hz, 1H), 2.97 (dd, *J* = 14.9, 5.4 Hz, 1H), 2.85 (t, *J* = 6.8 Hz, 2H), 2.39 - 2.33 (m, 2H), 2.07 - 2.04 (m, 1H), 2.00 - 1.88 (m, 2H), 1.60 - 1.53 (m, 1H), 1.43 - 1.37 (m, 1H), 1.02 (s, 3H), 0.79 (d, *J* = 1.8 Hz, 3H). LCMS (ESI) calcd for $C_{23}H_{27}N_4O_7S^+$ [M+H]$^+$: 503.16, found, 503.1.

**Example 212: preparation of 1-((1R,4R)-7,7-dimethyl-2-oxobicyclo[2.2.1]heptan-1-yl)-N-((2-(2,4-dioxotetrahydropyrimidin-1(2H)-yl)-1-oxoisoindolin-5-yl)methyl)methanesulfonamide (GT-08133)**

Referring to the method of Scheme 2, the target product GT-08133 was prepared as a white solid (10 mg, yield 37%). $^1$H NMR (400 MHz, DMSO-d6) δ 10.59 (s, 1H), 7.70 - 7.65 (m, 2H), 7.55 (s, 1H), 7.46 (d, *J* = 7.8 Hz, 1H), 4.69 (d, *J* = 16.2 Hz, 1H), 4.46 (d, *J* = 16.6 Hz, 1H), 4.28 (d, *J* = 6.3 Hz, 2H), 3.72 - 3.68 (m, 2H), 3.11 - 2.96 (m, 2H), 2.86 - 2.80 (m, 2H), 2.70 - 2.66 (m, 1H), 2.29 - 2.25 (m, 2H), 1.90 - 1.83 (m, 2H), 1.51 - 1.45 (m, 1H), 1.40 - 1.26 (m, 1H), 0.93 (s, 3H), 0.70 (s, 3H). LCMS (ESI) calcd for $C_{23}H_{29}N_4O_6S^+$ [M+H]$^+$: 489.18, found, 489.1.

**Example 213: preparation of 1-(5-((((3s,5s,7s)-adamantan-1-yl)amino)methyl)-1-oxoisoindolin-2-yl)dihydropyrimidine-2,4(1H,3H)-dione (GT-07806)**

Referring to the method of Scheme 1, the target product GT-07806 was prepared as a white solid (9 mg, yield 23%). $^1$H NMR (400 MHz, DMSO-d6) δ 10.62 (s, 1H), 8.94 (s, 2H), 7.79 (d, *J* = 7.8 Hz, 1H), 7.76 (s, 1H), 7.67 (d, *J* = 7.8 Hz, 1H), 4.74 (d, *J* = 17.2 Hz, 1H), 4.49 (d, *J* = 16.5 Hz, 1H), 4.18 (t, *J* = 5.7 Hz, 2H), 3.73 (t, *J* = 6.5 Hz, 2H), 2.88 - 2.63 (m, 2H), 2.10 (s, 3H), 1.91 (s, 6H), 1.60 (dd, *J* = 29.0, 12.1 Hz, 6H). LCMS (ESI) calcd for $C_{23}H_{29}N_4O_3^+$ [M+H]$^+$: 409.22, found, 409.2.

**Example 214: preparation of 4-(4-(4-(((2-(2,4-dioxotetrahydropyrimidin-1(2H)-yl)-1-oxoisoindolin-4-yl)oxy)methyl)benzyl)piperazin-1-yl)-3-fluorobenzonitrile (GT-06132)**

Referring to the method of Scheme 3, the target product was prepared as a white solid (32 mg, yield 23%). $^1$H NMR (400 MHz, DMSO-d6) δ 10.65 (s, 1H), 7.68 (dd, J = 13.4, 1.9 Hz, 1H), 7.59 - 7.43 (m, 4H), 7.38 (dd, J = 13.1, 8.0 Hz, 4H), 7.11 (t, J = 8.7 Hz, 1H), 5.26 (s, 2H), 4.73 (s, 1H), 4.45 (s, 1H), 3.77 (t, J = 6.6 Hz, 2H), 3.54 (s, 2H), 3.18 (s, 4H), 2.79 (d, J = 26.4 Hz, 2H), 2.52 (s, 4H). LCMS (ESI): calcd for $C_{31}H_{29}FN_6O_4$; [M+H]$^+$, 569.22; found, 569.3.

**Example 215: preparation of 1-(5-((4-(6-fluoro-1H-indazol-3-yl)piperidin-1-yl)methyl)-1-oxoisoindolin-2-yl)dihydropyrimidine-2,4(1H,3H)-dione (GT-07508)**

Referring to the method of Scheme 1, the target product GT-07508 was prepared as a white solid (6 mg, yield 10%). $^1$H NMR (400 MHz, DMSO) δ 10.94 (s, 1H), 10.70 (s, 1H), 7.98 - 7.92 (m, 2H), 7.90 (d, *J* = 7.9 Hz, 1H), 7.83 (t, *J* = 6.1 Hz, 1H), 7.25 (dd, *J* = 9.7, 2.1 Hz, 1H), 6.97 (td, *J* = 9.2, 2.2 Hz, 1H), 4.83 (d, *J* = 17.0 Hz, 1H), 4.60 (d, *J* = 16.5 Hz, 1H), 4.50 (d, *J* = 4.7 Hz, 2H), 3.80 (t, *J* = 6.8 Hz, 2H), 3.67 - 3.59 (m, 2H), 3.31 - 3.26 (m, 1H), 3.18 - 3.02 (m, 2H), 2.92 - 2.73 (m, 2H), 2.36 - 2.23 (m, 2H), 2.12 (d, *J* = 12.6 Hz, 2H). LCMS (ESI) calcd for $C_{25}H_{26}FN_6O_3^+$ [M+H]$^+$: 477.20, found, 477.3.

**Example 216: preparation of 2-(2,4-dioxotetrahydropyrimidin-1(2H)-yl)-5-((4-(6-fluoro-1H-indazol-3-yl)piperidin-1-yl)methyl)isoindoline-1,3-dione (GT-07530)**

Referring to the method of Scheme 1, the target product GT-07530 was prepared as a white solid (24 mg, yield 40%). $^1$H NMR (400 MHz, DMSO) δ 11.15 (s, 1H), 10.93 (s, 1H), 8.36 (s, 1H), 8.22 (d, *J* = 7.8 Hz, 1H), 8.13 (d, *J* = 7.7 Hz, 1H), 7.98 - 7.90 (m, 1H), 7.25 (dd, *J* = 9.7, 2.1 Hz, 1H), 6.97 (td, *J* = 9.2, 2.0 Hz, 1H), 4.59 (d, *J* = 3.8 Hz, 2H), 3.83 (t, *J* = 6.8 Hz, 2H), 3.38 - 3.24 (m, 2H), 3.19 - 3.10 (m, 2H), 2.86 (t, *J*= 6.7 Hz, 2H), 2.40 - 2.23 (m, 3H), 2.14 (d, *J* = 12.9 Hz, 2H). LCMS (ESI) calcd for $C_{25}H_{24}FN_6O_4^+$ [M+H]$^+$: 491.18, found, 491.3.

**Example 217: preparation of 1-(5-((4-(6-fluoro-1H-indazol-3-yl)-3,6-dihydropyridin-1(2H)-yl)methyl)-1-oxoisoindolin-2-yl)dihydropyrimidine-2,4(1H,3H)-dione (GT-07578)**

Referring to the method of Scheme 1, the target product GT-07578 was prepared as a white solid (20 mg, yield 33%). $^1$H NMR (400 MHz, DMSO) δ 13.29 (s, 1H), 11.21 (s, 1H), 10.70 (s, 1H), 8.01 (dd, *J* = 8.9, 5.1 Hz, 1H), 7.96 (brs, 1H), 7.90 (d, *J* = 7.8 Hz, 1H), 7.87 (brs, 1H), 7.35 (dd, *J* = 9.4, 2.2 Hz, 1H), 7.07 (td, *J* = 9.2, 2.3 Hz, 1H), 6.55 (s, 1H), 4.83 (d, *J* = 16.5 Hz, 1H), 4.68 - 4.53 (m, 3H), 3.94 - 3.88 (m, 2H), 3.81 (t, *J* = 6.9 Hz, 3H), 3.65 (brs, 1H), 3.31 - 3.27 (m, 1H), 3.03 (brs, 2H), 2.90 - 2.75 (m, 3H). LCMS (ESI) calcd for $C_{25}H_{24}FN_6O_3^+$ [M+H]$^+$: 475.19, found, 475.2.

**Example 218: preparation of 2-(2,4-dioxotetrahydropyrimidin-1(2H)-yl)-5-((4-(6-fluoro-1H-indazol-3-yl)-3,6-dihydropyridin-1(2H)-yl)methyl)isoindoline-1,3-dione (GT-07643)**

Referring to the method of Scheme 1, the target product GT-07643 was prepared as a white solid (25 mg, yield 42%). $^1$H NMR (400 MHz, DMSO) δ 13.28 (s, 1H), 11.22 (s, 1H), 10.93 (s, 1H), 8.37 (s, 1H), 8.24 (s, 1H), 8.14 (d, *J* = 7.7 Hz, 1H), 8.00 (dd, *J* = 8.8, 5.3 Hz, 1H), 7.34 (dd, *J* = 9.4, 2.1 Hz, 1H), 7.07 (td, *J* = 9.2, 2.1 Hz, 1H), 6.55 (s, 1H), 4.70 (brs, 2H), 4.02 - 3.85 (m, 3H), 3.84 (t, *J* = 6.7 Hz, 2H), 3.73 - 3.59 (m, 1H), 3.08 - 2.97 (m, 2H), 2.87 (t, *J* = 6.8 Hz, 2H). LCMS (ESI) calcd for $C_{25}H_{22}FN_6O_4^+$ [M+H]$^+$: 489.17, found, 489.1.

**Example 219: preparation of 1-(5-((((1r,3s,5R,7S)-3-hydroxyadamantan-1-yl)amino)methyl)-1-oxoisoindolin-2-yl)dihydropyrimidine-2,4(1H,3H)-dione (GT-07784)**

Referring to the method of Scheme 1, the target product GT-07784 was prepared as a white solid (15 mg, yield 37%). $^1$H NMR (400 MHz, DMSO) δ 10.68 (s, 1H), 9.29 (s, 2H), 7.87 (s, 1H), 7.84 (d, *J* = 7.9 Hz, 1H), 7.77 (d, *J* = 7.9 Hz, 1H), 4.86 (s, 1H), 4.81 (d, *J* = 16.5 Hz, 1H), 4.55 (d, *J* = 16.4 Hz, 1H), 4.24 (brs, 2H), 3.80 (t, *J* = 6.9 Hz, 2H), 2.93 - 2.69 (m, 2H), 2.28 (s, 2H), 1.89 (s, 6H), 1.58 (s, 4H), 1.49 (s, 2H). LCMS (ESI) calcd for $C_{23}H_{29}N_4O_4^+$ [M+H]$^+$: 425.22, found, 425.2.

**Example 220: preparation of 2-(2,4-dioxotetrahydropyrimidin-1(2H)-yl)-5-((((1r,3s,5R,7S)-3-hydroxyadamantan-1-yl)amino)methyl)isoindoline-1,3-dione (GT-07786)**

Referring to the method of Scheme 1, the target product GT-07786 was prepared as a white solid (19 mg, yield 47%). $^1$H NMR (400 MHz, DMSO) δ 10.92 (s, 1H), 9.33 (s, 2H), 8.26 (s, 1H), 8.14 (d, *J* = 7.8 Hz, 1H), 8.07 (d, *J* = 7.7 Hz, 1H), 4.87 (s, 1H), 4.36 (brs, 2H), 3.84 (t, *J* = 6.8 Hz, 2H), 2.86 (t, *J* = 6.8 Hz, 2H), 2.29 (s, 2H), 1.88 (s, 6H), 1.59 (s, 4H), 1.50 (s, 2H). LCMS (ESI) calcd for $C_{23}H_{27}N_4O_5^+$ [M+H]$^+$: 439.20, found, 439.2.

**Example 221: preparation of 5-((4-benzhydryl-3,5-dimethylpiperazin-1-yl)methyl)-2-(2,4-dioxotetrahydropyrimidin-1(2H)-yl)isoindoline-1,3-dione (GT-06430)**

Referring to the method of Scheme 1, the target product (GT-06430) was prepared as a white solid (24 mg, yield 45 %). $^1$H NMR (400 MHz, DMSO-d6) δ 10.92 (s, 1H), 8.26 - 7.87 (m, 5H), 7.51 (s, 1H), 7.43 (s, 2H), 7.37 (d, *J* = 7.5 Hz, 2H), 7.32 - 7.26 (m, 2H), 7.20 (t, *J* = 7.2 Hz, 1H), 3.82 (t, *J* = 6.5 Hz, 3H), 3.23 (s, 3H), 3.12 (s, 2H), 2.85 (s, 2H), 2.71 (d, *J* = 24.6 Hz, 2H), 1.47 (s, 3H), 1.24 - 1.13 (m, 3H). LCMS (ESI) calcd for $C_{32}H_{34}NaN_5O_4^+$ [M+H]$^+$: 575.24, found, 575.2.

**Example 222: preparation of 1-(5-((4-benzhydryl-3,5-dimethylpiperazin-1-yl)methyl)-1-oxoisoindolin-2-yl)dihydropyrimidine-2,4(1H,3H)-dione (GT-06431)**

Referring to the method of Scheme 1, the target product (GT-06431) was prepared as a white solid (26 mg, yield 48 %). $^1$H NMR (400 MHz, DMSO-d6) δ 10.69 (s, 1H), 8.13 (s, 1H), 7.84 (d, *J* = 7.9 Hz, 2H), 7.79 - 7.65 (m, 1H), 7.52 (s, 2H), 7.37 (d, *J* = 7.5 Hz, 3H), 7.35 - 7.23 (m, 3H), 7.22 - 7.14 (m, 1H), 4.77 (s, 1H), 4.57 (d, *J* = 16.0 Hz, 2H), 3.78 (s, 3H), 3.21 (s, 3H), 2.86 (d, *J* = 8.1 Hz, 1H), 2.78 (s, 1H), 2.68 (s, 1H), 1.48 (s, 2H), 1.26 (d, *J* = 6.5 Hz, 2H), 1.19 (s, 2H). LCMS (ESI) calcd

for $C_{32}H_{36}N_5O_3^+$ [M+H]$^+$: 538.28, found, 538.3.

**Example 223: preparation of 5-((4-((4'-chloro-[1,1'-biphenyl]-2-yl)methyl)piperazin-1-yl)methyl)-2-(2,4-dioxotetrahydropyrimidin-1(2H)-yl)isoindoline-1,3-dione (GT-06432)**

Referring to the method of Scheme 1, the target product (GT-06432) was prepared as a white solid (26 mg, yield 51 %). $^1$H NMR (400 MHz, DMSO-d6) δ 10.92 (s, 1H), 8.08 (d, *J* = 36.7 Hz, 3H), 7.85 - 7.63 (m, 1H), 7.51 (t, *J* = 11.4 Hz, 4H), 7.40 (d, *J* = 8.3 Hz, 2H), 7.31 (s, 1H), 4.18 (s, 3H), 3.82 (t, *J* = 6.7 Hz, 3H), 3.09 (s, 4H), 2.86 (t, J= 6.8 Hz, 3H), 2.75 (s, 1H). LCMS (ESI) calcd for $C_{30}H_{29}ClN_5O_4^+$ [M+H]$^+$: 558.19, found, 558.2.

**Example 224: preparation of 1-(5-((4-((4'-chloro-[1,1'-biphenyl]-2-yl)methyl)piperazin-1-yl)methyl)-1-oxoisoindolin-2-yl)dihydropyrimidine-2,4(1H,3H)-dione (GT-06433)**

Referring to the method of Scheme 1, the target product (GT-06433) was prepared as a white solid (26 mg, yield 50 %). $^1$H NMR (400 MHz, DMSO-d6) δ 10.62 (s, 1H), 7.76 (d, *J* = 7.7 Hz, 1H), 7.70 (s, 1H), 7.67 - 7.48 (m, 2H), 7.44 (d, *J* = 8.2 Hz, 2H), 7.39 (s, 2H), 7.33 (d, *J* = 8.3 Hz, 2H), 7.25 - 7.18 (m, 1H), 4.71 (d, *J* = 16.8 Hz, 1H), 4.48 (d, *J* = 16.7 Hz, 1H), 4.04 (d, *J* = 17.6 Hz, 3H), 3.71 (t, *J* = 6.9 Hz, 3H), 2.98 (s, 7H), 2.86 - 2.68 (m, 3H). LCMS (ESI) calcd for $C_{30}H_{31}ClN_5O_3^+$ [M+H]$^+$: 544.20, found, 544.3..

**Example 225: preparation of 1-(1-oxo-5-((4-phenylpiperazin-1-yl)methyl)isoindolin-2-yl)dihydropyrimidine-2,4(1H,3H)-dione (GT-06696)**

Referring to the method of Scheme 1, the target product (GT-06696) was prepared as a white solid (22 mg, yield 55 %). $^1$H NMR (400 MHz, DMSO-d6) δ 10.76 (s, 1H), 8.02 (s, 1H), 7.92 (q, *J* = 7.9 Hz, 2H), 7.32 (t, *J* = 7.9 Hz, 2H), 7.04 (d, *J* = 8.1 Hz, 2H), 6.92 (t, *J* = 7.3 Hz, 1H), 4.88 (d, *J* = 17.0 Hz, 1H), 4.59 (s, 2H), 3.89 - 3.83 (m, 4H), 3.45 (s, 2H), 3.25 (s, 4H), 3.01 - 2.75 (m, 2H). LCMS (ESI) calcd for $C_{30}H_{31}ClN_5O_3^+$ [M+H]$^+$: 420.20, found, 420.0.

**Example 226: preparation of 1-(5-((4-(2-methoxyphenyl)piperazin-1-yl)methyl)-1-oxoisoindolin-2-yl)dihydropyrimidine-2,4(1H,3H)-dione (GT-06697)**

Referring to the method of Scheme 1, the target product (GT-06697) was prepared as a white solid (26 mg, yield 60 %). $^1$H NMR (400 MHz, DMSO-d6) δ 10.62 (s, 1H), 7.88 (s, 1H), 7.80 (s, 1H), 7.79 (s, 1H), 6.93 (s, 1H), 6.91 (s, 1H), 6.84 (d, *J* = 1.3 Hz, 2H), 4.75 (d, *J* = 16.6 Hz, 1H), 4.45 (s, 2H), 3.71 (s, 4H), 3.40 (d, *J* = 12.2 Hz, 2H), 3.17 (d, *J* = 9.0 Hz, 2H), 3.04 (d, *J* = 13.1 Hz, 2H), 2.79 (t, *J* = 7.9 Hz, 1H), 2.70 (dd, *J* = 13.6, 7.9 Hz, 1H). LCMS (ESI) calcd for $C_{24}H_{28}N_5O_4^+$ [M+H]$^+$: 450.20, found, 450.0.

**Example 227: preparation of 1-(5-((4-(3-bromophenyl)piperazin-1-yl)methyl)-1-oxoisoindolin-2-yl)dihydropyrimidine-2,4(1H,3H)-dione (GT-06698)**

Referring to the method of Scheme 1, the target product (GT-06698) was prepared as a white solid (26 mg, yield 54 %). $^1$H NMR (400 MHz, DMSO-d6) δ 10.63 (s, 1H), 7.86 (s, 1H), 7.81 (d, *J* = 7.8 Hz, 1H), 7.76 (d, *J* = 7.9 Hz, 1H), 7.13 (t, *J* = 8.1 Hz, 1H), 7.08 (t, *J* = 2.0 Hz, 1H), 6.92 (s, 2H), 4.73 (s, 1H), 4.51 (d, *J* = 16.6 Hz, 1H), 4.45 (s, 2H), 3.80 (d, *J* = 12.5 Hz, 2H), 3.76 - 3.69 (m, 3H), 3.29 (s, 2H), 3.16 (d, *J* = 12.8 Hz, 2H), 2.80 (dd, *J* = 15.9, 8.0 Hz, 1H), 2.70 (dd, *J* = 13.7, 8.3 Hz, 1H). LCMS (ESI) calcd for $C_{23}H_{25}BrN_5O_3^+$ [M+H]$^+$: 498.11, found, 497.9.

**Example 228: preparation of 1-(5-((4-(3-methoxyphenyl)piperazin-1-yl)methyl)-1-oxoisoindolin-2-yl)dihydropyrimidine-2,4(1H,3H)-dione (GT-06699)**

Referring to the method of Scheme 1, the target product (GT-06699) was prepared as a white solid (31 mg, yield 72 %). $^1$H NMR (400 MHz, DMSO-d6) δ 10.68 (d, *J* = 11.1 Hz, 1H), 7.96 (s, 1H), 7.91 - 7.82 (m, 2H), 7.16 (t, *J* = 8.2 Hz, 1H), 6.56 (dd, *J* = 8.2, 2.1 Hz, 1H), 6.51 (t, *J* = 2.2 Hz, 1H), 6.45 (dd, *J* = 8.1, 2.2 Hz, 1H), 4.82 (d, *J* = 16.7 Hz, 1H), 4.54 (d, *J* = 15.4 Hz, 2H), 3.80 (t, *J* = 6.8 Hz, 3H), 3.72 (s, 2H), 3.37 (d, *J* = 5.4 Hz, 2H), 3.20 (d, *J* = 10.8 Hz, 3H), 2.87 (dd, *J* = 14.7, 6.8 Hz, 1H), 2.78 (t, *J* = 5.6 Hz, 1H). LCMS (ESI) calcd for $C_{24}H_{28}N_5O_4^+$ [M+H]$^+$: 450.21, found, 450.0.

**Example 229: preparation of 1-(1-oxo-5-((4-(4-(trifluoromethyl)phenyl)piperazin-1-yl)methyl)isoindolin-2-yl)dihydropyrimidine-2,4(1H,3H)-dione (GT-06700)**

Referring to the method of Scheme 1, the target product (GT-06700) was prepared as a white solid (24 mg, yield

51 %). $^1$H NMR (400 MHz, DMSO-d6) δ 10.70 (s, 1H), 7.95 - 7.86 (m, 2H), 7.82 (s, 1H), 7.58 (d, *J* = 8.8 Hz, 2H), 7.13 (d, *J* = 8.8 Hz, 2H), 4.82 (d, *J* = 16.5 Hz, 1H), 4.52 (s, 3H), 4.01 (d, *J* = 12.8 Hz, 2H), 3.80 (t, *J* = 6.9 Hz, 2H), 3.41 (s, 1H), 3.31 (s, 2H), 3.19 (s, 2H), 2.87 (dd, *J* = 14.8, 6.9 Hz, 1H), 2.78 (dd, *J* = 14.1, 8.5 Hz, 1H). LCMS (ESI) calcd for $C_{24}H_{25}F_3N_5O_3^+$ $[M+H]^+$: 488.19, found, 488.0.

**Example 230: preparation of 1-(5-((4-(3-fluorophenyl)piperazin-1-yl)methyl)-1-oxoisoindolin-2-yl)dihydropyrimidine-2,4(1H,3H)-dione (GT-06701)**

Referring to the method of Scheme 1, the target product (GT-06701) was prepared as a white solid (24 mg, yield 57 %). $^1$H NMR (400 MHz, DMSO-d6) δ 10.61 (s, 1H), 7.86 (s, 1H), 7.78 (t, *J* = 7.6 Hz, 2H), 7.18 (d, *J* = 7.0 Hz, 1H), 6.72 (t, *J* = 9.7 Hz, 2H), 6.55 (s, 1H), 4.71 (s, 1H), 4.56 - 4.38 (m, 3H), 3.84 - 3.73 (m, 3H), 3.28 (s, 2H), 3.15 (d, *J* = 12.5 Hz, 2H), 3.09 (s, 2H), 2.78 (d, *J* = 7.5 Hz, 1H), 2.70 (s, 1H). LCMS (ESI) calcd for $C_{23}H_{25}FN_5O_3^+$ $[M+H]^+$: 438.19, found, 438.0.

**Example** 231: **preparation** of **1-(5-((4-(4-fluorophenyl)piperazin-1-yl)methyl)-1-oxoisoindolin-2-yl)dihydropyrimidine-2,4(1H,3H)-dione** (GT-06702)

Referring to the method of Scheme 1, the target product (GT-06702) was prepared as a white solid (23 mg, yield 55 %). $^1$H NMR (400 MHz, DMSO-d6) δ 10.76 (s, 1H), 8.02 (s, 1H), 7.92 (s, 2H), 7.16 (t, *J* = 8.8 Hz, 2H), 7.07 (d, *J* = 4.6 Hz, 1H), 7.05 (d, *J* = 4.6 Hz, 1H), 4.88 (d, *J* = 16.6 Hz, 1H), 4.66 (s, 1H), 4.59 (s, 2H), 3.86 (t, *J* = 6.8 Hz, 2H), 3.76 (d, *J* = 8.1 Hz, 2H), 3.44 (s, 2H), 3.25 (d, *J* = 7.9 Hz, 4H), 2.92 (s, 1H), 2.84 (s, 1H). LCMS (ESI) calcd for $C_{23}H_{25}FN_5O_3^+$ $[M+H]^+$: 438.19, found, 438.0.

**Example 232: preparation of 1-(5-((4-(2-chlorophenyl)piperazin-1-yl)methyl)-1-oxoisoindolin-2-yl)dihydropyrimidine-2,4(1H,3H)-dione (GT-06703)**

Referring to the method of Scheme 1, the target product (GT-06703) was prepared as a white solid (21 mg, yield 48 %). $^1$H NMR (400 MHz, DMSO-d6) δ 10.70 (s, 1H), 7.96 (s, 1H), 7.87 (d, *J* = 2.0 Hz, 2H), 7.45 (d, *J* = 7.9 Hz, 1H), 7.34 (t, *J* = 7.0 Hz, 1H), 7.19 (d, *J* = 6.8 Hz, 1H), 7.11 (t, *J* = 6.9 Hz, 1H), 4.80 (s, 1H), 4.58 (d, *J* = 19.3 Hz, 3H), 3.80 (t, *J* = 6.8 Hz, 2H), 3.41 (d, *J* = 8.9 Hz, 4H), 3.24 (s, 4H), 2.86 (s, 1H), 2.78 (s, 1H). LCMS (ESI) calcd for $C_{23}H_{25}ClN_5O_3^+$ $[M+H]^+$: 454.16, found, 454.0.

**Example 233: preparation of 1-(5-((4-(4-chlorophenyl)piperazin-1-yl)methyl)-1-oxoisoindolin-2-yl)dihydropyrimidine-2,4(1H,3H)-dione (GT-06765)**

Referring to the method of Scheme 1, the target product (GT-06765) was prepared as a white solid (24 mg, yield 55 %). $^1$H NMR (400 MHz, DMSO-d6) δ 10.76 (s, 1H), 8.02 (s, 1H), 7.92 (s, 2H), 7.34 (d, *J* = 9.0 Hz, 2H), 7.05 (d, *J* = 9.1 Hz, 2H), 4.86 (s, 1H), 4.62 (d, *J* = 30.7 Hz, 3H), 3.90 - 3.80 (m, 4H), 3.44 (d, *J* = 10.0 Hz, 2H), 3.28 (d, *J* = 12.4 Hz, 4H), 2.92 (s, 1H), 2.84 (s, 1H). LCMS (ESI) calcd for $C_{23}H_{25}ClN_5O_3^+$ $[M+H]^+$: 454.16, found, 454.3.

**Example 234: preparation of 1-(5-((4-(2-bromophenyl)piperazin-1-yl)methyl)-1-oxoisoindolin-2-yl)dihydropyrimidine-2,4(1H,3H)-dione (GT-06766)**

Referring to the method of Scheme 1, the target product (GT-06766) was prepared as a white solid (25 mg, yield 52 %). LCMS (ESI) calcd for $C_{23}H_{25}BrN_5O_3^+$ $[M+H]^+$: 498.11, found, 498.2.

**Example 235: preparation of 1-(5-((4-(4-methoxyphenyl)piperazin-1-yl)methyl)-1-oxoisoindolin-2-yl)dihydropyrimidine-2,4(1H,3H)-dione (GT-06767)**

Referring to the method of Scheme 1, the target product (GT-06767) was prepared as a white solid (14 mg, yield 33 %). $^1$H NMR (400 MHz, DMSO-d6) δ 10.63 (s, 1H), 7.86 (s, 1H), 7.81 (d, *J* = 7.8 Hz, 1H), 7.77 (s, 1H), 6.87 (d, *J* = 9.1 Hz, 2H), 6.79 (d, *J* = 9.1 Hz, 2H), 4.73 (s, 1H), 4.54 (s, 1H), 4.45 (s, 2H), 3.73 (t, *J* = 6.8 Hz, 2H), 3.62 (s, 3H), 3.53 (s, 2H), 3.31 (s, 2H), 3.13 (s, 2H), 3.02 (s, 2H), 2.79 (s, 1H), 2.71 (s, 1H). LCMS (ESI) calcd for $C_{24}H_{28}N_5O_4^+$ $[M+H]^+$: 450.21, found, 450.3.

**Example 236: preparation of 1-(5-((4-(4-bromophenyl)piperazin-1-yl)methyl)-1-oxoisoindolin-2-yl)dihydropyrimidine-2,4(1H,3H)-dione (GT-06768)**

Referring to the method of Scheme 1, the target product (GT-06768) was prepared as a white solid (30 mg, yield 63 %). $^1$H NMR (400 MHz, DMSO-d6) δ 10.62 (s, 1H), 7.88 (s, 1H), 7.80 (t, *J* = 5.8 Hz, 2H), 7.33 (d, *J* = 9.0 Hz, 2H), 6.87 (d, *J*

= 9.1 Hz, 2H), 4.73 (s, 1H), 4.53 (s, 1H), 4.45 (s, 2H), 3.76 - 3.69 (m, 4H), 3.31 (d, *J* = 10.1 Hz, 2H), 3.16 (s, 1H), 3.13 (s, 3H), 2.79 (s, 1H), 2.71 (s, 1H). LCMS (ESI) calcd for $C_{23}H_{25}BrN_5O_3^+$ $[M+H]^+$: 498.11, found, 498.2.

**Example 237: preparation of 1-(1-oxo-5-((4-(o-tolyl)piperazin-1-yl)methyl)isoindolin-2-yl)dihydropyrimidine-2,4(1H,3H)-dione (GT-06769)**

Referring to the method of Scheme 1, the target product (GT-06769) was prepared as a white solid (27 mg, yield 65 %). $^1$H NMR (400 MHz, DMSO-d6) δ 10.63 (s, 1H), 7.92 (s, 1H), 7.81 (s, 2H), 7.11 (t, *J* = 7.8 Hz, 2H), 6.94 (t, *J* = 7.6 Hz, 2H), 4.74 (s, 1H), 4.51 (d, *J* = 21.4 Hz, 3H), 3.73 (t, *J* = 6.8 Hz, 2H), 3.31 (s, 2H), 3.15 (s, 2H), 3.11 (d, *J* = 8.5 Hz, 4H), 2.79 (s, 1H), 2.71 (s, 1H), 2.18 (s, 3H). LCMS (ESI) calcd for $C_{24}H_{28}N_5O_3^+$ $[M+H]^+$: 434.22, found, 434.3.

**Example 238: preparation of 1-(1-oxo-5-((4-(m-tolyl)piperazin-1-yl)methyl)isoindolin-2-yl)dihydropyrimidine-2,4(1H,3H)-dione (GT-06770)**

Referring to the method of Scheme 1, the target product (GT-06770) was prepared as a white solid (19 mg, yield 46 %). $^1$H NMR (400 MHz, DMSO-d6) δ 10.70 (s, 1H), 7.97 (s, 1H), 7.87 (s, 2H), 7.14 (t, *J* = 7.8 Hz, 1H), 6.84 - 6.74 (m, 2H), 6.69 (d, *J* = 7.4 Hz, 1H), 4.80 (s, 1H), 4.58 (d, *J* = 16.9 Hz, 1H), 4.53 (s, 2H), 3.79 (dd, *J* = 14.1, 7.4 Hz, 4H), 3.37 (s, 2H), 3.21 (d, *J* = 11.1 Hz, 4H), 2.86 (s, 1H), 2.78 (s, 1H), 2.26 (s, 3H). LCMS (ESI) calcd for $C_{24}H_{28}N_5O_3^+$ $[M+H]^+$: 434.22, found, 434.3.

**Example 239: preparation of 1-(1-oxo-5-((4-(p-tolyl)piperazin-1-yl)methyl)isoindolin-2-yl)dihydropyrimidine-2,4(1H,3H)-dione (GT-06771)**

Referring to the method of Scheme 1, the target product (GT-06771) was prepared as a white solid (4 mg, yield 9 %). $^1$H NMR (400 MHz, DMSO-d6) δ 10.63 (s, 1H), 7.84 (s, 1H), 7.81 (s, 1H), 7.74 (d, *J* = 8.0 Hz, 1H), 7.00 (d, *J* = 8.4 Hz, 2H), 6.81 (d, *J* = 8.6 Hz, 2H), 4.73 (s, 1H), 4.54 (s, 1H), 4.45 (s, 2H), 3.73 (t, *J* = 7.0 Hz, 2H), 3.66 (d, *J* = 12.2 Hz, 2H), 3.32 (d, *J* = 10.6 Hz, 2H), 3.12 (d, *J* = 9.1 Hz, 2H), 3.05 (s, 1H), 3.03 (s, 1H), 2.80 (dd, *J* = 16.0, 8.3 Hz, 1H), 2.71 (s, 1H), 2.14 (s, 3H). LCMS (ESI) calcd for $C_{24}H_{28}N_5O_3^+$ $[M+H]^+$: 434.22, found, 434.3.

**Example 240: preparation of 1-(5-((4-(2,4-dichlorophenyl)piperazin-1-yl)methyl)-1-oxoisoindolin-2-yl)dihydropyrimidine-2,4(1H,3H)-dione (GT-06772)**

Referring to the method of Scheme 1, the target product (GT-06772) was prepared as a white solid (21 mg, yield 46 %). $^1$H NMR (400 MHz, DMSO-d6) δ 10.62 (s, 1H), 7.87 (s, 1H), 7.80 (t, *J* = 7.6 Hz, 2H), 7.53 (d, *J*= 2.3 Hz, 1H), 7.34 (dd, *J* = 8.6, 2.4 Hz, 1H), 7.14 (d, *J* = 8.7 Hz, 1H), 4.73 (s, 1H), 4.51 (d, *J* = 19.1 Hz, 3H), 3.72 (t, *J* = 6.8 Hz, 2H), 3.34 (s, 3H), 3.27 - 3.23 (m, 1H), 3.15 (s, 4H), 2.79 (s, 1H), 2.71 (s, 1H). LCMS (ESI) calcd for $C_{23}H_{24}Cl_2N_5O_3^+$ $[M+H]^+$: 488.13, found, 488.2.

**Example 241: preparation of 1-(5-((4-(2,5-dichlorophenyl)piperazin-1-yl)methyl)-1-oxoisoindolin-2-yl)dihydropyrimidine-2,4(1H,3H)-dione (GT-06773)**

Referring to the method of Scheme 1, the target product (GT-06773) was prepared as a white solid (23 mg, yield 50 %). $^1$H NMR (400 MHz, DMSO-d6) δ 10.62 (s, 1H), 7.89 (s, 1H), 7.80 (s, 2H), 7.41 (d, *J* = 8.5 Hz, 1H), 7.15 (d, *J* = 2.3 Hz, 1H), 7.10 (dd, *J* = 8.5, 2.3 Hz, 1H), 4.73 (s, 1H), 4.51 (d, *J* = 18.5 Hz, 3H), 3.73 (t, *J* = 6.8 Hz, 2H), 3.37 (s, 4H), 3.18 (s, 4H), 2.79 (s, 1H), 2.71 (s, 1H). LCMS (ESI) calcd for $C_{23}H_{24}Cl_2N_5O_3^+$ $[M+H]^+$: 488.13, found, 488.2.

**Example 242: preparation of 1-(5-((4-(2,6-dichlorophenyl)piperazin-1-yl)methyl)-1-oxoisoindolin-2-yl)dihydropyrimidine-2,4(1H,3H)-dione (GT-06774)**

Referring to the method of Scheme 1, the target product (GT-06774) was prepared as a white solid (23 mg, yield 50 %). $^1$H NMR (400 MHz, DMSO-d6) δ 10.70 (s, 1H), 7.98 (s, 1H), 7.88 (s, 2H), 7.50 (s, 1H), 7.41 (s, 1H), 7.22 (t, *J* = 8.1 Hz, 1H), 4.81 (s, 1H), 4.59 (d, *J* = 16.5 Hz, 3H), 3.83 (d, *J* = 9.7 Hz, 2H), 3.79 (d, *J* = 6.9 Hz, 2H), 3.37 (d, *J* = 11.4 Hz, 2H), 3.19 (d, *J* = 11.2 Hz, 2H), 3.12 (d, *J* = 13.4 Hz, 2H), 2.86 (t, *J* = 7.8 Hz, 1H), 2.77 (dd, *J* = 13.8, 8.2 Hz, 1H). LCMS (ESI) calcd for $C_{23}H_{24}Cl_2N_5O_3^+$ $[M+H]^+$: 488.13, found, 488.2.

**Example 243: preparation of 1-(5-((4-(3,4-dichlorophenyl)piperazin-1-yl)methyl)-1-oxoisoindolin-2-yl)dihydropyrimidine-2,4(1H,3H)-dione (GT-06775)**

Referring to the method of Scheme 1, the target product (GT-06775) was prepared as a white solid (33 mg, yield

70 %). $^1H$ NMR (400 MHz, DMSO-d6) δ 10.75 (s, 1H), 8.01 (s, 1H), 7.92 (d, *J* = 3.9 Hz, 2H), 7.51 (d, *J* = 9.0 Hz, 1H), 7.28 (d, *J* = 2.7 Hz, 1H), 7.04 (d, *J* = 9.0 Hz, 1H), 4.86 (s, 1H), 4.66 (s, 1H), 4.58 (s, 2H), 3.94 (d, *J* = 12.4 Hz, 2H), 3.86 (t, *J* = 6.8 Hz, 2H), 3.38 (d, *J* = 14.7 Hz, 4H), 3.23 (s, 2H), 2.92 (s, 1H), 2.84 (s, 1H). LCMS (ESI) calcd for $C_{23}H_{24}Cl_2N_5O_3^+$ [M+H]$^+$: 488.13, found, 488.1.

**Example 244: preparation of 1-(5-((4-(3,5-dichlorophenyl)piperazin-1-yl)methyl)-1-oxoisoindolin-2-yl)dihydropyrimidine-2,4(1H,3H)-dione (GT-06776)**

Referring to the method of Scheme 1, the target product (GT-06776) was prepared as a white solid (31 mg, yield 66 %). $^1H$ NMR (400 MHz, DMSO-d6) δ 10.75 (s, 1H), 8.00 (s, 1H), 7.93 (d, *J* = 7.8 Hz, 1H), 7.90 (s, 1H), 7.08 (d, *J* = 1.7 Hz, 2H), 7.01 (t, *J* = 1.6 Hz, 1H), 4.88 (d, *J* = 16.4 Hz, 1H), 4.64 (d, *J* = 16.5 Hz, 1H), 4.57 (s, 2H), 4.01 (d, *J* = 13.1 Hz, 2H), 3.86 (t, *J* = 6.8 Hz, 2H), 3.40 (s, 4H), 3.21 (s, 2H), 2.93 (dd, *J* = 16.1, 8.2 Hz, 1H), 2.83 (dd, *J* = 13.9, 8.3 Hz, 1H). LCMS (ESI) calcd for $C_{23}H_{24}Cl_2N_5O_3^+$ [M+H]$^+$: 488.13, found, 488.1.

**Example 245: preparation of 1-(5-((4-(2,3-difluorophenyl)piperazin-1-yl)methyl)-1-oxoisoindolin-2-yl)dihydropyrimidine-2,4(1H,3H)-dione (GT-06777)**

Referring to the method of Scheme 1, the target product (GT-06777) was prepared as a white solid (22 mg, yield 51 %). $^1H$ NMR (400 MHz, DMSO-d6) δ 10.70 (s, 1H), 7.95 (s, 1H), 7.87 (t, *J* = 6.6 Hz, 2H), 7.14 (d, *J* = 8.1 Hz, 1H), 7.06 (d, *J* = 9.8 Hz, 1H), 6.92 (t, *J* = 7.9 Hz, 1H), 4.80 (s, 1H), 4.57 (d, *J* = 29.6 Hz, 3H), 3.80 (t, *J* = 6.8 Hz, 2H), 3.52 (d, *J* = 8.9 Hz, 2H), 3.40 (s, 2H), 3.28 (d, *J* = 6.6 Hz, 4H), 2.86 (s, 1H), 2.78 (s, 1H). LCMS (ESI) calcd for $C_{23}H_{24}F_2N_5O_3^+$ [M+H]$^+$: 456.18, found, 456.2.

**Example 246: preparation of 1-(5-((4-(2,4-difluorophenyl)piperazin-1-yl)methyl)-1-oxoisoindolin-2-yl)dihydropyrimidine-2,4(1H,3H)-dione (GT-06778)**

Referring to the method of Scheme 1, the target product (GT-06778) was prepared as a white solid (23 mg, yield 53 %). $^1H$ NMR (400 MHz, DMSO-d6) δ 10.62 (s, 1H), 7.87 (s, 1H), 7.79 (d, *J* = 6.4 Hz, 2H), 7.18 (s, 1H), 7.06 (d, *J* = 5.9 Hz, 1H), 6.96 (s, 1H), 4.73 (s, 1H), 4.57 - 4.41 (m, 3H), 3.73 (t, *J* = 6.8 Hz, 2H), 3.34 (s, 2H), 3.30 - 3.25 (m, 2H), 3.17 (s, 5H), 2.79 (s, 1H), 2.71 (s, 1H). LCMS (ESI) calcd for $C_{23}H_{24}F_2N_5O_3^+$ [M+H]$^+$: 456.18, found, 456.2.

**Example 247: preparation of 1-(5-((4-(2,5-difluorophenyl)piperazin-1-yl)methyl)-1-oxoisoindolin-2-yl)dihydropyrimidine-2,4(1H,3H)-dione (GT-06779)**

Referring to the method of Scheme 1, the target product (GT-06779) was prepared as a white solid (27 mg, yield 62 %). $^1H$ NMR (400 MHz, DMSO-d6) δ 10.75 (s, 1H), 8.03 (s, 1H), 7.92 (s, 2H), 7.27 (d, *J* = 1.9 Hz, 1H), 7.02 (d, *J* = 3.2 Hz, 1H), 6.90 (d, *J* = 7.9 Hz, 1H), 4.86 (s, 1H), 4.63 (d, *J* = 26.5 Hz, 3H), 3.86 (t, *J* = 6.8 Hz, 2H), 3.59 (d, *J* = 9.8 Hz, 2H), 3.44 (s, 2H), 3.34 (d, *J* = 9.2 Hz, 4H), 2.92 (s, 1H), 2.84 (s, 1H). LCMS (ESI) calcd for $C_{23}H_{24}F_2N_5O_3^+$ [M+H]$^+$: 456.18, found, 456.2.

**Example 248: preparation of 1-(5-((4-(2,6-difluorophenyl)piperazin-1-yl)methyl)-1-oxoisoindolin-2-yl)dihydropyrimidine-2,4(1H,3H)-dione (GT-06780)**

Referring to the method of Scheme 1, the target product (GT-06780) was prepared as a white solid (21 mg, yield 47 %). $^1H$ NMR (400 MHz, DMSO-d6) δ 10.62 (s, 1H), 7.87 (s, 1H), 7.79 (d, *J* = 6.9 Hz, 2H), 7.07 (s, 1H), 7.02 (d, *J* = 9.0 Hz, 2H), 4.73 (s, 1H), 4.50 (d, *J* = 30.8 Hz, 3H), 3.72 (t, *J* = 6.8 Hz, 2H), 3.51 (s, 2H), 3.30 (s, 2H), 3.22 (d, *J* = 12.9 Hz, 2H), 3.13 (d, *J* = 10.1 Hz, 2H), 2.79 (s, 1H), 2.71 (s, 1H). LCMS (ESI) calcd for $C_{23}H_{24}F_2N_5O_3^+$ [M+H]$^+$: 456.18, found, 456.2.

**Example 249: preparation of 1-(5-((4-(3,4-difluorophenyl)piperazin-1-yl)methyl)-1-oxoisoindolin-2-yl)dihydropyrimidine-2,4(1H,3H)-dione (GT-06781)**

Referring to the method of Scheme 1, the target product (GT-06781) was prepared as a white solid (26 mg, yield 59 %). $^1H$ NMR (400 MHz, DMSO-d6) δ 10.76 (s, 1H), 8.01 (s, 1H), 7.98-7.88 (m, 2H), 7.36 (d, *J* = 10.2 Hz, 1H), 7.19-7.10 (m, 1H), 6.83 (d, *J* = 9.0 Hz, 1H), 4.86 (s, 1H), 4.69 - 4.54 (m, 3H), 3.86 (t, *J* = 6.8 Hz, 4H), 3.42 (s, 2H), 3.27 (d, *J* = 11.6 Hz, 4H), 2.92 (s, 1H), 2.84 (s, 1H). LCMS (ESI) calcd for $C_{23}H_{23}F_2N_5NaO_3^+$ [M+H]$^+$: 478.18, found, 478.3.

**Example 250: preparation of 1-(5-((4-(3,5-difluorophenyl)piperazin-1-yl)methyl)-1-oxoisoindolin-2-yl)dihydropyrimidine-2,4(1H,3H)-dione (GT-06782)**

Referring to the method of Scheme 1, the target product (GT-06782) was prepared as a white solid (30 mg, yield 68 %). $^1$H NMR (400 MHz, DMSO-d6) δ 10.70 (s, 1H), 7.95 (s, 1H), 7.86 (d, *J* = 3.7 Hz, 2H), 6.70 (d, *J* = 9.1 Hz, 2H), 6.58 (t, *J* = 2.1 Hz, 1H), 4.80 (s, 1H), 4.56 (d, *J* = 33.1 Hz, 3H), 3.92 (d, *J* = 12.8 Hz, 2H), 3.80 (t, *J* = 6.8 Hz, 2H), 3.34 (d, *J* = 10.5 Hz, 4H), 3.16 (s, 2H), 2.86 (s, 1H), 2.78 (s, 1H). LCMS (ESI) calcd for $C_{23}H_{24}F_2N_5O_3^+$ [M+H]$^+$: 456.18, found, 456.3.

**Example 251: preparation of 1-(5-((4-(2,4-dimethylphenyl)piperazin-1-yl)methyl)-1-oxoisoindolin-2-yl)dihydropyrimidine-2,4(1H,3H)-dione (GT-06882)**

Referring to the method of Scheme 1, the target product (GT-06882) was prepared as a white solid (28 mg, yield 64 %). $^1$H NMR (400 MHz, DMSO-d6) δ 10.75 (s, 1H), 8.03 (s, 1H), 7.93 (s, 2H), 7.07 - 7.01 (m, 2H), 6.97 (d, *J* = 8.0 Hz, 1H), 4.88 (d, *J* = 16.7 Hz, 1H), 4.69 - 4.56 (m, 3H), 3.87 (s, 1H), 3.85 (s, 2H), 3.42 (s, 2H), 3.29 (d, *J* = 8.5 Hz, 2H), 3.18 (d, *J* = 9.6 Hz, 3H), 2.93 (dd, *J* = 16.2, 8.3 Hz, 1H), 2.83 (dd, *J* = 14.0, 8.2 Hz, 1H), 2.27 (s, 6H). LCMS (ESI) calcd for $C_{25}H_{30}N_5O_3^+$ [M+H]$^+$: 448.23, found, 448.3.

**Example 252: preparation of 1-(5-((4-(5-chloro-2-methylphenyl)piperazin-1-yl)methyl)-1-oxoisoindolin-2-yl)dihydropyrimidine-2,4(1H,3H)-dione (GT-06883)**

Referring to the method of Scheme 1, the target product (GT-06883) was prepared as a white solid (28 mg, yield 62 %). $^1$H NMR (400 MHz, DMSO-d6) δ 10.72 (s, 1H), 8.00 (s, 1H), 7.89 (s, 2H), 7.23 (d, *J* = 8.2 Hz, 1H), 7.09 (d, *J* = 8.1 Hz, 1H), 7.04 (s, 1H), 4.85 (d, *J* = 16.6 Hz, 1H), 4.58 (s, 3H), 3.82 (t, *J* = 6.8 Hz, 2H), 3.25 (s, 2H), 3.22 (s, 4H), 2.91 (s, 2H), 2.75 (s, 2H), 2.24 (s, 3H). LCMS (ESI) calcd for $C_{24}H_{27}ClN_5O_3^+$ [M+H]$^+$: 468.18, found, 468.2.

**Example 253: preparation of 1-(1-oxo-5-((4-phenylpiperidin-1-yl)methyl)isoindolin-2-yl)dihydropyrimidine-2,4(1H,3H)-dione (GT-06884)**

Referring to the method of Scheme 1, the target product (GT-06884) was prepared as a white solid (20 mg, yield 48 %). $^1$H NMR (400 MHz, DMSO-d6) δ 10.70 (s, 1H), 7.94 (s, 1H), 7.88 (d, *J* = 7.8 Hz, 1H), 7.84 (d, *J*= 8.1 Hz, 1H), 7.32 (d, *J* = 7.4 Hz, 2H), 7.23 (d, *J* = 6.0 Hz, 3H), 4.81 (s, 1H), 4.61 (s, 1H), 4.47 (d, *J* = 4.9 Hz, 2H), 3.80 (t, *J* = 6.8 Hz, 2H), 3.54 (d, *J* = 46.7 Hz, 1H), 3.31 (d, *J* = 15.9 Hz, 1H), 3.13 - 3.01 (m, 2H), 2.85 (dd, *J* = 14.6, 6.1 Hz, 1H), 2.79 (d, *J* = 5.6 Hz, 2H), 2.11 (s, 2H), 1.94 (d, *J* = 12.9 Hz, 2H). LCMS (ESI) calcd for $C_{24}H_{27}N_4O_3^+$ [M+H]$^+$: 419.21, found, 419.2.

**Example 254: preparation of 1-(5-((4-(3-bromophenyl)piperidin-1-yl)methyl)-1-oxoisoindolin-2-yl)dihydropyrimidine-2,4(1H,3H)-dione (GT-06885)**

Referring to the method of Scheme 1, the target product (GT-06885) was prepared as a white solid (22 mg, yield 46 %). $^1$H NMR (400 MHz, DMSO-d6) δ 10.70 (s, 1H), 7.95 (s, 1H), 7.88 (d, *J* = 7.9 Hz, 1H), 7.85 (s, 1H), 7.43 (d, *J* = 12.8 Hz, 2H), 7.31 (t, *J* = 7.7 Hz, 1H), 7.25 (d, *J* = 7.7 Hz, 1H), 4.80 (s, 1H), 4.61 (s, 1H), 4.47 (d, *J* = *4.6* Hz, 2H), 3.80 (t, *J* = 6.8 Hz, 2H), 3.45 (s, 1H), 3.05 (d, *J* = 10.7 Hz, 2H), 2.85 (d, *J* = 8.4 Hz, 2H), 2.81 - 2.75 (m, 1H), 2.12 (d, *J* = 12.5 Hz, 2H), 1.95 (d, *J* = 12.7 Hz, 2H). LCMS (ESI) calcd for $C_{24}H_{26}BrN_4O_3^+$ [M+H]$^+$: 497.12, found, 497.2.

**Example 255: preparation of 1-(5-((4-(4-bromophenyl)piperidin-1-yl)methyl)-1-oxoisoindolin-2-yl)dihydropyrimidine-2,4(1H,3H)-dione (GT-06886)**

Referring to the method of Scheme 1, the target product (GT-06886) was prepared as a white solid (18 mg, yield 38 %). $^1$H NMR (400 MHz, DMSO-d6) δ 10.62 (s, 1H), 7.87 (s, 1H), 7.79 (t, *J* = 8.6 Hz, 2H), 7.45 (d, *J* = 8.3 Hz, 2H), 7.13 (d, *J* = 8.3 Hz, 2H), 4.73 (s, 1H), 4.53 (s, 1H), 4.39 (s, 2H), 3.73 (t, *J* = 6.8 Hz, 2H), 3.36 (d, *J* = 10.1 Hz, 2H), 2.98 (d, *J* = 7.1 Hz, 2H), 2.79 (s, 1H), 2.72 (d, *J* = 5.6 Hz, 2H), 2.02 (s, 2H), 1.86 (d, *J* = 12.3 Hz, 2H). LCMS (ESI) calcd for $C_{24}H_{26}BrN_4O_3^+$ [M+H]$^+$: 497.12, found, 497.2.

**Example 256: preparation of 1-(5-((4-(2-chlorophenyl)piperidin-1-yl)methyl)-1-oxoisoindolin-2-yl)dihydropyrimidine-2,4(1H,3H)-dione (GT-06887)**

Referring to the method of Scheme 1, the target product (GT-06887) was prepared as a white solid (12 mg, yield 28 %). $^1$H NMR (400 MHz, DMSO-d6) δ 10.70 (s, 1H), 7.93 (s, 1H), 7.88 (d, *J* = 7.8 Hz, 1H), 7.83 (d, *J*= 8.0 Hz, 1H), 7.45 (d, *J*= 7.8 Hz, 1H), 7.37 (d, *J* = 7.3 Hz, 1H), 7.33 (s, 1H), 7.29 (d, *J* = 7.6 Hz, 1H), 4.80 (s, 1H), 4.61 (s, 1H), 4.46 (d, *J* = 4.5 Hz,

2H), 3.80 (t, *J* = 6.8 Hz, 2H), 3.46 (s, 2H), 3.16 (d, *J* = 10.9 Hz, 3H), 2.87 (dd, *J* = 15.8, 7.9 Hz, 1H), 2.82 - 2.72 (m, 1H), 2.15 (d, *J* = *12.4* Hz, 2H), 1.93 (d, *J* = 14.0 Hz, 2H). LCMS (ESI) calcd for $C_{24}H_{26}ClN_4O_3^+$ [M+H]$^+$: 453.17, found, 453.3.

**Example 257: preparation of 1-(5-((4-(4-chlorophenyl)piperidin-1-yl)methyl)-1-oxoisoindolin-2-yl)dihydropyrimidine-2,4(1H,3H)-dione (GT-06888)**

Referring to the method of Scheme 1, the target product (GT-06888) was prepared as a white solid (20 mg, yield 46 %). $^1$H NMR (400 MHz, DMSO-d6) δ 10.69 (s, 1H), 7.94 - 7.85 (m, 2H), 7.82 (s, 1H), 7.39 (d, *J* = 8.4 Hz, 2H), 7.25 (d, *J* = 8.3 Hz, 2H), 4.80 (s, 1H), 4.61 (s, 1H), 4.46 (s, 2H), 3.79 (t, *J* = 6.8 Hz, 2H), 3.45 (d, *J* = 11.3 Hz, 2H), 3.11 - 2.97 (m, 2H), 2.86 (s, 2H), 2.78 (s, 1H), 2.04 (s, 2H), 1.94 (d, *J* = 11.8 Hz, 2H). LCMS (ESI) calcd for $C_{24}H_{26}ClN_4O_3^+$ [M+H]$^+$: 453.17, found, 453.3.

**Example 258: preparation of 1-(5-((4-(2-fluorophenyl)piperidin-1-yl)methyl)-1-oxoisoindolin-2-yl)dihydropyrimidine-2,4(1H,3H)-dione (GT-06889)**

Referring to the method of Scheme 1, the target product (GT-06889) was prepared as a white solid (20 mg, yield 48 %). $^1$H NMR (400 MHz, DMSO-d6) δ 10.69 (s, 1H), 7.94 (s, 1H), 7.87 (d, *J* = 7.8 Hz, 1H), 7.84 (s, 1H), 7.31 - 7.26 (m, 2H), 7.23 - 7.19 (m, 1H), 7.17 (s, 1H), 4.80 (s, 1H), 4.61 (s, 1H), 4.46 (d, *J* = 4.9 Hz, 2H), 3.80 (t, *J* = 6.8 Hz, 2H), 3.44 (s, 2H), 3.12 (d, *J* = 12.5 Hz, 3H), 2.86 (s, 1H), 2.78 (s, 1H), 2.18 (d, *J* = 12.4 Hz, 2H), 1.91 (d, *J* = 13.5 Hz, 2H). LCMS (ESI) calcd for $C_{24}H_{26}FN_4O_3^+$ [M+H]$^+$: 437.20, found, 437.3.

**Example 259: preparation of 1-(5-((4-(4-fluorophenyl)piperidin-1-yl)methyl)-1-oxoisoindolin-2-yl)dihydropyrimidine-2,4(1H,3H)-dione (GT-06890)**

Referring to the method of Scheme 1, the target product (GT-06890) was prepared as a white solid (20 mg, yield 48 %). $^1$H NMR (400 MHz, DMSO-d6) δ 10.70 (s, 1H), 7.95 (s, 1H), 7.86 (d, *J* = 5.2 Hz, 2H), 7.30 - 7.23 (m, 2H), 7.17 (d, *J* = 8.9 Hz, 2H), 4.80 (s, 1H), 4.60 (s, 1H), 4.46 (d, *J* = *4.7* Hz, 2H), 3.80 (t, *J* = 6.8 Hz, 2H), 3.43 (d, *J* = 11.1 Hz, 2H), 3.06 (s, 2H), 2.86 (s, 1H), 2.78 (s, 2H), 2.13 (d, *J* = 12.1 Hz, 2H), 1.93 (d, *J* = 11.7 Hz, 2H). LCMS (ESI) calcd for $C_{24}H_{26}FN_4O_3^+$ [M+H]$^+$: 437.20, found, 437.3.

**Example 260: preparation of 1-(1-oxo-5-((4-(2-(trifluoromethyl)phenyl)piperidin-1-yl)methyl)isoindolin-2-yl)dihydropyrimidine-2,4(1H,3H)-dione (GT-06891)**

Referring to the method of Scheme 1, the target product (GT-06891) was prepared as a white [solid (24 mg, yield 52 %). $^1$H NMR (400 MHz, DMSO-d6) δ 10.70 (s, 1H), 7.94 (s, 1H), 7.86 (s, 1H), 7.84 (s, 1H), 7.70 (d, *J* = 8.1 Hz, 2H), 7.57 (d, *J* = 7.7 Hz, 1H), 7.47 (d, *J* = 7.6 Hz, 1H), 4.80 (s, 1H), 4.61 (s, 1H), 4.46 (d, *J* = 4.6 Hz, 2H), 3.80 (t, *J* = 6.8 Hz, 2H), 3.44 (d, *J* = 11.1 Hz, 2H), 3.23 - 3.08 (m, 3H), 2.87 (dd, *J* = 16.0, 8.2 Hz, 1H), 2.78 (s, 1H), 2.35 (d, *J* = 12.6 Hz, 2H), 1.85 (d, *J* = 13.5 Hz, 2H). LCMS (ESI) calcd for $C_{25}H_{26}F_3N_4O_3^+$ [M+H]$^+$: 487.20, found, 487.2.

**Example 261: preparation of 1-(1-oxo-5-((4-(3-(trifluoromethyl)phenyl)piperidin-1-yl)methyl)isoindolin-2-yl)dihydropyrimidine-2,4(1H,3H)-dione (GT-06892)**

Referring to the method of Scheme 1, the target product (GT-06892) was prepared as a white solid (24 mg, yield 52 %). $^1$H NMR (400 MHz, DMSO-d6) δ 10.70 (s, 1H), 7.98 (s, 1H), 7.87 (s, 2H), 7.61 (s, 2H), 7.57 (d, *J* = 1.6 Hz, 2H), 4.81 (s, 1H), 4.61 (s, 1H), 4.49 (d, *J* = 4.7 Hz, 2H), 3.80 (t, *J* = 6.8 Hz, 2H), 3.45 (d, *J* = 11.4 Hz, 2H), 3.07 (d, *J* = 10.5 Hz, 2H), 2.96 (s, 1H), 2.87 (t, *J* = 7.7 Hz, 1H), 2.78 (s, 1H), 2.21 (d, *J* = 12.1 Hz, 2H), 1.99 (d, *J* = 13.3 Hz, 2H). LCMS (ESI) calcd for $C_{25}H_{26}F_3N_4O_3^+$ [M+H]$^+$: 487.20, found, 487.3.

**Example 262: preparation of 1-(5-((4-(2,3-dichlorophenyl)piperidin-1-yl)methyl)-1-oxoisoindolin-2-yl)dihydropyrimidine-2,4(1H,3H)-dione (GT-06893)**

Referring to the method of Scheme 1, the target product (GT-06893) was prepared as a white solid (19 mg, yield 49 %). $^1$H NMR (400 MHz, DMSO-d6) δ 10.70 (s, 1H), 7.94 (s, 1H), 7.86 (s, 1H), 7.84 (s, 1H), 7.56 (d, *J* = 8.0 Hz, 1H), 7.41 (t, *J* = 7.9 Hz, 1H), 7.30 (d, *J* = 6.5 Hz, 1H), 4.80 (s, 1H), 4.61 (s, 1H), 4.46 (s, 2H), 3.80 (t, *J* = 6.8 Hz, 2H), 3.46 (d, *J* = 11.3 Hz, 2H), 3.29 (s, 1H), 3.16 (d, *J* = 11.1 Hz, 2H), 2.93 - 2.82 (m, 1H), 2.78 (s, 1H), 2.17 (d, *J* = 12.8 Hz, 2H), 1.95 (d, *J* = 13.7 Hz, 2H). LCMS (ESI) calcd for $C_{24}H_{25}Cl_2N_4O_3^+$ [M+H]$^+$: 487.13, found, 487.2.

**Example 263: preparation of 1-(5-((4-(2-chloro-3-fluorophenyl)piperidin-1-yl)methyl)-1-oxoisoindolin-2-yl)dihydropyrimidine-2,4(1H,3H)-dione (GT-06894)**

Referring to the method of Scheme 1, the target product (GT-06894) was prepared as a white solid (19 mg, yield 51 %). $^1$H NMR (400 MHz, DMSO-d6) δ 10.70 (s, 1H), 7.94 (s, 1H), 7.87 (d, *J* = 7.8 Hz, 1H), 7.83 (d, *J*= 8.0 Hz, 1H), 7.43 (d, *J* = 5.8 Hz, 1H), 7.32 (t, *J* = 8.2 Hz, 1H), 7.17 (d, *J* = 7.8 Hz, 1H), 4.80 (s, 1H), 4.61 (s, 1H), 4.46 (s, 2H), 3.80 (t, *J* = 6.8 Hz, 2H), 3.45 (d, *J* = 12.0 Hz, 2H), 3.26 (s, 1H), 3.16 (d, *J* = 10.9 Hz, 2H), 2.86 (s, 1H), 2.78 (s, 1H), 2.17 (d, *J* = 12.8 Hz, 2H), 1.94 (d, *J* = 13.6 Hz, 2H). LCMS (ESI) calcd for $C_{24}H_{25}ClFN_4O_3^+$ [M+H]$^+$: 471.16, found, 471.2.

**Example 264: preparation of 5-((4-(2-chloro-3-fluorophenyl)piperidin-1-yl)methyl)-2-(2,4-dioxotetrahydropyrimidin-1(2H)-yl)isoindoline-1,3-dione (GT-06895)**

Referring to the method of Scheme 1, the target product (GT-06895) was prepared as a white solid (22 mg, yield 50 %). $^1$H NMR (400 MHz, DMSO-d6) δ 10.93 (s, 1H), 8.35 (s, 1H), 8.20 (s, 1H), 8.10 (d, *J* = 7.5 Hz, 1H), 7.43 (d, *J = 6.2* Hz, 1H), 7.33 (d, *J* = 8.7 Hz, 1H), 7.18 (d, *J* = 7.6 Hz, 1H), 4.55 (s, 2H), 3.83 (t, *J* = 6.7 Hz, 2H), 3.45 (s, 2H), 3.25 (s, 1H), 3.19 (d, *J* = 23.5 Hz, 2H), 2.86 (t, *J* = 6.7 Hz, 2H), 2.14 (d, *J* = 8.3 Hz, 2H), 1.94 (d, *J* = 12.2 Hz, 2H). LCMS (ESI) calcd for $C_{24}H_{23}ClFN_4O_4^+$ [M+H]$^+$: 485.14, found, 485.2.

**Example 265: preparation of 5-((4-(2-chloro-3-fluorophenyl)-3,6-dihydropyridin-1(2H)-yl)methyl)-2-(2,4-dioxotetrahydropyrimidin-1(2H)-yl)isoindoline-1,3-dione (GT-06896)**

Referring to the method of Scheme 1, the target product (GT-06896) was prepared as a white solid (23 mg, yield 52 %). $^1$H NMR (400 MHz, DMSO-d6) δ 10.93 (s, 1H), 8.37 (s, 1H), 8.24 (s, 1H), 8.12 (d, *J* = 7.6 Hz, 1H), 7.40 (dd, *J* = 11.5, 4.4 Hz, 2H), 7.15 (d, *J* = 6.5 Hz, 1H), 5.78 (s, 1H), 4.67 (s, 2H), 3.83 (t, *J* = 6.7 Hz, 3H), 3.78 (s, 1H), 3.63 (d, *J* = 16.3 Hz, 1H), 3.30 - 3.19 (m, 1H), 2.90 (s, 1H), 2.86 (t, *J* = 6.7 Hz, 2H), 2.59 (s, 1H). LCMS (ESI) calcd for $C_{24}H_{23}ClFN_4O_4^+$ [M+H]$^+$: 483.12, found, 483.2.

**Example 266: preparation of 5-((4-(2,3-difluorophenyl)piperidin-1-yl)methyl)-2-(2,4-dioxotetrahydropyrimidin-1(2H)-yl)isoindoline-1,3-dione (GT-06897)**

Referring to the method of Scheme 1, the target product (GT-06897) was prepared as a white solid (19 mg, yield 44 %). $^1$H NMR (400 MHz, DMSO) δ 10.93 (s, 1H), 8.32 (s, 1H), 8.17 (s, 1H), 8.11 (d, *J* = 7.5 Hz, 1H), 7.32 (d, *J* = 9.0 Hz, 1H), 7.22 (d, *J* = 5.2 Hz, 1H), 7.11 (d, *J* = 6.8 Hz, 1H), 4.55 (s, 2H), 3.83 (s, 2H), 3.45 (s, 2H), 3.22-3.14 (m, 3H), 2.86 (s,2H), 2.12 (s, 2H), 1.97 (s, 2H). LCMS (ESI) calcd for $C_{24}H_{23}F_2N_4O_4^+$ [M+H]$^+$: 469.17, found, 469.2.

**Example 267: preparation of 2-(2,4-dioxotetrahydropyrimidin-1(2H)-yl)-5-((4-(6-fluoropyridin-2-yl)piperazin-1-yl)methyl)isoindoline-1,3-dione (GT-06964)**

Referring to the method of Scheme 1, the target product (GT-06964) was prepared as a white solid (31 mg, yield 55 %). $^1$H NMR (400 MHz, DMSO-d6) δ 10.93 (s, 1H), 8.33 (s, 1H), 8.19 (d, *J* = 7.7 Hz, 1H), 8.10 (d, *J* = 7.7 Hz, 1H), 7.76 (d, *J* = 8.3 Hz, 1H), 6.79 (dd, *J* = 8.2, 2.2 Hz, 1H), 6.40 (dd, *J = 7.7,* 2.4 Hz, 1H), 4.58 (s, 2H), 4.31 (s, 2H), 3.83 (t, *J* = 6.8 Hz, 2H), 3.41 (s, 2H), 3.36 - 3.25 (m, 2H), 3.12 (s, 2H), 2.86 (t, *J* = 6.8 Hz, 2H). LCMS (ESI) calcd for $C_{22}H_{22}FN_6O_4^+$ [M+H]$^+$: 453.17, found, 453.2.

**Example 268: preparation of 2-(2,4-dioxotetrahydropyrimidin-1(2H)-yl)-5-((4-(5-fluoropyridin-2-yl)piperazin-1-yl)methyl)isoindoline-1,3-dione (GT-06965)**

Referring to the method of Scheme 1, the target product (GT-06965) was prepared as a white solid (28 mg, yield 51 %). $^1$H NMR (400 MHz, DMSO-d6) δ 10.94 (s, 1H), 8.39 (s, 1H), 8.25 (dd, *J* = 7.8, 1.1 Hz, 1H), 8.16 (d, *J* = 3.0 Hz, 1H), 8.10 (d, *J* = 7.7 Hz, 1H), 7.70 - 7.60 (m, 1H), 7.03 (dd, *J* = 9.4, 3.4 Hz, 1H), 5.88 (s, 1H), 5.61 - 5.36 (m, 1H), 4.62 (s, 2H), 4.30 (d, *J* = 12.2 Hz, 2H), 3.83 (t, *J* = 6.7 Hz, 2H), 3.39 (d, *J = 6.9* Hz, 5H), 3.16 (s, 2H), 2.87 (t, *J* = 6.7 Hz, 2H). LCMS (ESI) calcd for $C_{22}H_{22}FN_6O_4^+$ [M+H]$^+$: 453.17, found, 453.2.

**Example 269: preparation of 2-(2,4-dioxotetrahydropyrimidin-1(2H)-yl)-5-((4-(3-(trifluoromethyl)pyridin-2-yl)piperazin-1-yl)methyl)isoindoline-1,3-dione (GT-06966)**

Referring to the method of Scheme 1, the target product (GT-06966) was prepared as a white solid (23 mg, yield 37 %). $^1$H NMR (400 MHz, DMSO-d6) δ 10.93 (s, 1H), 8.66 - 8.55 (m, 1H), 8.41 (s, 1H), 8.27 (dd, *J* = 7.8, 1.1 Hz, 1H), 8.16 -

8.08 (m, 2H), 7.33 (dd, $J$ = 7.6, 5.0 Hz, 1H), 4.64 (s, 2H), 3.83 (t, $J$ = 6.8 Hz, 2H), 3.51 (s, 4H), 3.43 (d, $J$ = 11.4 Hz, 2H), 3.23 (s, 2H), 2.86 (t, $J$ = 6.8 Hz, 2H). LCMS (ESI) calcd for $C_{23}H_{22}F_3N_6O_4^+$ [M+H]$^+$: 503.16, found, 503.2.

**Example 270: preparation of 2-(2,4-dioxotetrahydropyrimidin-1(2H)-yl)-5-((4-(6-(trifluoromethyl)pyridin-2-yl)piperazin-1-yl)methyl)isoindoline-1,3-dione (GT-06967)**

Referring to the method of Scheme 1, the target product (GT-06967) was prepared as a white solid (27 mg, yield 44 %). $^1$H NMR (400 MHz, DMSO-d6) δ 10.93 (s, 1H), 8.33 (s, 1H), 8.18 (s, 1H), 8.10 (d, $J$ = 7.6 Hz, 1H), 7.84 (d, $J$ = 8.0 Hz, 1H), 7.23 (d, $J$ = 8.7 Hz, 1H), 7.16 (d, $J$ = 7.3 Hz, 1H), 4.59 (s, 2H), 4.43 (s, 2H), 3.83 (t, $J$ = 6.8 Hz, 2H), 3.44 (s, 4H), 3.13 (s, 2H), 2.86 (t, $J$ = 6.8 Hz, 2H). LCMS (ESI) calcd for $C_{23}H_{22}F_3N_6O_4^+$ [M+H]$^+$: 503.16, found, 503.2.

**Example 271: preparation of 2-(2,4-dioxotetrahydropyrimidin-1(2H)-yl)-5-((4-(5-(trifluoromethyl)pyridin-2-yl)piperazin-1-yl)methyl)isoindoline-1,3-dione (GT-06968)**

Referring to the method of Scheme 1, the target product (GT-06968) was prepared as a white solid (40 mg, yield 65 %). $^1$H NMR (400 MHz, DMSO-d6) δ 10.93 (s, 1H), 8.48 (s, 1H), 8.36 (s, 1H), 8.22 (dd, $J$ = 7.7, 1.2 Hz, 1H), 8.10 (d, $J$ = 7.7 Hz, 1H), 7.92 (dd, $J$ = 9.0, 2.4 Hz, 1H), 7.09 (d, $J$ = 9.1 Hz, 1H), 4.60 (s, 2H), 4.52 (s, 2H), 3.83 (t, $J$ = 6.8 Hz, 2H), 3.57 - 3.45 (m, 2H), 3.40 (s, 2H), 3.14 (s, 2H), 2.87 (t, $J$ = 6.8 Hz, 2H). LCMS (ESI) calcd for $C_{23}H_{22}F_3N_6O_4^+$ [M+H]$^+$: 503.16, found, 503.2.

**Example 272: preparation of 5-((4-(3,5-dichloropyridin-2-yl)piperazin-1-yl)methyl)-2-(2,4-dioxotetrahydropyrimidin-1(2H)-yl)isoindoline-1,3-dione (GT-06969)**

Referring to the method of Scheme 1, the target product (GT-06969) was prepared as a white solid (26 mg, yield 43 %). $^1$H NMR (400 MHz, DMSO-d6) δ 10.93 (s, 1H), 8.38 (s, 1H), 8.33 (d, $J$ = 2.3 Hz, 1H), 8.27 - 8.22 (m, 1H), 8.12 (dd, $J$ = 8.6, 5.0 Hz, 2H), 4.62 (s, 2H), 3.83 (t, $J$ = 6.7 Hz, 4H), 3.42 (d, $J$ = 10.7 Hz, 4H), 3.25 (s, 2H), 2.86 (t, $J$ = 6.8 Hz, 2H). LCMS (ESI) calcd for $C_{22}H_{21}Cl_2N_6O_4^+$ [M+H]$^+$: 503.10, found, 503.2.

**Example 273: preparation of 2-(2,4-dioxotetrahydropyrimidin-1(2H)-yl)-5-((4-(3-fluoro-4-iodopyridin-2-yl)piperazin-1-yl)methyl)isoindoline-1,3-dione (GT-06970)**

Referring to the method of Scheme 1, the target product (GT-06970) was prepared as a white solid (29 mg, yield 55 %). $^1$H NMR (400 MHz, DMSO-d6) δ 10.93 (s, 1H), 8.35 (s, 1H), 8.26 - 8.18 (m, 1H), 8.10 (d, $J$ = 7.7 Hz, 1H), 7.73 (d, $J$ = 5.1 Hz, 1H), 7.39 (dd, $J$ = 5.0, 3.7 Hz, 1H), 4.59 (s, 2H), 4.04 (d, $J$ = 13.4 Hz, 3H), 3.89 (s, 1H), 3.40 (d, $J$ = 13.9 Hz, 4H), 3.21 (s, 2H), 2.86 (t, $J$ = 6.8 Hz, 2H). LCMS (ESI) calcd for $C_{22}H_{21}FIN_6O_4^+$ [M+H]$^+$: 579.06, found, 579.1.

**Example 274: preparation of 5-((4-(3-chloro-5-(trifluoromethyl)pyridin-2-yl)piperazin-1-yl)methyl)-2-(2,4-dioxotetrahydropyrimidin-1(2H)-yl)isoindoline-1,3-dione (GT-07026)**

Referring to the method of Scheme 1, the target product (GT-07026) was prepared as a white solid (38 mg, yield 58 %). $^1$H NMR (400 MHz, DMSO-d6) δ 10.93 (s, 1H), 8.63 (s, 1H), 8.35 (s, 1H), 8.30 (s, 1H), 8.21 (s, 1H), 8.11 (d, $J$ = 7.6 Hz, 1H), 4.62 (s, 2H), 4.09 (s, 2H), 3.83 (t, $J$ = 6.8 Hz, 2H), 3.48 (d, $J$ = 13.6 Hz, 3H), 3.41 (s, 1H), 3.24 (s, 2H), 2.86 (t, $J$ = 6.8 Hz, 2H). LCMS (ESI) calcd for $C_{23}H_{21}ClF_3N_6O_4^+$ [M+H]$^+$: 537.13, found, 537.2.

**Example 275: preparation of 2-(2,4-dioxotetrahydropyrimidin-1(2H)-yl)-5-((4-(pyridin-3-yl)piperazin-1-yl)methyl)isoindoline-1,3-dione (GT-06971)**

Referring to the method of Scheme 1, the target product (GT-06971) was prepared as a white solid (19 mg, yield 34 %). $^1$H NMR (400 MHz, DMSO-d6) δ 10.93 (s, 1H), 8.52 (s, 1H), 8.35 (s, 1H), 8.25 (d, $J$ = 5.0 Hz, 1H), 8.22 (d, $J$ = 8.0 Hz, 1H), 8.11 (d, $J$ = 7.6 Hz, 1H), 8.03 (d, $J$ = 7.7 Hz, 1H), 7.83 - 7.77 (m, 1H), 4.60 (s, 2H), 3.92 (s, 2H), 3.83 (t, $J$ = 6.7 Hz, 3H), 3.43 (s, 4H), 3.24 - 3.18 (m, 1H), 2.86 (t, $J$ = 6.6 Hz, 2H). LCMS (ESI) calcd for $C_{22}H_{23}N_6O_4^+$ [M+H]$^+$: 435.18, found, 435.2.

**Example 276: preparation of 5-((4-(6-chloropyridin-3-yl)piperazin-1-yl)methyl)-2-(2,4-dioxotetrahydropyrimidin-1(2H)-yl)isoindoline-1,3-dione (GT-07027)**

Referring to the method of Scheme 1, the target product (GT-07027) was prepared as a white solid (35 mg, yield 61 %). $^1$H NMR (400 MHz, DMSO-d6) δ 10.93 (s, 1H), 8.32 (s, 1H), 8.17 (s, 1H), 8.13 (dd, $J$ = 7.3, 5.5 Hz, 2H), 7.52 - 7.46 (m, 1H), 7.36 (d, $J$ = 8.8 Hz, 1H), 4.60 (s, 2H), 3.91 (d, $J$ = 9.7 Hz, 2H), 3.83 (t, $J$ = 6.8 Hz, 2H), 3.42 - 3.35 (m, 2H), 3.23 (s, 4H),

2.86 (t, $J$ = 6.7 Hz, 2H). LCMS (ESI) calcd for $C_{22}H_{22}ClN_6O_4^+$ [M+H]$^+$: 469.14, found, 469.2.

**Example 277: preparation of 5-((4-(4,5-dichloropyridin-3-yl)piperazin-1-yl)methyl)-2-(2,4-dioxotetrahydropyrimidin-1(2H)-yl)isoindoline-1,3-dione (GT-06972)**

Referring to the method of Scheme 1, the target product (GT-06972) was prepared as a white solid (36 mg, yield 58 %). $^1$H NMR (400 MHz, DMSO-d6) δ 10.94 (s, 1H), 8.52 (s, 1H), 8.41 (s, 2H), 8.28 (d, $J$ = 7.8 Hz, 1H), 8.11 (d, $J$ = 7.7 Hz, 1H), 4.67 (s, 3H), 4.62 (s, 3H), 3.83 (t, $J$ = 6.7 Hz, 2H), 3.55 (s, 2H), 3.29 (s, 2H), 2.87 (t, $J$ = 6.8 Hz, 2H). LCMS (ESI) calcd for $C_{22}H_{21}Cl_2N_6O_4^+$ [M+H]$^+$: 503.10, found, 503.1.

**Example 278: preparation of 5-((4-(2,4-dichloropyridin-3-yl)piperazin-1-yl)methyl)-2-(2,4-dioxotetrahydropyrimidin-1(2H)-yl)isoindoline-1,3-dione (GT-06973)**

Referring to the method of Scheme 1, the target product (GT-06973) was prepared as a white solid (28 mg, yield 45 %). $^1$H NMR (400 MHz, DMSO-d6) δ 10.93 (s, 1H), 8.39 (s, 1H), 8.25 (d, $J$ = 7.8 Hz, 1H), 8.20 (d, $J$ = 6.3 Hz, 1H), 8.11 (d, $J$ = 7.7 Hz, 1H), 7.62 (d, $J$ = 30.5 Hz, 1H), 4.65 (s, 2H), 3.83 (t, $J$ = 6.8 Hz, 3H), 3.79 (s, 1H), 3.38 (s, 2H), 3.23 (s, 4H), 2.86 (t, $J$ = 6.8 Hz, 2H). LCMS (ESI) calcd for $C_{22}H_{21}Cl_2N_6O_4^+$ [M+H]$^+$: 503.10, found, 503.1.

**Example 279: preparation of 1-(5-((4-(2,4-dichloropyridin-3-yl)piperazin-1-yl)methyl)-1-oxoisoindolin-2-yl)dihydropyrimidine-2,4(1H,3H)-dione (GT-06974)**

Referring to the method of Scheme 1, the target product (GT-06974) was prepared as a white solid (29 mg, yield 47 %). $^1$H NMR (400 MHz, DMSO-d6) δ 10.70 (s, 1H), 8.18 (s, 1H), 8.00 (s, 1H), 7.88 (s, 2H), 7.62 (d, $J$ = 29.3 Hz, 1H), 4.81 (s, 1H), 4.58 (d, $J$ = 17.3 Hz, 3H), 3.80 (t, $J$ = 6.8 Hz, 4H), 3.38 (d, $J$ = 10.3 Hz, 2H), 3.20 (d, $J$ = 9.7 Hz, 4H), 2.86 (s, 1H), 2.78 (s, 1H). LCMS (ESI) calcd for $C_{22}H_{23}Cl_2N_6O_3^+$ [M+H]$^+$: 489.12, found, 489.1.

Example 280: preparation of 1-(5-((4-(2-chloro-3-fluoropyridin-4-yl)piperazin-1-yl)methyl)-1-oxoisoindolin-2-yl)dihydropyrimidine-2,4(1H,3H)-dione (GT-07069)

Referring to the method of Scheme 1, the target product (GT-07069) was prepared as a white solid (28 mg, yield 46 %). $^1$H NMR (400 MHz, DMSO-d6) δ 10.70 (s, 1H), 8.18 (s, 1H), 8.00 (s, 1H), 7.88 (s, 2H), 7.62 (d, $J$ = 29.3 Hz, 1H), 4.81 (s, 1H), 4.58 (d, $J$ = 17.3 Hz, 3H), 3.80 (t, $J$ = 6.8 Hz, 4H), 3.38 (d, $J$ = 10.3 Hz, 2H), 3.20 (d, $J$ = 9.7 Hz, 4H), 2.86 (s, 1H), 2.78 (s, 1H). LCMS (ESI) calcd for $C_{22}H_{23}Cl_2N_6O_3^+$ [M+H]$^+$: 489.12, found, 489.1.

Example 281: preparation of 1-(1-oxo-5-((4-(pyrimidin-2-yl)piperazin-1-yl)methyl)isoindolin-2-yl)dihydropyrimidine-2,4(1H,3H)-dione (GT-07159)

Referring to the method of Scheme 1, the target product (GT-07159) was prepared as a white solid (28 mg, yield 69 %). $^1$H NMR (400 MHz, DMSO) δ 10.93 (s, 1H), 8.86 (s, 1H), 8.55 (d, $J$ = 6.3 Hz, 1H), 8.40 (s, 1H), 8.26 (d, $J$ = 7.8 Hz, 1H), 8.11 (d, $J$ = 7.7 Hz, 1H), 7.46 (d, $J$ = 6.5 Hz, 1H), 4.68 (s, 2H), 4.60 - 4.48 (m, 1H), 4.09 (s, 2H), 3.83 (t, $J$ = 6.7 Hz, 2H), 3.78 - 3.70 (m, 1H), 3.67 (d, $J$ = 6.7 Hz, 1H), 3.46 (d, $J$ = 6.9 Hz, 1H), 3.40 (s, 2H), 3.35 - 3.28 (m, 1H), 2.87 (dd, $J$ = 12.7, 6.0 Hz, 2H). LCMS (ESI) calcd for $C_{21}H_{24}N_7O_3^+$ [M+H]$^+$: 422.19, found, 422.2.

**Example 282: preparation of 1-(1-oxo-5-((4-(pyrazin-2-yl)piperazin-1-yl)methyl)isoindolin-2-yl)dihydropyrimidine-2,4(1H,3H)-dione (GT-07160)**

Referring to the method of Scheme 1, the target product (GT-07160) was prepared as a white solid (30 mg, yield 74 %). $^1$H NMR (400 MHz, DMSO) δ 10.95 (s, 1H), 8.35 (s, 1H), 8.22 (d, $J$ = 7.8 Hz, 1H), 8.11 (d, $J$ = 7.7 Hz, 1H), 8.01 (d, $J$ = 5.6 Hz, 1H), 7.97-7.91 (m, 1H), 7.14 (d, $J$ = 6.0 Hz, 1H), 4.62 (s, 2H), 4.51 (d, $J$ = 8.1 Hz, 2H), 3.86 (s, 1H), 3.86 - 3.79 (m, 3H), 3.51 (s, 2H), 3.39 (s, 2H), 3.26 (s, 2H), 2.86 (t, $J$ = 6.7 Hz, 2H). LCMS (ESI) calcd for $C_{21}H_{24}N_7O_3^+$ [M+H]$^+$: 422.19, found, 422.2.

**Example 283: preparation of 1-(5-((4-(2-bromo-3-fluoropyridin-4-yl)piperazin-1-yl)methyl)-1-oxoisoindolin-2-yl) dihydropyrimidine-2,4(1H,3H)-dione (GT-07071)**

Referring to the method of Scheme 1, the target product (GT-07071) was prepared as a white solid (29 mg, yield 44 %). $^1$H NMR (400 MHz, DMSO) δ 10.69 (s, 1H), 8.49 (d, $J$ = 3.4 Hz, 1H), 8.43 (s, 1H), 7.98 (s, 1H), 7.89-7.82 (m, 1H), 4.80 (s, 1H), 4.55 (s, 3H), 3.79 (t, $J$ = 6.8 Hz, 2H), 3.71 (s, 2H), 3.59 (d, $J$ = 13.0 Hz, 2H), 3.41 (d, $J$ = 11.3 Hz, 2H), 3.21 (s, 2H),

2.90-2.84 (m, 1H), 2.80-2.75 (m, 1H). LCMS (ESI) calcd for $C_{22}H_{23}BrFN_6O_3^+$ [M+H]$^+$: 517.10, found, 517.2.

**Example 284: preparation of 1-(5-((4-(3-bromo-2-chloropyridin-4-yl)piperazin-1-yl)methyl)-1-oxoisoindolin-2-yl)dihydropyrimidine-2,4(1H,3H)-dione (GT-07072)**

Referring to the method of Scheme 1, the target product (GT-07072) was prepared as a white solid (39 mg, yield 58 %). $^1$H NMR (400 MHz, DMSO) δ 10.66 (s, 1H), 8.51 (d, *J* = 3.4 Hz, 1H), 8.47 (s, 1H), 7.97 (s, 1H), 7.88-7.84 (m, 1H), 4.82 (s, 1H), 4.54 (s, 3H), 3.78 (d, *J* = 7.8 Hz, 2H), 3.73 (s, 2H), 3.57 (d, *J* = 12.8 Hz, 2H), 3.44 (s, 2H), 3.25-3.19 (m, 2H), 2.92-2.85 (m, 1H), 2.83-2.77 (m, 1H). LCMS (ESI) calcd for $C_{22}H_{23}BrClN_6O_3^+$ [M+H]$^+$: 533.07, found, 533.1.

**Example 285: preparation of 2-(2,4-dioxotetrahydropyrimidin-1(2H)-yl)-5-((4-(pyridin-4-yl)piperazin-1-yl) methyl)isoindoline-1,3-dione (GT-07073)**

Referring to the method of Scheme 1, the target product (GT-07073) was prepared as a white solid (39 mg, yield 73 %). $^1$H NMR (400 MHz, DMSO) δ 10.95 (s, 1H), 8.90 (d, *J* = 4.7 Hz, 2H), 8.37 (s, 1H), 8.28-8.22 (m, 1H), 8.11 (d, *J* = 7.7 Hz, 1H), 8.00 (d, *J* = 5.5 Hz, 1H), 7.15 - 7.10 (m, 1H), 4.63 (s, 2H), 3.83 (t, *J* = 6.8 Hz, 4H), 3.56 (s, 2H), 3.37 (s, 2H), 3.25 (s, 2H), 2.86 (d, *J* = 6.8 Hz, 2H). LCMS (ESI) calcd for $C_{22}H_{23}N_6O_4^+$ [M+H]$^+$: 435.18, found, 435.3.

**Example 286: preparation of 2-(2,4-dioxotetrahydropyrimidin-1(2H)-yl)-5-((4-(2-methylpyridin-4-yl)piperazin-1-yl)methyl)isoindoline-1,3-dione (GT-07161)**

Referring to the method of Scheme 1, the target product (GT-07161) was prepared as a white solid (9 mg, yield 16 %). $^1$H NMR (400 MHz, DMSO) δ 10.93 (s, 1H), 8.77 (s, 1H), 8.52 (d, *J* = 6.5 Hz, 1H), 8.39 (s, 1H), 8.26 (d, *J* = 7.8 Hz, 1H), 8.11 (d, *J* = 7.7 Hz, 1H), 7.48 (d, *J* = 6.6 Hz, 1H), 4.67 (s, 2H), 3.83 *(t, J* = 6.7 Hz, 2H), 3.70 - 3.62 (m, 1H), 3.40 (s, 5H), 3.17 (d, *J* = 13.1 Hz, 2H), 2.89 (s, 1H), 2.86 (t, *J* = 6.7 Hz, 2H), 2.73 (s, 1H). LCMS (ESI) calcd for $C_{23}H_{25}N_6O_4^+$ [M+H]$^+$: 449.19, found, 449.2.

**Example 287: preparation of 5-((4-(3,4-difluoropyridin-2-yl)piperazin-1-yl)methyl)-2-(2,4-dioxotetrahydropyrimidin-1(2H)-yl)isoindoline-1,3-dione (GT-07162)**

Referring to the method of Scheme 1, the target product (GT-07162) was prepared as a white solid (24 mg, yield 42 %). $^1$H NMR (400 MHz, DMSO) δ 10.88 (s, 1H), 8.53 (d, *J* = 3.9 Hz, 1H), 8.47 (d, *J* = 7.2 Hz, 1H), 7.98 (s, 1H), 7.92-7.85 (m, 2H), 4.80 (s, 1H), 4.57 (s, 2H), 3.79 (t, *J* = 6.8 Hz, 2H), 3.74 (s, 2H), 3.55 (d, *J* = 13.0 Hz, 2H), 3.41 (d, *J* = 11.3 Hz, 2H), 3.26 (s, 1H), 2.88-2.84 (m, 1H), 2.79-2.74 (m, 1H). LCMS (ESI) calcd for $C_{22}H_{21}F_2N_6O_4^+$ [M+H]$^+$: 471.16, found, 471.2.

**Example 288: preparation of 5-((4-(3-bromopyridin-4-yl)piperazin-1-yl)methyl)-2-(2,4-dioxotetrahydropyrimidin-1(2H)-yl)isoindoline-1,3-dione (GT-07163)**

Referring to the method of Scheme 1, the target product (GT-07163) was prepared as a white solid (29 mg, yield 46 %). $^1$H NMR (400 MHz, DMSO) δ 10.93 (s, 1H), 8.86 (s, 1H), 8.55 (d, *J* = 6.3 Hz, 1H), 8.40 (s, 1H), 8.26 (d, *J* = 7.8 Hz, 1H), 8.11 (d, *J* = 7.7 Hz, 1H), 7.46 (d, *J* = 6.5 Hz, 1H), 4.68 (s, 2H), 4.09 (s, 2H), 3.83 (t, *J* = 6.7 Hz, 2H), 3.78 - 3.70 (m, 1H), 3.66 (s, 1H), 3.46 (d, *J* = 6.9 Hz, 1H), 3.40 (s, 2H), 3.35 - 3.28 (m, 1H), 2.87 (dd, *J* = 12.7, 6.0 Hz, 2H). LCMS (ESI) calcd for $C_{22}H_{22}BrN_6O_4^+$ [M+H]$^+$: 513.09, found, 513.1.

**Example 289: preparation of 2-(2,4-dioxotetrahydropyrimidin-1(2H)-yl)-5-((4-(3-fluoropyridin-4-yl)piperazin-1-yl)methyl)isoindoline-1,3-dione (GT-07164)**

Referring to the method of Scheme 1, the target product (GT-07164) was prepared as a white solid (30 mg, yield 54 %). $^1$H NMR (400 MHz, DMSO) δ 10.93 (s, 1H), 8.78 (d, *J* = 8.4 Hz, 1H), 8.38 (d, *J* = 6.8 Hz, 2H), 8.24 (d, *J* = 7.8 Hz, 1H), 8.11 (d, *J* = 7.7 Hz, 1H), 7.49 (d, *J* = 7.4 Hz, 1H), 4.66 (s, 2H), 4.11 (s, 2H), 3.83 (t, *J* = 6.7 Hz, 2H), 3.78 - 3.64 (m, 1H), 3.40 (s, 5H), 2.86 (t, *J* = 6.7 Hz, 2H). LCMS (ESI) calcd for $C_{22}H_{22}FN_6O_4^+$ [M+H]$^+$: 453.17, found, 453.2.

**Example 290: preparation of 5-((4-(3-chloropyridin-4-yl)piperazin-1-yl)methyl)-2-(2,4-dioxotetrahydropyrimidin-1(2H)-yl)isoindoline-1,3-dione (GT-07165)**

Referring to the method of Scheme 1, the target product (GT-07165) was prepared as a white solid (29 mg, yield 50 %). $^1$H NMR (400 MHz, DMSO) δ 10.93 (s, 1H), 8.77 (s, 1H), 8.52 (d, *J* = 6.5 Hz, 1H), 8.39 (s, 1H), 8.26 (d, *J* = 7.8 Hz, 1H), 8.11 (d, *J* = 7.7 Hz, 1H), 7.48 (d, *J* = 6.6 Hz, 1H), 4.67 (s, 2H), 3.83 (t, *J* = 6.7 Hz, 2H), 3.70 - 3.62 (m, 1H), 3.40 (s, 5H), 2.89 (s,

1H), 2.86 (t, *J* = 6.7 Hz, 2H), 2.73 (s, 1H). LCMS (ESI) calcd for $C_{22}H_{22}ClN_6O_4^+$ [M+H]$^+$: 469.14, found, 469.2.

**Example 291: preparation of 5-((4-(2-chloro-3-fluoropyridin-4-yl)piperazin-1-yl)methyl)-2-(2,4-dioxotetrahydropyrimidin-1(2H)-yl)isoindoline-1,3-dione (GT-07166)**

Referring to the method of Scheme 1, the target product (GT-07166) was prepared as a white solid (20 mg, yield 34%). $^1$H NMR (400 MHz, DMSO) δ 10.93 (s, 1H), 8.35 (s, 1H), 8.21 (s, 1H), 8.11 (d, *J* = 7.7 Hz, 1H), 8.01 (d, *J* = 5.6 Hz, 1H), 7.13 (s, 1H), 4.62 (s, 2H), 3.86 (s, 1H), 3.83 (t, *J* = 6.8 Hz, 3H), 3.51 (s, 2H), 3.39 (s, 2H), 3.26 (s, 2H), 2.86 (t, *J* = 6.7 Hz, 2H). LCMS (ESI) calcd for $C_{22}H_{22}ClN_6O_4^+$ [M+H]$^+$: 487.13, found, 487.2.

**Example 292: preparation of 5-((4-(2-bromo-3-fluoropyridin-4-yl)piperazin-1-yl)methyl)-2-(2,4-dioxotetrahydropyrimidin-1(2H)-yl)isoindoline-1,3-dione (GT-07167)**

Referring to the method of Scheme 1, the target product (GT-07167) was prepared as a white solid (31 mg, yield 48%). $^1$H NMR (400 MHz, DMSO) δ 10.93 (s, 1H), 8.37 (s, 1H), 8.24 (dd, *J* = 7.8, 1.0 Hz, 1H), 8.11 (d, *J* = 7.7 Hz, 1H), 8.00 (d, *J*= 5.5 Hz, 1H), 7.15 - 7.10 (m, 1H), 4.63 (s, 2H), 3.83 (t, *J* = 6.8 Hz, 4H), 3.53 (s, 2H), 3.39 (s, 2H), 3.27 (s, 2H), 2.87 (t, *J* = 6.8 Hz, 2H). LCMS (ESI) calcd for $C_{22}H_{21}BrFN_6O_4^+$ [M+H]$^+$: 531.08, found, 531.1.

**Example 293: preparation of 5-((4-(3-bromo-2-chloropyridin-4-yl)piperazin-1-yl)methyl)-2-(2,4-dioxotetrahydropyrimidin-1(2H)-yl)isoindoline-1,3-dione (GT-07168)**

Referring to the method of Scheme 1, the target product (GT-07168) was prepared as a white solid (22 mg, yield 33%). $^1$H NMR (400 MHz, DMSO) δ 10.93 (s, 1H), 8.39 (s, 1H), 8.25 (d, *J* = 5.4 Hz, 2H), 8.11 (d, J= 7.7 Hz, 1H), 7.17 (d, *J* = 5.5 Hz, 1H), 4.66 (s, 2H), 3.83 (t, *J* = 6.8 Hz, 2H), 3.67 (s, 2H), 3.44 (s, 2H), 3.38 (s, 2H), 3.27 (s, 2H), 2.86 (t, *J* = 6.8 Hz, 2H). LCMS (ESI) calcd for $C_{22}H_{21}BrClN_6O_4^+$ [M+H]$^+$: 547.05, found, 547.1.

**Example 294: preparation of 1-(5-((4-(2,3-dichloropyridin-4-yl)piperazin-1-yl)methyl)-1-oxoisoindolin-2-yl)dihydropyrimidine-2,4(1H,3H)-dione (GT-07169)**

Referring to the method of Scheme 1, the target product (GT-07169) was prepared as a white solid (19 mg, yield 40%). $^1$H NMR (400 MHz, DMSO) δ 10.70 (s, 1H), 8.22 (d, *J* = 5.5 Hz, 1H), 7.97 (s, 1H), 7.87 (s, 2H), 7.20 (d, *J* = 5.5 Hz, 1H), 4.80 (s, 1H), 4.58 (d, *J* = 18.7 Hz, 3H), 3.80 (t, *J* = 6.8 Hz, 2H), 3.71 (d, *J* = 12.6 Hz, 2H), 3.39 (d, *J* = 13.0 Hz, 4H), 3.26 (s, 2H), 2.86 (s, 1H), 2.78 (s, 1H). LCMS (ESI) calcd for $C_{22}H_{23}Cl_2N_6O_3^+$ [M+H]$^+$: 489.12, found, 489.1.

**Example 295: preparation of 1-(5-((4-(3-bromo-5-chloropyridin-4-yl)piperazin-1-yl)methyl)-1-oxoisoindolin-2-yl)dihydropyrimidine-2,4(1H,3H)-dione (GT-07170)**

Referring to the method of Scheme 1, the target product (GT-07170) was prepared as a white solid (20 mg, yield 39%). $^1$H NMR (400 MHz, DMSO) δ 10.69 (s, 1H), 8.64 (s, 1H), 8.54 (s, 1H), 8.01 (s, 1H), 7.90 (d, *J* = 8.1 Hz, 1H), 7.86 (d, *J* = 7.8 Hz, 1H), 4.81 (s, 1H), 4.59 (d, *J* = 13.4 Hz, 3H), 3.88 (s, 2H), 3.80 (t, *J* = 6.8 Hz, 2H), 3.41 (d, *J* = 12.2 Hz, 4H), 3.21 (d, *J* = 9.4 Hz, 2H), 2.86 (s, 1H), 2.78 (s, 1H). LCMS (ESI) calcd for $C_{22}H_{23}BrClN_6O_3^+$ [M+H]$^+$: 533.07, found, 533.1.

**Example 296: preparation of 1-(5-((4-(3-chloro-3-fluoropyridin-4-yl)piperazin-1-yl)methyl)-1-oxoisoindolin-2-yl) dihydropyrimidine-2,4(1H,3H)-dione (GT-07171)**

Referring to the method of Scheme 1, the target product (GT-07171) was prepared as a white solid (19 mg, yield 41%). $^1$H NMR (400 MHz, DMSO) δ 10.69 (s, 1H), 8.49 (d, *J* = 3.4 Hz, 1H), 8.43 (s, 1H), 7.98 (s, 1H), 7.87 (s, 2H), 4.80 (s, 1H), 4.55 (s, 3H), 3.79 (t, *J* = 6.8 Hz, 2H), 3.71 (s, 2H), 3.59 (d, *J* = 13.0 Hz, 2H), 3.41 (d, *J* = 11.3 Hz, 2H), 3.21 (s, 2H), 2.86 (s, 1H), 2.78 (s, 1H). LCMS (ESI) calcd for $C_{22}H_{23}ClFN_6O_3^+$ [M+H]$^+$: 473.15, found, 473.2.

**Example 297: preparation of 2-(2,4-dioxotetrahydropyrimidin-1(2H)-yl)-5-((4-(pyrimidin-2-yl)piperazin-1-yl) methyl)isoindoline-1,3-dione (GT-07285)**

Referring to the method of Scheme 1, the target product (GT-07285) was prepared as a white solid (29 mg, yield 54%). $^1$H NMR (400 MHz, DMSO) δ 10.93 (s, 1H), 8.47 - 8.44 (m, 3H), 8.36 (s, 1H), 8.22 (d, *J*= 7.8 Hz, 1H), 8.10 (d, *J* = 7.7 Hz, 1H), 6.78 (t, *J* = 4.8 Hz, 2H), 4.59 (s, 2H), 4.48 (s, 1H), 3.98 (s, 1H), 3.83 (t, *J* = 6.8 Hz, 2H), 3.66 (s, 1H), 3.41 (d, *J* = 14.1 Hz, 3H), 3.17 - 3.01 (m, 4H), 2.86 (t, *J* = 6.8 Hz, 2H). LCMS (ESI) calcd for $C_{21}H_{22}N_7O_4^+$ [M+H]$^+$: 436.17, found, 436.2.

**Example 298: preparation of 2-(2,4-dioxotetrahydropyrimidin-1(2H)-yl)-5-((4-(pyrazin-2-yl)piperazin-1-yl) methyl)isoindoline-1,3-dione (GT-07286)**

Referring to the method of Scheme 1, the target product (GT-07286) was prepared as a white solid (25 mg, yield 47%). $^1$H NMR (400 MHz, DMSO) δ 10.93 (s, 1H), 8.44 (s, 1H), 8.36 (s, 1H), 8.21 (s, 1H), 8.19 (d, *J* = 1.5 Hz, 1H), 8.10 (d, *J* = 7.7 Hz, 1H), 7.96 (d, *J* = 2.6 Hz, 1H), 4.60 (s, 2H), 4.46 (d, *J* = 14.4 Hz, 3H), 3.83 (t, *J* = 6.7 Hz, 2H), 3.42 (d, *J* = 12.6 Hz, 3H), 3.16 (s, 2H), 2.86 (t, *J* = 6.8 Hz, 2H). LCMS (ESI) calcd for $C_{21}H_{22}N_7O_4^+$ [M+H]$^+$: 436.17, found, 436.3.

**Example 299: preparation of 5-((4-(2,3-dichloropyridin-4-yl)piperazin-1-yl)methyl)-2-(2,4-dioxotetrahydropyrimidin-1(2H)-yl)isoindoline-1,3-dione (GT-07287)**

Referring to the method of Scheme 1, the target product (GT-07287) was prepared as a white solid (36 mg, yield 59%). $^1$H NMR (400 MHz, DMSO) δ 10.93 (s, 1H), 8.39 (s, 1H), 8.26 (d, *J* = 8.0 Hz, 1H), 8.22 (d, *J* = 5.5 Hz, 1H), 8.11 (d, *J* = 7.7 Hz, 1H), 7.21 (d, *J* = 5.5 Hz, 1H), 4.65 (s, 2H), 3.83 (t, *J* = 6.8 Hz, 2H), 3.71 (s, 2H), 3.43 (s, 4H), 3.28 (s, 2H), 2.87 (t, *J* = 6.8 Hz, 2H). LCMS (ESI) calcd for $C_{22}H_{21}Cl_2N_6O_4^+$ [M+H]$^+$: 503.10, found, 503.1.

**Example 300: preparation of 5-((4-(2,3-difluoropyridin-4-yl)piperazin-1-yl)methyl)-2-(2,4-dioxotetrahydropyrimidin-1(2H)-yl)isoindoline-1,3-dione (GT-07288)**

Referring to the method of Scheme 1, the target product (GT-07288) was prepared as a white solid (31 mg, yield 54%). $^1$H NMR (400 MHz, DMSO) δ 10.93 (s, 1H), 8.36 (s, 1H), 8.23 (d, *J* = 7.8 Hz, 1H), 8.11 (d, *J*= 7.7 Hz, 1H), 7.95 -7.90 (m, 1H), 7.80 (d, *J* = 5.7 Hz, 1H), 7.04 (t, *J* = 6.0 Hz, 1H), 4.63 (s, 2H), 3.90 (s, 2H), 3.83 (t, *J* = 6.8 Hz, 2H), 3.55 (s, 2H), 3.40 (s, 2H), 3.27 (s, 2H), 2.87 (t, *J* = 6.7 Hz, 2H). LCMS (ESI) calcd for $C_{22}H_{21}F_2N_6O_4^+$ [M+H]$^+$: 471.16, found, 471.2.

**Example 301: preparation of 5-((4-(3-bromo-5-chloropyridin-4-yl)piperazin-1-yl)methyl)-2-(2,4-dioxotetrahydropyrimidin-1(2H)-yl)isoindoline-1,3-dione (GT-07289)**

Referring to the method of Scheme 1, the target product (GT-07289) was prepared as a white solid (36 mg, yield 53%). $^1$H NMR (400 MHz, DMSO) δ 10.94 (s, 1H), 8.65 (s, 1H), 8.55 (s, 1H), 8.44 (s, 1H), 8.31 (d, *J* = 7.8 Hz, 1H), 8.11 (d, *J* = 7.7 Hz, 1H), 4.68 (s, 2H), 3.89 (s, 2H), 3.84 (t, *J* = 6.8 Hz, 2H), 3.48 - 3.38 (m, 4H), 3.26 (s, 2H), 2.87 (t, *J* = 6.7 Hz, 2H). LCMS (ESI) calcd for $C_{22}H_{21}BrClN_6O_4^+$ [M+H]$^+$: 547.05, found, 547.1.

**Example 302: preparation of 5-((4-(3-chloro-5-fluoropyridin-4-yl)piperazin-1-yl)methyl)-2-(2,4-dioxotetrahydropyrimidin-1(2H)-yl)isoindoline-1,3-dione (GT-07290)**

Referring to the method of Scheme 1, the target product (GT-07290) was prepared as a white solid (27 mg, yield 45%). $^1$H NMR (400 MHz, DMSO) δ 10.93 (s, 1H), 8.52 (d, *J* = 3.6 Hz, 1H), 8.46 (s, 1H), 8.41 (s, 1H), 8.28 (d, *J* = 7.7 Hz, 1H), 8.11 (d, *J* = 7.7 Hz, 1H), 4.65 (s, 2H), 3.83 (t, *J* = 6.7 Hz, 2H), 3.73 (s, 2H), 3.64 (s, 2H), 3.42 (d, *J* = 10.8 Hz, 2H), 3.23 (s, 2H), 2.86 (t, *J* = 6.7 Hz, 2H). LCMS (ESI) calcd for $C_{22}H_{21}ClFN_6O_4^+$ [M+H]$^+$: 487.13, found, 487.2.

**Example 303: preparation of 5-((4-(3-bromo-4-chloropyridin-2-yl)piperazin-1-yl)methyl)-2-(2,4-dioxotetrahydropyrimidin-1(2H)-yl)isoindoline-1,3-dione (GT-07291)**

Referring to the method of Scheme 1, the target product (GT-07291) was prepared as a white solid (27 mg, yield 41%). $^1$H NMR (400 MHz, DMSO) δ 10.93 (s, 1H), 8.39 (s, 1H), 8.25 (dd, *J* = 5.8, 3.1 Hz, 2H), 8.11 (d, *J* = 7.7 Hz, 1H), 7.37 (d, *J* = 5.2 Hz, 1H), 4.64 (s, 2H), 3.83 (t, *J* = 6.7 Hz, 4H), 3.41 (t, *J* = 13.0 Hz, 4H), 3.26 (s, 2H), 2.86 (t, *J* = 6.8 Hz, 2H). LCMS (ESI) calcd for $C_{22}H_{21}BrClN_6O_4^+$ [M+H]$^+$: 574.05, found, 574.1.

**Example 304: preparation of 1-(5-((4-(2,3-dichlorophenyl)-3,6-dihydropyridin-1(2H)-yl)methyl)-1-oxoisoindolin-2-yl)dihydropyrimidine-2,4(1H,3H)-dione (GT-08054)**

Referring to the method of Scheme 1, the target product (GT-08054) was prepared as a white solid (34 mg, yield 55%). $^1$H NMR (400 MHz, DMSO-d6) δ 10.70 (s, 1H), 7.95 (s, 1H), 7.87 (d, *J* = 5.7 Hz, 2H), 7.63 (dd, *J*= 8.1, 1.5 Hz, 1H), 7.40 (t, *J* = 7.9 Hz, 1H), 7.25 (dd, *J* = 7.7, 1.5 Hz, 1H), 5.74 (s, 1H), 4.83 (d, *J* = *16.6* Hz, 1H), 4.60 (d, *J* = 13.5 Hz, 3H), 3.80 (t, *J* = 6.8 Hz, 4H), 3.59 (s, 1H), 3.29 (s, 1H), 2.89 - 2.76 (m, 2H), 2.57 (s, 1H). LCMS (ESI) calcd for $C_{24}H_{23}Cl_2N_4O_3^+$ [M+H]$^+$: 485.11, found, 485.1.

**Example 305: preparation of 1-(5-((4-(2-chloro-3-fluorophenyl)-3,6-dihydropyridin-1(2H)-yl)methyl)-1-oxoisoindolin-2-yl)dihydropyrimidine-2,4(1H,3H)-dione (GT-08055)**

Referring to the method of Scheme 1, the target product (GT-08055) was prepared as a white solid (35 mg, yield 59%). $^1$H NMR (400 MHz, DMSO-d6) δ 10.70 (s, 1H), 7.98 (s, 1H), 7.88 (s, 2H), 7.40 (dd, *J* = 11.4, 4.7 Hz, 2H), 7.14 (dd, *J* = 6.1, 2.3 Hz, 1H), 5.78 (s, 1H), 4.81 (s, 1H), 4.66 - 4.53 (m, 3H), 3.80 (dd, *J*= 12.7, 6.0 Hz, 4H), 3.59 (s, 1H), 3.29 (s, 1H), 2.86 (s, 2H), 2.78 (s, 1H), 2.56 (d, *J* = 17.9 Hz, 1H). LCMS (ESI) calcd for $C_{24}H_{23}ClFN_4O_3^+$ [M+H]$^+$: 469.14, found, 469.1.

**Example 306: preparation of 1-(1-oxo-5-((4-(thieno[2,3-d]pyrimidin-4-yl)piperazin-1-yl)methyl)isoindolin-2-yl) dihydropyrimidine-2,4(1H,3H)-dione (GT-08057)**

Referring to the method of Scheme 1, the target product (GT-08057) was prepared as a white solid (12 mg, yield 26%). $^1$H NMR (400 MHz, DMSO) δ 10.70 (s, 1H), 8.56 (s, 1H), 7.93 (s, 1H), 7.88 (d, *J* = 7.8 Hz, 1H), 7.83 (d, *J* = 7.9 Hz, 1H), 7.77 (d, *J* = 6.1 Hz, 1H), 7.70 (d, *J* = 6.2 Hz, 1H), 4.80 (s, 1H), 4.69 (d, *J*= 13.6 Hz, 2H), 4.58 (d, *J* = 16.6 Hz, 1H), 4.52 (s, 2H), 3.82 - 3.78 (m, 2H), 3.74 (s, 2H), 3.44 (s, 2H), 3.24 (s, 2H), 2.86 (s, 1H), 2.78 (s, 1H). LCMS (ESI) calcd for $C_{23}H_{24}N_7O_3S^+$ [M+H]$^+$: 478.17, found, 478.1.

**Example 307: preparation of 1-(5-((4-(5-methylthieno[2,3-d]pyrimidin-4-yl)piperazin-1-yl)methyl)-1-oxoisoindolin-2-yl)dihydropyrimidine-2,4(1H,3H)-dione (GT-08058)**

Referring to the method of Scheme 1, the target product (GT-08058) was prepared as a white solid (32 mg, yield 51%). $^1$H NMR (400 MHz, DMSO) δ 10.70 (s, 1H), 8.63 (s, 1H), 7.97 (s, 1H), 7.87 (s, 2H), 7.48 (d, *J* = 1.0 Hz, 1H), 4.82 (d, *J* = 16.7 Hz, 1H), 4.54 (s, 3H), 3.93 (d, *J* = 13.7 Hz, 2H), 3.80 (t, *J* = 6.8 Hz, 2H), 3.60 (s, 2H), 3.38 (s, 2H), 3.32 (s, 2H), 2.86 (t, *J* = 7.8 Hz, 1H), 2.77 (d, *J* = 5.8 Hz, 1H), 2.54 (s, 3H). LCMS (ESI) calcd for $C_{24}H_{26}N_7O_3S^+$ [M+H]$^+$: 492.18, found, 492.1.

**Example 308: preparation of 1-(5-((4-(6-methylthieno[2,3-d]pyrimidin-4-yl)piperazin-1-yl)methyl)-1-oxoisoindolin-2-yl)dihydropyrimidine-2,4(1H,3H)-dione (GT-08059)**

Referring to the method of Scheme 1, the target product (GT-08059) was prepared as a white solid (39 mg, yield 63%). $^1$H NMR (400 MHz, DMSO-d6) δ 10.70 (s, 1H), 8.52 (s, 1H), 7.95 (s, 1H), 7.88 (d, *J* = 7.8 Hz, 1H), 7.85 (s, 1H), 7.43 (d, *J* = 1.2 Hz, 1H), 4.82 (d, *J* = 16.7 Hz, 1H), 4.66 (s, 1H), 4.60 (s, 2H), 4.53 (s, 2H), 3.80 (t, *J* = 6.8 Hz, 2H), 3.73 (s, 2H), 3.48 - 3.40 (m, 2H), 3.23 (s, 2H), 2.86 (t, *J* = 7.8 Hz, 1H), 2.78 (t, *J*= 5.6 Hz, 1H), 2.57 (d, J= 0.8 Hz, 3H). LCMS (ESI) calcd for $C_{24}H_{26}N_7O_3S^+$ [M+H]$^+$: 492.18, found, 492.1.

**Example 309: preparation of 1-(5-((4-(6-isopropylthieno[2,3-d]pyrimidin-4-yl)piperazin-1-yl)methyl)-1-oxoisoindolin-2-yl)dihydropyrimidine-2,4(1H,3H)-dione (GT-08060)**

Referring to the method of Scheme 1, the target product (GT-08060) was prepared as a white solid (44 mg, yield 69%). $^1$H NMR (400 MHz, DMSO-d6) δ 10.70 (s, 1H), 8.51 (s, 1H), 7.94 (s, 1H), 7.87 (s, 1H), 7.85 (s, 1H), 7.37 (s, 1H), 4.82 (d, *J* = 16.7 Hz, 1H), 4.63 (d, *J* = 13.8 Hz, 2H), 4.60 (s, 2H), 4.53 (s, 2H), 3.80 (t, *J* = 6.8 Hz, 3H), 3.72 (s, 2H), 3.48 - 3.42 (m, 2H), 3.27 (d, *J* = 6.8 Hz, 1H), 3.22 (s, 1H), 2.86 (t, *J* = 7.8 Hz, 1H), 2.78 (t, *J* = 5.7 Hz, 1H), 1.34 (d, *J* = 6.8 Hz, 6H), 1.06 (t, *J* = 7.0 Hz, 1H). LCMS (ESI) calcd for $C_{26}H_{30}N_7O_3S^+$ [M+H]$^+$: 520.21, found, 520.2.

**Example 310: preparation of 1-(5-((4-(5,6-dimethylthieno[2,3-d]pyrimidin-4-yl)piperazin-1-yl)methyl)-1-oxoisoindolin-2-yl)dihydropyrimidine-2,4(1H,3H)-dione (GT-08061)**

Referring to the method of Scheme 1, the target product (GT-08061) was prepared as a white solid (48 mg, yield 75%). $^1$H NMR (400 MHz, DMSO-d6) δ 10.70 (s, 1H), 8.58 (s, 1H), 7.98 (s, 1H), 7.87 (s, 2H), 4.80 (s, 2H), 4.54 (s, 3H), 3.90 (d, *J* = 13.5 Hz, 2H), 3.80 (s, 2H), 3.59 (s, 2H), 3.38 (s, 3H), 2.86 (s, 1H), 2.78 (s, 1H), 2.46 (s, 3H), 2.41 (s, 3H). LCMS (ESI) calcd for $C_{25}H_{28}N_7O_3S^+$ [M+H]$^+$: 506.20, found, 506.2.

**Example** 311: **preparation** of **1-(5-((4-(5,6-dimethylthieno[2,3-d]pyrimidin-4-yl)-1,4-diazepan-1-yl)methyl)-1-oxoisoindolin-2-yl)dihydropyrimidine-2,4(1H,3H)-dione (GT-08062)**

Referring to the method of Scheme 1, the target product (GT-08062) was prepared as a white solid (45 mg, yield 69%). $^1$H NMR (400 MHz, DMSO) δ 10.69 (s, 1H), 8.52 (s, 1H), 7.94 (s, 1H), 7.85 (s, 2H), 4.79 (s, 1H), 4.59 (s, 1H), 4.49 (d, *J* = 3.4 Hz, 2H), 4.16 (s, 2H), 4.10 (s, 1H), 3.89 (d, *J* = 12.7 Hz, 2H), 3.79 (t, *J* = 6.8 Hz, 2H), 3.73 (d, *J* = 9.7 Hz, 1H), 3.54 (d, *J* = 21.1 Hz, 2H), 3.44 (d, *J* = 7.0 Hz, 1H), 3.14 (s, 1H), 2.86 (t, *J* = 7.8 Hz, 1H), 2.78 (t, *J* = 5.4 Hz, 1H), 2.43 (s, 3H), 2.34 (s, 3H),

2.15 (s, 1H). LCMS (ESI) calcd for $C_{26}H_{30}N_7O_3S^+$ $[M+H]^+$: 520.21, found, 520.2.

**Example 312: preparation of 1-(5-((4-(6-bromothieno[2,3-d]pyrimidin-4-yl)piperazin-1-yl)methyl)-1-oxoisoindolin-2-yl)dihydropyrimidine-2,4(1H,3H)-dione (GT-08063)**

Referring to the method of Scheme 1, the target product (GT-08063) was prepared as a white solid (35 mg, yield 66%). $^1H$ NMR (400 MHz, DMSO) δ 10.70 (s, 1H), 8.50 (s, 1H), 7.94 (d, *J* = 6.2 Hz, 2H), 7.87 (t, *J* = 6.1 Hz, 2H), 4.80 (s, 1H), 4.62 (d, *J* = 12.0 Hz, 2H), 4.53 (s, 2H), 3.80 (t, *J* = 6.8 Hz, 2H), 3.72 (s, 2H), 3.46 - 3.37 (m, 2H), 3.23 (s, 2H), 2.86 (s, 1H), 2.79 (s, 1H). LCMS (ESI) calcd for $C_{23}H_{23}BrN_7O_3S^+$ $[M+H]^+$: 556.08, found, 556.1.

**Example 313: preparation of 1-(1-oxo-5-((4-(6-phenylthieno[2,3-d]pyrimidin-4-yl)piperazin-1-yl)methyl)isoindolin-2-yl)dihydropyrimidine-2,4(1H,3H)-dione (GT-08064)**

Referring to the method of Scheme 1, the target product (GT-08064) was prepared as a white solid (34 mg, yield 65%). $^1H$ NMR (400 MHz, DMSO-d6) δ 10.70 (s, 1H), 8.52 (s, 1H), 8.01 (s, 1H), 7.94 (s, 1H), 7.88 (d, *J* = 3.9 Hz, 2H), 7.86 (s, 2H), 7.50 (t, *J* = 7.5 Hz, 2H), 7.43 (d, *J* = 7.3 Hz, 1H), 4.80 (s, 1H), 4.74 *(d, J* = 13.7 Hz, 2H), 4.58 (d, *J* = 22.9 Hz, 3H), 3.82 - 3.78 (m, 4H), 3.46 (s, 2H), 3.27 (s, 2H), 2.86 (s, 1H), 2.78 (s, 1H). LCMS (ESI) calcd for $C_{29}H_{28}N_7O_3S^+$ $[M+H]^+$: 554.20, found, 554.2.

**Example 314: preparation of 1-(1-oxo-5-((4-(5-phenylthieno[2,3-d]pyrimidin-4-yl)piperazin-1-yl)methyl)isoindolin-2-yl)dihydropyrimidine-2,4(1H,3H)-dione (GT-08065)**

Referring to the method of Scheme 1, the target product (GT-08065) was prepared as a white solid (32 mg, yield 61%). $^1H$ NMR (400 MHz, DMSO-d6) δ 10.70 (s, 1H), 8.69 (s, 1H), 7.86 (d, *J* = 7.8 Hz, 1H), 7.79 (d, *J* = 6.7 Hz, 2H), 7.68 (d, *J* = 7.8 Hz, 1H), 7.45 (dd, *J* = 7.6, 1.9 Hz, 2H), 7.40 (d, *J* = 7.2 Hz, 2H), 4.79 (s, 1H), 4.60 (s, 1H), 4.31 (s, 2H), 3.93 - 3.87 (m, 1H), 3.83 - 3.77 (m, 3H), 3.75 (s, 1H), 3.17 (s, 2H), 3.09 (d, *J* = 11.6 Hz, 2H), 2.86 (s, 1H), 2.78 (s, 1H), 2.48 - 2.43 (m, 1H). LCMS (ESI) calcd for $C_{29}H_{28}N_7O_3S^+$ $[M+H]^+$: 554.20, found, 554.2.

**Example 315: preparation of 1-(5-((4-(2-methyl-5-phenylthieno[2,3-d]pyrimidin-4-yl)piperazin-1-yl)methyl)-1-oxoisoindolin-2-yl)dihydropyrimidine-2,4(1H,3H)-dione (GT-08066)**

Referring to the method of Scheme 1, the target product (GT-08066) was prepared as a white solid (27 mg, yield 50%). $^1H$ NMR (400 MHz, DMSO) δ 10.70 (s, 1H), 7.86 (d, *J* = 7.8 Hz, 1H), 7.78 (s, 1H), 7.66 (d, *J* = *5.6* Hz, 2H), 7.43 (dd, *J* = *7.6,* 1.8 Hz, 2H), 7.39 (d, *J* = 7.2 Hz, 3H), 4.81 (d, *J* = 16.7 Hz, 1H), 4.57 (d, *J* = 16.3 Hz, 1H), 4.30 (s, 2H), 3.92 - 3.86 (m, 2H), 3.82 - 3.78 (m, 3H), 3.77 - 3.74 (m, 1H), 3.16 (s, 2H), 3.09 (d, *J* = 11.6 Hz, 2H), 2.88 - 2.83 (m, 1H), 2.78 (d, *J* = 5.8 Hz, 1H), 2.63 (s, 3H). LCMS (ESI) calcd for $C_{30}H_{30}N_7O_3S^+$ $[M+H]^+$: 568.21, found, 568.2.

**Example 316: preparation of 1-(5-((4-(2-methylthieno[2,3-d]pyrimidin-4-yl)piperazin-1-yl)methyl)-1-oxoisoindolin-2-yl)dihydropyrimidine-2,4(1H,3H)-dione (GT-08067)**

Referring to the method of Scheme 1, the target product (GT-08067) was prepared as a white solid (28 mg, yield 60%). $^1H$ NMR (400 MHz, DMSO) δ 10.70 (s, 1H), 7.93 (s, 1H), 7.88 (d, *J* = 7.8 Hz, 1H), 7.83 (d, *J* = 7.8 Hz, 1H), 7.73 - 7.65 (m, 2H), 4.82 (d, *J* = 16.4 Hz, 1H), 4.74 (s, 1H), 4.58 (d, *J* = 16.8 Hz, 1H), 4.53 (s, 2H), 3.82 - 3.77 (m, 5H), 3.46 (s, 2H), 3.25 (s, 2H), 2.86 (s, 1H), 2.78 (s, 1H), 2.60 - 2.60 (m, 1H), 2.57 (s, 3H). LCMS (ESI) calcd for $C_{24}H_{26}N_7O_3S^+$ $[M+H]^+$: 492.18, found, 492.1.

**Example 317: preparation of 1-(5-((4-(2-methyl-5,6,7,8-tetrahydrobenzo[4,5]thieno[2,3-d]pyrimidin-4-yl)piperazin-1-yl)methyl)-1-oxoisoindolin-2-yl)dihydropyrimidine-2,4(1H,3H)-dione (GT-08068)**

Referring to the method of Scheme 1, the target product (GT-08068) was prepared as a white solid (27 mg, yield 53%). $^1H$ NMR (400 MHz, DMSO-d6) δ 10.70 (s, 1H), 7.98 (s, 1H), 7.87 (s, 2H), 4.82 (d, *J* = 16.8 Hz, 1H), 4.62 (d, *J* = 12.8 Hz, 1H), 4.54 (s, 2H), 3.91 (d, *J* = 13.8 Hz, 3H), 3.80 (t, *J* = 6.8 Hz, 2H), 3.56 (t, *J* = 12.3 Hz, 2H), 3.39 (s, 2H), 3.31 (s, 2H), 2.87 (d, *J* = 8.0 Hz, 4H), 2.81 - 2.71 (m, 1H), 2.56 (s, 3H), 1.87 (d, *J* = 3.7 Hz, 2H), 1.75 (d, *J* = 3.4 Hz, 2H). LCMS (ESI) calcd for $C_{28}H_{32}N_7O_3S^+$ $[M+H]^+$: 546.23, found, 546.2.

Example 318: preparation of 1-(1-oxo-5-((4-(5,6,7,8-tetrahydrobenzo[4,5]thieno[2,3-d]pyrimidin-4-yl)piperazin-1-yl)methyl)isoindolin-2-yl)dihydropyrimidine-2,4(1H,3H)-dione (GT-08077)

Referring to the method of Scheme 1, the target product (GT-08077) was prepared as a white solid (31 mg, yield 61%). $^1H$ NMR (400 MHz, DMSO-d6) δ 10.70 (s, 1H), 8.58 (s, 1H), 7.99 (s, 1H), 7.87 (s, 2H), 4.83 (d, *J* = 16.6 Hz, 1H), 4.60 (s, 1H), 4.55 (s, 3H), 3.88 (d, *J* = 13.7 Hz, 2H), 3.80 (t, *J* = 6.8 Hz, 2H), 3.56 (s, 2H), 3.38 (s, 4H), 2.96 - 2.87 (m, 4H), 2.77 (dd, *J* = 14.1, 8.4 Hz, 1H), 1.88 (d, *J* = 4.4 Hz, 2H), 1.77 (d, J = 4.7 Hz, 2H). LCMS (ESI) calcd for $C_{27}H_{30}N_7O_3S^+$ [M+H]$^+$: 532.21, found, 532.1.

**Example** 319: **preparation** of **1-(5-((4-(6,7-dihydro-5H-cyclopenta[4,5]thieno[2,3-d]pyrimidin-4-yl)piperazin-1-yl)methyl)-1-oxoisoindolin-2-yl)dihydropyrimidine-2,4(1H,3H)-dione (GT-08079)**

Referring to the method of Scheme 1, the target product (GT-08079) was prepared as a white solid (23 mg, yield 47%). $^1H$ NMR (400 MHz, DMSO-d6) δ 10.70 (s, 1H), 8.53 (s, 1H), 7.97 (s, 1H), 7.87 (s, 2H), 4.82 (d, *J = 16.4* Hz, 1H), 4.58 (d, *J* = 16.9 Hz, 1H), 4.53 (s, 2H), 4.17 (s, 1H), 3.80 (t, *J* = 6.8 Hz, 2H), 3.64 (t, *J* = 12.5 Hz, 2H), 3.40 (s, 2H), 3.28 (s, 2H), 3.08 - 2.97 (m, 4H), 2.86 (t, *J* = 7.9 Hz, 1H), 2.77 (dd, *J* = 13.9, 8.2 Hz, 1H), 2.40 (d, *J* = 6.9 Hz, 2H). LCMS (ESI) calcd for $C_{26}H_{28}N_7O_3S^+$ [M+H]$^+$: 518.20, found, 518.2.

**Example 320: preparation of 1-(5-((4-(2-methyl-6,7-dihydro-5H-cyclopenta[4,5]thieno[2,3-d]pyrimidin-4-yl)piperazin-1-yl)methyl)-1-oxoisoindolin-2-yl)dihydropyrimidine-2,4(1H,3H)-dione (GT-08080)**

Referring to the method of Scheme 1, the target product (GT-08080) was prepared as a white solid (21 mg, yield 42%). $^1H$ NMR (400 MHz, DMSO-d6) δ 10.70 (s, 1H), 7.96 (s, 1H), 7.86 (d, *J* = 2.0 Hz, 2H), 4.82 *(d, J = 16.7* Hz, 1H), 4.60 (t, *J* = 14.4 Hz, 1H), 4.52 (s, 2H), 4.18 (s, 2H), 3.80 (t, *J* = 6.8 Hz, 2H), 3.62 (d, *J* = 13.0 Hz, 2H), 3.45 - 3.40 (m, 2H), 3.26 (s, 2H), 3.04 - 2.94 (m, 4H), 2.86 (t, *J* = 7.8 Hz, 1H), 2.78 (t, *J* = 5.6 Hz, 1H), 2.55 (s, 3H), 2.39 (d, *J* = 6.9 Hz, 2H). LCMS (ESI) calcd for $C_{27}H_{30}N_7O_3S^+$[M+H]$^+$: 532.21, found, 532.2.

**Example 321: preparation of 1-(1-oxo-5-((4-(thieno[2,3-d]pyrimidin-2-yl)piperazin-1-yl)methyl)isoindolin-2-yl)dihydropyrimidine-2,4(1H,3H)-dione (GT-08081)**

Referring to the method of Scheme 1, the target product (GT-08081) was prepared as a white solid (26 mg, yield 57%). $^1H$ NMR (400 MHz, DMSO-d6) δ 10.70 (s, 1H), 8.97 (s, 1H), 7.93 (s, 1H), 7.87 (d, *J* = 7.8 Hz, 1H), 7.83 (s, 1H), 7.45 (d, *J* = 6.0 Hz, 1H), 7.33 (d, *J* = 6.0 Hz, 1H), 4.79 (d, *J* = 6.6 Hz, 2H), 4.75 (s, 1H), 4.58 (d, *J* = 16.6 Hz, 1H), 4.49 (s, 2H), 3.80 (s, 1H), 3.78 (d, *J* = 3.5 Hz, 1H), 3.54 (s, 2H), 3.41 (d, *J* = 10.1 Hz, 2H), 3.14 (s, 2H), 2.86 (s, 1H), 2.78 (s, 1H). LCMS (ESI) calcd for $C_{23}H_{24}N_7O_3S^+$ [M+H]$^+$: 478.17, found, 478.1.

**Example 322: preparation of 1-(1-oxo-5-((4-(thieno[2,3-d]pyrimidin-4-yl)piperidin-1-yl)methyl)isoindolin-2-yl)dihydropyrimidine-2,4(1H,3H)-dione (GT-08082)**

Referring to the method of Scheme 1, the target product (GT-08082) was prepared as a white solid (21 mg, yield 46%). $^1H$ NMR (400 MHz, DMSO) δ 10.70 (s, 1H), 9.03 (s, 1H), 8.00 (d, *J* = 6.1 Hz, 1H), 7.95 (s, 1H), 7.89 (s, 1H), 7.86 (d, *J* = 7.5 Hz, 1H), 7.82 (d, *J* = 6.0 Hz, 1H), 4.82 (s, 1H), 4.62 (s, 1H), 4.50 (d, *J* = 4.2 Hz, 2H), 3.80 (t, *J* = 6.8 Hz, 2H), 3.55 (d, *J* = 12.7 Hz, 2H), 3.14 (s, 2H), 2.86 (t, *J* = 7.7 Hz, 1H), 2.79 (s, 1H), 2.34 (s, 2H), 2.06 (s, 2H). LCMS (ESI) calcd for $C_{24}H_{25}N_6O_3S^+$ [M+H]$^+$: 477.17, found, 477.1.

**Example 323: preparation of 1-(5-((4-(6-methylthieno[2,3-d]pyrimidin-4-yl)piperidin-1-yl)methyl)-1-oxoisoindolin-2-yl)dihydropyrimidine-2,4(1H,3H)-dione (GT-08083)**

Referring to the method of Scheme 1, the target product (GT-08083) was prepared as a white solid (23 mg, yield 49%). $^1H$ NMR (400 MHz, DMSO-d6) δ 10.70 (s, 1H), 8.93 (s, 1H), 7.95 (s, 1H), 7.90 (d, *J* = 7.8 Hz, 1H), 7.84 (s, 1H), 7.52 (d, *J* = 1.2 Hz, 1H), 4.84 (d, *J* = 16.4 Hz, 1H), 4.60 (d, *J* = 16.8 Hz, 1H), 4.49 (d, *J* = 4.3 Hz, 2H), 3.80 (t, *J* = 6.8 Hz, 2H), 3.53 (d, *J* = 11.1 Hz, 3H), 3.12 (s, 2H), 2.87 (dd, *J* = 16.2, 8.6 Hz, 1H), 2.78 (d, *J* = 5.5 Hz, 1H), 2.63 (t, *J* = 2.4 Hz, 3H), 2.33 (dd, *J* = 24.0, 12.8 Hz, 2H), 2.02 (d, *J*= 13.3 Hz, 2H). LCMS (ESI) calcd for $C_{25}H_{27}N_6O_3S^+$ [M+H]$^+$: 491.19, found, 491.1.

**Example 324: preparation of 1-(5-((4-(5,6-dimethylthieno[2,3-d]pyrimidin-4-yl)piperidin-1-yl)methyl)-1-oxoisoindolin-2-yl)dihydropyrimidine-2,4(1H,3H)-dione (GT-08084)**

Referring to the method of Scheme 1, the target product (GT-08084) was prepared as a white solid (21 mg, yield

44%). $^1$H NMR (400 MHz, DMSO-d6) δ 10.70 (s, 1H), 8.91 (s, 1H), 7.96 (s, 1H), 7.87 (q, *J* = 7.9 Hz, 2H), 4.81 (s, 1H), 4.59 (d, *J* = 16.8 Hz, 2H), 4.48 (d, *J* = 4.7 Hz, 2H), 3.95 (s, 1H), 3.91 (s, 1H), 3.82 - 3.74 (m, 5H), 3.45 (s, 2H), 3.24 (d, *J* = 11.1 Hz, 2H), 2.86 (s, 1H), 2.78 (s, 1H), 2.59 (d, *J* = 26.1 Hz, 1H), 2.37 (d, *J* = 13.0 Hz, 2H), 2.00 (d, *J* = 13.4 Hz, 2H). LCMS (ESI) calcd for $C_{26}H_{29}N_6O_3S^+$ [M+H]$^+$: 505.20, found, 505.1.

**Example 325: preparation of 1-(1-oxo-5-((4-(6-phenylthieno[2,3-d]pyrimidin-4-yl)piperidin-1-yl)methyl)isoindolin-2-yl)dihydropyrimidine-2,4(1H,3H)-dione (GT-08085)**

Referring to the method of Scheme 1, the target product (GT-08085) was prepared as a white solid (12 mg, yield 24%). $^1$H NMR (400 MHz, DMSO-d6) δ 10.70 (s, 1H), 9.00 (s, 1H), 8.29 (s, 1H), 7.94 (d, *J* = 6.3 Hz, 2H), 7.91 - 7.88 (m, 3H), 7.85 (s, 1H), 7.53 (d, *J* = 7.8 Hz, 2H), 7.48 (d, *J* = 7.2 Hz, 1H), 4.85 (d, *J = 16.8* Hz, 1H), 4.61 (d, *J* = 16.5 Hz, 1H), 4.53 (d, *J* = 3.8 Hz, 2H), 3.81 (t, *J = 6.8* Hz, 2H), 3.59 (d, *J* = 9.5 Hz, 3H), 3.11 (s, 2H), 2.86 (s, 1H), 2.78 (d, *J* = 5.8 Hz, 1H), 2.37 (d, *J* = 11.6 Hz, 2H), 2.11 (d, *J* = 13.1 Hz, 2H). LCMS (ESI) calcd for $C_{30}H_{29}N_6O_3S^+$ [M+H]$^+$: 553.20, found, 553.1.

**Example 326: preparation of 1-(1-oxo-5-((4-(5-phenylthieno[2,3-d]pyrimidin-4-yl)piperidin-1-yl)methyl)isoindolin-2-yl)dihydropyrimidine-2,4(1H,3H)-dione (GT-08086)**

Referring to the method of Scheme 1, the target product (GT-08086) was prepared as a white solid (29 mg, yield 54%). $^1$H NMR (400 MHz, DMSO) δ 10.71 (s, 1H), 9.06 (s, 1H), 7.88 (d, *J* = 7.9 Hz, 1H), 7.83 (d, J= 4.6 Hz, 2H), 7.72 (d, *J* = 7.9 Hz, 1H), 7.50 (d, *J* = 4.2 Hz, 1H), 7.48 (s, 1H), 7.44 (d, *J* = 5.8 Hz, 2H), 4.83 (d, *J* = 16.7 Hz, 1H), 4.63 (s, 1H), 4.34 (d, *J* = 3.7 Hz, 2H), 3.81 (t, *J* = 6.8 Hz, 2H), 3.42 (d, *J* = 7.0 Hz, 2H), 3.35 (s, 2H), 2.90 - 2.84 (m, 1H), 2.78 (d, *J* = 5.9 Hz, 1H), 2.17 (d, *J* = 8.2 Hz, 3H), 1.76 (d, *J* = 8.8 Hz, 2H). LCMS (ESI) calcd for $C_{30}H_{29}N_6O_3S^+$ [M+H]$^+$: 553.20, found, 553.1.

**Example 327: preparation of 1-(5-((4-(2-methyl-5-phenylthieno[2,3-d]pyrimidin-4-yl)piperidin-1-yl)methyl)-1-oxoisoindolin-2-yl)dihydropyrimidine-2,4(1H,3H)-dione (GT-08087)**

Referring to the method of Scheme 1, the target product (GT-08087) was prepared as a white solid (32 mg, yield 60%). $^1$H NMR (400 MHz, DMSO-d6) δ 10.72 (s, 1H), 7.88 (d, *J* = 7.8 Hz, 1H), 7.85 (s, 1H), 7.75 (s, 1H), 7.69 (s, 1H), 7.48 (dd, *J* = 5.8, 2.3 Hz, 1H), 7.45 (dd, *J* = 3.7, 2.3 Hz, 2H), 7.43 - 7.41 (m, 2H), 4.82 (s, 1H), 4.63 (s, 1H), 4.33 (s, 3H), 3.82 (t, *J* = 6.8 Hz, 2H), 3.33 (s, 2H), 2.90 - 2.85 (m, 1H), 2.79 (t, *J* = 5.7 Hz, 1H), 2.76 - 2.72 (m, 1H), 2.70 (s, 3H), 2.17 (s, 3H), 1.72 (s, 2H). LCMS (ESI) calcd for $C_{31}H_{31}N_6O_3S^+$ [M+H]$^+$: 567.22, found, 567.1.

**Example 328: preparation of 1-(5-((4-(2-methylthieno[2,3-d]pyrimidin-4-yl)piperidin-1-yl)methyl)-1-oxoisoindolin-2-yl)dihydropyrimidine-2,4(1H,3H)-dione (GT-08088)**

Referring to the method of Scheme 1, the target product (GT-08088) was prepared as a white solid (32 mg, yield 68%). $^1$H NMR (400 MHz, DMSO) δ 10.63 (s, 1H), 7.84 (d, *J* = 10.4 Hz, 2H), 7.79 (d, *J* = 6.0 Hz, 1H), 7.75 (d, *J* = 7.8 Hz, 1H), 7.68 (d, *J* = 6.1 Hz, 1H), 4.77 (d, *J* = 17.1 Hz, 1H), 4.55 (s, 1H), 4.43 (d, *J* = 4.4 Hz, 2H), 3.80 - 3.70 (m, 3H), 3.54 (s, 1H), 3.06 (d, *J* = 8.4 Hz, 2H), 2.80 (d, *J* = 7.6 Hz, 1H), 2.71 (s, 1H), 2.65 (s, 1H), 2.62 (s, 3H), 2.24 (d, *J* = 13.3 Hz, 2H), 1.96 (d, *J* = 13.4 Hz, 2H). LCMS (ESI) calcd for $C_{25}H_{27}N_6O_3S^+$ [M+H]$^+$: 491.19, found, 491.1.

**Example 329: preparation of 1-(5-((4-(2-methyl-5,6,7,8-tetrahydrobenzo[4,5]thieno[2,3-d]pyrimidin-4-yl)piperidin-1-yl)methyl)-1-oxoisoindolin-2-yl)dihydropyrimidine-2,4(1H,3H)-dione (GT-08089)**

Referring to the method of Scheme 1, the target product (GT-08089) was prepared as a white solid (19 mg, yield 37%). $^1$H NMR (400 MHz, DMSO-d6) δ 10.70 (s, 1H), 7.92 - 7.87 (m, 2H), 7.78 (d, *J* = 8.0 Hz, 1H), 4.83 (d, *J* = 16.3 Hz, 1H), 4.59 (d, *J* = 16.7 Hz, 1H), 4.47 (d, *J* = 4.8 Hz, 2H), 3.80 (t, *J* = 6.8 Hz, 2H), 3.62 (t, *J* = 11.7 Hz, 1H), 3.40 - 3.35 (m, 1H), 3.22 (d, *J* = 11.2 Hz, 2H), 2.98 (s, 2H), 2.85 (s, 3H), 2.77 (d, *J* = 5.7 Hz, 1H), 2.63 (s, 3H), 2.54 (s, 1H), 2.26 (s, 2H), 1.99 (d, *J* = 13.6 Hz, 2H), 1.86 (s, 4H). LCMS (ESI) calcd for $C_{29}H_{33}N_6O_3S^+$ [M+H]$^+$: 545.23, found, 545.2.

**Example 330: preparation of 2-(2,4-dioxotetrahydropyrimidin-1(2H)-yl)-5-((4-(thieno[2,3-d]pyrimidin-4-yl)piperazin-1-yl)methyl)isoindoline-1,3-dione (GT-08090)**

Referring to the method of Scheme 1, the target product (GT-08090) was prepared as a white solid (9 mg, yield 20%). $^1$H NMR (400 MHz, DMSO-d6) δ 10.93 (s, 1H), 8.55 (s, 1H), 8.33 (s, 1H), 8.18 (s, 1H), 8.12 (d, *J* = 7.7 Hz, 1H), 7.76 (d, *J* = 6.1 Hz, 1H), 7.70 (d, *J* = 6.2 Hz, 1H), 4.70 (s, 2H), 4.61 (s, 2H), 3.72 - 3.64 (m, 3H), 3.45 (s, 2H), 3.25 (s, 2H), 2.86 (t, *J* = 6.7 Hz, 2H), 2.55 (s, 1H). LCMS (ESI) calcd for $C_{23}H_{22}N_7O_4S^+$ [M+H]$^+$: 492.14, found, 492.1.

Example **331: preparation of 2-(2,4-dioxotetrahydropyrimidin-1(2H)-yl)-5-((4-(5-methylthieno[2,3-d]pyrimidin-4-yl)piperazin-1-yl)methyl)isoindoline-1,3-dione (GT-08091)**

Referring to the method of Scheme 1, the target product (GT-08091) was prepared as a white solid (24 mg, yield 52%). $^1$H NMR (400 MHz, DMSO-d6) δ 10.93 (s, 1H), 8.63 (s, 1H), 8.36 (s, 1H), 8.22 (d, *J* = 7.7 Hz, 1H), 8.12 (d, *J* = 7.7 Hz, 1H), 7.48 (s, 1H), 4.63 (s, 2H), 3.92 (d, *J* = 12.9 Hz, 4H), 3.54 (d, *J* = 12.8 Hz, 2H), 3.40 (s, 2H), 3.35 (s, 2H), 2.86 (t, *J* = 6.8 Hz, 2H), 2.55 (s, 3H). LCMS (ESI) calcd for $C_{24}H_{24}N_7O_4S^+$ [M+H]$^+$: 506.16, found, 506.1.

**Example 332: preparation of 2-(2,4-dioxotetrahydropyrimidin-1(2H)-yl)-5-((4-(6-methylthieno[2,3-d]pyrimidin-4-yl)piperazin-1-yl)methyl)isoindoline-1,3-dione (GT-08092)**

Referring to the method of Scheme 1, the target product (GT-08092) was prepared as a white solid (28 mg, yield 61%). $^1$H NMR (400 MHz, DMSO-d6) δ 10.94 (s, 1H), 8.54 (s, 1H), 8.36 (s, 1H), 8.22 (d, *J* = 1.0 Hz, 1H), 8.11 (d, *J* = 7.7 Hz, 1H), 7.44 (d, *J* = 1.0 Hz, 1H), 4.63 (s, 4H), 3.83 (s, 2H), 3.74 (s, 2H), 3.45 (s, 2H), 3.27 (s, 2H), 2.87 (t, *J* = 6.8 Hz, 2H), 2.57 (s, 3H). LCMS (ESI) calcd for $C_{24}H_{24}N_7O_4S^+$ [M+H]$^+$: 506.16, found, 506.1.

**Example 333: preparation of 2-(2,4-dioxotetrahydropyrimidin-1(2H)-yl)-5-((4-(6-isopropylthieno[2,3-d]pyrimidin-4-yl)piperazin-1-yl)methyl)isoindoline-1,3-dione (GT-08093)**

Referring to the method of Scheme 1, the target product (GT-08093) was prepared as a white solid (30 mg, yield 62%). $^1$H NMR (400 MHz, DMSO-d6) δ 10.93 (s, 1H), 8.49 (s, 1H), 8.34 (s, 1H), 8.23 - 8.19 (m, 1H), 8.12 (d, *J* = 7.7 Hz, 1H), 7.36 (s, 1H), 4.62 (s, 4H), 3.83 (t, *J* = 6.8 Hz, 2H), 3.50 - 3.39 (m, 3H), 3.32 - 3.18 (m, 3H), 2.86 (t, *J* = 6.8 Hz, 2H), 1.34 (d, *J* = 6.8 Hz, 6H), 1.06 (t, *J* = 7.0 Hz, 1H). LCMS (ESI) calcd for $C_{26}H_{28}N_7O_4S^+$ [M+H]$^+$: 534.19, found, 534.1.

**Example 334: preparation of 5-((4-(5,6-dimethylthieno[2,3-d]pyrimidin-4-yl)piperazin-1-yl)methyl)-2-(2,4-dioxotetrahydropyrimidin-1(2H)-yl)isoindoline-1,3-dione (GT-08094)**

Referring to the method of Scheme 1, the target product (GT-08094) was prepared as a white solid (32 mg, yield 68%). $^1$H NMR (400 MHz, DMSO-d6) δ 10.84 (s, 1H), 8.48 (s, 1H), 8.26 (s, 1H), 8.11 (s, 1H), 8.03 (d, *J* = 7.7 Hz, 1H), 4.54 (s, 2H), 3.79 (d, *J* = 13.7 Hz, 2H), 3.74 (t, *J* = 6.8 Hz, 2H), 3.42 (s, 2H), 3.31 (s, 2H), 3.27 (s, 2H), 2.78 (t, *J* = 6.8 Hz, 2H), 2.38 (s, 3H), 2.34 (s, 3H). LCMS (ESI) calcd for $C_{25}H_{26}N_7O_4S^+$ [M+H]$^+$: 520.18, found, 520.1.

**Example 335: preparation of 5-((4-(5,6-dimethylthieno[2,3-d]pyrimidin-4-yl)-1,4-diazepan-1-yl)methyl)-2-(2,4-dioxotetrahydropyrimidin-1(2H)-yl)isoindoline-1,3-dione (GT-08095)**

Referring to the method of Scheme 1, the target product (GT-08095) was prepared as a white solid (34 mg, yield 70%). $^1$H NMR (400 MHz, DMSO-d6) δ 10.93 (s, 1H), 8.50 (s, 1H), 8.35 (s, 1H), 8.22 (d, *J* = 7.7 Hz, 1H), 8.10 (d, *J* = 7.7 Hz, 1H), 4.59 (s, 2H), 4.10 (d, *J* = 37.4 Hz, 3H), 3.89 (s, 2H), 3.68 (s, 2H), 3.55 (s, 2H), 3.44 (d, *J* = 6.9 Hz, 1H), 3.16 (s, 1H), 2.86 (t, *J* = 6.8 Hz, 2H), 2.43 (s, 3H), 2.34 (s, 3H), 2.15 (s, 1H).LCMS (ESI) calcd for $C_{26}H_{28}N_7O_4S^+$ [M+H]$^+$: 534.19, found, 534.1.

**Example 336: preparation of 5-((4-(6-bromothieno[2,3-d]pyrimidin-4-yl)piperazin-1-yl)methyl)-2-(2,4-dioxotetrahydropyrimidin-1(2H)-yl)isoindoline-1,3-dione (GT-08096)**

Referring to the method of Scheme 1, the target product (GT-08096) was prepared as a white solid (34 mg, yield 66%). $^1$H NMR (400 MHz, DMSO-d6) δ 10.93 (s, 1H), 8.50 (s, 1H), 8.35 (s, 1H), 8.21 (d, *J* = 1.1 Hz, 1H), 8.12 (d, *J* = 7.7 Hz, 1H), 7.94 (s, 1H), 4.62 (s, 4H), 3.83 (s, 2H), 3.68 (d, *J* = 6.7 Hz, 2H), 3.44 (d, *J* = 7.0 Hz, 2H), 3.24 (s, 2H), 2.87 (t, *J* = 6.8 Hz, 2H). LCMS (ESI) calcd for $C_{23}H_{21}BrN_7O_4S^+$ [M+H]$^+$: 570.06, found, 570.0.

**Example 337: preparation of 2-(2,4-dioxotetrahydropyrimidin-1(2H)-yl)-5-((4-(6-phenylthieno[2,3-d]pyrimidin-4-yl)piperazin-1-yl)methyl)isoindoline-1,3-dione (GT-08097)**

Referring to the method of Scheme 1, the target product (GT-08097) was prepared as a white solid (34 mg, yield 66%). $^1$H NMR (400 MHz, DMSO-d6) δ 10.93 (s, 1H), 8.52 (s, 1H), 8.34 (s, 1H), 8.20 (s, 1H), 8.13 (d, *J* = 7.7 Hz, 1H), 8.01 (s, 1H), 7.90 - 7.84 (m, 2H), 7.50 (t, *J* = 7.5 Hz, 2H), 7.43 (s, 1H), 4.72 (s, 2H), 4.64 (s, 2H), 3.83 (t, *J* = 6.8 Hz, 2H), 3.68 (dd, *J* = 14.8, 8.8 Hz, 4H), 3.29 (s, 2H), 2.87 (d, *J* = 6.8 Hz, 2H). LCMS (ESI) calcd for $C_{29}H_{26}N_7O_4S^+$ [M+H]$^+$: 568.18, found, 568.1.

**Example 338: preparation of 2-(2,4-dioxotetrahydropyrimidin-1(2H)-yl)-5-((4-(5-phenylthieno[2,3-d]pyrimidin-4-yl)piperazin-1-yl)methyl)isoindoline-1,3-dione (GT-08098)**

Referring to the method of Scheme 1, the target product (GT-08098) was prepared as a white solid (33 mg, yield 64%). $^1$H NMR (400 MHz, DMSO-d6) δ 10.93 (s, 1H), 8.68 (s, 1H), 8.20 (s, 1H), 8.09 (s, 1H), 8.08 (s, 1H), 7.78 (s, 1H), 7.46 - 7.44 (m, 2H), 7.43 - 7.39 (m, 3H), 4.40 (s, 2H), 3.84 (d, *J* = 6.8 Hz, 5H), 3.14 (d, *J* = 11.4 Hz, 4H), 2.86 (s, 2H), 2.48 - 2.38 (m, 1H). LCMS (ESI) calcd for $C_{29}H_{26}N_7O_4S^+$ $[M+H]^+$: 568.18, found, 568.1.

**Example 339: preparation of 2-(2,4-dioxotetrahydropyrimidin-1(2H)-yl)-5-((4-(2-methyl-5-phenylthieno[2,3-d]pyrimidin-4-yl)piperazin-1-yl)methyl)isoindoline-1,3-dione (GT-08099)**

Referring to the method of Scheme 1, the target product (GT-08099) was prepared as a white solid (23 mg, yield 44%). $^1$H NMR (400 MHz, DMSO-d6) δ 10.93 (s, 1H), 8.20 (s, 1H), 8.09 (d, *J* = 4.8 Hz, 2H), 7.68 (s, 1H), 7.45 - 7.42 (m, 2H), 7.42 - 7.38 (m, 3H), 4.40 (s, 2H), 3.84 (d, *J* = 6.8 Hz, 4H), 3.79 - 3.74 (m, 1H), 3.13 (s, 4H), 2.86 (t, *J* = 6.7 Hz, 2H), 2.63 (s, 3H), 2.47 (s, 1H). LCMS (ESI) calcd for $C_{30}H_{28}N_7O_4S^+$ $[M+H]^+$: 582.19, found, 582.1.

**Example 340: preparation of 2-(2,4-dioxotetrahydropyrimidin-1(2H)-yl)-5-((4-(2-methylthieno[2,3-d]pyrimidin-4-yl)piperazin-1-yl)methyl)isoindoline-1,3-dione (GT-08100)**

Referring to the method of Scheme 1, the target product (GT-08100) was prepared as a white solid (31 mg, yield 67%). $^1$H NMR (400 MHz, DMSO-d6) δ 10.93 (s, 1H), 8.35 (s, 1H), 8.21 (d, *J* = 1.1 Hz, 1H), 8.12 (d, *J* = 7.7 Hz, 1H), 7.72 (s, 2H), 4.78 (s, 2H), 4.63 (s, 2H), 3.83 (t, *J* = 6.8 Hz, 4H), 3.45 (s, 2H), 3.29 (s, 2H), 2.86 (t, *J* = 6.8 Hz, 2H), 2.58 (s, 3H). LCMS (ESI) calcd for $C_{24}H_{24}N_7O_4S^+$ $[M+H]^+$: 506.16, found, 506.1.

**Example 341: preparation of 2-(2,4-dioxotetrahydropyrimidin-1(2H)-yl)-5-((4-(2-methyl-5,6,7,8-tetrahydrobenzo[4,5]thieno[2,3-d]pyrimidin-4-yl)piperazin-1-yl)methyl)isoindoline-1,3-dione (GT-08101)**

Referring to the method of Scheme 1, the target product (GT-08101) was prepared as a white solid (28 mg, yield 55%). $^1$H NMR (400 MHz, DMSO-d6) δ 10.99 (s, 1H), 8.45 (s, 1H), 8.31 (s, 1H), 8.17 (d, *J* = 7.7 Hz, 1H), 4.69 (s, 2H), 3.89 (t, *J* = 6.8 Hz, 3H), 3.61 (s, 2H), 3.46 (s, 2H), 3.40 (s, 2H), 2.93 (d, *J* = 6.7 Hz, 7H), 2.63 (s, 3H), 1.93 (d, *J* = 3.8 Hz, 2H), 1.81 (d, *J* = 3.3 Hz, 2H). LCMS (ESI) calcd for $C_{28}H_{30}N_7O_4S^+$ $[M+H]^+$: 560.21, found, 560.2.

**Example 342: preparation of 2-(2,4-dioxotetrahydropyrimidin-1(2H)-yl)-5-((4-(5,6,7,8-tetrahydrobenzo[4,5]thieno[2,3-d]pyrimidin-4-yl)piperazin-1-yl)methyl)isoindoline-1,3-dione** (GT-**08102)**

Referring to the method of Scheme 1, the target product (GT-08102) was prepared as a white solid (25 mg, yield 55%). $^1$H NMR (400 MHz, DMSO-d6) δ 10.93 (s, 1H), 8.58 (s, 1H), 8.39 (s, 1H), 8.25 (dd, *J* = 7.8, 1.1 Hz, 1H), 8.11 (d, *J* = 7.7 Hz, 1H), 4.64 (s, 2H), 3.83 (s, 4H), 3.58-3.40 (m, 2H), 3.40 (s, 4H), 2.92 (s, 2H), 2.86 (t, *J* = 6.8 Hz, 4H), 1.89 (s, 2H), 1.77 (s, 2H). LCMS (ESI) calcd for $C_{27}H_{28}N_7O_4S^+$ $[M+H]^+$: 546.19, found, 546.1.

**Example 343: preparation of 5-((4-(6,7-dihydro-5H-cyclopenta[4,5]thieno[2,3-d]pyrimidin-4-yl)piperazin-1-yl)methyl)-2-(2,4-dioxotetrahydropyrimidin-1(2H)-yl)isoindoline-1,3-dione** (GT-**08103)**

Referring to the method of Scheme 1, the target product (GT-08103) was prepared as a white solid (13 mg, yield 27%). $^1$H NMR (400 MHz, DMSO-d6) δ 10.93 (s, 1H), 8.52 (s, 1H), 8.36 (s, 1H), 8.22 (dd, *J* = 7.7, 1.2 Hz, 1H), 8.13 (s, 1H), 4.62 (s, 2H), 4.13 (s, 2H), 3.82 (d, *J* = 6.8 Hz, 2H), 3.59 (s, 2H), 3.42 (d, *J* = 7.0 Hz, 2H), 3.29 (s, 2H), 3.08 - 2.98 (m, 4H), 2.86 (s, 2H), 2.41 (dd, *J* = 14.0, 7.1 Hz, 2H). LCMS (ESI) calcd for $C_{26}H_{26}N_7O_4S^+$ $[M+H]^+$: 532.18, found, 532.1.

**Example 344: preparation of 2-(2,4-dioxotetrahydropyrimidin-1(2H)-yl)-5-((4-(2-methyl-6,7-dihydro-5H-cyclopenta[4,5]thieno[2,3-d]pyrimidin-4-yl)piperazin-1-yl)methyl)isoindoline-1,3-dione (GT-08104)**

Referring to the method of Scheme 1, the target product (GT-08104) was prepared as a white solid (21 mg, yield 42%). $^1$H NMR (400 MHz, DMSO-d6) δ 10.99 (s, 1H), 8.44 (s, 1H), 8.30 (dd, *J*= 7.8, 1.1 Hz, 1H), 8.17 (d, *J* = 7.7 Hz, 1H), 4.68 (s, 2H), 4.25 (s, 2H), 3.89 (s, 2H), 3.71 (d, *J* = 7.0 Hz, 2H), 3.49 (s, 2H), 3.35 (s, 2H), 3.06 (d, *J* = 18.2 Hz, 4H), 2.92 (t, *J* = 6.8 Hz, 2H), 2.62 (s, 3H), 2.44 (s, 2H). LCMS (ESI) calcd for $C_{27}H_{28}N_7O_4S^+$ $[M+H]^+$: 546.19, found, 546.1.

**Example 345: preparation of 2-(2,4-dioxotetrahydropyrimidin-1(2H)-yl)-5-((4-(thieno[2,3-d]pyrimidin-2-yl)piperazin-1-yl)methyl)isoindoline-1,3-dione (GT-08105)**

Referring to the method of Scheme 1, the target product (GT-08105) was prepared as a white solid (28 mg, yield 62%). $^1$H NMR (400 MHz, DMSO-d6) δ 10.93 (s, 1H), 8.97 (s, 1H), 8.32 (s, 1H), 8.16 (d, *J* = 1.2 Hz, 1H), 8.11 (d, *J*= 7.6 Hz, 1H), 7.45 (d, *J* = 5.9 Hz, 1H), 7.33 (d, *J* = 6.0 Hz, 1H), 4.77 (d, *J* = 12.6 Hz, 2H), 4.58 (s, 2H), 3.82 (d, *J* = 6.8 Hz, 2H), 3.51 (d, *J* = 12.4 Hz, 2H), 3.44 (d, *J* = 7.0 Hz, 2H), 3.14 (s, 2H), 2.87 (d, *J* = 6.7 Hz, 2H). LCMS (ESI) calcd for $C_{23}H_{22}N_7O_4S^+$ [M+H]$^+$: 492.14, found, 492.1.

**Example 346: preparation of 2-(2,4-dioxotetrahydropyrimidin-1(2H)-yl)-5-((4-(thieno[2,3-d]pyrimidin-4-yl)piperidin-1-yl)methyl)isoindoline-1,3-dione (GT-08106)**

Referring to the method of Scheme 1, the target product (GT-08106) was prepared as a white solid (19 mg, yield 42%). $^1$H NMR (400 MHz, DMSO-d6) δ 10.93 (s, 1H), 9.03 (s, 1H), 8.35 (s, 1H), 8.20 (s, 1H), 8.14 (d, *J* = 7.7 Hz, 1H), 8.01 (d, *J* = 6.0 Hz, 1H), 7.81 (d, *J* = 6.1 Hz, 1H), 4.60 (s, 2H), 3.83 (t, *J* = 6.7 Hz, 2H), 3.63 (d, *J* = 12.0 Hz, 1H), 3.56 (d, *J* = 11.9 Hz, 2H), 3.16 (d, *J* = 10.7 Hz, 2H), 2.87 (t, *J* = 6.8 Hz, 2H), 2.31 (d, *J* = 13.6 Hz, 2H), 2.08 (s, 2H). LCMS (ESI) calcd for $C_{24}H_{23}N_6O_4S^+$ [M+H]$^+$: 491.15, found, 491.1.

**Example** 347: **preparation** of **2-(2,4-dioxotetrahydropyrimidin-1(2H)-yl)-5-((4-(6-methylthieno[2,3-d]pyrimidin-4-yl)piperidin-1-yl)methyl)isoindoline-1,3-dione (GT-08107)**

Referring to the method of Scheme 1, the target product (GT-08107) was prepared as a white solid (18 mg, yield 39%). $^1$H NMR (400 MHz, DMSO-d6) δ 10.94 (s, 1H), 8.93 (s, 1H), 8.37 (s, 1H), 8.22 (d, *J* = 1.1 Hz, 1H), 8.14 (d, *J* = 7.7 Hz, 1H), 7.51 (d, *J* = 1.2 Hz, 1H), 4.60 (s, 2H), 3.84 (s, 2H), 3.53 (s, 3H), 3.36 (s, 1H), 3.14 (d, *J* = 10.3 Hz, 2H), 2.88 (d, *J* = 6.7 Hz, 2H), 2.63 (d, *J* = 0.8 Hz, 3H), 2.32 (dd, *J* = 26.4, 12.6 Hz, 2H), 2.04 (d, *J* = 12.7 Hz, 2H). LCMS (ESI) calcd for $C_{25}H_{25}N_6O_4S^+$ [M+H]$^+$: 505.17, found, 505.1.

**Example** 348: **preparation** of **2-(2,4-dioxotetrahydropyrimidin-1(2H)-yl)-5-((4-(6-phenylthieno[2,3-d]pyrimidin-4-yl)piperidin-1-yl)methyl)isoindoline-1,3-dione (GT-08108)**

Referring to the method of Scheme 1, the target product (GT-08108) was prepared as a white solid (19 mg, yield 37%). $^1$H NMR (400 MHz, DMSO-d6) δ 10.94 (s, 1H), 9.01 (s, 1H), 8.35 (s, 1H), 8.27 (s, 1H), 8.21 (d, *J* = 8.5 Hz, 1H), 8.17 (s, 1H), 7.89 (d, *J* = 7.3 Hz, 2H), 4.63 (s, 2H), 3.84 (t, *J* = 6.8 Hz, 2H), 3.61 (d, *J* = 12.0 Hz, 3H), 3.14 (d, *J* = 10.5 Hz, 2H), 2.88 (d, *J* = 6.8 Hz, 2H), 2.32 (d, *J* = 8.7 Hz, 2H), 2.16 - 2.10 (m, 2H). LCMS (ESI) calcd for $C_{30}H_{27}N_6O_4S^+$ [M+H]$^+$: 567.18, found, 567.1.

**Example** 349: **preparation** of **2-(2,4-dioxotetrahydropyrimidin-1(2H)-yl)-5-((4-(5-phenylthieno[2,3-d]pyrimidin-4-yl)piperidin-1-yl)methyl)isoindoline-1,3-dione (GT-08109)**

Referring to the method of Scheme 1, the target product (GT-08109) was prepared as a white solid (26 mg, yield 51%). $^1$H NMR (400 MHz, DMSO-d6) δ 10.95 (s, 1H), 9.07 (s, 1H), 8.30 (s, 1H), 8.18 (dd, *J* = 7.8, 1.1 Hz, 1H), 8.12 (s, 1H), 7.84 (s, 1H), 7.50 (d, *J* = 4.0 Hz, 3H), 7.47 (d, *J* = 2.5 Hz, 3H), 4.43 (s, 2H), 3.85 (t, *J* = 6.8 Hz, 3H), 3.34 (d, *J* = 8.5 Hz, 2H), 2.88 (t, *J* = 6.8 Hz, 2H), 2.78 (d, *J* = 7.1 Hz, 1H), 2.21 (s, 3H), 1.75 (s, 2H). LCMS (ESI) calcd for $C_{30}H_{27}N_6O_4S^+$ [M+H]$^+$: 567.18, found, 567.1.

**Example 350: preparation of 2-(2,4-dioxotetrahydropyrimidin-1(2H)-yl)-5-((4-(2-methyl-5-phenylthieno[2,3-d]pyrimidin-4-yl)piperidin-1-yl)methyl)isoindoline-1,3-dione (GT-08110)**

Referring to the method of Scheme 1, the target product (GT-08110) was prepared as a white solid (29 mg, yield 55%). $^1$H NMR (400 MHz, DMSO-d6) δ 10.95 (s, 1H), 8.28 (s, 1H), 8.16 (d, *J* = 7.8 Hz, 1H), 8.11 (d, *J* = 7.7 Hz, 1H), 7.69 (s, 1H), 7.49 - 7.45 (m, 5H), 4.43 (s, 2H), 3.84 (d, *J* = 6.7 Hz, 2H), 3.34 (s, 2H), 2.88 (t, *J* = 6.7 Hz, 2H), 2.77 (s, 1H), 2.70 (s, 3H), 2.18 (d, *J* = 8.5 Hz, 4H), 1.76 (s, 2H). LCMS (ESI) calcd for $C_{31}H_{29}N_6O_4S^+$ [M+H]$^+$: 581.20, found, 581.1.

**Example 351: preparation of 2-(2,4-dioxotetrahydropyrimidin-1(2H)-yl)-5-((4-(2-methyl-5,6,7,8-tetrahydrobenzo[4,5]thieno[2,3-d]pyrimidin-4-yl)piperidin-1-yl)methyl)isoindoline-1,3-dione (GT-08111)**

Referring to the method of Scheme 1, the target product (GT-08111) was prepared as a white solid (18 mg, yield 36%). $^1$H NMR (400 MHz, DMSO-d6) δ 10.94 (s, 1H), 8.35 (s, 1H), 8.20 (s, 1H), 8.13 (s, 1H), 4.58 (s, 2H), 3.83 (d, *J* = 5.8 Hz,

3H), 3.51 - 3.41 (m, 4H), 3.24 (d, *J* = 11.2 Hz, 2H), 2.99 (s, 3H), 2.86 (d, *J* = 5.4 Hz, 3H), 2.64 (s, 3H), 2.30 (d, *J* = 13.4 Hz, 3H), 2.01 (s, 2H), 1.86 (s, 4H). LCMS (ESI) calcd for $C_{29}H_{31}N_6O_4S^+$ $[M+H]^+$: 559.21, found, 559.1.

**Example 352: preparation of 1-(1-oxo-5-((4-(5,6,7,8-tetrahydrobenzo[4,5]thieno[2,3-d]pyrimidin-4-yl)piperidin-1-yl)methyl)isoindolin-2-yl)dihydropyrimidine-2,4(1H,3H)-dione (GT-08112)**

Referring to the method of Scheme 1, the target product (GT-08112) was prepared as a white solid (30 mg, yield 60%). $^1H$ NMR (400 MHz, DMSO-d6) δ 10.69 (s, 1H), 8.90 (s, 1H), 7.94 - 7.86 (m, 2H), 7.82 (s, 1H), 4.83 (d, *J* = 16.7 Hz, 1H), 4.62 (d, *J* = 7.3 Hz, 1H), 4.47 (d, *J* = 4.3 Hz, 2H), 3.68 - 3.66 (m, 1H), 3.44 (dd, *J* = 14.0, 7.0 Hz, 4H), 3.21 (d, *J* = 10.5 Hz, 2H), 3.01 (s, 2H), 2.89 (s, 3H), 2.78 (s, 1H), 2.33 (s, 2H), 2.00 (d, *J* = 12.9 Hz, 2H), 1.87 (s, 4H). LCMS (ESI) calcd for $C_{28}H_{31}N_6O_3S^+$ $[M+H]^+$: 531.22, found, 531.1.

**Example 353: preparation of 1-(5-((4-(6,7-dihydro-5H-cyclopenta[4,5]thieno[2,3-d]pyrimidin-4-yl)piperidin-1-yl)methyl)-1-oxoisoindolin-2-yl)dihydropyrimidine-2,4(1H,3H)-dione (GT-08113)**

Referring to the method of Scheme 1, the target product (GT-08113) was prepared as a white solid (26 mg, yield 53%). $^1H$ NMR (400 MHz, DMSO-d6) δ 10.70 (s, 1H), 8.91 (s, 1H), 7.97 (s, 1H), 7.87 (d, *J* = 3.5 Hz, 2H), 4.83 (d, *J* = 16.6 Hz, 1H), 4.66 - 4.53 (m, 2H), 4.48 (d, *J* = 4.7 Hz, 2H), 3.80 (t, *J* = 6.8 Hz, 2H), 3.47 (s, 3H), 3.29 - 3.19 (m, 2H), 3.13 (s, 2H), 3.06 (s, 2H), 2.87 (s, 1H), 2.78 (s, 1H), 2.48 (s, 1H), 2.35 (d, *J* = 11.7 Hz, 2H), 2.01 (s, 2H). LCMS (ESI) calcd for $C_{27}H_{29}N_6O_3S^+$ $[M+H]^+$: 517.20, found, 517.1.

**Example 354: preparation of 1-(5-((4-(2-methyl-6,7-dihydro-5H-cyclopenta[4,5]thieno[2,3-d]pyrimidin-4-yl)piperidin-1-yl)methyl)-1-oxoisoindolin-2-yl)dihydropyrimidine-2,4(1H,3H)-dione (GT-08114)**

Referring to the method of Scheme 1, the target product (GT-08114) was prepared as a white solid (32 mg, yield 63%). $^1H$ NMR (400 MHz, DMSO-d6) δ 10.69 (s, 1H), 7.96 - 7.85 (m, 2H), 7.83 (s, 1H), 4.83 (d, *J* = 16.7 Hz, 1H), 4.71 - 4.52 (m, 2H), 4.47 (d, *J* = 4.0 Hz, 2H), 3.80 (t, *J* = 6.7 Hz, 2H), 3.56 - 3.34 (m, 4H), 3.21 (d, *J* = 10.7 Hz, 2H), 3.10 (s, 2H), 3.02 (s, 2H), 2.87 (d, *J* = 7.6 Hz, 1H), 2.78 (s, 1H), 2.66 (s, 3H), 2.30 (d, *J* = 26.7 Hz, 2H), 2.00 (s, 2H). LCMS (ESI) calcd for $C_{28}H_{31}N_6O_3S^+$ $[M+H]^+$: 531.22, found, 531.2.

**Example 355: preparation of 1-(1-oxo-5-((4-(thieno[2,3-d]pyrimidin-2-yl)piperidin-1-yl)methyl)isoindolin-2-yl)dihydropyrimidine-2,4(1H,3H)-dione (GT-08115)**

Referring to the method of Scheme 1, the target product (GT-08115) was prepared as a white solid (13 mg, yield 28%). $^1H$ NMR (400 MHz, DMSO-d6) δ 10.70 (s, 1H), 9.31 (s, 1H), 7.97 (s, 1H), 7.94 (d, *J* = 5.9 Hz, 1H), 7.87 (s, 2H), 7.57 (d, *J* = 5.9 Hz, 1H), 4.81 (s, 1H), 4.61 (s, 1H), 4.49 (s, 2H), 3.81 (t, *J* = 6.7 Hz, 2H), 3.47 (s, 2H), 3.17 (s, 2H), 2.87 (s, 1H), 2.79 (s, 1H), 2.54 (s, 1H), 2.45 - 2.31 (m, 1H), 2.26 (s, 3H). LCMS (ESI) calcd for $C_{24}H_{25}N_6O_3S^+$ $[M+H]^+$: 477.17, found, 477.1.

**Example 356: preparation of 1-(1-oxo-5-((4-(thieno[2,3-d]pyrimidin-4-yl)-3,6-dihydropyridin-1(2H)-yl)methyl)isoindolin-2-yl)dihydropyrimidine-2,4(1H,3H)-dione (GT-08116)**

Referring to the method of Scheme 1, the target product (GT-08116) was prepared as a white solid (19 mg, yield 42%). $^1H$ NMR (400 MHz, DMSO-d6) δ 10.70 (s, 1H), 9.08 (s, 1H), 8.05 (d, *J* = 6.1 Hz, 1H), 8.00 (s, 1H), 7.90 (s, 2H), 7.78 (d, *J* = 6.1 Hz, 1H), 6.67 (s, 1H), 4.82 (s, 1H), 4.60 (d, *J* = 17.2 Hz, 3H), 3.98 (s, 2H), 3.89 - 3.86 (m, 1H), 3.81 (t, *J* = 6.8 Hz, 2H), 3.70 (d, *J* = 10.5 Hz, 1H), 3.34 (s, 1H), 3.10 (s, 1H), 3.04 (s, 1H), 2.87 (s, 1H), 2.79 (s, 1H). LCMS (ESI) calcd for $C_{24}H_{23}N_6O_3S^+$$[M+H]^+$: 475.15, found, 475.1.

**Example 357: preparation of 1-(5-((4-(5-methylthieno[2,3-d]pyrimidin-4-yl)-3,6-dihydropyridin-1(2H)-yl)methyl)-1-oxoisoindolin-2-yl)dihydropyrimidine-2,4(1H,3H)-dione (GT-08117)**

Referring to the method of Scheme 1, the target product (GT-08117) was prepared as a white solid (27 mg, yield 58%). $^1H$ NMR (400 MHz, DMSO-d6) δ 10.70 (s, 1H), 9.03 (s, 1H), 8.01 (s, 1H), 7.90 (t, *J* = 6.9 Hz, 2H), 7.65 (d, *J* = 1.1 Hz, 1H), 5.91 (s, 1H), 4.82 (s, 1H), 4.63 (d, *J* = 7.0 Hz, 3H), 3.83 - 3.78 (m, 4H), 3.69 (s, 1H), 3.37 (s, 1H), 3.06 (d, *J* = 24.2 Hz, 1H), 2.92 (d, *J* = 15.4 Hz, 1H), 2.88 (d, *J* = 8.2 Hz, 1H), 2.79 (s, 1H), 2.44 (s, 3H). LCMS (ESI) calcd for $C_{25}H_{25}N_6O_3S^+$ $[M+H]^+$: 489.17, found, 489.1.

**Example 358: preparation of 1-(5-((4-(6-methylthieno[2,3-d]pyrimidin-4-yl)-3,6-dihydropyridin-1(2H)-yl) methyl)-1-oxoisoindolin-2-yl)dihydropyrimidine-2,4(1H,3H)-dione (GT-08157)**

Referring to the method of Scheme 1, the target product (GT-08157) was prepared as a white solid (25 mg, yield 54%). $^1H$ NMR (400 MHz, DMSO-d6) δ 10.70 (s, 1H), 8.98 (s, 1H), 8.01 (s, 1H), 7.90 (s, 2H), 7.50 (s, 1H), 6.63 (s, 1H), 4.82 (s, 1H), 4.62 (s, 3H), 4.02 (s, 1H), 3.90 (s, 1H), 3.81 (s, 2H), 3.70 (d, *J* = 10.0 Hz, 1H), 3.44 (d, *J* = 7.0 Hz, 1H), 3.33 (s, 1H), 3.07 (s, 1H), 3.03 (s, 1H), 2.87 (s, 1H), 2.79 (s, 1H), 2.63 (s, 3H). LCMS (ESI) calcd for $C_{25}H_{25}N_6O_3S^+$ [M+H]$^+$: 489.17, found, 489.1.

**Example 359: preparation of 1-(5-((4-(6-isopropylthieno[2,3-d]pyrimidin-4-yl)-3,6-dihydropyridin-1(2H)-yl) methyl)-1-oxoisoindolin-2-yl)dihydropyrimidine-2,4(1H,3H)-dione (GT-08158)**

Referring to the method of Scheme 1, the target product (GT-08158) was prepared as a white solid (23 mg, yield 47%). $^1H$ NMR (400 MHz, DMSO-d6) δ 10.70 (s, 1H), 8.99 (s, 1H), 8.00 (s, 1H), 7.90 (s, 2H), 7.49 (s, 1H), 6.64 (s, 1H), 4.82 (s, 1H), 4.61 (dd, *J* = 15.7, 6.5 Hz, 3H), 3.95 (s, 2H), 3.83 - 3.79 (m, 3H), 3.71 (d, *J* = 11.1 Hz, 1H), 3.37 - 3.27 (m, 2H), 3.07 (s, 1H), 3.01 (d, *J* = 16.7 Hz, 1H), 2.87 (t, *J* = 7.8 Hz, 1H), 2.79 (t, *J* = 5.5 Hz, 1H), 1.36 (d, *J* = 6.8 Hz, 6H). LCMS (ESI) calcd for $C_{27}H_{29}N_6O_3S^+$ [M+H]$^+$: 517.20, found, 517.1.

**Example 360: preparation of 1-(5-((4-(5,6-dimethylthieno[2,3-d]pyrimidin-4-yl)-3,6-dihydropyridin-1(2H)-yl) methyl)-1-oxoisoindolin-2-yl)dihydropyrimidine-2,4(1H,3H)-dione (GT-08159)**

Referring to the method of Scheme 1, the target product (GT-08159) was prepared as a white solid (35 mg, yield 73%). $^1H$ NMR (400 MHz, DMSO-d6) δ 10.70 (s, 1H), 8.95 (s, 1H), 8.03 (s, 1H), 7.93 (s, 1H), 7.89 (d, *J* = 7.8 Hz, 1H), 5.85 (s, 1H), 4.82 (s, 1H), 4.62 (s, 3H), 4.33 (s, 2H), 4.27 - 4.19 (m, 1H), 3.91 (s, 1H), 3.85 - 3.77 (m, 3H), 3.68 (s, 1H), 3.40 (d, *J* = 15.2 Hz, 1H), 3.10 (s, 1H), 2.88 (d, *J* = 7.9 Hz, 2H), 2.78 (dd, *J* = 13.9, 8.0 Hz, 1H), 2.30 (s, 3H). LCMS (ESI) calcd for $C_{26}H_{27}N_6O_3S^+$ [M+H]$^+$: 503.19, found, 503.1.

**Example 361: preparation of 1-(1-oxo-5-((4-(6-phenylthieno[2,3-d]pyrimidin-4-yl)-3,6-dihydropyridin-1(2H)-yl) methyl)isoindolin-2-yl)dihydropyrimidine-2,4(1H,3H)-dione (GT-08173)**

Referring to the method of Scheme 1, the target product (GT-08173) was prepared as a white solid (29 mg, yield 56%). $^1H$ NMR (400 MHz, DMSO-d6) δ 10.71 (s, 1H), 9.06 (s, 1H), 8.14 (s, 1H), 7.98 (s, 1H), 7.93 - 7.88 (m, 3H), 7.54 (t, *J* = 7.3 Hz, 2H), 7.49 (d, *J* = 7.1 Hz, 1H), 6.79 (s, 1H), 4.85 (d, *J* = 16.4 Hz, 1H), 4.68 (d, *J* = 12.0 Hz, 1H), 4.63 (s, 2H), 3.98 (s, 2H), 3.81 (t, *J* = 6.9 Hz, 2H), 3.74 (s, 1H), 3.48 (s, 1H), 3.38 - 3.30 (m, 1H), 3.08 (s, 2H), 2.90 - 2.83 (m, 1H), 2.78 (d, *J* = 5.7 Hz, 1H). LCMS (ESI) calcd for $C_{30}H_{27}N_6O_3S^+$ [M+H]$^+$: 551.19, found, 551.1.

**Example 362: preparation of 1-(1-oxo-5-((4-(5-phenylthieno[2,3-d]pyrimidin-4-yl)-3,6-dihydropyridin-1(2H)-yl) methyl)isoindolin-2-yl)dihydropyrimidine-2,4(1H,3H)-dione (GT-08174)**

Referring to the method of Scheme 1, the target product (GT-08174) was prepared as a white solid (31 mg, yield 59%). $^1H$ NMR (400 MHz, DMSO-d6) δ 10.72 (s, 1H), 9.12 (s, 1H), 7.96 (s, 1H), 7.92 (d, *J* = 7.8 Hz, 1H), 7.88 (s, 1H), 7.80 (s, 1H), 7.32 (s, 3H), 7.06 (dt, *J* = 8.5, 4.2 Hz, 1H), 5.24 (s, 1H), 4.86 (s, 1H), 4.66 (s, 1H), 4.41 (s, 2H), 4.04 (s, 1H), 3.84 (t, *J* = 6.8 Hz, 2H), 3.56 (s, 1H), 3.23 (d, *J* = 16.7 Hz, 1H), 3.13 (s, 1H), 2.88 (s, 1H), 2.80 (s, 1H), 2.66 (d, *J* = 13.7 Hz, 2H). LCMS (ESI) calcd for $C_{30}H_{27}N_6O_3S^+$ [M+H]$^+$: 551.19, found, 551.1.

**Example 363: preparation of 1-(5-((4-(2-methyl-5-phenylthieno[2,3-d]pyrimidin-4-yl)-3,6-dihydropyridin-1(2H)-yl)methyl)-1-oxoisoindolin-2-yl)dihydropyrimidine-2,4(1H,3H)-dione (GT-08176)**

Referring to the method of Scheme 1, the target product (GT-08176) was prepared as a white solid (27 mg, yield 54%). $^1H$ NMR (400 MHz, DMSO) δ 10.72 (s, 1H), 7.92 (d, *J* = 7.8 Hz, 1H), 7.86 (s, 1H), 7.83 - 7.75 (m, 2H), 7.30 (s, 3H), 7.05 (d, *J* = 3.9 Hz, 1H), 5.25 (s, 1H), 4.85 (s, 1H), 4.64 (d, *J* = 9.8 Hz, 1H), 4.40 (s, 2H), 3.96 - 3.89 (m, 1H), 3.83 (t, *J* = 6.8 Hz, 2H), 3.50 (d, *J* = 24.9 Hz, 1H), 3.26 (d, *J* = 16.1 Hz, 1H), 3.08 (s, 1H), 2.88 (s, 1H), 2.81 (s, 1H), 2.75 (s, 2H), 2.68 (s, 2H), 2.54 (d, *J* = 9.7 Hz, 1H). LCMS (ESI) calcd for $C_{31}H_{29}N_6O_3S^+$ [M+H]$^+$: 565.20, found, 565.1.

**Example 364: preparation of 1-(5-((4-(2-methylthieno[2,3-d]pyrimidin-4-yl)-3,6-dihydropyridin-1(2H)-yl) methyl)-1-oxoisoindolin-2-yl)dihydropyrimidine-2,4(1H,3H)-dione (GT-08175)**

Referring to the method of Scheme 1, the target product (GT-08175) was prepared as a white solid (27 mg, yield

58%). $^1$H NMR (400 MHz, DMSO) δ 10.71 (s, 1H), 8.02 (s, 1H), 7.92 (s, 1H), 7.90 (d, *J* = 2.8 Hz, 2H), 7.70 (d, *J* = 6.2 Hz, 1H), 6.60 (s, 1H), 4.82 (s, 1H), 4.65 (d, *J* = 12.5 Hz, 1H), 4.59 (d, *J* = 17.7 Hz, 3H), 3.92 (d, *J* = 15.6 Hz, 2H), 3.81 (t, *J* = 6.8 Hz, 2H), 3.69 (d, *J* = 9.6 Hz, 1H), 3.33 (s, 1H), 3.09 (s, 1H), 3.02 (s, 1H), 2.85 (s, 1H), 2.79 (s, 1H), 2.72 (s, 3H). LCMS (ESI) calcd for $C_{25}H_{25}N_6O_3S^+$ [M+H]$^+$: 489.17, found, 489.1.

**Example 365: preparation of 1-(5-((4-(2-methyl-5,6,7,8-tetrahydrobenzo[4,5]thieno[2,3-d]pyrimidin-4-yl)-3,6-dihydropyridin-1(2H)-yl)methyl)-1-oxoisoindolin-2-yl)dihydropyrimidine-2,4(1H,3H)-dione (GT-08177)**

Referring to the method of Scheme 1, the target product (GT-08177) was prepared as a white solid (19 mg, yield 37%). $^1$H NMR (400 MHz, DMSO) δ 10.70 (s, 1H), 8.02 (s, 1H), 7.92 (s, 1H), 7.89 (d, *J* = 7.8 Hz, 1H), 5.86 (s, 1H), 4.82 (s, 1H), 4.66 (d, *J* = 11.5 Hz, 1H), 4.62 (s, 2H), 4.43 (s, 2H), 3.88 (s, 1H), 3.80 (t, *J* = 6.8 Hz, 3H), 3.65 (s, 1H), 3.37 (s, 1H), 3.03 (s, 1H), 2.87 (s, 4H), 2.79 (d, *J* = 5.0 Hz, 1H), 2.70 (s, 3H), 2.58 (d, *J* = 17.2 Hz, 1H), 1.79 (s, 4H). LCMS (ESI) calcd for $C_{29}H_{31}N_6O_3S^+$ [M+H]$^+$: 543.22, found, 543.1.

**Example 366: preparation of 1-(1-oxo-5-((4-(5,6,7,8-tetrahydrobenzo[4,5]thieno[2,3-d]pyrimidin-4-yl)-3,6-dihydropyridin-1(2H)-yl)methyl)isoindolin-2-yl)dihydropyrimidine-2,4(1H,3H)-dione (GT-08178)**

Referring to the method of Scheme 1, the target product (GT-08178) was prepared as a white solid (24 mg, yield 48%). $^1$H NMR (400 MHz, DMSO) δ 10.70 (s, 1H), 8.95 (s, 1H), 8.01 (s, 1H), 7.92 (s, 1H), 7.89 (d, *J* = 7.8 Hz, 1H), 5.87 (s, 1H), 4.82 (s, 1H), 4.67 - 4.56 (m, 3H), 4.33 (s, 1H), 3.90 (s, 1H), 3.81 (t, *J*= 6.8 Hz, 3H), 3.67 (s, 1H), 3.37 (s, 1H), 3.08 (s, 1H), 2.90 (d, *J* = 7.0 Hz, 3H), 2.87 (s, 1H), 2.80 (dd, *J* = 12.5, 6.3 Hz, 1H), 2.63 (s, 1H), 1.84 (d, *J* = 12.2 Hz, 4H). LCMS (ESI) calcd for $C_{28}H_{29}N_6O_3S^+$ [M+H]$^+$: 529.20, found, 529.1.

**Example 367: preparation of 5-((4-(4-chloro-3-fluoropyridin-2-yl)piperazin-1-yl)methyl)-2-(2,4-dioxotetrahydropyrimidin-1(2H)-yl)isoindoline-1,3-dione (GT-08190)**

Referring to the method of Scheme 1, the target product (GT-08190) was prepared as a white solid (26 mg, yield 58%). $^1$H NMR (400 MHz, DMSO) δ 10.93 (s, 1H), 8.36 (s, 1H), 8.22 (d, *J* = 7.7 Hz, 1H), 8.11 (d, *J* = 7.7 Hz, 1H), 8.02 (d, *J* = 5.3 Hz, 1H), 7.19 (t, *J* = 4.9 Hz, 1H), 4.60 (s, 2H), 4.09 (d, *J* = 13.8 Hz, 2H), 3.83 (t, *J* = 6.8 Hz, 2H), 3.49 (t, *J* = 12.3 Hz, 2H), 3.39 (s, 2H), 3.22 (s, 2H), 2.86 (t, *J* = 6.8 Hz, 2H). LCMS (ESI) calcd for $C_{22}H_{21}ClFN_6O_4^+$ [M+H]$^+$: 487.13, found, 487.1.

**Example 368: preparation of 2-(2,4-dioxotetrahydropyrimidin-1(2H)-yl)-5-((4-(5,6,7,8-tetrahydrobenzo[4,5]thieno[2,3-d]pyrimidin-4-yl)piperidin-1-yl)methyl)isoindoline-1,3-dione (GT-08424)**

Referring to the method of Scheme 1, the target product (GT-08424) was prepared as a white solid (16 mg, yield 32%). $^1$H NMR (400 MHz, DMSO) δ 10.93 (s, 1H), 8.90 (s, 1H), 8.36 (s, 1H), 8.22 (dd, *J* = 7.8, 1.2 Hz, 1H), 8.12 (d, *J* = 7.7 Hz, 1H), 4.57 (d, *J* = 3.9 Hz, 2H), 3.83 (t, *J* = 6.8 Hz, 3H), 3.70 - 3.63 (m, 1H), 3.47 (d, *J* = 11.5 Hz, 2H), 3.23 (dd, *J* = 22.1, 10.7 Hz, 2H), 3.02 (s, 2H), 2.86 (t, *J* = 6.8 Hz, 4H), 2.33 (dd, *J* = 13.5, 11.8 Hz, 2H), 2.00 (d, *J* = 12.9 Hz, 2H), 1.87 (s, 4H). LCMS (ESI) calcd for $C_{28}H_{29}N_6O_4S^+$ [M+H]$^+$: 545.20, found, 545.1.

**Example 369: preparation of 5-((4-(6,7-dihydro-5H-cyclopenta[4,5]thieno[2,3-d]pyrimidin-4-yl)piperidin-1-yl)methyl)-2-(2,4-dioxotetrahydropyrimidin-1(2H)-yl)isoindoline-1,3-dione (GT-08425)**

Referring to the method of Scheme 1, the target product (GT-08425) was prepared as a white solid (25 mg, yield 52%). $^1$H NMR (400 MHz, DMSO) δ 10.93 (s, 1H), 8.91 (s, 1H), 8.35 (s, 1H), 8.19 (s, 1H), 8.12 (d, *J* = 7.7 Hz, 1H), 4.58 (s, 2H), 3.83 (t, *J* = 6.8 Hz, 2H), 3.57 (s, 2H), 3.49 (s, 2H), 3.42 (dd, *J* = 16.9, 9.9 Hz, 1H), 3.23 (dd, *J* = 23.1, 11.1 Hz, 2H), 3.12 (d, *J* = 7.0 Hz, 2H), 3.06 (t, *J* = 7.2 Hz, 2H), 2.86 (t, *J* = 6.7 Hz, 2H), 2.35 - 2.26 (m, 2H), 2.03 (s, 2H). LCMS (ESI) calcd for $C_{27}H_{27}N_6O_4S^+$ [M+H]$^+$: 531.18, found, 531.1.

**Example 370: preparation of 2-(2,4-dioxotetrahydropyrimidin-1(2H)-yl)-5-((4-(2-methyl-6,7-dihydro-5H-cyclopenta[4,5]thieno[2,3-d]pyrimidin-4-yl)piperidin-1-yl)methyl)isoindoline-1,3-dione (GT-08426)**

Referring to the method of Scheme 1, the target product (GT-08426) was prepared as a white solid (21 mg, yield 43%). $^1$H NMR (400 MHz, DMSO) δ 10.93 (s, 2H), 8.34 (s, 1H), 8.18 (s, 1H), 8.13 (d, *J* = 7.7 Hz, 1H), 4.57 (s, 2H), 3.83 (t, *J* = 6.7 Hz, 2H), 3.67 (s, 1H), 3.48 (s, 3H), 3.41 (d, *J* = 18.1 Hz, 1H), 3.23 (dd, *J* = 22.8, 10.4 Hz, 2H), 3.10 (s, 2H), 3.02 (s, 2H), 2.86 (t, *J* = 6.7 Hz, 2H), 2.66 (s, 3H), 2.47 (s, 2H), 2.27 (d, *J* = 12.7 Hz, 2H), 2.01 (s, 2H). LCMS (ESI) calcd for $C_{28}H_{29}N_6O_4S^+$ [M+H]$^+$: 545.20, found, 545.1.

**Example 371: preparation of 2-(2,4-dioxotetrahydropyrimidin-1(2H)-yl)-5-((4-(thieno[2,3-d]pyrimidin-2-yl)piperidin-1-yl)methyl)isoindoline-1,3-dione (GT-08427)**

Referring to the method of Scheme 1, the target product (GT-08427) was prepared as a white solid (20 mg, yield 45%). $^1H$ NMR (400 MHz, DMSO) δ 10.94 (s, 1H), 10.90 (s, 1H), 9.31 (s, 1H), 8.39 (s, 1H), 8.25 (d, *J* = 7.8 Hz, 1H), 8.11 (d, *J* = 7.7 Hz, 1H), 7.93 (d, *J* = 6.1 Hz, 2H), 7.91 (s, 1H), 7.87 (d, *J* = 7.8 Hz, 1H), 7.56 (d, *J* = 5.9 Hz, 1H), 4.70 (s, 2H), 4.58 (s, 2H), 3.83 (d, *J* = 5.2 Hz, 3H), 3.48 (s, 2H), 3.18 (s, 1H), 2.86 (d, *J* = 6.6 Hz, 3H), 2.26 (s, 2H). LCMS (ESI) calcd for $C_{24}H_{23}N_6O_4S^+$ $[M+H]^+$: 491.15, found, 491.1.

**Example 372: preparation of 2-(2,4-dioxotetrahydropyrimidin-1(2H)-yl)-5-((4-(5-methylthieno[2,3-d]pyrimidin-4-yl)-3,6-dihydropyridin-1(2H)-yl)methyl)isoindoline-1,3-dione (GT-08428)**

Referring to the method of Scheme 1, the target product (GT-08428) was prepared as a white solid (25 mg, yield 55%). $^1H$ NMR (400 MHz, DMSO) δ 10.94 (s, 1H), 9.03 (s, 1H), 8.45 (d, *J* = 7.1 Hz, 1H), 8.32 (d, *J* = 8.5 Hz, 1H), 8.13 (d, *J* = 7.7 Hz, 1H), 7.65 (d, *J* = 1.0 Hz, 1H), 5.92 (s, 1H), 4.80 - 4.65 (m, 2H), 3.97 (s, 1H), 3.84 (t, *J* = 6.7 Hz, 3H), 3.71 (s, 1H), 3.39 (s, 1H), 3.09 (d, *J* = 17.8 Hz, 1H), 2.96 (s, 1H), 2.87 (t, *J* = 6.8 Hz, 2H), 2.44 (s, 3H). LCMS (ESI) calcd for $C_{25}H_{23}N_6O_4S^+$ $[M+H]^+$: 503.15, found, 503.1.

**Example 373: preparation of 2-(2,4-dioxotetrahydropyrimidin-1(2H)-yl)-5-((4-(6-isopropylthieno[2,3-d]pyrimidin-4-yl)-3,6-dihydropyridin-1(2H)-yl)methyl)isoindoline-1,3-dione (GT-08429)**

Referring to the method of Scheme 1, the target product (GT-08429) was prepared as a white solid (26 mg, yield 54%). $^1H$ NMR (400 MHz, DMSO) δ 10.94 (s, 1H), 8.99 (s, 1H), 8.42 (d, *J* = 11.3 Hz, 1H), 8.29 (dd, *J* = 11.6, 7.9 Hz, 1H), 8.14 (d, *J* = 7.7 Hz, 1H), 7.48 (s, 1H), 6.64 (s, 1H), 4.72 (d, *J* = 14.7 Hz, 2H), 3.94 - 3.90 (m, 2H), 3.84 (t, *J* = 6.8 Hz, 3H), 3.72 (s, 1H), 3.38 - 3.26 (m, 2H), 3.03 (t, *J* = 17.1 Hz, 2H), 2.87 (t, *J* = 6.8 Hz, 2H), 1.36 (d, *J* = 6.8 Hz, 6H). LCMS (ESI) calcd for $C_{27}H_{27}N_6O_4S^+$ $[M+H]^+$: 531.18, found, 531.1.

**Example 374: preparation of 2-(2,4-dioxotetrahydropyrimidin-1(2H)-yl)-5-((4-(6-phenylthieno[2,3-d]pyrimidin-4-yl)-3,6-dihydropyridin-1(2H)-yl)methyl)isoindoline-1,3-dione (GT-08430)**

Referring to the method of Scheme 1, the target product (GT-08430) was prepared as a white solid (27 mg, yield 53%). $^1H$ NMR (400 MHz, DMSO) δ 10.94 (s, 1H), 9.06 (d, *J* = 0.7 Hz, 1H), 8.40 (s, 1H), 8.32 - 8.23 (m, 1H), 8.16 (d, *J* = 10.8 Hz, 2H), 7.90 (d, *J* = 7.3 Hz, 2H), 7.54 (t, *J* = 7.2 Hz, 2H), 7.49 (d, *J* = 7.0 Hz, 1H), 6.79 (s, 1H), 4.74 (d, *J* = 17.1 Hz, 2H), 4.00 (d, *J* = 15.5 Hz, 2H), 3.84 (t, *J* = 6.7 Hz, 3H), 3.77 (s, 1H), 3.36 (s, 1H), 3.08 (s, 2H), 2.87 (t, *J* = 6.7 Hz, 2H). LCMS (ESI) calcd for $C_{30}H_{25}N_6O_4S^+$ $[M+H]^+$: 565.17, found, 565.1.

**Example 375: preparation of 2-(2,4-dioxotetrahydropyrimidin-1(2H)-yl)-5-((4-(5-phenylthieno[2,3-d]pyrimidin-4-yl)-3,6-dihydropyridin-1(2H)-yl)methyl)isoindoline-1,3-dione (GT-08431)**

Referring to the method of Scheme 1, the target product (GT-08431) was prepared as a white solid (29 mg, yield 57%). $^1H$ NMR (400 MHz, DMSO) δ 10.96 (s, 1H), 9.12 (s, 1H), 8.34 (d, *J* = 8.3 Hz, 1H), 8.21 (d, *J* = 5.1 Hz, 1H), 8.16 (d, *J* = 7.7 Hz, 1H), 7.97 (s, 1H), 7.36 (d, *J* = 21.8 Hz, 4H), 7.23 - 7.13 (m, 1H), 5.24 (s, 1H), 4.50 (s, 2H), 4.34 - 4.30 (m, 1H), 3.86 (t, *J* = 6.8 Hz, 2H), 3.58 (s, 1H), 3.33 - 3.20 (m, 1H), 3.15 (d, *J* = 20.8 Hz, 1H), 2.88 (t, *J* = 6.7 Hz, 2H), 2.70 (d, *J* = 20.4 Hz, 2H). LCMS (ESI) calcd for $C_{30}H_{25}N_6O_4S^+$ $[M+H]^+$: 565.17, found, 565.1.

**Example 376: preparation of 2-(2,4-dioxotetrahydropyrimidin-1(2H)-yl)-5-((4-(2-methyl-5-phenylthieno[2,3-d]pyrimidin-4-yl)-3,6-dihydropyridin-1(2H)-yl)methyl)isoindoline-1,3-dione (GT-08484)**

Referring to the method of Scheme 1, the target product (GT-08484) was prepared as a white solid (29 mg, yield 55%). $^1H$ NMR (400 MHz, DMSO) δ 10.96 (s, 1H), 8.34 (d, *J* = 6.5 Hz, 1H), 8.21 (s, 1H), 8.15 (d, *J* = 7.7 Hz, 1H), 7.83 (s, 1H), 7.39 - 7.29 (m, 4H), 7.16 (d, *J* = 7.4 Hz, 1H), 5.25 (s, 1H), 4.49 - 4.41 (m, 2H), 3.86 (t, *J* = 6.7 Hz, 2H), 3.49 (dd, *J* = 22.7, 9.9 Hz, 1H), 3.24 (dd, *J* = 29.6, 16.1 Hz, 1H), 3.07 (s, 1H), 2.88 (t, *J* = 6.7 Hz, 2H), 2.77 (d, *J* = 7.4 Hz, 4H), 2.66 (d, *J* = 22.4 Hz, 2H). LCMS (ESI) calcd for $C_{31}H_{27}N_6O_4S^+$ $[M+H]^+$: 579.18, found, 579.1.

**Example 377: preparation of 2-(2,4-dioxotetrahydropyrimidin-1(2H)-yl)-5-((4-(2-methyl-5,6,7,8-tetrahydrobenzo[4,5]thieno[2,3-d]pyrimidin-4-yl)-3,6-dihydropyridin-1(2H)-yl)methyl)isoindoline-1,3-dione (GT-08432)**

Referring to the method of Scheme 1, the target product (GT-08432) was prepared as a white solid (22 mg, yield

44%). $^1H$ NMR (400 MHz, DMSO) δ 10.94 (s, 1H), 8.44 (d, *J* = 7.7 Hz, 1H), 8.32 (s, 1H), 8.13 (d, *J* = 7.7 Hz, 1H), 5.88 (s, 1H), 4.81 - 4.69 (m, 3H), 4.66 (s, 1H), 3.91 (s, 1H), 3.84 (t, *J* = 6.6 Hz, 3H), 3.67 (s, 1H), 3.45 - 3.30 (m, 1H), 3.02 (s, 1H), 2.87 (t, *J* = 6.6 Hz, 4H), 2.71 (s, 3H), 2.59 (d, *J* = 16.9 Hz, 1H), 1.80 (d, *J* = 9.7 Hz, 4H). LCMS (ESI) calcd for $C_{29}H_{29}N_6O_4S^+$ [M+H]$^+$: 557.20, found, 557.1.

**Example 378: preparation of 2-(2,4-dioxotetrahydropyrimidin-1(2H)-yl)-5-((4-(5,6,7,8-tetrahydrobenzo[4,5]thieno[2,3-d]pyrimidin-4-yl)-3,6-dihydropyridin-1(2H)-yl)methyl)isoindoline-1,3-dione (GT-08433)**

Referring to the method of Scheme 1, the target product (GT-08433) was prepared as a white solid (31 mg, yield 63%). $^1H$ NMR (400 MHz, DMSO) δ 10.94 (s, 1H), 8.95 (s, 1H), 8.44 (d, *J* = 7.8 Hz, 1H), 8.32 (s, 1H), 8.13 (d, *J* = 7.7 Hz, 1H), 5.87 (s, 1H), 4.73 (d, *J* = 9.0 Hz, 3H), 4.64 (s, 2H), 3.94 (s, 1H), 3.84 (t, *J* = 6.7 Hz, 3H), 3.68 (s, 1H), 3.38 (s, 1H), 3.08 (s, 1H), 2.91 (s, 2H), 2.87 (t, *J* = 6.8 Hz, 4H), 2.78 (dd, *J* = 19.2, 12.1 Hz, 1H), 2.61 (d, *J* = 16.8 Hz, 1H). LCMS (ESI) calcd for $C_{28}H_{27}N_6O_4S^+$ [M+H]$^+$: 543.18, found, 543.1.

**Example 379: preparation of 5-((4-(6,7-dihydro-5H-cyclopenta[4,5]thieno[2,3-d]pyrimidin-4-yl)-3,6-dihydropyridin-1(2H)-yl)methyl)-2-(2,4-dioxotetrahydropyrimidin-1(2H)-yl)isoindoline-1,3-dione (GT-08434)**

Referring to the method of Scheme 1, the target product (GT-08434) was prepared as a white solid (28 mg, yield 58%). $^1H$ NMR (400 MHz, DMSO) δ 10.94 (s, 1H), 8.95 (s, 1H), 8.44 (d, *J* = 7.0 Hz, 1H), 8.33 (s, 1H), 8.13 (d, *J* = 7.7 Hz, 1H), 6.10 (s, 1H), 4.72 (d, *J* = 10.8 Hz, 3H), 3.97 (s, 1H), 3.84 (t, *J* = 6.7 Hz, 3H), 3.69 (s, 1H), 3.35 (s, 1H), 3.05 (t, *J* = 6.7 Hz, 4H), 2.87 (t, *J* = 6.7 Hz, 3H), 2.37 (d, *J* = 7.2 Hz, 2H). LCMS (ESI) calcd for $C_{27}H_{25}N_6O_4S^+$ [M+H]$^+$: 529.17, found, 529.1.

**Example 380: preparation of 2-(2,4-dioxotetrahydropyrimidin-1(2H)-yl)-5-((4-(2-methyl-6,7-dihydro-5H-cyclopenta[4,5]thieno[2,3-d]pyrimidin-4-yl)-3,6-dihydropyridin-1(2H)-yl)methyl)isoindoline-1,3-dione (GT-08435)**

Referring to the method of Scheme 1, the target product (GT-08435) was prepared as a white solid (20 mg, yield 41%). $^1H$ NMR (400 MHz, DMSO) δ 10.93 (s, 1H), 8.41 (d, *J* = 5.7 Hz, 1H), 8.29 (s, 1H), 8.13 (d, *J* = 7.7 Hz, 1H), 6.05 (s, 1H), 4.72 (s, 3H), 3.94 (s, 1H), 3.84 (t, *J* = 6.6 Hz, 3H), 3.69 (s, 1H), 3.33 (s, 1H), 3.06 - 2.97 (m, 5H), 2.86 (t, *J* = 6.7 Hz, 3H), 2.69 (s, 3H), 2.36 (d, *J* = 6.7 Hz, 2H). LCMS (ESI) calcd for $C_{28}H_{27}N_6O_4S^+$ [M+H]$^+$: 543.18, found, 543.1.

**Example 381: preparation of 1-(5-((4-(6,7-dihydro-5H-cyclopenta[4,5]thieno[2,3-d]pyrimidin-4-yl)-3,6-dihydropyridin-1(2H)-yl)methyl)-1-oxoisoindolin-2-yl)dihydropyrimidine-2,4(1H,3H)-dione (GT-08436)**

Referring to the method of Scheme 1, the target product (GT-08436) was prepared as a white solid (24 mg, yield 49%). $^1H$ NMR (400 MHz, DMSO) δ 10.70 (s, 1H), 8.95 (s, 1H), 8.01 (s, 1H), 7.92 (s, 1H), 7.89 (d, *J* = 7.8 Hz, 1H), 6.09 (s, 1H), 4.84 (d, *J* = 16.4 Hz, 1H), 4.62 (s, 3H), 3.93 (s, 1H), 3.81 (t, *J* = 6.8 Hz, 3H), 3.68 (s, 1H), 3.34 (s, 1H), 3.05 (t, *J* = 6.8 Hz, 4H), 2.97 (d, *J* = 17.8 Hz, 1H), 2.88 (d, *J* = 7.9 Hz, 2H), 2.79 (t, *J* = 5.7 Hz, 1H), 2.38 (d, *J* = 7.4 Hz, 2H). LCMS (ESI) calcd for $C_{27}H_{27}N_6O_3S^+$ [M+H]$^+$: 515.19, found, 515.1.

**Example 382: preparation of 1-(5-((4-(2-methyl-6,7-dihydro-5H-cyclopenta[4,5]thieno[2,3-d]pyrimidin-4-yl)-3,6-dihydropyridin-1(2H)-yl)methyl)-1-oxoisoindolin-2-yl)dihydropyrimidine-2,4(1H,3H)-dione (GT-08437)**

Referring to the method of Scheme 1, the target product (GT-08437) was prepared as a white solid (15 mg, yield 30%). $^1H$ NMR (400 MHz, DMSO) δ 10.70 (s, 1H), 7.99 (s, 1H), 7.90 (s, 2H), 6.05 (s, 1H), 4.84 (d, *J* = 16.8 Hz, 1H), 4.64 - 4.55 (m, 3H), 3.83 - 3.78 (m, 4H), 3.68 (s, 2H), 3.33 (s, 1H), 3.04 - 2.98 (m, 3H), 2.96 (s, 1H), 2.85 (d, *J* = 7.7 Hz, 2H), 2.81 - 2.76 (m, 1H), 2.68 (s, 3H), 2.37 (d, *J* = 7.3 Hz, 2H). LCMS (ESI) calcd for $C_{28}H_{29}N_6O_3S^+$ [M+H]$^+$: 529.20, found, 529.1.

**Example 383: preparation of 1-(1-oxo-5-((4-(thieno[2,3-d]pyrimidin-2-yl)-3,6-dihydropyridin-1(2H)-yl)methyl)isoindolin-2-yl)dihydropyrimidine-2,4(1H,3H)-dione (GT-08438)**

Referring to the method of Scheme 1, the target product (GT-08438) was prepared as a white solid (31 mg, yield 68%). $^1H$ NMR (400 MHz, DMSO) δ 10.71 (s, 1H), 9.33 (s, 1H), 8.01 - 7.94 (m, 2H), 7.89 (s, 2H), 7.59 (d, *J* = 5.9 Hz, 1H), 7.20 (s, 1H), 4.81 (s, 1H), 4.64 - 4.53 (m, 3H), 3.93 (s, 2H), 3.80 (dd, *J* = 13.9, 7.1 Hz, 3H), 3.72 (s, 1H), 3.29 (s, 1H), 3.06 (s, 2H), 2.87 (s, 1H), 2.79 (s, 1H). LCMS (ESI) calcd for $C_{24}H_{23}N_6O_3S^+$ [M+H]$^+$: 475.15, found, 475.1.

**Example 384: preparation of 1-(5-((4-(2-chloro-6,7-dihydrothieno[3,2-d]pyrimidin-4-yl)piperazin-1-yl)methyl)-1-oxoisoindolin-2-yl)dihydropyrimidine-2,4(1H,3H)-dione (GT-08439)**

Referring to the method of Scheme 1, the target product (GT-08439) was prepared as a white solid (29 mg, yield 59%). $^1$H NMR (400 MHz, DMSO) δ 10.69 (s, 1H), 7.89 (s, 1H), 7.87 (d, *J* = 7.8 Hz, 1H), 7.79 (d, *J* = 7.7 Hz, 1H), 4.81 (d, *J* = 16.6 Hz, 1H), 4.58 (d, *J* = 16.1 Hz, 1H), 4.47 (s, 2H), 4.40 (d, *J* = 12.9 Hz, 2H), 3.79 (t, *J* = 6.8 Hz, 3H), 3.54 (s, 2H), 3.41 (s, 1H), 3.36 (d, *J* = 8.0 Hz, 2H), 3.22 (t, *J* = 8.3 Hz, 2H), 3.16 (s, 2H), 2.86 (t, *J* = 7.8 Hz, 1H), 2.77 (d, *J* = 5.6 Hz, 1H). LCMS (ESI) calcd for $C_{23}H_{25}ClN_7O_3S^+$ [M+H]$^+$: 514.14, found, 514.1.

**Example 385: preparation of 1-(5-((4-(2-morpholino-6,7-dihydrothieno[3,2-d]pyrimidin-4-yl)piperazin-1-yl) methyl)-1-oxoisoindolin-2-yl)dihydropyrimidine-2,4(1H,3H)-dione (GT-08441)**

Referring to the method of Scheme 1, the target product (GT-08441) was prepared as a white solid (15 mg, yield 28%). $^1$H NMR (400 MHz, DMSO) δ 10.70 (s, 1H), 7.93 (s, 1H), 7.85 (t, *J* = 5.8 Hz, 2H), 4.79 (s, 2H), 4.64 (s, 1H), 4.57 (d, *J* = 16.4 Hz, 3H), 4.50 (s, 3H), 3.80 (d, *J* = 6.5 Hz, 2H), 3.76 (d, *J* = 9.2 Hz, 5H), 3.41 (d, *J* = 14.5 Hz, 2H), 3.37 (d, *J* = 6.2 Hz, 2H), 3.33 (d, *J* = 6.2 Hz, 2H), 3.16 (s, 2H), 2.86 (s, 1H), 2.78 (s, 1H). LCMS (ESI) calcd for $C_{27}H_{33}N_8O_4S^+$ [M+H]$^+$: 565.23, found, 565.2.

**Example 386: preparation of 1-(5-((4-(4-morpholino-6,7-dihydrothieno[3,2-d]pyrimidin-2-yl)piperazin-1-yl) methyl)-1-oxoisoindolin-2-yl)dihydropyrimidine-2,4(1H,3H)-dione (GT-08442)**

Referring to the method of Scheme 1, the target product (GT-08442) was prepared as a white solid (26 mg, yield 49%). $^1$H NMR (400 MHz, DMSO) δ 10.69 (s, 1H), 7.93 (s, 1H), 7.85 (s, 1H), 7.83 (s, 1H), 4.79 (s, 1H), 4.64 (s, 2H), 4.59 (s, 1H), 4.49 (s, 2H), 3.79 (s, 3H), 3.74 (s, 3H), 3.67 (s, 3H), 3.54 (s, 3H), 3.38 (s, 1H), 3.37 - 3.31 (m, 3H), 3.26 (d, *J* = 7.3 Hz, 2H), 3.11 (s, 2H), 2.86 (s, 1H), 2.78 (s, 1H). LCMS (ESI) calcd for $C_{27}H_{33}N_8O_4S^+$ [M+H]$^+$: 565.23, found, 565.2.

**Example 387: preparation of 1-(1-oxo-5-((4-(thieno[3,2-d]pyrimidin-4-yl)piperazin-1-yl)methyl)isoindolin-2-yl) dihydropyrimidine-2,4(1H,3H)-dione (GT-08443)**

Referring to the method of Scheme 1, the target product (GT-08443) was prepared as a white solid (21 mg, yield 46%). $^1$H NMR (400 MHz, DMSO) δ 10.70 (s, 1H), 8.88 (s, 1H), 8.58 (d, *J* = 5.5 Hz, 1H), 7.94 (s, 1H), 7.88 (d, *J* = 7.8 Hz, 1H), 7.84 (s, 1H), 7.65 (d, *J* = 5.5 Hz, 1H), 4.80 (s, 1H), 4.60 (s, 1H), 4.53 (s, 2H), 3.82 - 3.77 (m, 3H), 3.50 (d, *J* = 37.1 Hz, 4H), 3.36 (s, 3H), 2.86 (s, 1H), 2.78 (s, 1H). LCMS (ESI) calcd for $C_{23}H_{24}N_7O_3S^+$[M+H]$^+$: 478.17, found, 478.1.

**Example 388: preparation of 1-(5-((4-(2-chlorothieno[3,2-d]pyrimidin-4-yl)piperazin-1-yl)methyl)-1-oxoisoindolin-2-yl)dihydropyrimidine-2,4(1H,3H)-dione (GT-08444)**

Referring to the method of Scheme 1, the target product (GT-08444) was prepared as a white solid (31 mg, yield 64%). $^1$H NMR (400 MHz, DMSO) δ 10.70 (s, 1H), 8.38 (d, *J* = 5.5 Hz, 1H), 7.93 (s, 1H), 7.87 (d, *J = 7.8* Hz, 1H), 7.82 (d, *J* = 7.9 Hz, 1H), 7.48 - 7.45 (m, 1H), 4.80 (s, 1H), 4.73 (d, *J* = 16.5 Hz, 2H), 4.58 (d, *J* = 16.5 Hz, 1H), 4.50 (s, 2H), 3.81 (d, *J* = 6.8 Hz, 4H), 3.50 (s, 2H), 3.27 (s, 2H), 2.86 (s, 1H), 2.79 (s, 1H). LCMS (ESI) calcd for $C_{23}H_{23}ClN_7O_3S^+$ [M+H]$^+$: 512.13, found, 512.1.

**Example 389: preparation of 1-(5-((4-(2-morpholinothieno[3,2-d]pyrimidin-4-yl)piperazin-1-yl)methyl)-1-oxoisoindolin-2-yl)dihydropyrimidine-2,4(1H,3H)-dione (GT-08446)**

Referring to the method of Scheme 1, the target product (GT-08446) was prepared as a white solid (36 mg, yield 68%). $^1$H NMR (400 MHz, DMSO) δ 10.70 (s, 1H), 8.37 (d, *J* = 5.4 Hz, 1H), 7.93 (s, 1H), 7.87 (s, 1H), 7.84 (s, 1H), 7.62 (d, *J* = 4.5 Hz, 1H), 4.82 (d, *J* = 16.6 Hz, 2H), 4.57 (d, *J* = 17.0 Hz, 1H), 4.52 (s, 2H), 3.86 (s, 5H), 3.80 (t, *J* = 6.8 Hz, 3H), 3.74 - 3.70 (m, 4H), 3.53 (s, 2H), 3.32 - 3.18 (m, 3H), 2.86 (t, *J* = 7.8 Hz, 1H), 2.78 (t, *J* = 5.6 Hz, 1H).LCMS (ESI) calcd for $C_{27}H_{31}N_8O_4S^+$ [M+H]$^+$: 563.22, found, 563.2.

**Example 390: preparation of 1-(5-((4-(4-morpholinothieno[3,2-d]pyrimidin-2-yl)piperazin-1-yl)methyl)-1-oxoisoindolin-2-yl)dihydropyrimidine-2,4(1H,3H)-dione (GT-08447)**

Referring to the method of Scheme 1, the target product (GT-08447) was prepared as a white solid (27 mg, yield 51%). $^1$H NMR (400 MHz, DMSO) δ 10.69 (s, 1H), 8.32 (d, *J* = 5.1 Hz, 1H), 7.91 (s, 1H), 7.86 (s, 1H), 7.82 (s, 1H), 7.54 (s, 1H), 4.80 (s, 1H), 4.60 (s, 1H), 4.50 (s, 2H), 3.98 (s, 3H), 3.80 - 3.75 (m, 7H), 3.60 (d, *J* = 4.3 Hz, 4H), 3.18 (s, 4H), 2.86 (s,

1H), 2.78 (s, 1H). LCMS (ESI) calcd for $C_{27}H_{31}N_8O_4S^*$ [M+H]$^+$: 563.22, found, 563.2.

**Example 391: preparation of 5-((4-(2-chloro-6,7-dihydrothieno[3,2-d]pyrimidin-4-yl)piperazin-1-yl) methyl)-2-(2,4-dioxotetrahydropyrimidin-1(2H)-yl)isoindoline-1,3-dione (GT-08448)**

Referring to the method of Scheme 1, the target product (GT-08448) was prepared as a white solid (26 mg, yield 54%). $^1$H NMR (400 MHz, DMSO) δ 10.93 (s, 1H), 8.32 (s, 1H), 8.17 (d, *J* = 7.7 Hz, 1H), 8.10 (d, *J* = 7.7 Hz, 1H), 4.57 (s, 2H), 4.39 (s, 2H), 3.83 (t, *J* = 6.7 Hz, 2H), 3.65 (d, *J* = 13.9 Hz, 1H), 3.49 - 3.46 (m, 1H), 3.44 (d, *J* = 7.0 Hz, 1H), 3.38 (t, *J* = 8.3 Hz, 3H), 3.22 (t, *J* = 8.3 Hz, 3H), 3.17 - 3.10 (m, 1H), 2.86 (t, *J* = 6.7 Hz, 2H). LCMS (ESI) calcd for $C_{23}H_{23}ClN_7O_4S^+$ [M+H]$^+$: 528.12, found, 528.1.

**Example 392: preparation of 2-(2,4-dioxotetrahydropyrimidin-1(2H)-yl)-5-((4-(2-morpholino-6,7-dihydrothieno [3,2-d]pyrimidin-4-yl)piperazin-1-yl)methyl)isoindoline-1,3-dione (GT-08450)**

Referring to the method of Scheme 1, the target product (GT-08450) was prepared as a white solid (20 mg, yield 38%). $^1$H NMR (400 MHz, DMSO) δ 10.93 (s, 1H), 8.33 (s, 1H), 8.20 (d, *J* = 7.8 Hz, 1H), 8.10 (d, *J* = 7.7 Hz, 1H), 4.58 (s, 2H), 4.48 (s, 2H), 3.82 (dd, *J* = 8.4, 5.1 Hz, 4H), 3.70 (d, *J* = 4.5 Hz, 4H), 3.66 (d, *J* = 4.3 Hz, 4H), 3.42 (s, 2H), 3.33 (d, *J* = 7.2 Hz, 2H), 3.26 (d, *J* = 7.4 Hz, 2H), 3.17 (s, 2H), 2.86 (s, 2H). LCMS (ESI) calcd for $C_{27}H_{31}N_8O_5S^+$ [M+H]$^+$: 579.21, found, 579.1.

**Example 393: preparation of 2-(2,4-dioxotetrahydropyrimidin-1(2H)-yl)-5-((4-(4-morpholino-6,7-dihydrothieno [3,2-d]pyrimidin-2-yl)piperazin-1-yl)methyl)isoindoline-1,3-dione (GT-08451)**

Referring to the method of Scheme 1, the target product (GT-08451) was prepared as a white solid (32 mg, yield 61%). $^1$H NMR (400 MHz, DMSO) δ 10.93 (s, 1H), 8.34 (s, 1H), 8.20 (d, *J* = 7.7 Hz, 1H), 8.10 (d, *J* = 7.7 Hz, 1H), 4.63 (s, 1H), 4.59 (s, 2H), 3.83 (t, *J* = 6.7 Hz, 3H), 3.72 (d, *J* = 4.7 Hz, 4H), 3.67 (d, *J* = 4.6 Hz, 4H), 3.50 (d, *J* = 7.0 Hz, 2H), 3.38 (s, 2H), 3.32 (d, *J* = 7.4 Hz, 2H), 3.23 (d, *J* = 7.5 Hz, 2H), 3.12 (s, 2H), 2.86 (t, *J* = 6.7 Hz, 2H). LCMS (ESI) calcd for $C_{27}H_{31}N_8O_5S^+$ [M+H]$^+$: 579.21, found, 579.1.

**Example 394: preparation of 2-(2,4-dioxotetrahydropyrimidin-1(2H)-yl)-5-((4-(thieno[3,2-d]pyrimidin-4-yl)piperazin-1-yl)methyl)isoindoline-1,3-dione (GT-08452)**

Referring to the method of Scheme 1, the target product (GT-08452) was prepared as a white solid (22 mg, yield 49%). $^1$H NMR (400 MHz, DMSO) δ 10.93 (s, 1H), 8.89 (s, 1H), 8.59 (d, *J* = 5.5 Hz, 1H), 8.36 (s, 1H), 8.22 (d, *J* = 7.7 Hz, 1H), 8.11 (d, *J* = 7.7 Hz, 1H), 7.65 (d, *J* = 5.5 Hz, 1H), 4.86 (s, 2H), 4.62 (s, 3H), 3.83 (t, *J* = 6.7 Hz, 3H), 3.44 (s, 4H), 2.86 (t, *J* = 6.7 Hz, 2H). LCMS (ESI) calcd for $C_{23}H_{22}N_7O_4S^+$ [M+H]$^+$: 492.14, found, 492.1.

**Example 395: preparation of 5-((4-(2-chlorothieno[3,2-d]pyrimidin-4-yl)piperazin-1-yl)methyl)-2-(2,4-dioxotetrahydropyrimidin-1(2H)-yl)isoindoline-1,3-dione (GT-08453)**

Referring to the method of Scheme 1, the target product (GT-08453) was prepared as a white solid (25 mg, yield 52%). $^1$H NMR (400 MHz, DMSO) δ 10.93 (s, 1H), 8.38 (d, *J* = 5.5 Hz, 1H), 8.29 (s, 1H), 8.14 (s, 1H), 8.12 (s, 1H), 7.46 (d, *J* = 5.5 Hz, 1H), 4.71 (s, 2H), 4.56 (s, 2H), 3.83 (t, *J* = 6.7 Hz, 3H), 3.78 - 3.70 (m, 1H), 3.47 (s, 2H), 3.27 (s, 2H), 2.86 (t, *J* = 6.7 Hz, 2H). LCMS (ESI) calcd for $C_{23}H_{21}ClN_7O_4S^+$ [M+H]$^+$: 526.11, found, 526.1.

**Example 396: preparation of 2-(2,4-dioxotetrahydropyrimidin-1(2H)-yl)-5-((4-(2-morpholinothieno[3,2-d]pyrimidin-4-yl)piperazin-1-yl)methyl)isoindoline-1,3-dione (GT-08455)**

Referring to the method of Scheme 1, the target product (GT-08455) was prepared as a white solid (38 mg, yield 73%). $^1$H NMR (400 MHz, DMSO) δ 10.93 (s, 1H), 8.34 (s, 1H), 8.31 (s, 1H), 8.19 (d, *J* = 7.6 Hz, 1H), 8.11 (d, *J* = 7.7 Hz, 1H), 7.57 (s, 1H), 4.67 (d, *J* = 19.2 Hz, 2H), 4.59 (s, 2H), 3.84 (d, *J* = 4.8 Hz, 7H), 3.71 (s, 4H), 3.54 (s, 2H), 3.32 - 3.16 (m, 3H), 2.86 (t, *J* = 6.7 Hz, 2H). LCMS (ESI) calcd for $C_{27}H_{29}N_8O_5S^+$ [M+H]$^+$: 577.20, found, 577.1.

**Example 397: preparation of 2-(2,4-dioxotetrahydropyrimidin-1(2H)-yl)-5-((4-(4-morpholinothieno[3,2-d]pyrimidin-2-yl)piperazin-1-yl)methyl)isoindoline-1,3-dione (GT-08456)**

Referring to the method of Scheme 1, the target product (GT-08456) was prepared as a white solid (25 mg, yield 48%). $^1$H NMR (400 MHz, DMSO-d6) δ 10.93 (s, 1H), 8.32 (s, 2H), 8.17 (s, 1H), 8.11 (d, *J* = 7.6 Hz, 1H), 7.51 (s, 1H), 4.76 (s, 2H), 4.58 (s, 2H), 3.97 (s, 4H), 3.83 (t, *J* = 6.8 Hz, 3H), 3.77 (d, *J* = 4.6 Hz, 3H), 3.58 (s, 3H), 3.17 (s, 3H), 2.86 (t, *J* = 6.8 Hz,

2H). LCMS (ESI) calcd for $C_{27}H_{29}N_8O_5S^+$ [M+H]$^+$: 577.20, found, 577.1.

**Example 398: preparation of 1-(1-oxo-5-((4-(4-(piperazin-1-yl)-6,7-dihydrothieno[3,2-d]pyrimidin-2-yl)piperazin-1-yl)methyl)isoindolin-2-yl)dihydropyrimidine-2,4(1H,3H)-dione (GT-08948)**

Referring to the method of Scheme 1, the target product (GT-08948) was prepared as a white solid (2 mg, yield 22%). LCMS (ESI) calcd for $C_{27}H_{34}N_9O_3S^+$ [M+H]$^+$: 564.25, found, 564.3.

**Example 399: preparation of 1-(1-oxo-5-((4-(4-(piperazin-1-yl)thieno[3,2-d]pyrimidin-2-yl)piperazin-1-yl)methyl) isoindolin-2-yl)dihydropyrimidine-2,4(1H,3H)-dione (GT-08949)**

Referring to the method of Scheme 1, the target product (GT-08949) was prepared as a white solid (2 mg, yield 22%). $^1$H NMR (400 MHz, DMSO) δ 10.69 (s, 1H), 9.63 (s, 2H), 8.29 (d, *J* = 5.3 Hz, 1H), 7.92 (s, 1H), 7.88 (d, *J* = 7.8 Hz, 1H), 7.82 (d, *J* = 7.9 Hz, 1H), 7.46 (s, 1H), 4.77 (d, *J* = 20.8 Hz, 2H), 4.58 (d, *J* = 16.7 Hz, 1H), 4.51 (s, 2H), 4.17 (s, 3H), 3.79 (t, *J* = 6.8 Hz, 3H), 3.45 - 3.40 (m, 3H), 3.27 (s, 5H), 3.20 (s, 3H), 2.88-2.81 (m, 1H), 2.79-2.72 (m, 1H). LCMS (ESI) calcd for $C_{27}H_{32}N_9O_3S^+$ [M+H]$^+$: 562.23, found, 562.2.

**Example 400: preparation of 2-(2,4-dioxotetrahydropyrimidin-1(2H)-yl)-5-((4-(4-(piperazin-1-yl)-6,7-dihydrothieno[3,2-d]pyrimidin-2-yl)piperazin-1-yl)methyl)isoindoline-1,3-dione (GT-08950)**

Referring to the method of Scheme 1, the target product (GT-08950) was prepared as a white solid (4 mg, yield 44%). $^1$H NMR (400 MHz, DMSO) δ 10.93 (s, 1H), 9.69 (s, 2H), 8.36 (s, 1H), 8.21 (s, 1H), 8.10 (d, *J* = 7.7 Hz, 1H), 4.60 (s, 4H), 4.26 (s, 1H), 4.14 (s, 2H), 3.91 (s, 4H), 3.83 (t, *J* = 6.7 Hz, 3H), 3.49 (d, *J* = 7.8 Hz, 2H), 3.33 (t, *J* = 8.2 Hz, 3H), 3.21 (d, *J* = 7.7 Hz, 2H), 3.15 (s, 4H), 2.86 (t, *J* = 6.8 Hz, 2H). LCMS (ESI) calcd for $C_{27}H_{32}N_9O_4S^+$ [M+H]$^+$: 578.23, found, 578.2.

**Example 401: preparation of 2-(2,4-dioxotetrahydropyrimidin-1(2H)-yl)-5-((4-(4-(piperazin-1-yl)thieno[3,2-d] pyrimidin-2-yl)piperazin-1-yl)methyl)isoindoline-1,3-dione (GT-08951)**

Referring to the method of Scheme 1, the target product (GT-08951) was prepared as a white solid (2 mg, yield 17%). $^1$H NMR (400 MHz, DMSO) δ 10.93 (s, 1H), 9.68 (s, 2H), 8.34 (s, 1H), 8.31 (d, *J* = 5.2 Hz, 1H), 8.21 (d, *J* = 7.8 Hz, 1H), 8.11 (d, *J* = 7.7 Hz, 1H), 7.49 (s, 1H), 4.78 (s, 2H), 4.60 (s, 2H), 4.18 (s, 4H), 3.83 (t, *J* = 6.8 Hz, 3H), 3.46 - 3.42 (m, 2H), 3.27 (s, 5H), 3.17 (s, 2H), 2.86 (t, *J* = 6.8 Hz, 2H). LCMS (ESI) calcd for $C_{27}H_{30}N_9O_4S^+$ [M+H]$^+$: 576.21, found, 576.2.

**Example 402: preparation of 5-((4-(5-chloropyridin-2-yl)piperazin-1-yl)methyl)-2-(2,4-dioxotetrahydropyrimidin-1(2H)-yl)isoindoline-1,3-dione (GT-05350)**

Referring to the method of Scheme 1, the target product (GT-05350) was prepared as a white solid (28 mg, yield 49 %). $^1$H NMR (400 MHz, DMSO) δ 10.93 (s, 1H), 8.32 (s, 1H), 8.19 - 8.14 (m, 2H), 8.11 (d, *J* = 7.6 Hz, 1H), 7.71 (dd, *J* = 9.1, 2.6 Hz, 1H), 7.00 (d, 1H), 4.58 (s, 2H), 4.34 (s, 2H), 3.82 (t, *J* = 6.7 Hz, 2H), 3.40 - 3.29 (m, 4H), 3.11 (s, 2H), 2.86 (t, *J* = 6.8 Hz, 2H). LCMS (ESI) calcd for $C_{22}H_{22}ClN_6O_4^+$ [M+H]$^+$: 469.14, found, 469.2.

**Example 403: preparation of 2-(2,4-dioxotetrahydropyrimidin-1(2H)-yl)-5-((4-(pyridin-2-yl)piperazin-1-yl) methyl)isoindoline-1,3-dione (GT-05351)**

Referring to the method of Scheme 1, the target product (GT-05351) was prepared as a white solid (20 mg, yield 37 %). $^1$H NMR (400 MHz, DMSO) δ 10.93 (s, 1H), 9.25 (s, 1H), 8.34 (s, 1H), 8.20 (d, *J* = 7.7 Hz, 1H), 8.12 (t, *J* = 7.2 Hz, 2H), 7.89 - 7.79 (m, 1H), 7.19 (d, *J* = 8.6 Hz, 1H), 6.91 (t, *J* = 6.2 Hz, 1H), 4.60 (s, 2H), 4.46 (s, 1H), 3.91 (s, 1H), 3.83 (t, *J* = 6.7 Hz, 3H), 3.23 (s, 2H), 3.16 - 3.09 (m, 3H), 2.86 (t, *J* = 6.8 Hz, 2H). LCMS (ESI) calcd for $C_{22}H_{23}N_6O_4^+$ [M+H]$^+$: 435.18, found, 435.2.

**Example 404: preparation of 2-(2,4-dioxotetrahydropyrimidin-1(2H)-yl)-5-((4-(6-methylpyridin-2-yl)piperazin-1-yl)methyl)isoindoline-1,3-dione (GT-05352)**

Referring to the method of Scheme 1, the target product (GT-05352) was prepared as a white solid (16 mg, yield 29 %). $^1$H NMR (400 MHz, DMSO) δ 10.93 (s, 1H), 8.32 (s, 1H), 8.18 (d, *J* = 7.8 Hz, 1H), 8.11 (d, *J* = 7.6 Hz, 1H), 7.69 (s, 1H), 6.90 (s, 1H), 6.74 (d, *J* = 7.1 Hz, 1H), 4.59 (s, 1H), 4.49 (d, *J* = 4.7 Hz, 1H), 4.42 (s, 1H), 3.82 (d, *J* = 6.8 Hz, 2H), 3.68 - 3.64 (m, 2H), 3.17 - 3.12 (m, 2H), 2.86 (t, J = 6.8 Hz, 2H), 2.76 *(t, J* = 6.6 Hz, 1H), 2.42 (s, 3H). LCMS (ESI) calcd for $C_{23}H_{25}N_6O_4^+$ [M+H]$^+$: 449.19, found, 449.2.

**Example 405: preparation of 5-((4-(6-chloropyridin-2-yl)piperazin-1-yl)methyl)-2-(2,4-dioxotetrahydropyrimidin-1(2H)-yl)isoindoline-1,3-dione (GT-05353)**

Referring to the method of Scheme 1, the target product (GT-05353) was prepared as a white solid (30 mg, yield 52 %). $^1$H NMR (400 MHz, DMSO) δ 10.93 (s, 1H), 8.27 (s, 1H), 8.12 (s, 2H), 7.64 (t, *J* = 7.9 Hz, 1H), 6.89 (d, *J* = 8.5 Hz, 1H), 6.78 (d, *J* = 7.4 Hz, 1H), 4.58 (s, 3H), 3.83 (t, *J* = 6.8 Hz, 2H), 3.41 (s, 2H), 3.31 (s, 3H), 3.12 (s, 2H), 2.86 (t, *J* = 6.8 Hz, 2H). LCMS (ESI) calcd for $C_{22}H_{22}ClN_6O_4^+$ [M+H]$^+$: 469.14, found, 469.1.

**Example 406: preparation of 5-((4-(4-chloropyridin-2-yl)piperazin-1-yl)methyl)-2-(2,4-dioxotetrahydropyrimidin-1(2H)-yl)isoindoline-1,3-dione (GT-05354)**

Referring to the method of Scheme 1, the target product (GT-05354) was prepared as a white solid (28 mg, yield 49 %). $^1$H NMR (400 MHz, DMSO) δ 10.92 (s, 1H), 8.32 (s, 1H), 8.18 (d, *J* = 8.6 Hz, 1H), 8.11 *(t, J* = 6.6 Hz, 2H), 7.10 (d, *J* = 1.2 Hz, 1H), 6.84 (dd, *J* = 5.4, 1.4 Hz, 1H), 4.58 (s, 2H), 4.47 (s, 2H), 3.83 (t, *J* = 6.8 Hz, 2H), 3.38 (s, 4H), 3.11 (s, 2H), 2.86 (t, *J* = 6.8 Hz, 2H). LCMS (ESI) calcd for $C_{22}H_{22}ClN_6O_4^+$ [M+H]$^+$: 469.14, found, 469.1.

**Example 407: preparation of 2-(2,4-dioxotetrahydropyrimidin-1(2H)-yl)-5-((4-(3-fluoropyridin-2-yl)piperazin-1-yl)methyl)isoindoline-1,3-dione (GT-05355)**

Referring to the method of Scheme 1, the target product (GT-05355) was prepared as a white solid (24 mg, yield 43 %). $^1$H NMR (400 MHz, DMSO) δ 11.62 (s, 1H), 10.93 (s, 1H), 8.33 (s, 1H), 8.19 (dd, *J* = 7.8, 1.3 Hz, 1H), 8.11 (d, *J* = 7.7 Hz, 1H), 8.08 - 8.06 (m, 1H), 7.62 - 7.56 (m, 1H), 7.02 - 6.97 (m, 1H), 4.60 (s, 2H), 4.05 (d, *J* = 11.5 Hz, 2H), 3.84 (d, *J* = 6.8 Hz, 2H), 3.45 - 3.37 (m, 4H), 3.22 (s, 2H), 2.86 (t, *J* = 6.8 Hz, 2H). LCMS (ESI) calcd for $C_{22}H_{22}FN_6O_4^+$ [M+H]$^+$: 453.17, found, 453.2.

**Example 408: preparation of 1-(5-((4-(3,5-dichloropyridin-2-yl)piperazin-1-yl)methyl)-1-oxoisoindolin-2-yl)dihydropyrimidine-2,4(1H,3H)-dione (GT-05356)**

Referring to the method of Scheme 1, the target product (GT-05356) was prepared as a white solid (36 mg, yield 46 %). $^1$H NMR (400 MHz, DMSO) δ 11.22 (s, 1H), 10.69 (s, 1H), 8.34 (d, *J* = 2.3 Hz, 1H), 8.14 (d, *J* = 2.3 Hz, 1H), 7.90 (d, *J* = 3.0 Hz, 1H), 7.88 (s, 1H), 7.81 (d, *J* = 7.9 Hz, 1H), 4.80 (t, *J* = 19.2 Hz, 1H), 4.62 - 4.50 (m, 3H), 3.89 - 3.77 (m, 4H), 3.36 - 3.31 (m, 4H), 3.24 (s, 2H), 2.93 - 2.82 (m, 1H), 2.81 - 2.71 (m, 1H). LCMS (ESI) calcd for $C_{22}H_{23}Cl_2N_6O_3^+$ [M+H]$^+$: 489.12, found, 489.1.

**Example 409: preparation of 1-(5-((4-(3,4-difluoropyridin-2-yl)piperazin-1-yl)methyl)-1-oxoisoindolin-2-yl)dihydropyrimidine-2,4(1H,3H)-dione (GT-05357)**

Referring to the method of Scheme 1, the target product (GT-05357) was prepared as a white solid (39 mg, yield 53 %). $^1$H NMR (400 MHz, DMSO) δ 11.47 (s, 1H), 10.69 (s, 1H), 8.13 - 8.00 (m, 1H), 7.89 (t, 2H), 7.84 - 7.71 (m, 1H), 7.13 - 7.03 (m, 1H), 4.82 (d, *J* = 16.7 Hz, 1H), 4.63 - 4.45 (m, 3H), 4.10 (s, 1H), 3.80 (t, *J* = 6.9 Hz, 3H), 3.45 - 3.37 (m, 4H), 3.22 (s, 2H), 2.92 - 2.81 (m, 1H), 2.81 - 2.73 (m, 1H). LCMS (ESI) calcd for $C_{22}H_{23}F_2N_6O_3^+$ [M+H]$^+$: 457.18, found, 457.2.

**Example 410: preparation of 1-(5-((4-(4-chloro-3-fluoropyridin-2-yl)piperazin-1-yl)methyl)-1-oxoisoindolin-2-yl) dihydropyrimidine-2,4(1H,3H)-dione (GT-05358)**

Referring to the method of Scheme 1, the target product (GT-05358) was prepared as a white solid (23 mg, yield 31 %). $^1$H NMR (400 MHz, DMSO) δ 11.31 (s, 1H), 10.68 (s, 1H), 8.02 (d, *J* = 5.3 Hz, 1H), 7.93 - 7.85 (m, 2H), 7.83 - 7.76 (m, 1H), 7.19 (t, *J* = 4.9 Hz, 1H), 4.80 (t, *J* = 18.8 Hz, 1H), 4.62 - 4.48 (m, 3H), 4.09 (d, *J* = 13.0 Hz, 2H), 3.80 (t, *J* = 6.8 Hz, 2H), 3.41 (s, 4H), 3.21 (s, 2H), 2.92 - 2.83 (m, 1H), 2.81 - 2.74 (m, 1H). LCMS (ESI) calcd for $C_{22}H_{23}ClFN_6O_3^+$ [M+H]$^+$: 473.15, found, 473.2.

**Example 411: preparation of 1-(5-((4-(3-fluoro-4-iodopyridin-2-yl)piperazin-1-yl)methyl)-1-oxoisoindolin-2-yl) dihydropyrimidine-2,4(1H,3H)-dione (GT-05406)**

Referring to the method of Scheme 1, the target product (GT-05406) was prepared as a white solid (32 mg, yield 36 %). $^1$H NMR (400 MHz, DMSO) δ 11.29 (s, 1H), 10.69 (s, 1H), 7.95 - 7.85 (m, 2H), 7.79 (d, 1H), 7.73 (d, *J* = 5.1 Hz, 1H), 7.39 (dd, *J* = 5.0, 3.8 Hz, 1H), 4.82 (d, *J* = 16.6 Hz, 1H), 4.59 (d, *J* = 16.9 Hz, 1H), 4.50 (s, 2H), 4.05 (d, *J* = 12.4 Hz, 2H), 3.80 (t, *J* = 6.9 Hz, 2H), 3.59 (s, 1H), 3.40 (s, 4H), 3.20 (s, 2H), 2.92 - 2.81 (m, 1H), 2.81 - 2.71 (m, 1H). LCMS (ESI) calcd for

$C_{22}H_{23}FIN_6O_3^+$ [M+H]$^+$: 565.09, found, 565.1.

**Example 412: preparation of 1-(5-((4-(3-bromo-4-chloropyridin-2-yl)piperazin-1-yl)methyl)-1-oxoisoindolin-2-yl)dihydropyrimidine-2,4(1H,3H)-dione (GT-05432)**

Referring to the method of Scheme 1, the target product (GT-05432) was prepared as a white solid (44 mg, yield 52 %). $^1$H NMR (400 MHz, DMSO-d6) δ 11.44 (s, 1H), 10.71 (s, 1H), 8.25 (d, *J* = 5.2 Hz, 1H), 7.93 (s, 1H), 7.86 (dd, *J* = 21.4, 7.8 Hz, 2H), 7.38 (d, *J* = 5.2 Hz, 1H), 4.82 (d, *J* = 16.7 Hz, 1H), 4.59 (s, 1H), 4.55 (d, *J* = 3.6 Hz, 2H), 3.85 - 3.75 (m, 4H), 3.47 - 3.31 (m, 4H), 3.27 - 3.15 (m, 2H), 2.94 - 2.83 (m, 1H), 2.81 - 2.70 (m, 1H). LCMS (ESI) calcd for $C_{22}H_{23}BrClN_6O_3^+$ [M+H]$^+$: 533.07, found, 533.1.

**Example 413: preparation of 1-(5-((4-(3-chloro-5-(trifluoromethyl)pyridin-2-yl)piperazin-1-yl)methyl)-1-oxoisoindolin-2-yl)dihydropyrimidine-2,4(1H,3H)-dione (GT-05433)**

Referring to the method of Scheme 1, the target product (GT-05433) was prepared as a white solid (37 mg, yield 45 %). $^1$H NMR (400 MHz, DMSO-d6) δ 11.17 (s, 1H), 10.71 (s, 1H), 8.63 (s, 1H), 8.31 (s, 1H), 7.89 (d, *J* = 6.8 Hz, 2H), 7.79 (d, *J* = 7.3 Hz, 1H), 4.82 (d, *J* = 16.5 Hz, 1H), 4.59 (d, *J* = 15.0 Hz, 1H), 4.54 (s, 2H), 4.07 (d, *J* = 31.4, 10.1 Hz, 2H), 3.79 (t, *J* = 7.0 Hz, 2H), 3.47 - 3.38 (m, 4H), 3.30 - 3.19 (m, 2H), 2.93 - 2.82 (m, 1H), 2.80 - 2.70 (m, 1H). LCMS (ESI) calcd for $C_{23}H_{23}ClF_3N_6O_3^+$ [M+H]$^+$: 523.15, found, 523.2.

**Example 414: preparation of 1-(1-oxo-5-((4-(pyridin-3-yl)piperazin-1-yl)methyl)isoindolin-2-yl)dihydropyrimidine-2,4(1H,3H)-dione (GT-05434)**

Referring to the method of Scheme 1, the target product (GT-05434) was prepared as a brown solid (14 mg, yield 21 %). $^1$H NMR (400 MHz, DMSO-d6) δ 11.89 (s, 1H), 10.71 (s, 1H), 8.52 (d, *J* = 2.7 Hz, 1H), 8.26 (d, *J* = 5.2 Hz, 1H), 8.05 (d, *J* = 8.8, 2.0 Hz, 1H), 7.93 (s, 1H), 7.88 (d, *J* = 7.8 Hz, 1H), 7.86 - 7.77 (m, 2H), 4.82 (d, *J* = 16.6 Hz, 1H), 4.58 (d, *J* = 16.3 Hz, 1H), 4.52 (s, 2H), 4.05 (s, 2H), 3.79 (t, *J* = 6.8 Hz, 2H), 3.66 - 3.57 (m, 2H), 3.28 - 3.11 (m, 4H), 2.92 - 2.83 (m, 1H), 2.80 - 2.72 (m, 1H). LCMS (ESI) calcd for $C_{22}H_{25}N_6O_3^+$ [M+H]$^+$: 421.20, found, 421.2.

**Example 415: preparation of 1-(5-((4-(6-chloropyridin-3-yl)piperazin-1-yl)methyl)-1-oxoisoindolin-2-yl)dihydropyrimidine-2,4(1H,3H)-dione (GT-05435)**

Referring to the method of Scheme 1, the target product (GT-05435) was prepared as a white solid (22 mg, yield 30 %). $^1$H NMR (400 MHz, DMSO-d6) δ 11.51 (s, 1H), 10.71 (s, 1H), 8.13 (d, *J* = 3.1 Hz, 1H), 7.92 (s, 1H), 7.88 (d, *J* = 7.8 Hz, 1H), 7.81 (d, *J* = 8.0 Hz, 1H), 7.49 (dd, *J* = 8.9, 3.2 Hz, 1H), 7.37 (d, *J* = 8.8 Hz, 1H), 4.82 (d, *J* = 16.5 Hz, 1H), 4.58 (d, *J* = 16.7 Hz, 1H), 4.52 (s, 2H), 3.90 (d, *J* = 12.6 Hz, 2H), 3.79 (t, *J* = 6.8 Hz, 2H), 3.38 (d, 2H), 3.32 - 3.11 (m, 4H), 2.96 - 2.82 (m, 1H), 2.81 - 2.69 (m, 1H). LCMS (ESI) calcd for $C_{22}H_{24}ClN_6O_3^+$ [M+H]$^+$: 455.16, found, 455.1.

**Example 416: preparation of 1-(5-((4-(4,5-dichloropyridin-3-yl)piperazin-1-yl)methyl)-1-oxoisoindolin-2-yl)dihydropyrimidine-2,4(1H,3H)-dione (GT-05436)**

Referring to the method of Scheme 1, the target product (GT-05436) was prepared as a white solid (21 mg, yield 27 %). $^1$H NMR (400 MHz, DMSO-d6) δ 11.51 (s, 1H), 10.70 (s, 1H), 8.52 (s, 1H), 8.41 (s, 1H), 7.94 (s, 1H), 7.89 (d, *J* = 7.8 Hz, 1H), 7.84 (d, *J* = 8.1 Hz, 1H), 4.82 (d, *J* = 16.7 Hz, 1H), 4.59 (d, *J* = 17.1 Hz, 3H), 3.79 (t, 4H), 3.55 (d, *J* = 11.8 Hz, 2H), 3.49 - 3.33 (m, 4H), 2.94 - 2.82 (m, 1H), 2.80 - 2.70 (m, 1H). LCMS (ESI) calcd for $C_{22}H_{23}Cl_2N_6O_3^+$ [M+H]$^+$: 489.12, found, 489.1.

**Example 417: preparation of 1-(1-oxo-5-((4-(pyridin-4-yl)piperazin-1-yl)methyl)isoindolin-2-yl)dihydropyrimidine-2,4(1H,3H)-dione (GT-05437)**

Referring to the method of Scheme 1, the target product (GT-05437) was prepared as a white solid (7 mg, yield 11 %). $^1$H NMR (400 MHz, DMSO-d6) δ 13.93 (s, 1H), 10.69 (s, 1H), 8.34 (d, *J* = 6.5 Hz, 2H), 7.86 (d, *J* = 7.8 Hz, 2H), 7.78 (s, 1H), 7.25 (d, *J* = 7.1 Hz, 2H), 4.81 (d, *J* = 16.6 Hz, 1H), 4.57 (d, *J* = 16.5 Hz, 1H), 4.45 (s, 2H), 3.79 (t, *J* = 6.8 Hz, 2H), 3.74 - 3.56 (m, 4H), 3.45 (s, 2H), 3.25 - 3.12 (m, 2H), 2.88 - 2.82 (m, 1H), 2.81 - 2.75 (m, 1H). LCMS (ESI) calcd for $C_{22}H_{25}N_6O_3^+$ [M+H]$^+$: 421.20, found, 421.2.

**Example 418: preparation of 1-(5-((4-(2-methylpyridin-4-yl)piperazin-1-yl)methyl)-1-oxoisoindolin-2-yl)dihydropyrimidine-2,4(1H,3H)-dione (GT-05438)**

Referring to the method of Scheme 1, the target product (GT-05438) was prepared as a white solid (40 mg, yield 57 %). $^1$H NMR (400 MHz, DMSO-d6) δ 13.99 (s, 1H), 10.69 (s, 1H), 9.16 (s, 1H), 8.25 (d, *J = 6.8* Hz, 1H), 7.90 - 7.75 (m, 2H), 7.22 - 7.08 (m, 2H), 4.82 (d, *J* = 16.4 Hz, 1H), 4.57 (d, *J* = 16.5 Hz, 1H), 4.41 (s, 2H), 3.79 (t, *J* = 5.3 Hz, 1H), 3.75 - 3.64 (m, 2H), 3.63 - 3.52 (m, 4H), 3.27 - 3.14 (m, 3H), 3.14 - 3.08 (m, 3H), 2.90 - 2.80 (m, 1H), 2.79 - 2.72 (m, 1H). LCMS (ESI) calcd for $C_{23}H_{27}N_6O_3^+$ [M+H]$^+$: 435.21, found, 435.2.

**Example 419: preparation of 1-(5-((4-(3-bromopyridin-4-yl)piperazin-1-yl)methyl)-1-oxoisoindolin-2-yl)dihydropyrimidine-2,4(1H,3H)-dione (GT-05439)**

Referring to the method of Scheme 1, the target product (GT-05439) was prepared as a white solid (10 mg, yield 13 %). $^1$H NMR (400 MHz, DMSO-d6) δ 11.67 (s, 1H), 10.71 (s, 1H), 8.77 (s, 1H), 8.50 (d, *J* = 6.2 Hz, 1H), 7.95 - 7.86 (m, 2H), 7.82 (d, *J* = 7.9 Hz, 1H), 7.34 (d, *J* = 6.1 Hz, 1H), 4.82 (d, *J* = 16.6 Hz, 1H), 4.58 (d, *J* = 17.0 Hz, 3H), 3.92 (s, 2H), 3.78 (t, *J* = 6.7 Hz, 2H), 3.42 - 3.39 (m, 2H), 3.29 (s, 4H), 2.91 - 2.84 (m, 1H), 2.80 - 2.72 (m, 1H). LCMS (ESI) calcd for $C_{22}H_{24}BrN_6O_3^+$ [M+H]$^+$: 499.11, found, 499.1.

**Example 420: preparation of 1-(5-((4-(3-fluoropyridin-4-yl)piperazin-1-yl)methyl)-1-oxoisoindolin-2-yl)dihydropyrimidine-2,4(1H,3H)-dione (GT-05697)**

Referring to the method of Scheme 1, the target product (GT-05697) was prepared as a white solid (25 mg, yield 36 %). $^1$H NMR (400 MHz, DMSO-d6) δ 12.24 (s, 1H), 10.69 (s, 1H), 8.73 (d, *J* = 8.0 Hz, 1H), 8.36 (d, *J* = 6.4 Hz, 1H), 7.93 (s, 1H), 7.85 (dd, *J* = 21.0, 7.9 Hz, 2H), 7.47 - 7.37 (m, 1H), 4.81 (d, *J* = 16.6 Hz, 1H), 4.57 (d, *J* = 16.8 Hz, 1H), 4.51 (s, 2H), 4.16 (s, 2H), 3.79 (t, *J* = 6.8 Hz, 2H), 3.65 - 3.55 (m, 4H), 3.15 - 3.08 (m, 2H), 2.90 - 2.83 (m, 1H), 2.80 - 2.73 (m, 1H). LCMS (ESI) calcd for $C_{22}H_{24}FN_6O_3^+$ [M+H]$^+$: 439.19, found, 439.2.

**Example 421: preparation of 1-(5-((4-(3-chloropyridin-4-yl)piperazin-1-yl)methyl)-1-oxoisoindolin-2-yl)dihydropyrimidine-2,4(1H,3H)-dione (GT-05698)**

Referring to the method of Scheme 1, the target product (GT-05698) was prepared as a white solid (24 mg, yield 33 %). $^1$H NMR (400 MHz, DMSO-d6) δ 12.10 (s, 1H), 10.70 (s, 1H), 8.72 (s, 1H), 8.49 (d, *J* = 6.4 Hz, 1H), 7.95 (s, 1H), 7.90 - 7.83 (m, 2H), 7.42 (d, *J* = 6.5 Hz, 1H), 4.82 (d, *J* = 16.6 Hz, 1H), 4.58 (d, *J* = 17.3 Hz, 3H), 4.04 (s, 2H), 3.79 (t, *J* = 4.5 Hz, 2H), 3.44 (s, 4H), 3.31 (s, 2H), 2.90 - 2.83 (m, 1H), 2.81 - 2.73 (m, 1H). LCMS (ESI) calcd for $C_{22}H_{24}ClN_6O_3^+$ [M+H]$^+$: 455.16, found, 455.2.

**Example 422: preparation of 1-(5-((4-(2-chloropyridin-4-yl)piperazin-1-yl)methyl)-1-oxoisoindolin-2-yl)dihydropyrimidine-2,4(1H,3H)-dione (GT-05699)**

Referring to the method of Scheme 1, the target product (GT-05699) was prepared as a white solid (35 mg, yield 48 %). $^1$H NMR (400 MHz, DMSO-d6) δ 11.88 (s, 1H), 10.67 (s, 1H), 8.09 (d, *J* = 6.3 Hz, 1H), 7.91 (s, 1H), 7.87 (d, *J* = 7.8 Hz, 1H), 7.80 (d, *J* = 7.8 Hz, 1H), 7.08 (d, *J* = 4.9 Hz, 1H), 6.97 (dd, *J* = 6.3, 2.4 Hz, 1H), 4.81 (d, *J* = 16.4 Hz, 1H), 4.57 (d, *J* = 16.8 Hz, 1H), 4.50 (s, 2H), 4.28 - 4.11 (m, 2H), 3.73 - 3.57 (m, 2H), 3.49 (s, 2H), 3.36 (s, 2H), 3.21 - 3.05 (m, 2H), 2.92 - 2.82 (m, 1H), 2.81 - 2.69 (m, 1H). LCMS (ESI) calcd for $C_{22}H_{24}ClN_6O_3^+$ [M+H]$^+$: 455.16, found, 455.2.

**Example 423: preparation of 1-(1-oxo-5-((4-(phenyl(pyridin-3-yl)methyl)piperazin-1-yl)methyl)isoindolin-2-yl)dihydropyrimidine-2,4(1H,3H)-dione (GT-06376)**

Referring to the method of Scheme 1, the target product (GT-06376) was prepared as a white solid (26 mg, yield 44 %). $^1$H NMR (400 MHz, DMSO-d6) δ 11.42 (s, 1H), 10.67 (d, *J* = 14.1 Hz, 1H), 8.97 (s, 1H), 8.78 (d, *J* = 5.3 Hz, 1H), 8.53 (d, *J* = 8.0 Hz, 1H), 7.97 - 7.91 (m, 1H), 7.89 (s, 1H), 7.85 (d, *J* = 7.8 Hz, 1H), 7.79 (d, *J* = 7.9 Hz, 1H), 7.48 (d, *J* = 7.3 Hz, 2H), 7.38 (t, *J* = 7.5 Hz, 2H), 7.30 (t, *J* = 7.3 Hz, 1H), 5.00 (s, 1H), 4.80 (d, *J* = 16.9 Hz, 1H), 4.68 - 4.40 (m, 4H), 3.25 (d, *J* = 45.9 Hz, 5H), 2.96 - 2.71 (m, 5H), 2.68 (s, 1H). LCMS (ESI) calcd for $C_{29}H_{31}N_6O_3^+$ [M+H]$^+$: 511.25, found, 511.3.

**Example 424: preparation of 2-(2,4-dioxotetrahydropyrimidin-1(2H)-yl)-5-((4-(phenyl(pyridin-3-yl)methyl)piperazin-1-yl)methyl)isoindoline-1,3-dione (GT-06377)**

Referring to the method of Scheme 1, the target product (GT-06377) was prepared as a white solid (8 mg, yield 14

%). $^1H$ NMR (400 MHz, DMSO-d6) δ 10.93 (s, 1H), 10.54 (dd, $J$ = 51.0, 3.1 Hz, 1H), 8.93 (s, 1H), 8.75 (d, $J$ = 5.4 Hz, 1H), 8.47 (d, $J$ = 7.8 Hz, 1H), 8.09 (d, $J$ = 7.5 Hz, 1H), 7.93 - 7.88 (m, 2H), 7.47 (d, $J$ = 7.0 Hz, 2H), 7.38 (s, 2H), 7.31 (d, $J$ = 7.1 Hz, 1H), 4.95 (s, 1H), 4.55 (s, 1H), 4.43 (d, $J$ = 10.4 Hz, 2H), 3.81 (d, $J$ = 6.8 Hz, 2H), 3.32 - 3.31 (m, 1H), 3.23 - 3.17 (m, 4H), 2.86 (t, $J$ = 6.8 Hz, 4H). LCMS (ESI) calcd for $C_{29}H_{29}N_6O_4^+$ [M+H]$^+$: 525.22, found, 525.3.

**Example 425: preparation of 2-(2,4-dioxotetrahydropyrimidin-1(2H)-yl)-5-((4-(phenyl(pyridin-4-yl)methyl)piperazin-1-yl)methyl)isoindoline-1,3-dione (GT-06378)**

Referring to the method of Scheme 1, the target product (GT-06378) was prepared as a white solid (22 mg, yield 38 %). $^1H$ NMR (400 MHz, DMSO-d6) δ 10.93 (s, 1H), 10.54 (dd, $J$ = 48.4, 3.9 Hz, 1H), 8.78 (d, $J$ = 6.3 Hz, 3H), 8.10 (d, $J$ = 7.9 Hz, 1H), 7.98 (d, $J$ = 5.5 Hz, 2H), 7.44 (d, $J$ = 7.2 Hz, 2H), 7.38 (t, $J$ = 7.5 Hz, 3H), 4.91 (s, 1H), 4.50 (d, $J$ = 41.9 Hz, 2H), 3.83 (d, $J$ = 6.9 Hz, 2H), 3.66 - 3.65 (m, 1H), 3.21 - 3.18 (m, 4H), 2.86 (t, $J$ = 6.8 Hz, 3H), 2.75 (t, $J$ = 6.8 Hz, 2H). LCMS (ESI) calcd for $C_{29}H_{29}N_6O_4^+$ [M+H]$^+$: 525.22, found, 525.3.

**Example 426: preparation of 5-((4-(di(pyridin-2-yl)methyl)piperazin-1-yl)methyl)-2-(2,4-dioxotetrahydropyrimidin-1(2H)-yl)isoindoline-1,3-dione (GT-06379)**

Referring to the method of Scheme 1, the target product (GT-06379) was prepared as a white solid (26 mg, yield 44 %). $^1H$ NMR (400 MHz, DMSO-d6) δ 10.93 (t, 1H), 10.63 (s, 1H), 9.80 - 9.74 (m, 1H), 8.72 - 8.70 (m, 2H), 8.13 - 8.11 (m, 2H), 7.81 (t, 2H), 7.72 - 7.71 (m, 1H), 7.61 (s, 2H), 5.82 (s, 1H), 4.59 - 4.45 (m, 5H), 3.46 (s, 5H), 2.95 (s, 5H), 2.84 (s, 1H). LCMS (ESI) calcd for $C_{28}H_{28}N_7O_4^+$ [M+H]$^+$: 526.22, found, 526.3.

**Example 427: preparation of 1-(5-((3-benzhydryl-3,6-diazabicyclo[3.1.1]heptan-6-yl)methyl)-1-oxoisoindolin-2-yl)dihydropyrimidine-2,4(1H,3H)-dione (GT-06380)**

Referring to the method of Scheme 1, the target product (GT-06380) was prepared as a white solid (27 mg, yield 51 %). $^1H$ NMR (400 MHz, DMSO-d6) δ 10.69 (s, 1H), 7.89 - 7.68 (m, 3H), 7.59 - 7.44 (m, 4H), 7.35 (t, $J$ = 15.1, 7.4 Hz, 4H), 7.27 - 7.18 (m, 2H), 4.82 - 4.51 (m, 4H), 4.35 - 4.20 (m, 2H), 3.79 (dd, $J$ = 15.6, 8.4 Hz, 2H), 3.59 (s, 1H), 3.36 - 3.24 (m, 1H), 3.08 (s, 2H), 2.87 - 2.63 (m, 3H), 2.48 - 2.22 (m, 2H). LCMS (ESI) calcd for $C_{31}H_{32}N_5O_3^+$ [M+H]$^+$: 522.25, found, 522.3.

**Example 428: preparation of 5-((3-benzhydryl-3,6-diazabicyclo[3.1.1]heptan-6-yl)methyl)-2-(2,4-dioxotetrahydropyrimidin-1(2H)-yl)isoindoline-1,3-dione (GT-06381)**

Referring to the method of Scheme 1, the target product (GT-06381) was prepared as a white solid (25 mg, yield 48 %). $^1H$ NMR (400 MHz, DMSO-d6) δ 10.92 (s, 1H), 8.18 (d, $J$ = 6.3 Hz, 1H), 8.06 (t, $J$ = 5.0 Hz, 1H), 7.54 - 7.43 (m, 4H), 7.39 - 7.28 (m, 5H), 7.27 - 7.21 (m, 2H), 4.80 - 4.60 (m, 2H), 4.52 - 4.10 (m, 3H), 3.87 - 3.78 (m, 2H), 3.64 (s, 1H), 3.07 (d, $J$ = 11.6 Hz, 2H), 2.88 - 2.82 (m, 2H), 2.78 - 2.67 (m, 1H), 2.33 (d, $J$ = 1.9 Hz, 2H). LCMS (ESI) calcd for $C_{31}H_{30}N_5O_4^+$ [M+H]$^+$: 536.23, found, 536.3.

**Example 429: preparation of 1-(5-(((1R,4R)-5-benzhydryl-2,5-diazabicyclo[2.2.1]heptan-2-yl)methyl)-1-oxoisoindolin-2-yl)dihydropyrimidine-2,4(1H,3H)-dione (GT-06382)**

Referring to the method of Scheme 1, the target product (GT-06382) was prepared as a yellow solid (29 mg, yield 55 %). $^1H$ NMR (400 MHz, DMSO-d6) δ 10.69 (s, 1H), 7.98 (s, 1H), 7.96 (s, 1H), 7.92 - 7.70 (m, 3H), 7.55 (s, 1H), 7.54 - 7.32 (m, 5H), 7.31 - 7.05 (m, 2H), 4.69 (dd, $J$ = 92.6, 16.3 Hz, 4H), 4.32 (d, $J$ = 79.6 Hz, 2H), 3.79 (t, $J$ = 6.9 Hz, 2H), 3.53 (s, 1H), 3.39 - 2.98 (m, 3H), 2.93 - 2.65 (m, 3H), 2.50 - 2.02 (m, 2H). LCMS (ESI) calcd for $C_{31}H_{32}N_5O_3^+$ [M+H]$^+$: 522.25, found, 522.3.

**Example 430: preparation of 5-(((1R,4R)-5-benzhydryl-2,5-diazabicyclo[2.2.1]heptan-2-yl)methyl)-2-(2,4-dioxotetrahydropyrimidin-1(2H)-yl)isoindoline-1,3-dione (GT-06383)**

Referring to the method of Scheme 1, the target product (GT-06383) was prepared as a yellow solid (25 mg, yield 48 %). $^1H$ NMR (400 MHz, DMSO-d6) δ 10.93 (s, 1H), 8.33 (d, $J$ = 38.7 Hz, 2H), 8.14 - 7.86 (m, 4H), 7.40 - 7.16 (m, 7H), 4.69 - 4.18 (m, 5H), 3.83 (t, $J$ = 6.7 Hz, 2H), 3.68 - 3.63 (m, 1H), 3.31 - 3.04 (m, 3H), 2.86 (t, $J$ = 6.8 Hz, 2H), 2.76 (t, $J$ = 7.0 Hz, 1H), 2.56 (s, 1H), 2.49 - 2.28 (m, 1H). LCMS (ESI) calcd for $C_{31}H_{30}N_5O_4^+$ [M+H]$^+$: 536.23, found, 536.3.

**Example 431: preparation of 1-(5-((4-benzhydryl-3,3-dimethylpiperazin-1-yl)methyl)-1-oxoisoindolin-2-yl)dihydropyrimidine-2,4(1H,3H)-dione (GT-06384)**

Referring to the method of Scheme 1, the target product (GT-06384) was prepared as a white solid (26 mg, yield 48 %). $^1$H NMR (400 MHz, DMSO-d6) δ 10.69 (s, 1H), 10.31 (s, 1H), 8.07 - 7.77 (m, 3H), 7.43 - 7.27 (m, 7H), 7.22 (s, 2H), 5.48 (s, 1H), 4.81 (d, *J* = 15.8 Hz, 1H), 4.72 - 4.21 (m, 3H), 3.78 (t, *J* = 6.9 Hz, 2H), 3.30 (s, 1H), 3.13 (s, 1H), 3.02 - 2.80 (m, 3H), 2.82 - 2.65 (m, 3H), 1.38 - 1.15 (m, 3H), 1.13 - 0.78 (m, 3H). LCMS (ESI) calcd for $C_{32}H_{36}N_5O_3^+$ [M+H]$^+$: 538.28, found, 538.3.

**Example 432: preparation of 5-((4-benzhydryl-3,3-dimethylpiperazin-1-yl)methyl)-2-(2,4-dioxotetrahydropyrimidin-1(2H)-yl)isoindoline-1,3-dione (GT-06385)**

Referring to the method of Scheme 1, the target product (GT-06385) was prepared as a white solid (27 mg, yield 51 %). $^1$H NMR (400 MHz, DMSO-d6) δ 10.92 (s, 1H), 8.39 - 7.99 (m, 3H), 7.42 - 7.17 (m, 10H), 5.49 (s, 1H), 4.52 (s, 2H), 3.82 (t, *J* = 6.7 Hz, 2H), 3.06 (d, *J* = 52.8 Hz, 4H), 2.86 (t, *J* = 6.8 Hz, 2H), 2.77 - 2.66 (m, 2H), 1.38 - 1.16 (m, 3H), 1.13 - 0.86 (m, 3H). LCMS (ESI) calcd for $C_{32}H_{34}N_5O_4^+$ [M+H]$^+$: 552.26, found, 552.3.

**Example 433: preparation of (R)-1-(5-((4-benzhydryl-2-methylpiperazin-1-yl)methyl)-1-oxoisoindolin-2-yl)dihydropyrimidine-2,4(1H,3H)-dione (GT-06448)**

Referring to the method of Scheme 1, the target product (GT-06448) was prepared as a white solid (22 mg, yield 41 %). $^1$H NMR (400 MHz, DMSO-d6) δ 10.69 (s, 1H), 8.07 - 7.70 (m, 3H), 7.68 - 7.10 (m, 9H), 4.79 (d, *J* = 11.5 Hz, 2H), 4.59 - 4.27 (m, 2H), 3.87 - 3.72 (m, 4H), 3.58 (s, 3H), 3.08 (s, 2H), 2.98 - 2.70 (m, 4H), 1.43 (s, 2H). LCMS (ESI) calcd for $C_{31}H_{34}N_5O_3^+$ [M+H]$^+$: 524.27, found, 524.3.

**Example 434: preparation of (R)-5-((4-benzhydryl-2-methylpiperazin-1-yl)methyl)-2-(2,4-dioxotetrahydropyrimidin-1(2H)-yl)isoindoline-1,3-dione (GT-06449)**

Referring to the method of Scheme 1, the target product (GT-06449) was prepared as a white solid (25 mg, yield 48 %). $^1$H NMR (400 MHz, DMSO-d6) δ 10.85 (s, 1H), 8.46 - 7.83 (m, 3H), 7.39 (s, 4H), 7.26 (d, *J* = 6.4 Hz, 4H), 7.17 (s, 2H), 4.83 (s, 1H), 4.54 - 4.01 (m, 2H), 3.74 (t, *J* = 6.8 Hz, 2H), 3.62 - 3.48 (m, 1H), 3.00 (d, *J* = 35.6 Hz, 2H), 2.91 - 2.48 (m, 5H), 2.26 (s, 1H), 1.48 - 1.18 (m, 3H). LCMS (ESI) calcd for $C_{31}H_{32}N_5O_4^+$ [M+H]$^+$: 538.24, found, 538.3.

**Example 435: preparation of 1-(5-((5-benzhydryl-2,5-diazabicyclo[2.2.2]octan-2-yl)methyl)-1-oxoisoindolin-2-yl)dihydropyrimidine-2,4(1H,3H)-dione (GT-06450)**

Referring to the method of Scheme 1, the target product (GT-06450) was prepared as a white solid (25 mg, yield 46 %). $^1$H NMR (400 MHz, DMSO-d6) δ 10.69 (s, 1H), 8.21 - 7.92 (m, 3H), 7.85 (d, *J* = 7.7 Hz, 2H), 7.73 - 7.47 (m, 2H), 7.46 - 7.32 (m, 4H), 7.31 - 7.06 (m, 2H), 5.22 - 4.32 (m, 5H), 3.79 (t, *J* = 6.8 Hz, 2H), 3.47 (s, 3H), 3.24 - 3.07 (m, 1H), 3.06 - 2.55 (m, 4H), 2.45 - 1.50 (m, 4H). LCMS (ESI) calcd for $C_{32}H_{34}N_5O_3^+$ [M+H]$^+$: 536.27, found, 536.3.

**Example 436: preparation of 5-((5-benzhydryl-2,5-diazabicyclo[2.2.2]octan-2-yl)methyl)-2-(2,4-dioxotetrahydropyrimidin-1(2H)-yl)isoindoline-1,3-dione (GT-06451)**

Referring to the method of Scheme 1, the target product (GT-06451) was prepared as a white solid (24 mg, yield 45 %)$^1$H NMR (400 MHz, DMSO-d6) δ 10.85 (s, 1H), 8.29 (d, *J* = 50.5 Hz, 2H), 8.02 (d, *J* = 7.2 Hz, 2H), 7.61 - 7.38 (m, 3H), 7.36 - 7.13 (m, 6H), 4.73 (d, *J* = 91.4 Hz, 3H), 3.75 (t, *J* = 6.7 Hz, 2H), 3.58 (s, 1H), 3.17 - 2.88 (m, 2H), 2.85 - 2.65 (m, 3H), 2.31 - 1.99 (m, 2H), 1.91 - 1.35 (m, 2H). LCMS (ESI) calcd for $C_{32}H_{32}N_5O_4{}^+$ [M+H]$^+$: 550.24, found, 550.3.

**Example 437: preparation of 1-(5-((8-benzhydryl-3,8-diazabicyclo[3.2.1]octan-3-yl)methyl)-1-oxoisoindolin-2-yl)dihydropyrimidine-2,4(1H,3H)-dione (GT-06452)**

Referring to the method of Scheme 1, the target product (GT-06452) was prepared as a white solid (24 mg, yield 44 %). $^1$H NMR (400 MHz, DMSO-d6) δ 10.68 (s, 1H), 7.99 (s, 3H), 7.78 (s, 2H), 7.69 - 7.54 (m, 2H), 7.52 - 7.25 (m, 6H), 4.75 (d, 1H), 4.67 - 4.41 (m, 2H), 3.87 - 3.65 (m, 7H), 3.20 (d, *J* = 46.3 Hz, 3H), 2.93 - 2.74 (m, 3H), 2.34 (s, 1H), 2.25 - 2.05 (m, 2H). LCMS (ESI) calcd for $C_{32}H_{34}N_5O_3^+$ [M+H]$^+$: 536.27, found, 536.3.

**Example 438: preparation of 5-((8-benzhydryl-3,8-diazabicyclo[3.2.1]octan-3-yl)methyl)-2-(2,4-dioxotetrahydropyrimidin-1(2H)-yl)isoindoline-1,3-dione (GT-06453)**

Referring to the method of Scheme 1, the target product (GT-06453) was prepared as a white solid (30 mg, yield 57 %). $^1$H NMR (400 MHz, DMSO-d6) δ 10.84 (s, 1H), 7.92 (s, 1H), 7.92 - 7.78 (m, 4H), 7.47 (s, 1H), 7.44 - 7.35 (m, 3H), 7.35 - 7.28 (m, 2H), 7.27 - 7.21 (m, 1H), 7.15 (s, 1H), 3.74 (t, *J* = 6.7 Hz, 4H), 3.67 - 3.55 (m, 4H), 3.08 (s, 2H), 2.78 (t, *J* = 6.7 Hz, 2H), 2.64 (d, *J* = 25.4 Hz, 2H), 2.27 (s, 1H), 2.08 - 1.97 (m, 2H). LCMS (ESI) calcd for $C_{32}H_{32}N_5O_4^+$ [M+H]$^+$: 550.24, found, 550.3..

**Example 439: preparation of 1-(5-((3-benzhydryl-3,8-diazabicyclo[3.2.1]octan-8-yl)methyl)-1-oxoisoindolin-2-yl)dihydropyrimidine-2,4(1H,3H)-dione (GT-06454)**

Referring to the method of Scheme 1, the target product (GT-06454) was prepared as a white solid (27 mg, yield 50 %). $^1$H NMR (400 MHz, DMSO-d6) δ 10.68 (s, 2H), 7.96 (s, 1H), 7.90 - 7.79 (m, 2H), 7.51 - 7.46 (m, 4H), 7.31 (t, *J* = 7.5 Hz, 4H), 7.20 (t, *J* = 7.3 Hz, 2H), 4.80 - 4.72 (m, 1H), 4.55 (d, *J* = 16.3 Hz, 1H), 4.42 (s, 1H), 4.31 (d, *J* = 5.7 Hz, 2H), 3.84 - 3.74 (m, 4H), 2.87 - 2.72 (m, 2H), 2.66 (s, 4H), 2.34 - 2.21 (m, 4H). LCMS (ESI) calcd for $C_{32}H_{34}N_5O_3^+$ [M+H]$^+$: 536.27, found, 536.3.

**Example 440: preparation of 5-((3-benzhydryl-3,8-diazabicyclo[3.2.1]octan-8-yl)methyl)-2-(2,4-dioxotetrahydropyrimidin-1(2H)-yl)isoindoline-1,3-dione (GT-06455)**

Referring to the method of Scheme 1, the target product (GT-06455) was prepared as a white solid (19 mg, yield 36 %). $^1$H NMR (400 MHz, DMSO-d6) δ 10.92 (s, 1H), 10.57 (s, 1H), 8.36 (s, 1H), 8.24 (d, *J* = 7.7 Hz, 1H), 8.09 (d, *J* = 7.7 Hz, 1H), 7.47 (d, *J* = 7.4 Hz, 4H), 7.31 (t, *J* = 7.6 Hz, 4H), 7.21 (t, *J* = 7.3 Hz, 2H), 4.50 - 4.30 (m, 3H), 3.87 - 3.75 (m, 4H), 2.85 (t, *J* = 6.8 Hz, 2H), 2.71 - 2.59 (m, 4H), 2.36 - 2.22 (m, 4H). LCMS (ESI) calcd for $C_{32}H_{32}N_5O_4^+$ [M+H]$^+$: 550.24, found, 550.3.

**Example 441: preparation of 1-(5-((3-((4'-chloro-5,5-dimethyl-3,4,5,6-tetrahydro-[1,1'-biphenyl]-2-yl) methyl)-3,6-diazabicyclo[3.1.1]heptan-6-yl)methyl)-1-oxoisoindolin-2-yl)dihydropyrimidine-2,4(1H,3H)-dione (GT-06456)**

Referring to the method of Scheme 1, the target product (GT-06456) was prepared as a white solid (23 mg, yield 39 %). $^1$H NMR (400 MHz, DMSO-d6) δ 10.62 (s, 1H), 9.27 (s, 1H), 7.91 - 7.56 (m, 3H), 7.32 (d, *J* = 8.3 Hz, 2H), 7.02 (d, *J* = 8.3 Hz, 2H), 4.72 (t, *J* = 12.9 Hz, 1H), 4.61 - 4.41 (m, 2H), 4.35 - 4.07 (m, 2H), 3.72 (t, *J* = 6.9 Hz, 2H), 3.43 (s, 2H), 3.20 - 3.10 (m, 1H), 3.00 (s, 1H), 2.85 - 2.65 (m, 4H), 2.27 - 2.06 (m, 4H), 2.05 - 1.85 (m, 3H), 1.35 (t, J = 6.1 Hz, 2H), 0.89 (d, 6H). LCMS (ESI) calcd for $C_{33}H_{39}ClN_5O_3^+$ [M+H]$^+$: 588.27 , found, 588.3.

**Example 442: preparation of 5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-2-(2,4-dioxotetrahydropyrimidin-1(2H)-yl)isoindoline-1,3-dione (GT-06457)**

Referring to the method of Scheme 1, the target product (GT-06457) was prepared as a white solid (18 mg, yield 31 %). $^1$H NMR (400 MHz, DMSO-d6) δ 10.85 (s, 1H), 9.32 (s, 1H), 8.07 - 7.90 (m, 2H), 7.32 (d, *J* = 8.3 Hz, 2H), 7.03 (d, *J* = 8.3 Hz, 2H), 4.53 (s, 1H), 4.43 - 4.22 (m, 2H), 3.76 (t, *J* = 6.9 Hz, 2H), 3.62 - 3.40 (m, 3H), 2.79 (t, *J* = 6.7 Hz, 4H), 2.28 - 2.00 (m, 6H), 1.93 (s, 2H), 1.36 (t, *J* = 6.2 Hz, 2H), 0.89 (d, *J* = 2.8 Hz, 6H). LCMS (ESI) calcd for $C_{33}H_{37}ClN_5O_4^+$ [M+H]$^+$: 602.25, found, 602.3.

**Example 443: preparation of 1-(5-((6-((4'-chloro-5,5-dimethyl-3,4,5,6-tetrahydro-[1,1'-biphenyl]-2-yl) methyl)-3,6-diazabicyclo[3.1.1]heptan-3-yl)methyl)-1-oxoisoindolin-2-yl)dihydropyrimidine-2,4(1H,3H)-dione (GT-06458)**

Referring to the method of Scheme 1, the target product (GT-06458) was prepared as a white solid (12 mg, yield 20 %). $^1$H NMR (400 MHz, DMSO-d6) δ 10.60 (s, 1H), 9.10 (s, 1H), 7.68 (d, *J* = 7.3 Hz, 1H), 7.57 - 7.27 (m, 4H), 7.23 - 7.09 (m, 2H), 4.58 (dd, *J* = 90.7, 16.0 Hz, 3H), 4.12 (d, *J* = 57.7 Hz, 1H), 3.80 - 3.66 (m, 4H), 3.60 (s, 3H), 3.40 (s, 1H), 2.82 - 2.69 (m, 2H), 2.63 - 2.51 (m, 2H), 2.23 - 2.09 (m, 3H), 1.97 (d, *J* = 12.3 Hz, 3H), 1.37 (s, 2H), 0.89 (d, *J* = 5.2 Hz, 6H). LCMS (ESI) calcd for $C_{33}H_{39}ClN_5O_3^+$ [M+H]$^+$: 588.27, found, 588.3.

**Example 444: preparation of 5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-2-(2,4-dioxotetrahydropyrimidin-1(2H)-yl)isoindoline-1,3-dione (GT-06459)**

Referring to the method of Scheme 1, the target product (GT-06459) was prepared as a white solid (17 mg, yield 30 %). $^1$H NMR (400 MHz, DMSO-d6) δ 10.91 (s, 1H), 9.56 - 8.74 (m, 1H), 7.97 (d, *J* = 4.5 Hz, 2H), 7.88 - 7.69 (m, 1H), 7.57 - 7.32 (m, 3H), 7.27 - 7.17 (m, 2H), 4.18 (d, *J* = 70.8 Hz, 3H), 3.87 - 3.73 (m, 5H), 3.51 (s, 1H), 2.86 (t, *J* = 6.7 Hz, 2H), 2.76 - 2.64 (m, 2H), 2.30 - 2.12 (m, 4H), 2.04 (d, *J* = 11.7 Hz, 3H), 1.45 (s, 2H), 0.98 - 0.95 (m, 6H). LCMS (ESI) calcd for $C_{33}H_{37}ClN_5O_4^+$ [M+H]$^+$: 602.25, found, 602.3.

**Example 445: preparation of 2-(2,4-dioxotetrahydropyrimidin-1(2H)-yl)-5-((4-phenylpiperazin-1-yl)methyl)isoindoline-1,3-dione (GT-06719)**

Referring to the method of Scheme 1, the target product (GT-06719) was prepared as a white solid (23 mg, yield 43 %). $^1$H NMR (400 MHz, DMSO-d6) δ 11.54 (s, 1H), 10.99 (s, 1H), 8.40 (s, 1H), 8.27 (d, *J* = 7.8, 1.2 Hz, 1H), 8.18 (d, *J* = 7.7 Hz, 1H), 7.36 - 7.29 (m, 2H), 7.04 (d, *J* = 8.0 Hz, 2H), 6.92 (t, *J* = 7.3 Hz, 1H), 4.68 (s, 2H), 3.89 (t, *J* = 6.8 Hz, 4H), 3.49 - 3.44 (m, 2H), 3.25 (s, 4H), 2.92 (t, *J* = 6.8 Hz, 2H). LCMS (ESI) calcd for $C_{23}H_{24}N_5O_4^+$ [M+H]$^+$: 434.18, found, 434.0.

**Example 446: preparation of 2-(2,4-dioxotetrahydropyrimidin-1(2H)-yl)-5-((4-(2-methoxyphenyl)piperazin-1-yl)methyl)isoindoline-1,3-dione (GT-06720)**

Referring to the method of Scheme 1, the target product (GT-06720) was prepared as a white solid (22 mg, yield 39 %). $^1$H NMR (400 MHz, DMSO-d6) δ 11.26 (s, 1H), 10.86 (s, 1H), 8.27 (s, 1H), 8.14 (dd, *J* = 7.7, 1.2 Hz, 1H), 8.05 (d, *J* = 7.7 Hz, 1H), 6.98 - 6.90 (m, 2H), 6.88 - 6.81 (m, 2H), 4.55 (s, 1H), 3.81 - 3.69 (m, 6H), 3.40 (s, 2H), 3.34 (d, *J* = 11.2 Hz, 2H), 3.18 (s, 2H), 2.99 (t, *J* = 11.9 Hz, 2H), 2.79 (t, *J* = 6.8 Hz, 2H). LCMS (ESI) calcd for $C_{24}H_{26}N_5O_5^+$ [M+H]$^+$: 464.19, found, 464.0.

**Example 447: preparation of 2-(2,4-dioxotetrahydropyrimidin-1(2H)-yl)-5-((4-(3-methoxyphenyl)piperazin-1-yl)methyl)isoindoline-1,3-dione (GT-06721)**

Referring to the method of Scheme 1, the target product (GT-06721) was prepared as a white solid (25 mg, yield 44 %). $^1$H NMR (400 MHz, DMSO-d6) δ 11.29 (s, 1H), 10.86 (s, 1H), 8.26 (s, 1H), 8.12 (d, *J* = 7.7, 1.3 Hz, 1H), 8.05 (d, *J* = 7.7 Hz, 1H), 7.08 (d, *J* = 8.2 Hz, 1H), 6.49 (dd, *J* = 8.2, 2.0 Hz, 1H), 6.44 (t, *J* = 2.2 Hz, 1H), 6.38 (dd, *J* = 8.1, 2.1 Hz, 1H), 4.54 (s, 2H), 3.76 (t, *J* = 6.8 Hz, 4H), 3.65 (s, 3H), 3.34 - 3.29 (m, 2H), 3.14 - 3.04 (m, 4H), 2.79 (t, *J* = 6.8 Hz, 2H). LCMS (ESI) calcd for $C_{24}H_{26}N_5O_5^+$ [M+H]$^+$: 464.19 , found, 464.0.

**Example 448: preparation of 5-((4-(3-bromophenyl)piperazin-1-yl)methyl)-2-(2,4-dioxotetrahydropyrimidin-1(2H)-yl)isoindoline-1,3-dione (GT-06722)**

Referring to the method of Scheme 1, the target product (GT-06722) was prepared as a white solid (28 mg, yield 45 %). $^1$H NMR (400 MHz, DMSO-d6) δ 11.25 (s, 1H), 10.85 (s, 1H), 8.24 (s, 1H), 8.15 - 8.00 (m, 2H), 7.13 - 7.08 (m, 2H), 6.94 - 6.88 (m, 2H), 4.54 (s, 2H), 3.84 - 3.74 (m, 4H), 3.33 - 3.28 (m, 2H), 3.11 (s, 4H), 2.79 (t, *J* = 6.8 Hz, 2H). LCMS (ESI) calcd for $C_{23}H_{23}BrN_5O_4^+$ [M+H]$^+$: 512.09, found, 512.1.

**Example 449: preparation of 2-(2,4-dioxotetrahydropyrimidin-1(2H)-yl)-5-((4-(4-(trifluoromethyl)phenyl)piperazin-1-yl)methyl)isoindoline-1,3-dione (GT-06723)**

Referring to the method of Scheme 1, the target product (GT-06723) was prepared as a white solid (16 mg, yield 26 %). $^1$H NMR (400 MHz, DMSO-d6) δ 11.40 (s, 1H), 10.86 (s, 1H), 8.23 (s, 1H), 8.06 (t, *J = 10.2* Hz, 2H), 7.50 (d, *J* = 8.7 Hz, 2H), 7.06 (d, *J* = 8.6 Hz, 2H), 4.67 - 4.37 (m, 2H), 3.94 (s, 2H), 3.76 (t, *J* = 6.8 Hz, 2H), 3.32 (s, 2H), 3.21 - 3.03 (m, 4H), 2.80 (t, 2H). LCMS (ESI) calcd for $C_{24}H_{23}F_3N_5O_4^+$ [M+H]$^+$: 502.17, found, 502.0.

**Example 450: preparation of 2-(2,4-dioxotetrahydropyrimidin-1(2H)-yl)-5-((4-(3-fluorophenyl)piperazin-1-yl)methyl)isoindoline-1,3-dione (GT-06724)**

Referring to the method of Scheme 1, the target product (GT-06724) was prepared as a white solid (27 mg, yield 49 %). $^1$H NMR (400 MHz, DMSO-d6) δ 11.49 (s, 1H), 10.94 (s, 1H), 8.33 (s, 1H), 8.19 (d, *J* = 7.8 Hz, 1H), 8.12 (d, *J* = 7.6 Hz, 1H), 7.27 (q, *J* = 7.9 Hz, 1H), 6.84 - 6.78 (m, 2H), 6.63 (t, *J* = 8.3 Hz, 1H), 4.61 (s, 2H), 3.91 - 3.82 (m, 4H), 3.41 (s, 2H), 3.19

(s, 4H), 2.87 (t, *J* = 6.7 Hz, 2H). LCMS (ESI) calcd for $C_{23}H_{23}FN_5O_4$ $^+$ [M+H]$^+$: 452.17, found, 452.0.

**Example 451: preparation of 2-(2,4-dioxotetrahydropyrimidin-1(2H)-yl)-5-((4-(4-fluorophenyl)piperazin-1-yl)methyl)isoindoline-1,3-dione (GT-06725)**

Referring to the method of Scheme 1, the target product (GT-06725) was prepared as a white solid (33 mg, yield 59 %). $^1$H NMR (400 MHz, DMSO-d6) δ 11.41 (s, 1H), 10.86 (s, 1H), 8.27 (s, 1H), 8.13 (d, *J* = 7.8 Hz, 1H), 8.05 (d, *J* = 7.7 Hz, 1H), 7.03 (t, *J* = 8.8 Hz, 2H), 6.97 - 6.90 (m, 2H), 4.54 (s, 2H), 3.76 (t, *J* = 6.7 Hz, 2H), 3.65 (d, *J* = 10.0 Hz, 2H), 3.36 - 3.31 (m, 2H), 3.17 - 3.03 (m, 4H), 2.79 (t, *J* = 6.7 Hz, 2H).. LCMS (ESI) calcd for $C_{23}H_{23}FN_5O_4^+$ [M+H]$^+$: 452.17, found, 452.0.

**Example 452: preparation of 5-((4-(2-chlorophenyl)piperazin-1-yl)methyl)-2-(2,4-dioxotetrahydropyrimidin-1(2H)-yl)isoindoline-1,3-dione (GT-06726)**

Referring to the method of Scheme 1, the target product (GT-06726) was prepared as a white solid (35 mg, yield 55 %). $^1$H NMR (400 MHz, DMSO-d6) δ 11.20 (s, 1H), 10.94 (s, 1H), 8.34 (s, 1H), 8.20 (d, *J* = 7.8 Hz, 1H), 8.13 (d, *J* = 7.6 Hz, 1H), 7.46 (d, *J* = 7.9 Hz, 1H), 7.34 (t, *J* = 7.5 Hz, 1H), 7.21 (d, *J* = 7.9 Hz, 1H), 7.12 (t, *J* = 7.7 Hz, 1H), 4.60 (d, *J* = 36.7 Hz, 2H), 3.83 (t, *J* = 6.7 Hz, 2H), 3.45 (s, 2H), 3.33 - 3.25 (m, 4H), 3.19 - 3.13 (m, 2H), 2.87 (t, *J* = 6.6 Hz, 2H). LCMS (ESI) calcd for $C_{23}H_{23}ClN_5O_4^+$ [M+H]$^+$: 468.14, found, 468.0.

**Example 453: preparation of 5-((4-(4-chlorophenyl)piperazin-1-yl)methyl)-2-(2,4-dioxotetrahydropyrimidin-1(2H)-yl)isoindoline-1,3-dione (GT-06783)**

Referring to the method of Scheme 1, the target product (GT-06783) was prepared as a white solid (26 mg, yield 45 %). $^1$H NMR (400 MHz, DMSO-d6) δ 11.49 (s, 1H), 10.93 (s, 1H), 8.33 (s, 1H), 8.20 (d, *J* = 7.6 Hz, 1H), 8.12 (d, *J* = 7.7 Hz, 1H), 7.29 (d, *J* = 8.7 Hz, 2H), 7.00 (d, *J* = 8.8 Hz, 2H), 4.57 (d, *J* = 36.5 Hz, 2H), 3.83 (t, *J* = 6.6 Hz, 4H), 3.40 (s, 2H), 3.19 (s, 4H), 2.86 (t, *J* = 6.6 Hz, 2H). LCMS (ESI) calcd for $C_{23}H_{23}ClN_5O_4^+$ [M+H]$^+$: 468.14, found, 468.1.

**Example 454: preparation of 5-((4-(2-bromophenyl)piperazin-1-yl)methyl)-2-(2,4-dioxotetrahydropyrimidin-1(2H)-yl)isoindoline-1,3-dione (GT-06727)**

Referring to the method of Scheme 1, the target product (GT-06727) was prepared as a white solid (7 mg, yield 11 %). $^1$H NMR (400 MHz, DMSO-d6) δ 11.39 (s, 1H), 10.93 (s, 1H), 8.34 (d, *J* = 13.1 Hz, 1H), 8.26 - 8.10 (m, 2H), 7.62 (t, *J* = 11.7 Hz, 1H), 7.39 (t, *J* = 7.7 Hz, 1H), 7.21 (d, *J* = 7.7 Hz, 1H), 7.06 (t, *J* = 7.6 Hz, 1H), 4.66 (s, 2H), 3.83 (t, *J* = 6.7 Hz, 2H), 3.47 (s, 2H), 3.27 (s, 4H), 3.19 (d, *J* = 10.9 Hz, 2H), 2.87 (t, *J* = 6.7 Hz, 2H). LCMS (ESI) calcd for $C_{23}H_{23}BrN_5O_4^+$ [M+H]$^+$: 512.09, found, 512.2.

**Example 455: preparation of 2-(2,4-dioxotetrahydropyrimidin-1(2H)-yl)-5-((4-(4-methoxyphenyl)piperazin-1-yl)methyl)isoindoline-1,3-dione (GT-06728)**

Referring to the method of Scheme 1, the target product (GT-06728) was prepared as a white solid (24 mg, yield 42 %). $^1$H NMR (400 MHz, DMSO-d6) δ 11.25 (s, 1H), 10.94 (s, 1H), 8.33 (s, 1H), 8.19 (d, *J* = 8.8 Hz, 1H), 8.12 (d, *J* = 7.7 Hz, 1H), 6.95 (d, *J* = 9.0 Hz, 2H), 6.86 (d, *J* = 9.1 Hz, 2H), 4.57 (s, 2H), 3.83 *(t, J* = 6.8 Hz, 2H), 3.70 - 3.63 (m, 4H), 3.41 - 3.36 (m, 2H), 3.23 (s, 3H), 3.07 (s, 2H), 2.87 (t, *J* = 6.8 Hz, 2H). LCMS (ESI) calcd for $C_{24}H_{26}N_5O_5^+$ [M+H]$^+$: 464.19, found, 464.2.

**Example 456: preparation of 5-((4-(4-bromophenyl)piperazin-1-yl)methyl)-2-(2,4-dioxotetrahydropyrimidin-1(2H)-yl)isoindoline-1,3-dione (GT-06784)**

Referring to the method of Scheme 1, the target product (GT-06784) was prepared as a white solid (29 mg, yield 46 %). $^1$H NMR (400 MHz, DMSO-d6) δ 11.44 (s, 1H), 10.86 (s, 1H), 8.26 (s, 1H), 8.09 (dd, *J* = 30.9, 7.7 Hz, 2H), 7.34 (d, *J* = 9.0 Hz, 2H), 6.88 (d, *J* = 9.1 Hz, 2H), 4.49 (s, 2H), 3.76 (t, *J* = 6.7 Hz, 4H), 3.33 (s, 2H), 3.12 (s, 4H), 2.79 (t, *J* = 6.8 Hz, 2H). LCMS (ESI) calcd for $C_{23}H_{23}BrN_5O_4^+$ [M+H]$^+$: 512.09, found, 512.1.

**Example 457: preparation of 2-(2,4-dioxotetrahydropyrimidin-1(2H)-yl)-5-((4-(o-tolyl)piperazin-1-yl)methyl)isoindoline-1,3-dione (GT-06785)**

Referring to the method of Scheme 1, the target product (GT-06785) was prepared as a white solid (29 mg, yield 53 %). $^1$H NMR (400 MHz, DMSO-d6) δ 11.34 (s, 1H), 10.86 (s, 1H), 8.29 (s, 1H), 8.16 (d, *J* = 7.8 Hz, 1H), 8.05 (d, *J* = *7.7* Hz, 1H), 7.11 (t, *J* = 8.1 Hz, 2H), 6.99 - 6.91 (m, 2H), 4.57 (s, 2H), 3.76 (t, *J* = *6.8* Hz, 2H), 3.33 (d, *J* = 11.0 Hz, 2H), 3.21 (s, 2H),

3.15 - 3.00 (m, 4H), 2.79 (t, *J* = 6.8 Hz, 2H), 2.19 (s, 3H). LCMS (ESI) calcd for $C_{24}H_{26}N_5O_4^+$ [M+H]$^+$: 448.20, found, 448.2.

**Example 458: preparation of 2-(2,4-dioxotetrahydropyrimidin-1(2H)-yl)-5-((4-(m-tolyl)piperazin-1-yl)methyl)isoindoline-1,3-dione (GT-06786)**

Referring to the method of Scheme 1, the target product (GT-06786) was prepared as a white solid (31 mg, yield 56 %). $^1$H NMR (400 MHz, DMSO-d6) δ 11.33 (s, 1H), 10.85 (s, 1H), 8.26 (s, 1H), 8.13 (d, *J* = 8.7 Hz, 1H), 8.05 (d, *J* = 7.7 Hz, 1H), 7.07 (t, *J* = 7.8 Hz, 1H), 6.76 - 6.68 (m, 2H), 6.61 (d, *J* = 7.5 Hz, 1H), 4.54 (s, 2H), 3.76 (t, *J* = 6.8 Hz, 4H), 3.32 (s, 2H), 3.10 (s, 4H), 2.79 (t, *J* = 6.8 Hz, 2H), 2.19 (s, 3H).LCMS (ESI) calcd for $C_{24}H_{26}N_5O_4^+$ [M+H]$^+$: 448.20, found, 448.2.

**Example 459: preparation of 2-(2,4-dioxotetrahydropyrimidin-1(2H)-yl)-5-((4-(p-tolyl)piperazin-1-yl)methyl)isoindoline-1,3-dione (GT-06787)**

Referring to the method of Scheme 1, the target product (GT-06787) was prepared as a white solid (35 mg, yield 64 %). $^1$H NMR (400 MHz, DMSO-d6) δ 11.29 (s, 1H), 10.94 (s, 1H), 8.33 (s, 1H), 8.19 (d, 1H), 8.12 (d, *J* = 7.7 Hz, 1H), 7.08 (d, *J* = 8.4 Hz, 2H), 6.88 (d, *J* = 8.5 Hz, 2H), 4.61 (s, 2H), 3.83 (t, *J* = 6.8 Hz, 2H), 3.74 (d, *J* = 11.8 Hz, 2H), 3.41 (d, *J* = 11.2 Hz, 2H), 3.23 - 3.06 (m, 4H), 2.87 (t, *J* = 6.8 Hz, 2H), 2.22 (s, 3H). LCMS (ESI) calcd for $C_{24}H_{26}N_5O_4^+$ [M+H]$^+$: 448.20, found, 448.3.

**Example 460: preparation of 5-((4-(2,4-dichlorophenyl)piperazin-1-yl)methyl)-2-(2,4-dioxotetrahydropyrimidin-1(2H)-yl)isoindoline-1,3-dione (GT-06788)**

Referring to the method of Scheme 1, the target product (GT-06788) was prepared as a white solid (29 mg, yield 47 %). $^1$H NMR (400 MHz, DMSO) δ 11.06 (s, 1H), 10.86 (s, 1H), 8.20 - 7.97 (m, 2H), 7.53 (d, *J* = 2.3 Hz, 1H), 7.34 (dd, *J* = 8.7, 2.4 Hz, 1H), 7.15 (d, *J* = 8.7 Hz, 1H), 4.57 (s, 2H), 3.76 (t, *J* = 6.8 Hz, 2H), 3.36 (s, 2H), 3.25 - 3.14 (m, 4H), 3.11 - 3.01 (m, 2H), 2.79 (t, *J* = 6.8 Hz, 2H). LCMS (ESI) calcd for $C_{23}H_{22}Cl_2N_5O_4^+$ [M+H]$^+$: 502.10, found, 502.1.

**Example 461: preparation of 5-((4-(2,5-dichlorophenyl)piperazin-1-yl)methyl)-2-(2,4-dioxotetrahydropyrimidin-1(2H)-yl)isoindoline-1,3-dione (GT-06789)**

Referring to the method of Scheme 1, the target product (GT-06789) was prepared as a white solid (34 mg, yield 55 %). $^1$H NMR (400 MHz, DMSO) δ 11.13 (s, 1H), 10.86 (s, 1H), 8.42 - 7.90 (m, 3H), 7.41 (d, *J* = 8.5 Hz, 1H), 7.20 - 7.07 (m, 2H), 4.57 (s, 2H), 3.76 (t, *J* = 6.8 Hz, 2H), 3.49 - 3.34 (m, 4H), 3.20 - 3.03 (m, 4H), 2.79 (t, *J* = 6.8 Hz, 2H). LCMS (ESI) calcd for $C_{23}H_{22}Cl_2N_5O_4^+$ [M+H]$^+$: 502.10, found, 502.1.

**Example 462: preparation of 5-((4-(2,6-dichlorophenyl)piperazin-1-yl)methyl)-2-(2,4-dioxotetrahydropyrimidin-1(2H)-yl)isoindoline-1,3-dione (GT-06790)**

Referring to the method of Scheme 1, the target product (GT-06790) was prepared as a white solid (25 mg, yield 41 %). $^1$H NMR (400 MHz, DMSO) δ 10.70 (d, *J* = 126.9 Hz, 2H), 8.27 - 8.05 (m, 2H), 7.47 - 7.33 (m, 2H), 7.16 (t, *J* = 8.1 Hz, 1H), 4.54 (d, *J* = 38.1 Hz, 2H), 3.81 - 3.60 (m, 4H), 3.24 - 3.11 (m, 4H), 3.10 - 3.02 (m, 2H), 2.79 (t, J= 6.8 Hz, 2H). LCMS (ESI) calcd for $C_{23}H_{22}Cl_2N_5O_4^+$ [M+H]$^+$: 502.10, found, 502.2.

**Example 463: preparation of 5-((4-(3,4-dichlorophenyl)piperazin-1-yl)methyl)-2-(2,4-dioxotetrahydropyrimidin-1(2H)-yl)isoindoline-1,3-dione (GT-06791)**

Referring to the method of Scheme 1, the target product (GT-06791) was prepared as a white solid (30 mg, yield 49 %). $^1$H NMR (400 MHz, DMSO) δ 11.23 (s, 1H), 10.86 (s, 1H), 8.34 - 7.91 (m, 3H), 7.39 (d, *J* = 9.0 Hz, 1H), 7.16 (d, *J* = 2.7 Hz, 1H), 6.91 (dd, *J* = 9.0, 2.9 Hz, 1H), 4.67 - 4.29 (m, 2H), 3.92 - 3.68 (m, 4H), 3.35 (s, 2H), 3.12 (s, 4H), 2.79 (t, *J* = 6.8 Hz, 2H). LCMS (ESI) calcd for $C_{23}H_{22}Cl_2N_5O_4^+$ [M+H]$^+$: 502.10 , found, 502.2.

**Example 464: preparation of 5-((4-(3,5-dichlorophenyl)piperazin-1-yl)methyl)-2-(2,4-dioxotetrahydropyrimidin-1(2H)-yl)isoindoline-1,3-dione (GT-06792)**

Referring to the method of Scheme 1, the target product (GT-06792) was prepared as a white solid (32 mg, yield 52 %). $^1$H NMR (400 MHz, DMSO) δ 11.21 (s, 1H), 10.86 (s, 1H), 8.42 - 7.87 (m, 3H), 6.97 - 6.83 (m, 3H), 4.52 (s, 2H), 4.03 - 3.67 (m, 4H), 3.44 - 3.32 (m, 2H), 3.15 (d, *J* = 31.9 Hz, 4H), 2.79 (t, *J* = 6.8 Hz, 2H). LCMS (ESI) calcd for $C_{23}H_{22}Cl_2N_5O_4^+$ [M+H]$^+$: 502.10, found, 502.1.

**Example 465: preparation of 5-((4-(2,3-difluorophenyl)piperazin-1-yl)methyl)-2-(2,4-dioxotetrahydropyrimidin-1(2H)-yl)isoindoline-1,3-dione (GT-06793)**

Referring to the method of Scheme 1, the target product (GT-06793) was prepared as a white solid (25 mg, yield 43 %). $^1$H NMR (400 MHz, DMSO) δ 11.29 (s, 1H), 10.92 (s, 1H), 8.44 - 7.95 (m, 3H), 7.24 - 7.01 (m, 2H), 6.93 (t, *J* = 7.8 Hz, 1H), 4.63 (s, 2H), 3.83 (t, *J* = 6.8 Hz, 2H), 3.53 (s, 4H), 3.30 - 3.16 (m, 4H), 2.87 (t, *J* = 12.8, 6.1 Hz, 2H). LCMS (ESI) calcd for $C_{23}H_{22}F_2N_5O_4^+$ [M+H]$^+$: 470.16, found, 470.1.

**Example 466: preparation of 5-((4-(2,4-difluorophenyl)piperazin-1-yl)methyl)-2-(2,4-dioxotetrahydropyrimidin-1(2H)-yl)isoindoline-1,3-dione (GT-06794)**

Referring to the method of Scheme 1, the target product (GT-06794) was prepared as a white solid (35 mg, yield 61 %). $^1$H NMR (400 MHz, DMSO) δ 11.35 (s, 1H), 10.93 (s, 1H), 8.33 (s, 1H), 8.20 (d, *J* = 7.8, 1.3 Hz, 1H), 8.13 (d, *J* = 7.6 Hz, 1H), 7.31 - 7.21 (m, 1H), 7.21 - 7.11 (m, 1H), 7.10 - 6.98 (m, 1H), 4.63 (s, 2H), 3.83 (t, *J* = 6.8 Hz, 2H), 3.68 - 3.51 (m, 2H), 3.39 - 3.10 (m, 6H), 2.87 (t, *J* = 6.8 Hz, 2H). LCMS (ESI) calcd for $C_{23}H_{22}F_2N_5O_4^+$ [M+H]$^+$: 470.16, found, 470.2.

**Example 467: preparation of 5-((4-(2,5-difluorophenyl)piperazin-1-yl)methyl)-2-(2,4-dioxotetrahydropyrimidin-1(2H)-yl)isoindoline-1,3-dione (GT-06795)**

Referring to the method of Scheme 1, the target product (GT-06795) was prepared as a white solid (26 mg, yield 50 %). $^1$H NMR (400 MHz, DMSO) δ 11.25 (s, 1H), 10.92 (s, 1H), 8.38 - 7.94 (m, 3H), 7.30 - 7.15 (m, 1H), 6.99 (t, *J* = 7.3 Hz, 1H), 6.92 - 6.78 (m, 1H), 4.62 (s, 2H), 3.83 (t, *J* = 6.8 Hz, 2H), 3.54 (s, 2H), 3.33 - 3.08 (m, 6H), 2.92 - 2.83 (m, 2H). LCMS (ESI) calcd for $C_{23}H_{22}F_2N_5O_4^+$ [M+H]$^+$: 470.16, found, 470.3.

**Example 468: preparation of 5-((4-(2,6-difluorophenyl)piperazin-1-yl)methyl)-2-(2,4-dioxotetrahydropyrimidin-1(2H)-yl)isoindoline-1,3-dione (GT-06796)**

Referring to the method of Scheme 1, the target product (GT-06796) was prepared as a white solid (19 mg, yield 33 %). $^1$H NMR (400 MHz, DMSO) δ 11.22 (s, 1H), 10.92 (s, 1H), 8.33 (s, 1H), 8.19 (d, *J* = 7.7, 1.3 Hz, 1H), 8.13 (d, *J* = 7.6 Hz, 1H), 7.28 - 7.02 (m, 3H), 4.63 (s, 2H), 3.83 (t, *J* = 6.8 Hz, 2H), 3.61 - 3.48 (m, 4H), 3.34 - 3.21 (m, 4H), 2.87 (t, *J* = 6.8 Hz, 2H). LCMS (ESI) calcd for $C_{23}H_{22}F_2N_5O_4^+$ [M+H]$^+$: 470.16, found, 470.3.

**Example 469: preparation of 5-((4-(3,4-difluorophenyl)piperazin-1-yl)methyl)-2-(2,4-dioxotetrahydropyrimidin-1(2H)-yl)isoindoline-1,3-dione (GT-06797)**

Referring to the method of Scheme 1, the target product (GT-06797) was prepared as a white solid (32 mg, yield 56 %). $^1$H NMR (400 MHz, DMSO) δ 11.33 (s, 1H), 10.94 (s, 1H), 8.32 (s, 1H), 8.20 - 8.08 (m, 2H), 7.31 (dd, *J* = 19.8, 9.4 Hz, 1H), 7.16 - 7.04 (m, 1H), 6.79 (d, *J* = 9.2 Hz, 1H), 4.56 (d, *J* = 36.3 Hz, 2H), 3.83 (t, *J* = 6.8 Hz, 4H), 3.47 (s, 2H), 3.18 (s, 4H), 2.87 (t, *J* = 6.8 Hz, 2H). LCMS (ESI) calcd for $C_{23}H_{22}F_2N_5O_4^+$ [M+H]$^+$: 470.16, found, 470.2.

**Example 470: preparation of 3-((4-(3,5-difluorophenyl)piperazin-1-yl)methyl)-2-(2,4-dioxotetrahydropyrimidin-1(2H)-yl)isoindoline-1,3-dione (GT-06798)**

Referring to the method of Scheme 1, the target product (GT-06798) was prepared as a white solid (23 mg, yield 40 %). $^1$H NMR (400 MHz, DMSO-d6) δ 11.07 (s, 1H), 10.86 (s, 1H), 8.28 - 7.76 (m, 3H), 6.68 - 6.43 (m, 3H), 4.52 (s, 2H), 4.01 - 3.63 (m, 4H), 3.37 (d, *J* = 7.1 Hz, 2H), 3.18 - 3.03 (m, 4H), 2.79 (t, *J* = 6.7 Hz, 2H). LCMS (ESI) calcd for $C_{23}H_{22}F_2N_5O_4^+$ [M+H]$^+$: 470.16, found, 470.2.

**Example 471: preparation of 5-((4-(2,4-dimethylphenyl)piperazin-1-yl)methyl)-2-(2,4-dioxotetrahydropyrimidin-1(2H)-yl)isoindoline-1,3-dione (GT-06801)**

Referring to the method of Scheme 1, the target product (GT-06801) was prepared as a white solid (28 mg, yield 49 %). $^1$H NMR (400 MHz, DMSO-d6) δ 11.26 (s, 1H), 10.93 (s, 1H), 8.35 (s, 1H), 8.21 (d, *J* = 7.7 Hz, 1H), 8.13 (d, *J* = 7.7 Hz, 1H), 7.02 - 6.88 (m, 3H), 4.64 (s, 2H), 3.83 (t, *J* = 6.6 Hz, 2H), 3.40 (d, *J* = 10.0 Hz, 2H), 3.25 (s, 2H), 3.16 - 3.05 (m, 4H), 2.86 (t, *J* = 6.7 Hz, 2H), 2.22 (s, 6H). LCMS (ESI) calcd for $C_{25}H_{28}N_5O_4^+$ [M+H]$^+$: 462.21, found, 462.3.

**Example 472: preparation of 5-((4-(5-chloro-2-methylphenyl)piperazin-1-yl)methyl)-2-(2,4-dioxotetrahydropyrimidin-1(2H)-yl)isoindoline-1,3-dione (GT-06802)**

Referring to the method of Scheme 1, the target product (GT-06802) was prepared as a white solid (24 mg, yield 41 %). $^1$H NMR (400 MHz, DMSO-d6) δ 11.05 (s, 1H), 10.94 (s, 1H), 8.32 (s, 1H), 8.19 (d, *J* = 7.7 Hz, 1H), 8.13 (d, *J* = 7.7 Hz, 1H), 7.22 (d, *J* = 8.1 Hz, 1H), 7.09 - 7.04 (m, 2H), 4.64 (s, 2H), 3.83 (t, *J* = 6.8 Hz, 2H), 3.46 - 3.42 (m, 2H), 3.29 - 3.21 (m, 4H), 3.14 - 3.05 (m, 2H), 2.87 (t, *J* = 6.8 Hz, 2H), 2.23 (s, 3H). LCMS (ESI) calcd for $C_{24}H_{25}ClN_5O_4^+$ $[M+H]^+$: 482.16, found, 482.2.

**Example 473: preparation of 2-(2,4-dioxotetrahydropyrimidin-1(2H)-yl)-5-((4-phenylpiperidin-1-yl)methyl)isoindoline-1,3-dione (GT-06803)**

Referring to the method of Scheme 1, the target product (GT-06803) was prepared as a white solid (21 mg, yield 40 %). $^1$H NMR (400 MHz, DMSO-d6) δ 10.93 (s, 1H), 10.85 (s, 1H), 8.32 (s, 1H), 8.23 - 7.97 (m, 2H), 7.36 - 7.31 (m, 2H), 7.26 - 7.21 (m, 3H), 4.56 (s, 2H), 3.83 (t, *J* = 6.8 Hz, 2H), 3.46 (s, 2H), 3.18 - 3.04 (m, 2H), 2.90 - 2.78 (m, 3H), 2.07 - 1.92 (m, 4H). LCMS (ESI) calcd for $C_{24}H_{25}N_4O_4^+$ $[M+H]^+$: 433.19, found, 433.3.

**Example 474: preparation of 5-((4-(3-bromophenyl)piperidin-1-yl)methyl)-2-(2,4-dioxotetrahydropyrimidin-1(2H)-yl)isoindoline-1,3-dione (GT-06804)**

Referring to the method of Scheme 1, the target product (GT-06804) was prepared as a white solid (26 mg, yield 42 %). $^1$H NMR (400 MHz, DMSO-d6) δ 10.93 (s, 2H), 8.30 (s, 1H), 8.27 - 7.99 (m, 2H), 7.47 - 7.39 (m, 2H), 7.32 (t, *J* = 7.7 Hz, 1H), 7.25 (d, *J* = 7.8 Hz, 1H), 4.61 (s, 2H), 3.83 (t, *J* = 6.8 Hz, 2H), 3.51 - 3.40 (m, 2H), 3.07 (d, *J* = 10.3 Hz, 2H), 2.84 (t, 3H), 2.08 - 1.86 (m, 4H). LCMS (ESI) calcd for $C_{24}H_{24}BrN_4O_4^+$ $[M+H]^+$: 511.10 , found, 511.1.

**Example 475: preparation of 5-((4-(4-bromophenyl)piperidin-1-yl)methyl)-2-(2,4-dioxotetrahydropyrimidin-1(2H)-yl)isoindoline-1,3-dione (GT-08083)**

Referring to the method of Scheme 1, the target product (GT-06805) was prepared as a white solid (32 mg, yield 51 %). $^1$H NMR (400 MHz, DMSO-d6) δ 11.08 - 10.55 (m, 2H), 8.33 (s, 1H), 8.28 - 7.93 (m, 2H), 7.53 (d, *J* = 8.4 Hz, 2H), 7.20 (d, *J* = 8.5 Hz, 2H), 4.51 (s, 2H), 3.83 (t, *J* = 6.8 Hz, 2H), 3.52 - 3.42 (m, 2H), 3.07 (d, *J* = 10.0 Hz, 2H), 2.89 - 2.77 (m, 3H), 2.14 - 1.93 (m, 4H). LCMS (ESI) calcd for $C_{24}H_{24}BrN_4O_4^+$ $[M+H]^+$: 511.10 , found, 511.2.

**Example 476: preparation of 5-((4-(2-chlorophenyl)piperidin-1-yl)methyl)-2-(2,4-dioxotetrahydropyrimidin-1(2H)-yl)isoindoline-1,3-dione (GT-06875)**

Referring to the method of Scheme 1, the target product (GT-06875) was prepared as a white solid (13 mg, yield 45 %). $^1$H NMR (400 MHz, DMSO-d6) δ 11.07 - 10.78 (m, 2H), 8.24 (s, 1H), 8.15 - 7.99 (m, 2H), 7.38 (d, *J* = 7.9 Hz, 1H), 7.33 - 7.19 (m, 3H), 4.44 (s, 2H), 3.75 (t, 2H), 3.43 - 3.35 (m, 2H), 3.14 (d, *J* = 11.4 Hz, 3H), 2.79 (t, *J* = 6.7 Hz, 2H), 2.04 - 1.82 (m, 4H). LCMS (ESI) calcd for $C_{24}H_{24}ClN_4O_4$ $^+$ $[M+H]^+$: 467.15, found, 467.2.

**Example 477: preparation of 5-((4-(4-chlorophenyl)piperidin-1-yl)methyl)-2-(2,4-dioxotetrahydropyrimidin-1(2H)-yl)isoindoline-1,3-dione (GT-06876)**

Referring to the method of Scheme 1, the target product was prepared as a white solid (GT-06876) (18 mg, yield 31 %). $^1$H NMR (400 MHz, DMSO-d6) δ 10.86 (s, 2H), 8.24 (s, 1H), 8.15 - 7.91 (m, 2H), 7.33 (d, *J* = 8.4 Hz, 2H), 7.18 (d, *J* = 8.5 Hz, 2H), 4.44 (s, 2H), 3.76 (t, *J* = 6.8 Hz, 2H), 3.38 (s, 2H), 3.00 (s, 2H), 2.79 (t, *J* = 6.8 Hz, 3H), 2.07 - 1.87 (m, 4H). LCMS (ESI) calcd for $C_{24}H_{24}ClN_4O_4^+$ $[M+H]^+$: 467.15, found, 467.2.

**Example 478: preparation of 2-(2,4-dioxotetrahydropyrimidin-1(2H)-yl)-5-((4-(2-fluorophenyl)piperidin-1-yl)methyl)isoindoline-1,3-dione (GT-06877)**

Referring to the method of Scheme 1, the target product (GT-06877) was prepared as a white solid (18 mg, yield 33 %). $^1$H NMR (400 MHz, DMSO-d6) δ 11.02 (s, 1H), 10.86 (s, 1H), 8.23 (s, 1H), 8.19 - 7.92 (m, 2H), 7.22 (t, *J* = 6.9 Hz, 2H), 7.17 - 7.04 (m, 2H), 4.57 - 4.28 (m, 2H), 3.76 (t, *J* = 6.7 Hz, 2H), 3.38 (s, 2H), 3.05 (d, *J* = 11.6 Hz, 3H), 2.79 (t, *J* = 6.7 Hz, 2H), 2.06 (d, *J* = 12.7 Hz, 2H), 1.86 (d, *J* = 13.4 Hz, 2H). LCMS (ESI) calcd for $C_{24}H_{24}FN_4O_4^+$ $[M+H]^+$: 451.18, found, 451.3.

**Example 479: preparation of 2-(2,4-dioxotetrahydropyrimidin-1(2H)-yl)-5-((4-(4-fluorophenyl)piperidin-1-yl)methyl)isoindoline-1,3-dione (GT-06878)**

Referring to the method of Scheme 1, the target product (GT-06878) was prepared as a white solid (24 mg, yield 44 %). $^1$H NMR (400 MHz, DMSO-d6) δ 10.97 (s, 1H), 10.85 (s, 1H), 8.30 (s, 1H), 8.12 (d, *J* = 7.6 Hz, 1H), 8.04 (d, *J* = 7.7 Hz, 1H), 7.21 - 7.14 (m, 2H), 7.09 (t, *J* = 8.8 Hz, 2H), 4.61 - 4.31 (m, 2H), 3.76 (t, *J* = 6.8 Hz, 2H), 3.37 (s, 2H), 2.99 (s, 2H), 2.80 - 2.65 (m, 3H), 2.11 - 1.85 (m, 4H). LCMS (ESI) calcd for $C_{24}H_{24}FN_4O_4^+$ [M+H]$^+$: 451.18, found, 451.3.

**Example 480: preparation of 2-(2,4-dioxotetrahydropyrimidin-1(2H)-yl)-5-((4-(2-(trifluoromethyl)phenyl)piperidin-1-yl)methyl)isoindoline-1,3-dione (GT-06879)**

Referring to the method of Scheme 1, the target product (GT-06879) was prepared as a white solid (23 mg, yield 38 %). $^1$H NMR (400 MHz, DMSO-d6) δ 10.86 (s, 1H), 10.82 (s, 1H), 8.25 (s, 1H), 8.15 - 7.93 (m, 2H), 7.59 - 7.48 (m, 4H), 4.46 (s, 2H), 3.76 (t, *J* = 6.8 Hz, 2H), 3.51 - 3.38 (m, 2H), 2.96 (d, *J* = 54.9 Hz, 3H), 2.79 (t, *J* = 6.8 Hz, 2H), 2.07 - 1.82 (m, 4H). LCMS (ESI) calcd for $C_{25}H_{24}F_3N_4O_4^+$ [M+H]$^+$: 501.17, found, 501.2 .

**Example 481: preparation of 2-(2,4-dioxotetrahydropyrimidin-1(2H)-yl)-5-((4-(3-(trifluoromethyl)phenyl)piperidin-1-yl)methyl)isoindoline-1,3-dione (GT-06880)**

Referring to the method of Scheme 1, the target product (GT-06880) was prepared as a white solid (19 mg, yield 31 %). $^1$H NMR (400 MHz, DMSO-d6) δ 11.15 (s, 1H), 10.86 (s, 1H), 8.24 (s, 1H), 8.18 - 7.87 (m, 2H), 7.65 (t, *J* = 8.3 Hz, 2H), 7.48 (d, *J* = 7.6 Hz, 1H), 7.39 (t, *J* = 7.6 Hz, 1H), 4.43 (s, 2H), 3.76 *(t, J* = 6.7 Hz, 2H), 3.38 (s, 2H), 3.12 (d, *J* = 9.7 Hz, 3H), 2.79 (t, *J* = 6.7 Hz, 2H), 2.29 - 2.05 (m, 2H), 1.81 (d, *J* = 12.8 Hz, 2H). LCMS (ESI) calcd for $C_{25}H_{24}F_3N_4O_4{}^+$ [M+H]$^+$: 501.17, found, 501.2.

**Example 482: preparation of 1-(5-((4-(5,6-dichloropyrimidin-4-yl)piperazin-1-yl)methyl)-1-oxoisoindolin-2-yl)dihydropyrimidine-2,4(1H,3H)-dione (GT-07028)**

Referring to the method of Scheme 1, the target product (GT-07028) was prepared as a white solid (23 mg, yield 37 %). $^1$H NMR (400 MHz, DMSO-d6) δ 11.55 (s, 1H), 10.70 (s, 1H), 8.52 (s, 1H), 7.88 (d, *J* = 8.1 Hz, 2H), 7.79 (d, *J* = 7.5 Hz, 1H), 4.82 (d, *J* = 16.6 Hz, 1H), 4.65 - 4.47 (m, 3H), 4.39 (d, *J* = 12.6 Hz, 2H), 3.80 (t, *J* = 6.9 Hz, 2H), 3.56 (t, 2H), 3.42 (s, 2H), 3.21 (s, 2H), 2.94 - 2.73 (m, 2H). LCMS (ESI) calcd for $C_{21}H_{22}Cl_2N_7O_3^+$ [M+H]$^+$: 490.12, found, 490.1.

**Example 483: preparation of 1-(5-((4-(5,6-dichloropyridazin-4-yl)piperazin-1-yl)methyl)-1-oxoisoindolin-2-yl)dihydropyrimidine-2,4(1H,3H)-dione (GT-07029)**

Referring to the method of Scheme 1, the target product (GT-07029) was prepared as a white solid (24 mg, yield 39 %). $^1$H NMR (400 MHz, DMSO-d6) δ 11.35 (s, 1H), 10.70 (s, 1H), 9.06 (s, 1H), 7.89 (d, *J* = 7.5 Hz, 2H), 7.79 (d, *J* = 7.4 Hz, 1H), 4.82 (d, *J* = 16.6 Hz, 1H), 4.69 - 4.38 (m, 3H), 3.96 (d, 2H), 3.80 (t, *J* = 6.9 Hz, 2H), 3.58 - 3.48 (m, 2H), 3.47 - 3.40 (m, 2H), 3.28 (s, 2H), 2.96 - 2.67 (m, 2H). LCMS (ESI) calcd for $C_{21}H_{22}Cl_2N_7O_3^+$ [M+H]$^+$: 490.12, found, 490.2.

**Example 484: preparation of 1-(5-((4-(3,6-dichloropyridazin-4-yl)piperazin-1-yl)methyl)-1-oxoisoindolin-2-yl)dihydropyrimidine-2,4(1H,3H)-dione (GT-07030)**

Referring to the method of Scheme 1, the target product (GT-07030) was prepared as a white solid (16 mg, yield 26 %). $^1$H NMR (400 MHz, DMSO-d6) δ 11.77 (s, 1H), 10.70 (s, 1H), 7.93 (s, 1H), 7.89 (d, *J = 7.9* Hz, 1H), 7.86 - 7.77 (m, 1H), 7.59 (s, 1H), 4.90 - 4.78 (m, 1H), 4.64 - 4.46 (m, 3H), 3.91 (d, 2H), 3.80 (t, *J* = 6.8 Hz, 2H), 3.54 - 3.34 (m, 4H), 3.26 (s, 2H), 2.95 - 2.66 (m, 2H). LCMS (ESI) calcd for $C_{21}H_{22}Cl_2N_7O_3^+$ [M+H]$^+$: 490.12 , found, 490.1.

**Example 485: preparation of 1-(5-((4-(1,3,5-triazin-2-yl)piperazin-1-yl)methyl)-1-oxoisoindolin-2-yl)dihydropyrimidine-2,4(1H,3H)-dione (GT-07031)**

Referring to the method of Scheme 1, the target product (GT-07031) was prepared as a white solid (45 mg, yield 73 %). $^1$H NMR (400 MHz, DMSO-d6) δ 11.51 (s, 1H), 10.70 (s, 1H), 8.70 (s, 2H), 7.89 (d, *J* = 7.4 Hz, 2H), 7.78 (d, *J* = 8.2 Hz, 1H), 4.88 - 4.69 (m, 3H), 4.59 (d, *J* = 16.3 Hz, 1H), 4.49 (s, 2H), 3.56 - 3.41 (m, 4H), 3.14 (s, 2H), 2.90 - 2.72 (m, 2H). LCMS (ESI) calcd for $C_{20}H_{23}N_8O_3^+$ [M+H]$^+$: 423.19, found, 423.2.

**Example 486: preparation of 1-(5-((4-(3,5-dichloropyridin-4-yl)piperazin-1-yl)methyl)-1-oxoisoindolin-2-yl)dihydropyrimidine-2,4(1H,3H)-dione (GT-07032)**

Referring to the method of Scheme 1, the target product (GT-07032) was prepared as a white solid (24 mg, yield 39 %). $^1$H NMR (400 MHz, DMSO-d6) δ 11.68 (s, 1H), 10.70 (s, 1H), 8.52 (s, 2H), 7.98 (s, 1H), 7.87 (s, 2H), 4.82 (d, *J* = 16.6 Hz, 1H), 4.59 (d, *J* = 16.5 Hz, 3H), 3.80 (t, 4H), 3.51 - 3.38 (m, 4H), 3.29 - 3.14 (m, 2H), 2.97 - 2.66 (m, 2H). LCMS (ESI) calcd for $C_{22}H_{23}Cl_2N_6O_3^+$ [M+H]$^+$: 489.12, found, 489.1.

**Example 487: preparation of 1-(5-((4-(2,6-dimethylpyridin-4-yl)piperazin-1-yl)methyl)-1-oxoisoindolin-2-yl)dihydropyrimidine-2,4(1H,3H)-dione (GT-07033)**

Referring to the method of Scheme 1, the target product (GT-07033) was prepared as a white solid (49 mg, yield 86 %). $^1$H NMR (400 MHz, DMSO-d6) δ 13.83 (s, 1H), 12.27 (s, 1H), 10.70 (s, 1H), 7.94 - 7.84 (m, 2H), 7.81 (s, 1H), 7.05 (s, 2H), 4.81 (d, *J* = 16.7 Hz, 1H), 4.57 (d, *J* = 16.6 Hz, 1H), 4.42 (d, *J* = 58.8 Hz, 4H), 3.80 (t, *J* = 6.8 Hz, 2H), 3.76 - 3.61 (m, 2H), 3.47 - 3.41 (m, 2H), 3.22 - 3.09 (m, 2H), 2.90 - 2.73 (m, 2H), 2.48 (s, 6H). LCMS (ESI) calcd for $C_{24}H_{29}N_6O_3^+$ [M+H]$^+$: 449.23, found, 449.3.

**Example 488: preparation of 5-((4-(5,6-dichloropyrimidin-4-yl)piperazin-1-yl)methyl)-2-(2,4-dioxotetrahydropyrimidin-1(2H)-yl)isoindoline-1,3-dione (GT-07034)**

Referring to the method of Scheme 1, the target product (GT-07034) was prepared as a yellow solid (18 mg, yield 29 %). $^1$H NMR (400 MHz, DMSO-d6) δ 11.57 (s, 1H), 10.93 (s, 1H), 8.53 (s, 1H), 8.30 (s, 1H), 8.16 (d, *J* = 7.7 Hz, 1H), 8.12 (d, *J* = 7.6 Hz, 1H), 4.59 (s, 2H), 4.38 (s, 2H), 3.83 (t, *J* = 6.8 Hz, 2H), 3.54 (s, 2H), 3.23 (s, 2H), 2.87 (t, *J* = 6.8 Hz, 2H). LCMS (ESI) calcd for $C_{21}H_{20}Cl_2N_7O_4^+$ [M+H]$^+$: 504.09 , found, 504.1.

**Example 489: preparation of 5-((4-(5,6-dichloropyridazin-4-yl)piperazin-1-yl)methyl)-2-(2,4-dioxotetrahydropyrimidin-1(2H)-yl)isoindoline-1,3-dione (GT-07035)**

Referring to the method of Scheme 1, the target product (GT-07035) was prepared as a yellow solid (25 mg, yield 41 %). $^1$H NMR (400 MHz, DMSO-d6) δ 10.93 (s, 1H), 9.07 (s, 1H), 8.33 (s, 1H), 8.20 (d, *J* = 7.7 Hz, 1H), 8.12 (d, *J* = 7.7 Hz, 1H), 8.00 - 7.82 (m, 1H), 4.67 (s, 2H), 4.56 - 4.44 (m, 1H), 3.98 (s, 2H), 3.83 (t, *J* = 6.8 Hz, 2H), 3.44 (s, 2H), 3.28 (s, 2H), 2.87 (t, *J* = 6.8 Hz, 2H), 2.80 - 2.70 (m, 1H). LCMS (ESI) calcd for $C_{21}H_{20}Cl_2N_7O_4^+$ [M+H]$^+$: 504.09, found, 504.1.

**Example 490: preparation of 5-((4-(3,6-dichloropyridazin-4-yl)piperazin-1-yl)methyl)-2-(2,4-dioxotetrahydropyrimidin-1(2H)-yl)isoindoline-1,3-dione (GT-07036)**

Referring to the method of Scheme 1, the target product (GT-07036) was prepared as a white solid (21 mg, yield 34 %). $^1$H NMR (400 MHz, DMSO-d6) δ 11.62 (d, *J* = 85.5 Hz, 1H), 10.93 (s, 1H), 8.31 (d, *J*= 9.7 Hz, 1H), 8.22 - 8.04 (m, 2H), 7.58 (s, 1H), 4.60 (d, *J* = 19.1 Hz, 2H), 4.49 (s, 1H), 3.90 (s, 2H), 3.83 (t, *J* = 6.8 Hz, 2H), 3.67 - 3.62 (m, 1H), 3.45 - 3.40 (m, 2H), 3.30 - 3.20 (m, 2H), 2.87 (t, *J* = 6.8 Hz, 2H). LCMS (ESI) calcd for $C_{21}H_{20}Cl_2N_7O_4^+$ [M+H]$^+$: 504.09, found, 504.1.

**Example 491: preparation of 5-((4-(1,3,5-triazin-2-yl)piperazin-1-yl)methyl)-2-(2,4-dioxotetrahydropyrimidin-1(2H)-yl)isoindoline-1,3-dione (GT-07037)**

Referring to the method of Scheme 1, the target product (GT-07037) was prepared as a white solid (25 mg, yield 46 %). $^1$H NMR (400 MHz, DMSO-d6) δ 12.07 (s, 1H), 10.93 (s, 1H), 8.72 (s, 2H), 8.33 (s, 1H), 8.18 (d, *J* = 12.8, 6.4 Hz, 1H), 8.11 (d, *J* = 7.7 Hz, 1H), 4.73 (s, 2H), 4.64 - 4.55 (m, 2H), 3.83 (t, *J* = 6.8 Hz, 2H), 3.65 - 3.39 (m, 4H), 3.17 (s, 2H), 2.86 (t, *J* = 6.8 Hz, 2H). LCMS (ESI) calcd for $C_{20}H_{21}N_8O_4^+$ [M+H]$^+$: 437.17, found, 437.2.

**Example 492: preparation of 5-((4-(3,5-dichloropyridin-4-yl)piperazin-1-yl)methyl)-2-(2,4-dioxotetrahydropyrimidin-1(2H)-yl)isoindoline-1,3-dione (GT-07038)**

Referring to the method of Scheme 1, the target product (GT-07038) was prepared as a white solid (4 mg, yield 7 %). $^1$H NMR (400 MHz, DMSO-d6) δ 11.52 (s, 1H), 10.93 (s, 1H), 8.52 (s, 2H), 8.36 (s, 1H), 8.22 (d, *J* = 8.6 Hz, 1H), 8.13 (d, *J* = 7.7 Hz, 1H), 4.66 (s, 2H), 4.57 (s, 1H), 3.83 (t, *J* = 6.7 Hz, 2H), 3.73 (d, 2H), 3.51 - 3.48 (m, 2H), 3.24 (s, 2H), 2.87 (t, *J* = 6.8 Hz, 2H), 2.76 (t, *J* = 5.9 Hz, 1H). LCMS (ESI) calcd for $C_{22}H_{21}Cl_2N_6O_4^+$ [M+H]$^+$: 503.10 , found, 503.1.

**Example 493: preparation of 5-((4-(2,6-dimethylpyridin-4-yl)piperazin-1-yl)methyl)-2-(2,4-dioxotetrahydropyrimidin-1(2H)-yl)isoindoline-1,3-dione (GT-07039)**

Referring to the method of Scheme 1, the target product (GT-07039) was prepared as a white solid (18 mg, yield 32 %). $^1$H NMR (400 MHz, DMSO-d6) δ 13.76 (s, 1H), 9.15 (s, 1H), 8.38 - 7.99 (m, 2H), 7.05 (s, 2H), 4.51 (d, *J* = 40.1 Hz, 2H), 4.36 (s, 1H), 3.92 - 3.81 (m, 2H), 3.68 - 3.57 (m, 4H), 3.25 - 3.15 (m, 2H), 3.14 - 3.08 (m, 2H), 2.86 (t, *J* = 6.7 Hz, 1H), 2.48 (s, 6H). LCMS (ESI) calcd for $C_{24}H_{27}N_6O_4^+$ [M+H]$^+$: 463.21, found, 463.3.

**Example 494: preparation of 1-(1-oxo-5-((4-(perfluoropyridin-4-yl)piperazin-1-yl)methyl)isoindolin-2-yl)dihydropyrimidine-2,4(1H,3H)-dione (GT-07040)**

Referring to the method of Scheme 1, the target product (GT-07040) was prepared as a white solid (42 mg, yield 67 %). $^1$H NMR (400 MHz, DMSO-d6) δ 11.46 (s, 1H), 10.62 (s, 1H), 7.81 (d, *J* = 8.1 Hz, 2H), 7.73 (s, 1H), 4.75 (d, *J* = 16.6 Hz, 1H), 4.55 - 4.37 (m, 3H), 3.72 (t, 4H), 3.67 - 3.58 (m, 2H), 3.16 (s, 2H), 2.84 - 2.76 (m, 1H), 2.74 - 2.66 (m, 1H). LCMS (ESI) calcd for $C_{22}H_{21}F_4N_6O_3^+$ [M+H]$^+$: 493.16, found, 493.2.

**Example 495: preparation of 2-(2,4-dioxotetrahydropyrimidin-1(2H)-yl)-5-((4-(perfluoropyridin-4-yl)piperazin-1-yl)methyl)isoindoline-1,3-dione (GT-07041)**

Referring to the method of Scheme 1, the target product (GT-07041) was prepared as a white solid (32 mg, yield 52 %). $^1$H NMR (400 MHz, DMSO-d6) δ 11.40 (s, 1H), 10.86 (s, 1H), 8.21 (s, 1H), 8.05 (s, 2H), 4.53 (s, 2H), 3.76 (t, *J* = 6.8 Hz, 4H), 3.61 (s, 3H), 3.39 - 3.35 (m, 1H), 3.22 - 3.07 (m, 2H), 2.79 (t, *J* = 6.8 Hz, 2H). LCMS (ESI) calcd for $C_{22}H_{19}F_4N_6O_4^+$ [M+H]$^+$: 507.14, found, 507.1.

**Example 496: preparation of 1-(5-((4-(6-chloropyridazin-3-yl)piperazin-1-yl)methyl)-1-oxoisoindolin-2-yl)dihydropyrimidine-2,4(1H,3H)-dione (GT-07042)**

Referring to the method of Scheme 1, the target product (GT-07042) was prepared as a white solid (45 mg, yield 77 %). $^1$H NMR (400 MHz, DMSO-d6) δ 11.49 (s, 1H), 10.70 (s, 1H), 7.89 (d, *J* = 8.2 Hz, 2H), 7.80 (d, *J* = 7.8 Hz, 1H), 7.67 (d, *J* = 9.6 Hz, 1H), 7.49 (d, *J* = 9.6 Hz, 1H), 4.70 (dd, *J* = 92.8, 16.8 Hz, 2H), 4.56 - 4.40 (m, 4H), 3.80 (t, *J* = 6.8 Hz, 2H), 3.50 - 3.38 (m, 4H), 3.16 (s, 2H), 2.92 - 2.72 (m, 2H). LCMS (ESI) calcd for $C_{21}H_{23}ClN_7O_3^+$ [M+H]$^+$: 456.15 , found, 456.2.

**Example 497: preparation of 5-((4-(6-chloropyridazin-3-yl)piperazin-1-yl)methyl)-2-(2,4-dioxotetrahydropyrimidin-1(2H)-yl)isoindoline-1,3-dione (GT-07043)**

Referring to the method of Scheme 1, the target product (GT-07043) was prepared as a white solid (30 mg, yield 52 %). $^1$H NMR (400 MHz, DMSO-d6) δ 11.89 (s, 1H), 10.93 (s, 1H), 8.33 (s, 1H), 8.19 (d, 1H), 8.11 (d, *J* = 7.7 Hz, 1H), 7.67 (d, *J* = 9.6 Hz, 1H), 7.50 (d, *J* = 9.6 Hz, 1H), 4.64 - 4.55 (m, 2H), 4.50 - 4.41 (m, 2H), 3.82 (t, *J* = 6.2 Hz, 2H), 3.53 - 3.38 (m, 4H), 3.17 (s, 2H), 2.86 (t, *J* = 6.8 Hz, 2H). LCMS (ESI) calcd for $C_{21}H_{21}ClN_7O_4^+$ [M+H]$^+$: 470.13, found, 470.2.

**Example 498: preparation of 1-(5-((4-(2,3-difluorophenyl)piperidin-1-yl)methyl)-1-oxoisoindolin-2-yl)dihydropyrimidine-2,4(1H,3H)-dione (GT-08194)**

Referring to the method of Scheme 1, the target product (GT-08194) was prepared as a white solid (38 mg, yield 66%). $^1$H NMR (400 MHz, DMSO-d6) δ 10.70 (s, 1H), 7.95 (s, 1H), 7.87 (t, *J* = 5.6 Hz, 2H), 7.32 (d, *J* = 8.8 Hz, 1H), 7.22 (d, *J* = 5.1 Hz, 1H), 7.11 (d, *J* = 6.5 Hz, 1H), 4.81 (s, 1H), 4.59 (d, *J* = 17.4 Hz, 1H), 4.46 (d, *J* = 4.7 Hz, 2H), 3.80 (dd, *J* = 11.9, 5.2 Hz, 3H), 3.33 (s, 1H), 3.16 (t, *J* = 10.7 Hz, 3H), 2.86 (s, 1H), 2.78 (d, *J* = 5.6 Hz, 1H), 2.22 (d, *J* = 12.3 Hz, 2H), 1.93 (d, *J* = 14.0 Hz, 2H). LCMS (ESI) calcd for $C_{24}H_{25}F_2N_4O_3^+$ [M+H]$^+$: 455.17, found, 455.1.

**Biological activity assay**

**Reagents and Materials:**

| Reagents and materials | Suppliers or Source |
|---|---|
| Multiple myeloma cell line: MM.1S | ATCC (American Type Culture Collection) |

(continued)

| | |
|---|---|
| Human lung cancer cell line: H292 | Dalian Meilun Biotech Co., Ltd. |
| Human non-small cell lung cancer cell line: H2228 | ATCC |
| Human gastric cancer cell line: MGC803 | Dalian Meilun Biotech Co., Ltd. |
| Human T lymphocytic leukemia cell line: Jurkat | ATCC |
| Human myeloid monocytic leukemia cell line: MV-4-11 | ATCC |
| Human breast cancer cell line: MDA-MB-231 | ATCC |
| RPMI-1640 Medium | ATCC |
| EMEM Medium | ATCC |
| DMEM Medium | Dalian Meilun Biotech Co., Ltd. |
| IMDM Medium | Gibco Company |
| Fetal bovine serum (FBS) | Sunrise Science Products Company |
| Horse Serum (HS) | Dalian Meilun Biotech Co., Ltd. |
| Penicillin-Streptomycin | Sunrise Science Products Company |
| CellTiter-Meiluncell Luminescent Cell Viability Assay Kit | Dalian Meilun Biotech Co., Ltd. |
| Mycoplasma detection kit | Sunrise Science Products Company |
| STR genotype detection | Shanghai Biowing Applied Biotechnology Co. Ltd. |

**Methods:**

**Cell cultures**

The tumor cells used herein were cultured in a cell culture medium listed in table 2 at 37°C in an incubator with 5% $CO_2$. Prior to experimentation, all cell lines used were correctly identified by STR profiling, and tested negative for mycoplasma contamination using a mycoplasma detection kit through routine screenings.

Table 2. Tumor cells and cell culture medium used in the present disclosure

| Cell line | Cell culture medium used |
|---|---|
| MM.1S | RPMI 1640 culture medium supplemented with 10% FBS, as well as penicillin at a final concentration of 100 U/mL and streptomycin at a final concentration of 100 μg/mL |
| H2228 | |
| MGC803 | |
| Jurkat | |
| MV-4-11 | |
| H292 | RPMI 1640 culture medium supplemented with 10% FBS, 2.5% horse serum, as well as penicillin at a final concentration of 100 U/mL and streptomycin at a final concentration of 100 μg/mL |
| MDA-MB-231 | DMEM culture medium supplemented with 10% FBS, 2.5% horse serum, as well as penicillin at a final concentration of 100 U/mL and streptomycin at a final concentration of 100 μg/mL |

**I. Determination of half inhibitory concentration ($IC_{50}$) of the compounds against tumor cells:**

The $IC_{50}$ values of the compounds of the present disclosure (including the compounds in Table 1, and Examples 1-498) were determined using the CellTiter-Meiluncell Luminescent Cell Viability Assay Kit from Dalian Meilun Biotech Co., Ltd. The detailed procedure is as follows: Tumor cells were seeded in 96-well cell culture plates at the densities specified in Table 3. On the following day, the test compounds of the present disclosure were subjected to serial dilution. The dilution can be performed according to one of the following two methods:

(1) The first dilution method: The test compounds of the present disclosure (including the compounds in Table 1, and Examples 1-498) were subjected to 5-fold serial dilutions starting from a highest concentration of 2000 μM, resulting in a total of 9 concentrations from high to low (2000, 400, 80, 16, 3.2, 0.64, 0.128, 0.0256, 0.00512 μM). Subsequently, 0.5 μL of the test compound dilution was transferred into the 96-well plate containing the cells, with each well containing 100 μL of cell culture medium. This resulted in final test compound concentrations of 10, 2, 0.4, 0.08, 0.016, 0.0032, 0.00064, 0.000128, and 0.0000256 μM, respectively, or

(2) As an alternative, a second dilution method can be used: The test compounds of the present disclosure (including the compounds in Table 1, and Examples 1-498) were subjected to a 50-fold gradient dilution starting from a highest concentration of 100 μM, resulting in 2 concentrations (100 μM, 2 μM). Into the 96-well plate, the diluted test compounds and corresponding detection reagents were added, resulting in final test compound concentrations of 500 nM and 10 nM, respectively. The experimental procedure is described below using the diluted 2 μM test compound as an example.

0.5 μL of the aforementioned diluted compound of the present disclosure (including the compounds in Table 1, and Examples 1-498) was added to the seeded cells in 100 μL of medium, yielding a final test compound concentration of 10 nM. After the cells were treated with the compounds of the present disclosure for a specified period, the cell viability assay reagent was added to the culture medium according to the kit's instructions to determine cell viability. The negative control was DMSO, and the reference controls were corresponding commercial inhibitors immunomodulatory agents (including lenalidomide and pomalidomide). All controls were treated using the same procedure as the compounds of the present disclosure. The growth inhibition curves for the compounds of the present disclosure were plotted using Prism GraphPad software, and the $IC_{50}$ values were calculated therefrom. The results were listed in Tables A through F below.

Table 3. Seeding protocol and treatment time for tumor cells

| Cells | Cells seeding protocol | The times for treating cells with the compounds of the present disclosure (hours) |
|---|---|---|
| MM.1S<br>Jurkat<br>MV-4-11 | cells were seeded at a density of 10,000 cells per well in 100 μL of serum-containing RPMI-1640 medium per well | 96 hours |
| H2228<br>H292 | cells were seeded at a density of 4,000 cells per well in 100 μL of serum-containing RPMI-1640 medium per well | 96 hours |
| MGC803 | cells were seeded at a density of 2,000 cells per well in 100 μL of serum-containing RPMI-1640 medium per well | 96 hours |
| MDA-MB-231 | cells were seeded at a density of 4,000 cells per well in 100 μL of serum-containing DMEM medium per well | 96 hours |

Table A. The inhibitory activity ($IC_{50}$, nM) of the compounds of the present disclosure against tumor cells proliferation

| Comp. No. | MM.1S | MGC8 03 | Jurkat | MV-4-11 | Comp. No. | MM.1S | MGC8 03 | Jurkat | MV-4-11 |
|---|---|---|---|---|---|---|---|---|---|
| lenalid omide | D | F | F | F | GT-07577 | B | - | C | - |
| GT-04853 | A | C | - | - | GT-07578 | A+ | - | A+ | - |
| GT-04866 | A | B | B | - | GT-07590 | A++ | - | A+ | - |
| GT-04867 | C | - | - | - | GT-07293 | A | - | A | - |
| GT-04868 | A | B | B | - | GT-07371 | C | - | - | - |
| GT-04869 | A+ | B | B | - | GT-07425 | C | - | - | - |
| GT-04870 | A+ | A | A | - | GT-07432 | B | - | C | - |
| GT-04871 | A | B | B | - | GT-07511 | C | - | C | - |
| GT-04872 | A++ | A | A | - | GT-07575 | A | - | A | - |
| GT-04873 | A+ | A | A | - | GT-06770 | - | - | C | C |
| GT-04874 | A | - | C |  | GT-06773 | - | - | - | A |
| GT-04875 | B | - | - | - | GT-06776 | - | - | C | A |
| GT-04889 | A | B | B | - | GT-06777 | - | - | - | B |
| GT-04890 | A | C | - | - | GT-06884 | - | - | - | C |

(continued)

| Comp. No. | MM.1S | MGC8 03 | Jurkat | MV-4-11 | Comp. No. | MM.1S | MGC8 03 | Jurkat | MV-4-11 |
|---|---|---|---|---|---|---|---|---|---|
| GT-04894 | C | - | - | - | GT-06885 | - | - | - | A |
| GT-04939 | A++ | A | A | - | GT-06892 | - | - | - | C |
| GT-04940 | A+ | A | A | - | GT-06893 | - | - | C | A |
| GT-04943 | A++ | A | A | - | GT-06894 | - | - | C | B |
| GT-04944 | C | - | - | - | GT-07026 | - | - | C | - |
| GT-05126 | B | - | - | - | GT-06699 | - | - | - | A |
| GT-05127 | C | - | - | - | GT-06700 | - | - | - | A+ |
| GT-05163 | C | - | - | - | GT-06702 | - | - | - | C |
| GT-05164 | A | - | - | - | GT-06703 | - | - | - | C |
| GT-05165 | C | - | - | - | GT-06765 | - | - | - | C |
| GT-05166 | A | - | - | - | GT-06766 | - | - | - | C |
| GT-05167 | A++ | - | - | - | GT-06393 | C | - | - | - |
| GT-05168 | A | - | - | - | GT-06456 | A | - | - | - |
| GT-05169 | A | - | - | - | GT-06696 | - | - | - | A |
| GT-06132 | A | - | - | - | GT-06698 | - | - | - | A |
| GT-06392 | B | - | - | - | | | | | |

Note: In Table A, the symbols A+++, A++, A+, A, B, C, D, E, and F are defined as follows: 0 nM <A+++ ≤ 10 nM; 10 nM < A++ ≤ 50 nM; 50 nM < A+ ≤ 100 nM; 100 nM < A ≤ 300 nM; 300 nM < B ≤ 500 nM; 500 nM < C ≤ 1000 nM; 1000 nM < D ≤ 5000 nM; 5000 nM < E ≤ 10000 nM; F > 10000 nM. The symbol "-" indicates "not detected".

Table B. Inhibitory activity of the compounds of the present disclosure on the Proliferation of Human lung cancer cells (H292)

| Comp. No. | Inhibition Rate at 500 nM (%) | Inhibition Rate at 10 nM (%) | Comp. No. | Inhibition Rate at 500 nM (%) | Inhibition Rate at 10 nM (%) | Comp. No. | Inhibition Rate at 500 nM (%) | Inhibition Rate at 10 nM (%) |
|---|---|---|---|---|---|---|---|---|
| lenalido mide | b3 | b1 | GT-08099 | b6 | b6 | GT-07806 | b5 | b5 |
| GT-07784 | b6 | b6 | GT-08100 | b4 | b3 | GT-07888 | b4 | b3 |
| GT-07786 | b6 | b4 | GT-08103 | b5 | b5 | GT-07998 | b2 | b5 |
| GT-08057 | b5 | b5 | GT-08105 | b4 | b4 | GT-08190 | b6 | b6 |
| GT-08059 | b3 | b6 | GT-08107 | b4 | b3 | GT-08424 | b4 | b6 |
| GT-08060 | b6 | b6 | GT-08108 | b5 | b6 | GT-08425 | b3 | b5 |
| GT-08062 | b7 | b6 | GT-08109 | b2 | b5 | GT-08426 | b6 | b6 |
| GT-08063 | b6 | b6 | GT-08110 | b4 | b6 | GT-08427 | b6 | b5 |
| GT-08065 | b6 | b6 | GT-08111 | b6 | b6 | GT-08428 | b6 | b3 |
| GT-08066 | b6 | b6 | GT-08112 | b6 | b5 | GT-08429 | b6 | b6 |
| GT-08067 | b6 | b6 | GT-08113 | b6 | b6 | GT-08430 | b4 | b5 |
| GT-08068 | b6 | b6 | GT-08114 | b4 | b6 | GT-08484 | b5 | b6 |
| GT-08079 | b6 | b6 | GT-08115 | b5 | b6 | GT-08432 | b6 | b6 |
| GT-08080 | b6 | b7 | GT-08116 | b5 | b5 | GT-08433 | b5 | b6 |
| GT-08082 | b6 | b6 | GT-08117 | b6 | b6 | GT-08434 | b5 | b6 |

(continued)

| Comp. No. | Inhibition Rate at 500 nM (%) | Inhibition Rate at 10 nM (%) | Comp. No. | Inhibition Rate at 500 nM (%) | Inhibition Rate at 10 nM (%) | Comp. No. | Inhibition Rate at 500 nM (%) | Inhibition Rate at 10 nM (%) |
|---|---|---|---|---|---|---|---|---|
| GT-08083 | b6 | b6 | GT-08118 | b6 | b6 | GT-08437 | b5 | b6 |
| GT-08084 | b4 | b2 | GT-08119 | b6 | b5 | GT-08438 | b4 | b6 |
| GT-08085 | b7 | b6 | GT-07723 | b3 | b5 | GT-08439 | b6 | b6 |
| GT-08086 | b6 | b6 | GT-08157 | b5 | b6 | GT-08443 | b7 | b6 |
| GT-08088 | b6 | b6 | GT-08158 | b7 | b6 | GT-08446 | b6 | b6 |
| GT-08089 | b6 | b6 | GT-08159 | b6 | b5 | GT-08448 | b6 | b6 |
| GT-08090 | b5 | b5 | GT-08174 | b6 | b4 | GT-08451 | b6 | b5 |
| GT-08091 | b6 | b6 | GT-08176 | b6 | b5 | GT-08452 | b3 | b6 |
| GT-08093 | b5 | b5 | GT-08177 | b4 | b4 | GT-08453 | b4 | b6 |
| GT-08094 | b6 | b6 | GT-08178 | b6 | b7 | GT-08455 | b5 | b3 |
| GT-08096 | b6 | b7 | GT-08133 | b6 | b5 | GT-08456 | b6 | b3 |
| GT-08097 | b4 | b3 | | | | | | |

Note: in table B, the symbols b1, b2, b3, b4, b5, b6, b7, b8, and a9 are defined as follows: 0 < b1 < 1%, 1% ≤ b2 < 5%, 5% ≤ b3 < 10%, 10% ≤ b4 < 15%, 15% ≤ b5 < 20%, 20% ≤ b6 < 40%, 40% ≤ b7 < 60%, 60% ≤ b8 < 80%, 80% ≤ b9 ≤ 100%.

Table C. Inhibitory activity of the compounds of the present disclosure on the Proliferation of Human Multiple myeloma cells (MM.1S)

| Comp. No. | Inhibition Rate at 500 nM (%) | Inhibition Rate at 10 nM (%) | Comp. No. | Inhibition Rate at 500 nM (%) | Inhibition Rate at 10 nM (%) | Comp. No. | Inhibition Rate at 500 nM (%) | Inhibition Rate at 10 nM (%) |
|---|---|---|---|---|---|---|---|---|
| lenalido mide | c7 | c5 | GT-08090 | c8 | c7 | GT-08173 | c10 | c2 |
| GT-08054 | c10 | c4 | GT-08102 | c7 | c7 | GT-08175 | c10 | c1 |
| GT-08055 | c10 | c1 | GT-08104 | c7 | c6 | GT-08178 | c9 | c9 |
| GT-08088 | c8 | c8 | GT-08116 | c9 | c5 | GT-08484 | c6 | c8 |
| GT-08062 | c7 | c7 | GT-08157 | c10 | c2 | GT-08433 | c7 | c6 |
| GT-08065 | c6 | c6 | GT-08158 | c10 | c4 | GT-08438 | c10 | c6 |
| GT-08085 | c8 | c7 | GT-08159 | c9 | c3 | GT-08446 | c7 | c8 |

Note: in table C, the symbols c1, c2, c3, c4, c5, c6, c7, c8, c9, and c10 are defined as follows: -10% ≤ c1 < -5%, -5% ≤ c2 ≤ 0, 0 < c3 < 5%, 5% ≤ c4 < 10%, 10% ≤ c5 < 20%, 20% ≤ c6 < 30%, 30% ≤ c7 < 42%, 42% ≤ c8 < 60%, 60% ≤ c9 < 80%, 80% ≤ c10 ≤ 100%.

Table D. Inhibitory activity of the compounds of the present disclosure on the Proliferation of Human non-small cell lung cancer cells (H2228)

| Comp. No. | Inhibition Rate at 500 nM (%) | Inhibition Rate at 10 nM (%) | Comp. No. | Inhibition Rate at 500 nM (%) | Inhibition Rate at 10 nM (%) | Comp. No. | Inhibition Rate at 500 nM (%) | Inhibition Rate at 10 nM (%) |
|---|---|---|---|---|---|---|---|---|
| lenalido mide | d4 | d5 | GT-08080 | d5 | d7 | GT-08097 | d7 | d4 |
| GT-07784 | d7 | d6 | GT-08082 | d5 | d6 | GT-08098 | d6 | d3 |

(continued)

| Comp. No. | Inhibition Rate at 500 nM (%) | Inhibition Rate at 10 nM (%) | Comp. No. | Inhibition Rate at 500 nM (%) | Inhibition Rate at 10 nM (%) | Comp. No. | Inhibition Rate at 500 nM (%) | Inhibition Rate at 10 nM (%) |
|---|---|---|---|---|---|---|---|---|
| GT-07786 | d6 | d3 | GT-08083 | d7 | d6 | GT-08099 | d7 | d6 |
| GT-08054 | d8 | d5 | GT-08084 | d6 | d3 | GT-08102 | d5 | d5 |
| GT-08055 | d5 | d3 | GT-08085 | d7 | d6 | GT-08111 | d6 | d5 |
| GT-08194 | d5 | d5 | GT-08086 | d5 | d5 | GT-08112 | d6 | d4 |
| GT-08057 | d5 | d7 | GT-08088 | d6 | d6 | GT-08113 | d6 | d5 |
| GT-08059 | d4 | d7 | GT-08089 | d5 | d5 | GT-08116 | d5 | d4 |
| GT-08060 | d7 | d5 | GT-08090 | d5 | d5 | GT-08158 | d9 | d6 |
| GT-08061 | d6 | d3 | GT-08091 | d5 | d5 | GT-08159 | d6 | d3 |
| GT-08062 | d7 | d6 | GT-08093 | d5 | d5 | GT-08173 | d8 | d2 |
| GT-08065 | d6 | d5 | GT-08094 | d6 | d7 | GT-08174 | d5 | d4 |
| GT-08079 | d5 | d4 | GT-08096 | d5 | d7 | | | |
| Note: in table D, the symbols d1, d2, d3, d4, d5, d6, d7, d8, d9, and d10 are defined as follows: 0 < d1 < 5%, 5% ≤ d2 < 10%, 10% ≤ d3 < 15%, 15% ≤ d4 < 20%, 20% ≤ d5 < 25%, 25% ≤ d6 < 30%, 30% ≤ d7 < 40%, 40% ≤ d8 < 60%, 60% ≤ d9 < 80%, 80% ≤ d10 ≤ 100%. | | | | | | | | |

Table E. Inhibitory activity of the compounds of the present disclosure on the Proliferation of Human myeloid monocytic leukemia cells (MV-4-11)

| Comp. No. | Inhibition Rate at 500 nM (%) | Inhibition Rate at 10 nM (%) | Comp. No. | Inhibition Rate at 500 nM (%) | Inhibition Rate at 10 nM (%) | Comp. No. | Inhibition Rate at 500 nM (%) | Inhibition Rate at 10 nM (%) |
|---|---|---|---|---|---|---|---|---|
| lenalido mide | e3 | e3 | GT-08087 | e6 | e6 | GT-08113 | e3 | e6 |
| GT-08054 | e9 | e4 | GT-08088 | e6 | e7 | GT-08116 | e9 | e7 |
| GT-08055 | e9 | e5 | GT-08090 | e8 | e8 | GT-08118 | e7 | e7 |
| GT-08194 | e8 | e4 | GT-08092 | e6 | e3 | GT-08157 | e9 | e4 |
| GT-08063 | e2 | e4 | GT-08093 | e8 | e7 | GT-08158 | e10 | e6 |
| GT-08064 | e3 | e5 | GT-08095 | e4 | e5 | GT-08159 | e9 | e6 |
| GT-08065 | e3 | e6 | GT-08096 | e6 | e6 | GT-08173 | e9 | e3 |
| GT-08066 | e3 | e4 | GT-08098 | e4 | e4 | GT-08174 | e3 | e5 |
| GT-08067 | e7 | e8 | GT-08099 | e2 | e5 | GT-08176 | e6 | e8 |
| GT-08068 | e4 | e4 | GT-08101 | e5 | e4 | GT-08175 | e9 | e4 |
| GT-08077 | e5 | e5 | GT-08102 | e7 | e7 | GT-08177 | e6 | e7 |
| GT-08079 | e7 | e7 | GT-08104 | e7 | e7 | GT-08178 | e9 | e8 |
| GT-08081 | e5 | e4 | GT-08106 | e4 | e3 | GT-07806 | e5 | e2 |
| GT-08082 | e7 | e7 | GT-08107 | e7 | e6 | GT-07887 | e6 | e5 |
| GT-08084 | e3 | e4 | GT-08110 | e4 | e3 | GT-07888 | e7 | e7 |

(continued)

| Comp. No. | Inhibition Rate at 500 nM (%) | Inhibition Rate at 10 nM (%) | Comp. No. | Inhibition Rate at 500 nM (%) | Inhibition Rate at 10 nM (%) | Comp. No. | Inhibition Rate at 500 nM (%) | Inhibition Rate at 10 nM (%) |
|---|---|---|---|---|---|---|---|---|
| GT-08085 | e5 | e6 | | | | | | |
| Note: in table E, the symbols e1, e2, e3, e4, e5, e6, e7, e8, e9, and e10 are defined as follows: 0 < e1 < 5%, 5% ≤ e2 < 10%, 10% ≤ e3 < 18%, 18% ≤ e4 < 25%, 25% ≤ e5 < 30%, 30% ≤ e6 < 40%, 40% ≤ e7 < 60%, 60% ≤ e8 < 80%, 80% ≤ e9 ≤ 100%, 100% < e10 ≤ 110%. | | | | | | | | |

Table F. Inhibitory activity of the compounds of the present disclosure on the Proliferation of Human breast cancer cells (MDA-MB-231)

| Comp. No. | Inhibition Rate at 500 nM (%) | Inhibition Rate at 10 nM (%) | Comp. No. | Inhibition Rate at 500 nM (%) | Inhibition Rate at 10 nM (%) | Comp. No. | Inhibition Rate at 500 nM (%) | Inhibition Rate at 10 nM (%) |
|---|---|---|---|---|---|---|---|---|
| lenalido mide | f3 | f4 | GT-08082 | f5 | f6 | GT-08112 | f6 | f4 |
| GT-07784 | f6 | f5 | GT-08083 | f6 | f6 | GT-08113 | f7 | f6 |
| GT-07786 | f6 | f4 | GT-08084 | f5 | f2 | GT-08114 | f5 | f5 |
| GT-08054 | f9 | f6 | GT-08085 | f7 | f6 | GT-08116 | f6 | f6 |
| GT-08055 | f8 | f5 | GT-08086 | f6 | f6 | GT-08117 | f5 | f5 |
| GT-08194 | f6 | f5 | GT-08088 | f6 | f5 | GT-08118 | f6 | f5 |
| GT-08057 | f6 | f6 | GT-08089 | f6 | f5 | GT-08157 | f6 | f4 |
| GT-08058 | f4 | f6 | GT-08090 | f5 | f4 | GT-08158 | f9 | f5 |
| GT-08059 | f6 | f6 | GT-08091 | f5 | f4 | GT-08159 | f5 | f3 |
| GT-08060 | f6 | f6 | GT-08094 | f6 | f6 | GT-08173 | f8 | f3 |
| GT-08061 | f5 | f3 | GT-08096 | f5 | f6 | GT-08174 | f6 | f5 |
| GT-08062 | f6 | f4 | GT-08097 | f5 | f3 | GT-08178 | f5 | f5 |
| GT-08063 | f4 | f4 | GT-08099 | f6 | f5 | GT-07806 | f5 | f5 |
| GT-08066 | f4 | f4 | GT-08108 | f5 | f6 | GT-07888 | f5 | f5 |
| GT-08079 | f3 | f5 | GT-08109 | f2 | f5 | GT-07998 | f3 | f6 |
| GT-08080 | f4 | f5 | GT-08110 | f4 | f6 | GT-08190 | f3 | f5 |
| GT-08081 | f2 | f5 | GT-08111 | f6 | f6 | | | |
| Note: in table F, the symbols f1, f2, f3, f4, f5, f6, f7, f8, and f9 are defined as follows: 0 < f1 < 5%, 5% ≤ f2 < 10%, 10% ≤ f3 < 15%, 15% ≤ f4 < 18%, 18% ≤ f5 < 25%, 25% ≤ f6 < 40%, 40% ≤ f7 < 60%, 60% ≤ f8 < 80%, 80% ≤ f9 ≤ 100%. | | | | | | | | |

The results demonstrate that the designed and developed compounds of the present invention (including the compounds listed in Table 1, and Examples 1-498) effectively inhibit the proliferation of tumor cells (as shown in Tables A to F). The inhibitory effect is significantly superior to that of the corresponding positive control drugs.

**II. Western-blot assay**

Conventional Western blot assay was performed to evaluate the effects of the compounds of the present disclosure on the expression of target proteins in cells.

(1) Cell Seeding and Protein Harvesting: Human peripheral blood mononuclear cells (hPBMCs) at a density of $1x10^6$

cells/mL were thawed according to the PBMC Recovery Protocol (Milecell Biotechnologies Inc, Shanghai). The cells were then treated with the compounds of the present disclosure (including the compounds listed in Table 1, and the Examples 1-498 compounds) at concentrations of 50 nM and 500 nM. Controls included a negative control (DMSO) and a positive control (the commercial immunomodulatory drug lenalidomide), both processed following the identical experimental protocol as the compounds of the present disclosure. After 24 hours of compounds treatment, cells were harvested and lysed in RIPA protein lysate containing protease inhibitors on ice for 30 min, followed by centrifugation. The supernatant was aspirated as the extracted total cell protein. The protein concentration of the supernatant was determined using the conventional BCA (Bicinchoninic Acid) assay.

(2) To the collected supernatant, 5X SDS sample loading buffer was added, followed by denaturation at 95°C for 5 minutes. The denatured samples were then separated by SDS-PAGE electrophoresis on a polyacrylamide gel. Proteins were transferred to a nitrocellulose membrane (0.45 μm NC membrane), blocked with blocking buffer at room temperature for 1 hour, and subjected to antibody incubation and development procedures according to the antibody manufacturer's instructions (Cell Signaling Technology).

Half-Maximal Degradation Concentration value (the drug concentration required for degrading proteins by 50%, abbreviated as $DC_{50}$) reads method: comparing the grayscale values of Western blot bands from drug-treated samples with those from blank DMSO-treated controls, and identifying the drug concentration range at which the grayscale value is half that of the DMSO-treated control bands.

$DC_{50}$ values can be calculated using ImageJ software to measure grayscale values of Western blot bands from drug-treated samples. The concentration-response curve is fitted to estimate the drug concentration corresponding to half the grayscale value.

Target protein remaining (%) refers to the percentage of target protein remaining in cells after treatment with protein degrader compounds.

Calculation method for target protein remaining (%): Use ImageJ software to measure grayscale values of Western blot bands from drug (protein degrader)-treated samples. The grayscale value of drug-treated bands is divided by that of internal control protein bands, and the resulting quotient is normalized to obtain the relative percentage of remaining target protein after protein degrader treatment.

$$\text{Target protein degradation rate (\%)} = 1 - \text{target protein remaining (\%)}.$$

The effects of the compounds of the present disclosure (including the compounds listed in Table 1, and the Example 1-498 compounds) on substrate proteins expression in cells were shown in Table II-1 below.

Table II-1 Degradation of substrate proteins WEE1, IKZF1, IKZF2, IKZF3, CK1α, and GSPT1 by the compounds of the present disclosure

| Comp. No./names | WEE1 | IKZF1 | IKZF2 | IKZF3 | CK1α | GSPT1 |
|---|---|---|---|---|---|---|
| Lenalidomide | B | A | B | A | B | B |
| GT-04511 | B | B | B | B | B | A |
| GT-04536 | B | B | B | B | B | A |
| GT-04853 | A | A | A | B | B | A |
| GT-04854 | A | A | A | B | B | A |
| GT-04864 | B | A | B | B | B | A |
| GT-04865 | B | A | B | B | B | A |
| GT-04866 | A | A | B | B | B | A |
| GT-04867 | B | A | B | B | B | A |
| GT-04868 | A | A | B | B | A | A |
| GT-04869 | A | A | A | A | B | A |
| GT-04870 | A | A | A | A | B | A |
| GT-04871 | A | A | B | B | B | A |
| GT-04872 | A | A | B | B | B | A |

(continued)

| Comp. No./names | WEE1 | IKZF1 | IKZF2 | IKZF3 | CK1α | GSPT1 |
|---|---|---|---|---|---|---|
| GT-04873 | A | A | B | A | B | A |
| GT-04874 | A | A | B | A | B | A |
| GT-04875 | A | B | B | B | B | A |
| GT-04889 | A | A | A | A | B | A |
| GT-04890 | B | A | A | B | B | A |
| GT-04891 | B | A | A | B | B | A |
| GT-04894 | A | A | A | B | B | A |
| GT-04895 | B | A | A | B | B | A |
| GT-04938 | A | B | A | B | B | A |
| GT-04939 | A | A | A | A | A | A |
| GT-04940 | A | A | A | A | A | A |
| GT-04943 | A | A | A | A | B | A |
| GT-04944 | B | B | B | B | B | A |
| GT-04990 | A | B | B | B | B | A |
| GT-05073 | B | B | B | A | B | B |
| GT-05083 | B | B | A | B | B | B |
| GT-05084 | B | B | A | B | B | B |
| GT-05087 | B | B | B | B | B | A |
| GT-05125 | A | A | B | A | A | A |
| GT-05126 | A | A | A | A | A | A |
| GT-05127 | A | A | B | A | A | A |
| GT-05128 | A | A | A | A | A | A |
| GT-05129 | A | A | A | A | A | A |
| GT-05130 | A | A | A | A | A | A |
| GT-05131 | A | A | A | A | A | A |
| GT-05162 | B | B | B | A | B | A |
| GT-05163 | B | A | A | A | B | A |
| GT-05164 | B | A | A | B | B | A |
| GT-05165 | B | B | A | B | B | A |
| GT-05166 | A | A | A | A | B | A |
| GT-05167 | A | A | A | A | B | A |
| GT-05168 | B | B | B | A | B | A |
| GT-05169 | B | B | A | A | B | A |
| GT-05177 | B | B | B | B | B | A |
| GT-05179 | A | B | B | B | B | B |
| GT-05180 | A | B | B | B | B | B |
| GT-05181 | A | B | B | B | B | B |
| GT-05174 | A | B | B | B | B | B |
| GT-05175 | A | B | B | A | A | A |

(continued)

| Comp. No./names | WEE1 | IKZF1 | IKZF2 | IKZF3 | CK1α | GSPT1 |
|---|---|---|---|---|---|---|
| GT-05176 | B | B | B | A | B | B |
| GT-05249 | B | A | B | B | B | A |
| GT-05250 | B | B | A | B | B | B |
| GT-05251 | B | B | A | B | B | B |
| GT-05253 | B | B | B | B | B | A |
| GT-05254 | B | B | B | B | B | A |
| GT-05259 | B | A | A | B | B | A |
| GT-05260 | A | A | A | B | B | B |
| GT-05261 | B | B | A | B | B | B |
| GT-05351 | B | B | A | B | B | B |
| GT-05352 | B | B | A | B | B | A |
| GT-05353 | B | B | B | B | B | A |
| GT-05354 | B | B | A | A | B | A |
| GT-05355 | B | B | A | B | B | A |
| GT-05356 | B | B | A | B | B | B |
| GT-05357 | B | B | B | B | B | A |
| GT-05358 | B | B | B | B | B | A |
| GT-05267 | B | B | B | A | A | A |
| GT-05268 | B | B | B | A | A | A |
| GT-05269 | B | B | B | B | A | A |
| GT-05272 | B | B | A | B | B | B |
| GT-05273 | B | B | A | B | B | B |
| GT-05276 | B | B | B | A | B | A |
| GT-05309 | B | B | B | B | B | A |
| GT-05310 | B | B | B | B | B | A |
| GT-05313 | B | B | B | B | B | A |
| GT-05314 | B | B | B | B | B | A |
| GT-05315 | B | B | B | B | B | A |
| GT-05316 | B | B | B | B | B | A |
| GT-05317 | B | B | B | B | B | A |
| GT-05567 | A | A | B | A | A | A |
| GT-05568 | A | A | B | A | A | A |
| GT-05570 | A | A | A | A | A | A |
| GT-05575 | A | B | A | A | A | A |
| GT-05576 | A | B | A | A | A | A |
| GT-05580 | A | B | A | A | A | A |
| GT-05581 | A | B | B | A | A | A |
| GT-05582 | A | B | A | A | A | A |
| GT-05588 | B | B | B | A | B | B |

(continued)

| Comp. No./names | WEE1 | IKZF1 | IKZF2 | IKZF3 | CK1α | GSPT1 |
|---|---|---|---|---|---|---|
| GT-05589 | B | B | B | A | B | B |
| GT-05590 | B | B | B | A | B | B |
| GT-05591 | B | B | B | A | B | B |
| GT-05592 | B | B | B | A | B | B |
| GT-05593 | B | B | A | B | B | A |
| GT-05594 | B | B | A | B | B | A |
| GT-04990 | B | B | A | B | B | B |
| GT-05595 | B | B | A | B | B | B |
| GT-06388 | B | B | B | B | B | A |
| GT-06390 | B | B | B | B | B | A |
| GT-06392 | A | B | A | A | A | A |
| GT-06393 | A | B | A | A | A | A |
| GT-06394 | B | B | A | B | A | A |
| GT-06395 | B | B | B | B | A | A |
| GT-06396 | B | B | B | B | A | B |
| GT-06397 | A | B | A | B | B | A |
| GT-06376 | A | B | A | B | B | A |
| GT-06377 | A | B | A | B | B | B |
| GT-06378 | A | B | A | A | B | B |
| GT-06379 | A | B | A | A | B | A |
| GT-06385 | A | B | B | A | B | A |
| GT-06430 | A | A | B | A | B | A |
| GT-06431 | A | A | A | B | B | A |
| GT-06432 | A | A | B | B | B | A |
| GT-06433 | A | A | A | A | B | A |
| GT-06434 | A | B | A | A | B | A |
| GT-06435 | A | B | A | A | B | A |
| GT-06448 | B | B | A | A | B | A |
| GT-06449 | B | B | A | A | B | A |
| GT-06450 | A | B | A | A | B | A |
| GT-06451 | A | B | B | B | B | B |
| GT-06452 | A | B | B | B | B | B |
| GT-06453 | B | B | A | B | B | B |
| GT-06454 | A | B | B | B | B | B |
| GT-06455 | A | B | B | B | B | B |
| GT-06456 | A | A | A | A | A | A |
| GT-06457 | A | A | A | A | A | A |
| GT-06458 | A | A | A | A | A | A |
| GT-06459 | A | A | A | A | A | A |

(continued)

| Comp. No./names | WEE1 | IKZF1 | IKZF2 | IKZF3 | CK1α | GSPT1 |
|---|---|---|---|---|---|---|
| GT-06696 | B | B | B | B | B | A |
| GT-06698 | B | B | B | B | B | A |
| GT-06699 | B | B | B | B | B | A |
| GT-06701 | B | B | B | B | A | A |
| GT-06702 | B | B | B | B | A | A |
| GT-06703 | B | B | B | B | A | A |
| GT-06719 | B | B | B | A | B | B |
| GT-06720 | B | B | B | A | B | B |
| GT-06721 | B | A | A | A | B | B |
| GT-06722 | A | A | B | A | A | B |
| GT-06724 | A | A | B | B | B | B |
| GT-06725 | B | A | B | B | B | B |
| GT-06726 | A | B | A | A | A | B |
| GT-06727 | B | B | A | A | A | B |
| GT-06728 | B | B | A | A | A | B |
| GT-06765 | B | B | B | B | A | B |
| GT-06766 | A | A | A | A | A | A |
| GT-06767 | A | A | A | A | A | A |
| GT-06768 | A | B | B | A | A | A |
| GT-06769 | A | B | A | A | A | A |
| GT-06770 | A | A | A | A | A | A |
| GT-06771 | A | B | A | A | A | A |
| GT-06772 | A | B | A | A | A | A |
| GT-06773 | A | B | A | A | A | A |
| GT-06774 | B | B | A | A | A | A |
| GT-06775 | B | B | A | A | A | A |
| GT-06776 | A | B | A | B | A | A |
| GT-06777 | A | B | A | B | A | A |
| GT-06778 | B | B | B | B | A | A |
| GT-06779 | B | B | B | B | A | B |
| GT-06780 | B | B | B | B | A | B |
| GT-06781 | B | B | B | B | A | A |
| GT-06782 | B | B | B | B | A | A |
| GT-06783 | B | B | B | A | B | B |
| GT-06784 | B | B | B | B | A | B |
| GT-06793 | B | B | A | B | B | B |
| GT-06796 | A | B | B | B | B | B |
| GT-06797 | A | B | B | B | B | B |
| GT-06803 | B | B | B | B | A | B |

(continued)

| Comp. No./names | WEE1 | IKZF1 | IKZF2 | IKZF3 | CK1α | GSPT1 |
|---|---|---|---|---|---|---|
| GT-06804 | B | B | B | B | A | B |
| GT-06805 | B | B | B | B | A | B |
| GT-06877 | B | B | B | B | A | B |
| GT-06878 | B | B | B | B | A | A |
| GT-06879 | B | B | B | B | B | A |
| GT-06880 | A | B | B | B | B | B |
| GT-06882 | A | A | B | B | B | A |
| GT-06883 | A | A | B | B | B | A |
| GT-06884 | B | B | B | B | B | A |
| GT-06885 | B | A | A | B | B | A |
| GT-06886 | B | B | A | B | B | A |
| GT-06887 | A | B | A | B | B | A |
| GT-06888 | A | B | A | B | B | A |
| GT-06889 | A | B | A | B | B | A |
| GT-06890 | A | B | B | B | B | A |
| GT-06891 | A | B | A | B | B | A |
| GT-06892 | B | A | A | B | B | A |
| GT-06893 | B | A | A | B | B | A |
| GT-06894 | B | A | B | B | B | A |
| GT-06895 | B | A | B | B | B | B |
| GT-06896 | B | A | B | B | B | B |
| GT-05351 | A | B | A | B | B | A |
| GT-05353 | A | B | B | B | B | A |
| GT-05350 | B | B | B | B | B | A |
| GT-05354 | A | B | B | B | B | A |
| GT-05355 | B | B | B | B | B | A |
| GT-06965 | B | B | A | B | B | B |
| GT-06968 | A | B | A | B | B | B |
| GT-06969 | A | B | A | B | B | B |
| GT-06970 | A | B | A | B | B | B |
| GT-06971 | A | B | A | B | B | B |
| GT-06972 | A | B | A | B | B | B |
| GT-06973 | A | A | B | A | B | B |
| GT-06974 | A | A | B | A | B | B |
| GT-06979 | A | B | A | B | B | B |
| GT-07026 | A | B | B | B | B | A |
| GT-07027 | A | B | B | B | B | A |
| GT-05433 | A | B | B | B | B | A |
| GT-05434 | A | B | B | B | B | A |

(continued)

| Comp. No./names | WEE1 | IKZF1 | IKZF2 | IKZF3 | CK1α | GSPT1 |
|---|---|---|---|---|---|---|
| GT-05435 | A | B | B | B | B | A |
| GT-05436 | A | B | B | B | B | A |
| GT-05174 | B | B | A | B | B | A |
| GT-05175 | A | B | B | B | B | A |
| GT-05176 | A | B | B | B | B | A |
| GT-05356 | A | B | B | B | B | A |
| GT-05406 | A | B | A | B | B | A |
| GT-05357 | A | B | B | B | B | B |
| GT-05439 | A | B | B | B | B | B |
| GT-05697 | A | B | B | B | B | A |
| GT-07028 | A | B | B | B | B | A |
| GT-07029 | A | B | A | B | B | A |
| GT-07030 | B | B | A | B | B | B |
| GT-07031 | B | A | B | A | A | A |
| GT-07032 | B | B | B | B | B | A |
| GT-07033 | B | B | B | B | B | A |
| GT-07034 | A | A | A | A | A | A |
| GT-07035 | B | B | B | A | A | A |
| GT-07036 | B | B | B | B | B | A |
| GT-07037 | B | B | B | B | B | A |
| GT-07038 | A | A | A | A | A | A |
| GT-07069 | A | B | B | B | B | B |
| GT-07073 | A | B | B | B | B | B |
| GT-07160 | B | B | A | B | A | A |
| GT-07161 | B | B | A | B | B | A |
| GT-07162 | B | B | A | B | A | A |
| GT-07163 | B | B | A | B | B | A |
| GT-07164 | B | B | A | B | B | A |
| GT-07165 | B | B | A | B | B | A |
| GT-07166 | B | B | A | B | B | A |
| GT-07167 | B | B | A | B | B | A |
| GT-07168 | B | B | A | B | B | A |
| GT-07169 | A | B | B | B | B | B |
| GT-07170 | A | A | B | B | B | B |
| GT-07171 | A | B | B | B | B | B |
| GT-05432 | A | B | B | B | B | B |
| GT-07285 | A | A | B | B | B | B |
| GT-07291 | A | B | B | B | B | A |
| GT-07293 | A | B | A | A | B | A |

(continued)

| Comp. No./names | WEE1 | IKZF1 | IKZF2 | IKZF3 | CK1α | GSPT1 |
|---|---|---|---|---|---|---|
| GT-07295 | B | B | B | B | B | A |
| GT-07329 | A | B | B | B | B | A |
| GT-07330 | A | A | A | A | B | A |
| GT-07331 | A | A | B | A | A | A |
| GT-07296 | A | A | B | B | A | A |
| GT-07297 | A | B | A | A | A | A |
| GT-07376 | A | B | B | B | B | B |
| GT-07332 | A | A | B | A | B | A |
| GT-07333 | B | B | B | B | B | A |
| GT-07300 | A | B | B | B | B | B |
| GT-07301 | B | B | B | B | B | A |
| GT-07302 | A | B | B | B | B | B |
| GT-07377 | A | B | A | B | A | A |
| GT-07304 | B | B | B | B | A | A |
| GT-07334 | A | B | B | B | A | B |
| GT-07305 | B | B | A | A | B | A |
| GT-07306 | B | B | B | A | B | A |
| GT-07371 | B | B | B | A | B | A |
| GT-07372 | B | B | B | A | B | A |
| GT-07373 | B | B | B | A | B | A |
| GT-07374 | B | B | B | B | A | A |
| GT-07375 | B | B | B | B | A | A |
| GT-07420 | B | B | B | B | A | A |
| GT-07421 | B | B | B | B | A | A |
| GT-07422 | B | B | B | B | A | A |
| GT-07423 | A | B | B | A | A | A |
| GT-07442 | A | B | B | A | B | A |
| GT-07424 | A | A | B | A | B | A |
| GT-07425 | B | B | B | B | B | A |
| GT-07427 | A | A | B | B | A | A |
| GT-07428 | B | A | B | B | A | A |
| GT-07429 | B | A | B | B | B | B |
| GT-07430 | B | A | B | B | A | A |
| GT-07431 | B | A | B | B | A | B |
| GT-07432 | A | A | A | A | A | A |
| GT-07433 | B | B | A | B | A | B |
| GT-07434 | B | B | B | B | A | B |
| GT-07435 | B | B | B | B | A | B |
| GT-07436 | B | B | A | B | A | B |

(continued)

| Comp. No./names | WEE1 | IKZF1 | IKZF2 | IKZF3 | CK1α | GSPT1 |
|---|---|---|---|---|---|---|
| GT-07437 | A | B | B | B | A | A |
| GT-07438 | A | B | B | B | A | A |
| GT-07439 | B | B | B | B | A | A |
| GT-07440 | A | B | B | B | A | A |
| GT-07491 | A | B | B | B | B | A |
| GT-07492 | B | B | B | B | A | B |
| GT-07493 | B | B | B | A | A | B |
| GT-07494 | A | B | B | B | A | B |
| GT-07495 | B | B | B | B | A | A |
| GT-07496 | B | B | B | B | A | A |
| GT-07497 | A | A | A | B | A | A |
| GT-07500 | A | A | B | B | A | A |
| GT-07545 | A | A | B | B | A | A |
| GT-07502 | A | A | B | B | A | A |
| GT-07503 | A | A | B | B | A | A |
| GT-07508 | A | A | A | B | A | A |
| GT-07511 | A | A | A | B | A | A |
| GT-07522 | A | A | A | B | A | A |
| GT-07523 | A | A | A | B | A | A |
| GT-07524 | A | A | A | B | B | A |
| GT-07525 | B | B | B | B | A | A |
| GT-07526 | B | B | B | B | A | A |
| GT-07527 | B | B | B | B | A | A |
| GT-07528 | B | B | B | B | A | A |
| GT-07529 | B | B | B | B | A | A |
| GT-07530 | A | B | B | B | A | A |
| GT-07574 | A | B | A | B | A | A |
| GT-07575 | A | A | A | A | A | A |
| GT-07576 | A | A | A | B | A | A |
| GT-07577 | A | A | B | A | A | A |
| GT-07578 | A | A | A | B | B | A |
| GT-07579 | A | A | A | B | B | A |
| GT-07580 | A | A | A | A | B | A |
| GT-07581 | A | A | A | B | B | A |
| GT-07582 | A | A | A | B | B | A |
| GT-07583 | A | B | B | B | B | A |
| GT-07584 | A | A | B | B | B | A |
| GT-07585 | A | A | B | B | B | A |
| GT-07586 | A | A | B | B | B | A |

(continued)

| Comp. No./names | WEE1 | IKZF1 | IKZF2 | IKZF3 | CK1α | GSPT1 |
|---|---|---|---|---|---|---|
| GT-07587 | A | A | B | B | B | A |
| GT-07588 | B | B | B | B | B | A |
| GT-07589 | B | B | B | B | B | A |
| GT-07590 | A | A | A | A | B | A |
| GT-07642 | B | B | B | B | B | A |
| GT-07643 | B | B | B | B | B | A |
| GT-07644 | B | B | B | B | B | A |
| GT-07645 | B | A | B | B | B | A |
| GT-07681 | B | A | B | B | B | A |
| GT-07682 | B | B | B | B | B | A |
| GT-07683 | B | B | B | B | B | A |
| GT-07684 | B | B | B | B | B | A |
| GT-07685 | B | B | B | B | B | A |
| GT-07686 | B | B | B | B | B | A |
| GT-07687 | B | B | B | B | B | A |
| GT-07688 | B | B | B | B | B | A |
| GT-07689 | B | B | B | B | A | A |
| GT-07690 | B | B | A | B | A | A |
| GT-07711 | B | A | A | A | A | A |
| GT-07715 | B | B | B | B | A | A |
| GT-07718 | B | B | B | A | A | A |
| GT-07719 | B | B | A | A | A | A |
| GT-07720 | A | A | A | A | A | A |
| GT-07721 | A | A | A | A | A | A |
| GT-07722 | A | B | A | A | A | A |
| GT-07784 | B | B | A | B | B | B |
| GT-07786 | B | B | B | B | B | A |
| GT-08054 | A | A | A | A | A | A |
| GT-08055 | A | A | A | A | A | A |
| GT-08194 | B | A | B | B | B | A |
| GT-08057 | B | A | B | B | B | A |
| GT-08058 | B | A | B | B | B | B |
| GT-08059 | B | B | B | A | B | B |
| GT-08060 | B | B | A | A | B | B |
| GT-08061 | B | B | A | A | B | B |
| GT-08062 | B | B | A | A | B | B |
| GT-08063 | B | B | A | A | B | B |
| GT-08064 | B | B | A | B | B | B |
| GT-08065 | B | B | A | B | B | B |

(continued)

| Comp. No./names | WEE1 | IKZF1 | IKZF2 | IKZF3 | CK1α | GSPT1 |
|---|---|---|---|---|---|---|
| GT-08066 | B | B | A | B | A | B |
| GT-08067 | B | B | A | B | B | B |
| GT-08068 | B | B | A | B | A | B |
| GT-08077 | B | B | B | B | A | A |
| GT-08079 | A | B | B | B | A | A |
| GT-08080 | A | B | B | B | A | A |
| GT-08081 | A | B | B | B | A | A |
| GT-08082 | A | B | B | B | A | A |
| GT-08083 | B | B | B | B | A | A |
| GT-08084 | B | B | B | B | A | A |
| GT-08085 | B | B | B | B | A | A |
| GT-08088 | A | B | B | B | A | A |
| GT-08089 | A | B | B | B | A | A |
| GT-08090 | A | B | B | B | A | A |
| GT-08091 | A | B | B | B | A | A |
| GT-08092 | A | B | B | B | A | A |
| GT-08093 | A | B | B | B | A | A |
| GT-08094 | A | B | B | B | A | A |
| GT-08095 | A | B | B | B | A | B |
| GT-08096 | A | B | B | B | A | A |
| GT-08097 | A | B | B | B | A | A |
| GT-08098 | B | B | B | B | B | A |
| GT-08099 | B | B | B | B | B | A |
| GT-08103 | B | B | A | A | B | B |
| GT-08104 | A | B | A | A | B | A |
| GT-08105 | B | B | A | A | B | A |
| GT-08106 | A | B | B | B | B | A |
| GT-08107 | A | A | A | A | A | A |
| GT-08108 | A | A | A | A | A | A |
| GT-08109 | A | A | A | A | A | A |
| GT-08110 | A | A | A | A | A | A |
| GT-08111 | B | B | B | B | A | A |
| GT-08112 | B | B | B | B | B | A |
| GT-08113 | B | B | B | B | A | A |
| GT-08114 | B | B | B | B | B | A |
| GT-08115 | B | B | B | B | A | A |
| GT-08116 | A | A | B | A | A | A |
| GT-08117 | B | B | B | A | A | A |
| GT-07968 | A | A | A | A | A | B |

(continued)

| Comp. No./names | WEE1 | IKZF1 | IKZF2 | IKZF3 | CK1α | GSPT1 |
|---|---|---|---|---|---|---|
| GT-05406 | B | B | B | B | B | A |
| GT-05432 | A | B | B | B | B | A |
| GT-05433 | A | B | A | B | B | A |
| GT-05434 | A | B | A | B | A | A |
| GT-05436 | A | B | B | B | B | A |
| GT-05437 | B | B | B | B | B | A |
| GT-05438 | A | A | B | A | A | A |
| GT-05439 | A | B | B | A | A | A |
| GT-08424 | B | B | B | B | A | A |
| GT-08427 | A | B | A | B | B | B |
| GT-08428 | A | B | A | A | B | B |
| GT-08430 | A | B | B | B | B | B |
| GT-08431 | A | B | A | B | B | B |
| GT-08484 | A | A | A | A | A | A |
| GT-08432 | B | B | B | A | A | A |
| GT-08433 | B | B | B | A | A | A |
| GT-08434 | B | A | A | A | A | A |
| GT-08435 | A | A | B | A | B | A |
| GT-08437 | A | A | B | A | B | A |
| GT-08438 | A | A | B | A | B | A |
| GT-08439 | A | A | B | A | B | A |
| GT-08443 | A | B | B | B | B | B |
| GT-08444 | A | B | B | B | B | B |
| GT-08446 | B | B | B | A | B | A |
| GT-08949 | B | B | B | B | B | A |
| GT-08448 | B | B | B | B | B | A |
| GT-08450 | B | B | B | B | B | A |
| GT-08451 | B | B | B | A | A | B |
| GT-08452 | A | A | B | A | A | B |
| GT-08453 | B | A | B | A | A | A |
| GT-08455 | B | B | B | A | A | A |
| **Note:** In Table II-1, "A" is defined as 0% ≤ substrate protein remaining % ≤ 80%, and "B" is defined as 80%< substrate protein remaining %≤ 100%. | | | | | | |

The degradation effects of the compounds of the present invention on the target substrate proteins were shown in Table II-1 and Figure 1. From the experimental results in Table II-1 and Figure 1, it can be concluded that the compounds of the present invention significantly degrade the target substrate proteins (e.g., proteins WEE1, IKZF, CK1α, and GSPT1). Compared to lenalidomide, the compounds of the present invention exhibited significantly enhanced efficiency in inducing substrate protein degradation and also showed selective induction of degradation, thereby potentially enabling their use in treating indications associated with these substrates.

**III. Determination of CRBN-binding affinity of compounds:**

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:

**1.** According to the instructions of the CEREBLON BINDING KITS, serial dilutions were performed for the test compounds of the present disclosure (including the compounds in Table 1, and Examples 1-498) and for lenalidomide. Specifically, diluent #9 (1X) solution was used as the diluent, and multiple concentration gradients were set. The dilution operation can be performed according to one of the following two methods:

(1) The first dilution method: The test compounds of the present disclosure (including the compounds in Table 1, and Examples 1-498) were subjected to 5-fold serial dilutions starting from a highest concentration of 40 μM, resulting in a total of 7 concentrations from high to low (40, 8, 1.6, 0.32, 0.064, 0.0128, 0.00256 μM). Subsequently, the diluted test compounds and corresponding detection reagents were added to each well of a 96-well plate, resulting in final test compound concentrations of 10, 2, 0.4, 0.08, 0.016, 0.0032, and 0.00064 μM, respectively.

(2) As an alternative, a second dilution method can be used: The test compounds of the present disclosure (including the compounds in Table 1, and Examples 1-498) were subjected to 4-fold gradient dilutions starting from a highest concentration of 8 μM, resulting in 2 concentrations (8 μM, 2 μM). Into the 96-well plate, the diluted test compounds and corresponding detection reagents were added, resulting in final test compound concentrations of 2 μM and 0.5 μM, respectively. The experimental procedure is described below using the diluted 8 μM test compound and lenalidomide solutions as examples.

**2.** 2.5 μL of the above 8 μM of the tested compounds and lenalidomide solution, as well as the same volume of diluent #9 (1X) solution (solvent control group, Std0) were added to each well of a 96-well plate, respectively. Then, 2.5 μL of human Cereblon WT GST-tagged protein solution was added to each well. Finally, 5 μL of the thoroughly mixed Thalidomide-Re reagent and GST Eu antibody working solution were added to each of the aforementioned wells. The final concentration of the tested compounds and lenalidomide in each well was 2 μM.

**3**. The blank control wells were sequentially added with 2.5 μL of diluent #9 (1X) solution, 2.5 μL of binding buffer, and 5 μL of thoroughly mixed Thalidomide-Re reagent and GST Eu antibody working solution.

**4**. After sealing and incubating the solutions in the aforementioned wells at room temperature for 3 hours, the absorbance values at emission wavelengths of 620 nm and 665 nm were detected by the HTRF method using a Spark microplate reader (V3.1 SP1).

The corresponding CRBN binding inhibition rate was calculated using the following method:

$$\mathbf{R_{compound} = OD\ 665\ nm_{compound} / OD\ 620\ nm_{compound} - OD\ 665\ nm_{control} / OD\ 620\ nm_{control}}$$

$$\mathbf{R_{Std0} = OD\ 665\ nm_{Std0} / OD\ 620\ nm_{Std0} - OD\ 665\ nm_{control} / OD\ 620\ nm_{control}}$$

$$\mathbf{inhibition\ rate\ (\%) = (1 - R_{compound} / R_{Std0}) * 100}$$

Note: OD 665 nm $_{compound}$ is the absorbance value of each well of the compounds to be tested of the present disclosure at an emission wavelength of 665 nm.

OD 620 nm $_{compound}$ is the absorbance value of each well of the compounds to be tested of the present disclosure at an emission wavelength of 620 nm.

OD 665 nm $_{control}$ is the absorbance value of the blank control well at an emission wavelength of 665 nm. OD 620 nm $_{control}$ is the absorbance value of the blank control well at an emission wavelength of 620 nm. OD 665 nm $_{Std0}$ is the absorbance value of the solvent control well at an emission wavelength of 665 nm. OD 620 nm $_{Std0}$ is the absorbance value of the solvent control well at an emission wavelength of 620 nm.

The test results were shown in Tables III-1 and III-2 below.

**Table III-1.** HTRF inhibitory activity ($IC_{50}$, μM) of the compounds of the present disclosure (including but not limited to the compounds in Table 1, and the Examples 1-498 compounds)

| Comp. No. | HTRF $IC_{50}$ (μM) | Comp. No. | HTRF $IC_{50}$ (μM) |
|---|---|---|---|
| Lenalidomide | e | GT-06132 | c |
| GT-05073 | c | GT-06450 | d |
| GT-05568 | c | GT-07574 | d |
| Note: in Table III-1, the symbols a, b, c, d, and e are defined as follows: 0<a≤0.5μM, 0.5μM<b≤1μM, 1μM<c≤1.5μM, 1.5μM<d<1.9μM, 1.9μM ≤ e ≤10μM, 10μM < f. | | | |

**Table III-2:** Screening of CRBN Binding Affinity of the Compounds of the Present Invention (including but not limited to the compounds in Table 1, and Examples 1-498 Compounds) at Concentrations of 2 μM and 0.5 μM

| Comp. No. | Inhibition Rate at 2 μM (%) | Inhibition Rate at 0.5 μM (%) | Comp. No. | Inhibition Rate at 2 μM (%) | Inhibition Rate at 0.5 μM (%) |
|---|---|---|---|---|---|
| Lenalidomide | a4 | a2 | GT-08119 | a5 | a3 |
| GT-08065 | a6 | a2 | GT-08173 | a5 | a2 |
| GT-08066 | a6 | a2 | GT-08174 | a5 | a2 |
| GT-08079 | a5 | a2 | GT-08176 | a6 | a3 |
| GT-08080 | a5 | a2 | GT-08177 | a5 | a2 |
| GT-08087 | a6 | a2 | | | |
| Note: in Table III-2, the symbols a1, a2, a3, a4, a5, a6 , and a7 are defined as follows: 10% < a1 ≤ 20%, 20% < a2 ≤ 30%, 30% < a3 ≤ 40%, 40% < a4 ≤ 53%, 53% < a5 ≤ 60%, 60% < a6 ≤ 80%, 80% < a7 ≤100%. | | | | | |

The results in Table III-1 and Table III-2 demonstrate that the compounds of the present invention (including but not limited to the compounds in Table 1, and Examples 1-498) exhibit lower or comparable $IC_{50}$ values, or higher inhibition rates compared to lenalidomide, indicating stronger or comparable binding affinity to CRBN.

**IV. TNF-α Activity Inhibition Assay**

PBMCs cells were cultured at 37°C with 5% $CO_2$ and seeded in 96-well plates at $1\times10^7$ cells/well. The compounds to be tested (including those listed in Table 1, and Examples 1-93 compounds) were dissolved in DMSO and diluted to appropriate concentrations, ensuring that the final DMSO concentration in cell culture did not exceed 0.5%. After 1-hour incubation in medium with or without the compounds, the cells were stimulated with Lipopolysaccharide (LPS, 1 ng/ml) and continued to be cultured for 18-20 hours. Culture supernatants were then collected, diluted with serum-free medium, and analyzed for TNF-α levels using an ELISA kit. $IC_{50}$ values were calculated using GraphPad Prism 7.0.
TNF-α downregulation is a key mechanism for the anti-tumor action of immunomodulators. The compounds of the present disclosure demonstrated dose-dependent inhibition of TNF-α levels.

**V. Pharmacokinetic Study of the compounds of the present disclosure**

The pharmacokinetic properties of the compounds of the present disclosure were evaluated using conventional pharmacokinetic experimental methods.
The test compounds of the present disclosure (including the compounds in Table 1, and the Examples 1-93 compounds) were orally administered to experimental animals to evaluate their pharmacokinetic properties. The experimental animals used may be those commonly used in pharmacokinetic experiments, e.g., adult and healthy rodents (e.g., mice, rats (such as Sprague-Dawley (SD) rats), guinea pigs) or non-rodents (rabbits, dogs, and monkeys). The experimental animals used in this study were mice.
The experimental animals were divided into two groups: a control group (for blank plasma collection) and an oral administration group (dosed at 10 mg/kg or 30mg/kg). Blood samples were collected at predetermined time points after administration, such as 0.25h, 0.5h, 1h, 2h, 4h, 8h, and 24h post-dose. Blank plasma was collected from the control group.
Prior to analysis, plasma samples were processed as follows: To 10 μL of plasma sample were added 10 μL of 50% acetonitrile and 200 μL of internal standard solution (containing 50 ng/mL dexamethasone in acetonitrile). For blank

control plasma samples, no internal standard was added, instead an equivalent volume of acetonitrile was supplemented. The processed samples were vortex-mixed, centrifuged, and the supernatant was subjected to LC-MS/MS analysis.

After preparation of calibration curves and QC samples, the concentrations of compounds in plasma samples were determined by LC-MS/MS analysis. The pharmacokinetic parameters of the test compounds were calculated using the pharmacokinetic calculation software WinNonlin v8.1 with non-compartmental analysis based on the plasma concentration data obtained from LC-MS/MS.

The results demonstrated that the compounds of the present disclosure administered via oral route exhibited favorable drug metabolism and pharmacokinetic (DMPK) properties, indicating their potential as therapeutic agents for cancer patients.

## VI. Effects of the compounds of the present disclosure on TNF-α, IL-10, IL-6, IL-1β, IFN-γ, GM-CSF, IL-2, and IL-12p40 release from PBMCs induced by Lipopolysaccharide (LPS) or Phytohemagglutinin (PHA)

The following materials were used in this study:

VI.1 Reagents and Consumable materials

| Name | Supplier or Source |
|---|---|
| Human PBMC cell | Shanghai Sai Li |
| Lipopolysaccharide (LPS) | Sigma |
| Phytohemagglutinin (PHA) | MCE |
| RPMI 1640 Medium | Gibco |
| Human TNF-alpha DuoSet ELISA | R&D Systems |
| Human IL-6 DuoSet ELISA | R&D Systems |
| Human IL-10 DuoSet ELISA | R&D Systems |
| Human IFN-gamma DuoSet ELISA | R&D Systems |
| Human IL-2 DuoSet ELISA | R&D Systems |
| Human IL-1 beta ELISA Kit | Abclonal |
| Human GM-CSF ELISA Kit | Abclonal |
| Human IL-12/IL-23 p40 ELISA Kit | Abclonal |

VI.2 Methods

VI.2.1 Cell Thawing and Plating

A complete medium was prepared and pre-warmed to 37°C. Cryovials containing the cells were retrieved from liquid nitrogen, and the area immediately below the cap-notch interface was firmly gripped with forceps. The cryovials were immersed in a 37°C water bath, and shook occasionally to facilitate thawing. The cell suspension was aspirated and transferred to a centrifuge tube containing 10 mL complete medium, followed by thorough mixing and centrifugation at 400 × g for 10 min at room temperature. The supernatant was discarded, and the pellet was resuspended in fresh complete medium. After cell counting and the cell density adjustment, the cell suspension was seeded in 96-well plates at a density of $1\times10^5$ cells/well (75 μL/well) and incubated in a incubator for 2 h at 37°C with 5% $CO_2$ under high humidity conditions.

VI.2.2 Compound Preparation and Addition to Cell Plates

1) The test compounds were prepared as 10 mM stock solutions in DMSO.
2) The test compounds were serially diluted 5-fold in DMSO starting from 1 mM (highest concentration) to generate 9 concentration gradients.
3) The test compounds were further diluted in culture medium to five times their final working concentration intended for use.
4) The diluted compounds were added to designated wells according to the plate layout at 25 μL/well, achieving final

concentrations starting from 1 μM with 5-fold serial dilution (9 concentration points in total).

5) After 1 h incubation, LPS or PHA (25 μL/well) was added to the cell wells to reach final concentrations of: 1 μg/mL LPS (for TNF-α, IL-10, IL-6, GM-CSF and IL-12p40), 5 μg/mL LPS (IL-1β), 50 μg/mL LPS (IFN-γ), or 1 μg/mL PHA (IL-2).

6) The plates were incubated in a incubator for 24 h at 37°C with 5% $CO_2$ under high humidity.

Cell plate layout:

| | 1 | 2 | 3 | 4 | 5 | 6 | 7 | 8 | 9 | 10 | 11 | 12 |
|---|---|---|---|---|---|---|---|---|---|---|---|---|
| A | $H_2O$ | $H_2O$ | $H_2O$ | $H_2O$ | $H_2O$ | $H_2O$ | $H_2O$ | $H_2O$ | $H_2O$ | $H_2O$ | $H_2O$ | $H_2O$ |
| B | $H_2O$ | Cmpd 1# (1 μM Starting, 5-fold dilution, 9 points) | | | | | | | | | LPS/PHA | $H_2O$ |
| C | $H_2O$ | | | | | | | | | | | $H_2O$ |
| D | $H_2O$ | Cmpd 2# (1 μM Starting, 5-fold dilution, 9 points) | | | | | | | | | | $H_2O$ |
| E | $H_2O$ | | | | | | | | | | | $H_2O$ |
| F | $H_2O$ | Cmpd 3# (1 μM Starting, 5-fold dilution, 9 points) | | | | | | | | | Blank | $H_2O$ |
| G | $H_2O$ | | | | | | | | | | | $H_2O$ |
| H | $H_2O$ | $H_2O$ | $H_2O$ | $H_2O$ | $H_2O$ | $H_2O$ | $H_2O$ | $H_2O$ | $H_2O$ | $H_2O$ | $H_2O$ | $H_2O$ |

VI.2.3 Detection

(1) The cell plate was centrifuged and the culture supernatant was aspirated. TNF-α, IL-10, IL-6, IL-1β, IFN-γ, GM-CSF, IL-2, and IL-12p40 cytokines in the culture supernatant were detected according to the ELISA kit instructions.

The concentrations of TNF-α, IL-10, IL-6, IL-1β, IFN-γ, GM-CSF, IL-2, and IL-12p40 in the samples were calculated based on the standard curve. Inhibition rate % = [(Ac-As)/(Ac-Ab)]×100%

As: OA of the samples (cells + LPS/PHA + test compound)

Ac: OA of positive cell control (cells + LPS/PHA + DMSO)

Ab: OA of blank control (cells + culture medium + DMSO)

(2) $IC_{50}$ value was calculated as follows: using GraphPad Prism 6 software and applying the calculation formula XY-analysis/Nonlinear regression (curve fit)/Dose response-Inhibition/log (inhibitor) vs. response-Variable slope (four parameters) to perform $IC_{50}$ curve fitting and determine the $IC_{50}$ values.

The experimental results demonstrated that the compounds of the present disclosure can modulate the production levels of cytokines associated with autoimmune diseases and may be used for the treatment of autoimmune diseases.

**VII. Efficacy experiment of the compounds of the present disclosure on Psoriasis-like skin lesions induced by Imiquimod in Mice**

The compounds of the present disclosure (test compounds, including the compounds in Table 1, and Examples) were administered orally via gavage once daily for 7 consecutive days to mice with psoriasis-like skin lesions induced by imiquimod, to investigate their efficacy on such lesions.

A total of 60 female C57BL/6J mice (purchased from Shanghai SLAC Laboratory Animal Co., Ltd.) were randomly divided into 6 groups (n=10 per group) based on body weight:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 7 days |
| 2 | Model: Vehicle | 10 | NA | 10 | NA | ig. qd. 7 days |
| 3 | Positive Control | 10 | 3 | 10 | 0.3 | sc. qd. 7 days |
| 4 | Low-dose test compounds group | 10 | 3 | 10 | 0.3 | ig. qd. 7 days |
| 5 | Medium-dose test compounds group | 10 | 10 | 10 | 1 | ig. qd. 7 days |
| 6 | High-dose test compounds group | 10 | 30 | 10 | 3 | ig. qd. 7 days |
| Note: ig., oral gavage; sc., subcutaneous injection; qd., once daily. | | | | | | |

After grouping the experimental animals, the backs of all mice were shaved. Except for the Control group, the remaining groups were topically treated with imiquimod cream once daily for 7 consecutive days. Simultaneously, each group was administered with a vehicle control, a positive control drug, or the test compounds, according to the above table, once daily for 7 consecutive days.

All clinical symptoms of each animal were observed at the beginning and throughout the experiment. The animals' weights were measured and recorded every 2 days.

Scoring

Gross Observation Scoring of Skin Lesions (PASI Method): during the last 3 days of modeling and drug administration, the changes in skin lesions of mice in each group were observed once daily. PASI Method: the erythema, scaling, and thickening/infiltration of the lesions were scored separately, and the scores were summed to obtain the total score. PASI Scoring Criteria: 0 = none; 1 = mild; 2 = moderate; 3 = severe; 4 = very severe.

The skin lesions were photographed once daily during the last 3 days of modeling and drug administration.

After the experiment, the mice were euthanized by $CO_2$ inhalation. Three different areas of skin from the lesion site on the back were collected using a 6 mm punch, weighed, and recorded, and the average weight was calculated.

The skin tissue from the lesion site was homogenized and the levels of IL-23p19, IL-12p40, IL-17, and TNF-$\alpha$ were determined.

The local lesion tissue was fixed, stained with HE, and subjected to pathological scoring (based on epidermal thickness, degree of epidermal keratinization, thickness of the basal layer, and degree of dermal inflammatory cell infiltration). Scoring Criteria: 0 = none; 1 = mild; 2 = moderate; 3 = severe; 4 = very severe.

Experimental data were presented as Mean $\pm$ SD. Statistical analysis between two groups was performed using SPSS, with $p < 0.05$ considered statistically significant.

The experimental results demonstrated that the compounds of the present disclosure improve psoriasis-like skin lesions on the back of mice induced by imiquimod and may be used for the treatment of psoriasis.

**VIII. Efficacy Experiment of the compounds of the present disclosure on the CIA Model in Rats Induced by Type II Collagen**

The compounds of the present disclosure (test compounds, including the compounds in Table 1, and Examples 1-93) were administered orally via gavage once daily for 21 consecutive days to Wistar rats with collagen-induced arthritis (CIA) that was induced using bovine type II collagen (Chondrex, Inc.), in order to investigate their efficacy in the rat arthritis model.

Preparation of the Modeling Agent:

10 mg of CII (Immunization Grade Bovine Type II Collagen, purchased from Chondrex, Inc.) was dissolved in 5

mL of 0.05M acetic acid solution, allowing it to be fully dissolved, thereby preparing a solution with a concentration of 2 mg/mL, which was then stored at 4°C in the dark overnight. On the day of the experiment, the 2 mg/mL CII solution was mixed with an equal volume of a 4 mg/mL solution of Complete Freund's Adjuvant (CFA, also purchased from Chondrex, Inc.) on ice, and was thoroughly emulsified into an emulsion.

On day 0, 10 Wistar rats (purchased from Beijing Vital River Laboratory Animal Technology Co., Ltd.) were randomly selected as the blank control group and were not immunized. The remaining 100 Wistar rats were intradermally injected at the base of the tail with an emulsion prepared by mixing equal volumes of CII (2 mg/mL) and CFA (4 mg/mL) for the first immunization. A second booster immunization was administered to them on day 7. Between days 10 and 14, i.e., 3 to 7 days after the second booster immunization, 50 rats with an arthritis index (AI) score ranging from 1 to 2 were selected from the modeled animals and were divided into 5 groups based on their body weight, foot volume, and AI score, with 10 rats per group:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 21 days |
| 2 | Model: Vehicle | 10 | NA | 10 | NA | ig. qd. 21 days |
| 3 | Positive Control | 10 | 0.1 | 10 | 0.01 | sc. qd. 21 days |
| 4 | Low-dose test compounds group | 10 | 3 | 10 | 0.3 | ig. qd. 21 days |
| 5 | Medium-dose test compounds group | 10 | 10 | 10 | 1 | ig. qd. 21 days |
| 6 | High-dose test compounds group | 10 | 30 | 10 | 3 | ig. qd. 21 days |
| Note: ig. = oral gavage; sc. = subcutaneous injection | | | | | | |

Administration commenced between 3 and 7 days after the second booster immunization, once the AI score of the affected paws reached 1-2. According to the table above, animals in each group received daily oral gavage (or subcutaneous injection) of either the vehicle control, positive control drug, or the test compounds for 21 consecutive days.

Beginning on day 3 after the second booster immunization, the volume of both hind paws (or toes) was measured and recorded twice weekly.

Foot Volume Measurement: Before the measurement, a line was marked on the rat's ankle joint with a marker pen to indicate the position. After clean water was added to the instrument, the instrument's reading was reset to zero in preparation for the measurement. The hind limb of the rat was placed in the water so that the marked line at the ankle joint was at the surface of the liquid. The reading obtained by pressing the foot pedal at this time was the foot volume of the rat. After the measurement was completed, the foot pedal was pressed again to reset the instrument to zero, preparing it for the measurement of the next rat.

Scoring

Arthritis Index (AI): The foot volume was measured twice a week, and the swelling of the four toes was scored simultaneously. Each foot was scored on a scale from 0 to 4, with a maximum score of 16 for each rat.

Arthritis Index (AI) Scoring Criteria:

| Score | Degree |
|---|---|
| 0 | No swelling, normal appearance |
| 1 | Mild swelling or redness of the ankle, wrist, or finger joints |

(continued)

| Score | Degree |
|---|---|
| 2 | Moderate swelling or redness of the ankle, wrist, or finger joints |
| 3 | Severe swelling or redness of the ankle, wrist, or finger joints |
| 4 | Very severe swelling or redness of the ankle, wrist, or finger joints |

The day of starting drug administration is recorded as Day 0. Five days after starting drug administration, i.e., 2 hours after Day 5 administration, blood was collected from the jugular vein of the experimental animals, allowed to stand for more than 30 minutes, centrifuged to separate the serum, which was stored at -80°C. The levels of TNF-$\alpha$, IL-1$\beta$, and IL-6 in serum were measured by ELISA.

After the experiment, the animals were euthanized by $CO_2$ inhalation. The spleen and thymus of the animals were collected and weighed, and the organ coefficients were calculated.

Pathological Examination: One side of the foot joint tissue was fixed in 10% neutral formalin solution, followed by decalcification and paraffin embedding to prepare pathological sections. The sections were then stained with hematoxylin and eosin (HE) for pathological observation.

Observation Indices:

1. Infiltration of synovial inflammatory cells (lymphocytes, plasma cells);
2. Synovial tissue hyperplasia;
3. Synovial pannus;
4. Fibrinoid necrosis on the synovial surface.

Scoring and Counting Method: 0, 1, 2, 3, and 4 levels, where "0" indicates no observation; "1" is mild; "2" is moderate; "3" is severe; and "4" is extremely severe.

Experimental data were presented as Mean $\pm$ SEM. Data between two groups were analyzed using Excel-T test, with $p<0.05$ considered statistically significant. Scoring data were analyzed using the non-parametric Mann-Whitney U test in SPSS, with $p<0.05$ considered statistically significant.
The experimental results demonstrated that the compounds of the present disclosure can regulate the serum levels of inflammatory cytokines in rats with collagen-induced arthritis (CIA) model that was induced using bovine type II collagen, and significantly improve the swelling of rat limbs, and thus may be used for the treatment of arthritis.

The above descriptions represent only preferred embodiments of the present invention, but the scope of protection is not limited thereto. Any person skilled in the art, within the technical scope disclosed by the invention, may make equivalent replacements or modifications based on the technical solutions and inventive concepts of the present invention, and such changes shall fall within the protection scope of the invention.

## Claims

**1.** A compound of Formula (I)

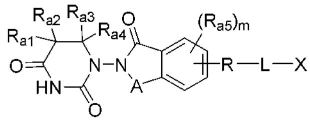

Formula (I)

or salts, enantiomers, stereoisomers, solvates, or polymorphs thereof,

wherein A represents CO, $CH_2$, or $CD_2$;
$R_{a1}$, $R_{a2}$, $R_{a3}$, and $R_{a4}$ each independently represent hydrogen, deuterium, halogen, $C_{1-6}$ alkyl, halogenated $C_{1-6}$ alkyl, deuterated $C_{1-6}$ alkyl, $C_{1-6}$ alkoxy, deuterated $C_{1-6}$ alkoxy, or halogenated $C_{1-6}$ alkoxy;
$(R_{a5})_m$ indicates that the isoindoline ring to which it is attached is optionally substituted with m $R_{a5}$, with each $R_{a5}$

being the same or different and each independently representing deuterium, halogen, hydroxy, mercapto, nitro, amino, cyano, $C_{1-6}$ alkyl, halogenated $C_{1-6}$ alkyl, deuterated $C_{1-6}$ alkyl, $C_{1-6}$ alkoxy, deuterated $C_{1-6}$ alkoxy, halogenated $C_{1-6}$ alkoxy, $C_{2-6}$ alkenyl or $C_{2-6}$ alkynyl;

m represents an integer of 0, 1, 2, or 3;

R represents O, S, S(O), $S(O)_2$, alkenylene, alkynylene, or $N(R_{a6})$, wherein $R_{a6}$ represents H or $C_{1-3}$ alkyl, or R represents a bond; or

R represents:

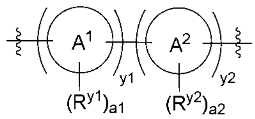

wherein each ring $A^1$ is the same or different and independently represents nitrogen-containing heterocyclylene, arylene, or heteroarylene, y1 represents an integer of 1 or 2, and $(R^{y1})_{a1}$ indicates that each ring $A^1$ is independently optionally substituted with a1 $R^{y1}$ substituents, wherein each $R^{y1}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a1 represents an integer of 0-20; each ring $A^2$ is the same or different and independently represents heterocyclylene, cycloalkylene, arylene, or heteroarylene, y2 represents an integer of 0 or 1, and $(R^{y2})_{a2}$ indicates that each ring $A^2$ is independently optionally substituted with a2 $R^{y2}$ substituents, wherein each $R^{y2}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a2 represents an integer of 0-20;

L represents:

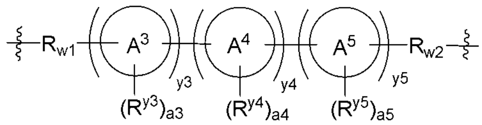

wherein $R_{w1}$ is linked to R, and $R_{w2}$ is linked to X;

$R_{w2}$ represents a bond, C(O), C(O)NH, NHC(O), or $N(R_{a6})$, wherein $R_{a6}$ represents H or $C_{1-3}$ alkyl; each ring $A^3$ is the same or different and independently represents heterocyclylene, cycloalkylene, arylene, or heteroarylene, y3 represents an integer of 0, 1, 2, or 3, and $(R^{y3})_{a3}$ indicates that each ring $A^3$ is independently optionally substituted with a3 $R^{y3}$ substituents, wherein each $R^{y3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a3 represents an integer of 0-20; each ring $A^4$ is the same or different and independently represents heterocyclylene, cycloalkylene, arylene, or heteroarylene, y4 represents an integer of 0, 1, 2, or 3, and $(R^{y4})_{a4}$ indicates that each ring $A^4$ is independently optionally substituted with a4 $R^{y4}$ substituents, wherein each $R^{y4}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a4 represents an integer of 0-20; each ring $A^5$ is the same or different and independently represents heterocyclylene, cycloalkylene, arylene, or heteroarylene, y5 represents an integer of 0, 1, 2, or 3, and $(R^{y5})_{a5}$ indicates that each ring $A^5$ is independently optionally substituted with a5 $R^{y5}$ substituents, wherein each $R^{y5}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a5 represents an integer of 0-20; $R_{w1}$ represents a bond or optionally substituted linear or branched $C_{1-20}$ alkylene, wherein any one or more methylene groups in the main carbon chain skeleton of the linear or branched $C_{1-20}$ alkylene are optionally replaced by one or more groups selected from $R_a$, $R_b$, and any combination thereof; wherein each $R_a$ is independently selected from the group consisting of: O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, where each $R_{a7}$ independently represents H or $C_{1-6}$ alkyl, and when any two or more methylene in the main carbon chain skeleton of the linear or branched $C_{1-20}$ alkylene are replaced by two or more $R_a$ groups, the $R_a$ groups are not directly connected to each other; and wherein each $R_b$ is independently selected from the group consisting of: optionally substituted cycloalkylene, optionally substituted arylene, optionally substituted heterocyclylene, optionally

substituted heteroarylene, alkynylene, alkenylene, and any combination thereof;

X represents:

$NR_1R_2$, $C(O)NR_3R_4$, $NHC(O)R_5$, $NHC(O)NR_6R_7$, $OR_8$, $SR_9$, $S(O)_2NR_{10}R_{11}$, or $N(R_{12})S(O)_2R_{13}$, wherein $R_1$, $R_3$, $R_4$, $R_5$, $R_6$, $R_7$, $R_9$, $R_{10}$, $R_{11}$, and $R_{12}$ each independently represent H, optionally substituted linear or branched alkyl, optionally substituted cycloalkyl, optionally substituted aryl, optionally substituted heterocyclyl, or optionally substituted heteroaryl, and wherein $R_2$, $R_8$, and $R_{13}$ each independently represent optionally substituted linear or branched alkyl, optionally substituted cycloalkyl, optionally substituted aryl, optionally substituted heterocyclyl, or optionally substituted heteroaryl, or wherein $R_3$ and $R_4$, together with the nitrogen atom to which they are attached, form an optionally substituted 4- to 30-membered nitrogen-containing heterocyclyl; or

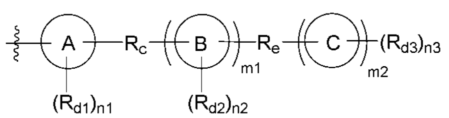

wherein ring A represents heterocyclylene, cycloalkylene, arylene, or heteroarylene, and $(R_{d1})_{n1}$ indicates that ring A is optionally substituted with n1 $R_{d1}$ substituents, wherein each $R_{d1}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n1 represents an integer of 0-20;
$R_c$ represents $NR_{c1}$, C(O), $CR_{c2}R_{c3}$, or a bond, wherein $R_{c1}$ represents H, optionally substituted linear or branched alkyl, optionally substituted cycloalkyl, optionally substituted aryl, optionally substituted heterocyclyl, or optionally substituted heteroaryl, wherein $R_{c2}$ and $R_{c3}$ each independently represent H, halogen, optionally substituted linear or branched alkyl, optionally substituted cycloalkyl, optionally substituted aryl, optionally substituted heterocyclyl, or optionally substituted heteroaryl;
each ring B is the same or different and independently represents heterocyclylene, cycloalkylene, arylene, or heteroarylene, m1 represents an integer of 0, 1, 2, or 3, and $(R_{d2})_{n2}$ indicates that each ring B is independently optionally substituted with n2 $R_{d2}$ substituents, wherein each $R_{d2}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n2 represents an integer of 0-20;
$R_c$ represents S, O, $CR_{c1}R_{c2}$, or a bond, wherein $R_{c1}$ and $R_{c2}$ each independently represent H, halogen, optionally substituted linear or branched alkyl, optionally substituted cycloalkyl, optionally substituted aryl, optionally substituted heterocyclyl, or optionally substituted heteroaryl; and
each ring C is the same or different and independently represents heterocyclyl, cycloalkyl, aryl, or heteroaryl, m2 represents an integer of 0, 1, 2, or 3, and $(R_{d3})_{n3}$ indicates that each ring C is independently optionally substituted with n3 $R_{d3}$ substituents, wherein each $R_{d3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n3 represents an integer of 0-20; or

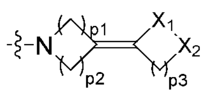

wherein $-X_1-X_2-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;
p1, p2, and p3 each independently represent an integer of 1, 2, 3, or 4; and
the ring containing $-X_1-X_2-$ is optionally substituted with one or more substituents selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, or any combination thereof; or

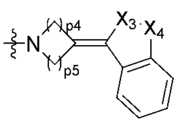

wherein $-X_3-X_4-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;
p4 and p5 each independently represent an integer of 1, 2, 3, or 4; and
the benzo-fused ring containing $-X_3-X_4-$ is optionally substituted with one or more substituents selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, $C_{2-6}$ alkenyl, or any combination thereof.

**2.** The compound of Formula (I) or salts, enantiomers, stereoisomers, solvates, or polymorphs thereof as claimed in claim 1, wherein

(i) $R_{a1}$, $R_{a2}$, $R_{a3}$, and $R_{a4}$ each independently represent H; and/or
(ii) A represents C(O); or

A represents $CH_2$; or
A represents $CD_2$; and/or

(iii) R represents O, S, S(O), $S(O)_2$, $C_{2-6}$ alkenylene, $C_{2-6}$ alkynylene, or $N(R_{a6})$, wherein $R_{a6}$ represents H or $C_{1-3}$ alkyl; preferably, R represents O, S, S(O), $S(O)_2$, $C_{2-4}$ alkenylene, $C_{2-4}$ alkynylene, or $N(R_{a6})$, wherein $R_{a6}$ represents H or $C_{1-3}$ alkyl; or

R represents a bond; or
R represents:

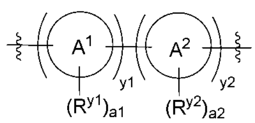

wherein each ring $A^1$ is the same or different and independently represents 4- to 30-membered nitrogen-containing heterocyclylene, $C_{5-30}$ arylene, or 5- to 30-membered heteroarylene (preferably, 4- to 20-membered nitrogen-containing heterocyclylene, $C_{5-20}$ arylene, or 5- to 20-membered heteroarylene; or more preferably, 4- to 15-membered nitrogen-containing heterocyclylene, $C_{5-15}$ arylene, or 5- to 15-membered heteroarylene), y1 represents an integer of 1 or 2, and $(R^{y1})_{a1}$ indicates that each ring $A^1$ is independently optionally substituted with a1 $R^{y1}$ substituents, wherein each $R^{y1}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a1 represents an integer of 0-20; each ring $A^2$ is the same or different and independently represents 4- to 30-membered heterocyclylene, $C_{3-30}$ cycloalkylene, $C_{5-30}$ arylene, or 5- to 30-membered heteroarylene (preferably, 4- to 20-membered heterocyclylene, $C_{3-20}$ cycloalkylene, $C_{5-20}$ arylene, or 5- to 20-membered heteroarylene; or more preferably, 4- to 15-membered heterocyclylene, $C_{3-15}$ cycloalkylene, $C_{5-15}$ arylene, or 5- to 15-membered heteroarylene), y2 represents an integer of 0 or 1, and $(R^{y2})_{a2}$ indicates that each ring $A^2$ is independently optionally substituted with a2 $R^{y2}$ substituents, wherein each $R^{y2}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a2 represents an integer of 0-20; and/or

(iv) L represents:

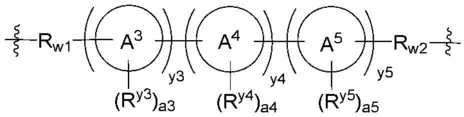

wherein $R_{w1}$ is linked to R, and $R_{w2}$ is linked to X;
$R_{w2}$ represents a bond, C(O), C(O)NH, NHC(O), or $N(R_{a6})$, wherein $R_{a6}$ represents H or $C_{1-3}$ alkyl; each ring $A^3$ is the same or different and independently represents 4- to 30-membered heterocyclylene, $C_{3-30}$ cycloalkylene, $C_{5-30}$ arylene, or 5- to 30-membered heteroarylene (preferably, 4- to 20-membered heterocyclylene, $C_{3-20}$ cycloalkylene, $C_{5-20}$ arylene, or 5- to 20-membered heteroarylene; or more preferably, 4- to

15-membered heterocyclylene, $C_{3-15}$ cycloalkylene, $C_{5-15}$ arylene, or 5- to 15-membered heteroarylene), y3 represents an integer of 0, 1, 2, or 3, and $(R^{y3})_{a3}$ indicates that each ring $A^3$ is independently optionally substituted with a3 $R^{y3}$ substituents, wherein each $R^{y3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a3 represents an integer of 0-20;

each ring $A^4$ is the same or different and independently represents 4- to 30-membered heterocyclylene, $C_{3-30}$ cycloalkylene, $C_{5-30}$ arylene, or 5- to 30-membered heteroarylene (preferably, 4- to 20-membered heterocyclylene, $C_{3-20}$ cycloalkylene, $C_{5-20}$ arylene, or 5- to 20-membered heteroarylene; or more preferably, 4- to 15-membered heterocyclylene, $C_{3-15}$ cycloalkylene, $C_{5-15}$ arylene, or 5- to 15-membered heteroarylene), y4 represents an integer of 0, 1, 2, or 3, and $(R^{y4})_{a4}$ indicates that each ring $A^4$ is independently optionally substituted with a4 $R^{y4}$ substituents, wherein each $R^{y4}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a4 represents an integer of 0-20;

each ring $A^5$ is the same or different and independently represents 4- to 30-membered heterocyclylene, $C_{3-30}$ cycloalkylene, $C_{5-30}$ arylene, or 5- to 30-membered heteroarylene (preferably, 4- to 20-membered heterocyclylene, $C_{3-20}$ cycloalkylene, $C_{5-20}$ arylene, or 5- to 20-membered heteroarylene; or more preferably, 4- to 15-membered heterocyclylene, $C_{3-15}$ cycloalkylene, $C_{5-15}$ arylene, or 5- to 15-membered heteroarylene), y5 represents an integer of 0, 1, 2, or 3, and $(R^{y5})_{a5}$ indicates that each ring $A^5$ is independently optionally substituted with a5 $R^{y5}$ substituents, wherein each $R^{y5}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a5 represents an integer of 0-20;

$R_{w1}$ represents a bond or optionally substituted linear or branched $C_{1-20}$ alkylene, wherein any one or more methylene groups in the main carbon chain skeleton of the linear or branched $C_{1-20}$ alkylene are optionally replaced by one or more groups selected from $R_a$, $R_b$, and any combination thereof; wherein each $R_a$ is independently selected from the group consisting of: O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, where each $R_{a7}$ independently represents H or $C_{1-6}$ alkyl, and when any two or more methylene in the main carbon chain skeleton of the linear or branched $C_{1-20}$ alkylene are replaced by two or more $R_a$ groups, the $R_a$ groups are not directly connected to each other; and wherein each $R_b$ is independently selected from the group consisting of: optionally substituted $C_{3-30}$ cycloalkylene, optionally substituted $C_{5-30}$ arylene, optionally substituted 4- to 30-membered heterocyclylene, optionally substituted 5- to 30-membered heteroarylene, $C_{2-6}$ alkenylene, or $C_{2-6}$ alkynylene (preferably, optionally substituted $C_{3-20}$ cycloalkylene, optionally substituted $C_{5-20}$ arylene, optionally substituted 4- to 20-membered heterocyclylene, optionally substituted 5- to 20-membered heteroarylene, $C_{2-4}$ alkenylene, or $C_{2-4}$ alkynylene; or more preferably, optionally substituted $C_{3-15}$ cycloalkylene, optionally substituted $C_{5-15}$ arylene, optionally substituted 4- to 15-membered heterocyclylene, optionally substituted 5- to 15-membered heteroarylene, $C_{2-4}$ alkenylene, or $C_{2-4}$ alkynylene) or any combination thereof; and/or

(v) X represents:

$NR_1R_2$, $C(O)NR_3R_4$, $NHC(O)R_5$, $NHC(O)NR_6R_7$, $OR_8$, $SR_9$, $S(O)_2NR_{10}R_{11}$, or $N(R_{12})S(O)_2R_{13}$, wherein $R_1$, $R_3$, $R_4$, $R_5$, $R_6$, $R_7$, $R_9$, $R_{10}$, $R_{11}$, and $R_{12}$ each independently represent H, optionally substituted linear or branched $C_{1-10}$ alkyl (preferably, optionally substituted linear or branched $C_{1-6}$ alkyl), optionally substituted $C_{3-30}$ cycloalkyl (preferably, optionally substituted $C_{3-20}$ cycloalkyl; more preferably, optionally substituted $C_{3-15}$ cycloalkyl), optionally substituted $C_{5-30}$ aryl (preferably, optionally substituted $C_{5-20}$ aryl; more preferably, optionally substituted $C_{5-15}$ aryl), optionally substituted 4- to 30-membered heterocyclyl (preferably, optionally substituted 4- to 20-membered heterocyclyl; more preferably, optionally substituted 4- to 15-membered heterocyclyl) or optionally substituted 5- to 30-membered heteroaryl (preferably, optionally substituted 5- to 20-membered heteroaryl; more preferably, optionally substituted 5- to 15-membered heteroaryl), and $R_2$, $R_8$, and $R_{13}$ each independently represent optionally substituted linear or branched $C_{1-10}$ alkyl (preferably, optionally substituted linear or branched $C_{1-6}$ alkyl), optionally substituted $C_{3-30}$ cycloalkyl (preferably, optionally substituted $C_{3-20}$ cycloalkyl; more preferably, optionally substituted $C_{3-15}$ cycloalkyl), optionally substituted $C_{5-30}$ aryl (preferably, optionally substituted $C_{5-20}$ aryl; more preferably, optionally substituted $C_{5-15}$ aryl), optionally substituted 4- to 30-membered heterocyclyl (preferably, optionally substituted 4- to 20-membered heterocyclyl; more preferably, optionally substituted 4- to 15-membered heterocyclyl) or optionally substituted 5- to 30-membered heteroaryl (preferably, optionally substituted 5- to 20-membered heteroaryl; more preferably, optionally substituted 5- to 15-membered heteroaryl), or wherein $R_3$ and $R_4$, together with the nitrogen atom to which they are attached, form an optionally substituted 4- to 30-membered nitrogen-containing heterocyclyl; or

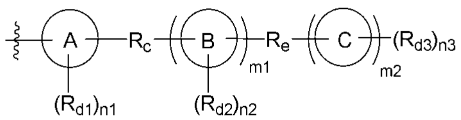

wherein ring A represents 4- to 30-membered heterocyclylene (preferably, 4- to 20-membered heterocyclylene; more preferably, 4- to 15-membered heterocyclylene), $C_{3-30}$ cycloalkylene (preferably, $C_{3-20}$ cycloalkylene; more preferably, $C_{3-15}$ cycloalkylene), $C_{5-30}$ arylene (preferably, $C_{5-20}$ arylene; more preferably, $C_{5-15}$ arylene) or 5- to 30-membered heteroarylene (preferably, 5- to 20-membered heteroarylene; more preferably, 5- to 15-membered heteroarylene), and $(R_{d1})_{n1}$ indicates that ring A is optionally substituted with n1 $R_{d1}$ substituents, wherein each $R_{d1}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n1 represents an integer of 0-20;

$R_c$ represents $NR_{c1}$, C(O), $CR_{c2}R_{c3}$, or a bond, wherein $R_{c1}$ represents H, optionally substituted linear or branched $C_{1-10}$ alkyl (preferably, optionally substituted linear or branched $C_{1-6}$ alkyl), optionally substituted $C_{3-30}$ cycloalkyl (preferably, optionally substituted $C_{3-20}$ cycloalkyl; more preferably, optionally substituted $C_{3-15}$ cycloalkyl), optionally substituted $C_{5-30}$ aryl (preferably, optionally substituted $C_{5-20}$ aryl; more preferably, optionally substituted $C_{5-15}$ aryl), optionally substituted 4- to 30-membered heterocyclyl (preferably, optionally substituted 4- to 20-membered heterocyclyl; more preferably, optionally substituted 4- to 15-membered heterocyclyl) or optionally substituted 5- to 30-membered heteroaryl (preferably, optionally substituted 5- to 20-membered heteroaryl; more preferably, optionally substituted 5- to 15-membered heteroaryl), wherein $R_{c2}$ and $R_{c3}$ each independently represent H, halogen, optionally substituted linear or branched $C_{1-10}$ alkyl (preferably, optionally substituted linear or branched $C_{1-6}$ alkyl), optionally substituted $C_{3-30}$ cycloalkyl (preferably, optionally substituted $C_{3-20}$ cycloalkyl; more preferably, optionally substituted $C_{3-15}$ cycloalkyl), optionally substituted $C_{5-30}$ aryl (preferably, optionally substituted $C_{5-20}$ aryl; more preferably, optionally substituted $C_{5-15}$ aryl), optionally substituted 4- to 30-membered heterocyclyl (preferably, optionally substituted 4- to 20-membered heterocyclyl; more preferably, optionally substituted 4- to 15-membered heterocyclyl) or optionally substituted 5- to 30-membered heteroaryl (preferably, optionally substituted 5- to 20-membered heteroaryl; more preferably, optionally substituted 5- to 15-membered heteroaryl);

each ring B is the same or different and independently represents 4- to 30-membered heterocyclylene (preferably, 4- to 20-membered heterocyclylene; more preferably, 4- to 15-membered heterocyclylene), $C_{3-30}$ cycloalkylene (preferably, $C_{3-20}$ cycloalkylene; more preferably, $C_{3-15}$ cycloalkylene), $C_{5-30}$ arylene (preferably, $C_{5-20}$ arylene; more preferably, $C_{5-15}$ arylene) or 5- to 30-membered heteroarylene (preferably, 5- to 20-membered heteroarylene; more preferably, 5- to 15-membered heteroarylene), m1 represents an integer of 0, 1, 2, or 3, and $(R_{d2})_{n2}$ indicates that each ring B is independently optionally substituted with n2 $R_{d2}$ substituents, wherein each $R_{d2}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n2 represents an integer of 0-20;

$R_c$ represents S, O, $CR_{c1}R_{c2}$, or a bond, wherein $R_{c1}$ and $R_{c2}$ each independently represent H, halogen, optionally substituted linear or branched $C_{1-10}$ alkyl (preferably, optionally substituted linear or branched $C_{1-6}$ alkyl), optionally substituted $C_{3-30}$ cycloalkyl (preferably, optionally substituted $C_{3-20}$ cycloalkyl; more preferably, optionally substituted $C_{3-15}$ cycloalkyl), optionally substituted $C_{5-30}$ aryl (preferably, optionally substituted $C_{5-20}$ aryl; more preferably, optionally substituted $C_{5-15}$ aryl), optionally substituted 4- to 30-membered heterocyclyl (preferably, optionally substituted 4- to 20-membered heterocyclyl; more preferably, optionally substituted 4- to 15-membered heterocyclyl) or optionally substituted 5- to 30-membered heteroaryl (preferably, optionally substituted 5- to 20-membered heteroaryl; more preferably, optionally substituted 5- to 15-membered heteroaryl); and

each ring C is the same or different and independently represents 4- to 30-membered heterocyclyl (preferably, 4- to 20-membered heterocyclyl; more preferably, 4- to 15-membered heterocyclyl), $C_{3-30}$ cycloalkyl (preferably, $C_{3-20}$ cycloalkyl; more preferably, $C_{3-15}$ cycloalkyl), $C_{5-30}$ aryl (preferably, $C_{5-20}$ aryl; more preferably, $C_{5-15}$ aryl) or 5- to 30-membered heteroaryl (preferably, 5- to 20-membered heteroaryl; more preferably, 5- to 15-membered heteroaryl), m2 represents an integer of 0, 1, 2, or 3, and $(R_{d3})_{n3}$ indicates that each ring C is independently optionally substituted with n3 $R_{d3}$ substituents, wherein each $R_{d3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n3

represents an integer of 0-20; or

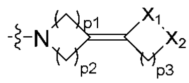

wherein $-X_1-X_2-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;
p1, p2, and p3 each independently represent an integer of 1, 2, or 3; and
ring containing $-X_1-X_2-$ is optionally substituted with one or more substituents selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, $C_{2-6}$ alkenyl, or any combination thereof; or

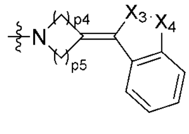

wherein $-X_3-X_4-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;
p4 and p5 each independently represent an integer of 1, 2, or 3; and
the benzo-fused ring containing-$X_3-X_4-$ is optionally substituted with one or more substituents selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, $C_{2-6}$ alkenyl, or any combination thereof.

**3.** The compound of Formula (I) or salts, enantiomers, stereoisomers, solvates, or polymorphs thereof as claimed in claim 1 or 2, wherein

(i) R represents O, S, S(O), $S(O)_2$, alkenylene, alkynylene, NH, or $N(CH_3)$, or R represents a bond; or
(ii) each ring $A^1$ independently represents:

piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, 3-methyl-2-oxa-8-azaspiro[4.5]decenylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene; or preferably,

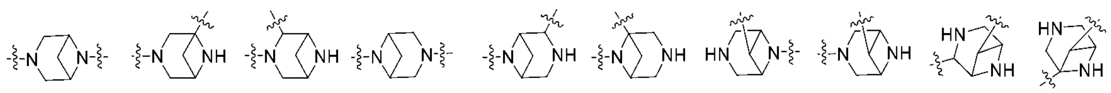

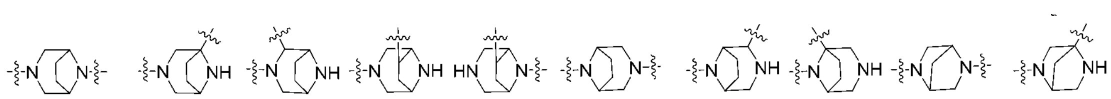

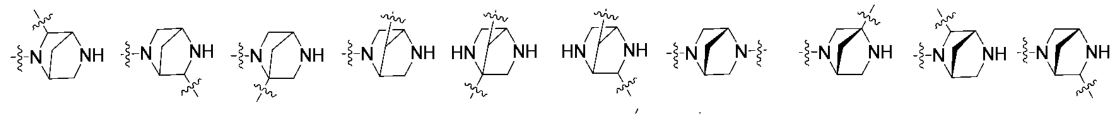

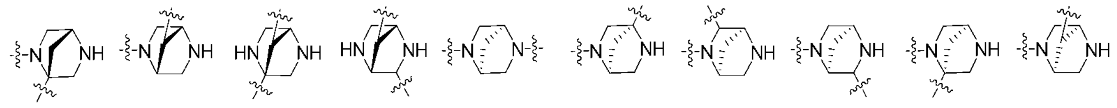

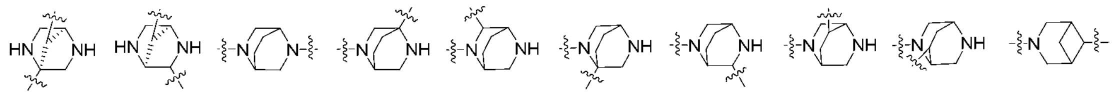

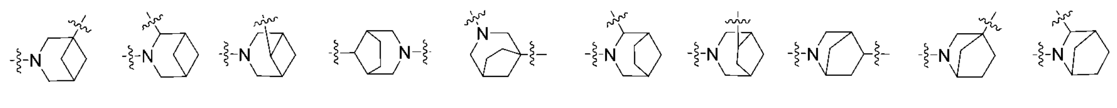

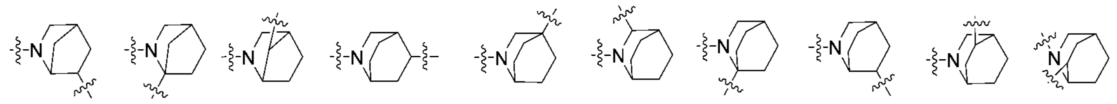

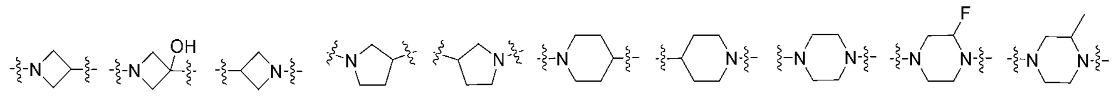

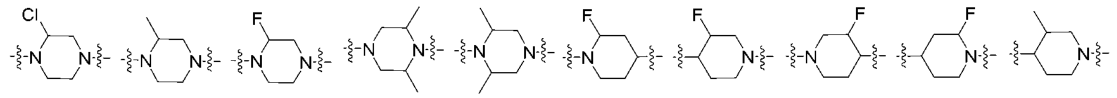

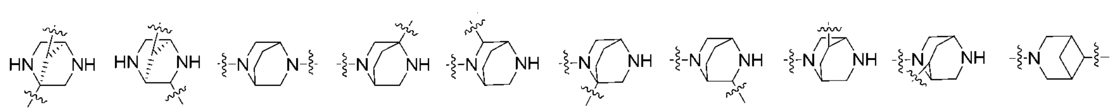

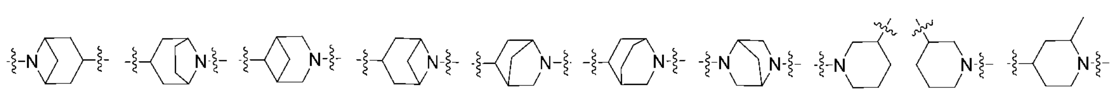

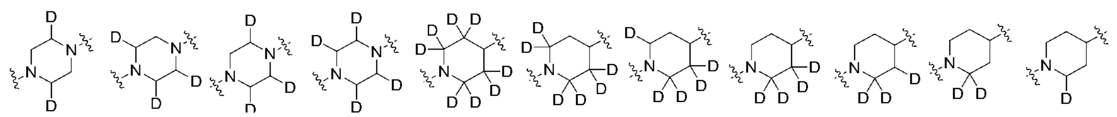

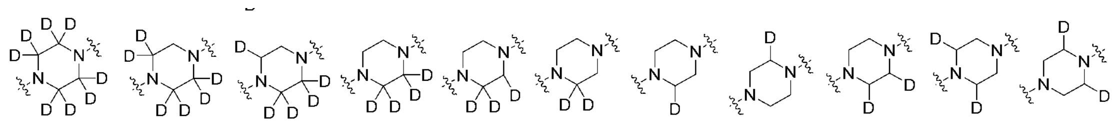

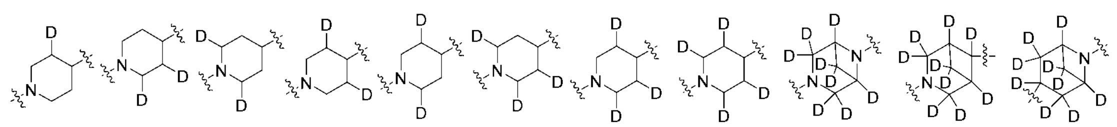

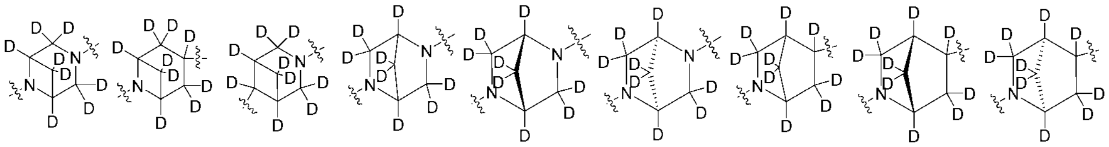

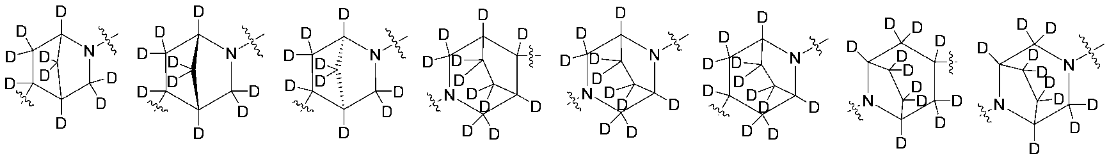

, or

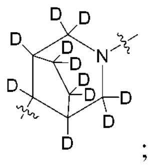

;

each ring $A^1$ is independently optionally substituted with a1 $R^{y1}$ substituents, wherein each $R^{y1}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl; and a1 represents an integer of 0-20;
and/or
each ring $A^2$ independently represents:

azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, $C_{5-15}$ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene; or preferably,

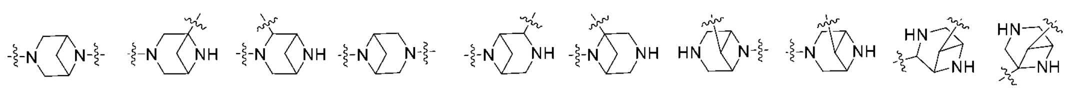

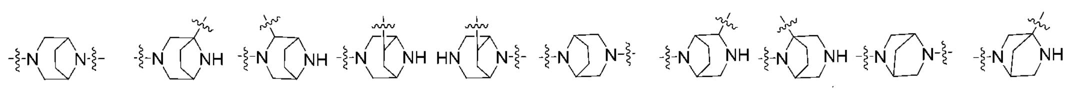

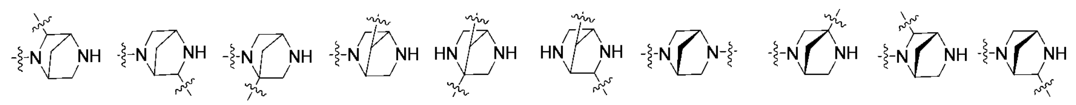

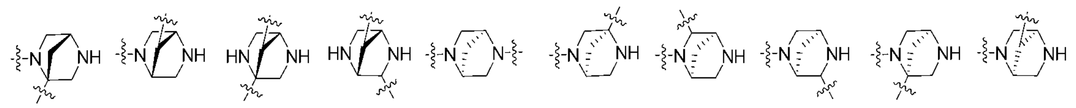

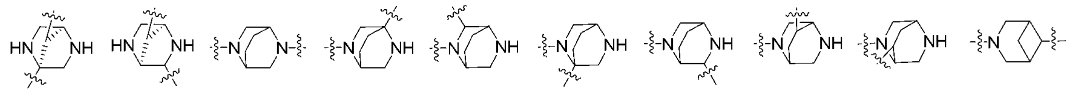

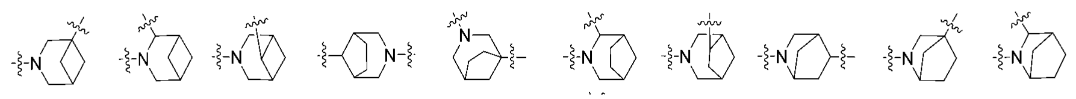

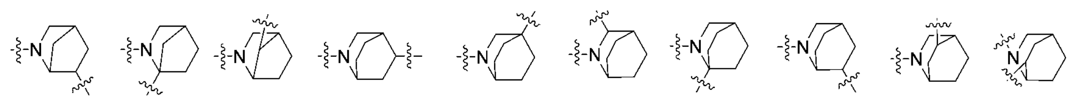

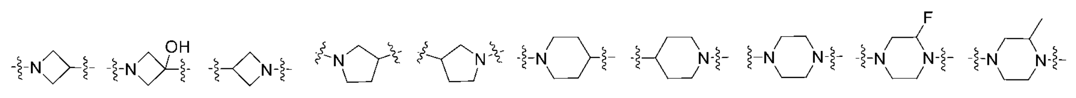

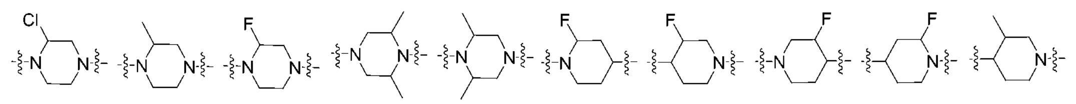

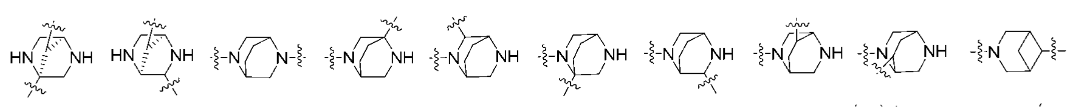

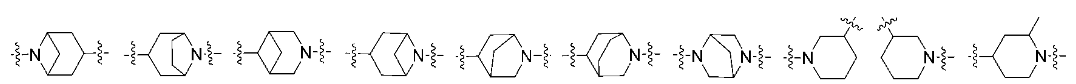

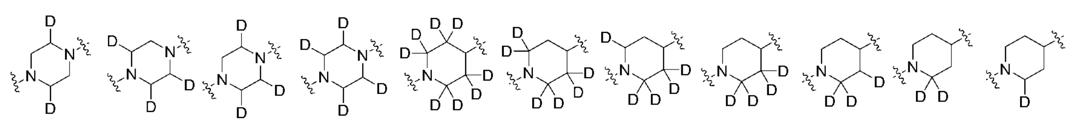

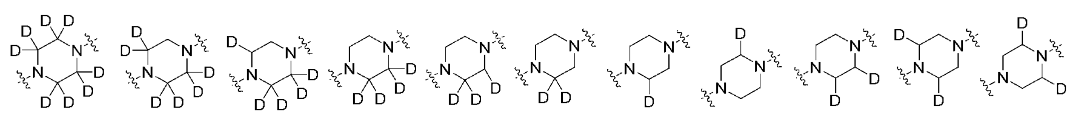

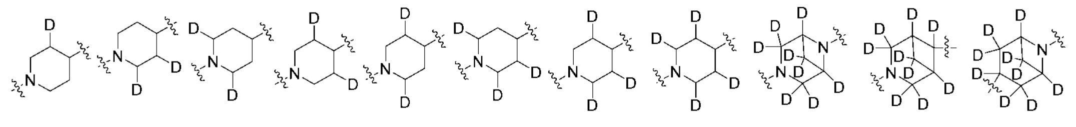

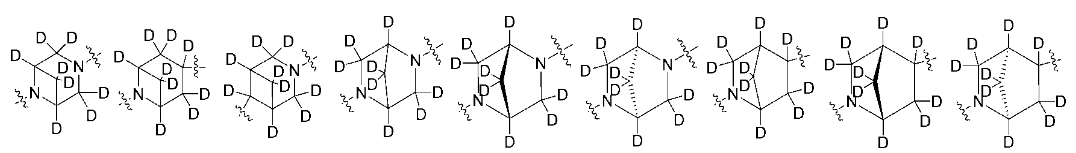

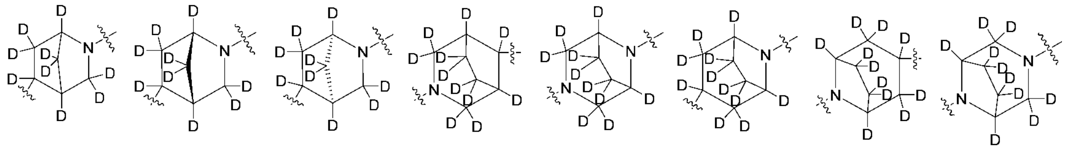

, or

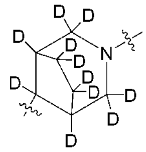

;

each ring $A^2$ is independently optionally substituted with a2 $R^{y2}$ groups, wherein each $R^{y2}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a2 represents an integer of 0-20;

preferably, R represents:

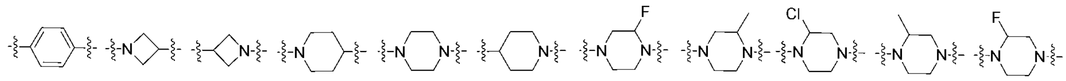

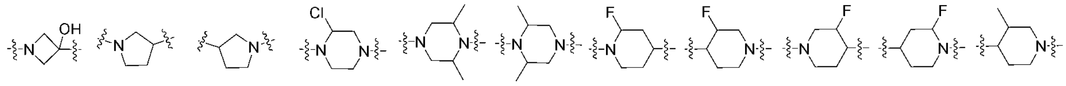

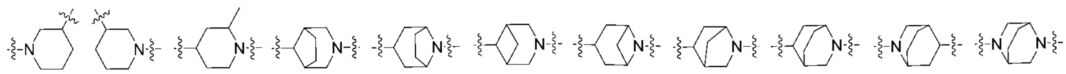

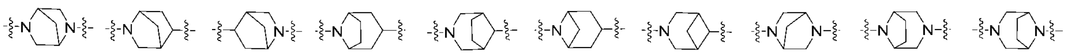

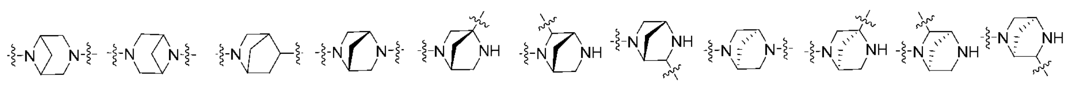

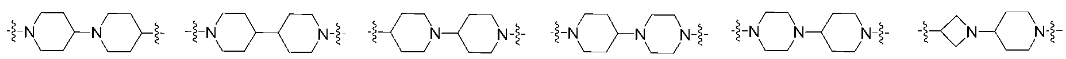

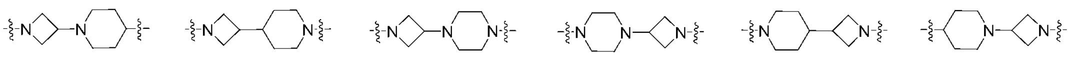

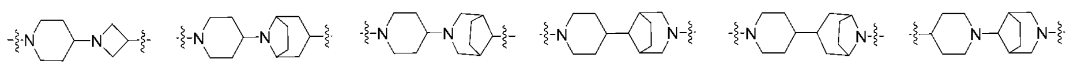

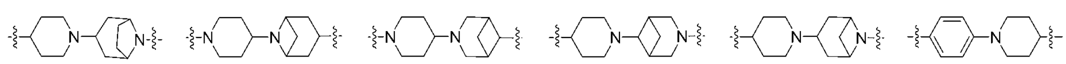

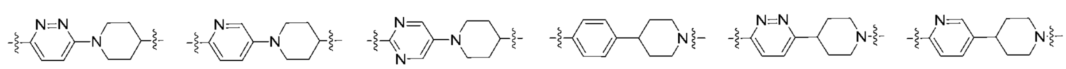

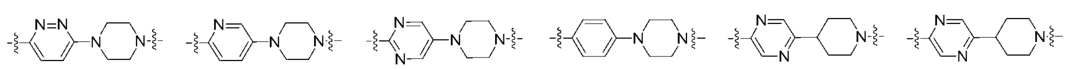

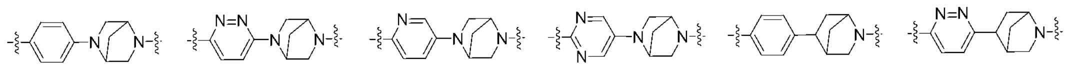

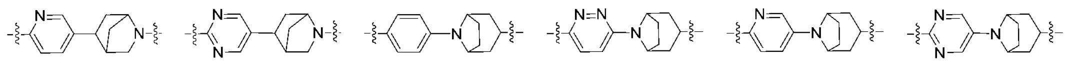

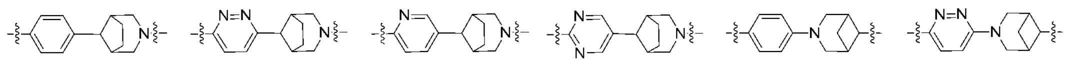

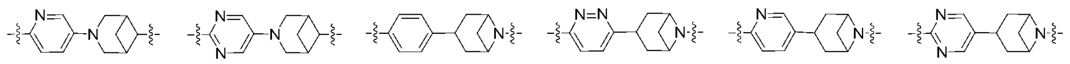

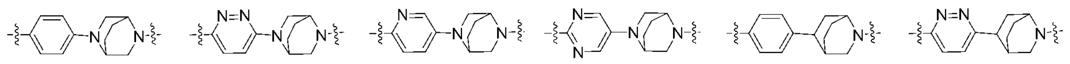

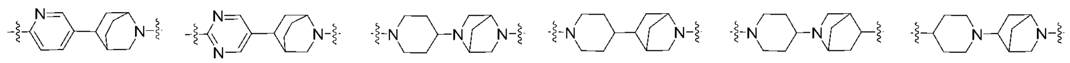

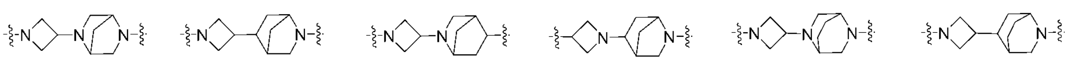

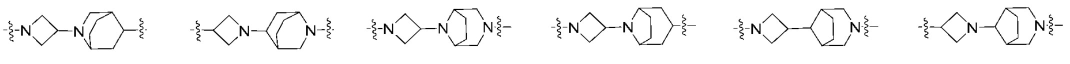

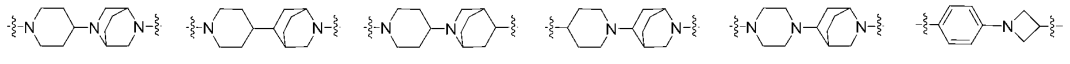

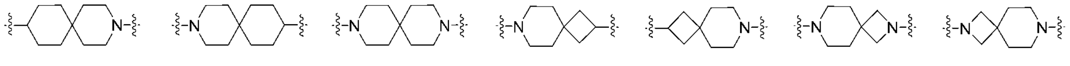

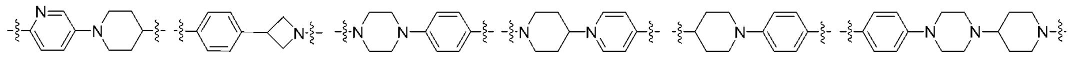

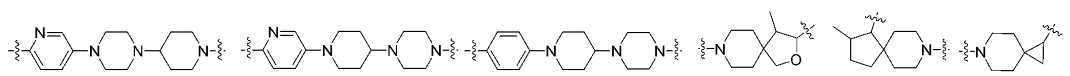

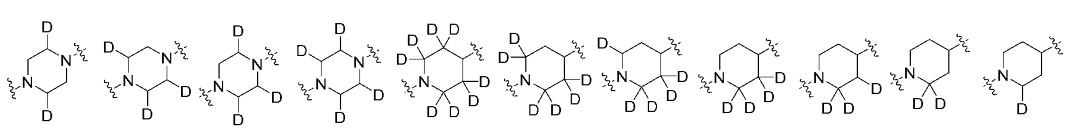

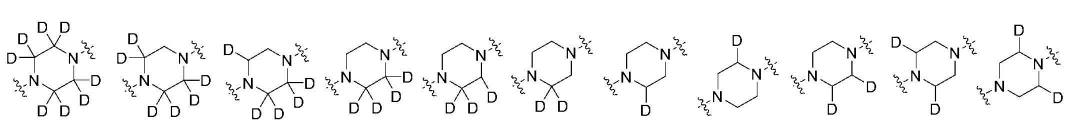

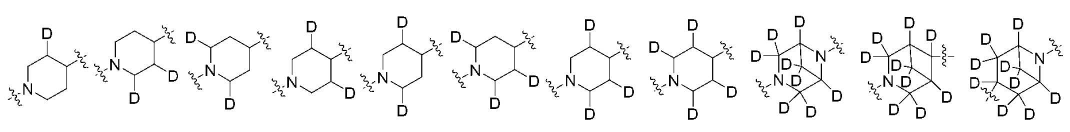

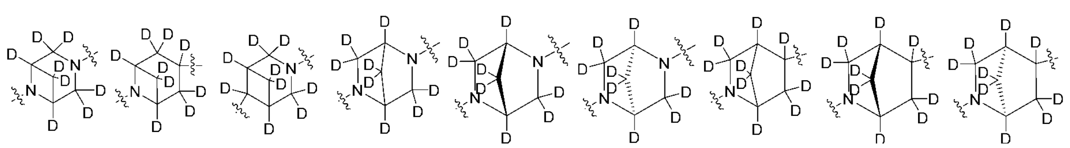

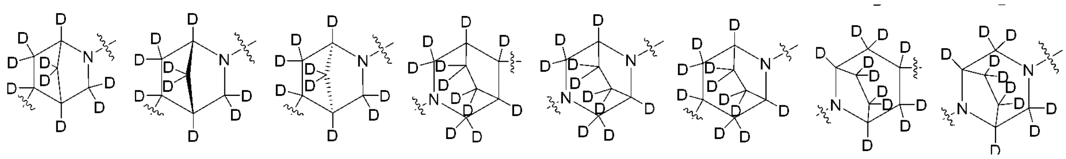

, or

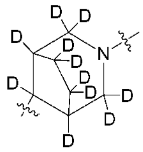

;

wherein the groups are optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl;
and/or

(iii) ring $A^3$, ring $A^4$, and ring $A^5$ each independently represent:

azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, $C_{5-15}$ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene; or preferably ring $A^3$, ring $A^4$, and ring $A^5$ each independently represent:

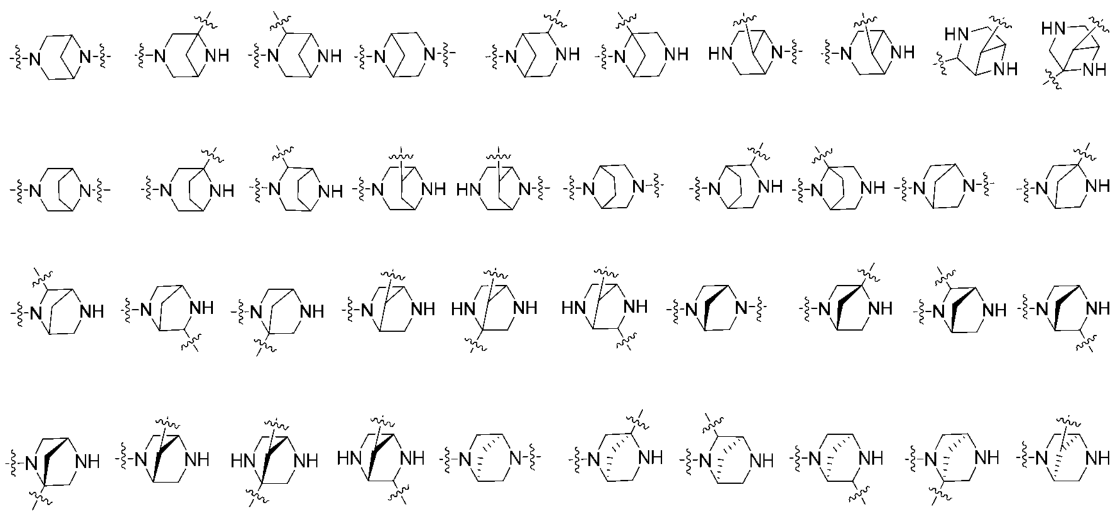

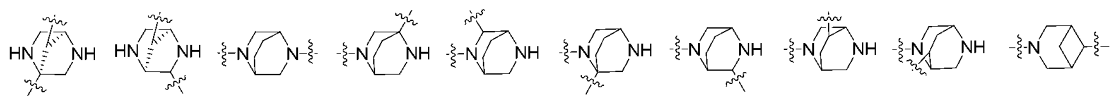

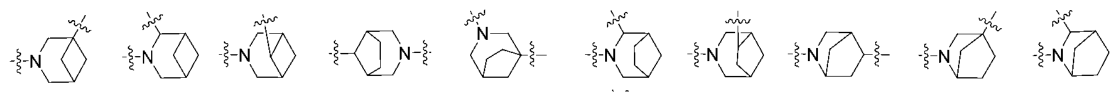

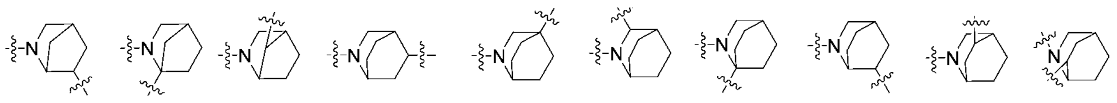

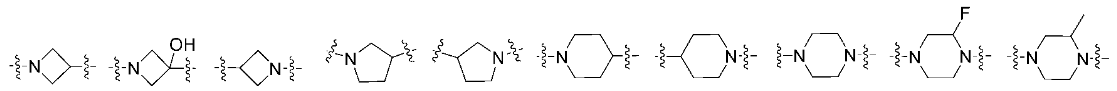

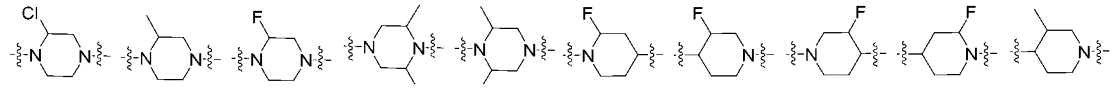

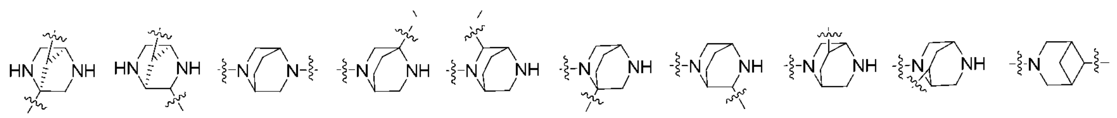

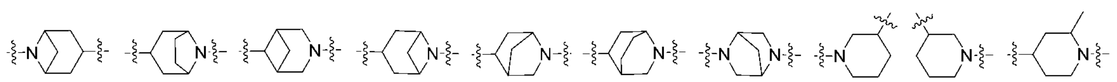

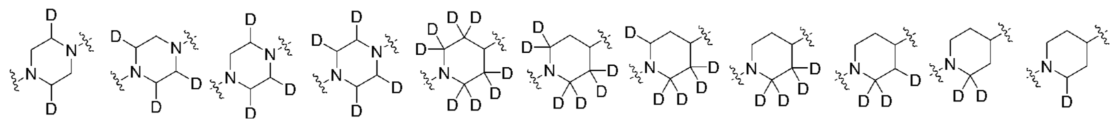

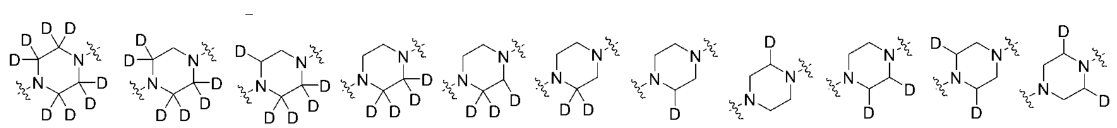

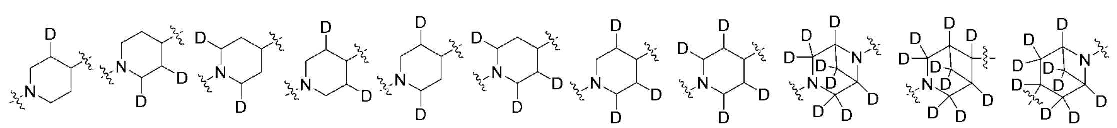

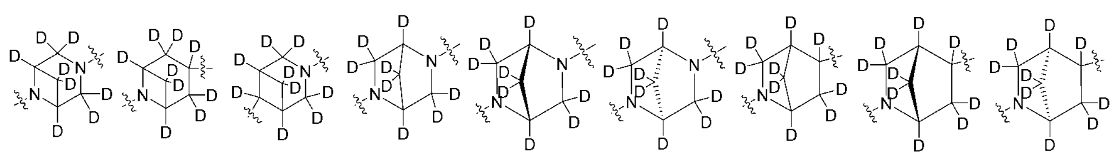

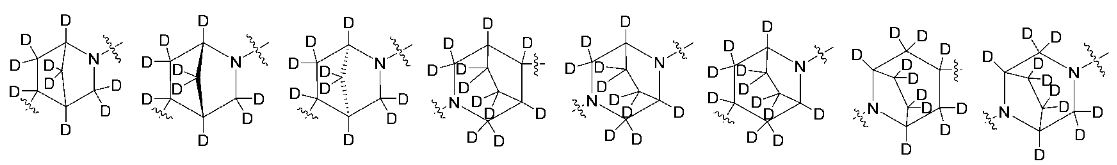

, or

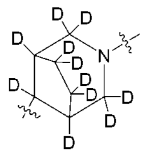
;

and

y3 represents an integer of 0, 1, 2, or 3, and each ring $A^3$ is independently optionally substituted with a3 $R^{y3}$ substituents, with each $R^{y3}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a3 represents an integer of 0-20;

y4 represents an integer of 0, 1, 2, or 3, and each ring $A^4$ is independently optionally substituted with a4 $R^{y4}$ substituents, with each $R^{y4}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a4 represents an integer of 0-20; or

y5 represents an integer of 0, 1, 2, or 3, and each ring $A^5$ is independently optionally substituted with a5 $R^{y5}$ substituents, with each $R^{y5}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, carboxyl, $C_{2-6}$ alkynyl or $C_{2-6}$ alkenyl, and a5 represents an integer of 0-20.

**4.** The compound of Formula (I) or salts, enantiomers, stereoisomers, solvates, or polymorphs thereof as claimed in claim 1, wherein the

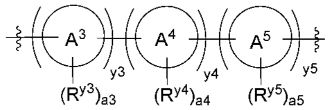

moiety in the general formula of L represents:

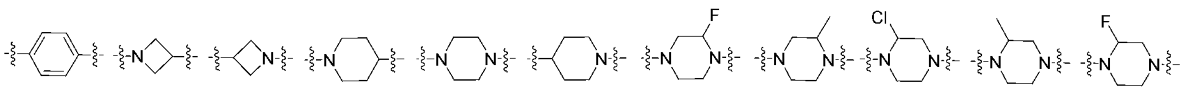

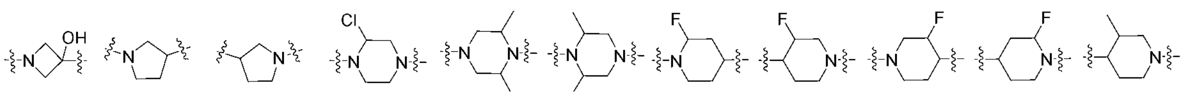

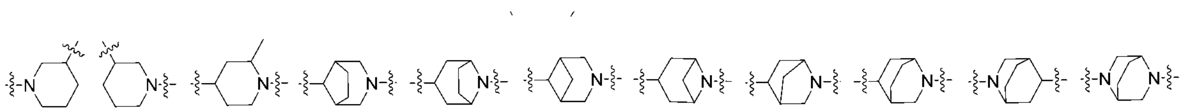

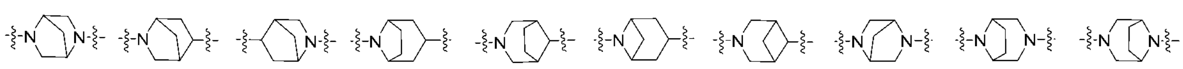

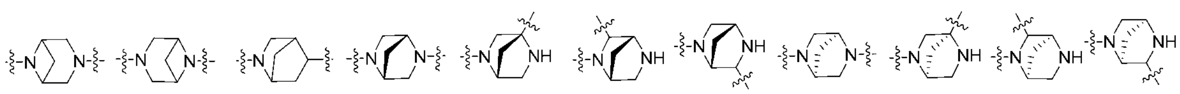

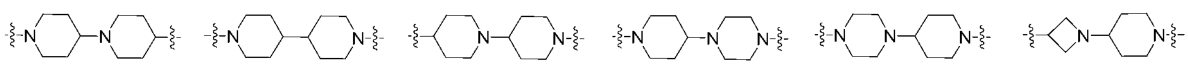

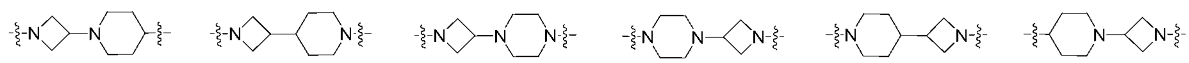

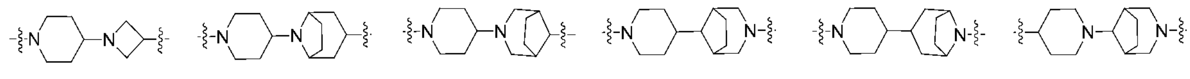

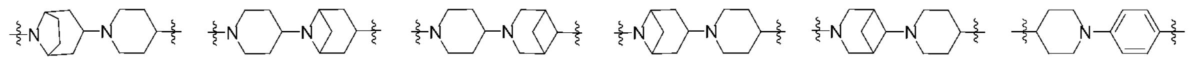

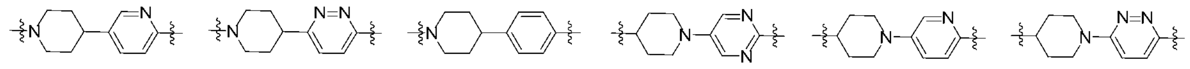

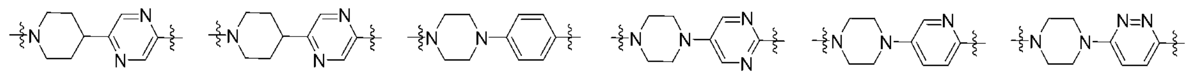

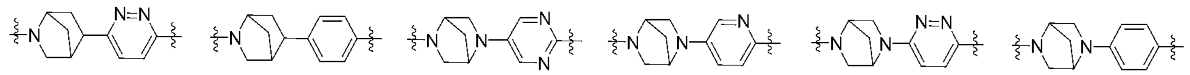

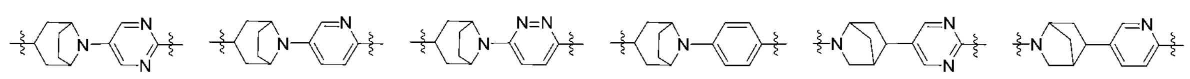

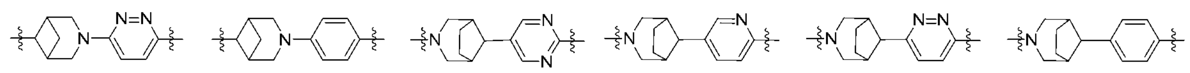

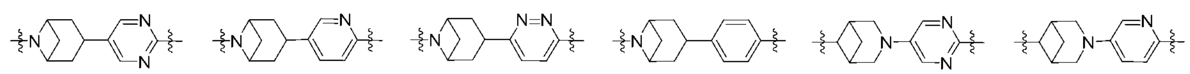

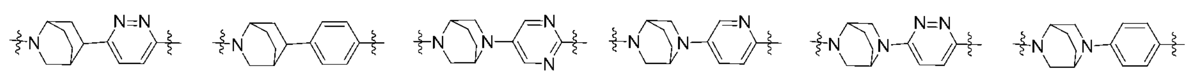

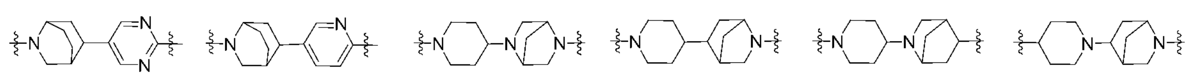

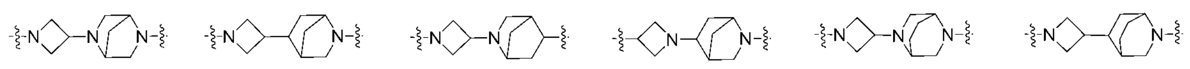

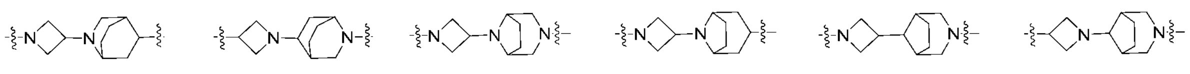

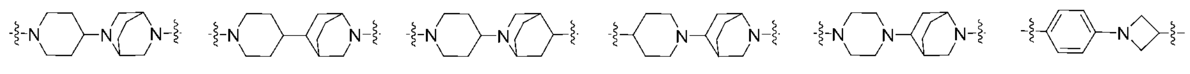

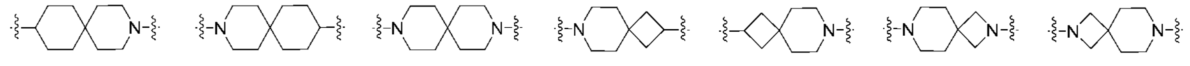

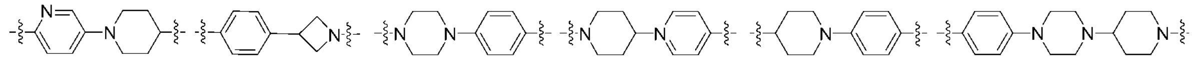

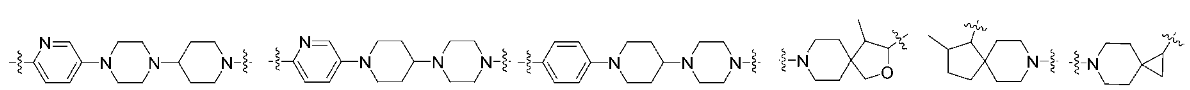

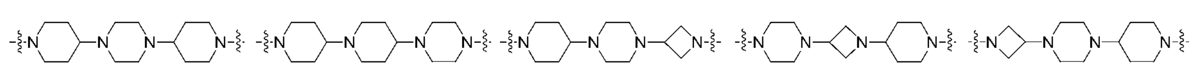

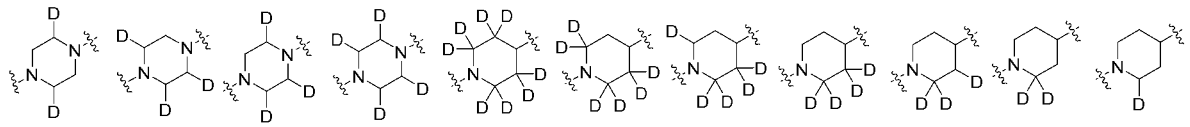

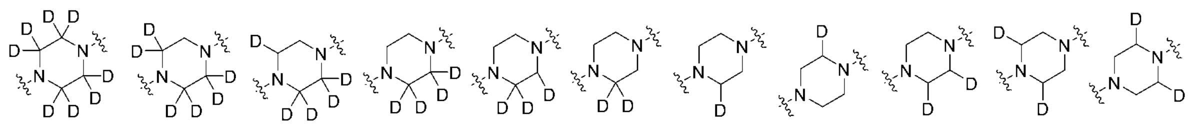

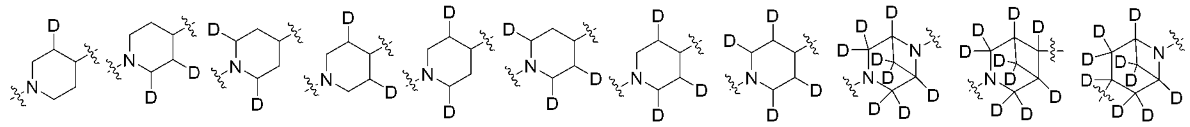

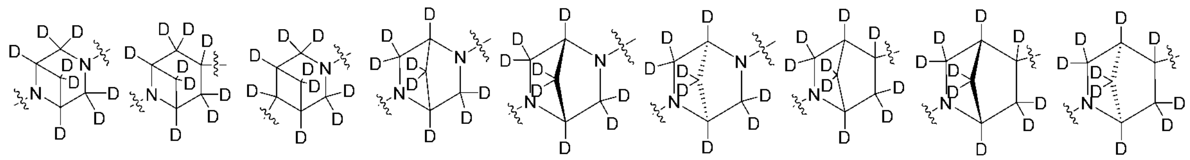

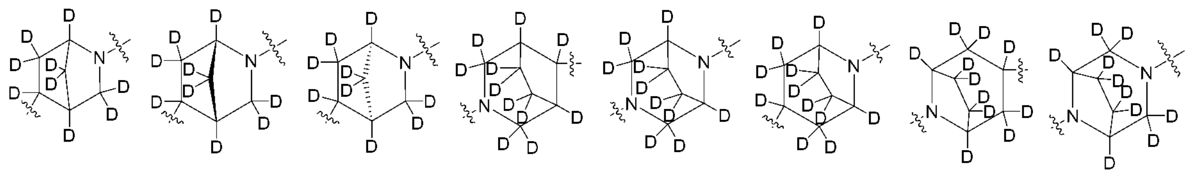

, or

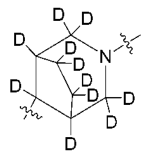

;

wherein the groups are optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl;
and/or

$R_{w1}$ represents a bond, or
$R_{w1}$ represents:

$-C_{1-15}$ alkylene-;

$-(C(R_{a8})(R_{a9}))_{r1}-(R_a(C(R_{a10})(R_{a11}))_{r2})_{s1}-*$;

$-(C(R_{a8})(R_{a9}))_{r1}-(R_a(C(R_{a10})(R_{a11}))_{r2})_{s1}-(R_a(C(R_{a12})(R_{a13}))_{r2})_{s2}-*$;

$-(C(R_{a8})(R_{a9}))_{r1}-(R_a(C(R_{a10})(R_{a11}))_{r2})_{s1}-(R_a(C(R_{a12})(R_{a13}))_{r3})_{s2}-(R_a(C(R_{a14})(R_{a15}))_{r4})_{s3}-*$;

$-(C(R_{a8})(R_{a9}))_{r1}-((C(R_{a10})(R_{a11})_{r2}R_a)_{s1}-*$;

$-(C(R_{a8})(R_{a9}))_{r1}-((C(R_{a10})(R_{a11}))_{r2}R_a)_{s1}-((C(R_{a12})(R_{a13}))_{r3}R_a)_{s2}-*$;

$-(C(R_{a8})(R_{a9}))_{r1}-((C(R_{a10})(R_{a11}))_{r2}R_a)_{s1}-((C(R_{a12})(R_{a13}))_{r3}R_a)_{s2}-((C(R_{a14})(R_{a15}))_{r4}R_a)_{s3}-*$;

$-(C(R_{a8})(R_{a9}))_{r1}-(R_b(C(R_{a10})(R_{a11}))_{r2})_{s1}-*$;

$-(C(R_{a8})(R_{a9}))_{r1}-(R_3(C(R_{a10})(R_{a11}))_{r2})_{s1}-(R_3(C(R_{a12})(R_{a13}))_{r3})_{s2}-*$;

$-(C(R_{a8})(R_{a9}))_{r1}-(R_b(C(R_{a10})(R_{a11}))_{r2})_{s1}-(R_3(C(R_{a12})(R_{a13}))_{r3})_{s2}-(R_b(C(R_{a14})(R_{a15}))_{r4})_{s3}-*$;

$-(C(R_{a8})(R_{a9}))_{r1}-((C(R_{a10})(R_{a11}))_{r2}R_b)_{s1}-*$;

$-(C(R_{a8})(R_{a9}))_{r1}-((C(R_{a10})(R_{a11}))_{r2}R_b)_{s1}-((C(Ra_{12})(Ra_{13}))_{r3}R_b)_{s2}-*$;

$-(C(R_{a8})(R_{a9}))_{r1}-((CR_{a10})(R_{a11}))_{r2}R_b)_{s1}-((C(R_{a12})(R_{a13}))_{r3}R_b)_{s2}-((C(R_{a14})(R_{a15}))_{r4}R_b)_{s3}-*$;

$-(C(R_{a8})(R_{a9}))_{r1}-(R_a-R_b-(C(R_{a10})(R_{a11}))_{r2})_{s1}-*$;

$-(C(R_{a8})(R_{a9}))_{r1}-(R_a(C(R_{a10})(R_{a11}))_{r2})_{s1}-(R_3(C(R_{a12})(R_{a13}))_{r3})_{s2}-*$;

$-(C(R_{a8})(R_{a9}))_{r1}-(R_b-R_a-(C(R_{a10})(R_{a11}))_{r2})_{s1}-*$;

$-(C(R_{a8})(R_{a9}))_{r1}-(R_b(C(R_{a10})(R_{a11}))_{r2})_{s1}-(R_a(C(R_{a12})(R_{a13}))_{r3})_{s2}-*$"

$-(C(R_{a8})(R_{a9}))_{r1}-((C(R_{a10})(R_{a11}))_{r2}-R_a-R_b)_{s1}-*$;

$-(C(R_{a8})(R_{a9}))_{r1}-((C(R_{a10})(R_{a11}))_{r2}R_a)_{s1}-((C(R_{a12})(R_{a13}))r_3R_b)_{s2}-*$;

$-(C(R_{a8})(R_{a9}))_{r1}-((C(R_{a10})(R_{a11}))_{r2}-R_3-R_a)_{s1}-*$; or

$-(C(R_{a8})(R_{a9}))_{r1}-((C(R_{a10})(R_{a11}))_{r2}R_3)_{s1}-((C(R_{a12})(R_{a13}))_{r3}R_a)_{s2}-*$;

wherein each $R_a$ is independently selected from the group consisting of O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, where each $R_{a7}$ independently represents H or $C_{1-6}$ alkyl; and each $R_b$ is independently selected from the group consisting of optionally substituted cycloalkylene (e.g., optionally substituted $C_{3-20}$ cycloalkylene), optionally substituted arylene (e.g., optionally substituted $C_{5-20}$ arylene), optionally substituted heterocyclylene (e.g., optionally substituted 4- to 20-membered heterocyclylene), optionally substituted heteroarylene (e.g., 5- to 20-membered heteroarylene), alkynylene (e.g., $C_{2-6}$ alkynylene), alkenylene (e.g., $C_{2-6}$ alkenylene), and any combination thereof, wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof;

$R_{a8}$, $R_{a9}$, $R_{a10}$, $R_{a11}$, $R_{a12}$, $R_{a13}$, $R_{a14}$, and $R_{a15}R_{a15}$ each independently represent **H,** deuterium, hydroxy, amino, mercapto, nitro, halogen, cyano, oxo, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; and

r1, r2, r3, r4, s1, s2, and s3 each independently represent an integer of 1, 2, 3, 4, 5, 6, 7, 8, 9, or 10;

symbol * indicates the point of attachment to R; and

wherein any one or more hydrogen atoms in the main carbon chain skeleton of the said $C_{1-15}$ alkylene are optionally replaced by a substituent selected from the group consisting of: deuterium, hydroxy, amino, mercapto, nitro, halogen, cyano, oxo, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.

**5.** The compound of Formula (I) or salts, enantiomers, stereoisomers, solvates, or polymorphs thereof as claimed in claim 1, wherein $R_{w1}$ represents a structure of the following Formulae:

$-(C(R_{a8})(R_{a9}))_{r1}-(O(C(R_{a10})(R_{a11}))_{r2})_{s1}-*$;

$-(C(R_{a8})(R_{a9}))_{r1}-(O(C(R_{a10})(R_{a11}))_{r2})_{s1}-(O(C(R_{a12})(R_{a13}))_{r3})_{s2}-*$;

$-(C(R_{a8})(R_{a9}))_{r1}-(O(C(R_{a10})(R_{a11}))_{r2})_{s1}-(O(C(R_{a12})(R_{a13}))_{r3})_{s2}-$ $(O(C(R_{a14})(R_{a15}))_{r4})_{s3}-*$;

$-(C(R_{a8})(R_{a9}))_{r1}-((C(R_{a10})(R_{a11}))_{r2}O)_{s1}-*$;

$-(C(R_{a8})(R_{a9}))_{r1}-((C(R_{a10})(R_{a11}))_{r2}O)_{s1}-((C(R_{a12})(R_{a13}))_{r3}O)_{s2}-*$;

$-(C(R_{a8})(R_{a9}))_{r1}-((C(R_{a10}))(R_{a11}))_{r2}O)_{s1}-((C(R_{a12})(R_{a13}))_{r3}O)_{s2}-$ $((C(R_{a14})(R_{a15}))_{r4}O)_{s3}-*$;

$-(C(R_{a8})(R_{a9}))_{r1}-(N(R_{a7})(C(R_{a10})(R_{a11}))_{r2})_{s1}-*$;

$-(C(R_{a8})(R_{a9}))_{r1}-(N(R_{a7})(C(R_{a10})(R_{a11}))_{r2})_{s1}-(N(R_{a7})(C(R_{a12})(R_{a13}))_{r3})_{s2}-*$;

$-(C(R_{a8})(R_{a9}))_{r1}-(N(R_{a7})(C(R_{a10})(R_{a11}))_{r2})_{s1}-(N(R_{a7})(C(R_{a12})(R_{a13}))_{r3})_{s2}-(N(R_{a7})(C(R_{a14})(R_{a15}))_{r4})_{s3}-*$;

$-(C(R_{a8})(R_{a9}))_{r1}-((C(R_{a10})(R_{a11}))_{r2}N(R_{a7}))_{s1}-*$;

$-(C(R_{a8})(R_{a9}))_{r1}-((C(R_{a10})(R_{a11}))_{r2}N(R_{a7}))_{s1}-((C(R_{a12})(R_{a13}))_{r3}N(R_{a7}))_{s2}-*$;

$-(C(R_{a8})(R_{a9}))_{r1}-((C(R_{a10})(R_{a11}))_{r2}N(R_{a7}))_{s1}-((C(R_{a12})(R_{a13}))_{r3}N(R_{a7}))_{s2}-((C(R_{a14})(R_{a15}))_{r4}N(R_{a7}))_{s3}-*$;

$-(C(R_{a8})(R_{a9}))_{r1}-(C(O)N(R_{a7})-(C(R_{a10})(R_{a11}))_{r2})_{s1}-*$;

$-(C(R_{a8})(R_{a9}))_{r1}-(C(O)N(R_{a7})-(C(R_{a10})(R_{a11}))_{r2})_{s1}-(C(O)N(R_{a7})-(C(R_{a12})(R_{a13}))_{r3})_{s2}-*$;

$-(C(R_{a8})(R_{a9}))_{r1}-(C(O)N(R_{a7})-(C(R_{a10})(R_{a11}))_{r2})_{s1}-(C(O)N(R_{a7})-(C(R_{a12})(R_{a13}))_{r3})_{s2}-(CO)N(R_{a7})-(C(R_{a14})(R_{a15}))_{r4})_{s3}-*$;

$-(C(R_{a8})(R_{a9}))_{r1}-((C(R_{a10})(R_{a11}))_{r2}-C(O)N(R_{a7}))_{s1}-*$;

$-(C(R_{a8})(R_{a9}))_{r1}-((C(R_{a10})(R_{a11}))_{r2}-C(O)N(R_{a7}))_{s1}-((C(R_{a12})(R_{a13}))_{r3}-C(O)N(R_{a7}))s_2-*$;

$-(C(R_{a8})(R_{a9}))_{r1}-((C(R_{a10})(R_{a11}))_{r2}-C(O)N(R_{a7}))_{s1}-((C(R_{a12})(R_{a13}))_{r3}-C(O)N(R_{a7}))_{s2}-((C(R_{a14})(R_{a15}))_{r4}-C(O)N(R_{a7}))_{s3}-*$;

$-(C(R_{a8})(R_{a9}))_{r1}-(C(O)N(R_{a7})-(C(R_{a10})(R_{a11}))_{r2})_{s1}-(O-(C(R_{a12})(R_{a13}))_{r3})_{s2}-*$;

$-(C(R_{a8})(R_{a9}))_{r1}-C(O)N(R_{a7})-(C(R_{a10})(R_{a11}))_{r2}-(O(C(R_{a12})(R_{a13}))_{r3})_{s1}-*$;

$-(C(R_{a8})(R_{a9}))_{r1}-((C(R_{a10})(R_{a11}))_{r2}-C(O)N(R_{a7}))_{s1}-( (C(R_{a12})(R_{a13}))_{r3}O)_{s2}-*$;

$-(C(R_{a8})(R_{a9}))_{r1}-(C(R_{a10})(R_{a11}))_{r2}-C(O)N(R_{a7})-((C(R_{a12})(R_{a13}))_{r3}O)_{s1}-*$;

$-(C(R_{a8})(R_{a9}))_{r1}-(N(R_{a7})C(O)-(C(R_{a10})(R_{a11}))_{r2})_{s1}-*$;

$-(C(R_{a8})(R_{a9}))_{r1}-(N(R_{a7})C(O)-(C(R_{a10})(R_{a11}))_{r2})_{s1}-(O-(C(R_{a12})(R_{a13})))_{r4})_{s2}-*$;

$-(C(R_{a8})(R_{a9}))_{r1}-(N(R_{a7})C(O)-(C(R_{a10})(R_{a11}))_{r2})_{s1}-(O-(C(R_{a12})(R_{a13}))_{r3})_{s2}-(O-(C(R_{a14})(R_{a15}))_{r4})_{s3}-*$;

$-(C(R_{a8})(R_{a9}))_{r1}-N(R_{a7})C(O)-(C(R_{a10})(R_{a11}))_{r2}-(O(C(R_{a12})(R_{a13}))_{r3})_{s1}-*$;

$-(C(R_{a8})(R_{a9}))_{r1}-N(R_{a7})C(O)N(R_{a7})-(C(R_{a10})(R_{a11}))_{r2}-*$;

$-(C(R_{a8})(R_{a9}))_{r1}-((C(R_{a10})(R_{a11}))_{r2}-N(R_{a7})C(O))_{s1}-*$;

$-(C(R_{a8})(R_{a9}))_{r1}-((C(R_{a10})(R_{a11}))_{r2}-N(R_{a7})C(O))_{s1}-((C(R_{a12})(R_{a13})))_{r3}-O)_{s2}-*$;

$-(C(R_{a8})(R_{a9}))_{r1}-((C(R_{a10})(R_{a11}))_{r2}-N(R_{a7})C(O))_{s1}-((C(R_{a12})(R_{a13}))_{r3}O)_{s2}-((C(R_{a14})(R_{a15}))_{r4}O)_{s3}-*$;

$-(C(R_{a8})(R_{a9}))_{r1}-(C(R_{a10})(R_{a11}))_{r2}-N(R_{a7})C(O)-((C(R_{a12})(R_{a13}))_{r3}O)_{s1}-*$;

$-(C(R_{a8})(R_{a9}))_{r1}$-(arylene-$(C(R_{a10})(R_{a11}))_{r2})_{s1}-*$;

$-(C(R_{a8})(R_{a9}))_{r1}$-(arylene-$(C(R_{a10})(R_{a11}))_{r2})_{s1}$-arylene-$(C(R_{a12})(R_{a13}))_{r3}-*$;

$-(C(R_{a8})(R_{a9}))_{r1}-((C(R_{a10})(R_{a11}))_{r2}$-arylene$)_{s1}-*$;

$-(C(R_{a8})(R_{a9}))_{r1}-((C(R_{a10})(R_{a11}))_{r2}$-arylene$)_{s1}-(C(R_{a12})(R_{a13}))_{r3}$-arylene-*;

$-(C(R_{a8})(R_{a9}))_{r1}$-(heterocyclylene-$(C(R_{a10})(R_{a11}))_{r2})_{s1}-*$;

$-(C(R_{a8})(R_{a9}))_{r1}$-(heterocyclylene-$(C(R_{a10})(R_{a11}))_{r2})_{s1}$-(heterocyclylene-$(C(R_{a12})(R_{a13}))_{r3})_{s2}-*$;

$-(C(R_{a8})(R_{a9}))_{r1}-((C(R_{a10})(R_{a11}))_{r2}$-heterocyclylene$)_{s1}-*$;

$-(C(R_{a8})(R_{a9}))_{r1}-((C(R_{a10})(R_{a11}))_{r2}$-heterocyclylene$)_{s1}-((C(R_{a12})(R_{a13}))_{r3}$-heterocyclylene$)_{s2}-*$;

$-(C(R_{a8})(R_{a9}))_{r1}$-(heteroarylene-$(C(R_{a10})(R_{a11}))_{r2})_{s1}-*$;

$-(C(R_{a8})(R_{a9}))_{r1}$-(heteroarylene-$(C(R_{a10})(R_{a11}))_{r2})_{s1}$-(heteroarylene-$(C(R_{a12})(R_{a13}))_{r3})_{s2}-*$;

$-(C(R_{a8})(R_{a9}))_{r1}-((C(R_{a10})(R_{a11}))_{r2}$-heteroarylene$)_{s1}-*$;

$-(C(R_{a8})(R_{a9}))_{r1}-((C(R_{a10})(R_{a11}))_{r2}$-heteroarylene$)_{s1}-((C(R_{a12})(R_{a13}))_{r3}$-heteroarylene$)_{s2}-*$;

$-(C(R_{a8})(R_{a9}))_{r1}$-(cycloalkylene-$(C(R_{a10})(R_{a11}))_{r2})_{s1}-*$;

$-(C(R_{a8})(R_{a9}))_{r1}$-(cycloalkylene-$(C(R_{a10})(R_{a11}))_{r2})_{s1}$-(cycloalkylene-$(C(R_{a12})(R_{a13}))_{r3})_{s2}-*$;

$-(C(R_{a8})(R_{a9}))_{r1}-((C(R_{a10})(R_{a11}))_{r2}$- cycloalkylene$)_{s1}-*$; or

$-(C(R_{a8})(R_{a9}))_{r1}-((C(R_{a10})(R_{a11}))_{r2}$- cycloalkylene$)_{s1}-((C(R_{a12})(R_{a13}))_{r3}$- cycloalkylene$)_{s2}-*$;

wherein each $R_{a7}$ independently represents H or $C_{1-6}$ alkyl;
the cycloalkylene (e.g., $C_{3-20}$ cycloalkylene), arylene (e.g., $C_{5-20}$ arylene), heterocyclylene (e.g., 4- to 20-membered heterocyclylene), and heteroarylene (e.g., 5- to 20-membered heteroarylene) are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof;
$R_{a8}$, $R_{a9}$, $R_{a10}$, $R_{a11}$, $R_{a12}$, $R_{a13}$, $R_{a14}$, and $R_{a15}$ each independently represent H, deuterium, hydroxy, amino, mercapto, nitro, halogen, cyano, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; and
r1, r2, r3, r4, s1, s2, and s3 each independently represent an integer of 1, 2, 3, 4, 5, 6, 7, 8, 9, or 10; and symbol * indicates the point of attachment to R.

**6.** The compound of Formula (I) or salts, enantiomers, stereoisomers, solvates, or polymorphs thereof as claimed in claim 1, wherein $R_{w1}$ represents:

$-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$; $-O-CH_2-$, $-O-(CH_2)_2-$, $-O-(CH_2)_3-$, $-O-(CH_2)_4-$, $-O-(CH_2)_5-$, $-O-(CH_2)_6-$, $-O-(CH_2)_7-$, $-O-(CH_2)_8-$, $-O-(CH_2)_9-$, $-O-(CH_2)_{10}-$, $-(CH_2)_1-O-$, $-(CH_2)_2-O-$, $-(CH_2)_3-O-$, $-(CH_2)_4-O-$, $-(CH_2)_5-O-$, $-(CH_2)_6-O-$, $-(CH_2)_7-O-$, $-(CH_2)_8-O-$, $-(CH_2)_9-O-$, $-(CH_2)_{10}-O-$, $-CH_2-O-CH_2-$, $-CH_2-O-(CH_2)_2-$, $-(CH_2)_1-O-(CH_2)_3-$, $-(CH_2)_1-O-(CH_2)_4-$, $-(CH_2)_1-O-(CH_2)_5-$, $-(CH_2)_1-O-(CH_2)_6-$, $-(CH_2)_1-O-(CH_2)_7-$, $-(CH_2)_1-O-(CH_2)_8-$, $-(CH_2)_1-O-(CH_2)_9-$, $-(CH_2)_1-O-(CH_2)_{10}-$, $-(CH_2)_2-O-(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, $-(CH_2)_2-O-(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$, $-(CH_2)_3-O-(CH_2)_1-$, $-(CH_2)_3-O-(CH_2)_2-$, $-(CH_2)_3-O-(CH_2)_3-$, $-(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_1-$, $-(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, $-(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, $-(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, $-(CH_2)_7-O-(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$, $-CH(CH_3)-O-(CH_2)_2-$, $-CH(CH_3)-O-(CH_2)_3-$, $-CH(CH_3)-O-(CH_2)_4-$, $-CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-CH(CH_3)-O-(CH_2)_9-$, $-CH(CH_3)-O-(CH_2)_{10}-$, $-(O(CH_2)_2)_2-$, $-(O(CH_2)_2)_3-$, $-(O(CH_2)_2)_4-$, $-(O(CH_2)_2)_5-$, $-(O(CH_2)_2)_6-$, $-(O(CH_2)_2)_7-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_7-$, $-CH_2-(O(CH_2)_2)_1-OCH_2-$, $-CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, $-(CH_2)_2-(O(CH_2)_2)_5-$, $-(CH_2)_2-(O(CH_2)_2)_6-$, $-(CH_2)_3-(O(CH_2)_2)_2-$, $-(CH_2)_3-(O(CH_2)_2)_3-$, $-(CH_2)_3-(O(CH_2)_2)_4-$, $-(CH_2)_3-(O(CH_2)_2)_5-$, $-(CH_2)_4-(O(CH_2)_2)_2-$, $-(CH_2)_4-(O(CH_2)_2)_3-$, $-(CH_2)_4-(O(CH_2)_2)_4-$, $-(CH_2)_4-(O(CH_2)_2)_5-$, $-CH_2-(O(CH_2)_3)_2-$, $-CH_2-(O(CH_2)_3)_3-$, $-CH_2-(O(CH_2)_3)_4-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_3-(O(CH_2)_3)_2-$, $-(CH_2)_3-(O(CH_2)_3)_3-$, $-(CH_2)_3-(O(CH_2)_3)_4-$, $-CH_2-O-(CH_2)_2-O-(CH_2)_3-$, $-CH_2-(O(CH_2)_2)_2-(O(CH_2)_3)_2-$, $(CH_2)_2-O-(CH_2)_2-O-(CH_2)_3-$, $-(CH_2)_2-(O(CH_2)_2)_2-(O(CH_2)_3)_2-$, $-(CH_2)_3-O-(CH_2)_2-O-(CH_2)_3-$, $-(CH_2)_3-(O(CH_2)_2)_2-(O(CH_2)_3)_2-$, $-CH_2-O-(CH_2)_3-O-(CH_2)_2-$, $-CH_2-(O(CH_2)_3)_2-(O(CH_2)_2)_2-$, $-(CH_2)_2-O-(CH_2)_3-O-(CH_2)_2-$, $-(CH_2)_2-(O(CH_2)_3)_2-(O(CH_2)_2)_2-$, $-(CH_2)_3-O-(CH_2)_3-O-(CH_2)_2-$, $-(CH_2)_3-(O(CH_2)_3)_2-(O(CH_2)_2)_2-$, $-CH_2-O-(CH_2)_2-O-CH_2-$, $-(CH_2)_2-O-(CH_2)_2-O-CH_2-$, $-(CH_2)_2-(O(CH_2)_2)_2-O-(CH_2)_3-$, $-(CH_2)_2-(O(CH_2)_2)_3-O-(CH_2)_3-$, $-(CH_2)_2-(O(CH_2)_2)_4-O-(CH_2)_3-$, $-(CH_2)_5-(O(CH_2)_2)_2-O-(CH_2)_5-$, $-(CH_2)_5-(O(CH_2)_2)_2-O-(CH_2)_6-$, $-(CH_2)_1-N(R_{a7})-(CH_2)_1-$, $-(CH_2)_1-N(R_{a7})-(CH_2)_2-$, $-(CH_2)_1-N(R_{a7})-(CH_2)_3-$, $-(CH_2)_1-N(R_{a7})-(CH_2)_4-$, $-(CH_2)_1-N(R_{a7})-(CH_2)_5-$, $-(CH_2)_1-N(R_{a7})-(CH_2)_6-$, $-(CH_2)_1-N(R_{a7})-(CH_2)_7-$, $-(CH_2)_1-N(R_{a7})-(CH_2)_8-$, $-(CH_2)_1-N(R_{a7})-(CH_2)_9-$, $-(CH_2)_1-N(R_{a7})-(CH_2)_{10}-$, $-N(R_{a7})-(CH_2)_1-$, $-N(R_{a7})-(CH_2)_2-$, $-N(R_{a7})-(CH_2)_3-$, $-N(R_{a7})-(CH_2)_4-$, $-N(R_{a7})-(CH_2)_5-$, $-N(R_{a7})-(CH_2)_6-$, $-N(R_{a7})-(CH_2)_7-$, $-N(R_{a7})-(CH_2)_8-$, $-N(R_{a7})-(CH_2)_9-$, $-N(R_{a7})-(CH_2)_{10}-$, $-(CH_2)_2-N(R_{a7})-(CH_2)_1-$, $-(CH_2)_2-N(R_{a7})-(CH_2)_2-$, $-(CH_2)_2-N(R_{a7})-(CH_2)_3-$, $-(CH_2)_2-N(R_{a7})-(CH_2)_4-$, $-(CH_2)_2-N(R_{a7})-(CH_2)_5-$, $-(CH_2)_2-N(R_{a7})-(CH_2)_6-$, $-(CH_2)_2-N(R_{a7})-(CH_2)_7-$, $-(CH_2)_2-N(R_{a7})-(CH_2)_8-$, $-(CH_2)_2-N(R_{a7})-(CH_2)_9-$, $-(CH_2)_2-N(R_{a7})-(CH_2)_{10}-$, $-(CH_2)_3-N(R_{a7})-(CH_2)_1-$, $-(CH_2)_3-N(R_{a7})-(CH_2)_2-$, $-(CH_2)_3-N(R_{a7})-(CH_2)_3-$, $-(CH_2)_4-N(R_{a7})-(CH_2)_1-$, $-(CH_2)_4-N(R_{a7})-(CH_2)_2-$, $-(CH_2)_4-N(R_{a7})-(CH_2)_3-$, $-(CH_2)_4-N(R_{a7})-(CH_2)_4-$, $-(CH_2)_5-N(R_{a7})-(CH_2)_1-$, $-(CH_2)_5-N(R_{a7})-(CH_2)_2-$, $-(CH_2)_5-N(R_{a7})-(CH_2)_3-$, $-(CH_2)_5-N(R_{a7})-(CH_2)_4-$, $-(CH_2)_5-N(R_{a7})-(CH_2)_5-$, $-(CH_2)_6-N(R_{a7})-(CH_2)_1-$, $-(CH_2)_6-N(R_{a7})-(CH_2)_2-$, $-(CH_2)_6-N(R_{a7})-(CH_2)_3-$, $-(CH_2)_7-N(R_{a7})-(CH_2)_1-$, $-(CH_2)_7-N(R_{a7})-(CH_2)_2-$, $-(CH_2)_7-N(R_{a7})-(CH_2)_3-$, $-(CH_2)_8-N(R_{a7})-(CH_2)_1-$, $-(CH_2)_8-N(R_{a7})-(CH_2)_2-$, $-(CH_2)_8-N(R_{a7})-(CH_2)_3-$, $-CH(CH_3)-N(R_{a7})-(CH_2)_1-$, $-CH(CH_3)-N(R_{a7})-(CH_2)_2-$, $-CH(CH_3)-N(R_{a7})-(CH_2)_3-$, $-CH(CH_3)-N(R_{a7})-(CH_2)_4-$, $-CH(CH_3)-N(R_{a7})-(CH_2)_5-$, $-CH(CH_3)-N(R_{a7})-(CH_2)_6-$, $-CH(CH_3)-N(R_{a7})-(CH_2)_7-$, $-CH(CH_3)-N(R_{a7})-(CH_2)_8-$, $-CH(CH_3)-N(R_{a7})-(CH_2)_9-$, $-CH(CH_3)-N(R_{a7})-(CH_2)_{10}-$, $-(CH_2)_1-N(R_{a7})-$, $-(CH_2)_2-N(R_{a7})-$, $-(CH_2)_3-N(R_{a7})-$, $-(CH_2)_4-N(R_{a7})-$, $-(CH_2)_5-N(R_{a7})-$, $-(CH_2)_6-N(R_{a7})-$, $-(CH_2)_7-N(R_{a7})-$, $-(CH_2)_8-N(R_{a7})-$, $-(CH_2)_9-N(R_{a7})-$, $-(CH_2)_{10}-N(R_{a7})-$, $-C(O)NHCH_2-$, $-C(O)NH(CH_2)_2-$, $-C(O)NH(CH_2)_3-$, $-C(O)NH(CH_2)_4-$, $-C(O)NH(CH_2)_5-$, $-CH_2C(O)NHCH_2-$, $-(CH_2)_2C(O)NH(CH_2)_2-$, $-(CH_2)_2C(O)NH(CH_2)_3-$, $-(CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$, $-(CH_2)_4C(O)NH(CH_2)_4-$, $-(CH_2)_5C(O)NH(CH_2)_5-$, $-(CH_2)_6C(O)NH(CH_2)_7-$, $-(CH_2)_6C(O)NH(CH_2)_6-$, $-(CH_2)_7C(O)NH(CH_2)_7-$, $-(CH_2)_2C(O)NH(CH_2)_2-O-(CH_2)_2-$, $-NHC(O)CH_2-$, $-NHC(O)(CH_2)_2-$, $-NHC(O)(CH_2)_3-$, $-NHC(O)(CH_2)_4-$, $-NHC(O)(CH_2)_5-$, $-CH_2NHC(O)CH_2-$, $-(CH_2)_2NHC(O)(CH_2)_2-$, $-(CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$, $-(CH_2)_3NHC(O)(CH_2)_4-$, $-(CH_2)_4NHC(O)(CH_2)_4-$, $-(CH_2)_5NHC(O)(CH_2)_5-$, $-(CH_2)_6NHC(O)(CH_2)_7-$, $-(CH_2)_6NHC(O)(CH_2)_6-$, $-(CH_2)_7NHC(O)(CH_2)_7-$, $-(CH_2)_4NHC(O)(CH_2)_8-$, $-(CH_2)_2NHC(O)(CH_2)_2-O-(CH_2)_2-$, $-(CH_2)_4NHC(O)CH_2-$, -phenylene-$CH_2$-, -phenylene-$(CH_2)_2$-, -phenylene-$(CH_2)_3$-, -phenylene-$(CH_2)_4$-, -phenylene-$(CH_2)_5$-, -phenylene-$(CH_2)_6$-, -phenylene-$(CH_2)_7$-, -phenylene-$(CH_2)_8$-, -$CH_2$-phenylene-$CH_2$-, -$CH_2$-phenylene-$(CH_2)_2$-, -$CH_2$-

phenylene-$(CH_2)_3$-, -$CH_2$-phenylene-$(CH_2)_4$-, -$CH_2$-phenylene-$(CH_2)_5$-, -$CH_2$-phenylene-$(CH_2)_6$-, -$CH_2$-phenylene-$(CH_2)_7$-, -$CH_2$-phenylene-$(CH_2)_8$-, -$(CH_2)_2$-phenylene-$(CH_2)_1$-, -$(CH_2)_2$-phenylene-$(CH_2)_2$-, -$(CH_2)_2$-phenylene-$(CH_2)_3$-, -$(CH_2)_2$-phenylene-$(CH_2)_4$-, - $(CH_2)_2$-phenylene-$(CH_2)_5$-, -$(CH_2)_2$-phenylene-$(CH_2)_6$-, -$(CH_2)_2$-phenylene-$(CH_2)_7$-, -$(CH_2)_2$-phenylene-$(CH_2)_8$-, -$(CH_2)_3$-phenylene-$CH_2$-, -$(CH_2)_3$-phenylene-$(CH_2)_2$-, -$(CH_2)_3$-phenylene-$(CH_2)_3$-, -$(CH_2)_3$-phenylene-$(CH_2)_4$-, -$(CH_2)_3$-phenylene-$(CH_2)_5$-, -$(CH_2)_3$-phenylene-$(CH_2)_6$-, -$(CH_2)_3$-phenylene-$(CH_2)_7$-, - $(CH_2)_3$-phenylene-$(CH_2)_8$-, -$(CH_2)_4$-phenylene-$CH_2$-, -$(CH_2)_4$-phenylene-$(CH_2)_2$-, -$(CH_2)_4$-phenylene-$(CH_2)_3$-, -$(CH_2)_4$-phenylene-$(CH_2)_4$-, -$(CH_2)_4$-phenylene-$(CH_2)_5$-, -$(CH_2)_4$-phenylene-$(CH_2)_6$-, -$(CH_2)_4$-phenylene-$(CH_2)_7$-, -$(CH_2)_4$-phenylene-$(CH_2)_8$-, -$(CH_2)_5$-phenylene-$(CH_2)_1$-, -$(CH_2)_5$-phenylene-$(CH_2)_2$-, - $(CH_2)_5$-phenylene-$(CH_2)_3$-, -$(CH_2)_5$-phenylene-$(CH_2)_4$-, -$(CH_2)_5$-phenylene-$(CH_2)_5$-, -$(CH_2)_5$-phenylene-$(CH_2)_6$-, -$(CH_2)_5$-phenylene-$(CH_2)_7$-, -$(CH_2)_5$-phenylene-$(CH_2)_8$-, -$(CH_2)_6$-phenylene-$(CH_2)_1$-, -$(CH_2)_6$-phenylene-$(CH_2)_2$-, -$(CH_2)_6$-phenylene-$(CH_2)_3$-, -$(CH_2)_6$-phenylene-$(CH_2)_4$-, -$(CH_2)_6$-phenylene-$(CH_2)_5$-, - $(CH_2)_6$-phenylene-$(CH_2)_6$-, -$(CH_2)_6$-phenylene-$(CH_2)_7$-, -$(CH_2)_6$-phenylene-$(CH_2)_8$-, -$(CH_2)_7$-phenylene-$(CH_2)_1$-, -$(CH_2)_7$-phenylene-$(CH_2)_2$-, -$(CH_2)_7$-phenylene-$(CH_2)_3$-, -$(CH_2)_7$-phenylene-$(CH_2)_4$-, -$(CH_2)_7$-phenylene-$(CH_2)_8$-, -$(CH_2)_8$-phenylene-$CH_2$-, -$(CH_2)_8$-phenylene-$(CH_2)_2$-, -$(CH_2)_8$-phenylene-$(CH_2)_3$-, -$(CH_2)_8$-phenylene-$(CH_2)_4$-, -$(CH_2)_8$-phenylene-$(CH_2)_5$-, -$(CH_2)_8$-phenylene-$(CH_2)_6$-, -$(CH_2)_8$-phenylene-$(CH_2)_7$-, -$N(R_{a7})$-$CH_2$-phenylene-$CH_2$-, -$N(R_{a7})$-$CH_2$-phenylene-$(CH_2)_2$-, -$N(R_{a7})$-$CH_2$-phenylene-$(CH_2)_3$-, -$N(R_{a7})$-$CH_2$-phenylene-$(CH_2)_4$-,-$N(R_{a7})$-$CH_2$-phenylene-$(CH_2)_5$-,-$N(R_{a7})$-$CH_2$-phenylene-$(CH_2)_6$-,-$N(R_{a7})$-$CH_2$-phenylene-$(CH_2)_7$-, -$N(R_{a7})$-$CH_2$-phenylene-$(CH_2)_8$-, -$CH_2$-$N(R_{a7})$-$CH_2$-phenylene-$CH_2$-, -$CH_2$-$N(R_{a7})$-$CH_2$-phenylene-$(CH_2)_2$-, -$CH_2$-$N(R_{a7})$-$CH_2$-phenylene-$(CH_2)_3$-, -$CH_2$-$N(R_{a7})$-$CH_2$-phenylene-$(CH_2)_4$-, -$CH_2$-$N(R_{a7})$-$CH_2$-phenylene-$(CH_2)_5$-, -$CH_2$-$N(R_{a7})$-$CH_2$-phenylene-$(CH_2)_6$-, -$CH_2$-$N(R_{a7})$-$CH_2$-phenylene-$(CH_2)_7$-, -$CH_2$-$N(R_{a7})$-$CH_2$-phenylene-$(CH_2)_8$-, -$CH_2$-$N(R_{a7})$-$(CH_2)_2$-phenylene-$CH_2$-, -$CH_2$-$N(R_{a7})$-$(CH_2)_2$-phenylene-$(CH_2)_2$-, -$CH_2$-$N(R_{a7})$-$(CH_2)_2$-phenylene-$(CH_2)_3$-, -$CH_2$-$N(R_{a7})$-$(CH_2)_2$-phenylene-$(CH_2)_4$-, -$CH_2$-$N(R_{a7})$-$(CH_2)_2$-phenylene-$(CH_2)_5$-, -$CH_2$-$N(R_{a7})$-$(CH_2)_2$-phenylene-$(CH_2)_6$-, -$CH_2$-$N(R_{a7})$-$(CH_2)_2$-phenylene-$(CH_2)_7$-, -$CH_2$-$N(R_{a7})$-$(CH_2)_2$-phenylene-$(CH_2)_8$-, -$(CH_2)_2$-$N(R_{a7})$-$CH_2$-phenylene-$CH_2$-, -$(CH_2)_2$-$N(R_{a7})$-$CH_2$-phenylene-$(CH_2)_2$-, -$(CH_2)_2$-$N(R_{a7})$-$CH_2$-phenylene-$(CH_2)_3$-, -$(CH_2)_2$-$N(R_{a7})$-$CH_2$-phenylene-$(CH_2)_4$-, -$(CH_2)_2$-$N(R_{a7})$-$CH_2$-phenylene-$(CH_2)_5$-, -$(CH_2)_2$-$N(R_{a7})$-$CH_2$-phenylene-$(CH_2)_6$-, -$(CH_2)_2$-$N(R_{a7})$-$CH_2$-phenylene-$(CH_2)_7$-, -$(CH_2)_2$-$N(R_{a7})$-$CH_2$-phenylene-$(CH_2)_8$-, -$(CH_2)_3$-$N(R_{a7})$-$CH_2$-phenylene-$CH_2$-, -$(CH_2)_3$-$N(R_{a7})$-$CH_2$-phenylene-$(CH_2)_2$-, -$(CH_2)_3$-$N(R_{a7})$-$CH_2$-phenylene-$(CH_2)_3$-, -$(CH_2)_3$-$N(R_{a7})$-$CH_2$-phenylene-$(CH_2)_8$-, -$(CH_2)_4$-$N(R_{a7})$-$CH_2$-phenylene-$CH_2$-, -$(CH_2)_4$-$N(R_{a7})$-$CH_2$-phenylene-$(CH_2)_2$-, -$(CH_2)_4$-$N(R_{a7})$-$CH_2$-phenylene-$(CH_2)_3$-, -$(CH_2)_4$-$N(R_{a7})$-$CH_2$-phenylene-$(CH_2)_8$-, -$(CH_2)_5$-$N(R_{a7})$-$CH_2$-phenylene-$(CH_2)_3$-, -$(CH_2)_5$-$N(R_{a7})$-$CH_2$-phenylene-$(CH_2)_8$-, -$(CH_2)_6$-$N(R_{a7})$-$CH_2$-phenylene-$(CH_2)_3$-, -$(CH_2)_6$-$N(R_{a7})$-$CH_2$-phenylene-$(CH_2)_8$-, -$(CH_2)_7$-$N(R_{a7})$-$CH_2$-phenylene-$(CH_2)_3$-, -$(CH_2)_8$-$N(R_{a7})$-$CH_2$-phenylene-$CH_2$-, -$(CH_2)_8$-$N(R_{a7})$-$CH_2$-phenylene-$(CH_2)_2$-, -$(CH_2)_8$-$N(R_{a7})$-$CH_2$-phenylene-$(CH_2)_3$-, -$(CH_2)_8$-$N(R_{a7})$-$CH_2$-phenylene-$(CH_2)_4$-, -$(CH_2)_8$-$N(R_{a7})$-$CH_2$-phenylene-$(CH_2)_5$-, -$(CH_2)_8$-$N(R_{a7})$-$CH_2$-phenylene-$(CH_2)_6$-, -piperidinylene-$CH_2$-, -piperidinylene-$(CH_2)_2$-, -piperidinylene-$(CH_2)_3$-, -piperidinylene-$(CH_2)_4$-, -piperidinylene-$(CH_2)_5$-, -piperidinylene-$(CH_2)_6$-,-piperidinylene-$(CH_2)_7$-, -piperidinylene-$(CH_2)_8$-, -$CH_2$-piperidinylene-$CH_2$-, -$CH_2$-piperidinylene-$(CH_2)_2$-, - $CH_2$-piperidinylene-$(CH_2)_3$-, -$CH_2$-piperidinylene-$(CH_2)_4$-, -$CH_2$-piperidinylene-$(CH_2)_5$-, -$CH_2$-piperidinylene-$(CH_2)_6$-, -$CH_2$-piperidinylene-$(CH_2)_7$-, -$CH_2$-piperidinylene-$(CH_2)_8$-, -$(CH_2)_2$-piperidinylene-$(CH_2)_1$-, -$(CH_2)_2$-piperidinylene-$(CH_2)_2$-, -$(CH_2)_2$-piperidinylene-$(CH_2)_3$-, -$(CH_2)_2$-piperidinylene-$(CH_2)_4$-, -$(CH_2)_2$-piperidinylene-$(CH_2)_5$-, -$(CH_2)_2$-piperidinylene-$(CH_2)_6$-, -$(CH_2)_2$-piperidinylene-$(CH_2)_7$-, -$(CH_2)_2$-piperidinylene-$(CH_2)_8$-, -$(CH_2)_3$-piperidinylene-$CH_2$-, -$(CH_2)_3$-piperidinylene-$(CH_2)_2$-, -$(CH_2)_3$-piperidinylene-$(CH_2)_3$-, -$(CH_2)_3$-piperidinylene-$(CH_2)_4$-, -$(CH_2)_3$-piperidinylene-$(CH_2)_5$-, -$(CH_2)_3$-piperidinylene-$(CH_2)_6$-, -$(CH_2)_3$-piperidinylene-$(CH_2)_7$-, -$(CH_2)_3$-piperidinylene-$(CH_2)_8$-, -$(CH_2)_4$-piperidinylene-$CH_2$-, -$(CH_2)_4$-piperidinylene-$(CH_2)_2$-, -$(CH_2)_4$-piperidinylene-$(CH_2)_3$-, -$(CH_2)_4$-piperidinylene-$(CH_2)_4$-, -$(CH_2)_4$-piperidinylene-$(CH_2)_5$-, -$(CH_2)_4$-piperidinylene-$(CH_2)_6$-, -$(CH_2)_4$-piperidinylene-$(CH_2)_7$-, -$(CH_2)_4$-piperidinylene-$(CH_2)_8$-, -$(CH_2)_5$-piperidinylene-$(CH_2)_1$-, -$(CH_2)_5$-piperidinylene-$(CH_2)_2$-, -$(CH_2)_5$-piperidinylene-$(CH_2)_3$-, -$(CH_2)_5$-piperidinylene-$(CH_2)_4$-, -$(CH_2)_5$-piperidinylene-$(CH_2)_5$-, -$(CH_2)_5$-piperidinylene-$(CH_2)_6$-, -$(CH_2)_5$-piperidinylene-$(CH_2)_7$-, -$(CH_2)_5$-piperidinylene-$(CH_2)_8$-, -$(CH_2)_6$-piperidinylene-$(CH_2)_1$-, -$(CH_2)_6$-piperidinylene-$(CH_2)_2$-, -$(CH_2)_6$-piperidinylene-$(CH_2)_3$-, -$(CH_2)_6$-piperidinylene-$(CH_2)_4$-, -$(CH_2)_6$-piperidinylene-$(CH_2)_5$-, -$(CH_2)_6$-piperidinylene-$(CH_2)_6$-, -$(CH_2)_6$-piperidinylene-$(CH_2)_7$-, -$(CH_2)_6$-piperidinylene-$(CH_2)_8$-, -$(CH_2)_7$-piperidinylene-$(CH_2)_1$-, -$(CH_2)_7$-piperidinylene-$(CH_2)_2$-, -$(CH_2)_7$-piperidinylene-$(CH_2)_3$-, -$(CH_2)_7$-piperidinylene-$(CH_2)_4$-, -$(CH_2)_7$-piperidinylene-$(CH_2)_8$-, -$(CH_2)_8$-piperidinylene-$CH_2$-, -$(CH_2)_8$-piperidinylene-$(CH_2)_2$-, -$(CH_2)_8$-piperidinylene-$(CH_2)_3$-, -$(CH_2)_8$-piperidinylene-$(CH_2)_4$-, -$(CH_2)_8$-piperidinylene-$(CH_2)_5$-, -$(CH_2)_8$-piperidinylene-$(CH_2)_6$-, -$(CH_2)_8$-piperidinylene-$(CH_2)_7$-, -$N(R_{a7})$-$CH_2$-piperidinylene-$CH_2$-, -$N(R_{a7})$-$CH_2$-piperidinylene-$(CH_2)_2$-, -$N(R_{a7})$-$CH_2$-piperidinylene-$(CH_2)_3$-, -$N(R_{a7})$-$CH_2$-piperidinylene-$(CH_2)_4$-, -$N(R_{a7})$-$CH_2$-piperidinylene-$(CH_2)_5$-, - $N(R_{a7})$-$CH_2$-piperidinylene-$(CH_2)_6$-, -$N(R_{a7})$-$CH_2$-piperidinylene-$(CH_2)_7$-, -$N(R_{a7})$-$CH_2$-piperidinylene-$(CH_2)_8$-, -$CH_2$-$N(R_{a7})$-$CH_2$-piperidinylene-$CH_2$-, -$CH_2$-$N(R_{a7})$-$CH_2$-piperidinylene-$(CH_2)_2$-, -$CH_2$-$N(R_{a7})$-$CH_2$-piperidinylene-$(CH_2)_3$-,

$-CH_2-N(R_{a7})-CH_2$-piperidinylene-$(CH_2)_4$-, $-CH_2-N(R_{a7})-CH_2$-piperidinylene-$(CH_2)_5$-, $-CH_2-N(R_{a7})-CH_2$-piperidinylene-$(CH_2)_6$-, $-CH_2-N(R_{a7})-CH_2$-piperidinylene-$(CH_2)_7$-, $-CH_2-N(R_{a7})-CH_2$-piperidinylene-$(CH_2)_8$-, $-CH_2-N(R_{a7})-(CH_2)_2$-piperidinylene-$CH_2$-, $-CH_2-N(R_{a7})-(CH_2)_2$-piperidinylene-$(CH_2)_2$-, $-CH_2-N(R_{a7})-(CH_2)_2$-piperidinylene-$(CH_2)_3$-, $-CH_2-N(R_{a7})-(CH_2)_2$-piperidinylene-$(CH_2)_4$-, $-CH_2-N(R_{a7})-(CH_2)_2$-piperidinylene-$(CH_2)_5$-, $-CH_2-N(R_{a7})-(CH_2)_2$-piperidinylene-$(CH_2)_6$-, $-CH_2-N(R_{a7})-(CH_2)_2$-piperidinylene-$(CH_2)_7$-, $-CH_2-N(R_{a7})-(CH_2)_2$-piperidinylene-$(CH_2)_8$-, $-(CH_2)_2-N(R_{a7})-CH_2$-piperidinylene-$CH_2$-, $-(CH_2)_2-N(R_{a7})-CH_2$-piperidinylene-$(CH_2)_2$-, $-(CH_2)_2-N(R_{a7})-CH_2$-piperidinylene-$(CH_2)_3$-, $-(CH_2)_2-N(R_{a7})-CH_2$-piperidinylene-$(CH_2)_4$-, $-(CH_2)_2-N(R_{a7})-CH_2$-piperidinylene-$(CH_2)_5$-, $-(CH_2)_2-N(R_{a7})-CH_2$-piperidinylene-$(CH_2)_6$-, $-(CH_2)_2-N(R_{a7})-CH_2$-piperidinylene-$(CH_2)_7$-, $-(CH_2)_2-N(R_{a7})-CH_2$-piperidinylene-$(CH_2)_8$-, $-(CH_2)_3-N(R_{a7})-CH_2$-piperidinylene-$CH_2$-, $-(CH_2)_3-N(R_{a7})-CH_2$-piperidinylene-$(CH_2)_2$-, $-(CH_2)_3-N(R_{a7})-CH_2$-piperidinylene-$(CH_2)_3$-, $-(CH_2)_3-N(R_{a7})-CH_2$-piperidinylene-$(CH_2)_8$-, $-(CH_2)_4-N(R_{a7})-CH_2$-piperidinylene-$CH_2$-, $-(CH_2)_4-N(R_{a7})-CH_2$-piperidinylene-$(CH_2)_2$-, $-(CH_2)_4-N(R_{a7})-CH_2$-piperidinylene-$(CH_2)_3$-, $-(CH_2)_4-N(R_{a7})-CH_2$-piperidinylene-$(CH_2)_8$-, $-(CH_2)_5-N(R_{a7})-CH_2$-piperidinylene-$(CH_2)_3$-, $-(CH_2)_5-N(R_{a7})-CH_2$-piperidinylene-$(CH_2)_8$-, $-(CH_2)_6-N(R_{a7})-CH_2$-piperidinylene-$(CH_2)_3$-, $-(CH_2)_6-N(R_{a7})-CH_2$-Piperidinylene-$(CH_2)_8$-, $-(CH_2)_7-N(R_{a7})-CH_2$-piperidinylene-$(CH_2)_3$-, $-(CH_2)_8-N(R_{a7})-CH_2$-piperidinylene-$CH_2$-, $-(CH_2)_8-N(R_{a7})-CH_2$-piperidinylene-$(CH_2)_2$-, $-(CH_2)_8-N(R_{a7})-CH_2$-piperidinylene-$(CH_2)_3$-, $-(CH_2)_8-N(R_{a7})-CH_2$-piperidinylene-$(CH_2)_4$-, $-(CH_2)_8-N(R_{a7})-CH_2$-piperidinylene-$(CH_2)_5$-, $-(CH_2)_8-N(R_{a7})-CH_2$-piperidinylene-$(CH_2)_6$-, -piperazinylene-$CH_2$-, -piperazinylene-$(CH_2)_2$-, -piperazinylene-$(CH_2)_3$-, -piperazinylene-$(CH_2)_4$-, -piperazinylene-$(CH_2)_5$-, -piperazinylene-$(CH_2)_6$-, -piperazinylene-$(CH_2)_7$-, -piperazinylene-$(CH_2)_8$-, $-CH_2$-piperazinylene-$CH_2$-, $-CH_2$-piperazinylene-$(CH_2)_2$-, $-CH_2$-piperazinylene-$(CH_2)_3$-, $-CH_2$-piperazinylene-$(CH_2)_4$-, $-CH_2$-piperazinylene-$(CH_2)_5$-, $-CH_2$-piperazinylene-$(CH_2)_6$-, $-CH_2$-piperazinylene-$(CH_2)_7$-, $-CH_2$-piperazinylene-$(CH_2)_8$-, $-(CH_2)_2$-piperazinylene-$(CH_2)_1$-,$-(CH_2)_2$-piperazinylene-$(CH_2)_2$-, $-(CH_2)_2$-piperazinylene-$(CH_2)_3$-, $-(CH_2)_2$-piperazinylene-$(CH_2)_4$-, $-(CH_2)_2$-piperazinylene-$(CH_2)_5$-, $-(CH_2)_2$-piperazinylene-$(CH_2)_6$-, $-(CH_2)_2$-piperazinylene-$(CH_2)_7$-, $-(CH_2)_2$-piperazinylene-$(CH_2)_8$-, $-(CH_2)_3$-piperazinylene-$CH_2$-, $-(CH_2)_3$-piperazinylene-$(CH_2)_2$-, $-(CH_2)_3$-piperazinylene-$(CH_2)_3$-, $-(CH_2)_3$-piperazinylene-$(CH_2)_4$-, $-(CH_2)_3$-piperazinylene-$(CH_2)_5$-, $-(CH_2)_3$-piperazinylene-$(CH_2)_6$-, $-(CH_2)_3$-piperazinylene-$(CH_2)_7$-, $-(CH_2)_3$-piperazinylene-$(CH_2)_8$-, $-(CH_2)_4$-piperazinylene-$CH_2$-, $-(CH_2)_4$-piperazinylene-$(CH_2)_2$-, $-(CH_2)_4$-piperazinylene-$(CH_2)_3$-, $-(CH_2)_4$-piperazinylene-$(CH_2)_4$-, $-(CH_2)_4$-piperazinylene-$(CH_2)_5$-, $-(CH_2)_4$-piperazinylene-$(CH_2)_6$-, $-(CH_2)_4$-piperazinylene-$(CH_2)_7$-, $-(CH_2)_4$-piperazinylene-$(CH_2)_8$-, $-(CH_2)_5$-piperazinylene-$(CH_2)_1$-, $-(CH_2)_5$-piperazinylene-$(CH_2)_2$-, $-(CH_2)_5$-piperazinylene-$(CH_2)_3$-, $-(CH_2)_5$-piperazinylene-$(CH_2)_4$-, $-(CH_2)_5$-piperazinylene-$(CH_2)_5$-, $-(CH_2)_5$-piperazinylene-$(CH_2)_6$-, $-(CH_2)_5$-piperazinylene-$(CH_2)_7$-, $-(CH_2)_5$-piperazinylene-$(CH_2)_8$-, $-(CH_2)_6$-piperazinylene-$(CH_2)_1$-, $-(CH_2)_6$-piperazinylene-$(CH_2)_2$-, -(CH2)6-piperazinylene-$(CH_2)_3$-, $-(CH_2)_6$-piperazinylene-$(CH_2)_4$-, $-(CH_2)_6$-piperazinylene-$(CH_2)_5$-, $-(CH_2)_6$-piperazinylene-$(CH_2)_6$-, $-(CH_2)_6$-piperazinylene-$(CH_2)_7$-, $-(CH_2)_6$-piperazinylene-$(CH_2)_8$-, $-(CH_2)_7$-piperazinylene-$(CH_2)_1$-, $-(CH_2)_7$-piperazinylene-$(CH_2)_2$-, $-(CH_2)_7$-piperazinylene-$(CH_2)_3$-, $-(CH_2)_7$-piperazinylene-$(CH_2)_4$-, $-(CH_2)_8$-piperazinylene-$CH_2$-, $-(CH_2)_8$-piperazinylene-$(CH_2)_2$-, $-(CH_2)_8$-piperazinylene-$(CH_2)_3$-, $-(CH_2)_8$-piperazinylene-$(CH_2)_4$-, $-(CH_2)_8$-piperazinylene-$(CH_2)_5$-, or $-(CH_2)_8$-piperazinylene-$(CH_2)_6$-;

wherein each $R_{a7}$ independently represents H or $C_{1-6}$ alkyl (e.g., methyl, ethyl, propyl, or isopropyl);

the phenylene is optionally substituted with a substituent selected from the group consisting of deuterium, hydroxy, amino, mercapto, nitro, halogen, cyano, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; and

the piperazinylene and piperidinylene are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.

**7.** The compound of Formula (I) or salts, enantiomers, stereoisomers, solvates, or polymorphs thereof as claimed in claim 1, wherein L represents:

a bond, C(O), C(O)NH, NHC(O), $-CH_2$-, $-(CH_2)_2$-, $-(CH_2)_3$-, $-(CH_2)_4$-, $-(CH_2)_5$-, $-(CH_2)_6$-, $-(CH_2)_7$-, - $(CH_2)_8$-, $-(CH_2)_9$-, $-(CH_2)_{10}$-, $-(CH_2)_{11}$-, $-(CH_2)_{12}$-, $-(CH_2)_{13}$-, $-(CH_2)_{14}$-, $-(CH_2)_{15}$-; $-O-CH_2$-, $-O-(CH_2)_2$-, $-O-(CH_2)_3$-, $-O-(CH_2)_4$-, $-O-(CH_2)_5$-, $-O-(CH_2)_6$-, $-O-(CH_2)_7$-, $-O-(CH_2)_8$-, $-O-(CH_2)_9$-, $-O-(CH_2)_{10}$-, $-(CH_2)_1-O$-, $-(CH_2)_2-O$-, $-(CH_2)_3-O$-, $-(CH_2)_4-O$-, $-(CH_2)_5-O$-, $-(CH_2)_6-O$-, $-(CH_2)_7-O$-, $-(CH_2)_3-O$-, $-(CH_2)_9-O$-, $-(CH_2)_{10}-O$-, $-CH_2-O-CH_2$-, $-CH_2-O-(CH_2)_2$-, $-(CH_2)_1-O-(CH_2)_3$-, $-(CH_2)_1-O-(CH_2)_4$-, $-(CH_2)_1-O-(CH_2)_5$-, $-(CH_2)_1-O-(CH_2)_6$-, $-(CH_2)_1-O-(CH_2)_7$-, $-(CH_2)_1-O-(CH_2)_8$-, $-(CH_2)_2-O-(CH_2)_1$-, $-(CH_2)_2-O-(CH_2)_2$-, $-(CH_2)_2-O-(CH_2)_3$-, $-(CH_2)_2-O-(CH_2)_4$-, $-(CH_2)_2-O-(CH_2)_5$-, $-(CH_2)_2-O-(CH_2)_6$-, $-(CH_2)_2-O-(CH_2)_7$-, $-(CH_2)_2-O-(CH_2)_8$-, $-(CH_2)_3-O-(CH_2)_1$-, $-(CH_2)_3-O-(CH_2)_2$-, $-(CH_2)_3-O-(CH_2)_3$-, $-(CH_2)_3-O-(CH_2)_4$-, $-(CH_2)_3-O-(CH_2)_5$-,

$-(CH_2)_3-O-(CH_2)_6-$, $-(CH_2)_3-O-(CH_2)_7-$, $-(CH_2)_4-O-(CH_2)_1-$, $-(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, $-(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, $-(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, $-(CH_2)_7-O-(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$, $-CH(CH_3)-O-(CH_2)_2-$, $-CH(CH_3)-O-(CH_2)_3-$, $-CH(CH_3)-O-(CH_2)_4-$, $-CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-(O(CH_2)_2)_2-$, $-(O(CH_2)_2)_3-$, $-(O(CH_2)_2)_4-$, $-(O(CH_2)_2)_5-$, $-(O(CH_2)_2)_6-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_1-OCH_2-$, $-CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-CH_2-(O(CH_2)_2)_6-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, $-(CH_2)_2-(O(CH_2)_2)_5-$, $-(CH_2)_2-(O(CH_2)_2)_6-$, $-(CH_2)_3-(O(CH_2)_2)_2-$, $-(CH_2)_3-(O(CH_2)_2)_3-$, $-(CH_2)_3-(O(CH_2)_2)_4-$, $-(CH_2)_3-(O(CH_2)_2)_5-$, $-(CH_2)_4-(O(CH_2)_2)_2-$, $-(CH_2)_4-(O(CH_2)_2)_3-$, $-(CH_2)_4-(O(CH_2)_2)_4-$, $-(CH_2)_4-(O(CH_2)_2)_5-$, $-CH_2-(O(CH_2)_3)_2-$, $-CH_2-(O(CH_2)_3)_3-$, $-CH_2-(O(CH_2)_3)_4-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_3-(O(CH_2)_3)_2-$, $-(CH_2)_3-(O(CH_2)_3)_3-$, $-CH_2-O-(CH_2)_2-O-(CH_2)_3-$, $-CH_2-(O(CH_2)_2)_2-(O(CH_2)_3)_2-$, $(CH_2)_2-O-(CH_2)_2-O-(CH_2)_3-$, $-(CH_2)_2-(O(CH_2)_2)_2-(O(CH_2)_3)_2-$, $-(CH_2)_3-O-(CH_2)_2-O-(CH_2)_3-$, $-(CH_2)_3-(O(CH_2)_2)_2-(O(CH_2)_3)_2-$, $-CH_2-O-(CH_2)_3-O-(CH_2)_2-$, $-CH_2-(O(CH_2)_3)_2-(O(CH_2)_2)_2-$, $(CH_2)_1-N(R_{a7})-(CH_2)_1-$, $-(CH_2)_1-N(R_{a7})-(CH_2)_2-$, $-(CH_2)_1-N(R_{a7})-(CH_2)_3-$, $-(CH_2)_1-N(R_{a7})-(CH_2)_4-$, $-(CH_2)_1-N(R_{a7})-(CH_2)_5-$, $-(CH_2)_1-N(R_{a7})-(CH_2)_6-$, $-N(R_{a7})-(CH_2)_1-$, $-N(R_{a7})-(CH_2)_2-$, $-N(R_{a7})-(CH_2)_3-$, $-N(R_{a7})-(CH_2)_4-$, $-N(R_{a7})-(CH_2)_5-$, $-N(R_{a7})-(CH_2)_6-$, $-(CH_2)_2-N(R_{a7})-(CH_2)_1-$, $-(CH_2)_2-N(R_{a7})-(CH_2)_2-$, $-(CH_2)_2-N(R_{a7})-(CH_2)_3-$, $-(CH_2)_2-N(R_{a7})-(CH_2)_4-$, $-(CH_2)_2-N(R_{a7})-(CH_2)_5-$, $-(CH_2)_2-N(R_{a7})-(CH_2)_6-$, $-(CH_2)_3-N(R_{a7})-(CH_2)_1-$, $-(CH_2)_3-N(R_{a7})-(CH_2)_2-$, $-(CH_2)_3-N(R_{a7})-(CH_2)_3-$, $-(CH_2)_4-N(R_{a7})-(CH_2)_1-$, $-(CH_2)_4-N(R_{a7})-(CH_2)_2-$, $-(CH_2)_4-N(R_{a7})-(CH_2)_3-$, $-(CH_2)_4-N(R_{a7})-(CH_2)_4-$, $-(CH_2)_5-N(R_{a7})-(CH_2)_1-$, $-(CH_2)_5-N(R_{a7})-(CH_2)_2-$, $-(CH_2)_5-N(R_{a7})-(CH_2)_3-$, $-(CH_2)_5-N(R_{a7})-(CH_2)_4-$, $-(CH_2)_5-N(R_{a7})-(CH_2)_5-$, $-(CH_2)_6-N(R_{a7})-(CH_2)_1-$, $-(CH_2)_6-N(R_{a7})-(CH_2)_2-$, $-(CH_2)_6-N(R_{a7})-(CH_2)_3-$, $-(CH_2)_7-N(R_{a7})-(CH_2)_1-$, $-(CH_2)_7-N(R_{a7})-(CH_2)_2-$, $-(CH_2)_7-N(R_{a7})-(CH_2)_3-$, $-(CH_2)_8-N(R_{a7})-(CH_2)_1-$, $-(CH_2)_8-N(R_{a7})-(CH_2)_2-$, $-(CH_2)_8-N(R_{a7})-(CH_2)_3-$, $-CH(CH_3)-N(R_{a7})-(CH_2)_1-$, $-CH(CH_3)-N(R_{a7})-(CH_2)_2-$, $-CH(CH_3)-N(R_{a7})-(CH_2)_3-$, $-CH(CH_3)-N(R_{a7})-(CH_2)_4-$, $-CH(CH_3)-N(R_{a7})-(CH_2)_5-$, $-CH(CH_3)-N(R_{a7})-(CH_2)_6-$, $-(CH_2)_1-N(R_{a7})-$, $-(CH_2)_2-N(R_{a7})-$, $-(CH_2)_3-N(R_{a7})-$, $-(CH_2)_4-N(R_{a7})-$, $-(CH_2)_5-N(R_{a7})-$, $-(CH_2)_6-N(R_{a7})-$, $-(CH_2)_7-N(R_{a7})-$, $-(CH_2)_8-N(R_{a7})-$, $-(CH_2)_9-N(R_{a7})-$, $-(CH_2)_{10}-N(R_{a7})-$, $-C(O)NHCH_2-$, $-C(O)NH(CH_2)_2-$, $-C(O)NH(CH_2)_3-$, $-C(O)NH(CH_2)_4-$, $-C(O)NH(CH_2)_5-$, $-CH_2C(O)NHCH_2-$, $-(CH_2)_2C(O)NH(CH_2)_2-$, $-(CH_2)_2C(O)NH(CH_2)_3-$, $-(CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$, $-(CH_2)_4C(O)NH(CH_2)_4-$, $-(CH_2)_5C(O)NH(CH_2)_5-$, $-(CH_2)_2C(O)NH(CH_2)_2-O-(CH_2)_2-$, $-NHC(O)CH_2-$, $-NHC(O)(CH_2)_2-$, $-NHC(O)(CH_2)_3-$, $-NHC(O)(CH_2)_4-$, $-NHC(O)(CH_2)_5-$, $-CH_2NHC(O)CH_2-$, $-(CH_2)_2NHC(O)(CH_2)_2-$, $-(CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$, $-(CH_2)_3NHC(O)(CH_2)_4-$, $-(CH_2)_4NHC(O)(CH_2)_4-$, $-(CH_2)_5NHC(O)(CH_2)_5-$, $-(CH_2)_2NHC(O)(CH_2)_2-O-(CH_2)_2-$, $-(CH_2)_4NHC(O)CH_2-$, $-CH_2$-phenylene-$CH_2-$, $-CH_2$-phenylene-$(CH_2)_2-$, $-CH_2$-phenylene-$(CH_2)_3-$, $-CH_2$-phenylene-$(CH_2)_4-$, $-CH_2$-phenylene-$(CH_2)_5-$, $-CH_2$-phenylene-$(CH_2)_6-$, $-(CH_2)_2$-phenylene-$(CH_2)_1-$, $-(CH_2)_2$-phenylene-$(CH_2)_2-$, $-(CH_2)_2$-phenylene-$(CH_2)_3-$, $-(CH_2)_2$-phenylene-$(CH_2)_4-$, $-(CH_2)_2$-phenylene-$(CH_2)_5-$, $-(CH_2)_3$-phenylene-$CH_2-$, $-(CH_2)_3$-phenylene-$(CH_2)_2-$, $-(CH_2)_3$-phenylene-$(CH_2)_3-$, $-(CH_2)_3$-phenylene-$(CH_2)_4-$, $-(CH_2)_3$-phenylene-$(CH_2)_5-$, $-(CH_2)_4$-phenylene-$CH_2-$, $-(CH_2)_4$-phenylene-$(CH_2)_2-$, $-(CH_2)_4$-phenylene-$(CH_2)_3-$, $-(CH_2)_4$-phenylene-$(CH_2)_4-$, $-(CH_2)_4$-phenylene-$(CH_2)_5-$, $-CH_2$-piperidinylene-$CH_2-$, $-CH_2$-piperidinylene-$(CH_2)_2-$, $-CH_2$-piperidinylene-$(CH_2)_3-$, $-CH_2$-piperidinylene-$(CH_2)_4-$, $-CH_2$-piperidinylene-$(CH_2)_5-$, $-CH_2$-piperidinylene-$(CH_2)_6-$, $-(CH_2)_2$-piperidinylene-$(CH_2)_1-$, $-(CH_2)_2$-piperidinylene-$(CH_2)_2-$, $-(CH_2)_2$-piperidinylene-$(CH_2)_3-$, $-(CH_2)_2$-piperidinylene-$(CH_2)_4-$, $-(CH_2)_2$-piperidinylene-$(CH_2)_5-$, $-(CH_2)_3$-piperidinylene-$CH_2-$, $-(CH_2)_3$-piperidinylene-$(CH_2)_2-$, $-(CH_2)_3$-piperidinylene-$(CH_2)_3-$, $-(CH_2)_3$-piperidinylene-$(CH_2)_4-$, $-(CH_2)_3$-piperidinylene-$(CH_2)_5-$, $-CH_2$-piperazinylene-$CH_2-$, $-CH_2$-piperazinylene-$(CH_2)_2-$, $-CH_2$-piperazinylene-$(CH_2)_3-$, $-CH_2$-piperazinylene-$(CH_2)_4-$, $-CH_2$-piperazinylene-$(CH_2)_5-$, $-(CH_2)_2$-piperazinylene-$(CH_2)_1-$, $-(CH_2)_2$-piperazinylene-$(CH_2)_2-$, $-(CH_2)_2$-piperazinylene-$(CH_2)_3-$, $-(CH_2)_2$-piperazinylene-$(CH_2)_4-$, $-(CH_2)_2$-piperazinylene-$(CH_2)_5-$, $-(CH_2)_3$-piperazinylene-$CH_2-$, $-(CH_2)_3$-piperazinylene-$(CH_2)_2-$, $-(CH_2)_3$-piperazinylene-$(CH_2)_3-$, $-(CH_2)_3$-piperazinylene-$(CH_2)_4-$, $-(CH_2)_3$-piperazinylene-$(CH_2)_5-$, $-(CH_2)_4$-piperazinylene-$CH_2-$, $-(CH_2)_4$-piperazinylene-$(CH_2)_2-$, $-(CH_2)_4$-piperazinylene-$(CH_2)_3-$, $-(CH_2)_4$-piperazinylene-$(CH_2)_4-$, $-(CH_2)_4$-piperazinylene-$(CH_2)_5-$, $-(CH_2)_8$-piperazinylene-$CH_2-$, $-(CH_2)_8$-piperazinylene-$(CH_2)_2-$, $-(CH_2)_8$-piperazinylene-$(CH_2)_3-$, $-(CH_2)_8$-piperazinylene-$(CH_2)_4-$, or $-(CH_2)_8$-piperazinylene-$(CH_2)_5-$;

wherein each $R_{a7}$ independently represents H or $C_{1-6}$ alkyl (e.g., methyl, ethyl, propyl, or isopropyl);

the phenylene is optionally substituted with a substituent selected from the group consisting of deuterium, hydroxy, amino, mercapto, nitro, halogen, cyano, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof;

the piperazinylene and piperidinylene are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; or L represents:

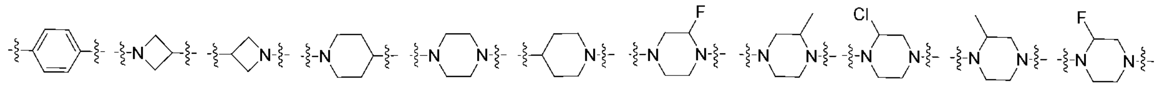

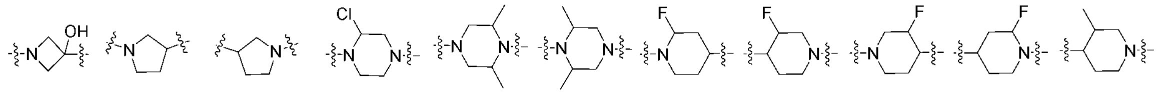

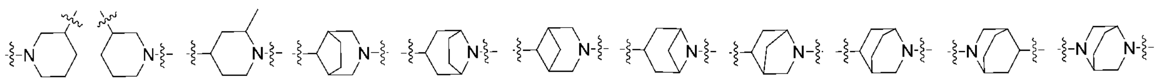

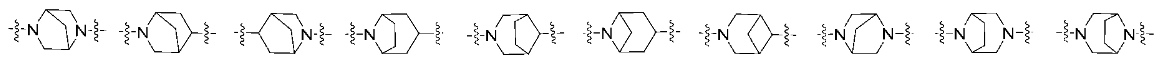

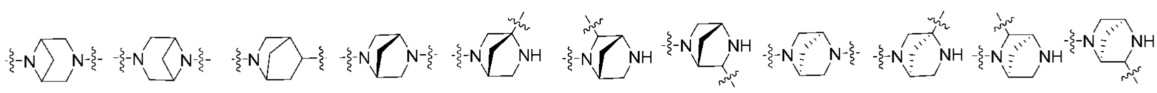

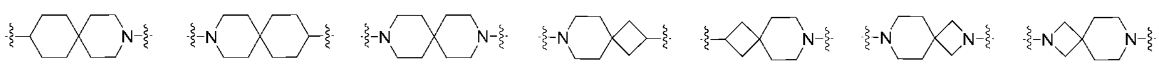

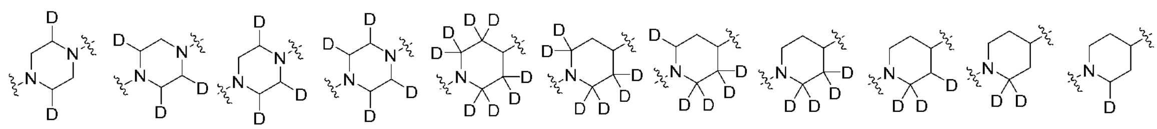

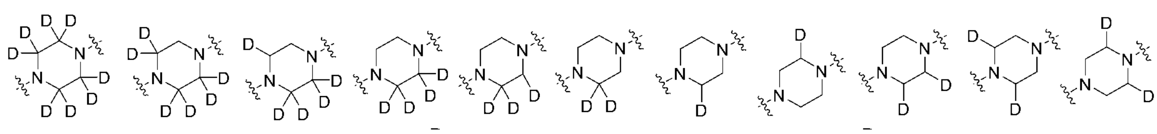

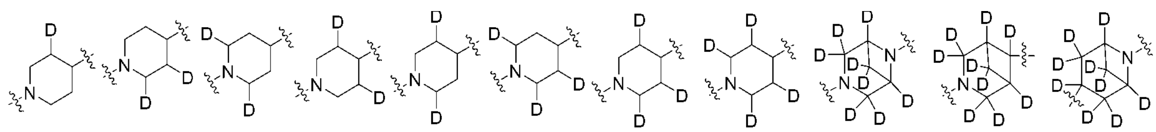

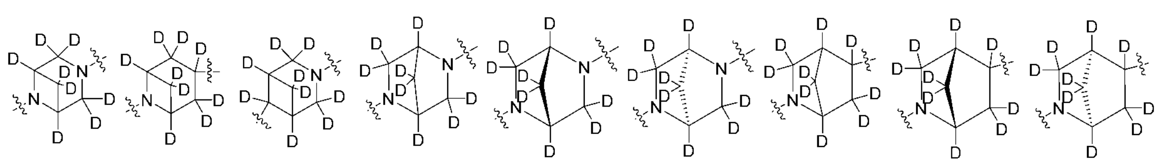

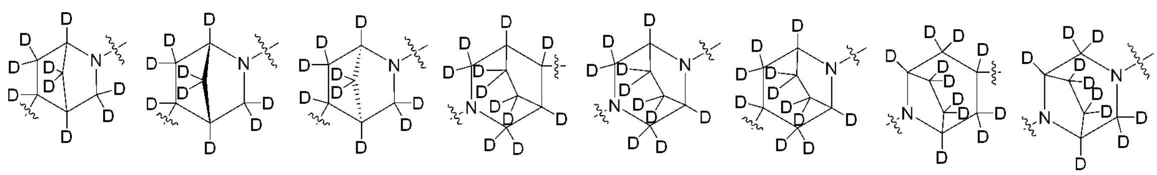

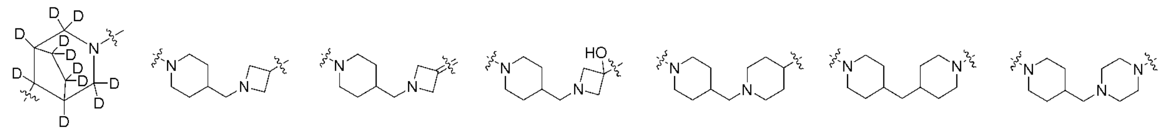

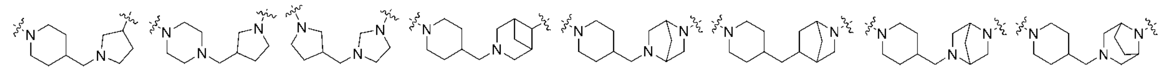

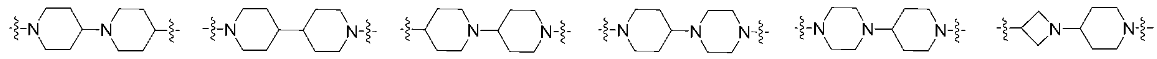

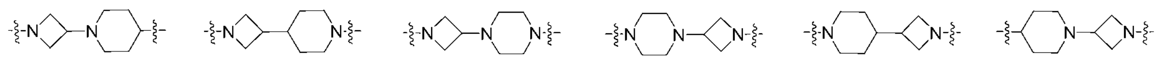

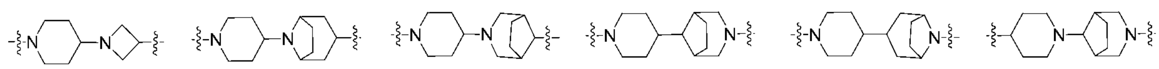

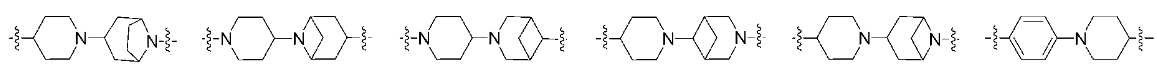

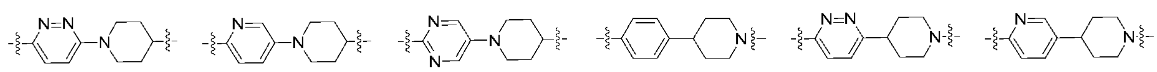

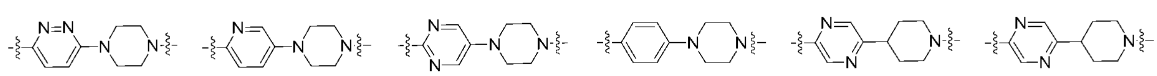

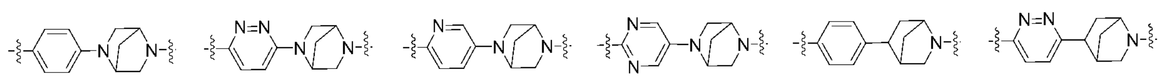

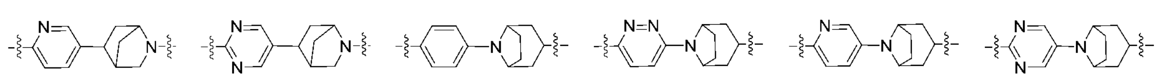

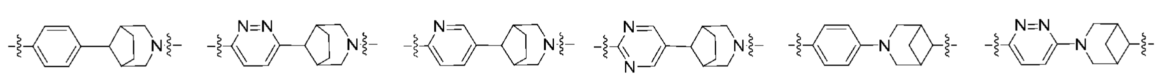

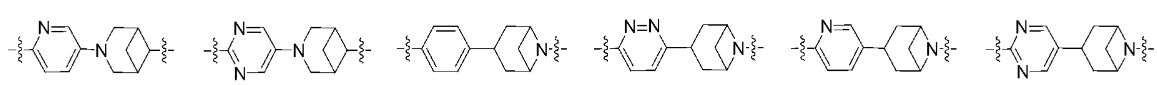

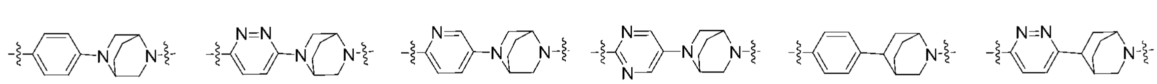

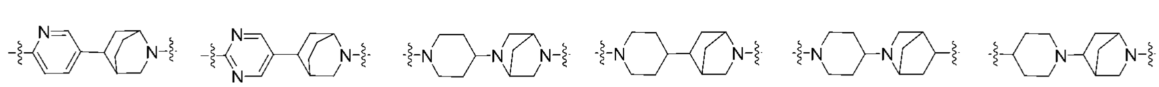

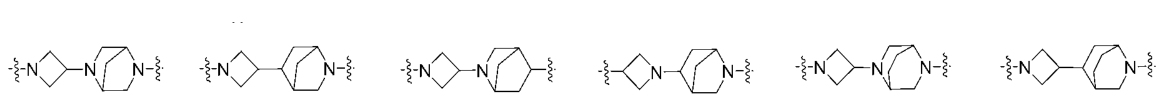

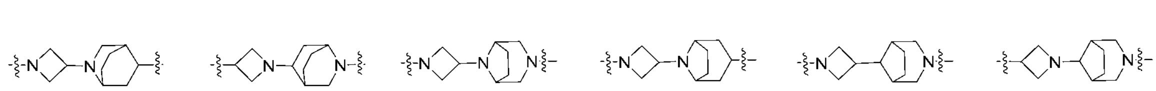

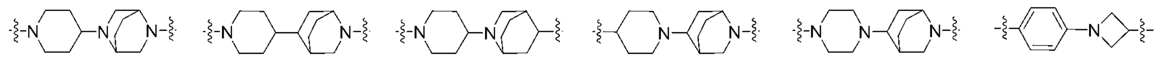

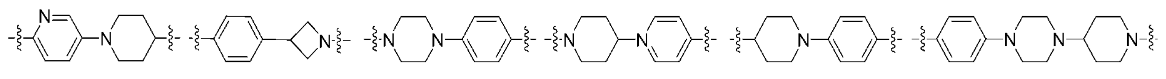

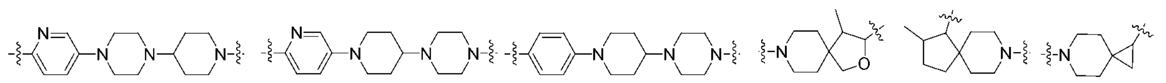

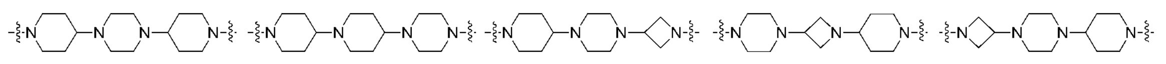

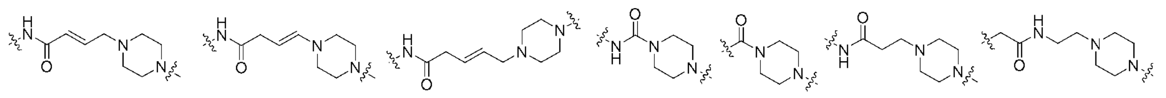

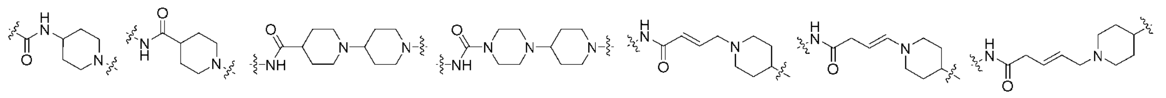

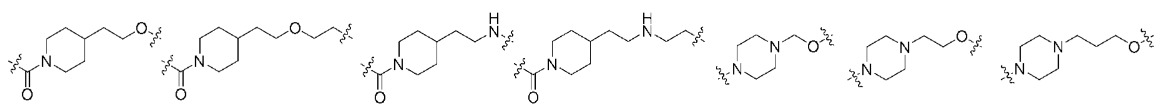

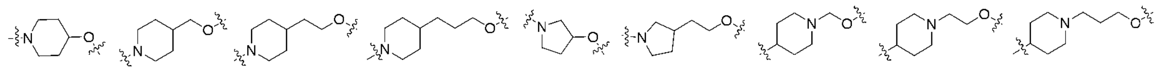

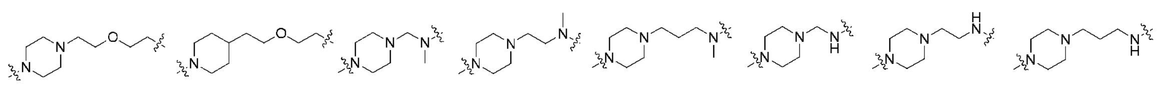

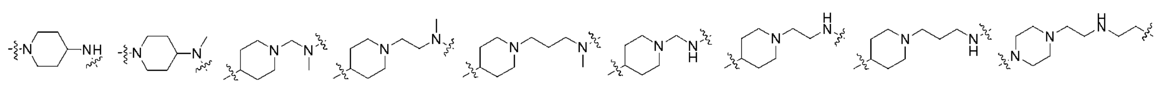

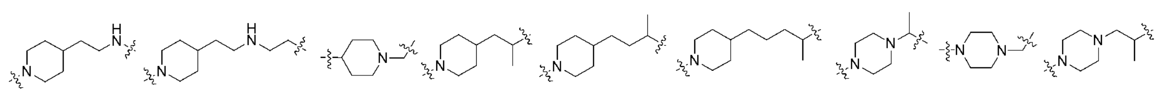

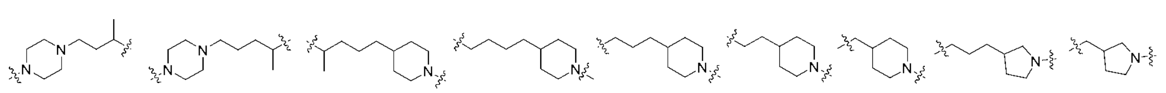

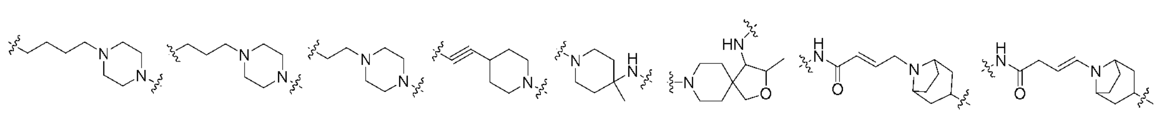

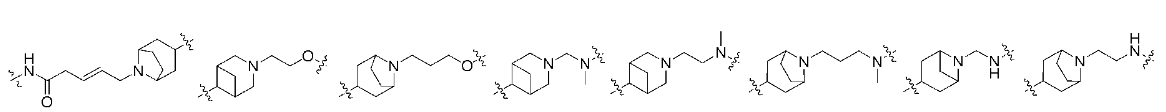

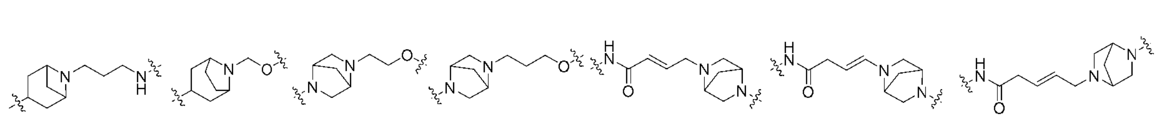

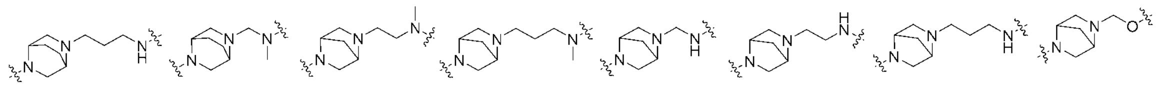

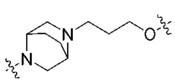 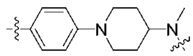 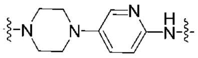 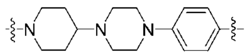 or 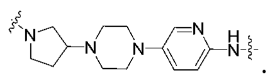 ;

wherein the groups are optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl.

**8.** The compound of Formula (I) or salts, enantiomers, stereoisomers, solvates, or polymorphs thereof as claimed in claim 1, wherein X represents:

$NR_1R_2$, $C(O)NR_3R_4$, $NHC(O)R_5$, $NHC(O)NR_6R_7$, $OR_8$, $SR_9$, $S(O)_2NR_{10}R_{11}$, or $N(R_{12})S(O)_2R_{13}$, wherein $R_1$, $R_3$, $R_4$, $R_5$, $R_6$, $R_7$, $R_9$, $R_{10}$, $R_{11}$, and $R_{12}$ each independently represent H, optionally substituted linear or branched $C_{1-10}$ alkyl, optionally substituted $C_{3-30}$ cycloalkyl, optionally substituted $C_{5-30}$ aryl, optionally substituted 4- to 30-membered heterocyclyl, or optionally substituted 5- to 30-membered heteroaryl, and $R_2$, $R_8$, and $R_{13}$ each independently represent optionally substituted linear or branched $C_{1-10}$ alkyl, optionally substituted $C_{3-30}$ cycloalkyl, optionally substituted $C_{5-30}$ aryl, optionally substituted 4- to 30-membered heterocyclyl, or optionally substituted 5- to 30-membered heteroaryl, or wherein $R_3$ and $R_4$, together with the nitrogen atom to which they are attached, form an optionally substituted 4- to 30-membered nitrogen-containing heterocyclyl; or

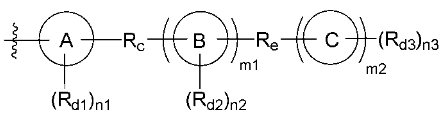

wherein ring A represents 4- to 30-membered heterocyclylene, $C_{3-30}$ cycloalkylene, $C_{5-30}$ arylene, or 5- to 30-membered heteroarylene, and $(R_{d1})_{n1}$ indicates that ring A is optionally substituted with n1 $R_{d1}$ substituents, wherein each $R_{d1}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n1 represents an integer of 0-20;
$R_c$ represents $NR_{c1}$, C(O), $CR_{c2}R_{c3}$, or a bond, wherein $R_{c1}$ represents H, optionally substituted linear or branched $C_{1-10}$ alkyl, optionally substituted $C_{3-30}$ cycloalkyl, optionally substituted $C_{5-30}$ aryl, optionally substituted 4- to 30-membered heterocyclyl, or optionally substituted 5- to 30-membered heteroaryl, wherein $R_{c2}$ and $R_{c3}$ each independently represent H, halogen, optionally substituted linear or branched $C_{1-10}$ alkyl, optionally substituted $C_{3-30}$ cycloalkyl, optionally substituted $C_{5-30}$ aryl, optionally substituted 4- to 30-membered heterocyclyl, or optionally substituted 5- to 30-membered heteroaryl;
each ring B is the same or different and independently represents 4- to 30-membered heterocyclylene, $C_{3-30}$ cycloalkylene, $C_{5-30}$ arylene, or 5- to 30-membered heteroarylene, m1 represents an integer of 0, 1, 2, or 3, and $(R_{d2})_{n2}$ indicates that each ring B is independently optionally substituted with n2 $R_{d2}$ substituents, wherein each $R_{d2}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n2 represents an integer of 0-20;
$R_e$ represents S, O, $CR_{e1}R_{e2}$, or a bond, wherein $R_{e1}$ and $R_{e2}$ each independently represent H, halogen, optionally substituted linear or branched $C_{1-10}$ alkyl, optionally substituted $C_{3-30}$ cycloalkyl, optionally substituted $C_{5-30}$ aryl, optionally substituted 4- to 30-membered heterocyclyl, or optionally substituted 5- to 30-membered heteroaryl; and
each ring C is the same or different and independently represents 4- to 30-membered heterocyclyl, $C_{3-30}$ cycloalkyl, $C_{5-30}$ aryl, or 5- to 30-membered heteroaryl, m2 represents an integer of 0, 1, 2, or 3, and $(R_{d3})_{n3}$ indicates that each ring C is independently optionally substituted with n3 $R_{d3}$ substituents, wherein each $R_{d3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n3 represents an integer of 0-20;
wherein each linear or branched chain $C_{1-10}$ alkyl is independently optionally substituted with a substituent selected from the group consisting of deuterium, halogen, amino, hydroxy, mercapto, cyano, nitro, oxo, $C_{1-6}$ alkyl,

deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl or $C_{2-6}$ alkenyl; and

each of the $C_{3-30}$ cycloalkylene, $C_{5-30}$ arylene, 4- to 30-membered heterocyclylene, 5- to 30-membered heteroarylene, $C_{3-30}$ cycloalkyl, $C_{5-30}$ aryl, 4- to 30-membered heterocyclyl, and 5- to 30-membered heteroaryl is independently optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl.

**9.** The compound of Formula (I) or salts, enantiomers, stereoisomers, solvates, or polymorphs thereof as claimed in claim 1, wherein

(i) $R_1$, $R_3$, $R_4$, $R_5$, $R_6$, $R_7$, $R_9$, $R_{10}$, $R_{11}$, $R_{12}$, and $R_{c1}$ each independently represent:

H, or optionally substituted linear or branched $C_{1-10}$ alkyl;

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornyl, bicyclo[2.2.1]heptanyl, 2-oxobicyclo[2.2.1]heptanyl, or bicyclo[2.2.1]heptenyl), each of which is optionally substituted with one or more (e.g., 1-10) substituents independently selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydro-pyridinyl, dihydroxy-piperidinyl, difluoro-piperidinyl, morpholinyl, thiomorpholinyl,azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-10) substituents independently selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

phenyl or naphthyl, each of which is optionally substituted with one or more (e.g., 1-10) substituents selected from deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each of which is optionally substituted with one or more (e.g., 1-10) substituents selected from deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, haloge-

nated $C_{1-6}$ alkoxy, $C_{2-6}$ alkenyl, $C_{2-6}$ alkynyl, and any combination thereof; and/or

(ii) $R_2$, $R_8$, and $R_{13}$ each independently represent:

optionally substituted linear or branched $C_{1-10}$ alkyl;
cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornyl, bicyclo[2.2.1]heptanyl, 2-oxobicyclo[2.2.1]heptanyl, or bicyclo[2.2.1]heptenyl), each of which is optionally substituted with one or more (e.g., 1-10) substituents independently selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydro-pyridinyl, dihydroxy-piperidinyl, difluoro-piperidinyl, morpholinyl, thiomorpholinyl,azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-10) substituents independently selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;
phenyl or naphthyl, each of which is optionally substituted with one or more (e.g., 1-10) substituents selected from deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or
furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each of which is optionally substituted with one or more (e.g., 1-10) substituents independently selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; and/or

(iii) $R_{c2}$, $R_{c3}$, $R_{c1}$ and $R_{e2}$ each independently represent:

H, halogen, or optionally substituted linear or branched $C_{1-10}$ alkyl; or
cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornyl, bicyclo[2.2.1]heptanyl, 2-oxobicyclo[2.2.1]heptanyl, or bicyclo[2.2.1]heptenyl), each of which is optionally substituted with one or more (e.g., 1-10) substituents independently selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydro-pyridinyl, dihydroxy-piperidinyl, difluoro-piperidinyl, morpholinyl, thiomorpholinyl,azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-10) substituents independently selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

phenyl or naphthyl, each of which is optionally substituted with one or more (e.g., 1-10) substituents selected from deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each of which is optionally substituted with one or more (e.g., 1-10) substituents independently selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

and/or

(iv) $R_3$ and $R_4$, together with the nitrogen atom to which they are attached, form:

azetidinyl, pyrrolidinyl, imidazolidinyl, pyrazolidyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, diazacycloheptanyl, diazacyclooctyl, nitrogen-containing bridged heterocyclyl (e.g., 6- to 20-membered nitrogen-containing bridged heterocyclyl, such as 3-azabicyclo[3.1.0]hexanyl, 3-azabicyclo[3.1.1]heptanyl, 2-azabicyclo[2.2.1]heptanyl, 6-azabicyclo[3.1.1]heptanyl, 2-azabicyclo[2.2.2]octanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 2,5-diazabicyclo[2.2.2]octanyl, and quinuclidinyl), or azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-10) substituents independently selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated

$C_{1-6}$ alkoxy, halogenated $C_{1-6}$ alkoxy, $C_{2-6}$ alkenyl, $C_{2-6}$ alkynyl, and any combination thereof; and/or
(v) ring A and ring B each independently represent:

cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_5$-$C_{20}$ spiro-cycloalkylene, such as spiro[3.3]heptylene, spiro[2.5]octylene, spiro[3.5]nonylene, spiro[4.4]nonylene, spiro[4.5]decylene, or spiro[5.5]undecylene), p-menthanylene, m-menthanylene, or bridged cycloalkylene (e.g., $C_6$-$C_{20}$ bridged cycloalkylene, such as adamantanylene, noradamantanylene, bornylene, norbornylene, bicyclo[2.2.1]heptanylene, 2-oxobicyclo[2.2.1]heptanylene, or bicyclo[2.2.1]heptenylene), each of which is optionally substituted with one or more (e.g., 1-10) substituents independently selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or
azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, tetrahydro-pyridinylene, dihydroxy-piperidinylene, difluoro-piperidinylene, morpholinylene, thiomorpholinylene, azacycloheptanylene, azacyclooctylene, dioxacyclohexylene, azacycloheptanylene, azacyclooctylene, diazacycloheptanylene (e.g., 1,4-diazacycloheptanylene, 4,5-diazacycloheptanylene, 1,3-diazacycloheptanylene), diazacyclooctylene, bridged heterocyclylene (e.g., 6- to 20-membered bridged heterocyclylene, such as 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and quinuclidinylene), azaspirocycloalkylene (e.g., 5- to 20-membered azaspirocycloalkylene, such as 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene, and 7-azaspiro[3.5]nonanylene), or octahydropyrrolo[3,4-c]pyrrolylene, each of which is optionally substituted with one or more (e.g., 1-10) substituents independently selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;
phenylene or naphthylene, each of which is optionally substituted with one or more (e.g., 1-10) substituents selected from deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or
furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, triazinylene, indolylene, isoindolylene, isoindolinylene, benzofuranylene, chromanylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, 2-oxo-2,3-dihydro-1H-benzo[d]imidazolylene, benzo[b][1,4]oxazinylene, 3,4-dihydro-2H-benzo[b][1,4]oxazinylene, quinolinylene, isoquinolinylene, 1,2,3,4-tetrahydroquinolinylene, naphthyridinylene, cinnolinylene, quinazolinylene, quinoxalinylene, 1,2,3,4-tetrahydroquinoxalinylene, phthalazinylene, 5,6,7,8-tetrahydro-[1,2,4]triazolo[4,3-a]pyrazinylene, 4,5,6,7-tetrahydrothieno[3,2-c]pyridinylene, 5-oxo-6,7-dihydrothieno[3,2-d]pyrimidinylene, thieno[2,3-d]pyrimidinylene, thieno[3,2-d]pyrimidinylene, isoxazolo[4,5-c]pyridinylene, isoxazolo[4,5-c]pyrimidinylene, isoxazolo[4,5-d]pyrimidinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, 5,6,7,8-tetrahydrobenzo[4,5]thieno[2,3-d]pyrimidinylene, or 6,7-dihydro-5H-cyclopenta[4,5]thieno[2,3-d]pyrimidinylene, each of which is optionally substituted with one or more (e.g., 1-10) substituents independently selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; and/or

(vi) each ring C is the same or different and independently represents :

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornyl, bicyclo[2.2.1]heptanyl, 2-oxobicyclo[2.2.1]heptanyl, or bicyclo[2.2.1]heptenyl), each of which is optionally substituted with one or more (e.g., 1-10) substituents independently selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydro-pyridinyl, dihydroxy-piperidinyl, difluoro-piperidinyl, morpholinyl, thiomorpholinyl,azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-10) substituents independently selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

phenyl or naphthyl, each of which is optionally substituted with one or more (e.g., 1-10) substituents selected from deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each of which is optionally substituted with one or more (e.g., 1-10) substituents independently selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

**10.** The compound of Formula (I) or salts, enantiomers, stereoisomers, solvates, or polymorphs thereof as claimed in claim 1, wherein X represents:

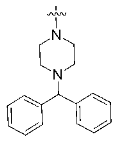 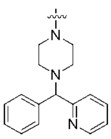 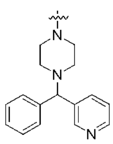 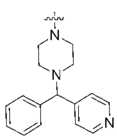 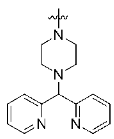 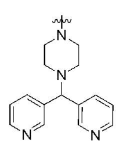 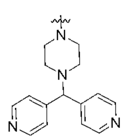 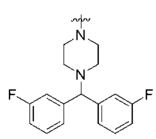 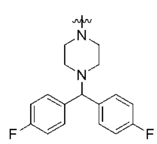

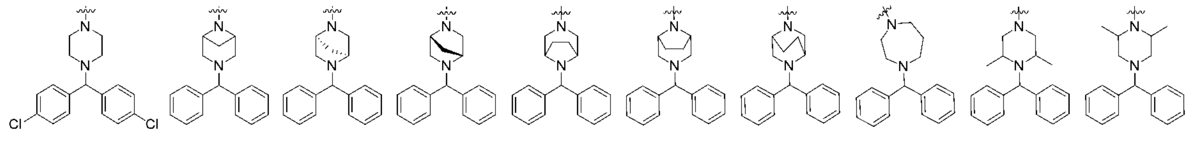

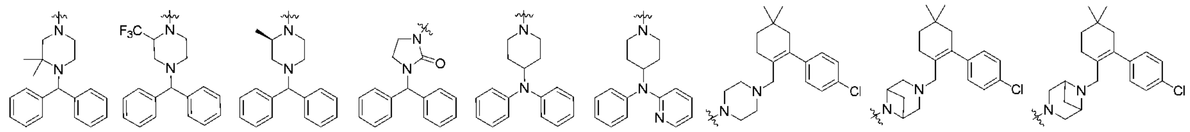

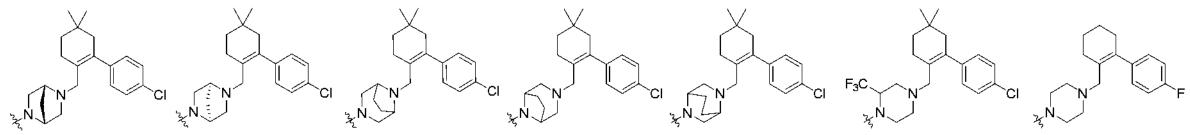

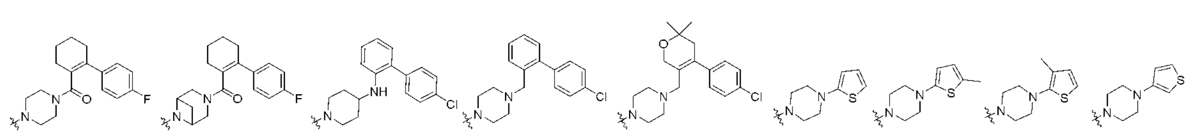

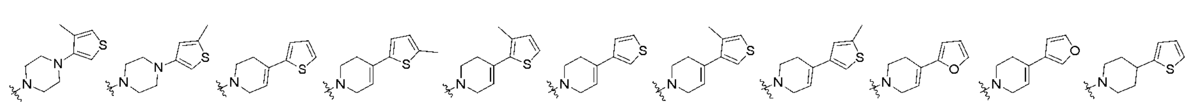

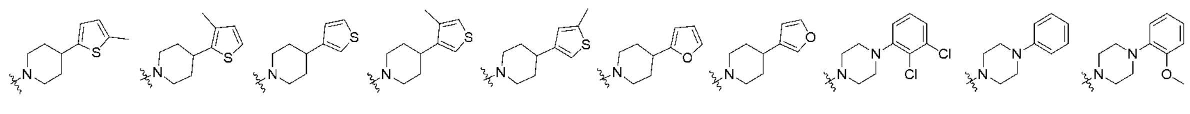

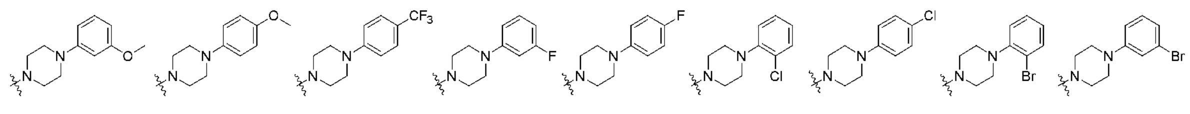

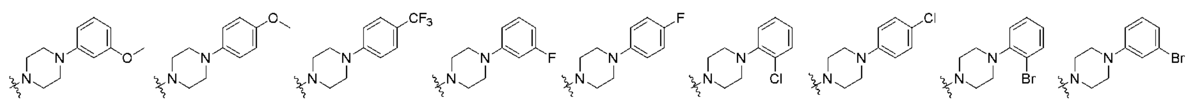

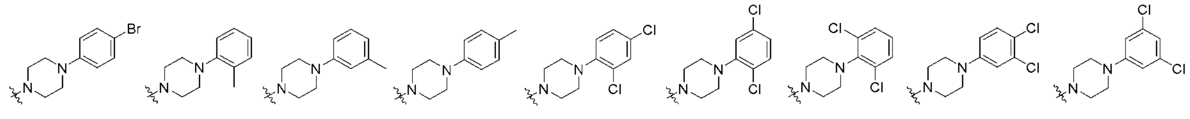

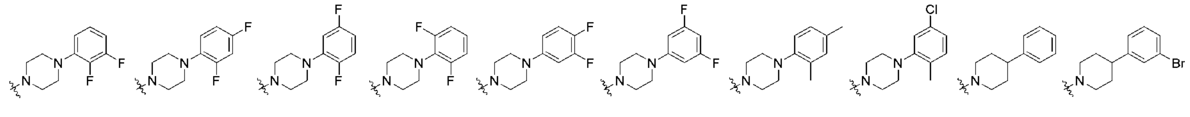

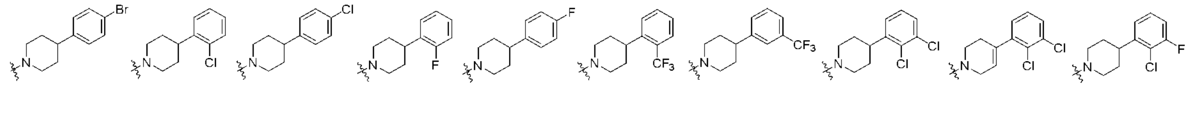

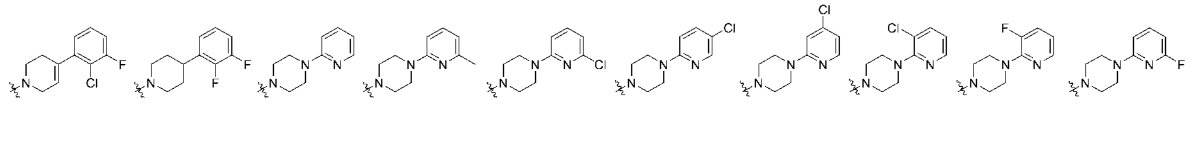

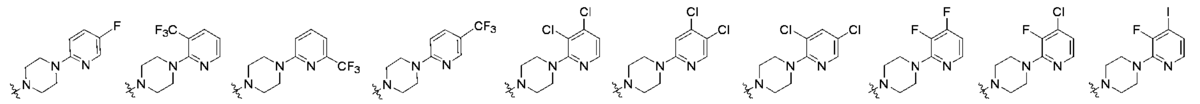

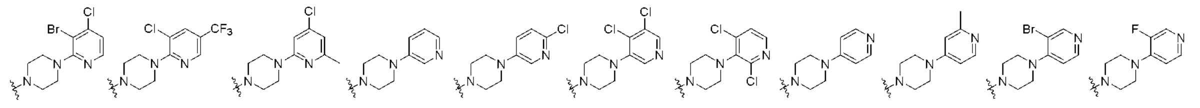

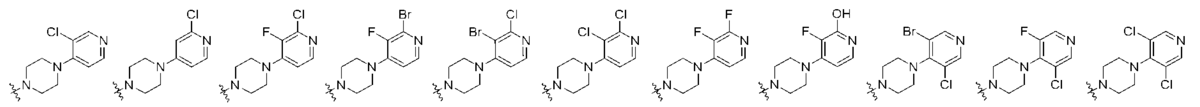

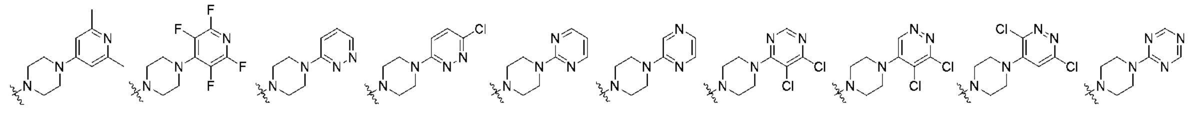

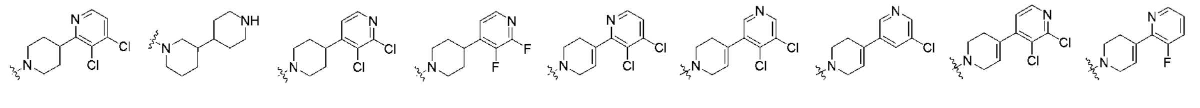

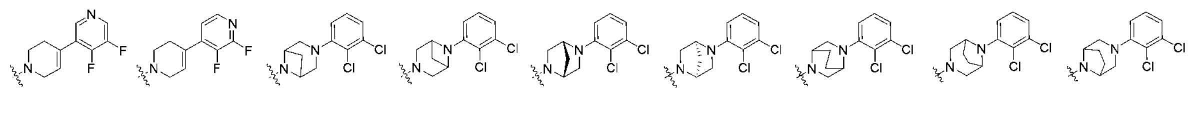

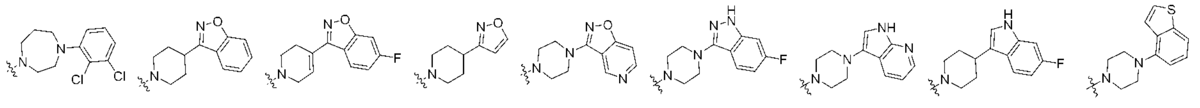

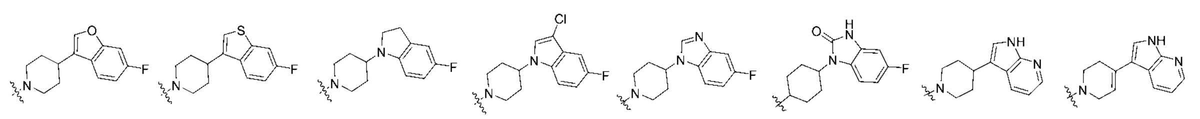

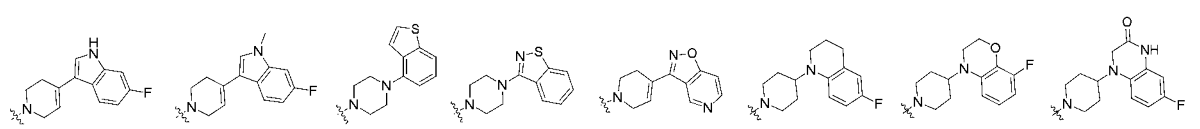

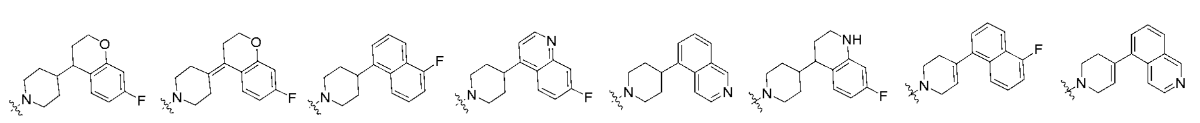

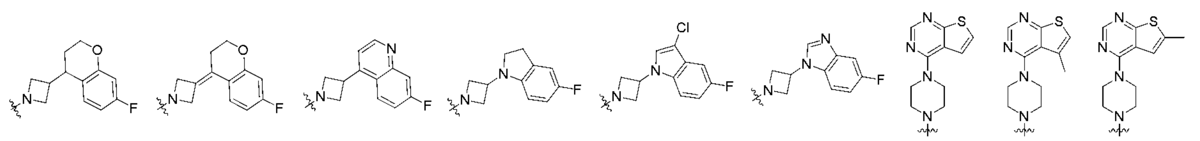

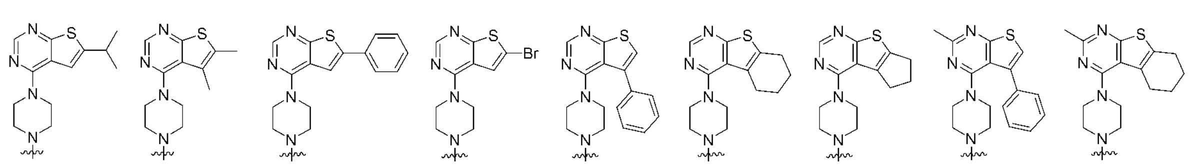

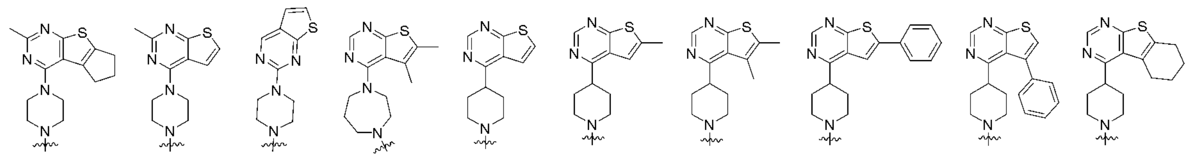

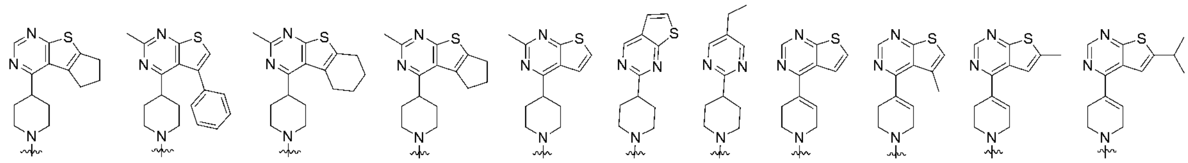

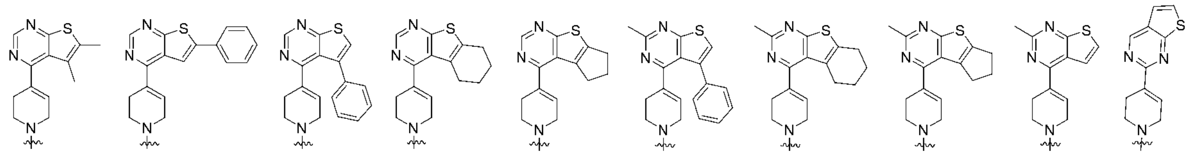

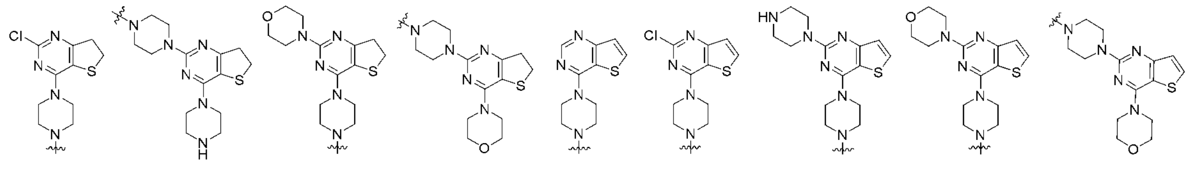

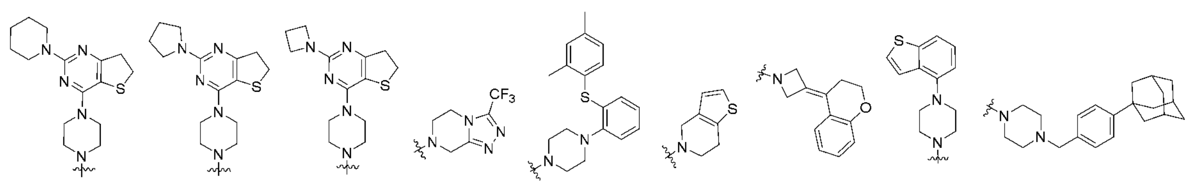

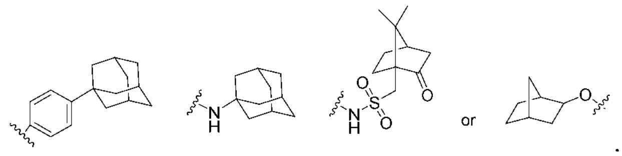

or

.

11. The compound of Formula (I) or salts, enantiomers, stereoisomers, solvates, or polymorphs thereof as claimed in claim 1, which is selected from:

1-(5-((4-benzhydrylpiperazin-1-yl)methyl)-1-oxoisoindolin-2-yl)dihydropyrimidine-2,4(1H,3H)-dione;
1-(1-oxo-5-((4-(phenyl(pyridin-2-yl)methyl)piperazin-1-yl)methyl)isoindolin-2-yl)dihydropyrimidine-2,4(1H,3H)-dione;
1-(1-oxo-5-((4-(phenyl(pyridin-3-yl)methyl)piperazin-1-yl)methyl)isoindolin-2-yl)dihydropyrimidine-2,4(1H,3H)-dione;
1-(1-oxo-5-((4-(phenyl(pyridin-4-yl)methyl)piperazin-1-yl)methyl)isoindolin-2-yl)dihydropyrimidine-2,4(1H,3H)-dione;
1-(5-((4-(di(pyridin-2-yl)methyl)piperazin-1-yl)methyl)-1-oxoisoindolin-2-yl)dihydropyrimidine-2,4(1H,3H)-dione;
1-(5-((4-(di(pyridin-3-yl)methyl)piperazin-1-yl)methyl)-1-oxoisoindolin-2-yl)dihydropyrimidine-2,4(1H,3H)-dione;
1-(5-((4-(di(pyridin-4-yl)methyl)piperazin-1-yl)methyl)-1-oxoisoindolin-2-yl)dihydropyrimidine-2,4(1H,3H)-dione;
1-(5-((4-(bis(3-fluorophenyl)methyl)piperazin-1-yl)methyl)-1-oxoisoindolin-2-yl)dihydropyrimidine-2,4(1H,3H)-dione;
1-(5-((4-(bis(4-fluorophenyl)methyl)piperazin-1-yl)methyl)-1-oxoisoindolin-2-yl)dihydropyrimidine-2,4(1H,3H)-dione;
1-(5-((4-(bis(4-chlorophenyl)methyl)piperazin-1-yl)methyl)-1-oxoisoindolin-2-yl)dihydropyrimidine-2,4(1H,3H)-dione;
1-(5-((3-benzhydryl-3,6-diazabicyclo[3.1.1]heptan-6-yl)methyl)-1-oxoisoindolin-2-yl)dihydropyrimidine-2,4(1H,3H)-dione;
1-(5-(((1S,4S)-5-benzhydryl-2,5-diazabicyclo[2.2.1]heptan-2-yl)methyl)-1-oxoisoindolin-2-yl)dihydropyrimidine-2,4(1H,3H)-dione;
1-(5-(((1R,4R)-5-benzhydryl-2,5-diazabicyclo[2.2.1]heptan-2-yl)methyl)-1-oxoisoindolin-2-yl)dihydropyrimidine-2,4(1H,3H)-dione;
1-(5-((8-benzhydryl-3,8-diazabicyclo[3.2.1]octan-3-yl)methyl)-1-oxoisoindolin-2-yl)dihydropyrimidine-2,4(1H,3H)-dione;
1-(5-((3-benzhydryl-3,8-diazabicyclo[3.2.1]octan-8-yl)methyl)-1-oxoisoindolin-2-yl)dihydropyrimidine-2,4(1H,3H)-dione;
1-(5-((5-benzhydryl-2,5-diazabicyclo[2.2.2]octan-2-yl)methyl)-1-oxoisoindolin-2-yl)dihydropyrimidine-2,4(1H,3H)-dione;
1-(5-((4-benzhydryl-1,4-diazepan-1-yl)methyl)-1-oxoisoindolin-2-yl)dihydropyrimidine-2,4(1H,3H)-dione;
1-(5-((4-benzhydryl-3,5-dimethylpiperazin-1-yl)methyl)-1-oxoisoindolin-2-yl)dihydropyrimidine-2,4(1H,3H)-dione;
1-(5-((4-benzhydryl-2,6-dimethylpiperazin-1-yl)methyl)-1-oxoisoindolin-2-yl)dihydropyrimidine-2,4(1H,3H)-dione;
1-(5-((4-benzhydryl-3,3-dimethylpiperazin-1-yl)methyl)-1-oxoisoindolin-2-yl)dihydropyrimidine-2,4(1H,3H)-dione;
1-(5-((4-benzhydryl-2-(trifluoromethyl)piperazin-1-yl)methyl)-1-oxoisoindolin-2-yl)dihydropyrimidine-2,4(1H,3H)-dione;
(R)-1-(5-((4-benzhydryl-2-methylpiperazin-1-yl)methyl)-1-oxoisoindolin-2-yl)dihydropyrimidine-2,4(1H,3H)-dione;
1-(5-((3-benzhydryl-2-oxoimidazolidin-1-yl)methyl)-1-oxoisoindolin-2-yl)dihydropyrimidine-2,4(1H,3H)-dione;
1-(5-((4-(diphenylamino)piperidin-1-yl)methyl)-1-oxoisoindolin-2-yl)dihydropyrimidine-2,4(1H,3H)-dione;
1-(1-oxo-5-((4-(phenyl(pyridin-2-yl)amino)piperidin-1-yl)methyl)isoindolin-2-yl)dihydropyrimidine-2,4(1H,3H)-dione;
1-(5-((4-((4'-chloro-5,5-dimethyl-3,4,5,6-tetrahydro-[1,1'-biphenyl]-2-yl)methyl)piperazin-1-yl)methyl)-1-oxoisoindolin-2-yl)dihydropyrimidine-2,4(1H,3H)-dione;
1-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-1-oxoisoindolin-2-yl)dihydropyrimidine-2,4(1H,3H)-dione;
1-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-oxoisoindolin-2-yl)dihydropyrimidine-2,4(1H,3H)-dione;
1-(5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)dihydropyrimidine-2,4(1H,3H)-dione;
1-(5-(((1S,4S)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)dihydropyrimidine-2,4(1H,3H)-dione;
1-(5-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-oxoisoindolin-2-yl)dihydropyrimidine-2,4(1H,3H)-dione;
1-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-1-oxoisoindolin-2-yl)dihydropyrimidine-2,4(1H,3H)-dione;
1-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-oxoisoindolin-2-yl)dihydropyrimidine-2,4(1H,3H)-dione;
1-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-oxoisoindolin-2-yl)dihydropyrimidine-2,4(1H,3H)-dione;
1-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-1-oxoisoindolin-2-yl)dihydropyrimidine-2,4(1H,3H)-dione;
1-(5-((4-((4'-fluoro-3,4,5,6-tetrahydro-[1,1'-biphenyl]-2-yl)methyl)piperazin-1-yl)methyl)-1-oxoisoindolin-2-yl)dihydropyrimidine-2,4(1H,3H)-dione;
1-(5-((4-(4'-fluoro-3,4,5,6-tetrahydro-[1,1'-biphenyl]-2-carbonyl)piperazin-1-yl)methyl)-1-oxoisoindolin-2-yl)dihydropyrimidine-2,4(1H,3H)-dione;
1-(5-((3-(4'-fluoro-3,4,5,6-tetrahydro-[1,1'-biphenyl]-2-carbonyl)-3,6-diazabicyclo[3.1.1]heptan-6-yl)methyl)-1-oxoisoindolin-2-yl)dihydropyrimidine-2,4(1H,3H)-dione;
1-(5-((4-((4'-chloro-[1,1'-biphenyl]-2-yl)amino)piperidin-1-yl)methyl)-1-oxoisoindolin-2-yl)dihydropyrimidine-2,4(1H,3H)-dione;

1-(5-((4-((4'-chloro-[1,1'-biphenyl]-2-yl)methyl)piperazin-1-yl)methyl)-1-oxoisoindolin-2-yl)dihydropyrimidine-2,4(1H,3H)-dione;
1-(5-((4-((4-(4-chlorophenyl)-6,6-dimethyl-5,6-dihydro-2H-pyran-3-yl)methyl)piperazin-1-yl)methyl)-1-oxoisoindolin-2-yl)dihydropyrimidine-2,4(1H,3H)-dione;
1-(1-oxo-5-((4-(thiophen-2-yl)piperazin-1-yl)methyl)isoindolin-2-yl)dihydropyrimidine-2,4(1H,3H)-dione;
1-(5-((4-(5-methylthiophen-2-yl)piperazin-1-yl)methyl)-1-oxoisoindolin-2-yl)dihydropyrimidine-2,4(1H,3H)-dione;
1-(5-((4-(3-methylthiophen-2-yl)piperazin-1-yl)methyl)-1-oxoisoindolin-2-yl)dihydropyrimidine-2,4(1H,3H)-dione;
1-(1-oxo-5-((4-(thiophen-3-yl)piperazin-1-yl)methyl)isoindolin-2-yl)dihydropyrimidine-2,4(1H,3H)-dione;
1-(5-((4-(4-methylthiophen-3-yl)piperazin-1-yl)methyl)-1-oxoisoindolin-2-yl)dihydropyrimidine-2,4(1H,3H)-dione;
1-(5-((4-(5-methylthiophen-3-yl)piperazin-1-yl)methyl)-1-oxoisoindolin-2-yl)dihydropyrimidine-2,4(1H,3H)-dione;
1-(1-oxo-5-((4-(thiophen-2-yl)-3,6-dihydropyridin-1(2H)-yl)methyl)isoindolin-2-yl)dihydropyrimidine-2,4(1H,3H)-dione;
1-(5-((4-(5-methylthiophen-2-yl)-3,6-dihydropyridin-1(2H)-yl)methyl)-1-oxoisoindolin-2-yl)dihydropyrimidine-2,4(1H,3H)-dione;
1-(5-((4-(3-methylthiophen-2-yl)-3,6-dihydropyridin-1(2H)-yl)methyl)-1-oxoisoindolin-2-yl)dihydropyrimidine-2,4(1H,3H)-dione;
1-(1-oxo-5-((4-(thiophen-3-yl)-3,6-dihydropyridin-1(2H)-yl)methyl)isoindolin-2-yl)dihydropyrimidine-2,4(1H,3H)-dione;
1-(5-((4-(4-methylthiophen-3-yl)-3,6-dihydropyridin-1(2H)-yl)methyl)-1-oxoisoindolin-2-yl)dihydropyrimidine-2,4(1H,3H)-dione;
1-(5-((4-(5-methylthiophen-3-yl)-3,6-dihydropyridin-1(2H)-yl)methyl)-1-oxoisoindolin-2-yl)dihydropyrimidine-2,4(1H,3H)-dione;
1-(5-((4-(furan-2-yl)-3,6-dihydropyridin-1(2H)-yl)methyl)-1-oxoisoindolin-2-yl)dihydropyrimidine-2,4(1H,3H)-dione;
1-(5-((4-(furan-3-yl)-3,6-dihydropyridin-1(2H)-yl)methyl)-1-oxoisoindolin-2-yl)dihydropyrimidine-2,4(1H,3H)-dione;
1-(1-oxo-5-((4-(thiophen-2-yl)piperidin-1-yl)methyl)isoindolin-2-yl)dihydropyrimidine-2,4(1H,3H)-dione;
1-(5-((4-(5-methylthiophen-2-yl)piperidin-1-yl)methyl)-1-oxoisoindolin-2-yl)dihydropyrimidine-2,4(1H,3H)-dione;
1-(5-((4-(3-methylthiophen-2-yl)piperidin-1-yl)methyl)-1-oxoisoindolin-2-yl)dihydropyrimidine-2,4(1H,3H)-dione;
1-(1-oxo-5-((4-(thiophen-3-yl)piperidin-1-yl)methyl)isoindolin-2-yl)dihydropyrimidine-2,4(1H,3H)-dione;
1-(5-((4-(5-methylthiophen-3-yl)piperidin-1-yl)methyl)-1-oxoisoindolin-2-yl)dihydropyrimidine-2,4(1H,3H)-dione;
1-(5-((4-(4-methylthiophen-3-yl)piperidin-1-yl)methyl)-1-oxoisoindolin-2-yl)dihydropyrimidine-2,4(1H,3H)-dione;
1-(5-((4-(furan-2-yl)piperidin-1-yl)methyl)-1-oxoisoindolin-2-yl)dihydropyrimidine-2,4(1H,3H)-dione;
1-(5-((4-(furan-3-yl)piperidin-1-yl)methyl)-1-oxoisoindolin-2-yl)dihydropyrimidine-2,4(1H,3H)-dione;
1-(5-((4-(2,3-dichlorophenyl)piperazin-1-yl)methyl)-1-oxoisoindolin-2-yl)dihydropyrimidine-2,4(1H,3H)-dione;
1-(1-oxo-5-((4-phenylpiperazin-1-yl)methyl)isoindolin-2-yl)dihydropyrimidine-2,4(1H,3H)-dione;
1-(5-((4-(2-methoxyphenyl)piperazin-1-yl)methyl)-1-oxoisoindolin-2-yl)dihydropyrimidine-2,4(1H,3H)-dione;
1-(5-((4-(3-methoxyphenyl)piperazin-1-yl)methyl)-1-oxoisoindolin-2-yl)dihydropyrimidine-2,4(1H,3H)-dione;
1-(5-((4-(4-methoxyphenyl)piperazin-1-yl)methyl)-1-oxoisoindolin-2-yl)dihydropyrimidine-2,4(1H,3H)-dione;
1-(1-oxo-5-((4-(4-(trifluoromethyl)phenyl)piperazin-1-yl)methyl)isoindolin-2-yl)dihydropyrimidine-2,4(1H,3H)-dione;
1-(5-((4-(3-fluorophenyl)piperazin-1-yl)methyl)-1-oxoisoindolin-2-yl)dihydropyrimidine-2,4(1H,3H)-dione;
1-(5-((4-(4-fluorophenyl)piperazin-1-yl)methyl)-1-oxoisoindolin-2-yl)dihydropyrimidine-2,4(1H,3H)-dione;
1-(5-((4-(2-chlorophenyl)piperazin-1-yl)methyl)-1-oxoisoindolin-2-yl)dihydropyrimidine-2,4(1H,3H)-dione;
1-(5-((4-(4-chlorophenyl)piperazin-1-yl)methyl)-1-oxoisoindolin-2-yl)dihydropyrimidine-2,4(1H,3H)-dione;
1-(5-((4-(2-bromophenyl)piperazin-1-yl)methyl)-1-oxoisoindolin-2-yl)dihydropyrimidine-2,4(1H,3H)-dione;
1-(5-((4-(3-bromophenyl)piperazin-1-yl)methyl)-1-oxoisoindolin-2-yl)dihydropyrimidine-2,4(1H,3H)-dione;
1-(5-((4-(4-bromophenyl)piperazin-1-yl)methyl)-1-oxoisoindolin-2-yl)dihydropyrimidine-2,4(1H,3H)-dione;
1-(1-oxo-5-((4-(o-tolyl)piperazin-1-yl)methyl)isoindolin-2-yl)dihydropyrimidine-2,4(1H,3H)-dione;
1-(1-oxo-5-((4-(m-tolyl)piperazin-1-yl)methyl)isoindolin-2-yl)dihydropyrimidine-2,4(1H,3H)-dione;

1-(1-oxo-5-((4-(p-tolyl)piperazin-1-yl)methyl)isoindolin-2-yl)dihydropyrimidine-2,4(1H,3H)-dione;
1-(5-((4-(2,4-dichlorophenyl)piperazin-1-yl)methyl)-1-oxoisoindolin-2-yl)dihydropyrimidine-2,4(1H,3H)-dione;
1-(5-((4-(2,5-dichlorophenyl)piperazin-1-yl)methyl)-1-oxoisoindolin-2-yl)dihydropyrimidine-2,4(1H,3H)-dione;
1-(5-((4-(2,6-dichlorophenyl)piperazin-1-yl)methyl)-1-oxoisoindolin-2-yl)dihydropyrimidine-2,4(1H,3H)-dione;
1-(5-((4-(3,4-dichlorophenyl)piperazin-1-yl)methyl)-1-oxoisoindolin-2-yl)dihydropyrimidine-2,4(1H,3H)-dione;
1-(5-((4-(3,5-dichlorophenyl)piperazin-1-yl)methyl)-1-oxoisoindolin-2-yl)dihydropyrimidine-2,4(1H,3H)-dione;
1-(5-((4-(2,3-difluorophenyl)piperazin-1-yl)methyl)-1-oxoisoindolin-2-yl)dihydropyrimidine-2,4(1H,3H)-dione;
1-(5-((4-(2,4-difluorophenyl)piperazin-1-yl)methyl)-1-oxoisoindolin-2-yl)dihydropyrimidine-2,4(1H,3H)-dione;
1-(5-((4-(2,5-difluorophenyl)piperazin-1-yl)methyl)-1-oxoisoindolin-2-yl)dihydropyrimidine-2,4(1H,3H)-dione;
1-(5-((4-(2,6-difluorophenyl)piperazin-1-yl)methyl)-1-oxoisoindolin-2-yl)dihydropyrimidine-2,4(1H,3H)-dione;
1-(5-((4-(3,4-difluorophenyl)piperazin-1-yl)methyl)-1-oxoisoindolin-2-yl)dihydropyrimidine-2,4(1H,3H)-dione;
1-(5-((4-(3,5-difluorophenyl)piperazin-1-yl)methyl)-1-oxoisoindolin-2-yl)dihydropyrimidine-2,4(1H,3H)-dione;
1-(5-((4-(2,4-dimethylphenyl)piperazin-1-yl)methyl)-1-oxoisoindolin-2-yl)dihydropyrimidine-2,4(1H,3H)-dione;
1-(5-((4-(5-chloro-2-methylphenyl)piperazin-1-yl)methyl)-1-oxoisoindolin-2-yl)dihydropyrimidine-2,4(1H,3H)-dione;
1-(1-oxo-5-((4-phenylpiperidin-1-yl)methyl)isoindolin-2-yl)dihydropyrimidine-2,4(1H,3H)-dione;
1-(5-((4-(3-bromophenyl)piperidin-1-yl)methyl)-1-oxoisoindolin-2-yl)dihydropyrimidine-2,4(1H,3H)-dione;
1-(5-((4-(4-bromophenyl)piperidin-1-yl)methyl)-1-oxoisoindolin-2-yl)dihydropyrimidine-2,4(1H,3H)-dione;
1-(5-((4-(2-chlorophenyl)piperidin-1-yl)methyl)-1-oxoisoindolin-2-yl)dihydropyrimidine-2,4(1H,3H)-dione;
1-(5-((4-(4-chlorophenyl)piperidin-1-yl)methyl)-1-oxoisoindolin-2-yl)dihydropyrimidine-2,4(1H,3H)-dione;
1-(5-((4-(2-fluorophenyl)piperidin-1-yl)methyl)-1-oxoisoindolin-2-yl)dihydropyrimidine-2,4(1H,3H)-dione;
1-(5-((4-(4-fluorophenyl)piperidin-1-yl)methyl)-1-oxoisoindolin-2-yl)dihydropyrimidine-2,4(1H,3H)-dione;
1-(1-oxo-5-((4-(2-(trifluoromethyl)phenyl)piperidin-1-yl)methyl)isoindolin-2-yl)dihydropyrimidine-2,4(1H,3H)-dione;
1-(1-oxo-5-((4-(3-(trifluoromethyl)phenyl)piperidin-1-yl)methyl)isoindolin-2-yl)dihydropyrimidine-2,4(1H,3H)-dione;
1-(5-((4-(2,3-dichlorophenyl)piperidin-1-yl)methyl)-1-oxoisoindolin-2-yl)dihydropyrimidine-2,4(1H,3H)-dione;
1-(5-((4-(2-chloro-3-fluorophenyl)piperidin-1-yl)methyl)-1-oxoisoindolin-2-yl)dihydropyrimidine-2,4(1H,3H)-dione;
1-(5-((4-(2,3-difluorophenyl)piperidin-1-yl)methyl)-1-oxoisoindolin-2-yl)dihydropyrimidine-2,4(1H,3H)-dione;
1-(5-((4-(2,3-dichlorophenyl)-3,6-dihydropyridin-1(2H)-yl)methyl)-1-oxoisoindolin-2-yl)dihydropyrimidine-2,4(1H,3H)-dione;
1-(5-((4-(2-chloro-3-fluorophenyl)-3,6-dihydropyridin-1(2H)-yl)methyl)-1-oxoisoindolin-2-yl)dihydropyrimidine-2,4(1H,3H)-dione;
1-(1-oxo-5-((4-(pyridin-2-yl)piperazin-1-yl)methyl)isoindolin-2-yl)dihydropyrimidine-2,4(1H,3H)-dione;
1-(5-((4-(6-methylpyridin-2-yl)piperazin-1-yl)methyl)-1-oxoisoindolin-2-yl)dihydropyrimidine-2,4(1H,3H)-dione;
1-(5-((4-(6-chloropyridin-2-yl)piperazin-1-yl)methyl)-1-oxoisoindolin-2-yl)dihydropyrimidine-2,4(1H,3H)-dione;
1-(5-((4-(5-chloropyridin-2-yl)piperazin-1-yl)methyl)-1-oxoisoindolin-2-yl)dihydropyrimidine-2,4(1H,3H)-dione;
1-(5-((4-(4-chloropyridin-2-yl)piperazin-1-yl)methyl)-1-oxoisoindolin-2-yl)dihydropyrimidine-2,4(1H,3H)-dione;
1-(5-((4-(3-chloropyridin-2-yl)piperazin-1-yl)methyl)-1-oxoisoindolin-2-yl)dihydropyrimidine-2,4(1H,3H)-dione;
1-(5-((4-(3-fluoropyridin-2-yl)piperazin-1-yl)methyl)-1-oxoisoindolin-2-yl)dihydropyrimidine-2,4(1H,3H)-dione;
1-(5-((4-(6-fluoropyridin-2-yl)piperazin-1-yl)methyl)-1-oxoisoindolin-2-yl)dihydropyrimidine-2,4(1H,3H)-dione;
1-(5-((4-(5-fluoropyridin-2-yl)piperazin-1-yl)methyl)-1-oxoisoindolin-2-yl)dihydropyrimidine-2,4(1H,3H)-dione;
1-(1-oxo-5-((4-(3-(trifluoromethyl)pyridin-2-yl)piperazin-1-yl)methyl)isoindolin-2-yl)dihydropyrimidine-2,4(1H,3H)-dione;
1-(1-oxo-5-((4-(6-(trifluoromethyl)pyridin-2-yl)piperazin-1-yl)methyl)isoindolin-2-yl)dihydropyrimidine-2,4(1H,3H)-dione;
1-(1-oxo-5-((4-(5-(trifluoromethyl)pyridin-2-yl)piperazin-1-yl)methyl)isoindolin-2-yl)dihydropyrimidine-2,4(1H,3H)-dione;
1-(5-((4-(3,4-dichloropyridin-2-yl)piperazin-1-yl)methyl)-1-oxoisoindolin-2-yl)dihydropyrimidine-2,4(1H,3H)-dione;
1-(5-((4-(3,5-dichloropyridin-2-yl)piperazin-1-yl)methyl)-1-oxoisoindolin-2-yl)dihydropyrimidine-2,4(1H,3H)-dione;
1-(5-((4-(4,5-dichloropyridin-2-yl)piperazin-1-yl)methyl)-1-oxoisoindolin-2-yl)dihydropyrimidine-2,4(1H,3H)-dione;
1-(5-((4-(3,4-difluoropyridin-2-yl)piperazin-1-yl)methyl)-1-oxoisoindolin-2-yl)dihydropyrimidine-2,4(1H,3H)-dione;
1-(5-((4-(4-chloro-3-fluoropyridin-2-yl)piperazin-1-yl)methyl)-1-oxoisoindolin-2-yl)dihydropyrimidine-2,4(1H,3H)-dione;
1-(5-((4-(3-fluoro-4-iodopyridin-2-yl)piperazin-1-yl)methyl)-1-oxoisoindolin-2-yl)dihydropyrimidine-2,4(1H,3H)-dione;
1-(5-((4-(3-bromo-4-chloropyridin-2-yl)piperazin-1-yl)methyl)-1-oxoisoindolin-2-yl)dihydropyrimidine-2,4(1H,3H)-dione;
1-(5-((4-(3-chloro-5-(trifluoromethyl)pyridin-2-yl)piperazin-1-yl)methyl)-1-oxoisoindolin-2-yl)dihydropyrimidine-2,4(1H,3H)-dione;
1-(5-((4-(4-chloro-6-methylpyridin-2-yl)piperazin-1-yl)methyl)-1-oxoisoindolin-2-yl)dihydropyrimidine-2,4(1H,3H)-dione;
1-(1-oxo-5-((4-(pyridin-3-yl)piperazin-1-yl)methyl)isoindolin-2-yl)dihydropyrimidine-2,4(1H,3H)-dione;
1-(5-((4-(6-chloropyridin-3-yl)piperazin-1-yl)methyl)-1-oxoisoindolin-2-yl)dihydropyrimidine-2,4(1H,3H)-dione;
1-(5-((4-(4,5-dichloropyridin-3-yl)piperazin-1-yl)methyl)-1-oxoisoindolin-2-yl)dihydropyrimidine-2,4(1H,3H)-dione;
1-(5-((4-(2,4-dichloropyridin-3-yl)piperazin-1-yl)methyl)-1-oxoisoindolin-2-yl)dihydropyrimidine-2,4(1H,3H)-dione;
1-(1-oxo-5-((4-(pyridin-4-yl)piperazin-1-yl)methyl)isoindolin-2-yl)dihydropyrimidine-2,4(1H,3H)-dione;
1-(5-((4-(2-methylpyridin-4-yl)piperazin-1-yl)methyl)-1-oxoisoindolin-2-yl)dihydropyrimidine-2,4(1H,3H)-dione;
1-(5-((4-(3-bromopyridin-4-yl)piperazin-1-yl)methyl)-1-oxoisoindolin-2-yl)dihydropyrimidine-2,4(1H,3H)-dione;
1-(5-((4-(3-fluoropyridin-4-yl)piperazin-1-yl)methyl)-1-oxoisoindolin-2-yl)dihydropyrimidine-2,4(1H,3H)-dione;
1-(5-((4-(3-chloropyridin-4-yl)piperazin-1-yl)methyl)-1-oxoisoindolin-2-yl)dihydropyrimidine-2,4(1H,3H)-dione;
1-(5-((4-(2-chloropyridin-4-yl)piperazin-1-yl)methyl)-1-oxoisoindolin-2-yl)dihydropyrimidine-2,4(1H,3H)-dione;
1-(5-((4-(2-chloro-3-fluoropyridin-4-yl)piperazin-1-yl)methyl)-1-oxoisoindolin-2-yl)dihydropyrimidine-2,4(1H,3H)-dione;
1-(5-((4-(2-bromo-3-fluoropyridin-4-yl)piperazin-1-yl)methyl)-1-oxoisoindolin-2-yl)dihydropyrimidine-2,4(1H,3H)-dione;
1-(5-((4-(3-bromo-2-chloropyridin-4-yl)piperazin-1-yl)methyl)-1-oxoisoindolin-2-yl)dihydropyrimidine-2,4(1H,3H)-dione;
1-(5-((4-(2,3-dichloropyridin-4-yl)piperazin-1-yl)methyl)-1-oxoisoindolin-2-yl)dihydropyrimidine-2,4(1H,3H)-dione;
1-(5-((4-(2,3-difluoropyridin-4-yl)piperazin-1-yl)methyl)-1-oxoisoindolin-2-yl)dihydropyrimidine-2,4(1H,3H)-dione;
1-(5-((4-(3-fluoro-2-hydroxypyridin-4-yl)piperazin-1-yl)methyl)-1-oxoisoindolin-2-yl)dihydropyrimidine-2,4(1H,3H)-dione;
1-(5-((4-(3-bromo-5-chloropyridin-4-yl)piperazin-1-yl)methyl)-1-oxoisoindolin-2-yl)dihydropyrimidine-2,4(1H,3H)-dione;
1-(5-((4-(3-chloro-5-fluoropyridin-4-yl)piperazin-1-yl)methyl)-1-oxoisoindolin-2-yl)dihydropyrimidine-2,4(1H,3H)-dione;
1-(5-((4-(3,5-dichloropyridin-4-yl)piperazin-1-yl)methyl)-1-oxoisoindolin-2-yl)dihydropyrimidine-2,4(1H,3H)-dione;
1-(5-((4-(2,6-dimethylpyridin-4-yl)piperazin-1-yl)methyl)-1-oxoisoindolin-2-yl)dihydropyrimidine-2,4(1H,3H)-dione;
1-(1-oxo-5-((4-(perfluoropyridin-4-yl)piperazin-1-yl)methyl)isoindolin-2-yl)dihydropyrimidine-2,4(1H,3H)-dione;
1-(1-oxo-5-((4-(pyridazin-3-yl)piperazin-1-yl)methyl)isoindolin-2-yl)dihydropyrimidine-2,4(1H,3H)-dione;
1-(5-((4-(6-chloropyridazin-3-yl)piperazin-1-yl)methyl)-1-oxoisoindolin-2-yl)dihydropyrimidine-2,4(1H,3H)-dione;
1-(1-oxo-5-((4-(pyrimidin-2-yl)piperazin-1-yl)methyl)isoindolin-2-yl)dihydropyrimidine-2,4(1H,3H)-dione;
1-(1-oxo-5-((4-(pyrazin-2-yl)piperazin-1-yl)methyl)isoindolin-2-yl)dihydropyrimidine-2,4(1H,3H)-dione;
1-(5-((4-(5,6-dichloropyrimidin-4-yl)piperazin-1-yl)methyl)-1-oxoisoindolin-2-yl)dihydropyrimidine-2,4(1H,3H)-dione;
1-(5-((4-(5,6-dichloropyridazin-4-yl)piperazin-1-yl)methyl)-1-oxoisoindolin-2-yl)dihydropyrimidine-2,4(1H,3H)-dione;
1-(5-((4-(3,6-dichloropyridazin-4-yl)piperazin-1-yl)methyl)-1-oxoisoindolin-2-yl)dihydropyrimidine-2,4(1H,3H)-dione;
1-(5-((4-(1,3,5-triazin-2-yl)piperazin-1-yl)methyl)-1-oxoisoindolin-2-yl)dihydropyrimidine-2,4(1H,3H)-dione;
1-(5-((4-(3,4-dichloropyridin-2-yl)piperidin-1-yl)methyl)-1-oxoisoindolin-2-yl)dihydropyrimidine-2,4(1H,3H)-dione;
1-(5-((4-(2,3-dichloropyridin-4-yl)piperidin-1-yl)methyl)-1-oxoisoindolin-2-yl)dihydropyrimidine-2,4(1H,3H)-dione;

1-(5-((4-(2,3-difluoropyridin-4-yl)piperidin-1-yl)methyl)-1-oxoisoindolin-2-yl)dihydropyrimidine-2,4(1H,3H)-dione;
1-(5-([3,4'-bipiperidin]-1-ylmethyl)-1-oxoisoindolin-2-yl)dihydropyrimidine-2,4(1H,3H)-dione;
1-(5-((3,4-dichloro-3',6'-dihydro-[2,4'-bipyridin]-1'(2'H)-yl)methyl)-1-oxoisoindolin-2-yl)dihydropyrimidine-2,4(1H,3H)-dione;
1-(5-((4,5-dichloro-3',6'-dihydro-[3,4'-bipyridin]-1'(2'H)-yl)methyl)-1-oxoisoindolin-2-yl)dihydropyrimidine-2,4(1H,3H)-dione;
1-(5-((5-chloro-3',6'-dihydro-[3,4'-bipyridin]-1'(2'H)-yl)methyl)-1-oxoisoindolin-2-yl)dihydropyrimidine-2,4(1H,3H)-dione;
1-(5-((2',3'-dichloro-3,6-dihydro-[4,4'-bipyridin]-1(2H)-yl)methyl)-1-oxoisoindolin-2-yl)dihydropyrimidine-2,4(1H,3H)-dione;
1-(5-((3-fluoro-3',6'-dihydro-[2,4'-bipyridin]-1'(2'H)-yl)methyl)-1-oxoisoindolin-2-yl)dihydropyrimidine-2,4(1H,3H)-dione;
1-(5-((4,5-difluoro-3',6'-dihydro-[3,4'-bipyridin]-1'(2'H)-yl)methyl)-1-oxoisoindolin-2-yl)dihydropyrimidine-2,4(1H,3H)-dione;
1-(5-((2',3'-difluoro-3,6-dihydro-[4,4'-bipyridin]-1(2H)-yl)methyl)-1-oxoisoindolin-2-yl)dihydropyrimidine-2,4(1H,3H)-dione;
1-(5-((3-(2,3-dichlorophenyl)-3,6-diazabicyclo[3.1.1]heptan-6-yl)methyl)-1-oxoisoindolin-2-yl)dihydropyrimidine-2,4(1H,3H)-dione;
1-(5-((6-(2,3-dichlorophenyl)-3,6-diazabicyclo[3.1.1]heptan-3-yl)methyl)-1-oxoisoindolin-2-yl)dihydropyrimidine-2,4(1H,3H)-dione;
1-(5-(((1R,4R)-5-(2,3-dichlorophenyl)-2,5-diazabicyclo[2.2.1]heptan-2-yl)methyl)-1-oxoisoindolin-2-yl)dihydropyrimidine-2,4(1H,3H)-dione;
1-(5-(((1S,4S)-5-(2,3-dichlorophenyl)-2,5-diazabicyclo[2.2.1]heptan-2-yl)methyl)-1-oxoisoindolin-2-yl)dihydropyrimidine-2,4(1H,3H)-dione;
1-(5-((5-(2,3-dichlorophenyl)-2,5-diazabicyclo[2.2.2]octan-2-yl)methyl)-1-oxoisoindolin-2-yl)dihydropyrimidine-2,4(1H,3H)-dione;
1-(5-((8-(2,3-dichlorophenyl)-3,8-diazabicyclo[3.2.1]octan-3-yl)methyl)-1-oxoisoindolin-2-yl)dihydropyrimidine-2,4(1H,3H)-dione;
1-(5-((3-(2,3-dichlorophenyl)-3,8-diazabicyclo[3.2.1]octan-8-yl)methyl)-1-oxoisoindolin-2-yl)dihydropyrimidine-2,4(1H,3H)-dione;
1-(5-((4-(2,3-dichlorophenyl)-1,4-diazepan-1-yl)methyl)-1-oxoisoindolin-2-yl)dihydropyrimidine-2,4(1H,3H)-dione;
1-(5-((4-(6-fluorobenzo[d]isoxazol-3-yl)piperidin-1-yl)methyl)-1-oxoisoindolin-2-yl)dihydropyrimidine-2,4(1H,3H)-dione;
1-(5-((4-(benzo[d]isoxazol-3-yl)piperidin-1-yl)methyl)-1-oxoisoindolin-2-yl)dihydropyrimidine-2,4(lH,3H)-dione;
1-(5-((4-(isoxazol-3-yl)piperidin-1-yl)methyl)-1-oxoisoindolin-2-yl)dihydropyrimidine-2,4(1H,3H)-dione;
1-(5-((4-(6-fluorobenzo[d]isoxazol-3-yl)piperazin-1-yl)methyl)-1-oxoisoindolin-2-yl)dihydropyrimidine-2,4(1H,3H)-dione;
1-(5-((4-(isoxazolo[4,5-c]pyridin-3-yl)piperazin-1-yl)methyl)-1-oxoisoindolin-2-yl)dihydropyrimidine-2,4(1H,3H)-dione;
1-(5-((4-(6-fluorobenzo[d]isothiazol-3-yl)piperazin-1-yl)methyl)-1-oxoisoindolin-2-yl)dihydropyrimidine-2,4(1H,3H)-dione;
1-(5-((4-(6-fluoro-1H-indazol-3-yl)piperazin-1-yl)methyl)-1-oxoisoindolin-2-yl)dihydropyrimidine-2,4(1H,3H)-dione;
1-(5-((4-(1H-pyrrolo[2,3-b]pyridin-3-yl)piperazin-1-yl)methyl)-1-oxoisoindolin-2-yl)dihydropyrimidine-2,4(1H,3H)-dione;
1-(5-((4-(6-fluoro-1H-indol-3-yl)piperidin-1-yl)methyl)-1-oxoisoindolin-2-yl)dihydropyrimidine-2,4(1H,3H)-dione;
1-(5-((4-(6-fluoro-1H-indazol-3-yl)piperidin-1-yl)methyl)-1-oxoisoindolin-2-yl)dihydropyrimidine-2,4(1H,3H)-dione;
1-(5-((4-(6-fluoro-1-methyl-1H-indol-3-yl)piperidin-1-yl)methyl)-1-oxoisoindolin-2-yl)dihydropyrimidine-2,4(1H,3H)-dione;
1-(5-((4-(5-fluoroindolin-1-yl)piperidin-1-yl)methyl)-1-oxoisoindolin-2-yl)dihydropyrimidine-2,4(1H,3H)-dione;
1-(5-((4-(5-fluoro-1H-benzo[d]imidazol-1-yl)piperidin-1-yl)methyl)-1-oxoisoindolin-2-yl)dihydropyrimidine-2,4(1H,3H)-dione;
1-(5-((4-(5-fluoro-2-oxo-2,3-dihydro-1H-benzo[d]imidazol-1-yl)piperidin-1-yl)methyl)-1-oxoisoindolin-2-yl)dihydropyrimidine-2,4(1H,3H)-dione;

1-(5-((4-(1H-pyrrolo[2,3-b]pyridin-3-yl)piperidin-1-yl)methyl)-1-oxoisoindolin-2-yl)dihydropyrimidine-2,4(1H,3H)-dione;
1-(5-((4-(1H-pyrrolo[2,3-b]pyridin-3-yl)-3,6-dihydropyridin-1(2H)-yl)methyl)-1-oxoisoindolin-2-yl)dihydropyrimidine-2,4(1H,3H)-dione;
1-(5-((4-(6-fluoro-1H-indol-3-yl)-3,6-dihydropyridin-1(2H)-yl)methyl)-1-oxoisoindolin-2-yl)dihydropyrimidine-2,4(1H,3H)-dione;
1-(5-((4-(6-fluoro-1-methyl-1H-indol-3-yl)-3,6-dihydropyridin-1(2H)-yl)methyl)-1-oxoisoindolin-2-yl)dihydropyrimidine-2,4(1H,3H)-dione;
1-(5-((4-(6-fluoro-1H-indazol-3-yl)-3,6-dihydropyridin-1(2H)-yl)methyl)-1-oxoisoindolin-2-yl)dihydropyrimidine-2,4(1H,3H)-dione;
1-(5-((4-(6-fluoro-3,4-dihydroquinolin-1(2H)-yl)piperidin-1-yl)methyl)-1-oxoisoindohn-2-yl)dihydropyrimidine-2,4(1H,3H)-dione;
1-(5-((4-(7-fluoro-2,3-dihydro-4H-benzo[b][1,4]oxazin-4-yl)piperidin-1-yl)methyl)-1-oxoisoindolin-2-yl)dihydropyrimidine-2,4(1H,3H)-dione;
1-(5-((4-(7-fluoro-3-oxo-2,3-dihydro-4H-benzo[b][1,4]oxazin-4-yl)piperidin-1-yl)methyl)-1-oxoisoindolin-2-yl)dihydropyrimidine-2,4(1H,3H)-dione;
1-(5-((4-(7-fluorochroman-4-yl)piperidin-1-yl)methyl)-1-oxoisoindolin-2-yl)dihydropyrimidine-2,4(1H,3H)-dione;
1-(5-((4-(7-fluorochroman-4-ylidene)piperidin-1-yl)methyl)-1-oxoisoindolin-2-yl)dihydropyrimidine-2,4(1H,3H)-dione;
1-(5-((4-(5-fluoronaphthalen-1-yl)piperidin-1-yl)methyl)-1-oxoisoindolin-2-yl)dihydropyrimidine-2,4(1H,3H)-dione;
1-(5-((4-(7-fluoroquinolin-4-yl)piperidin-1-yl)methyl)-1-oxoisoindolin-2-yl)dihydropyrimidine-2,4(1H,3H)-dione;
1-(5-((4-(isoquinolin-5-yl)piperidin-1-yl)methyl)-1-oxoisoindolin-2-yl)dihydropyrimidine-2,4(1H,3H)-dione;
1-(5-((4-(5-fluoronaphthalen-1-yl)-3,6-dihydropyridin-1(2H)-yl)methyl)-1-oxoisoindolin-2-yl)dihydropyrimidine-2,4(1H,3H)-dione;
1-(5-((4-(7-fluoroquinolin-4-yl)-3,6-dihydropyridin-1(2H)-yl)methyl)-1-oxoisoindolin-2-yl)dihydropyrimidine-2,4(1H,3H)-dione;
1-(5-((4-(isoquinolin-5-yl)-3,6-dihydropyridin-1 (2H)-yl)methyl)-1-oxoisoindolin-2-yl)dihydropyrimidine-2,4(1H,3H)-dione;
1-(5-((3-(7-fluorochroman-4-yl)azetidin-1-yl)methyl)-1-oxoisoindolin-2-yl)dihydropyrimidine-2,4(1H,3H)-dione;
1-(5-((3-(7-fluorochroman-4-ylidene)azetidin-1-yl)methyl)-1-oxoisoindolin-2-yl)dihydropyrimidine-2,4(1H,3H)-dione;
1-(5-((3-(7-fluoroquinolin-4-yl)azeridin-1-yl)methyl)-1-oxoisoindolin-2-yl)dihydropyrimidine-2,4(1H,3H)-dione;
1-(5-((3-(5-fluoroindolin-1-yl)azetidin-1-yl)methyl)-1-oxoisoindolin-2-yl)dihydropyrimidine-2,4(1H,3H)-dione;
1-(5-((3-(5-fluoro-1H-benzo[d]imidazol-1-yl)azetidin-1-yl)methyl)-1-oxoisoindolin-2-yl)dihydropyrimidine-2,4(1H,3H)-dione;
1-(1-oxo-5-((4-(thieno[2,3-d]pyrimidin-4-yl)piperazin-1-yl)methyl)isoindolin-2-yl)dihydropyrimidine-2,4(1H,3H)-dione;
1-(5-((4-(5-methylthieno[2,3-d]pyrimidin-4-yl)piperazin-1-yl)methyl)-1-oxoisoindolin-2-yl)dihydropyrimidine-2,4(1H,3H)-dione;
1-(5-((4-(6-methylthieno[2,3-d]pyrimidin-4-yl)piperazin-1-yl)methyl)-1-oxoisoindolin-2-yl)dihydropyrimidine-2,4(1H,3H)-dione;
1-(5-((4-(6-isopropylthieno[2,3-d]pyrimidin-4-yl)piperazin-1-yl)methyl)-1-oxoisoindolin-2-yl)dihydropyrimidine-2,4(1H,3H)-dione;
1-(5-((4-(5,6-dimethylthieno[2,3-d]pyrimidin-4-yl)piperazin-1-yl)methyl)-1-oxoisoindolin-2-yl)dihydropyrimidine-2,4(1H,3H)-dione;
1-(1-oxo-5-((4-(6-phenylthieno[2,3-d]pyrimidin-4-yl)piperazin-1-yl)methyl)isoindolin-2-yl)dihydropyrimidine-2,4(1H,3H)-dione;
1-(5-((4-(6-bromothieno[2,3-d]pyrimidin-4-yl)piperazin-1-yl)methyl)-1-oxoisoindolin-2-yl)dihydropyrimidine-2,4(1H,3H)-dione;
1-(1-oxo-5-((4-(5-phenylthieno[2,3-d]pyrimidin-4-yl)piperazin-1-yl)methyl)isoindolin-2-yl)dihydropyrimidine-2,4(1H,3H)-dione;
1-(1-oxo-5-((4-(5,6,7,8-tetrahydrobenzo[4,5]thieno[2,3-d]pyrimidin-4-yl)piperazin-1-yl)methyl)isoindolin-2-yl) dihydropyrimidine-2,4(1H,3H)-dione;
1-(5-((4-(6,7-dihydro-5H-cyclopenta[4,5]thieno[2,3-d]pyrimidin-4-yl)piperazin-1-yl)methyl)-1-oxoisoindolin-2-yl)dihydropyrimidine-2,4(1H,3H)-dione;
1-(5-((4-(2-methyl-5-phenylthieno[2,3-d]pyrimidin-4-yl)piperazin-1-yl)methyl)-1-oxoisoindolin-2-yl)dihydropyrimidine-2,4(1H,3H)-dione;
1-(5-((4-(2-methyl-5,6,7,8-tetrahydrobenzo[4,5]thieno[2,3-d]pyrimidin-4-yl)piperazin-1-yl)methyl)-1-oxoisoindolin-2-yl)dihydropyrimidine-2,4(1H,3H)-dione;
1-(5-((4-(2-methyl-6,7-dihydro-5H-cyclopenta[4,5]thieno[2,3-d]pyrimidin-4-yl)piperazin-1-yl)methyl)-1-oxoisoindolin-2-yl)dihydropyrimidine-2,4(1H,3H)-dione;
1-(5-((4-(2-methylthieno[2,3-d]pyrimidin-4-yl)piperazin-1-yl)methyl)-1-oxoisoindolin-2-yl)dihydropyrimidine-2,4(1H,3H)-dione;
1-(1-oxo-5-((4-(thieno[2,3-d]pyrimidin-2-yl)piperazin-1-yl)methyl)isoindolin-2-yl)dihydropyrimidine-2,4(1H,3H)-dione;
1-(5-((4-(5,6-dimethylthieno[2,3-d]pyrimidin-4-yl)-1,4-diazepan-1-yl)methyl)-1-oxoisoindolin-2-yl)dihydropyrimidine-2,4(1H,3H)-dione;
1-(1-oxo-5-((4-(thieno[2,3-d]pyrimidin-4-yl)piperidin-1-yl)methyl)isoindolin-2-yl)dihydropyrimidine-2,4(1H,3H)-dione;
1-(5-((4-(6-methylthieno[2,3-d]pyrimidin-4-yl)piperidin-1-yl)methyl)-1-oxoisoindolin-2-yl)dihydropyrimidine-2,4(1H,3H)-dione;
1-(5-((4-(5,6-dimethylthieno[2,3-d]pyrimidin-4-yl)piperidin-1-yl)methyl)-1-oxoisoindolin-2-yl)dihydropyrimidine-2,4(1H,3H)-dione;
1-(1-oxo-5-((4-(6-phenylthieno[2,3-d]pyrimidin-4-yl)piperidin-1-yl)methyl)isoindolin-2-yl)dihydropyrimidine-2,4(1H,3H)-dione;
1-(1-oxo-5-((4-(5-phenylthieno[2,3-d]pyrimidin-4-yl)piperidin-1-yl)methyl)isoindolin-2-yl)dihydropyrimidine-2,4(1H,3H)-dione;
1-(1-oxo-5-((4-(5,6,7,8-tetrahydrobenzo[4,5]thieno[2,3-d]pyrimidin-4-yl)piperidin-1-yl)methyl)isoindolin-2-yl)dihydropyrimidine-2,4(1H,3H)-dione;
1-(5-((4-(6,7-dihydro-5H-cyclopenta[4,5]thieno[2,3-d]pyrimidin-4-yl)piperidin-1-yl)methyl)-1-oxoisoindolin-2-yl) dihydropyrimidine-2,4(1H,3H)-dione;
1-(5-((4-(2-methyl-5-phenylthieno[2,3-d]pyrimidin-4-yl)piperidin-1-yl)methyl)-1-oxoisoindolin-2-yl)dihydropyrimidine-2,4(1H,3H)-dione;
1-(5-((4-(2-methyl-5,6,7,8-tetrahydrobenzo[4,5]thieno[2,3-d]pyrimidin-4-yl)piperidin-1-yl)methyl)-1-oxoisoindo1in-2-yl)dihydropyrimidine-2,4(1H,3H)-dione;
1-(5-((4-(2-methyl-6,7-dihydro-5H-cyclopenta[4,5]thieno[2,3-d]pyrimidin-4-yl)piperidin-1-yl)methyl)-1-oxoisoindolin-2-yl)dihydropyrimidine-2,4(1H,3H)-dione;
1-(5-((4-(2-methylthieno[2,3-d]pyrimidin-4-yl)piperidin- 1-yl)methyl)- 1 -oxoisoindolin-2-yl)dihydropyrimidine-2,4(1H,3H)-dione;
1-(1-oxo-5-((4-(thieno[2,3-d]pyrimidin-2-yl)piperidin-1-yl)methyl)isoindolin-2-yl)dihydropyrimidine-2,4(1H,3H)-dione;
1-(5-((4-(5-ethylpyrimidin-2-yl)piperidin-1-yl)methyl)-1-oxoisoindolin-2-yl)dihydropyrimidine-2,4(1H,3H)-dione;
1-(1-oxo-5-((4-(thieno[2,3-d]pyrimidin-4-yl)-3,6-dihydropyridin-1(2H)-yl)methyl)isoindolin-2-yl)dihydropyrimidine-2,4(1H,3H)-dione;
1-(5-((4-(5-methylthieno[2,3-d]pyrimidin-4-yl)-3,6-dihydropyridin-1(2H)-yl)methyl)-1-oxoisoindolin-2-yl)dihydropyrimidine-2,4(1H,3H)-dione;
1-(5-((4-(6-methylthieno[2,3-d]pyrimidin-4-yl)-3,6-dihydropyridin-1(2H)-yl)methyl)-1-oxoisoindolin-2-yl)dihydropyrimidine-2,4(1H,3H)-dione;
1-(5-((4-(6-isopropylthieno[2,3-d]pyrimidin-4-yl)-3,6-dihydropyridin-1(2H)-yl)methyl)-1-oxoisoindolin-2-yl)dihydropyrimidine-2,4(1H,3H)-dione;
1-(5-((4-(5,6-dimethylthieno[2,3-d]pyrimidin-4-yl)-3,6-dihydropyridin-1(2H)-yl)methyl)-1-oxoisoindolin-2-yl)dihydropyrimidine-2,4(1H,3H)-dione;
1-(1-oxo-5-((4-(6-phenylthieno[2,3-d]pyrimidin-4-yl)-3,6-dihydropyridin-1(2H)-yl)methyl)isoindolin-2-yl)dihydropyrimidine-2,4(1H,3H)-dione;
1-(1-oxo-5-((4-(5-phenylthieno[2,3-d]pyrimidin-4-yl)-3,6-dihydropyridin-1(2H)-yl)methyl)isoindolin-2-yl)dihydropyrimidine-2,4(1H,3H)-dione;
1-(1-oxo-5-((4-(5,6,7,8-tetrahydrobenzo[4,5]thieno[2,3-d]pyrimidin-4-yl)-3,6-dihydropyridin-1(2H)-yl)methyl) isoindolin-2-yl)dihydropyrimidine-2,4(1H,3H)-dione;
1-(5-((4-(6,7-dihydro-5H-cyclopenta[4,5]thieno[2,3-d]pyrimidin-4-yl)-3,6-dihydropyridin-1(2H)-yl)methyl)-1-oxoisoindolin-2-yl)dihydropyrimidine-2,4(1H,3H)-dione;
1-(5-((4-(2-methyl-5-phenylthieno[2,3-d]pyrimidin-4-yl)-3,6-dihydropyridin-1(2H)-yl)methyl)-1-oxoisoindolin-2-yl)dihydropyrimidine-2,4(1H,3H)-dione;
1-(5-((4-(2-methyl-5,6,7,8-tetrahydrobenzo[4,5]thieno[2,3-d]pyrimidin-4-yl)-3,6-dihydropyridin-1(2H)-yl) methyl)-1-oxoisoindolin-2-yl)dihydropyrimidine-2,4(1H,3H)-dione;

1-(5-((4-(2-methyl-6,7-dihydro-5H-cyclopenta[4,5]thieno[2,3-d]pyrimidin-4-yl)-3,6-dihydropyridin-1(2H)-yl) methyl)-1-oxoisoindolin-2-yl)dihydropyrimidine-2,4(1H,3H)-dione;
1-(5-((4-(2-methylthieno[2,3-d]pyrimidin-4-yl)-3,6-dihydropyridin-1(2H)-yl)methyl)-1-oxoisoindolin-2-yl)dihydropyrimidine-2,4(1H,3H)-dione;
1-(1-oxo-5-((4-(thieno[2,3-d]pyrimidin-2-yl)-3,6-dihydropyridin-1(2H)-yl)methyl)isoindolin-2-yl)dihydropyrimidine-2,4(1H,3H)-dione;
1-(5-((4-(2-chloro-6,7-dihydrothieno[3,2-d]pyrimidin-4-yl)piperazin-1-yl)methyl)-1-oxoisoindolin-2-yl)dihydropyrimidine-2,4(1H,3H)-dione;
1-(5-((4-(2-morpholino-6,7-dihydrothieno[3,2-d]pyrimidin-4-yl)piperazin-1-yl)methyl)-1-oxoisoindolin-2-yl)dihydropyrimidine-2,4(1H,3H)-dione;
1-(1-oxo-5-((4-(4-(piperazin-1-yl)-6,7-dihydrothieno[3,2-d]pyrimidin-2-yl)piperazin-1-yl)methyl)isoindolin-2-yl) dihydropyrimidine-2,4(1H,3H)-dione;
1-(5-((4-(4-morpholino-6,7-dihydrothieno[3,2-d]pyrimidin-2-yl)piperazin-1-yl)methyl)-1-oxoisoindolin-2-yl)dihydropyrimidine-2,4(1H,3H)-dione;
1-(1-oxo-5-((4-(2-(piperidin-1-yl)-6,7-dihydrothieno[3,2-d]pyrimidin-4-yl)piperazin-1-yl)methyl)isoindolin-2-yl) dihydropyrimidine-2,4(1H,3H)-dione;
1-(1-oxo-5-((4-(2-(pyrrolidin-1-yl)-6,7-dihydrothieno[3,2-d]pyrimidin-4-yl)piperazin-1-yl)methyl)isoindolin-2-yl) dihydropyrimidine-2,4(1H,3H)-dione;
1-(5-((4-(2-(azetidin-1-yl)-6,7-dihydrothieno[3,2-d]pyrimidin-4-yl)piperazin-1-yl)methyl)-1-oxoisoindolin-2-yl)dihydropyrimidine-2,4(1H,3H)-dione;
1-(1-oxo-5-((4-(thieno[3,2-d]pyrimidin-4-yl)piperazin-1-yl)methyl)isoindolin-2-yl)dihydropyrimidine-2,4(1H,3H)-dione;
1-(5-((4-(2-chlorothieno[3,2-d]pyrimidin-4-yl)piperazin-1-yl)methyl)-1-oxoisoindolin-2-yl)dihydropyrimidine-2,4(1H,3H)-dione;
1-(5-((4-(2-morpholinothieno[3,2-d]pyrimidin-4-yl)piperazin-1-yl)methyl)-1-oxoisoindolin-2-yl)dihydropyrimidine-2,4(1H,3H)-dione;
1-(1-oxo-5-((4-(4-(piperazin-1-yl)thieno[3,2-d]pyrimidin-2-yl)piperazin-1-yl)methyl)isoindolin-2-yl)dihydropyrimidine-2,4(1H,3H)-dione;
1-(5-((4-(4-morpholinothieno[3,2-d]pyrimidin-2-yl)piperazin-1-yl)methyl)-1-oxoisoindolin-2-yl)dihydropyrimidine-2,4(1H,3H)-dione;
1-(5-((4-(benzo[b]thiophen-4-yl)piperazin-1-yl)methyl)-1-oxoisoindolin-2-yl)dihydropyrimidine-2,4(1H,3H)-dione;
1-(1-oxo-5-((3-(trifluoromethyl)-5,6-dihydro-[1,2,4]triazolo[4,3-a]pyrazin-7(8H)-yl)methyl)isoindolin-2-yl)dihydropyrimidine-2,4(1H,3H)-dione;
1-(5-((4-(2-((2,4-dimethylphenyl)thio)phenyl)piperazin-1-yl)methyl)-1-oxoisoindolin-2-yl)dihydropyrimidine-2,4(1H,3H)-dione;
1-(5-((6,7-dihydrothieno[3,2-c]pyridin-5(4H)-yl)methyl)-1-oxoisoindolin-2-yl)dihydropyrimidine-2,4(1H,3H)-dione;
1-(5-((4-(benzo[d]isothiazol-3-yl)piperazin-1-yl)methyl)-1-oxoisoindolin-2-yl)dihydropyrimidine-2,4(1H,3H)-dione;
1-(5-((4-(4-((3r,5r,7r)-adamantan-1-yl)benzyl)piperazin-1-yl)methyl)-1-oxoisoindolin-2-yl)dihydropyrimidine-2,4(1H,3H)-dione;
1-(5-((((3s,5s,7s)-adamantan-1-yl)amino)methyl)-1-oxoisoindolin-2-yl)dihydropyrimidine-2,4(1H,3H)-dione;
1-(5-((((1r,3s,5R,7S)-3-hydroxyadamantan-1-yl)amino)methyl)-1-oxoisoindolin-2-yl)dihydropyrimidine-2,4(1H,3H)-dione;
1-((1R,4R)-7,7-dimethyl-2-oxobicyclo[2.2.1]heptan-1-yl)-N-((2-(2,4-dioxotetrahydropyrimidin-1(2H)-yl)-1-oxoisoindolin-5-yl)methyl)methanesulfonamide;
1-(5-((((1R,4S)-bicyclo[2.2.1]heptan-2-yl)oxy)methyl)-1-oxoisoindolin-2-yl)dihydropyrimidine-2,4(1H,3H)-dione;
5-((4-benzhydrylpiperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(phenyl(pyridin-2-yl)methyl)piperazin-1-yl)methyl)isoindoline-1,3-dione;
2-(2,4-dioxotetrahydropyrimidin-1(2H)-yl)-5-((4-(phenyl(pyridin-3-yl)methyl)piperazin-1-yl)methyl)isoindoline-1,3-dione;
2-(2,4-dioxotetrahydropyrimidin-1(2H)-yl)-5-((4-(phenyl(pyridin-4-yl)methyl)piperazin-1-yl)methyl)isoindoline-1,3-dione;
5-((4-(di(pyridin-2-yl)methyl)piperazin-1-yl)methyl)-2-(2,4-dioxotetrahydropyrimidin-1(2H)-yl)isoindoline-1,3-dione;

5-((4-(di(pyridin-3-yl)methyl)piperazin-1-yl)methyl)-2-(2,4-dioxotetrahydropyrimidin-1(2H)-yl)isoindoline-1,3-dione;
5-((4-(di(pyridin-4-yl)methyl)piperazin-1-yl)methyl)-2-(2,4-dioxotetrahydropyrimidin-1(2H)-yl)isoindoline-1,3-dione;
5-((4-(bis(3-fluorophenyl)methyl)piperazin-1-yl)methyl)-2-(2,4-dioxotetrahydropyrimidin-1(2H)-yl)isoindoline-1,3-dione;
5-((4-(bis(4-fluorophenyl)methyl)piperazin-1-yl)methyl)-2-(2,4-dioxotetrahydropyrimidin-1(2H)-yl)isoindoline-1,3-dione;
5-((4-(bis(4-chlorophenyl)methyl)piperazin-1-yl)methyl)-2-(2,4-dioxotetrahydropyrimidin-1(2H)-yl)isoindoline-1,3-dione;
5-((3-benzhydryl-3,6-diazabicyclo[3.1.1]heptan-6-yl)methyl)-2-(2,4-dioxotetrahydropyrimidin-1(2H)-yl)isoindoline-1,3-dione;
5-(((1S,4S)-5-benzhydryl-2,5-diazabicyclo[2.2.1]heptan-2-yl)methyl)-2-(2,4-dioxotetrahydropyrimidin-1(2H)-yl)isoindoline-1,3-dione;
5-(((1R,4R)-5-benzhydryl-2,5-diazabicyclo[2.2.1]heptan-2-yl)methyl)-2-(2,4-dioxotetrahydropyrimidin-1(2H)-yl)isoindoline-1,3-dione;
5-((8-benzhydryl-3,8-diazabicyclo[3.2.1]octan-3-yl)methyl)-2-(2,4-dioxotetrahydropyrimidin-1(2H)-yl)isoindoline-1,3-dione;
5-((3-benzhydryl-3,8-diazabicyclo[3.2.1]octan-8-yl)methyl)-2-(2,4-dioxotetrahydropyrimidin-1(2H)-yl)isoindoline-1,3-dione;
5-((5-benzhydryl-2,5-diazabicyclo[2.2.2]octan-2-yl)methyl)-2-(2,4-dioxotetrahydropyrimidin-1(2H)-yl)isoindoline-1,3-dione;
5-((4-benzhydryl-1,4-diazepan-1-yl)methyl)-2-(2,4-dioxotetrahydropyrimidin-1(2H)-yl)isoindoline-1,3-dione;
5-((4-benzhydryl-3,5-dimethylpiperazin-1-yl)methyl)-2-(2,4-dioxotetrahydropyrimidin-1(2H)-yl)isoindoline-1,3-dione;
5-((4-benzhydryl-2,6-dimethylpiperazin-1-yl)methyl)-2-(2,4-dioxotetrahydropyrimidin-1(2H)-yl)isoindoline-1,3-dione;
5-((4-benzhydryl-3,3-dimethylpiperazin-1-yl)methyl)-2-(2,4-dioxotetrahydropyrimidin-1(2H)-yl)isoindoline-1,3-dione;
5-((4-benzhydryl-2-(trifluoromethyl)piperazin-1-yl)methyl)-2-(2,4-dioxotetrahydropyrimidin-1(2H)-yl)isoindoline-1,3-dione;
(R)-5-((4-benzhydryl-2-methylpiperazin-1-yl)methyl)-2-(2,4-dioxotetrahydropyrimidin-1(2H)-yl)isoindoline-1,3-dione;
5-((3-benzhydryl-2-oxoimidazolidin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(diphenylamino)piperidin-1-yl)methyl)isoindoline-1,3-dione;
2-(2,4-dioxotetrahydropyrimidin-1(2H)-yl)-5-((4-(phenyl(pyridin-2-yl)amino)piperidin-1-yl)methyl)isoindoline-1,3-dione;
5-((4-((4'-chloro-5,5-dimethyl-3,4,5,6-tetrahydro-[1,1'-biphenyl]-2-yl)methyl)piperazin-1-yl)methyl)-2-(2,4-dioxotetrahydropyrimidin-1(2H)-yl)isoindoline-1,3-dione;
5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-2-(2,4-dioxotetrahydropyrimidin-1(2H)-yl)isoindoline-1,3-dione;
5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-2-(2,4-dioxotetrahydropyrimidin-1(2H)-yl)isoindoline-1,3-dione;
5-(((1R,4R)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,4-dioxotetrahydropyrimidin-1(2H)-yl)isoindoline-1,3-dione;
5-(((1S,4S)-5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,4-dioxotetrahydropyrimidin-1(2H)-yl)isoindoline-1,3-dione;
5-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-2-(2,4-dioxotetrahydropyrimidin-1(2H)-yl)isoindoline-1,3-dione;
5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-2-(2,4-dioxotetrahydropyrimidin-1(2H)-yl)isoindoline-1,3-dione;
5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-2-(2,4-dioxotetrahydropyrimidin-1(2H)-yl)isoindoline-1,3-dione;
5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-2-(2,4-dioxotetrahydropyrimidin-1(2H)-yl)isoindoline-1,3-dione;
5-((4-((4'-chloro-5,5-dimethyl-3,4,5,6-tetrahydro-[1,1'-biphenyl]-2-yl)methyl)-2-(trifluoromethyl)piperazin-1-yl)methyl)-2-(2,4-dioxotetrahydropyrimidin-1(2H)-yl)isoindoline-1,3-dione;

2-(2,4-dioxotetrahydropyrimidin-1(2H)-yl)-5-((4-((4'-fluoro-3,4,5,6-tetrahydro-[1,1'-biphenyl]-2-yl)methyl)piperazin-1-yl)methyl)isoindoline-1,3-dione;
2-(2,4-dioxotetrahydropyrimidin-1(2H)-yl)-5-((4-(4'-fluoro-3,4,5,6-tetrahydro-[1,1'-biphenyl]-2-carbonyl)piperazin-1-yl)methyl)isoindoline-1,3-dione;
2-(2,4-dioxotetrahydropyrimidin-1(2H)-yl)-5-((3-(4'-fluoro-3,4,5,6-tetrahydro-[1,1'-biphenyl]-2-carbonyl)-3,6-diazabicyclo[3.1.1]heptan-6-yl)methyl)isoindoline-1,3-dione;
5-((4-((4'-chloro-[1,1'-biphenyl]-2-yl)amino)piperidin-1-yl)methyl)-2-(2,4-dioxotetrahydropyrimidin-1(2H)-yl)isoindoline-1,3-dione;
5-((4-((4'-chloro-[1,1'-biphenyl]-2-yl)methyl)piperazin-1-yl)methyl)-2-(2,4-dioxotetrahydropyrimidin-1(2H)-yl) isoindoline-1,3-dione;
5-((4-((4-(4-chlorophenyl)-6,6-dimethyl-5,6-dihydro-2H-pyran-3-yl)methyl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(thiophen-2-yl)piperazin-1-yl)methyl)isoindoline-1,3-dione;
2-(2,4-dioxotetrahydropyrimidin-1(2H)-yl)-5-((4-(5-methylthiophen-2-yl)piperazin-1-yl)methyl)isoindoline-1,3-dione;
2-(2,4-dioxotetrahydropyrimidin-1(2H)-yl)-5-((4-(3-methylthiophen-2-yl)piperazin-1-yl)methyl)isoindoline-1,3-dione;
2-(2,4-dioxotetrahydropyrimidin-1(2H)-yl)-5-((4-(thiophen-3-yl)piperazin-1-yl)methyl)isoindoline-1,3-dione;
2-(2,4-dioxotetrahydropyrimidin-1(2H)-yl)-5-((4-(4-methylthiophen-3-yl)piperazin-1-yl)methyl)isoindoline-1,3-dione;
2-(2,4-dioxotetrahydropyrimidin-1(2H)-yl)-5-((4-(5-methylthiophen-3-yl)piperazin-1-yl)methyl)isoindoline-1,3-dione;
2-(2,4-dioxotetrahydropyrimidin-1(2H)-yl)-5-((4-(thiophen-2-yl)-3,6-dihydropyridin-1(2H)-yl)methyl)isoindoline-1,3-dione;
2-(2,4-dioxotetrahydropyrimidin-1(2H)-yl)-5-((4-(5-methylthiophen-2-yl)-3,6-dihydropyridin-1(2H)-yl)methyl) isoindoline-1,3-dione;
2-(2,4-dioxotetrahydropyrimidin-1(2H)-yl)-5-((4-(3-methylthiophen-2-yl)-3,6-dihydropyridin-1(2H)-yl)methyl) isoindoline-1,3-dione;
2-(2,4-dioxotetrahydropyrimidin-1(2H)-yl)-5-((4-(thiophen-3-yl)-3,6-dihydropyridin-1(2H)-yl)methyl)isoindoline-1,3-dione;
2-(2,4-dioxotetrahydropyrimidin-1(2H)-yl)-5-((4-(4-methylthiophen-3-yl)-3,6-dihydropyridin-1(2H)-yl)methyl) isoindoline-1,3-dione;
2-(2,4-dioxotetrahydropyrimidin-1(2H)-yl)-5-((4-(5-methylthiophen-3-yl)-3,6-dihydropyridin-1(2H)-yl)methyl) isoindoline-1,3-dione;
2-(2,4-dioxotetrahydropyrimidin-1(2H)-yl)-5-((4-(furan-2-yl)-3,6-dihydropyridin-1(2H)-yl)methyl)isoindoline-1,3-dione;
2-(2,4-dioxotetrahydropyrimidin-1(2H)-yl)-5-((4-(furan-3-yl)-3,6-dihydropyridin-1(2H)-yl)methyl)isoindoline-1,3-dione;
2-(2,4-dioxotetrahydropyrimidin-1(2H)-yl)-5-((4-(thiophen-2-yl)piperidin-1-yl)methyl)isoindoline-1,3-dione;
2-(2,4-dioxotetrahydropyrimidin-1(2H)-yl)-5-((4-(5-methylthiophen-2-yl)piperidin-1-yl)methyl)isoindoline-1,3-dione;
2-(2,4-dioxotetrahydropyrimidin-1(2H)-yl)-5-((4-(3-methylthiophen-2-yl)piperidin-1-yl)methyl)isoindoline-1,3-dione;
2-(2,4-dioxotetrahydropyrimidin-1(2H)-yl)-5-((4-(thiophen-3-yl)piperidin-1-yl)methyl)isoindoline-1,3-dione;
2-(2,4-dioxotetrahydropyrimidin-1(2H)-yl)-5-((4-(5-methylthiophen-3-yl)piperidin-1-yl)methyl)isoindoline-1,3-dione;
2-(2,4-dioxotetrahydropyrimidin-1(2H)-yl)-5-((4-(4-methylthiophen-3-yl)piperidin-1-yl)methyl)isoindoline-1,3-dione;
2-(2,4-dioxotetrahydropyrimidin-1(2H)-yl)-5-((4-(furan-2-yl)piperidin-1-yl)methyl)isoindoline-1,3-dione;
2-(2,4-dioxotetrahydropyrimidin-1(2H)-yl)-5-((4-(furan-3-yl)piperidin-1-yl)methyl)isoindoline-1,3-dione;
5-((4-(2,3-dichlorophenyl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-phenylpiperazin-1-yl)methyl)isoindoline-1,3-dione;
2-(2,4-dioxotetrahydropyrimidin-1(2H)-yl)-5-((4-(2-methoxyphenyl)piperazin-1-yl)methyl)isoindoline-1,3-dione;
2-(2,4-dioxotetrahydropyrimidin-1(2H)-yl)-5-((4-(3-methoxyphenyl)piperazin-1-yl)methyl)isoindoline-1,3-dione;
2-(2,4-dioxotetrahydropyrimidin-1(2H)-yl)-5-((4-(4-methoxyphenyl)piperazin-1-yl)methyl)isoindoline-1,3-dione;
2-(2,4-dioxotetrahydropyrimidin-1(2H)-yl)-5-((4-(4-(trifluoromethyl)phenyl)piperazin-1-yl)methyl)isoindoline-1,3-dione;
2-(2,4-dioxotetrahydropyrimidin-1(2H)-yl)-5-((4-(3-fluorophenyl)piperazin-1-yl)methyl)isoindoline-1,3-dione;
2-(2,4-dioxotetrahydropyrimidin-1(2H)-yl)-5-((4-(4-fluorophenyl)piperazin-1-yl)methyl)isoindoline-1,3-dione;
5-((4-(2-chlorophenyl)piperazin-1-yl)methyl)-2-(2,4-dioxotetrahydropyrimidin-1(2H)-yl)isoindoline-1,3-dione;
5-((4-(4-chlorophenyl)piperazin-1-yl)methyl)-2-(2,4-dioxotetrahydropyrimidin-1(2H)-yl)isoindoline-1,3-dione;
5-((4-(2-bromophenyl)piperazin-1-yl)methyl)-2-(2,4-dioxotetrahydropyrimidin-1(2H)-yl)isoindoline-1,3-dione;
5-((4-(3-bromophenyl)piperazin-1-yl)methyl)-2-(2,4-dioxotetrahydropyrimidin-1(2H)-yl)isoindoline-1,3-dione;
5-((4-(4-bromophenyl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(o-tolyl)piperazin-1-yl)methyl)isoindoline-1,3-dione;
2-(2,4-dioxotetrahydropyrimidin-1(2H)-yl)-5-((4-(m-tolyl)piperazin-1-yl)methyl)isoindoline-1,3-dione;
2-(2,4-dioxotetrahydropyrimidin-1(2H)-yl)-5-((4-(p-tolyl)piperazin-1-yl)methyl)isoindoline-1,3-dione;
5-((4-(2,4-dichlorophenyl)piperazin-1-yl)methyl)-2-(2,4-dioxotetrahydropyrimidin-1(2H)-yl)isoindoline-1,3-dione;
5-((4-(2,5-dichlorophenyl)piperazin-1-yl)methyl)-2-(2,4-dioxotetrahydropyrimidin-1(2H)-yl)isoindoline-1,3-dione;
5-((4-(2,6-dichlorophenyl)piperazin-1-yl)methyl)-2-(2,4-dioxotetrahydropyrimidin-1(2H)-yl)isoindoline-1,3-dione;
5-((4-(3,4-dichlorophenyl)piperazin-1-yl)methyl)-2-(2,4-dioxotetrahydropyrimidin-1(2H)-yl)isoindoline-1,3-dione;
5-((4-(3,5-dichlorophenyl)piperazin-1-yl)methyl)-2-(2,4-dioxotetrahydropyrimidin-1(2H)-yl)isoindoline-1,3-dione;
5-((4-(2,3-difluorophenyl)piperazin-1-yl)methyl)-2-(2,4-dioxotetrahydropyrimidin-1(2H)-yl)isoindoline-1,3-dione;
5-((4-(2,4-difluorophenyl)piperazin-1-yl)methyl)-2-(2,4-dioxotetrahydropyrimidin-1(2H)-yl)isoindoline-1,3-dione;
5-((4-(2,5-difluorophenyl)piperazin-1-yl)methyl)-2-(2,4-dioxotetrahydropyrimidin-1(2H)-yl)isoindoline-1,3-dione;
5-((4-(2,6-difluorophenyl)piperazin-1-yl)methyl)-2-(2,4-dioxotetrahydropyrimidin-1(2H)-yl)isoindoline-1,3-dione;
5-((4-(3,4-difluorophenyl)piperazin-1-yl)methyl)-2-(2,4-dioxotetrahydropyrimidin-1(2H)-yl)isoindoline-1,3-dione;
5-((4-(3,5-difluorophenyl)piperazin-1-yl)methyl)-2-(2,4-dioxotetrahydropyrimidin-1(2H)-yl)isoindoline-1,3-dione;
5-((4-(2,4-dimethylphenyl)piperazin-1-yl)methyl)-2-(2,4-dioxotetrahydropyrimidin-1(2H)-yl)isoindoline-1,3-dione;
5-((4-(5-chloro-2-methylphenyl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-phenylpiperidin-1-yl)methyl)isoindoline-1,3-dione;
5-((4-(3-bromophenyl)piperidin-1-yl)methyl)-2-(2,4-dioxotetrahydropyrimidin-1(2H)-yl)isoindoline-1,3-dione;
5-((4-(4-bromophenyl)piperidin-1-yl)methyl)-2-(2,4-dioxotetrahydropyrimidin-1(2H)-yl)isoindoline-1,3-dione;
5-((4-(2-chlorophenyl)piperidin-1-yl)methyl)-2-(2,4-dioxotetrahydropyrimidin-1(2H)-yl)isoindoline-1,3-dione;
5-((4-(4-chlorophenyl)piperidin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(2-fluorophenyl)piperidin-1-yl)methyl)isoindoline-1,3-dione;
2-(2,4-dioxotetrahydropyrimidin-1(2H)-yl)-5-((4-(4-fluorophenyl)piperidin-1-yl)methyl)isoindoline-1,3-dione;
2-(2,4-dioxotetrahydropyrimidin-1(2H)-yl)-5-((4-(2-(trifluoromethyl)phenyl)piperidin-1-yl)methyl)isoindoline-1,3-dione;
2-(2,4-dioxotetrahydropyrimidin-1(2H)-yl)-5-((4-(3-(trifluoromethyl)phenyl)piperidin-1-yl)methyl)isoindoline-1,3-dione;
5-((4-(2,3-dichlorophenyl)piperidin-1-yl)methyl)-2-(2,4-dioxotetrahydropyrimidin-1(2H)-yl)isoindoline-1,3-dione;
5-((4-(2-chloro-3-fluorophenyl)piperidin-1-yl)methyl)-2-(2,4-dioxotetrahydropyrimidin-1(2H)-yl)isoindoline-1,3-dione;
5-((4-(2,3-difluorophenyl)piperidin-1-yl)methyl)-2-(2,4-dioxotetrahydropyrimidin-1(2H)-yl)isoindoline-1,3-dione;
5-((4-(2,3-dichlorophenyl)-3,6-dihydropyridin-1(2H)-yl)methyl)-2-(2,4-dioxotetrahydropyrimidin-1(2H)-yl)isoindoline-1,3-dione;

5-((4-(2-chloro-3-fluorophenyl)-3,6-dihydropyridin-1(2H)-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(pyridin-2-yl)piperazin-1-yl)methyl)isoindoline-1,3-dione;
2-(2,4-dioxotetrahydropyrimidin-1(2H)-yl)-5-((4-(6-methylpyridin-2-yl)piperazin-1-yl)methyl)isoindoline-1,3-dione;
5-((4-(6-chloropyridin-2-yl)piperazin-1-yl)methyl)-2-(2,4-dioxotetrahydropyrimidin-1(2H)-yl)isoindoline-1,3-dione;
5-((4-(5-chloropyridin-2-yl)piperazin-1-yl)methyl)-2-(2,4-dioxotetrahydropyrimidin-1(2H)-yl)isoindoline-1,3-dione;
5-((4-(4-chloropyridin-2-yl)piperazin-1-yl)methyl)-2-(2,4-dioxotetrahydropyrimidin-1(2H)-yl)isoindoline-1,3-dione;
5-((4-(3-chloropyridin-2-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(3-fluoropyridin-2-yl)piperazin-1-yl)methyl)isoindoline-1,3-dione;
2-(2,4-dioxotetrahydropyrimidin-1(2H)-yl)-5-((4-(6-fluoropyridin-2-yl)piperazin-1-yl)methyl)isoindoline-1,3-dione;
2-(2,4-dioxotetrahydropyrimidin-1(2H)-yl)-5-((4-(5-fluoropyridin-2-yl)piperazin-1-yl)methyl)isoindoline-1,3-dione;
2-(2,4-dioxotetrahydropyrimidin-1(2H)-yl)-5-((4-(3-(trifluoromethyl)pyridin-2-yl)piperazin-1-yl)methyl)isoindoline-1,3-dione;
2-(2,4-dioxotetrahydropyrimidin-1(2H)-yl)-5-((4-(6-(trifluoromethyl)pyridin-2-yl)piperazin-1-yl)methyl)isoindoline-1,3-dione;
2-(2,4-dioxotetrahydropyrimidin-1(2H)-yl)-5-((4-(5-(trifluoromethyl)pyridin-2-yl)piperazin-1-yl)methyl)isoindoline-1,3-dione;
5-((4-(3,4-dichloropyridin-2-yl)piperazin-1-yl)methyl)-2-(2,4-dioxotetrahydropyrimidin-1(2H)-yl)isoindoline-1,3-dione;
5-((4-(3,5-dichloropyridin-2-yl)piperazin-1-yl)methyl)-2-(2,4-dioxotetrahydropyrimidin-1(2H)-yl)isoindoline-1,3-dione;
5-((4-(4,5-dichloropyridin-2-yl)piperazin-1-yl)methyl)-2-(2,4-dioxotetrahydropyrimidin-1(2H)-yl)isoindoline-1,3-dione;
5-((4-(3,4-difluoropyridin-2-yl)piperazin-1-yl)methyl)-2-(2,4-dioxotetrahydropyrimidin-1(2H)-yl)isoindoline-1,3-dione;
5-((4-(4-chloro-3-fluoropyridin-2-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(3-fluoro-4-iodopyridin-2-yl)piperazin-1-yl)methyl)isoindoline-1,3-dione;
5-((4-(3-bromo-4-chloropyridin-2-yl)piperazin-1-yl)methyl)-2-(2,4-dioxotetrahydropyrimidin-1(2H)-yl)isoindoline-1,3-dione;
5-((4-(3-chloro-5-(trifluoromethyl)pyridin-2-yl)piperazin-1-yl)methyl)-2-(2,4-dioxotetrahydropyrimidin-1(2H)-yl)isoindoline-1,3-dione;
5-((4-(4-chloro-6-methylpyridin-2-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(pyridin-3-yl)piperazin-1-yl)methyl)isoindoline-1,3-dione;
5-((4-(6-chloropyridin-3-yl)piperazin-1-yl)methyl)-2-(2,4-dioxotetrahydropyrimidin-1(2H)-yl)isoindoline-1,3-dione;
5-((4-(4,5-dichloropyridin-3-yl)piperazin-1-yl)methyl)-2-(2,4-dioxotetrahydropyrimidin-1(2H)-yl)isoindoline-1,3-dione;
5-((4-(2,4-dichloropyridin-3-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(pyridin-4-yl)piperazin-1-yl)methyl)isoindoline-1,3-dione;
2-(2,4-dioxotetrahydropyrimidin-1(2H)-yl)-5-((4-(2-methylpyridin-4-yl)piperazin-1-yl)methyl)isoindoline-1,3-dione;
5-((4-(3-bromopyridin-4-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(3-fluoropyridin-4-yl)piperazin-1-yl)methyl)isoindoline-1,3-dione;
5-((4-(3-chloropyridin-4-yl)piperazin-1-yl)methyl)-2-(2,4-dioxotetrahydropyrimidin-1(2H)-yl)isoindoline-1,3-dione;
5-((4-(2-chloropyridin-4-yl)piperazin-1-yl)methyl)-2-(2,4-dioxotetrahydropyrimidin-1(2H)-yl)isoindoline-1,3-dione;
5-((4-(2-chloro-3-fluoropyridin-4-yl)piperazin-1-yl)methyl)-2-(2,4-dioxotetrahydropyrimidin-1(2H)-yl)isoindoline-1,3-dione;
5-((4-(2-bromo-3-fluoropyridin-4-yl)piperazin-1-yl)methyl)-2-(2,4-dioxotetrahydropyrimidin-1(2H)-yl)isoindoline-1,3-dione;
5-((4-(3-bromo-2-chloropyridin-4-yl)piperazin-1-yl)methyl)-2-(2,4-dioxotetrahydropyrimidin-1(2H)-yl)isoindoline-1,3-dione;
5-((4-(2,3-dichloropyridin-4-yl)piperazin-1-yl)methyl)-2-(2,4-dioxotetrahydropyrimidin-1(2H)-yl)isoindoline-1,3-dione;
5-((4-(2,3-difluoropyridin-4-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(3-fluoro-2-hydroxypyridin-4-yl)piperazin-1-yl)methyl)isoindoline-1,3-dione;
5-((4-(3-bromo-5-chloropyridin-4-yl)piperazin-1-yl)methyl)-2-(2,4-dioxotetrahydropyrimidin-1(2H)-yl)isoindoline-1,3-dione;
5-((4-(3-chloro-5-fluoropyridin-4-yl)piperazin-1-yl)methyl)-2-(2,4-dioxotetrahydropyrimidin-1(2H)-yl)isoindoline-1,3-dione;
5-((4-(3,5-dichloropyridin-4-yl)piperazin-1-yl)methyl)-2-(2,4-dioxotetrahydropyrimidin-1(2H)-yl)isoindoline-1,3-dione;
5-((4-(2,6-dimethylpyridin-4-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(perfluoropyridin-4-yl)piperazin-1-yl)methyl)isoindoline-1,3-dione;
2-(2,4-dioxotetrahydropyrimidin-1(2H)-yl)-5-((4-(pyridazin-3-yl)piperazin-1-yl)methyl)isoindoline-1,3-dione;
5-((4-(6-chloropyridazin-3-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(pyrimidin-2-yl)piperazin-1-yl)methyl)isoindoline-1,3-dione;
2-(2,4-dioxotetrahydropyrimidin-1(2H)-yl)-5-((4-(pyrazin-2-yl)piperazin-1-yl)methyl)isoindoline-1,3-dione;
5-((4-(5,6-dichloropyrimidin-4-yl)piperazin-1-yl)methyl)-2-(2,4-dioxotetrahydropyrimidin-1(2H)-yl)isoindoline-1,3-dione;
5-((4-(5,6-dichloropyridazin-4-yl)piperazin-1-yl)methyl)-2-(2,4-dioxotetrahydropyrimidin-1(2H)-yl)isoindoline-1,3-dione;
5-((4-(3,6-dichloropyridazin-4-yl)piperazin-1-yl)methyl)-2-(2,4-dioxotetrahydropyrimidin-1(2H)-yl)isoindoline-1,3-dione;
5-((4-(1,3,5-triazin-2-yl)piperazin-1-yl)methyl)-2-(2,4-dioxotetrahydropyrimidin-1(2H)-yl)isoindoline-1,3-dione;
5-((4-(3,4-dichloropyridin-2-yl)piperidin-1-yl)methyl)-2-(2,4-dioxotetrahydropyrimidin-1(2H)-yl)isoindoline-1,3-dione;
5-((4-(2,3-dichloropyridin-4-yl)piperidin-1-yl)methyl)-2-(2,4-dioxotetrahydropyrimidin-1(2H)-yl)isoindoline-1,3-dione;
5-((4-(2,3-difluoropyridin-4-yl)piperidin-1-yl)methyl)-2-(2,4-dioxotetrahydropyrimidin-1(2H)-yl)isoindoline-1,3-dione;
5-([3,4'-bipiperidin]-1-ylmethyl)-2-(2,4-dioxotetrahydropyrimidin-1(2H)-yl)isoindoline-1,3-dione;
5-((3,4-dichloro-3',6'-dihydro-[2,4'-bipyridin]-1'(2'H)-yl)methyl)-2-(2,4-dioxotetrahydropyrimidin-1 (2H)-yl)isoindoline-1,3-dione;
5-((4,5-dichloro-3',6'-dihydro-[3,4'-bipyridin]-1'(2'H)-yl)methyl)-2-(2,4-dioxotetrahydropyrimidin-1 (2H)-yl)isoindoline-1,3-dione;
5-((5-chloro-3',6'-dihydro-[3,4'-bipyridin]-1'(2'H)-yl)methyl)-2-(2,4-dioxotetrahydropyrimidin-1(2H)-yl)isoindoline-1,3-dione;
5-((2',3'-dichloro-3,6-dihydro-[4,4'-bipyridin]-1(2H)-yl)methyl)-2-(2,4-dioxotetrahydropyrimidin-1(2H)-yl)isoindoline-1,3-dione;
2-(2,4-dioxotetrahydropyrimidin-1(2H)-yl)-5-((3-fluoro-3',6'-dihydro-[2,4'-bipyridin]-1'(2'H)-yl)methyl)isoindoline-1,3-dione;
5-((2',3'-difluoro-3,6-dihydro-[4,4'-bipyridin]-1(2H)-yl)methyl)-2-(2,4-dioxotetrahydropyrimidin-1(2H)-yl)isoindoline-1,3-dione;
5-((3-(2,3-dichlorophenyl)-3,6-diazabicyclo[3.1.1]heptan-6-yl)methyl)-2-(2,4-dioxotetrahydropyrimidin-1(2H)-yl)isoindoline-1,3-dione;

5-((6-(2,3-dichlorophenyl)-3,6-diazabicyclo[3.1.1]heptan-3-yl)methyl)-2-(2,4-dioxotetrahydropyrimidin-1(2H)-yl)isoindoline-1,3-dione;
5-(((1R,4R)-5-(2,3-dichlorophenyl)-2,5-diazabicyclo[2.2.1]heptan-2-yl)methyl)-2-(2,4-dioxotetrahydropyrimidin-1(2H)-yl)isoindoline-1,3-dione;
5-(((1S,4S)-5-(2,3-dichlorophenyl)-2,5-diazabicyclo[2.2.1]heptan-2-yl)methyl)-2-(2,4-dioxotetrahydropyrimidin-1(2H)-yl)isoindoline-1,3-dione;
5-((5-(2,3-dichlorophenyl)-2,5-diazabicyclo[2.2.2]octan-2-yl)methyl)-2-(2,4-dioxotetrahydropyrimidin-1(2H)-yl)isoindoline-1,3-dione;
5-((8-(2,3-dichlorophenyl)-3,8-diazabicyclo[3.2.1]octan-3-yl)methyl)-2-(2,4-dioxotetrahydropyrimidin-1(2H)-yl)isoindoline-1,3-dione;
5-((3-(2,3-dichlorophenyl)-3,8-diazabicyclo[3.2.1]octan-8-yl)methyl)-2-(2,4-dioxotetrahydropyrimidin-1(2H)-yl)isoindoline-1,3-dione;
5-((4-(2,3-dichlorophenyl)-1,4-diazepan-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(6-fluorobenzo[d]isoxazol-3-yl)piperidin-1-yl)methyl)isoindoline-1,3-dione;
5-((4-(benzo[d]isoxazol-3-yl)piperidin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(isoxazol-3-yl)piperidin-1-yl)methyl)isoindoline-1,3-dione;
2-(2,4-dioxotetrahydropyrimidin-1(2H)-yl)-5-((4-(6-fluorobenzo[d]isoxazol-3-yl)piperazin-1-yl)methyl)isoindoline-1,3-dione;
2-(2,4-dioxotetrahydropyrimidin-1(2H)-yl)-5-((4-(isoxazolo[4,5-c]pyridin-3-yl)piperazin-1-yl)methyl)isoindoline-1,3-dione;
2-(2,4-dioxotetrahydropyrimidin-1(2H)-yl)-5-((4-(6-fluorobenzo[d]isothiazol-3-yl)piperazin-1-yl)methyl)isoindoline-1,3-dione;
2-(2,4-dioxotetrahydropyrimidin-1(2H)-yl)-5-((4-(6-fluoro-1H-indazol-3-yl)piperazin-1-yl)methyl)isoindoline-1,3-dione;
5-((4-(1H-pyrrolo[2,3-b]pyridin-3-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(6-fluoro-1H-indol-3-yl)piperidin-1-yl)methyl)isoindoline-1,3-dione;
2-(2,4-dioxotetrahydropyrimidin-1(2H)-yl)-5-((4-(6-fluoro-1H-indazol-3-yl)piperidin-1-yl)methyl)isoindoline-1,3-dione;
2-(2,4-dioxotetrahydropyrimidin-1(2H)-yl)-5-((4-(6-fluoro-1-methyl-1H-indol-3-yl)piperidin-1-yl)methyl)isoindoline-1,3-dione;
2-(2,4-dioxotetrahydropyrimidin-1(2H)-yl)-5-((4-(5-fluoroindolin-1-yl)piperidin-1-yl)methyl)isoindoline-1,3-dione;
2-(2,4-dioxotetrahydropyrimidin-1(2H)-yl)-5-((4-(5-fluoro-1H-benzo[d]imidazol-1-yl)piperidin-1-yl)methyl)isoindoline-1,3-dione;
2-(2,4-dioxotetrahydropyrimidin-1(2H)-yl)-5-((4-(5-fluoro-2-oxo-2,3-dihydro-1H-benzo[d]imidazol-1-yl)piperidin-1-yl)methyl)isoindoline-1,3-dione;
5-((4-(1H-pyrrolo[2,3-b]pyridin-3-yl)piperidin-1-yl)methyl)-2-(2,4-dioxotetrahydropyrimidin-1(2H)-yl)isoindoline-1,3-dione;
5-((4-(1H-pyrrolo[2,3-b]pyridin-3-yl)-3,6-dihydropyridin-1(2H)-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(6-fluoro-1H-indol-3-yl)-3,6-dihydropyridin-1(2H)-yl)methyl)isoindoline-1,3-dione;
2-(2,4-dioxotetrahydropyrimidin-1(2H)-yl)-5-((4-(6-fluoro-1-methyl-1H-indol-3-yl)-3,6-dihydropyridin-1(2H)-yl)methyl)isoindoline-1,3-dione;
2-(2,4-dioxotetrahydropyrimidin-1(2H)-yl)-5-((4-(6-fluoro-1H-indazol-3-yl)-3,6-dihydropyridin-1(2H)-yl)methyl)isoindoline-1,3-dione;
2-(2,4-dioxotetrahydropyrimidin-1(2H)-yl)-5-((4-(6-fluoro-3,4-dihydroquinolin-1(2H)-yl)piperidin-1-yl)methyl)isoindoline-1,3-dione;
2-(2,4-dioxotetrahydropyrimidin-1(2H)-yl)-5-((4-(7-fluoro-2,3-dihydro-4H-benzo[b][1,4]oxazin-4-yl)piperidin-1-yl)methyl)isoindoline-1,3-dione;
2-(2,4-dioxotetrahydropyrimidin-1(2H)-yl)-5-((4-(7-fluoro-3-oxo-2,3-dihydro-4H-benzo[b][1,4]oxazin-4-yl)piperidin-1-yl)methyl)isoindoline-1,3-dione;
2-(2,4-dioxotetrahydropyrimidin-1(2H)-yl)-5-((4-(7-fluorochroman-4-yl)piperidin-1-yl)methyl)isoindoline-1,3-dione;
2-(2,4-dioxotetrahydropyrimidin-1(2H)-yl)-5-((4-(7-fluorochroman-4-ylidene)piperidin-1-yl)methyl)isoindoline-1,3-dione;
2-(2,4-dioxotetrahydropyrimidin-1(2H)-yl)-5-((4-(5-fluoronaphthalen-1-yl)piperidin-1-yl)methyl)isoindoline-1,3-dione;
2-(2,4-dioxotetrahydropyrimidin-1(2H)-yl)-5-((4-(7-fluoroquinolin-4-yl)piperidin-1-yl)methyl)isoindoline-1,3-dione;
2-(2,4-dioxotetrahydropyrimidin-1(2H)-yl)-5-((4-(isoquinolin-5-yl)piperidin-1-yl)methyl)isoindoline-1,3-dione;
2-(2,4-dioxotetrahydropyrimidin-1(2H)-yl)-5-((4-(5-fluoronaphthalen-1-yl)-3,6-dihydropyridin-1(2H)-yl)methyl)isoindoline-1,3-dione;
2-(2,4-dioxotetrahydropyrimidin-1(2H)-yl)-5-((4-(7-fluoroquinolin-4-yl)-3,6-dihydropyridin-1(2H)-yl)methyl)isoindoline-1,3-dione;
2-(2,4-dioxotetrahydropyrimidin-1(2H)-yl)-5-((4-(isoquinolin-5-yl)-3,6-dihydropyridin-1(2H)-yl)methyl)isoindoline-1,3-dione;
2-(2,4-dioxotetrahydropyrimidin-1(2H)-yl)-5-((3-(7-fluorochroman-4-yl)azetidin-1-yl)methyl)isoindoline-1,3-dione;
2-(2,4-dioxotetrahydropyrimidin-1(2H)-yl)-5-((3-(7-fluorochroman-4-ylidene)azetidin-1-yl)methyl)isoindoline-1,3-dione;
2-(2,4-dioxotetrahydropyrimidin-1(2H)-yl)-5-((3-(7-fluoroquinolin-4-yl)azetidin-1-yl)methyl)isoindoline-1,3-dione;
2-(2,4-dioxotetrahydropyrimidin-1(2H)-yl)-5-((3-(5-fluoroindolin-1-yl)azetidin-1-yl)methyl)isoindoline-1,3-dione;
2-(2,4-dioxotetrahydropyrimidin-1(2H)-yl)-5-((3-(5-fluoro-1H-benzo[d]imidazol-1-yl)azetidin-1-yl)methyl)isoindoline-1,3-dione;
2-(2,4-dioxotetrahydropyrimidin-1(2H)-yl)-5-((4-(thieno[2,3-d]pyrimidin-4-yl)piperazin-1-yl)methyl)isoindoline-1,3-dione;
2-(2,4-dioxotetrahydropyrimidin-1(2H)-yl)-5-((4-(5-methylthieno[2,3-d]pyrimidin-4-yl)piperazin-1-yl)methyl)isoindoline-1,3-dione;
2-(2,4-dioxotetrahydropyrimidin-1(2H)-yl)-5-((4-(6-methylthieno[2,3-d]pyrimidin-4-yl)piperazin-1-yl)methyl)isoindoline-1,3-dione;
2-(2,4-dioxotetrahydropyrimidin-1(2H)-yl)-5-((4-(6-isopropylthieno[2,3-d]pyrimidin-4-yl)piperazin-1-yl)methyl)isoindoline-1,3-dione;
5-((4-(5,6-dimethylthieno[2,3-d]pyrimidin-4-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(6-phenylthieno[2,3-d]pyrimidin-4-yl)piperazin-1-yl)methyl)isoindoline-1,3-dione;
5-((4-(6-bromothieno[2,3-d]pyrimidin-4-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(5-phenylthieno[2,3-d]pyrimidin-4-yl)piperazin-1-yl)methyl)isoindoline-1,3-dione;
2-(2,4-dioxotetrahydropyrimidin-1(2H)-yl)-5-((4-(5,6,7,8-tetrahydrobenzo[4,5]thieno[2,3-d]pyrimidin-4-yl)piperazin-1-yl)methyl)isoindoline-1,3-dione;
5-((4-(6,7-dihydro-5H-cyclopenta[4,5]thieno[2,3-d]pyrimidin-4-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(2-methyl-5-phenylthieno[2,3-d]pyrimidin-4-yl)piperazin-1-yl)methyl)isoindoline-1,3-dione;
2-(2,4-dioxotetrahydropyrimidin-1(2H)-yl)-5-((4-(2-methyl-5,6,7,8-tetrahydrobenzo[4,5]thieno[2,3-d]pyrimidin-4-yl)piperazin-1-yl)methyl)isoindoline-1,3-dione;
2-(2,4-dioxotetrahydropyrimidin-1(2H)-yl)-5-((4-(2-methyl-6,7-dihydro-5H-cyclopenta[4,5]thieno[2,3-d]pyrimidin-4-yl)piperazin-1-yl)methyl)isoindoline-1,3-dione;
2-(2,4-dioxotetrahydropyrimidin-1(2H)-yl)-5-((4-(2-methylthieno[2,3-d]pyrimidin-4-yl)piperazin-1-yl)methyl)isoindoline-1,3-dione;
2-(2,4-dioxotetrahydropyrimidin-1(2H)-yl)-5-((4-(thieno[2,3-d]pyrimidin-2-yl)piperazin-1-yl)methyl)isoindoline-1,3-dione;
5-((4-(5,6-dimethylthieno[2,3-d]pyrimidin-4-yl)-1,4-diazepan-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(thieno[2,3-d]pyrimidin-4-yl)piperidin-1-yl)methyl)isoindoline-1,3-dione;

2-(2,4-dioxotetrahydropyrimidin-1(2H)-yl)-5-((4-(6-methylthieno[2,3-d]pyrimidin-4-yl)piperidin-1-yl)methyl)isoindoline-1,3-dione;
5-((4-(5,6-dimethylthieno[2,3-d]pyrimidin-4-yl)piperidin-1-yl)methyl)-2-(2,4-dioxotetrahydropyrimidin-1(2H)-yl) isoindoline-1,3-dione;
2-(2,4-dioxotetrahydropyrimidin-1(2H)-yl)-5-((4-(6-phenylthieno[2,3-d]pyrimidin-4-yl)piperidin-1-yl)methyl)isoindoline-1,3-dione;
2-(2,4-dioxotetrahydropyrimidin-1(2H)-yl)-5-((4-(5-phenylthieno[2,3-d]pyrimidin-4-yl)piperidin-1-yl)methyl)isoindoline-1,3-dione;
2-(2,4-dioxotetrahydropyrimidin-1(2H)-yl)-5-((4-(5,6,7,8-tetrahydrobenzo[4,5]thieno[2,3-d]pyrimidin-4-yl)piperidin-1-yl)methyl)isoindoline-1,3-dione;
5-((4-(6,7-dihydro-5H-cyclopenta[4,5]thieno[2,3-d]pyrimidin-4-yl)piperidin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(2-methyl-5-phenylthieno[2,3-d]pyrimidin-4-yl)piperidin-1-yl) methyl)isoindoline-1,3-dione;
2-(2,4-dioxotetrahydropyrimidin-1(2H)-yl)-5-((4-(2-methyl-5,6,7,8-tetrahydrobenzo[4,5]thieno[2,3-d]pyrimidin-4-yl)piperidin-1-yl)methyl)isoindoline-1,3-dione;
2-(2,4-dioxotetrahydropyrimidin-1(2H)-yl)-5-((4-(2-methyl-6,7-dihydro-5H-cyclopenta[4,5]thieno[2,3-d]pyrimidin-4-yl)piperidin-1-yl)methyl)isoindoline-1,3-dione;
2-(2,4-dioxotetrahydropyrimidin-1(2H)-yl)-5-((4-(2-methylthieno[2,3-d]pyrimidin-4-yl)piperidin-1-yl)methyl)isoindoline-1,3-dione;
2-(2,4-dioxotetrahydropyrimidin-1(2H)-yl)-5-((4-(thieno[2,3-d]pyrimidin-2-yl)piperidin-1-yl)methyl)isoindoline-1,3-dione;
2-(2,4-dioxotetrahydropyrimidin-1(2H)-yl)-5-((4-(5-ethylpyrimidin-2-yl)piperidin-1-yl)methyl)isoindoline-1,3-dione;
2-(2,4-dioxotetrahydropyrimidin-1(2H)-yl)-5-((4-(thieno[2,3-d]pyrimidin-4-yl)-3,6-dihydropyridin-1(2H)-yl) methyl)isoindoline-1,3-dione;
2-(2,4-dioxotetrahydropyrimidin-1(2H)-yl)-5-((4-(5-methylthieno[2,3-d]pyrimidin-4-yl)-3,6-dihydropyridin-1(2H)-yl)methyl)isoindoline-1,3-dione;
2-(2,4-dioxotetrahydropyrimidin-1(2H)-yl)-5-((4-(6-methylthieno[2,3-d]pyrimidin-4-yl)-3,6-dihydropyridin-1(2H)-yl)methyl)isoindoline-1,3-dione;
2-(2,4-dioxotetrahydropyrimidin-1(2H)-yl)-5-((4-(6-isopropylthieno[2,3-d]pyrimidin-4-yl)-3,6-dihydropyridin-1(2H)-yl)methyl)isoindoline-1,3-dione;
5-((4-(5,6-dimethylthieno[2,3-d]pyrimidin-4-yl)-3,6-dihydropyridin-1(2H)-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(6-phenylthieno[2,3-d]pyrimidin-4-yl)-3,6-dihydropyridin-1(2H)-yl)methyl)isoindoline-1,3-dione;
2-(2,4-dioxotetrahydropyrimidin-1(2H)-yl)-5-((4-(5-phenylthieno[2,3-d]pyrimidin-4-yl)-3,6-dihydropyridin-1(2H)-yl)methyl)isoindoline-1,3-dione;
2-(2,4-dioxotetrahydropyrimidin-1(2H)-yl)-5-((4-(5,6,7,8-tetrahydrobenzo[4,5]thieno[2,3-d]pyrimidin-4-yl)-3,6-dihydropyridin-1(2H)-yl)methyl)isoindoline-1,3-dione;
5-((4-(6,7-dihydro-5H-cyclopenta[4,5]thieno[2,3-dlpyrimidin-4-yl)-3,6-dihydropyridin-1(2H)-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(2-methyl-5-phenylthieno[2,3-d]pyrimidin-4-yl)-3,6-dihydropyridin-1(2H)-yl)methyl)isoindoline-1,3-dione;
2-(2,4-dioxotetrahydropyrimidin-1(2H)-yl)-5-((4-(2-methyl-5,6,7,8-tetrahydrobenzo[4,5]thieno[2,3-d]pyrimidin-4-yl)-3,6-dihydropyridin-1(2H)-yl)methyl)isoindoline-1,3-dione;
2-(2,4-dioxotetrahydropyrimidin-1(2H)-yl)-5-((4-(2-methyl-6,7-dihydro-5H-cyclopenta[4,5]thieno[2,3-d]pyrimidin-4-yl)-3,6-dihydropyridin-1(2H)-yl)methyl)isoindoline-1,3-dione;
2-(2,4-dioxotetrahydropyrimidin-1(2H)-yl)-5-((4-(2-methylthieno[2,3-d]pyrimidin-4-yl)-3,6-dihydropyridin-1(2H)-yl)methyl)isoindoline-1,3-dione;
2-(2,4-dioxotetrahydropyrimidin-1(2H)-yl)-5-((4-(thieno[2,3-d]pyrimidin-2-yl)-3,6-dihydropyridin-1(2H)-yl) methyl)isoindoline-1,3-dione;
5-((4-(2-chloro-6,7-dihydrothieno[3,2-d]pyrimidin-4-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(2-morpholino-6,7-dihydrothieno[3,2-d]pyrimidin-4-yl)piperazin-1-yl)methyl)isoindoline-1,3-dione;
2-(2,4-dioxotetrahydropyrimidin-1(2H)-yl)-5-((4-(4-(piperazin-1-yl)-6,7-dihydrothieno[3,2-d]pyrimidin-2-yl)piperazin-1-yl)methyl)isoindoline-1,3-dione;

2-(2,4-dioxotetrahydropyrimidin-1(2H)-yl)-5-((4-(4-morpholino-6,7-dihydrothieno[3,2-d]pyrimidin-2-yl)piperazin-1-yl)methyl)isoindoline-1,3-dione;
2-(2,4-dioxotetrahydropyrimidin-1(2H)-yl)-5-((4-(2-(piperidin-1-yl)-6,7-dihydrothieno[3,2-d]pyrimidin-4-yl)piperazin-1-yl)methyl)isoindoline-1,3-dione;
2-(2,4-dioxotetrahydropyrimidin-1(2H)-yl)-5-((4-(2-(pyrrolidin-1-yl)-6,7-dihydrothieno[3,2-d]pyrimidin-4-yl)piperazin-1-yl)methyl)isoindoline-1,3-dione;
5-((4-(2-(azetidin-1-yl)-6,7-dihydrothieno[3,2-d]pyrimidin-4-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(thieno[3,2-dlpyrimidin-4-yl)piperazin-1-yl)methyl)isoindoline-1,3-dione;
5-((4-(2-chlorothieno[3,2-d]pyrimidin-4-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((4-(2-morpholinothieno[3,2-d]pyrimidin-4-yl)piperazin-1-yl)methyl)isoindoline-1,3-dione;
2-(2,4-dioxotetrahydropyrimidin-1(2H)-yl)-5-((4-(4-(piperazin-1-yl)thieno[3,2-d]pyrimidin-2-yl)piperazin-1-yl)methyl)isoindoline-1,3-dione;
2-(2,4-dioxotetrahydropyrimidin-1(2H)-yl)-5-((4-(4-morpholinothieno[3,2-d]pyrimidin-2-yl)piperazin-1-yl)methyl)isoindoline-1,3-dione;
5-((4-(benzo[b]thiophen-4-yl)piperazin-1-yl)methyl)-2-(2,4-dioxotetrahydropyrimidin-1(2H)-yl)isoindoline-1,3-dione;
2-(2,4-dioxotetrahydropyrimidin-1(2H)-yl)-5-((3-(trifluoromethyl)-5,6-dihydro-[1,2,4]triazolo[4,3-a]pyrazin-7(8H)-yl)methyl)isoindoline-1,3-dione;
5-((4-(2-((2,4-dimethylphenyl)thio)phenyl)piperazin-1-yl)methyl)-2-(2,4-dioxotetrahydropyrimidin-1(2H)-yl)isoindoline-1,3-dione;
5-((6,7-dihydrothieno[3,2-c]pyridin-5(4H)-yl)methyl)-2-(2,4-dioxotetrahydropyrimidin-1(2H)-yl)isoindoline-1,3-dione;
5-((4-(benzo[d]isothiazol-3-yl)piperazin-1-yl)methyl)-2-(2,4-dioxotetrahydropyrimidin-1(2H)-yl)isoindoline-1,3-dione;
5-((4-(4-((3r,5r,7r)-adamantan-1-yl)benzyl)piperazin-1-yl)methyl)-2-(2,4-dioxotetrahydropyrimidin-1(2H)-yl)isoindoline-1,3-dione;
5-((((3s,5s,7s)-adamantan-1-yl)amino)methyl)-2-(2,4-dioxotetrahydropyrimidin-1(2H)-yl)isoindoline-1,3-dione;
2-(2,4-dioxotetrahydropyrimidin-1(2H)-yl)-5-((((1r,3s,5R,7S)-3-hydroxyadamantan-1-yl)amino)methyl)isoindoline-1,3-dione;
1-((1R,4R)-7,7-dimethyl-2-oxobicyclo[2.2.1]heptan-1-yl)-N-((2-(2,4-dioxotetrahydropyrimidin-1(2H)-yl)-1,3-dioxoisoindolin-5-yl)methyl)methanesulfonamide;
5-((((1R,4S)-bicyclo[2.2.1]heptan-2-yl)oxy)methyl)-2-(2,4-dioxotetrahydropyrimidin-1(2H)-yl)isoindoline-1,3-dione; and
4-(4-(4-(((2-(2,4-dioxotetrahydropyrimidin-1(2H)-yl)-1-oxoisoindolin-4-yl)oxy)methyl)benzyl)piperazin-1-yl)-3-fluorobenzonitrile.

**12.** The compound of Formula (I) or salts, enantiomers, stereoisomers, solvates, or polymorphs thereof as claimed in any one of claims 1-11, which is hydrohalide (including hydrochloride, hydrobromide), sulfate, citrate, maleate, sulfonate, lactate, lactobionate, L-tartrate, fumarate, L-malate, α-ketoglutarate, hippurate, D-glucuronate, D-gluconate, α-D-glucoheptonate, glycolate, mucate, L-ascorbate, orotate, picrate, glycinate, alaninate, arginate, cinnamate, laurate, pamoate, sebacate, besylate, methanesulfonate, ethanesulfonate, edisylate, formate, acetate, 2,2-dichloroacetate, trimethylacetate, propionate, valerate, palmitate, triphenylacetate, 2-ethyl-butane-1,4-dioate, iodate, nicotinate, L-pyroglutamate, L-prolinate, ferulate, 2-hydroxyethanesulfonate, nitrate, gentisate, cholate, salicylate, terephthalate, glutarate, adipate, stearate, oleate, undecylenate, camphorate, camsylate, dodecylsulfate, phosphate, thiocyanate, dihydrogen phosphate, pyrophosphate, metaphosphate, oxalate, malonate, benzoate, mandelate, succinate, pyruvate, 4-chlorobenzenesulfonate, 1,5-naphthalenedisulfonate, 3-hydroxy-2-naphthoate, 1-hydroxy-2-naphthoate, 2-naphthalenesulfonate, trifluoroacetate, methanesulfonate, hippurate, glycolate, or 4-methylbenzenesulfonate of the compound of Formula (I).

**13.** A pharmaceutical composition, comprising: the compound of Formula (I) or pharmaceutically acceptable salts thereof as claimed in any one of claims 1-12, and at least one pharmaceutically acceptable carrier or excipient.

**14.** The pharmaceutical composition as claimed in claim 13, further comprising at least one additional therapeutic agent, e.g., an anticancer agent.

**15.** The compound of Formula (I) or salts, enantiomers, stereoisomers, solvates, or polymorphs thereof as claimed in any one of claims 1-12, for use in the prevention or treatment of a disease or disorder associated with a cereblon protein.

**16.** The compound of Formula (I) or salts, enantiomers, stereoisomers, solvates, or polymorphs thereof as claimed in claim 15, wherein the disease or disorder associated with cereblon protein comprises tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, or diabetes.

**17.** The compound of Formula (I) or pharmaceutically acceptable salts thereof as claimed in claim 15, wherein the disease or disorder associated with cereblon protein is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including chronic myelogenous leukemia, B-cell chronic lymphocytic leukemia, leukemia-associated anemia, acute myeloid leukemia (AML), chronic myeloid leukemia (CML), monocytic leukemia, myelomonocytic leukemia, acute lymphoblastic leukemia (ALL), acute T-lymphocytic leukemia, T-lymphocytic leukemia, chronic lymphocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, and lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

**18.** Use of the compound of Formula (I) or pharmaceutically acceptable salts thereof as claimed in any one of claims 1-12, or of the pharmaceutical composition as claimed in claim 13 or 14, for the manufacture of a medicament for the prevention or treatment of a disease or disorder associated with a cereblon protein.

**19.** A method for treating or preventing a disease or disorder associated with a cereblon protein in a subject, comprising administering to the subject a therapeutically effective amount of the compound of Formula (I) or pharmaceutically acceptable salts thereof as claimed in any one of claims 1-12, or the pharmaceutical composition as claimed in claim 13 or 14.

**20.** The use as claimed in claim 18, or the method as claimed in claim 19, wherein the disease or disorder associated with cereblon protein comprises tumors, cancers, infectious diseases, inflammatory diseases, autoimmune diseases, anemia, hemorrhagic shock, transplant rejection, multiple organ dysfunction syndrome (MODS), sarcoidosis, adult

respiratory distress syndrome, cardiovascular diseases, Richter syndrome (RS), acute liver failure, or diabetes.

**21.** The use as claimed in claim 18, or the method as claimed in claim 19, wherein the disease or disorder associated with cereblon protein is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including chronic myelogenous leukemia, B-cell chronic lymphocytic leukemia, leukemia-associated anemia, acute myeloid leukemia (AML), chronic myeloid leukemia (CML), monocytic leukemia, myelomonocytic leukemia, acute lymphoblastic leukemia (ALL), acute T-lymphocytic leukemia, T-lymphocytic leukemia, chronic lymphocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, and lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

**22.** A method for preparing a compound of Formula (II), comprising:

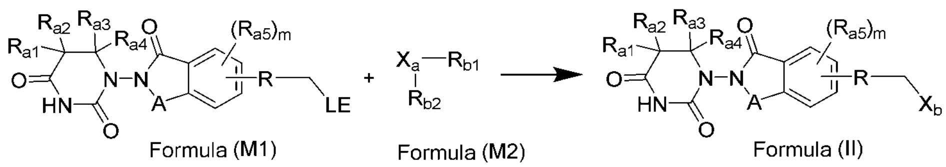

Formula (M1) Formula (M2) Formula (II)

wherein $R_{a1}$, $R_{a2}$, $R_{a3}$, $R_{a4}$, $(R_{a5})_m$, A, and R are as defined in claim 1; and wherein

(i) group LE represents Cl, Br, I, OMs, OTs, or ONs; group $X_a$ represents NH; group $R_{b1}$ represents $R_1$; group $R_{b2}$ represents $R_2$; and $X_b$ accordingly represents $NR_1R_2$, wherein $R_1$ and $R_2$ are as defined in any one of claims 1-11; or

(ii) group LE represents Cl, Br, I, OMs, OTs, or ONs; groups $R_{b1}$ and $R_{b2}$, together with the nitrogen atom to which they are attached, form an optionally substituted nitrogen-containing heterocycle $A_a$ represented by the following general formula:

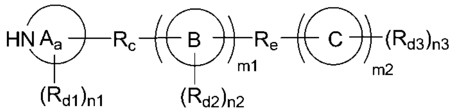

and $X_b$ accordingly represents a structure represented by the following formula:

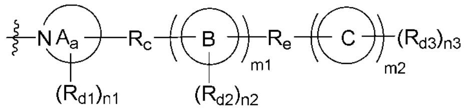

,

wherein nitrogen-containing heterocycle $A_a$ represents 4- to 30-membered nitrogen-containing heterocyclyl (preferably, 4- to 20-membered nitrogen-containing heterocyclyl; more preferably, 4- to 15-membered nitrogen-containing heterocyclyl); and $(R_{d1})_{n1}$, $R_c$, ring B, m1, $(R_{d2})_{n2}$, $R_c$, ring C, m2, and $(R_{d3})_{n3}$ are as defined in any one of claims 1-11; or

(iii) group LE represents C(O)Cl, COOH, or $S(O)_2Cl$; the compound of Formula (M2), $X_aR_{b1}R_{b2}$, is $NHR_3R_4$ or $NHR_{10}R_{11}$; and $X_b$ accordingly represents $C(O)NR_3R_4$ or $S(O)_2NR_{10}R_{11}$, wherein $R_3$, $R_4$, $R_{10}$, and $R_{11}$ are as defined in any one of claims 1-11; or

(iv) group LE represents $NH_2$; group $X_a$ represents C(O) or $S(O)_2$; group $R_{b1}$ represents OH or Cl; $R_{b2}$ is group $R_5$ or $R_{13}$ of the compound of formula (I) as claimed in any one of claims 1-11; and $X_b$ accordingly represents $NHC(O)R_5$ or $NHS(O)_2R_{13}$, wherein $R_5$ and $R_{13}$ are as defined in any one of claims 1-11; or

(v) group LE represents Cl, Br, or I; group $X_a$ represents O or S; group $R_{b1}$ represents H; $R_{b2}$ is group $R_8$ or $R_9$ of the compound of formula (I) as claimed in any one of claims 1-11; and $X_b$ accordingly represents $OR_8$ or $SR_9$, wherein $R_8$, and $R_9$ are as defined in any one of claims 1-11.

**23.** The method for preparing the compound of Formula (II) as claimed in claim 22, wherein when the group LE represents Cl, Br, or I, the compound of Formula (M1) is prepared by a halogenation reaction of a compound of formula (M3) with a hydrohalic acid:

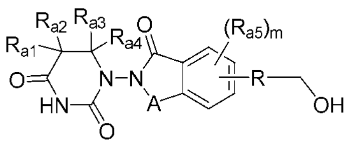

Formula (M3)

wherein $R_{a1}$, $R_{a2}$, $R_{a3}$, $R_{a4}$, $(R_{a5})_m$, and A are as defined in claim 1, and R represents a bond.

**24.** The method for preparing the compound of Formula (II) as claimed in claim 23, wherein the compound of Formula (M3) is prepared by a coupling reaction of a compound of Formula (M4) with (tributylstannyl)methanol:

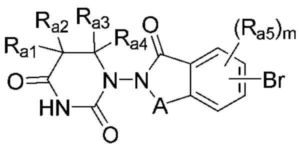

Formula (M4)

wherein $R_{a1}$, $R_{a2}$, $R_{a3}$, $R_{a4}$, $(R_{a5})_m$, and A are as defined in claim 1.

**25.** The method for preparing the compound of Formula (II) as claimed in claim 24, wherein the compound of Formula (M4) is prepared by an aminolysis reaction of an ester with an amine, wherein the ester is a compound of Formula (M6) and the amine is a compound of Formula (M5):

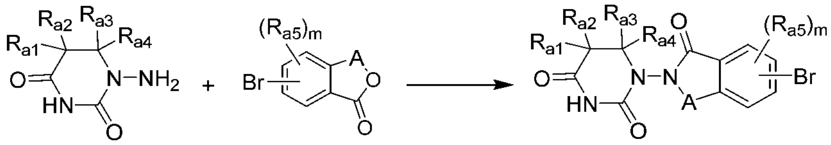

Formula (M5) Formula (M6) Formula (M4)

wherein $R_{a1}$, $R_{a2}$, $R_{a3}$, $R_{a4}$, $(R_{a5})_m$, and A are as defined in claim 1.

**26.** A method for preparing a compound of Formula (M4), comprising preparing the compound of Formula (M4) by an aminolysis reaction of an ester with an amine, wherein the ester is a compound of Formula (M6) and the amine is a compound of Formula (M5):

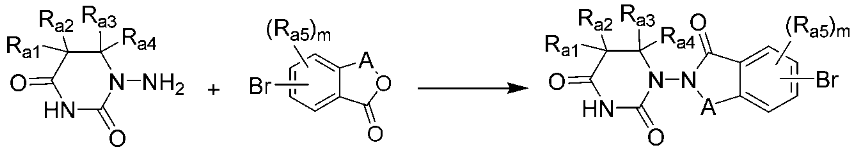

Formula (M5) Formula (M6) Formula (M4)

wherein $R_{a1}$, $R_{a2}$, $R_{a3}$, $R_{a4}$, $(R_{a5})_m$, and A are as defined in claim 1.

**27.** A method for preparing a compound of Formula (M1), comprising preparing the compound of Formula (M1) by a halogenation reaction of a compound of Formula (M3) with a hydrohalic acid:

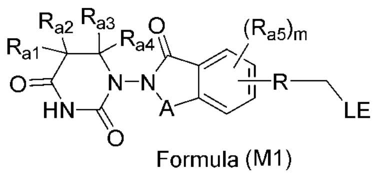

Formula (M1)

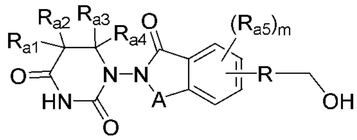

Formula (M3)

wherein group LE represents Cl, Br, or I; $R_{a1}$, $R_{a2}$, $R_{a3}$, $R_{a4}$, $(R_{a5})_m$, and A are as defined in claim 1; and R represents a bond.

**28.** The method for preparing the compound of Formula (M1) as claimed in claim 27, wherein the compound of Formula (M3) is prepared by a coupling reaction of a compound of Formula (M4) with (tributylstannyl)methanol:

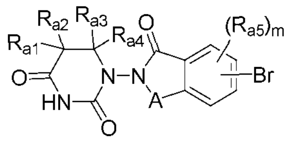

Formula (M4)

wherein $R_{a1}$, $R_{a2}$, $R_{a3}$, $R_{a4}$, $(R_{a5})_m$, and A are as defined in claim 1.

**29.** The method for preparing the compound of Formula (M1) as claimed in claim 28, comprising: preparing the compound of Formula (M4) by an aminolysis reaction of an ester with an amine, wherein the ester is a compound of Formula (M6) and the amine is a compound of Formula (M5):

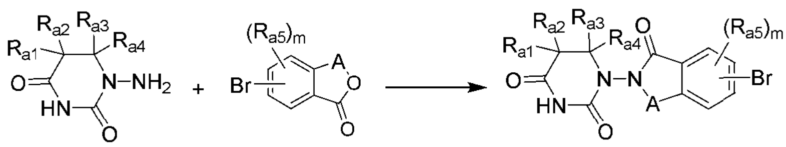

Formula (M5) Formula (M6) Formula (M4)

wherein $R_{a1}$, $R_{a2}$, $R_{a3}$, $R_{a4}$, $(R_{a5})_m$, and A are as defined in claim 1.

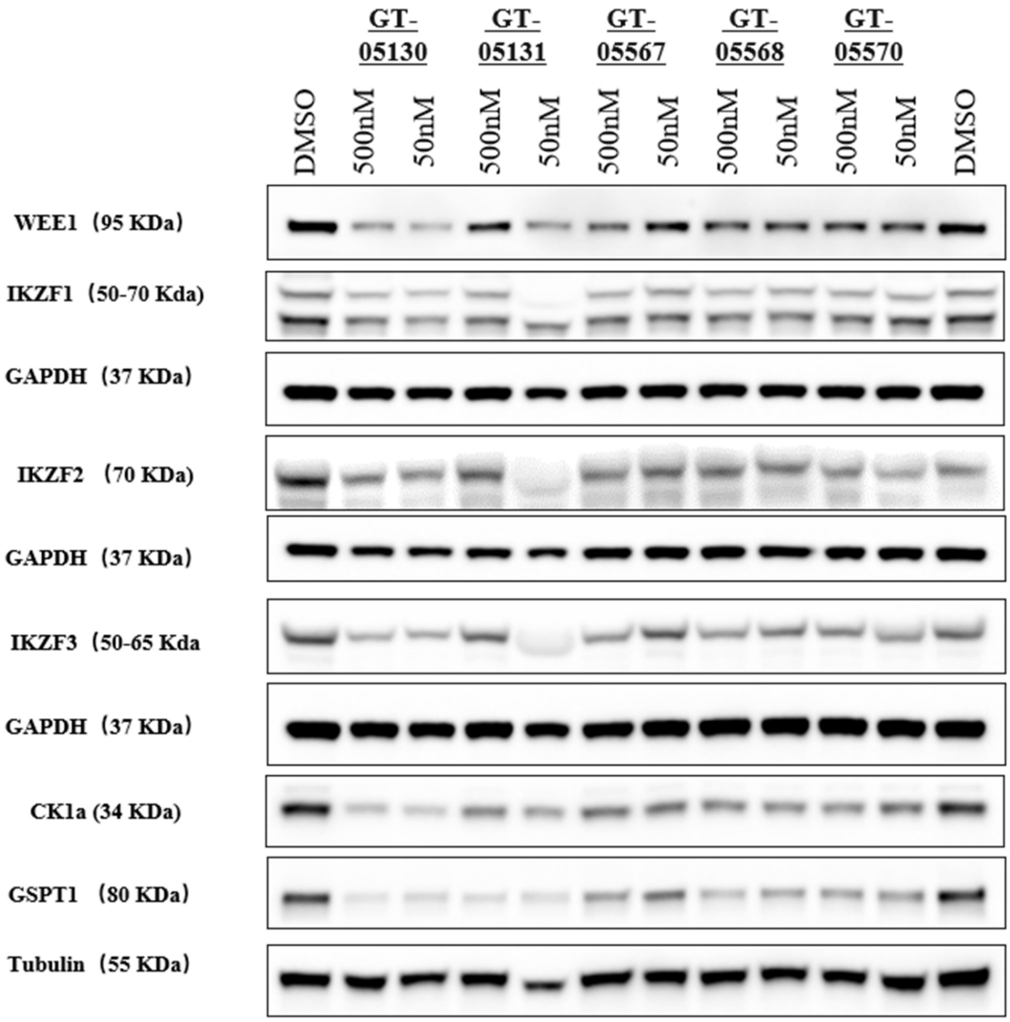
GT-05130
GT-05131
GT-05567
GT-05568
GT-05570
DMSO
500nM
50nM
500nM
50nM
500nM
50nM
500nM
50nM
500nM
50nM
DMSO
WEE1 (95 KDa)
IKZF1 (50-70 Kda)
GAPDH (37 KDa)
IKZF2 (70 KDa)
GAPDH (37 KDa)
IKZF3 (50-65 Kda
GAPDH (37 KDa)
CK1a (34 KDa)
GSPT1 (80 KDa)
Tubulin (55 KDa)

FIG. 1

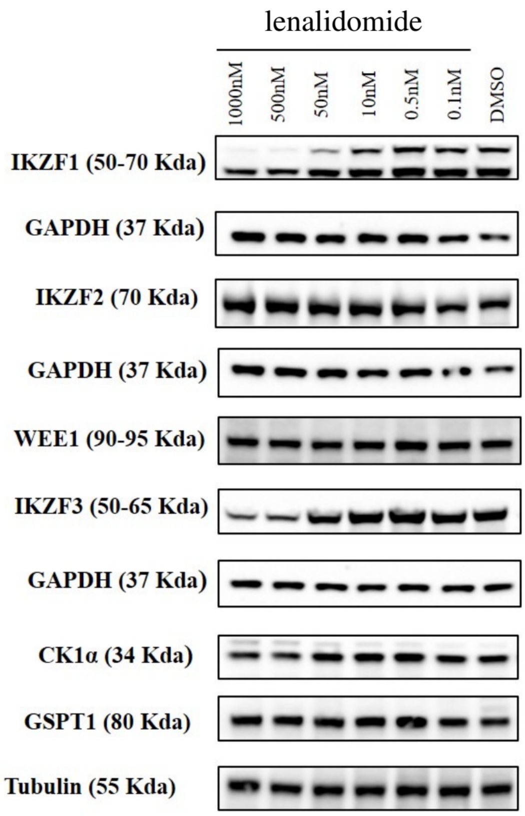


FIG. 1 (continued)

## INTERNATIONAL SEARCH REPORT

International application No.
**PCT/CN2024/097059**

**A. CLASSIFICATION OF SUBJECT MATTER**

C07D403/04(2006.01)i; C07D401/04(2006.01)i; C07D401/14 (2006.01)i; C07D487/00(2006.01)i; A61K31/505(2006.01)i; A61K31/454(2006.01)i; A61K31/517(2006.01)i; A61P37/02(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, ENTXTC, WPABS, WPABSC, STN(REGISTRY, CAPLUS, MARPAT): 标新生物, 杨小宝, 李岩, 周跃东, 孙仁红, 赵宝寅, 分子胶, E3泛素连接酶, 氧代异吲哚啉, 四氢嘧啶, 肿瘤, 炎症, 结构检索, structure search, GLUETACS, BIAOXIN, molecular glue?, cereblon, CRBN, oxoisoindolinyl, tetrahydropyrimidin?, cancer, inflam+

**C. DOCUMENTS CONSIDERED TO BE RELEVANT**

| Category* | Citation of document, with indication, where appropriate, of the relevant passages | Relevant to claim No. |
|---|---|---|
| X | CN 109153644 A (H. LEE MOFFITT CANCER CENTER & RESEARCH INSTITUTE, INC.) 04 January 2019 (2019-01-04)<br>claims 35 and 40, and description, paragraphs 0467 and 0928-0929 | 1-29 |
| Y | CN 109153644 A (H. LEE MOFFITT CANCER CENTER & RESEARCH INSTITUTE, INC.) 04 January 2019 (2019-01-04)<br>claims 35 and 40, and description, paragraphs 0467 and 0928-0929 | 1-29 |
| Y | CN 110612294 A (ARVINAS OPERATIONS, INC.) 24 December 2019 (2019-12-24)<br>claim 1 | 1-29 |
| Y | CN 116003418 A (GLUETACS THERAPEUTICS (SHANGHAI) CO., LTD.) 25 April 2023 (2023-04-25)<br>claims 1-50, and description, paragraphs 0240-0265 | 1-29 |
| Y | CN 111285850 A (SHANGHAI INSTITUTE OF MATERIA MEDICA, CHINESE ACADEMY OF SCIENCES) 16 June 2020 (2020-06-16)<br>claims 1-15 | 1-29 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 August 2024** | **05 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/097059**

**C. DOCUMENTS CONSIDERED TO BE RELEVANT**

| Category* | Citation of document, with indication, where appropriate, of the relevant passages | Relevant to claim No. |
|---|---|---|
| Y | CN 110963994 A (SHANGHAI INSTITUTE OF MATERIA MEDICA, CHINESE ACADEMY OF SCIENCES) 07 April 2020 (2020-04-07) claims 1-18 | 1-29 |
| Y | CN 111606883 A (SHANGHAITECH UNIVERSITY) 01 September 2020 (2020-09-01) claims 1-73, and description, paragraphs 0174-0212 | 1-29 |
| A | CN 105566290 A (KANGPU BIOPHARMACEUTICALS, LTD.) 11 May 2016 (2016-05-11) claims 1-13 | 1-29 |
| A | CN 112512589 A (H. LEE MOFFITT CANCER CENTER AND RESEARCH INSTITUTE, INC.) 16 March 2021 (2021-03-16) claims 1-35 | 1-29 |
| A | CN 113412259 A (NURIX THERAPEUTICS, INC.) 17 September 2021 (2021-09-17) claims 1-106 | 1-29 |
| A | WO 2020118098 A1 (VIVIDION THERAPEUTICS, INC.) 11 June 2020 (2020-06-11) claims 1-44 | 1-29 |

Form PCT/ISA/210 (second sheet) (July 2022)

**INTERNATIONAL SEARCH REPORT**

International application No.
**PCT/CN2024/097059**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19-21**
because they relate to subject matter not required to be searched by this Authority, namely:

The subject matter of claims 19-21 falls within the methods for treatment of a disease in a human body (PCT Rule 39.1(iv)). The search is carried out on the basis of the use of the compound or the pharmaceutical composition in the preparation of a drug for treating a corresponding disease.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/097059**

| Patent document cited in search report | Publication date (day/month/year) | Patent family member(s) | Publication date (day/month/year) |
|---|---|---|---|
| CN 109153644 A | 04 January 2019 | JP 2019512497 A | 16 May 2019 |
| | | JP 7264642 B2 | 25 April 2023 |
| | | BR 112018068906 A2 | 22 January 2019 |
| | | US 2021177825 A1 | 17 June 2021 |
| | | US 11395820 B2 | 26 July 2022 |
| | | MX 2018011216 A | 29 August 2019 |
| | | US 2023226035 A1 | 20 July 2023 |
| | | JP 2022105141 A | 12 July 2022 |
| | | AU 2022203645 A1 | 16 June 2022 |
| | | WO 2017161119 A1 | 21 September 2017 |
| | | EP 3429996 A1 | 23 January 2019 |
| | | EP 3429996 A4 | 12 February 2020 |
| | | CA 3017740 A1 | 21 September 2017 |
| | | AU 2017232906 A1 | 27 September 2018 |
| | | AU 2017232906 B2 | 31 March 2022 |
| | | MX 2022008085 A | 11 July 2022 |
| | | KR 20180125521 A | 23 November 2018 |
| | | KR 102520814 B1 | 11 April 2023 |
| CN 110612294 A | 24 December 2019 | KR 20190116315 A | 14 October 2019 |
| | | JP 2020506922 A | 05 March 2020 |
| | | CO 2019009424 A2 | 28 February 2020 |
| | | BR 112019015484 A2 | 28 April 2020 |
| | | AU 2022221386 A1 | 15 September 2022 |
| | | EP 3577109 A1 | 11 December 2019 |
| | | EP 3577109 A4 | 18 November 2020 |
| | | CA 3050309 A1 | 09 August 2018 |
| | | AU 2024203251 A1 | 06 June 2024 |
| | | IL 268069 A | 26 September 2019 |
| | | RU 2019123462 A | 27 January 2021 |
| | | RU 2019123462 A3 | 24 May 2021 |
| | | MX 2019009046 A | 30 October 2019 |
| | | MX 2023008056 A | 12 September 2023 |
| | | US 2023183209 A1 | 15 June 2023 |
| | | AU 2018215212 A1 | 11 July 2019 |
| | | AU 2018215212 B2 | 02 June 2022 |
| | | IL 312367 A | 01 June 2024 |
| | | WO 2018144649 A1 | 09 August 2018 |
| | | WO 2018144649 A8 | 22 August 2019 |
| | | JP 2024023277 A | 21 February 2024 |
| | | US 2018215731 A1 | 02 August 2018 |
| CN 116003418 A | 25 April 2023 | AU 2022370021 A1 | 09 May 2024 |
| | | KR 20240110576 A | 15 July 2024 |
| | | CA 3235512 A1 | 27 April 2023 |
| | | WO 2023066350 A1 | 27 April 2023 |
| CN 111285850 A | 16 June 2020 | CA 3122317 A1 | 11 June 2020 |
| | | CA 3122317 C | 03 October 2023 |
| | | AU 2019392231 A1 | 29 July 2021 |
| | | AU 2019392231 B2 | 20 October 2022 |
| | | WO 2020114482 A1 | 11 June 2020 |
| | | EP 3896062 A1 | 20 October 2021 |

Form PCT/ISA/210 (patent family annex) (July 2022)

**INTERNATIONAL SEARCH REPORT**
**Information on patent family members**

International application No.
**PCT/CN2024/097059**

| Patent document cited in search report | Publication date (day/month/year) | Patent family member(s) | Publication date (day/month/year) |
|---|---|---|---|
| | | EP 3896062 A4 | 15 June 2022 |
| | | US 2022041578 A1 | 10 February 2022 |
| CN 110963994 A | 07 April 2020 | BR 112021006458 A2 | 06 July 2021 |
| | | KR 20210069085 A | 10 June 2021 |
| | | KR 102672549 B1 | 10 June 2024 |
| | | CA 3121667 A1 | 02 April 2020 |
| | | AU 2019348094 A1 | 27 May 2021 |
| | | AU 2019348094 B2 | 22 December 2022 |
| | | WO 2020064002 A1 | 02 April 2020 |
| | | EP 3862348 A1 | 11 August 2021 |
| | | EP 3862348 A4 | 22 June 2022 |
| | | US 2022041576 A1 | 10 February 2022 |
| | | JP 2022503942 A | 12 January 2022 |
| | | JP 7168773 B2 | 09 November 2022 |
| CN 111606883 A | 01 September 2020 | AU 2020228803 A1 | 30 September 2021 |
| | | AU 2020228803 B2 | 18 May 2023 |
| | | WO 2020173426 A1 | 03 September 2020 |
| | | JP 2022521746 A | 12 April 2022 |
| | | JP 2024023405 A | 21 February 2024 |
| | | CA 3132308 A1 | 03 September 2020 |
| | | EP 3932922 A1 | 05 January 2022 |
| | | EP 3932922 A4 | 11 May 2022 |
| | | US 2022143002 A1 | 12 May 2022 |
| | | KR 20210142114 A | 24 November 2021 |
| CN 105566290 A | 11 May 2016 | PT 3214081 T | 31 August 2020 |
| | | PL 3643709 T3 | 21 February 2022 |
| | | DK 3643709 T3 | 20 December 2021 |
| | | KR 20170078740 A | 07 July 2017 |
| | | KR 102191256 B1 | 15 December 2020 |
| | | AU 2015341301 A1 | 01 June 2017 |
| | | AU 2015341301 B2 | 16 May 2019 |
| | | CA 2966038 A1 | 06 May 2016 |
| | | CA 2966038 C | 21 April 2020 |
| | | CY 1123361 T1 | 31 December 2021 |
| | | RU 2017118453 A | 04 December 2018 |
| | | RU 2017118453 A3 | 17 January 2019 |
| | | RU 2695521 C2 | 23 July 2019 |
| | | RU 2695521 C9 | 19 August 2019 |
| | | PL 3214081 T3 | 06 April 2021 |
| | | DK 3214081 T3 | 28 September 2020 |
| | | JP 2017533955 A | 16 November 2017 |
| | | JP 6546997 B2 | 17 July 2019 |
| | | PT 3643709 T | 20 December 2021 |
| | | US 2017313676 A1 | 02 November 2017 |
| | | US 10017492 B2 | 10 July 2018 |
| | | NZ 731789 A | 26 April 2019 |
| | | EP 3214081 A1 | 06 September 2017 |
| | | EP 3214081 A4 | 15 August 2018 |
| | | EP 3214081 B1 | 08 July 2020 |
| | | ES 2812877 T3 | 18 March 2021 |

Form PCT/ISA/210 (patent family annex) (July 2022)

**INTERNATIONAL SEARCH REPORT**
**Information on patent family members**

International application No.
**PCT/CN2024/097059**

| Patent document cited in search report | Publication date (day/month/year) | Patent family member(s) | Publication date (day/month/year) |
| --- | --- | --- | --- |
| | | ES 2901509 T3 | 22 March 2022 |
| | | EP 3643709 A1 | 29 April 2020 |
| | | EP 3643709 B1 | 20 October 2021 |
| | | WO 2016065980 A1 | 06 May 2016 |
| CN 112512589 A | 16 March 2021 | EA 202190248 A1 | 16 June 2021 |
| | | CL 2021003098 A1 | 09 September 2022 |
| | | EP 3820532 A2 | 19 May 2021 |
| | | EP 3820532 A4 | 06 April 2022 |
| | | SG 11202012464 WA | 28 January 2021 |
| | | CL 2021000061 A1 | 25 June 2021 |
| | | CA 3105506 A1 | 16 January 2020 |
| | | AU 2019301679 A1 | 28 January 2021 |
| | | BR 112021000395 A2 | 06 April 2021 |
| | | IL 279906 A | 01 March 2021 |
| | | WO 2020014489 A2 | 16 January 2020 |
| | | WO 2020014489 A3 | 30 July 2020 |
| | | MX 2021000295 A | 31 March 2021 |
| | | US 2022362229 A1 | 17 November 2022 |
| | | US 11730726 B2 | 22 August 2023 |
| | | CO 2021001292 A2 | 20 May 2021 |
| | | JP 2021532077 A | 25 November 2021 |
| | | PE 20210184 A1 | 02 February 2021 |
| | | KR 20210032430 A | 24 March 2021 |
| | | US 2024165095 A1 | 23 May 2024 |
| CN 113412259 A | 17 September 2021 | BR 122023024103 A2 | 20 February 2024 |
| | | AU 2019360928 A1 | 13 May 2021 |
| | | AU 2019360928 B2 | 09 November 2023 |
| | | US 2024124475 A1 | 18 April 2024 |
| | | US 2023029378 A1 | 26 January 2023 |
| | | US 11866442 B2 | 09 January 2024 |
| | | BR 112021007115 A2 | 20 July 2021 |
| | | WO 2020081450 A1 | 23 April 2020 |
| | | KR 20210077719 A | 25 June 2021 |
| | | US 11479556 B1 | 25 October 2022 |
| | | EP 3867242 A1 | 25 August 2021 |
| | | AU 2023266254 A1 | 07 December 2023 |
| | | IL 281904 A | 31 May 2021 |
| | | MX 2021004191 A | 27 May 2021 |
| | | JP 2022508705 A | 19 January 2022 |
| | | CA 3115526 A1 | 23 April 2020 |
| WO 2020118098 A1 | 11 June 2020 | US 2023045737 A1 | 09 February 2023 |
| | | EP 3891128 A1 | 13 October 2021 |
| | | EP 3891128 A4 | 17 August 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Clinical Pharmacology. People's Public Health Press, 2008
- *CHEMICAL ABSTRACTS*, 768-94-5